# EUROPEAN PATENT APPLICATION

(11) **EP 3 616 753 A1**
(43) Date of publication of application: **04.03.2020**
(21) Application number: 19194681.3
(22) Date of filing: 30.08.2019
(51) Int. Cl.: A61P 25/00, A61P 35/00, C07D 513/04, A61K 31/519

(54) **COMPOUNDS FOR MODULATING ADENOSINE A2B RECEPTOR AND ADENOSINE A2A RECEPTOR**

(30) Priority: 31.08.2018 US 201862725621 P
(71) Applicant: Corvus Pharmaceuticals, Inc., Burlingame, CA 94010 (US)
(72) Inventor: LI, Zhihong, Millbrae, California 94030 (US); FILONOVA, Lubov, Konstantinovna, Redwood City, California 94061 (US); VERNER, Erik, Belmont, California 94002 (US)
(74) Representative: HGF Limited

(57) **Abstract**

Disclosed herein, *inter alia,* are compounds of formula (I) and their use in methods for modulating Adenosine Receptors.

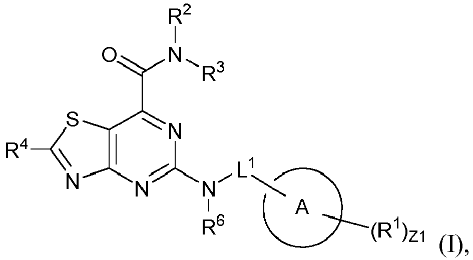

SEQUENCE LISTING

<110> Corvus Pharmaceuticals, Inc.
Li, Zhihong
Filonova, Lubov Konstantinovna
Verner, Erik

<120> COMPOUNDS AND METHODS FOR MODULATING ADENOSINE A2B RECEPTOR AND
ADENOSINE A2A RECEPTOR

<130> 048517-531001EP

<150> US 62/725,621
<151> 2018-08-31

<160> 2

<170> PatentIn version 3.5

<210> 1
<211> 411
<212> PRT
<213> Artificial Sequence

<220>
<223> Synthetic polypeptide

<400> 1

Met Pro Ile Met Gly Ser Ser Val Tyr Ile Thr Val Glu Leu Ala Ile
1               5                   10                  15

Ala Val Leu Ala Ile Leu Gly Asn Val Leu Val Cys Trp Ala Val Trp
            20                  25                  30

Leu Asn Ser Asn Leu Gln Asn Val Thr Asn Tyr Phe Val Val Ser Leu
        35                  40                  45

Ala Ala Ala Asp Ile Ala Val Gly Val Leu Ala Ile Pro Phe Ala Ile
    50                  55                  60

Thr Ile Ser Thr Gly Phe Cys Ala Ala Cys His Gly Cys Leu Phe Ile
65                  70                  75                  80

Ala Cys Phe Val Leu Val Leu Thr Gln Ser Ser Ile Phe Ser Leu Leu
                85                  90                  95

Ala Ile Ala Ile Asp Arg Tyr Ile Ala Ile Arg Ile Pro Leu Arg Tyr
            100                 105                 110

Asn Gly Leu Val Thr Gly Thr Arg Ala Lys Gly Ile Ile Ala Ile Cys
        115                 120                 125

Trp Val Leu Ser Phe Ala Ile Gly Leu Thr Pro Met Leu Gly Trp Asn
    130                 135                 140

Asn Cys Gly Gln Pro Lys Glu Gly Lys Asn His Ser Gln Gly Cys Gly
145                 150                 155                 160

Glu Gly Gln Val Ala Cys Leu Phe Glu Asp Val Pro Met Asn Tyr Met
                165                 170                 175

Val Tyr Phe Asn Phe Phe Ala Cys Val Leu Val Pro Leu Leu Leu Met
            180                 185                 190

Leu Gly Val Tyr Leu Arg Ile Phe Leu Ala Ala Arg Arg Gln Leu Lys
        195                 200                 205

Gln Met Glu Ser Gln Pro Leu Pro Gly Glu Arg Ala Arg Ser Thr Leu
    210                 215                 220

Gln Lys Glu Val His Ala Ala Lys Ser Leu Ala Ile Ile Val Gly Leu
225                 230                 235                 240

Phe Ala Leu Cys Trp Leu Pro Leu His Ile Ile Asn Cys Phe Thr Phe
                245                 250                 255

Phe Cys Pro Asp Cys Ser His Ala Pro Leu Trp Leu Met Tyr Leu Ala
            260                 265                 270

Ile Val Leu Ser His Thr Asn Ser Val Val Asn Pro Phe Ile Tyr Ala
        275                 280                 285

Tyr Arg Ile Arg Glu Phe Arg Gln Thr Phe Arg Lys Ile Ile Arg Ser
    290                 295                 300

His Val Leu Arg Gln Gln Glu Pro Phe Lys Ala Ala Gly Thr Ser Ala
305                 310                 315                 320

Arg Val Leu Ala Ala His Gly Ser Asp Gly Glu Gln Val Ser Leu Arg
                325                 330                 335

Leu Asn Gly His Pro Pro Gly Val Trp Ala Asn Gly Ser Ala Pro His
            340                 345                 350

Pro Glu Arg Arg Pro Asn Gly Tyr Ala Leu Gly Leu Val Ser Gly Gly
        355                 360                 365

Ser Ala Gln Glu Ser Gln Gly Asn Thr Gly Leu Pro Asp Val Glu Leu
    370                 375                 380

Leu Ser His Glu Leu Lys Gly Val Cys Pro Glu Pro Pro Gly Leu Asp

385                 390                 395                 400

Asp Pro Leu Ala Gln Asp Gly Ala Gly Val Ser
                405                 410

<210>  2
<211>  332
<212>  PRT
<213>  Artificial Sequence

<220>
<223>  Synthetic polypeptide

<400>  2

Met Leu Leu Glu Thr Gln Asp Ala Leu Tyr Val Ala Leu Glu Leu Val
1               5                   10                  15

Ile Ala Ala Leu Ser Val Ala Gly Asn Val Leu Val Cys Ala Ala Val
            20                  25                  30

Gly Thr Ala Asn Thr Leu Gln Thr Pro Thr Asn Tyr Phe Leu Val Ser
        35                  40                  45

Leu Ala Ala Ala Asp Val Ala Val Gly Leu Phe Ala Ile Pro Phe Ala
    50                  55                  60

Ile Thr Ile Ser Leu Gly Phe Cys Thr Asp Phe Tyr Gly Cys Leu Phe
65                  70                  75                  80

Leu Ala Cys Phe Val Leu Val Leu Thr Gln Ser Ser Ile Phe Ser Leu
                85                  90                  95

Leu Ala Val Ala Val Asp Arg Tyr Leu Ala Ile Cys Val Pro Leu Arg
            100                 105                 110

Tyr Lys Ser Leu Val Thr Gly Thr Arg Ala Arg Gly Val Ile Ala Val
        115                 120                 125

Leu Trp Val Leu Ala Phe Gly Ile Gly Leu Thr Pro Phe Leu Gly Trp
    130                 135                 140

Asn Ser Lys Asp Ser Ala Thr Asn Asn Cys Thr Glu Pro Trp Asp Gly
145                 150                 155                 160

Thr Thr Asn Glu Ser Cys Cys Leu Val Lys Cys Leu Phe Glu Asn Val
                165                 170                 175

Val Pro Met Ser Tyr Met Val Tyr Phe Asn Phe Phe Gly Cys Val Leu
            180                 185                 190

Pro Pro Leu Leu Ile Met Leu Val Ile Tyr Ile Lys Ile Phe Leu Val
        195                 200                 205

Ala Cys Arg Gln Leu Gln Arg Thr Glu Leu Met Asp His Ser Arg Thr
    210                 215                 220

Thr Leu Gln Arg Glu Ile His Ala Ala Lys Ser Leu Ala Met Ile Val
225                 230                 235                 240

Gly Ile Phe Ala Leu Cys Trp Leu Pro Val His Ala Val Asn Cys Val
                245                 250                 255

Thr Leu Phe Gln Pro Ala Gln Gly Lys Asn Lys Pro Lys Trp Ala Met
            260                 265                 270

Asn Met Ala Ile Leu Leu Ser His Ala Asn Ser Val Val Asn Pro Ile
        275                 280                 285

Val Tyr Ala Tyr Arg Asn Arg Asp Phe Arg Tyr Thr Phe His Lys Ile
    290                 295                 300

Ile Ser Arg Tyr Leu Leu Cys Gln Ala Asp Val Lys Ser Gly Asn Gly
305                 310                 315                 320

Gln Ala Gly Val Gln Pro Ala Leu Gly Val Gly Leu
                325                 330

## Description

### CROSS-REFERENCES TO RELATED APPLICATIONS

This application claims the benefit of U.S. Provisional Application No. 62/725,621, filed August 31, 2018, which is incorporated herein by reference in its entirety and for all purposes.

### REFERENCE TO A "SEQUENCE LISTING," A TABLE, OR A COMPUTER PROGRAM LISTING APPENDIX SUBMITTED AS AN ASCII FILE

The Sequence Listing written in file 048517-531001EP_Sequence_Listing_ST25.txt, created August 23, 2019, 6,938 bytes, machine format IBM-PC, MS Windows operating system, is hereby incorporated by reference.

### BACKGROUND

G protein-coupled receptors constitute a large superfamily of transmembrane proteins that represent the target for nearly half of all marketed drugs. Two of the GPCR family members, the Adenosine A2B Receptor and the Adenosine A2A Receptor are involved in many physiological functions. Thus there is a need in the art for Adenosine A2B Receptor modulators and Adenosine A2A Receptor modulators. Provided herein, *inter alia*, are solutions to these and other problems in the art.

### BRIEF SUMMARY

In an aspect is provided a compound having the formula:

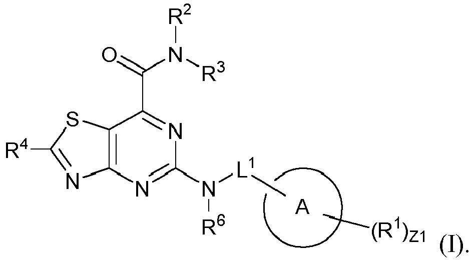

Ring A is a cycloalkyl, heterocycloalkyl, aryl, or heteroaryl.

L¹ is a bond, -SOₙ₅L^{1A}-, -SOᵥ₅NR⁵L^{1A}-, -NHC(O)NR⁵L^{1A}-, -NR⁵L^{1A}-, -C(O)L^{1A}-, -C(O)OL^{1A}-, -C(O)NR⁵L^{1A}-, -OL^{1A}-, -NR⁵SO₂L^{1A}-, -NR⁵C(O)L^{1A}-, -NR⁵C(O)OL^{1A}-, -NR⁵OL^{1A}-, -SL^{1A}-, substituted or unsubstituted alkylene, substituted or unsubstituted heteroalkylene, substituted or unsubstituted cycloalkylene, substituted or unsubstituted heterocycloalkylene, substituted or unsubstituted arylene, or substituted or unsubstituted heteroarylene.

R¹ is halogen, -CX¹₃, -CHX¹₂, -CH₂X¹, -OCX¹₃, -OCH₂X¹, -OCHX¹₂, -N₃, -CN, -SOₙ₁R^{1D}, -SOᵥ₁NR^{1A}R^{1B}, -NHC(O)NR^{1A}R^{1B}, -N(O)ₘ₁, -NR^{1A}R^{1B}, -C(O)R^{1C}, -C(O)-OR^{1C}, -C(O)NR^{1A}R^{1B}, -OR^{1D}, -NR^{1A}SO₂R^{1D}, -NR^{1A}C(O)R^{1C}, -NR^{1A}C(O)OR^{1C}, -NR^{1A}OR^{1C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

R² is hydrogen, -CX²₃, -CHX²₂, -CH₂X², -OCX²₃, -OCH₂X², -OCHX²₂, -C(O)R^{2C}, -C(O)OR^{2C}, -C(O)NR^{2A}R^{2B}, -OR^{2D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

R³ is hydrogen, -CX³₃, -CHX³₂, -CH₂X³, -OCX³₃, -OCH₂X³, -OCHX³₂, -C(O)R^{3C}, -C(O)OR^{3C}, -C(O)NR^{3A}R^{3B}, -OR^{3D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

R² and R³ together with the nitrogen attached thereto may be optionally joined to form a substituted or unsubstituted heterocycloalkyl, or substituted or unsubstituted heteroaryl.

R⁴ is hydrogen, halogen, -CX⁴₃, -CHX⁴₂, -CH₂X⁴, -OCX⁴₃, -OCH₂X⁴, -OCHX⁴₂, -N₃, -CN, -SOₙ₄R^{4D}, -SOᵥ₄NR^{4A}R^{4B}, -NHC(O)NR^{4A}R^{4B}, -N(O)ₘ₄, -NR^{4A}R^{4B}, -C(O)R^{4C}, -C(O)-OR^{4C}, -C(O)NR^{4A}R^{4B}, -OR^{4D}, -NR^{4A}SO₂R^{4D}, -NR^{4A}C(O)R^{4C}, -NR^{4A}C(O)OR^{4C}, -NR^{4A}OR^{4C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

R⁵ is hydrogen, -CX⁵₃, -CHX⁵₂, -CH₂X⁵, -OCX⁵₃, -OCH₂X⁵, -OCHX⁵₂, -C(O)R^{5C}, -C(O)OR^{5C}, -C(O)NR^{5A}R^{5B}, -OR^{5D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

R⁶ is hydrogen, -CX⁶₃, -CHX⁶₂, -CH₂X⁶, -OCX⁶₃, -OCH₂X⁶, -OCHX⁶₂, -C(O)R^{6C}, -C(O)OR^{6C}, -C(O)NR^{6A}R^{6B}, -OR^{6D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

R^{1A}, R^{1B}, R^{1C}, R^{1D}, R^{2A}, R^{2B}, R^{2C}, R^{2D}, R^{3A}, R^{3B}, R^{3C}, R^{3D}, R^{4A}, R^{4B}, R^{4C}, R^{4D}, R^{5A}, R^{5B}, R^{5C}, R^{5D}, R^{6A}, R^{6B}, R^{6C}, and R^{6D} are independently hydrogen, -CX₃, -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

L^{1A} is a bond, substituted or unsubstituted alkylene, substituted or unsubstituted heteroalkylene, substituted or unsubstituted cycloalkylene, substituted or unsubstituted heterocycloalkylene, substituted or unsubstituted arylene, or substituted or unsubstituted heteroarylene.

z1 is an integer from 0-12. n1 and n4 are independently an integer from 0 to 4. m1, m4, v1, and v4 are independently an integer from 1 to 2. X, X¹, X², X³, X⁴, X⁵ and X⁶ are independently -F, -Cl, -Br, or -I.

In an aspect is provided a pharmaceutical composition including a compound as described herein, and a pharmaceutically acceptable excipient.

In an aspect is provided a method of inhibiting Adenosine A2B Receptor activity and Adenosine A2A Receptor activity, the method including contacting the Adenosine A2B Receptor and Adenosine A2A Receptor with a compound as described herein, including embodiments.

In another aspect is provided a method of inhibiting Adenosine A2B Receptor activity, the method including contacting the Adenosine A2B Receptor with a compound as described herein, including embodiments.

In an aspect is provided a method of treating cancer, the method including administering to a subject in need thereof an effective amount of a compound as described herein, including embodiments.

In an aspect is provided a method of treating an inflammatory disease, said method comprising administering to a subject in need thereof an effective amount of a compound as described herein, including embodiments.

In an aspect is provided a method of treating a neurodegenerative disorder, said method comprising administering to a subject in need thereof an effective amount of a compound as described herein, including embodiments.

In an aspect is provided a method of treating a fibrotic disease, said method comprising administering to a subject in need thereof an effective amount of a compound as described herein, including embodiments.

In an aspect is provided a method of treating a disease or disorder associated with Adenosine A2B Receptor activity and/or Adenosine A2A Receptor activity, the method comprising administering to a subject in need thereof an effective amount of compound as described herein, including embodiments.

### DETAILED DESCRIPTION

### I. Definitions

The abbreviations used herein have their conventional meaning within the chemical and biological arts. The chemical structures and formulae set forth herein are constructed according to the standard rules of chemical valency known in the chemical arts.

Where substituent groups are specified by their conventional chemical formulae, written from left to right, they equally encompass the chemically identical substituents that would result from writing the structure from right to left, e.g., -CH₂O- is equivalent to-OCH₂-.

The term "alkyl," by itself or as part of another substituent, means, unless otherwise stated, a straight (i.e., unbranched) or branched carbon chain (or carbon), or combination thereof, which may be fully saturated, mono- or polyunsaturated and can include mono-, di- and multivalent radicals. The alkyl may include a designated number of carbons (e.g., C₁-C₁₀ means one to ten carbons). Alkyl is an uncyclized chain. Examples of saturated hydrocarbon radicals include, but are not limited to, groups such as methyl, ethyl, n-propyl, isopropyl, n-butyl, t-butyl, isobutyl, sec-butyl, methyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. An alkoxy is an alkyl attached to the remainder of the molecule via an oxygen linker (-O-). An alkyl moiety may be an alkenyl moiety. An alkyl moiety may be an alkynyl moiety. An alkyl moiety may be fully saturated. An alkenyl may include more than one double bond and/or one or more triple bonds in addition to the one or more double bonds. An alkynyl may include more than one triple bond and/or one or more double bonds in addition to the one or more triple bonds.

The term "alkylene," by itself or as part of another substituent, means, unless otherwise stated, a divalent radical derived from an alkyl, as exemplified, but not limited by, -CH₂CH₂CH₂CH₂-. Typically, an alkyl (or alkylene) group will have from 1 to 24 carbon atoms, with those groups having 10 or fewer carbon atoms being preferred herein. A "lower alkyl" or "lower alkylene" is a shorter chain alkyl or alkylene group, generally having eight or fewer carbon atoms. The term "alkenylene," by itself or as part of another substituent, means, unless otherwise stated, a divalent radical derived from an alkene.

The term "heteroalkyl," by itself or in combination with another term, means, unless otherwise stated, a stable straight or branched chain, or combinations thereof, including at least one carbon atom and at least one heteroatom (e.g., O, N, P, Si, and S), and wherein the nitrogen and sulfur atoms may optionally be oxidized, and the nitrogen heteroatom may optionally be quaternized. The heteroatom(s) (e.g., O, N, S, Si, or P) may be placed at any interior position of the heteroalkyl group or at the position at which the alkyl group is attached to the remainder of the molecule. Heteroalkyl is an uncyclized chain. Examples include, but are not limited to:
-CH₂-CH₂-O-CH₃, -CH₂-CH₂-NH-CH₃, -CH₂-CH₂-N(CH₃)-CH₃, -CH₂-S-CH₂-CH₃, -CH₂-S-CH₂, -S(O)-CH₃, -CH₂-CH₂-S(O)₂-CH₃, -CH=CH-O-CH₃, -Si(CH₃)₃, -CH₂-CH=N-OCH₃, -CH=CH-N(CH₃)-CH₃, -O-CH₃, -O-CH₂-CH₃, and -CN. Up to two or three heteroatoms may be consecutive, such as, for example, -CH₂-NH-OCH₃ and -CH₂-O-Si(CH₃)₃. A heteroalkyl moiety may include one heteroatom (e.g., O, N, S, Si, or P). A heteroalkyl moiety may include two optionally different heteroatoms (e.g., O, N, S, Si, or P). A heteroalkyl moiety may include three optionally different heteroatoms (e.g., O, N, S, Si, or P). A heteroalkyl moiety may include four optionally different heteroatoms (e.g., O, N, S, Si, or P). A heteroalkyl moiety may include five optionally different heteroatoms (e.g., O, N, S, Si, or P). A heteroalkyl moiety may include up to 8 optionally different heteroatoms (e.g., O, N, S, Si, or P). The term "heteroalkenyl," by itself or in combination with another term, means, unless otherwise stated, a heteroalkyl including at least one double bond. A heteroalkenyl may optionally include more than one double bond and/or one or more triple bonds in additional to the one or more double bonds. The term "heteroalkynyl," by itself or in combination with another term, means, unless otherwise stated, a heteroalkyl including at least one triple bond. A heteroalkynyl may optionally include more than one triple bond and/or one or more double bonds in additional to the one or more triple bonds.

Similarly, the term "heteroalkylene," by itself or as part of another substituent, means, unless otherwise stated, a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini (e.g., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula-C(O)₂R'- represents both -C(O)₂R'- and -R'C(O)₂-. As described above, heteroalkyl groups, as used herein, include those groups that are attached to the remainder of the molecule through a heteroatom, such as -C(O)R', -C(O)NR', -NR'R", -OR', -SR', and/or -SO₂R'. Where "heteroalkyl" is recited, followed by recitations of specific heteroalkyl groups, such as-NR'R" or the like, it will be understood that the terms heteroalkyl and -NR'R" are not redundant or mutually exclusive. Rather, the specific heteroalkyl groups are recited to add clarity. Thus, the term "heteroalkyl" should not be interpreted herein as excluding specific heteroalkyl groups, such as -NR'R" or the like.

The terms "cycloalkyl" and "heterocycloalkyl," by themselves or in combination with other terms, mean, unless otherwise stated, cyclic versions of "alkyl" and "heteroalkyl," respectively. Cycloalkyl and heterocycloalkyl are not aromatic. Additionally, for heterocycloalkyl, a heteroatom can occupy the position at which the heterocycle is attached to the remainder of the molecule. Examples of cycloalkyl include, but are not limited to, cyclopropyl, cyclobutyl, cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like. A "cycloalkylene" and a "heterocycloalkylene," alone or as part of another substituent, means a divalent radical derived from a cycloalkyl and heterocycloalkyl, respectively.

In embodiments, the term "cycloalkyl" means a monocyclic, bicyclic, or a multicyclic cycloalkyl ring system. In embodiments, monocyclic ring systems are cyclic hydrocarbon groups containing from 3 to 8 carbon atoms, where such groups can be saturated or unsaturated, but not aromatic. In embodiments, cycloalkyl groups are fully saturated. Examples of monocyclic cycloalkyls include cyclopropyl, cyclobutyl, cyclopentyl, cyclopentenyl, cyclohexyl, cyclohexenyl, cycloheptyl, and cyclooctyl. Bicyclic cycloalkyl ring systems are bridged monocyclic rings or fused bicyclic rings. In embodiments, bridged monocyclic rings contain a monocyclic cycloalkyl ring where two non adjacent carbon atoms of the monocyclic ring are linked by an alkylene bridge of between one and three additional carbon atoms (i.e., a bridging group of the form (CH₂)_{w}, where w is 1, 2, or 3). Representative examples of bicyclic ring systems include, but are not limited to, bicyclo[3.1.1]heptane, bicyclo[2.2.1]heptane, bicyclo[2.2.2]octane, bicyclo[3.2.2]nonane, bicyclo[3.3.1]nonane, and bicyclo[4.2.1]nonane. In embodiments, fused bicyclic cycloalkyl ring systems contain a monocyclic cycloalkyl ring fused to either a phenyl, a monocyclic cycloalkyl, a monocyclic cycloalkenyl, a monocyclic heterocyclyl, or a monocyclic heteroaryl. In embodiments, the bridged or fused bicyclic cycloalkyl is attached to the parent molecular moiety through any carbon atom contained within the monocyclic cycloalkyl ring. In embodiments, cycloalkyl groups are optionally substituted with one or two groups which are independently oxo or thia. In embodiments, the fused bicyclic cycloalkyl is a 5 or 6 membered monocyclic cycloalkyl ring fused to either a phenyl ring, a 5 or 6 membered monocyclic cycloalkyl, a 5 or 6 membered monocyclic cycloalkenyl, a 5 or 6 membered monocyclic heterocyclyl, or a 5 or 6 membered monocyclic heteroaryl, wherein the fused bicyclic cycloalkyl is optionally substituted by one or two groups which are independently oxo or thia. In embodiments, multicyclic cycloalkyl ring systems are a monocyclic cycloalkyl ring (base ring) fused to either (i) one ring system selected from the group consisting of a bicyclic aryl, a bicyclic heteroaryl, a bicyclic cycloalkyl, a bicyclic cycloalkenyl, and a bicyclic heterocyclyl; or (ii) two other ring systems independently selected from the group consisting of a phenyl, a bicyclic aryl, a monocyclic or bicyclic heteroaryl, a monocyclic or bicyclic cycloalkyl, a monocyclic or bicyclic cycloalkenyl, and a monocyclic or bicyclic heterocyclyl. In embodiments, the multicyclic cycloalkyl is attached to the parent molecular moiety through any carbon atom contained within the base ring. In embodiments, multicyclic cycloalkyl ring systems are a monocyclic cycloalkyl ring (base ring) fused to either (i) one ring system selected from the group consisting of a bicyclic aryl, a bicyclic heteroaryl, a bicyclic cycloalkyl, a bicyclic cycloalkenyl, and a bicyclic heterocyclyl; or (ii) two other ring systems independently selected from the group consisting of a phenyl, a monocyclic heteroaryl, a monocyclic cycloalkyl, a monocyclic cycloalkenyl, and a monocyclic heterocyclyl. Examples of multicyclic cycloalkyl groups include, but are not limited to tetradecahydrophenanthrenyl, perhydrophenothiazin-1-yl, and perhydrophenoxazin-1-yl.

In embodiments, a cycloalkyl is a cycloalkenyl. The term "cycloalkenyl" is used in accordance with its plain ordinary meaning. In embodiments, a cycloalkenyl is a monocyclic, bicyclic, or a multicyclic cycloalkenyl ring system. In embodiments, monocyclic cycloalkenyl ring systems are cyclic hydrocarbon groups containing from 3 to 8 carbon atoms, where such groups are unsaturated (i.e., containing at least one annular carbon carbon double bond), but not aromatic. Examples of monocyclic cycloalkenyl ring systems include cyclopentenyl and cyclohexenyl. In embodiments, bicyclic cycloalkenyl rings are bridged monocyclic rings or a fused bicyclic rings. In embodiments, bridged monocyclic rings contain a monocyclic cycloalkenyl ring where two non adjacent carbon atoms of the monocyclic ring are linked by an alkylene bridge of between one and three additional carbon atoms (i.e., a bridging group of the form (CH₂)_{w}, where w is 1, 2, or 3). Representative examples of bicyclic cycloalkenyls include, but are not limited to, norbornenyl and bicyclo[2.2.2]oct 2 enyl. In embodiments, fused bicyclic cycloalkenyl ring systems contain a monocyclic cycloalkenyl ring fused to either a phenyl, a monocyclic cycloalkyl, a monocyclic cycloalkenyl, a monocyclic heterocyclyl, or a monocyclic heteroaryl. In embodiments, the bridged or fused bicyclic cycloalkenyl is attached to the parent molecular moiety through any carbon atom contained within the monocyclic cycloalkenyl ring. In embodiments, cycloalkenyl groups are optionally substituted with one or two groups which are independently oxo or thia. In embodiments, multicyclic cycloalkenyl rings contain a monocyclic cycloalkenyl ring (base ring) fused to either (i) one ring system selected from the group consisting of a bicyclic aryl, a bicyclic heteroaryl, a bicyclic cycloalkyl, a bicyclic cycloalkenyl, and a bicyclic heterocyclyl; or (ii) two ring systems independently selected from the group consisting of a phenyl, a bicyclic aryl, a monocyclic or bicyclic heteroaryl, a monocyclic or bicyclic cycloalkyl, a monocyclic or bicyclic cycloalkenyl, and a monocyclic or bicyclic heterocyclyl. In embodiments, the multicyclic cycloalkenyl is attached to the parent molecular moiety through any carbon atom contained within the base ring. In embodiments, multicyclic cycloalkenyl rings contain a monocyclic cycloalkenyl ring (base ring) fused to either (i) one ring system selected from the group consisting of a bicyclic aryl, a bicyclic heteroaryl, a bicyclic cycloalkyl, a bicyclic cycloalkenyl, and a bicyclic heterocyclyl; or (ii) two ring systems independently selected from the group consisting of a phenyl, a monocyclic heteroaryl, a monocyclic cycloalkyl, a monocyclic cycloalkenyl, and a monocyclic heterocyclyl.

In embodiments, a heterocycloalkyl is a heterocyclyl. The term "heterocyclyl" as used herein, means a monocyclic, bicyclic, or multicyclic heterocycle. The heterocyclyl monocyclic heterocycle is a 3, 4, 5, 6 or 7 membered ring containing at least one heteroatom independently selected from the group consisting of O, N, and S where the ring is saturated or unsaturated, but not aromatic. The 3 or 4 membered ring contains 1 heteroatom selected from the group consisting of O, N and S. The 5 membered ring can contain zero or one double bond and one, two or three heteroatoms selected from the group consisting of O, N and S. The 6 or 7 membered ring contains zero, one or two double bonds and one, two or three heteroatoms selected from the group consisting of O, N and S. The heterocyclyl monocyclic heterocycle is connected to the parent molecular moiety through any carbon atom or any nitrogen atom contained within the heterocyclyl monocyclic heterocycle. Representative examples of heterocyclyl monocyclic heterocycles include, but are not limited to, azetidinyl, azepanyl, aziridinyl, diazepanyl, 1,3-dioxanyl, 1,3-dioxolanyl, 1,3-dithiolanyl, 1,3-dithianyl, imidazolinyl, imidazolidinyl, isothiazolinyl, isothiazolidinyl, isoxazolinyl, isoxazolidinyl, morpholinyl, oxadiazolinyl, oxadiazolidinyl, oxazolinyl, oxazolidinyl, piperazinyl, piperidinyl, pyranyl, pyrazolinyl, pyrazolidinyl, pyrrolinyl, pyrrolidinyl, tetrahydrofuranyl, tetrahydrothienyl, thiadiazolinyl, thiadiazolidinyl, thiazolinyl, thiazolidinyl, thiomorpholinyl, 1,1-dioxidothiomorpholinyl (thiomorpholine sulfone), thiopyranyl, and trithianyl. The heterocyclyl bicyclic heterocycle is a monocyclic heterocycle fused to either a phenyl, a monocyclic cycloalkyl, a monocyclic cycloalkenyl, a monocyclic heterocycle, or a monocyclic heteroaryl. The heterocyclyl bicyclic heterocycle is connected to the parent molecular moiety through any carbon atom or any nitrogen atom contained within the monocyclic heterocycle portion of the bicyclic ring system. Representative examples of bicyclic heterocyclyls include, but are not limited to, 2,3-dihydrobenzofuran-2-yl, 2,3-dihydrobenzofuran-3-yl, indolin-1-yl, indolin-2-yl, indolin-3-yl, 2,3-dihydrobenzothien-2-yl, decahydroquinolinyl, decahydroisoquinolinyl, octahydro-1H-indolyl, and octahydrobenzofuranyl. In embodiments, heterocyclyl groups are optionally substituted with one or two groups which are independently oxo or thia. In certain embodiments, the bicyclic heterocyclyl is a 5 or 6 membered monocyclic heterocyclyl ring fused to a phenyl ring, a 5 or 6 membered monocyclic cycloalkyl, a 5 or 6 membered monocyclic cycloalkenyl, a 5 or 6 membered monocyclic heterocyclyl, or a 5 or 6 membered monocyclic heteroaryl, wherein the bicyclic heterocyclyl is optionally substituted by one or two groups which are independently oxo or thia. Multicyclic heterocyclyl ring systems are a monocyclic heterocyclyl ring (base ring) fused to either (i) one ring system selected from the group consisting of a bicyclic aryl, a bicyclic heteroaryl, a bicyclic cycloalkyl, a bicyclic cycloalkenyl, and a bicyclic heterocyclyl; or (ii) two other ring systems independently selected from the group consisting of a phenyl, a bicyclic aryl, a monocyclic or bicyclic heteroaryl, a monocyclic or bicyclic cycloalkyl, a monocyclic or bicyclic cycloalkenyl, and a monocyclic or bicyclic heterocyclyl. The multicyclic heterocyclyl is attached to the parent molecular moiety through any carbon atom or nitrogen atom contained within the base ring. In embodiments, multicyclic heterocyclyl ring systems are a monocyclic heterocyclyl ring (base ring) fused to either (i) one ring system selected from the group consisting of a bicyclic aryl, a bicyclic heteroaryl, a bicyclic cycloalkyl, a bicyclic cycloalkenyl, and a bicyclic heterocyclyl; or (ii) two other ring systems independently selected from the group consisting of a phenyl, a monocyclic heteroaryl, a monocyclic cycloalkyl, a monocyclic cycloalkenyl, and a monocyclic heterocyclyl. Examples of multicyclic heterocyclyl groups include, but are not limited to 10H-phenothiazin-10-yl, 9,10-dihydroacridin-9-yl, 9,10-dihydroacridin-10-yl, 10H-phenoxazin-10-yl, 10,11-dihydro-5H-dibenzo[b,f]azepin-5-yl, 1,2,3,4-tetrahydropyrido[4,3-g]isoquinolin-2-yl, 12H-benzo[b]phenoxazin-12-yl, and dodecahydro-1H-carbazol-9-yl.

The terms "halo" or "halogen," by themselves or as part of another substituent, mean, unless otherwise stated, a fluorine, chlorine, bromine, or iodine atom. Additionally, terms such as "haloalkyl" are meant to include monohaloalkyl and polyhaloalkyl. For example, the term "halo(C₁-C₄)alkyl" includes, but is not limited to, fluoromethyl, difluoromethyl, trifluoromethyl, 2,2,2-trifluoroethyl, 4-chlorobutyl, 3-bromopropyl, and the like.

The term "acyl" means, unless otherwise stated, -C(O)R where R is a substituted or unsubstituted alkyl, substituted or unsubstituted cycloalkyl, substituted or unsubstituted heteroalkyl, substituted or unsubstituted heterocycloalkyl, substituted or unsubstituted aryl, or substituted or unsubstituted heteroaryl.

The term "aryl" means, unless otherwise stated, a polyunsaturated, aromatic, hydrocarbon substituent, which can be a single ring or multiple rings (preferably from 1 to 3 rings) that are fused together (i.e., a fused ring aryl) or linked covalently. A fused ring aryl refers to multiple rings fused together wherein at least one of the fused rings is an aryl ring. The term "heteroaryl" refers to aryl groups (or rings) that contain at least one heteroatom such as N, O, or S, wherein the nitrogen and sulfur atoms are optionally oxidized, and the nitrogen atom(s) are optionally quaternized. Thus, the term "heteroaryl" includes fused ring heteroaryl groups (i.e., multiple rings fused together wherein at least one of the fused rings is a heteroaromatic ring). A 5,6-fused ring heteroarylene refers to two rings fused together, wherein one ring has 5 members and the other ring has 6 members, and wherein at least one ring is a heteroaryl ring. Likewise, a 6,6-fused ring heteroarylene refers to two rings fused together, wherein one ring has 6 members and the other ring has 6 members, and wherein at least one ring is a heteroaryl ring. And a 6,5-fused ring heteroarylene refers to two rings fused together, wherein one ring has 6 members and the other ring has 5 members, and wherein at least one ring is a heteroaryl ring. A heteroaryl group can be attached to the remainder of the molecule through a carbon or heteroatom. Non-limiting examples of aryl and heteroaryl groups include phenyl, naphthyl, pyrrolyl, pyrazolyl, pyridazinyl, triazinyl, pyrimidinyl, imidazolyl, pyrazinyl, purinyl, oxazolyl, isoxazolyl, thiazolyl, furyl, thienyl, pyridyl, pyrimidyl, benzothiazolyl, benzoxazoyl benzimidazolyl, benzofuran, isobenzofuranyl, indolyl, isoindolyl, benzothiophenyl, isoquinolyl, quinoxalinyl, quinolyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of the above noted aryl and heteroaryl ring systems are selected from the group of acceptable substituents described below. An "arylene" and a "heteroarylene," alone or as part of another substituent, mean a divalent radical derived from an aryl and heteroaryl, respectively. A heteroaryl group substituent may be -O- bonded to a ring heteroatom nitrogen.

A fused ring heterocyloalkyl-aryl is an aryl fused to a heterocycloalkyl. A fused ring heterocycloalkyl-heteroaryl is a heteroaryl fused to a heterocycloalkyl. A fused ring heterocycloalkyl-cycloalkyl is a heterocycloalkyl fused to a cycloalkyl. A fused ring heterocycloalkyl-heterocycloalkyl is a heterocycloalkyl fused to another heterocycloalkyl. Fused ring heterocycloalkyl-aryl, fused ring heterocycloalkyl-heteroaryl, fused ring heterocycloalkyl-cycloalkyl, or fused ring heterocycloalkyl-heterocycloalkyl may each independently be unsubstituted or substituted with one or more of the substitutents described herein.

Spirocyclic rings are two or more rings wherein adjacent rings are attached through a single atom. The individual rings within spirocyclic rings may be identical or different. Individual rings in spirocyclic rings may be substituted or unsubstituted and may have different substituents from other individual rings within a set of spirocyclic rings. Possible substituents for individual rings within spirocyclic rings are the possible substituents for the same ring when not part of spirocyclic rings (e.g. substituents for cycloalkyl or heterocycloalkyl rings). Spirocylic rings may be substituted or unsubstituted cycloalkyl, substituted or unsubstituted cycloalkylene, substituted or unsubstituted heterocycloalkyl or substituted or unsubstituted heterocycloalkylene and individual rings within a spirocyclic ring group may be any of the immediately previous list, including having all rings of one type (e.g. all rings being substituted heterocycloalkylene wherein each ring may be the same or different substituted heterocycloalkylene). When referring to a spirocyclic ring system, heterocyclic spirocyclic rings means a spirocyclic rings wherein at least one ring is a heterocyclic ring and wherein each ring may be a different ring. When referring to a spirocyclic ring system, substituted spirocyclic rings means that at least one ring is substituted and each substituent may optionally be different.

The symbol " 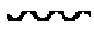 " denotes the point of attachment of a chemical moiety to the remainder of a molecule or chemical formula.

The term "oxo," as used herein, means an oxygen that is double bonded to a carbon atom.

The term "alkylsulfonyl," as used herein, means a moiety having the formula -S(O₂)-R', where R' is a substituted or unsubstituted alkyl group as defined above. R' may have a specified number of carbons (e.g., "C₁-C₄ alkylsulfonyl").

The term "alkylarylene" as an arylene moiety covalently bonded to an alkylene moiety (also referred to herein as an alkylene linker). In embodiments, the alkylarylene group has the formula:

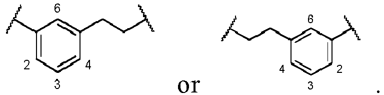

An alkylarylene moiety may be substituted (e.g. with a substituent group) on the alkylene moiety or the arylene linker (e.g. at carbons 2, 3, 4, or 6) with halogen, oxo, -N₃,-CF₃, -CCl₃, -CBr₃, -CI₃, -CN, -CHO, -OH, -NH₂, -COOH, -CONH₂, -NO₂, -SH, -SO₂CH₃-SO₃H, , -OSO₃H, -SO₂NH₂, -NHNH₂, -ONH₂, -NHC(O)NHNH₂, substituted or unsubstituted C₁-C₅ alkyl or substituted or unsubstituted 2 to 5 membered heteroalkyl). In embodiments, the alkylarylene is unsubstituted.

Each of the above terms (e.g., "alkyl," "heteroalkyl," "cycloalkyl," "heterocycloalkyl," "aryl," and "heteroaryl") includes both substituted and unsubstituted forms of the indicated radical. Preferred substituents for each type of radical are provided below.

Substituents for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) can be one or more of a variety of groups selected from, but not limited to, -OR', =O, =NR', =N-OR', -NR'R", -SR', -halogen,-SiR'R"R"', -OC(O)R',
-C(O)R', -CO₂R', -CONR'R", -OC(O)NR'R", -NR"C(O)R', -NR'-C(O)NR"R"', -NR"C(O)₂R', -NR-C(NR'R"R"')=NR"", -NR-C(NR'R")=NR"', -S(O)R', -S(O)₂R', -S(O)₂NR'R", -NRSO₂R', -NR'NR'R'", -ONR'R", -NR'C(O)NR''NR'''R'''', -CN, -NO₂, -NR'SO₂R", -NR'C(O)R", -NR'C(O)-OR", -NR'OR", in a number ranging from zero to (2m'+1), where m' is the total number of carbon atoms in such radical. R, R', R", R"', and R"" each preferably independently refer to hydrogen, substituted or unsubstituted heteroalkyl, substituted or unsubstituted cycloalkyl, substituted or unsubstituted heterocycloalkyl, substituted or unsubstituted aryl (e.g., aryl substituted with 1-3 halogens), substituted or unsubstituted heteroaryl, substituted or unsubstituted alkyl, alkoxy, or thioalkoxy groups, or arylalkyl groups. When a compound described herein includes more than one R group, for example, each of the R groups is independently selected as are each R', R", R"', and R"" group when more than one of these groups is present. When R' and R" are attached to the same nitrogen atom, they can be combined with the nitrogen atom to form a 4-, 5-, 6-, or 7-membered ring. For example, - NR'R" includes, but is not limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl (e.g., -CF₃ and -CH₂CF₃) and acyl (e.g., -C(O)CH₃, -C(O)CF₃, -C(O)CH₂OCH₃, and the like).

Similar to the substituents described for the alkyl radical, substituents for the aryl and heteroaryl groups are varied and are selected from, for example: -OR', -NR'R", -SR', -halogen, -SiR'R"R"', -OC(O)R', -C(O)R', -CO₂R', -CONR'R", -OC(O)NR'R", -NR"C(O)R', -NR'-C(O)NR"R"', -NR"C(O)₂R', -NR-C(NR'R"R"')=NR"", -NR-C(NR'R")=NR"', -S(O)R', -S(O)₂R', -S(O)₂NR'R", -NRSO₂R', -NR'NR"R"', -ONR'R", -NR'C(O)NR''NR'''R'''', -CN, -NO₂, -R', -N₃, -CH(Ph)₂, fluoro(C₁-C₄)alkoxy, and fluoro(C₁-C₄)alkyl, -NR'SO₂R", -NR'C(O)R", -NR'C(O)-OR", -NR'OR", in a number ranging from zero to the total number of open valences on the aromatic ring system; and where R', R", R'", and R"" are preferably independently selected from hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl, and substituted or unsubstituted heteroaryl. When a compound described herein includes more than one R group, for example, each of the R groups is independently selected as are each R', R", R"', and R"" groups when more than one of these groups is present.

Substituents for rings (e.g. cycloalkyl, heterocycloalkyl, aryl, heteroaryl, cycloalkylene, heterocycloalkylene, arylene, or heteroarylene) may be depicted as substituents on the ring rather than on a specific atom of a ring (commonly referred to as a floating substituent). In such a case, the substituent may be attached to any of the ring atoms (obeying the rules of chemical valency) and in the case of fused rings or spirocyclic rings, a substituent depicted as associated with one member of the fused rings or spirocyclic rings (a floating substituent on a single ring), may be a substituent on any of the fused rings or spirocyclic rings (a floating substituent on multiple rings). When a substituent is attached to a ring, but not a specific atom (a floating substituent), and a subscript for the substituent is an integer greater than one, the multiple substituents may be on the same atom, same ring, different atoms, different fused rings, different spirocyclic rings, and each substituent may optionally be different. Where a point of attachment of a ring to the remainder of a molecule is not limited to a single atom (a floating substituent), the attachment point may be any atom of the ring and in the case of a fused ring or spirocyclic ring, any atom of any of the fused rings or spirocyclic rings while obeying the rules of chemical valency. Where a ring, fused rings, or spirocyclic rings contain one or more ring heteroatoms and the ring, fused rings, or spirocyclic rings are shown with one more floating substituents (including, but not limited to, points of attachment to the remainder of the molecule), the floating substituents may be bonded to the heteroatoms. Where the ring heteroatoms are shown bound to one or more hydrogens (e.g. a ring nitrogen with two bonds to ring atoms and a third bond to a hydrogen) in the structure or formula with the floating substituent, when the heteroatom is bonded to the floating substituent, the substituent will be understood to replace the hydrogen, while obeying the rules of chemical valency.

Two or more substituents may optionally be joined to form aryl, heteroaryl, cycloalkyl, or heterocycloalkyl groups. Such so-called ring-forming substituents are typically, though not necessarily, found attached to a cyclic base structure. In one embodiment, the ring-forming substituents are attached to adjacent members of the base structure. For example, two ring-forming substituents attached to adjacent members of a cyclic base structure create a fused ring structure. In another embodiment, the ring-forming substituents are attached to a single member of the base structure. For example, two ring-forming substituents attached to a single member of a cyclic base structure create a spirocyclic structure. In yet another embodiment, the ring-forming substituents are attached to non-adjacent members of the base structure.

Two of the substituents on adjacent atoms of the aryl or heteroaryl ring may optionally form a ring of the formula -T-C(O)-(CRR')_{q}-U-, wherein T and U are independently -NR-, -O-, -CRR'-, or a single bond, and q is an integer of from 0 to 3. Alternatively, two of the substituents on adjacent atoms of the aryl or heteroaryl ring may optionally be replaced with a substituent of the formula -A-(CH₂)ᵣ-B-, wherein A and B are independently -CRR'-, -O-, -NR-, -S-, -S(O) -, -S(O)₂-, -S(O)₂NR'-, or a single bond, and r is an integer of from 1 to 4. One of the single bonds of the new ring so formed may optionally be replaced with a double bond. Alternatively, two of the substituents on adjacent atoms of the aryl or heteroaryl ring may optionally be replaced with a substituent of the formula-(CRR')ₛ-X'- (C"R"R"')_{d}-, where s and d are independently integers of from 0 to 3, and X' is-O-, -NR'-, -S-, -S(O)-, -S(O)₂-, or -S(O)₂NR'-. The substituents R, R', R", and R'" are preferably independently selected from hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl, and substituted or unsubstituted heteroaryl.

As used herein, the terms "heteroatom" or "ring heteroatom" are meant to include oxygen (O), nitrogen (N), sulfur (S), phosphorus (P), and silicon (Si).

A "substituent group," as used herein, means a group selected from the following moieties:
(A) oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂,
   -NHC(O)NH₂, -NHSO₂H, -NHC(O)H,
   -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl), and
(B) alkyl, heteroalkyl, cycloalkyl, heterocycloalkyl, aryl, heteroaryl, substituted with at least one substituent selected from:
   (i) oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO ₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl), and
   (ii) alkyl, heteroalkyl, cycloalkyl, heterocycloalkyl, aryl, heteroaryl, substituted with at least one substituent selected from:
      (a) oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂,
         -NHC(O)NH₂, -NHSO₂H,
         -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl), and
      (b) alkyl, heteroalkyl, cycloalkyl, heterocycloalkyl, aryl, heteroaryl, substituted with at least one substituent selected from: oxo,
         halogen, -CCl₃, -CBr₃, -CF₃, -CI₃, CHCl₂, -CHBr₂, -CHF₂, -CHI₂,-CH₂Cl, -CH₂Br, -CH₂F, -CH₂I, -CN, -OH, -NH₂,
         -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

In embodiments, a "substituent group," as used herein, means a group selected from the following moieties:
(A) oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl), and
(B) alkyl (e.g., C₁-C₂₀, C₁-C₁₂, C₁-C₈, C₁-C₆, C₁-C₄, or C₁-C₂), heteroalkyl (e.g., 2 to 20 membered, 2 to 12 membered, 2 to 8 membered, 2 to 6 membered, 4 to 6 membered, 2 to 3 membered, or 4 to 5 membered), cycloalkyl (e.g., C₃-C₁₀, C₃-C₈, C₃-C₆, C₄-C₆, or C₅-C₆), heterocycloalkyl (e.g., 3 to 10 membered, 3 to 8 membered, 3 to 6 membered, 4 to 6 membered, 4 to 5 membered, or 5 to 6 membered), aryl (e.g., C₆-C₁₂, C₆-C₁₀, or phenyl), or heteroaryl (e.g., 5 to 12 membered, 5 to 10 membered, 5 to 9 membered, or 5 to 6 membered), substituted with at least one substituent selected from:
   (i) oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl), and
   (ii) alkyl (e.g., C₁-C₂₀, C₁-C₁₂, C₁-C₈, C₁-C₆, C₁-C₄, or C₁-C₂), heteroalkyl (e.g., 2 to 20 membered, 2 to 12 membered, 2 to 8 membered, 2 to 6 membered, 4 to 6 membered, 2 to 3 membered, or 4 to 5 membered), cycloalkyl (e.g., C₃-C₁₀, C₃-C₈, C₃-C₆, C₄-C₆, or C₅-C₆), heterocycloalkyl (e.g., 3 to 10 membered, 3 to 8 membered, 3 to 6 membered, 4 to 6 membered, 4 to 5 membered, or 5 to 6 membered), aryl (e.g., C₆-C₁₂, C₆-C₁₀, or phenyl), or heteroaryl (e.g., 5 to 12 membered, 5 to 10 membered, 5 to 9 membered, or 5 to 6 membered), substituted with at least one substituent selected from:
      (a) oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl), and
      (b) alkyl (e.g., C₁-C₂₀, C₁-C₁₂, C₁-C₈, C₁-C₆, C₁-C₄, or C₁-C₂), heteroalkyl (e.g., 2 to 20 membered, 2 to 12 membered, 2 to 8 membered, 2 to 6 membered, 4 to 6 membered, 2 to 3 membered, or 4 to 5 membered), cycloalkyl (e.g., C₃-C₁₀, C₃-C₈, C₃-C₆, C₄-C₆, or C₅-C₆), heterocycloalkyl (e.g., 3 to 10 membered, 3 to 8 membered, 3 to 6 membered, 4 to 6 membered, 4 to 5 membered, or 5 to 6 membered), aryl (e.g., C₆-C₁₂, C₆-C₁₀, or phenyl), or heteroaryl (e.g., 5 to 12 membered, 5 to 10 membered, 5 to 9 membered, or 5 to 6 membered), substituted with at least one substituent selected from: oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

A "size-limited substituent" or " size-limited substituent group," as used herein, means a group selected from all of the substituents described above for a "substituent group," wherein each substituted or unsubstituted alkyl is a substituted or unsubstituted C₁-C₂₀ alkyl, each substituted or unsubstituted heteroalkyl is a substituted or unsubstituted 2 to 20 membered heteroalkyl, each substituted or unsubstituted cycloalkyl is a substituted or unsubstituted C₃-C₈ cycloalkyl, each substituted or unsubstituted heterocycloalkyl is a substituted or unsubstituted 3 to 8 membered heterocycloalkyl, each substituted or unsubstituted aryl is a substituted or unsubstituted C₆-C₁₀ aryl, and each substituted or unsubstituted heteroaryl is a substituted or unsubstituted 5 to 10 membered heteroaryl.

A "lower substituent" or " lower substituent group," as used herein, means a group selected from all of the substituents described above for a "substituent group," wherein each substituted or unsubstituted alkyl is a substituted or unsubstituted C₁-C₈ alkyl, each substituted or unsubstituted heteroalkyl is a substituted or unsubstituted 2 to 8 membered heteroalkyl, each substituted or unsubstituted cycloalkyl is a substituted or unsubstituted C₃-C₇ cycloalkyl, each substituted or unsubstituted heterocycloalkyl is a substituted or unsubstituted 3 to 7 membered heterocycloalkyl, each substituted or unsubstituted aryl is a substituted or unsubstituted C₆-C₁₀ aryl, and each substituted or unsubstituted heteroaryl is a substituted or unsubstituted 5 to 9 membered heteroaryl.

In some embodiments, each substituted group described in the compounds herein is substituted with at least one substituent group. More specifically, in some embodiments, each substituted alkyl, substituted heteroalkyl, substituted cycloalkyl, substituted heterocycloalkyl, substituted aryl, substituted heteroaryl, substituted alkylene, substituted heteroalkylene, substituted cycloalkylene, substituted heterocycloalkylene, substituted arylene, and/or substituted heteroarylene described in the compounds herein are substituted with at least one substituent group. In other embodiments, at least one or all of these groups are substituted with at least one size-limited substituent group. In other embodiments, at least one or all of these groups are substituted with at least one lower substituent group.

In other embodiments of the compounds herein, each substituted or unsubstituted alkyl may be a substituted or unsubstituted C₁-C₂₀ alkyl, each substituted or unsubstituted heteroalkyl is a substituted or unsubstituted 2 to 20 membered heteroalkyl, each substituted or unsubstituted cycloalkyl is a substituted or unsubstituted C₃-C₈ cycloalkyl, each substituted or unsubstituted heterocycloalkyl is a substituted or unsubstituted 3 to 8 membered heterocycloalkyl, each substituted or unsubstituted aryl is a substituted or unsubstituted C₆-C₁₀ aryl, and/or each substituted or unsubstituted heteroaryl is a substituted or unsubstituted 5 to 10 membered heteroaryl. In some embodiments of the compounds herein, each substituted or unsubstituted alkylene is a substituted or unsubstituted C₁-C₂₀ alkylene, each substituted or unsubstituted heteroalkylene is a substituted or unsubstituted 2 to 20 membered heteroalkylene, each substituted or unsubstituted cycloalkylene is a substituted or unsubstituted C₃-C₈ cycloalkylene, each substituted or unsubstituted heterocycloalkylene is a substituted or unsubstituted 3 to 8 membered heterocycloalkylene, each substituted or unsubstituted arylene is a substituted or unsubstituted C₆-C₁₀ arylene, and/or each substituted or unsubstituted heteroarylene is a substituted or unsubstituted 5 to 10 membered heteroarylene.

In some embodiments, each substituted or unsubstituted alkyl is a substituted or unsubstituted C₁-C₈ alkyl, each substituted or unsubstituted heteroalkyl is a substituted or unsubstituted 2 to 8 membered heteroalkyl, each substituted or unsubstituted cycloalkyl is a substituted or unsubstituted C₃-C₇ cycloalkyl, each substituted or unsubstituted heterocycloalkyl is a substituted or unsubstituted 3 to 7 membered heterocycloalkyl, each substituted or unsubstituted aryl is a substituted or unsubstituted C₆-C₁₀ aryl, and/or each substituted or unsubstituted heteroaryl is a substituted or unsubstituted 5 to 9 membered heteroaryl. In some embodiments, each substituted or unsubstituted alkylene is a substituted or unsubstituted C₁-C₈ alkylene, each substituted or unsubstituted heteroalkylene is a substituted or unsubstituted 2 to 8 membered heteroalkylene, each substituted or unsubstituted cycloalkylene is a substituted or unsubstituted C₃-C₇ cycloalkylene, each substituted or unsubstituted heterocycloalkylene is a substituted or unsubstituted 3 to 7 membered heterocycloalkylene, each substituted or unsubstituted arylene is a substituted or unsubstituted C₆-C₁₀ arylene, and/or each substituted or unsubstituted heteroarylene is a substituted or unsubstituted 5 to 9 membered heteroarylene. In some embodiments, the compound is a chemical species set forth in the Examples section, figures, or tables below.

In embodiments, a substituted or unsubstituted moiety (e.g., substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl, substituted or unsubstituted alkylene, substituted or unsubstituted heteroalkylene, substituted or unsubstituted cycloalkylene, substituted or unsubstituted heterocycloalkylene, substituted or unsubstituted arylene, and/or substituted or unsubstituted heteroarylene) is unsubstituted (e.g., is an unsubstituted alkyl, unsubstituted heteroalkyl, unsubstituted cycloalkyl, unsubstituted heterocycloalkyl, unsubstituted aryl, unsubstituted heteroaryl, unsubstituted alkylene, unsubstituted heteroalkylene, unsubstituted cycloalkylene, unsubstituted heterocycloalkylene, unsubstituted arylene, and/or unsubstituted heteroarylene, respectively). In embodiments, a substituted or unsubstituted moiety (e.g., substituted or unsubstituted alkyl, substituted or unsubstituted heteroalkyl, substituted or unsubstituted cycloalkyl, substituted or unsubstituted heterocycloalkyl, substituted or unsubstituted aryl, substituted or unsubstituted heteroaryl, substituted or unsubstituted alkylene, substituted or unsubstituted heteroalkylene, substituted or unsubstituted cycloalkylene, substituted or unsubstituted heterocycloalkylene, substituted or unsubstituted arylene, and/or substituted or unsubstituted heteroarylene) is substituted (e.g., is a substituted alkyl, substituted heteroalkyl, substituted cycloalkyl, substituted heterocycloalkyl, substituted aryl, substituted heteroaryl, substituted alkylene, substituted heteroalkylene, substituted cycloalkylene, substituted heterocycloalkylene, substituted arylene, and/or substituted heteroarylene, respectively).

In embodiments, a substituted moiety (e.g., substituted alkyl, substituted heteroalkyl, substituted cycloalkyl, substituted heterocycloalkyl, substituted aryl, substituted heteroaryl, substituted alkylene, substituted heteroalkylene, substituted cycloalkylene, substituted heterocycloalkylene, substituted arylene, and/or substituted heteroarylene) is substituted with at least one substituent group, wherein if the substituted moiety is substituted with a plurality of substituent groups, each substituent group may optionally be different. In embodiments, if the substituted moiety is substituted with a plurality of substituent groups, each substituent group is different.

In embodiments, a substituted moiety (e.g., substituted alkyl, substituted heteroalkyl, substituted cycloalkyl, substituted heterocycloalkyl, substituted aryl, substituted heteroaryl, substituted alkylene, substituted heteroalkylene, substituted cycloalkylene, substituted heterocycloalkylene, substituted arylene, and/or substituted heteroarylene) is substituted with at least one size-limited substituent group, wherein if the substituted moiety is substituted with a plurality of size-limited substituent groups, each size-limited substituent group may optionally be different. In embodiments, if the substituted moiety is substituted with a plurality of size-limited substituent groups, each size-limited substituent group is different.

In embodiments, a substituted moiety (e.g., substituted alkyl, substituted heteroalkyl, substituted cycloalkyl, substituted heterocycloalkyl, substituted aryl, substituted heteroaryl, substituted alkylene, substituted heteroalkylene, substituted cycloalkylene, substituted heterocycloalkylene, substituted arylene, and/or substituted heteroarylene) is substituted with at least one lower substituent group, wherein if the substituted moiety is substituted with a plurality of lower substituent groups, each lower substituent group may optionally be different. In embodiments, if the substituted moiety is substituted with a plurality of lower substituent groups, each lower substituent group is different.

In embodiments, a substituted moiety (e.g., substituted alkyl, substituted heteroalkyl, substituted cycloalkyl, substituted heterocycloalkyl, substituted aryl, substituted heteroaryl, substituted alkylene, substituted heteroalkylene, substituted cycloalkylene, substituted heterocycloalkylene, substituted arylene, and/or substituted heteroarylene) is substituted with at least one substituent group, size-limited substituent group, or lower substituent group; wherein if the substituted moiety is substituted with a plurality of groups selected from substituent groups, size-limited substituent groups, and lower substituent groups; each substituent group, size-limited substituent group, and/or lower substituent group may optionally be different. In embodiments, if the substituted moiety is substituted with a plurality of groups selected from substituent groups, size-limited substituent groups, and lower substituent groups; each substituent group, size-limited substituent group, and/or lower substituent group is different.

Certain compounds of the present disclosure possess asymmetric carbon atoms (optical or chiral centers) or double bonds; the enantiomers, racemates, diastereomers, tautomers, geometric isomers, stereoisometric forms that may be defined, in terms of absolute stereochemistry, as (R)-or (S)- or, as (D)- or (L)- for amino acids, and individual isomers are encompassed within the scope of the present disclosure. The compounds of the present disclosure do not include those that are known in art to be too unstable to synthesize and/or isolate. The present disclosure is meant to include compounds in racemic and optically pure forms. Optically active (R)- and (S)-, or (D)- and (L)-isomers may be prepared using chiral synthons or chiral reagents, or resolved using conventional techniques. When the compounds described herein contain olefinic bonds or other centers of geometric asymmetry, and unless specified otherwise, it is intended that the compounds include both E and Z geometric isomers.

The symbols 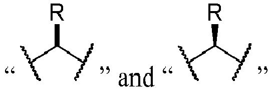 are used interchangeably and refer to absolute stereochemistry in accordance with standard convention in the chemical arts. Similarly, the symbols 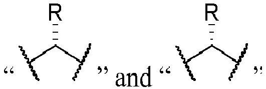 are used interchangeably and refer to absolute stereochemistry in accordance with standard convention in the chemical arts.

As used herein, the term "isomers" refers to compounds having the same number and kind of atoms, and hence the same molecular weight, but differing in respect to the structural arrangement or configuration of the atoms.

The term "tautomer," as used herein, refers to one of two or more structural isomers which exist in equilibrium and which are readily converted from one isomeric form to another.

It will be apparent to one skilled in the art that certain compounds of this disclosure may exist in tautomeric forms, all such tautomeric forms of the compounds being within the scope of the disclosure.

Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the disclosure.

Unless otherwise stated, structures depicted herein are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon are within the scope of this disclosure.

The compounds of the present disclosure may also contain unnatural proportions of atomic isotopes at one or more of the atoms that constitute such compounds. For example, the compounds may be radiolabeled with radioactive isotopes, such as for example tritium (³H), iodine-125 (¹²⁵I), or carbon-14 (¹⁴C). All isotopic variations of the compounds of the present disclosure, whether radioactive or not, are encompassed within the scope of the present disclosure.

It should be noted that throughout the application that alternatives are written in Markush groups, for example, each amino acid position that contains more than one possible amino acid. It is specifically contemplated that each member of the Markush group should be considered separately, thereby comprising another embodiment, and the Markush group is not to be read as a single unit.

"Analog," or "analogue" is used in accordance with its plain ordinary meaning within Chemistry and Biology and refers to a chemical compound that is structurally similar to another compound (i.e., a so-called "reference" compound) but differs in composition, e.g., in the replacement of one atom by an atom of a different element, or in the presence of a particular functional group, or the replacement of one functional group by another functional group, or the absolute stereochemistry of one or more chiral centers of the reference compound. Accordingly, an analog is a compound that is similar or comparable in function and appearance but not in structure or origin to a reference compound.

The terms "a" or "an," as used in herein means one or more. In addition, the phrase "substituted with a[n]," as used herein, means the specified group may be substituted with one or more of any or all of the named substituents. For example, where a group, such as an alkyl or heteroaryl group, is "substituted with an unsubstituted C₁-C₂₀ alkyl, or unsubstituted 2 to 20 membered heteroalkyl," the group may contain one or more unsubstituted C₁-C₂₀ alkyls, and/or one or more unsubstituted 2 to 20 membered heteroalkyls.

Moreover, where a moiety is substituted with an R substituent, the group may be referred to as "R-substituted." Where a moiety is R-substituted, the moiety is substituted with at least one R substituent and each R substituent is optionally different. Where a particular R group is present in the description of a chemical genus (such as Formula (I)), a Roman alphabetic symbol may be used to distinguish each appearance of that particular R group. For example, where multiple R¹³ substituents are present, each R¹³ substituent may be distinguished as R^{13A}, R^{13B}, R^{13C}, R^{13D}, etc., wherein each of R^{13A}, R^{13B}, R^{13C}, R^{13D}, etc. is defined within the scope of the definition of R¹³ and optionally differently.

The term "pharmaceutically acceptable salts" is meant to include salts of the active compounds that are prepared with relatively nontoxic acids or bases, depending on the particular substituents found on the compounds described herein. When compounds of the present disclosure contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds of the present disclosure contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids like acetic, propionic, isobutyric, maleic, malonic, benzoic, succinic, suberic, fumaric, lactic, mandelic, phthalic, benzenesulfonic, p-tolylsulfonic, citric, tartaric, oxalic, methanesulfonic, and the like. Also included are salts of amino acids such as arginate and the like, and salts of organic acids like glucuronic or galactunoric acids and the like (*see*, for example, Berge et al., "Pharmaceutical Salts", Journal of Pharmaceutical Science, 1977, 66, 1-19). Certain specific compounds of the present disclosure contain both basic and acidic functionalities that allow the compounds to be converted into either base or acid addition salts.

Thus, the compounds of the present disclosure may exist as salts, such as with pharmaceutically acceptable acids. The present disclosure includes such salts. Non-limiting examples of such salts include hydrochlorides, hydrobromides, phosphates, sulfates, methanesulfonates, nitrates, maleates, acetates, citrates, fumarates, proprionates, tartrates (e.g., (+)-tartrates, (-)-tartrates, or mixtures thereof including racemic mixtures), succinates, benzoates, and salts with amino acids such as glutamic acid, and quaternary ammonium salts (e.g. methyl iodide, ethyl iodide, and the like). These salts may be prepared by methods known to those skilled in the art.

The neutral forms of the compounds are preferably regenerated by contacting the salt with a base or acid and isolating the parent compound in the conventional manner. The parent form of the compound may differ from the various salt forms in certain physical properties, such as solubility in polar solvents.

In addition to salt forms, the present disclosure provides compounds, which are in a prodrug form. Prodrugs of the compounds described herein are those compounds that readily undergo chemical changes under physiological conditions to provide the compounds of the present disclosure. Prodrugs of the compounds described herein may be converted *in vivo* after administration. Additionally, prodrugs can be converted to the compounds of the present disclosure by chemical or biochemical methods in an *ex vivo* environment, such as, for example, when contacted with a suitable enzyme or chemical reagent.

Certain compounds of the present disclosure can exist in unsolvated forms as well as solvated forms, including hydrated forms. In general, the solvated forms are equivalent to unsolvated forms and are encompassed within the scope of the present disclosure. Certain compounds of the present disclosure may exist in multiple crystalline or amorphous forms. In general, all physical forms are equivalent for the uses contemplated by the present disclosure and are intended to be within the scope of the present disclosure.

"Pharmaceutically acceptable excipient" and "pharmaceutically acceptable carrier" refer to a substance that aids the administration of an active agent to and absorption by a subject and can be included in the compositions of the present disclosure without causing a significant adverse toxicological effect on the patient. Non-limiting examples of pharmaceutically acceptable excipients include water, NaCl, normal saline solutions, lactated Ringer's, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, fatty acid esters, hydroxymethycellulose, polyvinyl pyrrolidine, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the compounds of the disclosure. One of skill in the art will recognize that other pharmaceutical excipients are useful in the present disclosure.

The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

An "Adenosine A2B Receptor inhibitor" refers to a compound (e.g. compounds described herein) that reduces the activity of the Adenosine A2B Receptor when compared to a control, such as absence of the compound or a compound with known inactivity. An "Adenosine A2A Receptor inhibitor" refers to a compound (e.g. compounds described herein) that reduces the activity of Adenosine A2A Receptor when compared to a control, such as absence of the compound or a compound with known inactivity.

"Contacting" is used in accordance with its plain ordinary meaning and refers to the process of allowing at least two distinct species (e.g. chemical compounds including biomolecules or cells) to become sufficiently proximal to react, interact or physically touch. It should be appreciated; however, the resulting reaction product can be produced directly from a reaction between the added reagents or from an intermediate from one or more of the added reagents that can be produced in the reaction mixture.

The term "contacting" may include allowing two species to react, interact, or physically touch, wherein the two species may be a compound as described herein and a protein or enzyme. In some embodiments contacting includes allowing a compound described herein to interact with a protein or enzyme that is involved in a signaling pathway.

As defined herein, the term "activation", "activate", "activating", "activator" and the like in reference to a protein-inhibitor interaction means positively affecting (e.g. increasing) the activity or function of the protein relative to the activity or function of the protein in the absence of the activator. In embodiments activation means positively affecting (e.g. increasing) the concentration or levels of the protein relative to the concentration or level of the protein in the absence of the activator. The terms may reference activation, or activating, sensitizing, or up-regulating signal transduction or enzymatic activity or the amount of a protein decreased in a disease. Thus, activation may include, at least in part, partially or totally increasing stimulation, increasing or enabling activation, or activating, sensitizing, or up-regulating signal transduction or enzymatic activity or the amount of a protein associated with a disease (e.g., a protein which is decreased in a disease relative to a non-diseased control). Activation may include, at least in part, partially or totally increasing stimulation, increasing or enabling activation, or activating, sensitizing, or up-regulating signal transduction or enzymatic activity or the amount of a protein

The terms "agonist," "activator," "upregulator," etc. refer to a substance capable of detectably increasing the expression or activity of a given gene or protein. The agonist can increase expression or activity 10%, 20%, 30%, 40%, 50%, 60%, 70%, 80%, 90% or more in comparison to a control in the absence of the agonist. In certain instances, expression or activity is 1.5-fold, 2-fold, 3-fold, 4-fold, 5-fold, 10-fold or higher than the expression or activity in the absence of the agonist.

As defined herein, the term "inhibition", "inhibit", "inhibiting" and the like in reference to a protein-inhibitor interaction means negatively affecting (e.g. decreasing) the activity or function of the protein relative to the activity or function of the protein in the absence of the inhibitor. In embodiments inhibition means negatively affecting (e.g. decreasing) the concentration or levels of the protein relative to the concentration or level of the protein in the absence of the inhibitor. In embodiments inhibition refers to reduction of a disease or symptoms of disease. In embodiments, inhibition refers to a reduction in the activity of a particular protein target. Thus, inhibition includes, at least in part, partially or totally blocking stimulation, decreasing, preventing, or delaying activation, or inactivating, desensitizing, or down-regulating signal transduction or enzymatic activity or the amount of a protein. In embodiments, inhibition refers to a reduction of activity of a target protein resulting from a direct interaction (e.g. an inhibitor binds to the target protein). In embodiments, inhibition refers to a reduction of activity of a target protein from an indirect interaction (e.g. an inhibitor binds to a protein that activates the target protein, thereby preventing target protein activation). An "Adenosine A2B Receptor inhibitor" is a compound that negatively affects (e.g. decreases) the activity or function of Adenosine A2B Receptor relative to the activity or function of Adenosine A2B Receptor in the absence of the inhibitor. An "Adenosine A2A Receptor inhibitor" is a compound that negatively affects (e.g. decreases) the activity or function of Adenosine A2A Receptor relative to the activity or function of Adenosine A2A Receptor in the absence of the inhibitor.

The terms "inhibitor," "repressor" or "antagonist" or "downregulator" interchangeably refer to a substance capable of detectably decreasing the expression or activity of a given gene or protein. The antagonist can decrease expression or activity 10%, 20%, 30%, 40%, 50%, 60%, 70%, 80%, 90% or more in comparison to a control in the absence of the antagonist. In certain instances, expression or activity is 1.5-fold, 2-fold, 3-fold, 4-fold, 5-fold, 10-fold or lower than the expression or activity in the absence of the antagonist.

The terms "Adenosine A2A Receptor" and "AA2A Receptor" and "A2A" and "ADORA2A" refer to a protein (including homologs, isoforms, and functional fragments thereof) with adenylate cyclase activity. The term includes any recombinant or naturally-occurring form of Adenosine A2A Receptor variants thereof that maintain Adenosine A2A Receptor activity (e.g. within at least 30%, 40%, 50%, 60%, 70%, 80%, 90%, 95%, or 100% activity compared to wildtype Adenosine A2A Receptor). In embodiments, the Adenosine A2A Receptor protein encoded by the ADORA2A gene has the amino acid sequence set forth in or corresponding to Entrez 135, UniProt P29274, or RefSeq (protein) NP_000666. In embodiments, the ADORA2A gene has the nucleic acid sequence set forth in RefSeq (mRNA) NM_000675. In embodiments, the amino acid sequence or nucleic acid sequence is the sequence known at the time of filing of the present application. In embodiments, the sequence corresponds to GI: 156142194. In embodiments, the sequence corresponds to NM_000675.5. In embodiments, the sequence corresponds to NP_000667.2. In embodiments, the sequence corresponds to GI: 4501949. In embodiments, the A2A protein corresponds to the sequence:

MPIMGSSVYITVELAIAVLAILGNVLVCWAVWLNSNLQNVTNYFVVSLAAADIAVG
VLAIPFAITISTGFCAACHGCLFIACFVLVLTQSSIFSLLAIAIDRYIAIRIPLRYNGLVTG
TRAKGIIAICWVLSFAIGLTPMLGWNNCGQPKEGKNHSQGCGEGQVACLFEDVPMN

YMVYFNFFACVLVPLLLMLGVYLRIFLAARRQLKQMESQPLPGERARSTLQKEVHA
AKSLAIIVGLFALCWLPLHIINCFTFFCPDCSHAPLWLMYLAIVLSHTNSVVNPFIYAY
RIREFRQTFRKIIRSHVLRQQEPFKAAGTSARVLAAHGSDGEQVSLRLNGHPPGVWA
NGSAPHPERRPNGYALGLVSGGSAQESQGNTGLPDVELLSHELKGVCPEPPGLDDPL
AQDGAGVS (SEQ ID NO: 1).

The terms "Adenosine A2B Receptor" and "AA2B Receptor" and "A2B" and "ADORA2B" refer to a protein (including homologs, isoforms, and functional fragments thereof) with adenylate cyclase activity. The term includes any recombinant or naturally-occurring form of Adenosine A2B Receptor variants thereof that maintain Adenosine A2B Receptor activity (e.g. within at least 30%, 40%, 50%, 60%, 70%, 80%, 90%, 95%, or 100% activity compared to wildtype Adenosine A2B Receptor). In embodiments, the Adenosine A2B Receptor protein encoded by the ADORA2B gene has the amino acid sequence set forth in or corresponding to Entrez 136, UniProt P29275, or RefSeq (protein) NP_000667. In embodiments, the ADORA2B gene has the nucleic acid sequence set forth in RefSeq (mRNA) NM_000676. In embodiments, the amino acid sequence or nucleic acid sequence is the sequence known at the time of filing of the present application. In embodiments, the sequence corresponds to GI: 4501950. In embodiments, the sequence corresponds to NM_000676.2. In embodiments, the sequence corresponds to NP_000667.1. In embodiments, the A2B protein corresponds to the sequence:

MLLETQDALYVALELVIAALSVAGNVLVCAAVGTANTLQTPTNYFLVSLAAADVAV
GLFAIPFAITISLGFCTDFYGCLFLACFVLVLTQSSIFSLLAVAVDRYLAICVPLRYKSL
VTGTRARGVIAVLWVLAFGIGLTPFLGWNSKDSATNNCTEPWDGTTNESCCLVKCL
FENVVPMSYMVYFNFFGCVLPPLLIMLVIYIKIFLVACRQLQRTELMDHSRTTLQREI
HAAKSLAMIVGIFALCWLPVHAVNCVTLFQPAQGKNKPKWAMNMAILLSHANSVV
NPIVYAYRNRDFRYTFHKIISRYLLCQADVKSGNGQAGVQPALGVGL (SEQ ID NO:
2).

The term "expression" includes any step involved in the production of the polypeptide including, but not limited to, transcription, post-transcriptional modification, translation, post-translational modification, and secretion. Expression can be detected using conventional techniques for detecting protein (*e.g*., ELISA, Western blotting, flow cytometry, immunofluorescence, immunohistochemistry, *etc.*).

The terms "disease" or "condition" refer to a state of being or health status of a patient or subject capable of being treated with the compounds or methods provided herein. The disease may be a cancer. The disease may be an autoimmune disease. The disease may be a fibrotic disease (e.g., fibrosis). The disease may be an inflammatory disease. The disease may be an infectious disease. The disease may be a neurodegenerative disease. In some further instances, "cancer" refers to human cancers and carcinomas, sarcomas, adenocarcinomas, lymphomas, leukemias, etc., including solid and lymphoid cancers, kidney, breast, lung, bladder, colon, ovarian, prostate, pancreas, stomach, brain, head and neck, skin, uterine, testicular, glioma, esophagus, and liver cancer, including hepatocarcinoma, lymphoma, including B-acute lymphoblastic lymphoma, non-Hodgkin's lymphomas (e.g., Burkitt's, Small Cell, and Large Cell lymphomas), Hodgkin's lymphoma, leukemia (including AML, ALL, and CML), or multiple myeloma.

As used herein, the term "cancer" refers to all types of cancer, neoplasm or malignant tumors found in mammals (e.g. humans), including leukemia, carcinomas and sarcomas. Exemplary cancers that may be treated with a compound or method provided herein include brain cancer, glioma, glioblastoma, neuroblastoma, prostate cancer, colorectal cancer, pancreatic cancer, cervical cancer, gastric cancer, ovarian cancer, lung cancer, and cancer of the head. Exemplary cancers that may be treated with a compound or method provided herein include cancer of the thyroid, endocrine system, brain, breast, cervix, colon, head & neck, liver, kidney, lung, non-small cell lung, melanoma, mesothelioma, ovary, sarcoma, stomach, uterus, Medulloblastoma, colorectal cancer, pancreatic cancer. Additional examples include, Hodgkin's Disease, Non-Hodgkin's Lymphoma, multiple myeloma, neuroblastoma, glioma, glioblastoma multiforme, ovarian cancer, rhabdomyosarcoma, primary thrombocytosis, primary macroglobulinemia, primary brain tumors, cancer, malignant pancreatic insulanoma, malignant carcinoid, urinary bladder cancer, premalignant skin lesions, testicular cancer, lymphomas, thyroid cancer, neuroblastoma, esophageal cancer, genitourinary tract cancer, malignant hypercalcemia, endometrial cancer, adrenal cortical cancer, neoplasms of the endocrine or exocrine pancreas, medullary thyroid cancer, medullary thyroid carcinoma, melanoma, colorectal cancer, papillary thyroid cancer, hepatocellular carcinoma, or prostate cancer.

The term "leukemia" refers broadly to progressive, malignant diseases of the blood-forming organs and is generally characterized by a distorted proliferation and development of leukocytes and their precursors in the blood and bone marrow. Leukemia is generally clinically classified on the basis of (1) the duration and character of the disease-acute or chronic; (2) the type of cell involved; myeloid (myelogenous), lymphoid (lymphogenous), or monocytic; and (3) the increase or non-increase in the number abnormal cells in the blood-leukemic or aleukemic (subleukemic). Exemplary leukemias that may be treated with a compound or method provided herein include, for example, acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, multiple myeloma, plasmacytic leukemia, promyelocytic leukemia, Rieder cell leukemia, Schilling's leukemia, stem cell leukemia, subleukemic leukemia, or undifferentiated cell leukemia.

The term "sarcoma" generally refers to a tumor which is made up of a substance like the embryonic connective tissue and is generally composed of closely packed cells embedded in a fibrillar or homogeneous substance. Sarcomas that may be treated with a compound or method provided herein include a chondrosarcoma, fibrosarcoma, lymphosarcoma, melanosarcoma, myxosarcoma, osteosarcoma, Abemethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, or telangiectaltic sarcoma.

The term "melanoma" is taken to mean a tumor arising from the melanocytic system of the skin and other organs. Melanomas that may be treated with a compound or method provided herein include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, or superficial spreading melanoma.

The term "carcinoma" refers to a malignant new growth made up of epithelial cells tending to infiltrate the surrounding tissues and give rise to metastases. Exemplary carcinomas that may be treated with a compound or method provided herein include, for example, medullary thyroid carcinoma, familial medullary thyroid carcinoma, acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma ex ulcere, carcinoma fibrosum, gelatiniforni carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypernephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, transitional cell carcinoma, carcinoma tuberosum, tuberous carcinoma, verrucous carcinoma, or carcinoma villosum.

As used herein, the term "lymphoma" refers to a group of cancers affecting hematopoietic and lymphoid tissues. It begins in lymphocytes, the blood cells that are found primarily in lymph nodes, spleen, thymus, and bone marrow. Two main types of lymphoma are non-Hodgkin lymphoma and Hodgkin's disease. Hodgkin's disease represents approximately 15% of all diagnosed lymphomas. This is a cancer associated with Reed-Sternberg malignant B lymphocytes. Non-Hodgkin's lymphomas (NHL) can be classified based on the rate at which cancer grows and the type of cells involved. There are aggressive (high grade) and indolent (low grade) types of NHL. Based on the type of cells involved, there are B-cell and T-cell NHLs. Exemplary B-cell lymphomas that may be treated with a compound or method provided herein include, but are not limited to, small lymphocytic lymphoma, Mantle cell lymphoma, follicular lymphoma, marginal zone lymphoma, extranodal (MALT) lymphoma, nodal (monocytoid B-cell) lymphoma, splenic lymphoma, diffuse large cell B-lymphoma, Burkitt's lymphoma, lymphoblastic lymphoma, immunoblastic large cell lymphoma, or precursor B-lymphoblastic lymphoma. Exemplary T-cell lymphomas that may be treated with a compound or method provided herein include, but are not limited to, cunateous T-cell lymphoma, peripheral T-cell lymphoma, anaplastic large cell lymphoma, mycosis fungoides, and precursor T-lymphoblastic lymphoma.

As used herein, the term "autoimmune disease" refers to a disease or condition in which a subject's immune system has an aberrant immune response against a substance that does not normally elicit an immune response in a healthy subject. Examples of autoimmune diseases that may be treated with a compound, pharmaceutical composition, or method described herein include Acute Disseminated Encephalomyelitis (ADEM), Acute necrotizing hemorrhagic leukoencephalitis, Addison's disease, Agammaglobulinemia, Alopecia areata, Amyloidosis, Ankylosing spondylitis, Anti-GBM/Anti-TBM nephritis, Antiphospholipid syndrome (APS), Autoimmune angioedema, Autoimmune aplastic anemia, Autoimmune dysautonomia, Autoimmune hepatitis, Autoimmune hyperlipidemia, Autoimmune immunodeficiency, Autoimmune inner ear disease (AIED), Autoimmune myocarditis, Autoimmune oophoritis, Autoimmune pancreatitis, Autoimmune retinopathy, Autoimmune thrombocytopenic purpura (ATP), Autoimmune thyroid disease, Autoimmune urticaria, Axonal or neuronal neuropathies, Balo disease, Behcet's disease, Bullous pemphigoid, Cardiomyopathy, Castleman disease, Celiac disease, Chagas disease, Chronic fatigue syndrome, Chronic inflammatory demyelinating polyneuropathy (CIDP), Chronic recurrent multifocal ostomyelitis (CRMO), Churg-Strauss syndrome, Cicatricial pemphigoid/benign mucosal pemphigoid, Crohn's disease, Cogans syndrome, Cold agglutinin disease, Congenital heart block, Coxsackie myocarditis, CREST disease, Essential mixed cryoglobulinemia, Demyelinating neuropathies, Dermatitis herpetiformis, Dermatomyositis, Devic's disease (neuromyelitis optica), Discoid lupus, Dressler's syndrome, Endometriosis, Eosinophilic esophagitis, Eosinophilic fasciitis, Erythema nodosum, Experimental allergic encephalomyelitis, Evans syndrome, Fibromyalgia , Fibrosing alveolitis, Giant cell arteritis (temporal arteritis), Giant cell myocarditis, Glomerulonephritis, Goodpasture's syndrome, Granulomatosis with Polyangiitis (GPA) (formerly called Wegener's Granulomatosis), Graves' disease, Guillain-Barre syndrome, Hashimoto's encephalitis, Hashimoto's thyroiditis, Hemolytic anemia, Henoch-Schonlein purpura, Herpes gestationis, Hypogammaglobulinemia, Idiopathic thrombocytopenic purpura (ITP), IgA nephropathy, IgG4-related sclerosing disease, Immunoregulatory lipoproteins, Inclusion body myositis, Interstitial cystitis, Juvenile arthritis, Juvenile diabetes (Type 1 diabetes), Juvenile myositis, Kawasaki syndrome, Lambert-Eaton syndrome, Leukocytoclastic vasculitis, Lichen planus, Lichen sclerosus, Ligneous conjunctivitis, Linear IgA disease (LAD), Lupus (SLE), Lyme disease, chronic, Meniere's disease, Microscopic polyangiitis, Mixed connective tissue disease (MCTD), Mooren's ulcer, Mucha-Habermann disease, Multiple sclerosis, Myasthenia gravis, Myositis, Narcolepsy, Neuromyelitis optica (Devic's), Neutropenia, Ocular cicatricial pemphigoid, Optic neuritis, Palindromic rheumatism, PANDAS (Pediatric Autoimmune Neuropsychiatric Disorders Associated with Streptococcus), Paraneoplastic cerebellar degeneration, Paroxysmal nocturnal hemoglobinuria (PNH), Parry Romberg syndrome, Parsonnage-Turner syndrome, Pars planitis (peripheral uveitis), Pemphigus, Peripheral neuropathy, Perivenous encephalomyelitis, Pernicious anemia, POEMS syndrome, Polyarteritis nodosa, Type I, II, & III autoimmune polyglandular syndromes, Polymyalgia rheumatica, Polymyositis, Postmyocardial infarction syndrome, Postpericardiotomy syndrome, Progesterone dermatitis, Primary biliary cirrhosis, Primary sclerosing cholangitis, Psoriasis, Psoriatic arthritis, Idiopathic pulmonary fibrosis, Pyoderma gangrenosum, Pure red cell aplasia, Raynauds phenomenon, Reactive Arthritis, Reflex sympathetic dystrophy, Reiter's syndrome, Relapsing polychondritis, Restless legs syndrome, Retroperitoneal fibrosis, Rheumatic fever, Rheumatoid arthritis, Sarcoidosis, Schmidt syndrome, Scleritis, Scleroderma, Sjogren's syndrome, Sperm & testicular autoimmunity, Stiff person syndrome, Subacute bacterial endocarditis (SBE), Susac's syndrome, Sympathetic ophthalmia, Takayasu's arteritis, Temporal arteritis/Giant cell arteritis, Thrombocytopenic purpura (TTP), Tolosa-Hunt syndrome, Transverse myelitis, Type 1 diabetes, Ulcerative colitis, Undifferentiated connective tissue disease (UCTD), Uveitis, Vasculitis, Vesiculobullous dermatosis, Vitiligo, or Wegener's granulomatosis (i.e., Granulomatosis with Polyangiitis (GPA).

As used herein, the term "inflammatory disease" refers to a disease or condition characterized by aberrant inflammation (e.g. an increased level of inflammation compared to a control such as a healthy person not suffering from a disease). Examples of inflammatory diseases include traumatic brain injury, arthritis, rheumatoid arthritis, psoriatic arthritis, juvenile idiopathic arthritis, multiple sclerosis, systemic lupus erythematosus (SLE), myasthenia gravis, juvenile onset diabetes, diabetes mellitus type 1, Guillain-Barre syndrome, Hashimoto's encephalitis, Hashimoto's thyroiditis, ankylosing spondylitis, psoriasis, Sjogren's syndrome,vasculitis, glomerulonephritis, auto-immune thyroiditis, Behcet's disease, Crohn's disease, ulcerative colitis, bullous pemphigoid, sarcoidosis, ichthyosis, Graves ophthalmopathy, inflammatory bowel disease, Addison's disease, Vitiligo,asthma, asthma, allergic asthma, acne vulgaris, celiac disease, chronic prostatitis, inflammatory bowel disease, pelvic inflammatory disease, reperfusion injury, sarcoidosis, transplant rejection, interstitial cystitis, atherosclerosis, and atopic dermatitis.

As used herein, the term "neurodegenerative disorder" or "neurodegenerative disease" refers to a disease or condition in which the function of a subject's nervous system becomes impaired. Examples of neurodegenerative diseases that may be treated with a compound, pharmaceutical composition, or method described herein include Alexander's disease, Alper's disease, Alzheimer's disease, Amyotrophic lateral sclerosis, Ataxia telangiectasia, Batten disease (also known as Spielmeyer-Vogt-Sjogren-Batten disease), Bovine spongiform encephalopathy (BSE), Canavan disease, chronic fatigue syndrome, Cockayne syndrome, Corticobasal degeneration, Creutzfeldt-Jakob disease, frontotemporal dementia, Gerstmann-Sträussler-Scheinker syndrome, Huntington's disease, HIV-associated dementia, Kennedy's disease, Krabbe's disease, kuru, Lewy body dementia, Machado-Joseph disease (Spinocerebellar ataxia type 3), Multiple sclerosis, Multiple System Atrophy, myalgic encephalomyelitis, Narcolepsy, Neuroborreliosis, Parkinson's disease, Pelizaeus-Merzbacher Disease, Pick's disease, Primary lateral sclerosis, Prion diseases, Refsum's disease, Sandhoffs disease, Schilder's disease, Subacute combined degeneration of spinal cord secondary to Pernicious Anaemia, Schizophrenia, Spinocerebellar ataxia (multiple types with varying characteristics), Spinal muscular atrophy, Steele-Richardson-Olszewski disease , progressive supranuclear palsy, or Tabes dorsalis.

As used herein, the term "fibrosis" refers to the formation of excess fibrous connective tissue. As used herein, the term "fibrotic disorder" or "fibrotic disease" refers to a disease or condition associated with excess formation of fibrous tissue (e.g., fibrous connective tissue) in an organs or tissue of a subject. Examples of fibrotic disorders that may be treated with a compound, pharmaceutical composition, or method described herein include rheumatoid arthritis, surgical adhesions, osteoarthritis, visible skin scars, a cardiovascular fibrotic disorder. In embodiments, the fibrotic disorder is a liver fibrotic disorder, a kidney fibrotic disorder, a lung fibrotic disorder, periodontal fibrotic disorder, diseases entailing excess collagen and/or elastin deposition, cutaneous keloid formation, progressive systemic sclerosis, liver cirrhosis, idiopathic and pharmacologically induced pulmonary fibrosis, chronic graft-versus-host disease, scleroderma (local and systemic), Peyronie's disease, pharmacologically induced fibrosis of the penis. In embodiments, the fibrotic disorder is post-cystoscopic urethral stenosis, post-surgical internal adhesions, myelofibrosis, idiopathic and pharmacologically induced retroperitoneal fibrosis.

The terms "treating", or "treatment" refers to any indicia of success in the therapy or amelioration of an injury, disease, pathology or condition, including any objective or subjective parameter such as abatement; remission; diminishing of symptoms or making the injury, pathology or condition more tolerable to the patient; slowing in the rate of degeneration or decline; making the final point of degeneration less debilitating; improving a patient's physical or mental well-being. The treatment or amelioration of symptoms can be based on objective or subjective parameters; including the results of a physical examination, neuropsychiatric exams, and/or a psychiatric evaluation. The term "treating" and conjugations thereof, may include prevention of an injury, pathology, condition, or disease. In embodiments, treating is preventing. In embodiments, treating does not include preventing.

"Treating" or "treatment" as used herein (and as well-understood in the art) also broadly includes any approach for obtaining beneficial or desired results in a subject's condition, including clinical results. Beneficial or desired clinical results can include, but are not limited to, alleviation or amelioration of one or more symptoms or conditions, diminishment of the extent of a disease, stabilizing (*i.e.*, not worsening) the state of disease, prevention of a disease's transmission or spread, delay or slowing of disease progression, amelioration or palliation of the disease state, diminishment of the reoccurrence of disease, and remission, whether partial or total and whether detectable or undetectable. In other words, "treatment" as used herein includes any cure, amelioration, or prevention of a disease. Treatment may prevent the disease from occurring; inhibit the disease's spread; relieve the disease's symptoms (*e.g*., ocular pain, seeing halos around lights, red eye, very high intraocular pressure), fully or partially remove the disease's underlying cause, shorten a disease's duration, or do a combination of these things.

"Treating" and "treatment" as used herein include prophylactic treatment. Treatment methods include administering to a subject a therapeutically effective amount of an active agent. The administering step may consist of a single administration or may include a series of administrations. The length of the treatment period depends on a variety of factors, such as the severity of the condition, the age of the patient, the concentration of active agent, the activity of the compositions used in the treatment, or a combination thereof. It will also be appreciated that the effective dosage of an agent used for the treatment or prophylaxis may increase or decrease over the course of a particular treatment or prophylaxis regime. Changes in dosage may result and become apparent by standard diagnostic assays known in the art. In some instances, chronic administration may be required. For example, the compositions are administered to the subject in an amount and for a duration sufficient to treat the patient. In embodiments, the treating or treatment is no prophylactic treatment.

The term "prevent" refers to a decrease in the occurrence of disease symptoms (e.g., associated with Adenosine A2B Receptor activity or function thereof) in a patient. As indicated above, the prevention may be complete (no detectable symptoms) or partial, such that fewer symptoms are observed than would likely occur absent treatment.

"Patient" or "subject in need thereof' refers to a living organism suffering from or prone to a disease or condition that can be treated by administration of a pharmaceutical composition as provided herein. Non-limiting examples include humans, other mammals, bovines, rats, mice, dogs, monkeys, goat, sheep, cows, deer, and other non-mammalian animals. In some embodiments, a patient is human.

An "effective amount" is an amount sufficient for a compound to accomplish a stated purpose relative to the absence of the compound (e.g. achieve the effect for which it is administered, treat a disease, reduce enzyme activity, increase enzyme activity, reduce a signaling pathway, or reduce one or more symptoms of a disease or condition). An example of an "effective amount" is an amount sufficient to contribute to the treatment, prevention, or reduction of a symptom or symptoms of a disease, which could also be referred to as a "therapeutically effective amount." A "reduction" of a symptom or symptoms (and grammatical equivalents of this phrase) means decreasing of the severity or frequency of the symptom(s), or elimination of the symptom(s). A "prophylactically effective amount" of a drug is an amount of a drug that, when administered to a subject, will have the intended prophylactic effect, e.g., preventing or delaying the onset (or reoccurrence) of an injury, disease, pathology or condition, or reducing the likelihood of the onset (or reoccurrence) of an injury, disease, pathology, or condition, or their symptoms. The full prophylactic effect does not necessarily occur by administration of one dose, and may occur only after administration of a series of doses. Thus, a prophylactically effective amount may be administered in one or more administrations. An "activity decreasing amount," as used herein, refers to an amount of antagonist required to decrease the activity of an enzyme relative to the absence of the antagonist. A "function disrupting amount," as used herein, refers to the amount of antagonist required to disrupt the function of an enzyme or protein relative to the absence of the antagonist. The exact amounts will depend on the purpose of the treatment, and will be ascertainable by one skilled in the art using known techniques (*see*, *e.g.*, Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

For any compound described herein, the therapeutically effective amount can be initially determined from cell culture assays. Target concentrations will be those concentrations of active compound(s) that are capable of achieving the methods described herein, as measured using the methods described herein or known in the art.

As is well known in the art, therapeutically effective amounts for use in humans can also be determined from animal models. For example, a dose for humans can be formulated to achieve a concentration that has been found to be effective in animals. The dosage in humans can be adjusted by monitoring compounds effectiveness and adjusting the dosage upwards or downwards, as described above. Adjusting the dose to achieve maximal efficacy in humans based on the methods described above and other methods is well within the capabilities of the ordinarily skilled artisan.

The term "therapeutically effective amount," as used herein, refers to that amount of the therapeutic agent sufficient to ameliorate the disorder, as described above. For example, for the given parameter, a therapeutically effective amount will show an increase or decrease of at least 5%, 10%, 15%, 20%, 25%, 40%, 50%, 60%, 75%, 80%, 90%, or at least 100%. Therapeutic efficacy can also be expressed as "-fold" increase or decrease. For example, a therapeutically effective amount can have at least a 1.2-fold, 1.5-fold, 2-fold, 5-fold, or more effect over a control.

Dosages may be varied depending upon the requirements of the patient and the compound being employed. The dose administered to a patient, in the context of the present disclosure, should be sufficient to effect a beneficial therapeutic response in the patient over time. The size of the dose also will be determined by the existence, nature, and extent of any adverse side-effects. Determination of the proper dosage for a particular situation is within the skill of the practitioner. Generally, treatment is initiated with smaller dosages which are less than the optimum dose of the compound. Thereafter, the dosage is increased by small increments until the optimum effect under circumstances is reached. Dosage amounts and intervals can be adjusted individually to provide levels of the administered compound effective for the particular clinical indication being treated. This will provide a therapeutic regimen that is commensurate with the severity of the individual's disease state.

As used herein, the term "administering" means oral administration, administration as a suppository, topical contact, intravenous, parenteral, intraperitoneal, intramuscular, intralesional, intrathecal, intranasal or subcutaneous administration, or the implantation of a slow-release device, *e.g.*, a mini-osmotic pump, to a subject. Administration is by any route, including parenteral and transmucosal (*e.g*., buccal, sublingual, palatal, gingival, nasal, vaginal, rectal, or transdermal). Parenteral administration includes, *e.g*., intravenous, intramuscular, intra-arteriole, intradermal, subcutaneous, intraperitoneal, intraventricular, and intracranial. Other modes of delivery include, but are not limited to, the use of liposomal formulations, intravenous infusion, transdermal patches, *etc.* In embodiments, the administering does not include administration of any active agent other than the recited active agent.

"Co-administer" it is meant that a composition described herein is administered at the same time, just prior to, or just after the administration of one or more additional therapies. The compounds provided herein can be administered alone or can be coadministered to the patient. Coadministration is meant to include simultaneous or sequential administration of the compounds individually or in combination (more than one compound). Thus, the preparations can also be combined, when desired, with other active substances (e.g. to reduce metabolic degradation). The compositions of the present disclosure can be delivered transdermally, by a topical route, or formulated as applicator sticks, solutions, suspensions, emulsions, gels, creams, ointments, pastes, jellies, paints, powders, and aerosols.

As used herein, the term "about" means a range of values including the specified value, which a person of ordinary skill in the art would consider reasonably similar to the specified value. In embodiments, about means within a standard deviation using measurements generally acceptable in the art. In embodiments, about means a range extending to +/- 10% of the specified value. In embodiments, about includes the specified value.

A "cell" as used herein, refers to a cell carrying out metabolic or other function sufficient to preserve or replicate its genomic DNA. A cell can be identified by well-known methods in the art including, for example, presence of an intact membrane, staining by a particular dye, ability to produce progeny or, in the case of a gamete, ability to combine with a second gamete to produce a viable offspring. Cells may include prokaryotic and eukaroytic cells. Prokaryotic cells include but are not limited to bacteria. Eukaryotic cells include but are not limited to yeast cells and cells derived from plants and animals, for example mammalian, insect (e.g., spodoptera) and human cells. Cells may be useful when they are naturally nonadherent or have been treated not to adhere to surfaces, for example by trypsinization.

"Control" or "control experiment" is used in accordance with its plain ordinary meaning and refers to an experiment in which the subjects or reagents of the experiment are treated as in a parallel experiment except for omission of a procedure, reagent, or variable of the experiment. In some instances, the control is used as a standard of comparison in evaluating experimental effects. In some embodiments, a control is the measurement of the activity of a protein in the absence of a compound as described herein (including embodiments and examples).

The term "modulator" refers to a composition that increases or decreases the level of a target molecule or the function of a target molecule or the physical state of the target of the molecule. In some embodiments, an Adenosine A2B Receptor or Adenosine A2A Receptor associated disease modulator is a compound that reduces the severity of one or more symptoms of a disease associated with Adenosine A2B Receptor or Adenosine A2A Receptor (e.g. cancer). An Adenosine A2B Receptor or Adenosine A2A Receptor modulator is a compound that increases or decreases the activity or function or level of activity or level of function of Adenosine A2B Receptor or Adenosine A2A Receptor relative to the activity or function or level of activity or level of function of Adenosine A2B Receptor or Adenosine A2A Receptor in the absence of the Adenosine A2B Receptor or Adenosine A2A Receptor modulator.

The term "modulate" is used in accordance with its plain ordinary meaning and refers to the act of changing or varying one or more properties. "Modulation" refers to the process of changing or varying one or more properties. For example, as applied to the effects of a modulator on a target protein, to modulate means to change by increasing or decreasing a property or function of the target molecule or the amount of the target molecule.

The term "associated" or "associated with" in the context of a substance or substance activity or function associated with a disease (e.g. a protein associated disease, a cancer associated with Adenosine A2B Receptor activity, Adenosine A2B Receptor associated cancer, Adenosine A2B Receptor associated disease (e.g., cancer, inflammatory disease, neurodegenerative disorder, or fibrotic disorder)) means that the disease (e.g., cancer, inflammatory disease, neurodegenerative disorder, or fibrotic disorder) is caused by (in whole or in part), or a symptom of the disease is caused by (in whole or in part) the substance or substance activity or function. For example, a cancer associated with Adenosine A2B Receptor or Adenosine A2A Receptor activity or function may be a cancer that results (entirely or partially) from aberrant Adenosine A2B Receptor or Adenosine A2A Receptor function (e.g. enzyme activity, protein-protein interaction, signaling pathway) or a cancer wherein a particular symptom of the disease is caused (entirely or partially) by aberrant Adenosine A2B Receptor or Adenosine A2A Receptor activity or function. As used herein, what is described as being associated with a disease, if a causative agent, could be a target for treatment of the disease. For example, a cancer associated with Adenosine A2B Receptor or Adenosine A2A Receptor activity or function or a Adenosine A2B Receptor or Adenosine A2A Receptor associated disease (e.g., cancer, inflammatory disease, neurodegenerative disorder, or fibrotic disorder), may be treated with a Adenosine A2B Receptor or Adenosine A2A Receptor modulator or Adenosine A2B Receptor or Adenosine A2A Receptor inhibitor, in the instance where increased Adenosine A2B Receptor or Adenosine A2A Receptor activity or function (e.g. signaling pathway activity) causes the disease (e.g., cancer, inflammatory disease, neurodegenerative disorder, or fibrotic disorder).

The term "aberrant" as used herein refers to different from normal. When used to describe enzymatic activity or protein function, aberrant refers to activity or function that is greater or less than a normal control or the average of normal non-diseased control samples. Aberrant activity may refer to an amount of activity that results in a disease, wherein returning the aberrant activity to a normal or non-disease-associated amount (e.g. by administering a compound or using a method as described herein), results in reduction of the disease or one or more disease symptoms.

"Anti-cancer agent" or "anti-cancer drug" is used in accordance with its plain ordinary meaning and refers to a composition (e.g., compound, drug, antagonist, inhibitor, modulator) having antineoplastic properties or the ability to inhibit the growth or proliferation of cells. In some embodiments, an anti-cancer agent is a chemotherapeutic. In some embodiments, an anti-cancer agent is an agent approved by the FDA or similar regulatory agency of a country other than the USA, for treating cancer. Examples of anti-cancer agents include, but are not limited to, anti-androgens (e.g., Casodex, Flutamide, MDV3100, or ARN-509), MEK (e.g., MEK1, MEK2, or MEK1 and MEK2) inhibitors (e.g., XL518, CI-1040, PD035901, selumetinib/ AZD6244, GSK1120212/ trametinib, GDC-0973, ARRY-162, ARRY-300, AZD8330, PD0325901, U0126, PD98059, TAK-733, PD318088, AS703026, BAY 869766), alkylating agents (e.g., cyclophosphamide, ifosfamide, chlorambucil, busulfan, melphalan, mechlorethamine, uramustine, thiotepa, nitrosoureas, nitrogen mustards (e.g., mechloroethamine, cyclophosphamide, chlorambucil, meiphalan), ethylenimine and methylmelamines (e.g., hexamethlymelamine, thiotepa), alkyl sulfonates (e.g., busulfan), nitrosoureas (e.g., carmustine, lomusitne, semustine, streptozocin), triazenes (decarbazine)), anti-metabolites (e.g., 5- azathioprine, leucovorin, capecitabine, fludarabine, gemcitabine, pemetrexed, raltitrexed, folic acid analog (e.g., methotrexate), pyrimidine analogs (e.g., fluorouracil, floxouridine, Cytarabine), purine analogs (e.g., mercaptopurine, thioguanine, pentostatin), etc.), plant alkaloids (e.g., vincristine, vinblastine, vinorelbine, vindesine, podophyllotoxin, paclitaxel, docetaxel, etc.), topoisomerase inhibitors (e.g., irinotecan, topotecan, amsacrine, etoposide (VP16), etoposide phosphate, teniposide, etc.), antitumor antibiotics (e.g., doxorubicin, adriamycin, daunorubicin, epirubicin, actinomycin, bleomycin, mitomycin, mitoxantrone, plicamycin, etc.), platinum-based compounds (e.g., cisplatin, oxaloplatin, carboplatin), anthracenedione (e.g., mitoxantrone), substituted urea (e.g., hydroxyurea), methyl hydrazine derivative (e.g., procarbazine), adrenocortical suppressant (e.g., mitotane, aminoglutethimide), epipodophyllotoxins (e.g., etoposide), antibiotics (e.g., daunorubicin, doxorubicin, bleomycin), enzymes (e.g., L-asparaginase), inhibitors of mitogen-activated protein kinase signaling (e.g. U0126, PD98059, PD184352, PD0325901, ARRY-142886, SB239063, SP600125, BAY 43-9006, wortmannin, or LY294002), mTOR inhibitors, antibodies (e.g., rituxan), 5-aza-2'-deoxycytidine, doxorubicin, vincristine, etoposide, gemcitabine, imatinib (Gleevec.RTM.), geldanamycin, 17-N-Allylamino-17-Demethoxygeldanamycin (17-AAG), bortezomib, trastuzumab, anastrozole; angiogenesis inhibitors; antiandrogen, antiestrogen; antisense oligonucleotides; apoptosis gene modulators; apoptosis regulators; arginine deaminase; BCR/ABL antagonists; beta lactam derivatives; bFGF inhibitor; bicalutamide; camptothecin derivatives; casein kinase inhibitors (ICOS); clomifene analogues; cytarabine dacliximab; dexamethasone; estrogen agonists; estrogen antagonists; etanidazole; etoposide phosphate; exemestane; fadrozole; finasteride; fludarabine; fluorodaunorunicin hydrochloride; gadolinium texaphyrin; gallium nitrate; gelatinase inhibitors; gemcitabine; glutathione inhibitors; hepsulfam; immunostimulant peptides; insulin-like growth factor-1 receptor inhibitor; interferon agonists; interferons; interleukins; letrozole; leukemia inhibiting factor; leukocyte alpha interferon; leuprolide+estrogen+progesterone; leuprorelin; matrilysin inhibitors; matrix metalloproteinase inhibitors; MIF inhibitor; mifepristone; mismatched double stranded RNA; monoclonal antibody,; mycobacterial cell wall extract; nitric oxide modulators; oxaliplatin; panomifene; pentrozole; phosphatase inhibitors; plasminogen activator inhibitor; platinum complex; platinum compounds; prednisone; proteasome inhibitors; protein A-based immune modulator; protein kinase C inhibitor; protein tyrosine phosphatase inhibitors; purine nucleoside phosphorylase inhibitors; ras farnesyl protein transferase inhibitors; ras inhibitors; ras-GAP inhibitor; ribozymes; signal transduction inhibitors; signal transduction modulators; single chain antigen-binding protein; stem cell inhibitor; stem-cell division inhibitors; stromelysin inhibitors; synthetic glycosaminoglycans; tamoxifen methiodide; telomerase inhibitors; thyroid stimulating hormone; translation inhibitors; tyrosine kinase inhibitors; urokinase receptor antagonists; steroids (e.g., dexamethasone), finasteride, aromatase inhibitors, gonadotropin-releasing hormone agonists (GnRH) such as goserelin or leuprolide, adrenocorticosteroids (e.g., prednisone), progestins (e.g., hydroxyprogesterone caproate, megestrol acetate, medroxyprogesterone acetate), estrogens (e.g., diethlystilbestrol, ethinyl estradiol), antiestrogen (e.g., tamoxifen), androgens (e.g., testosterone propionate, fluoxymesterone), antiandrogen (e.g., flutamide), immunostimulants (e.g., Bacillus Calmette-Guérin (BCG), levamisole, interleukin-2, alpha-interferon, etc.), monoclonal antibodies (e.g., anti-CD20, anti-HER2, anti-CD52, anti-HLA-DR, and anti-VEGF monoclonal antibodies), immunotoxins (e.g., anti-CD33 monoclonal antibody-calicheamicin conjugate, anti-CD22 monoclonal antibody-pseudomonas exotoxin conjugate, etc.), radio immunotherapy (e.g., anti-CD20 monoclonal antibody conjugated to ¹¹¹In, ⁹⁰Y, or ¹³¹I, etc.), triptolide, homoharringtonine, dactinomycin, doxorubicin, epirubicin, topotecan, itraconazole, vindesine, cerivastatin, vincristine, deoxyadenosine, sertraline, pitavastatin, irinotecan, clofazimine, 5-nonyloxytryptamine, vemurafenib, dabrafenib, erlotinib, gefitinib, EGFR inhibitors, epidermal growth factor receptor (EGFR)-targeted therapy or therapeutic (e.g., gefitinib (Iressa ™), erlotinib (Tarceva ™), cetuximab (Erbitux™), lapatinib (Tykerb™), panitumumab (Vectibix™), vandetanib (Caprelsa™), afatinib/BIBW2992, CI-1033/canertinib, neratinib/HKI-272, CP-724714, TAK-285, AST-1306, ARRY334543, ARRY-380, AG-1478, dacomitinib/PF299804, OSI-420/desmethyl erlotinib, AZD8931, AEE788, pelitinib/EKB-569, CUDC-101, WZ8040, WZ4002, WZ3146, AG-490, XL647, PD153035, BMS-599626), sorafenib, imatinib, sunitinib, dasatinib, pyrrolo benzodiazepines (e.g., tomaymycin), carboplatin, CC-1065 and CC-1065 analogs including amino-CBIs, nitrogen mustards (such as chlorambucil and melphalan), dolastatin and dolastatin analogs (including auristatins: e.g., monomethyl auristatin E), anthracycline antibiotics (such as doxorubicin, daunorubicin, etc.), duocarmycins and duocarmycin analogs, enediynes (such as neocarzinostatin and calicheamicins), leptomycin derivaties, maytansinoids and maytansinoid analogs (e.g., mertansine), methotrexate, mitomycin C, taxoids, vinca alkaloids (such as vinblastine and vincristine), epothilones (e.g., epothilone B), camptothecin and its clinical analogs topotecan and irinotecan, or the like.

The term "signaling pathway" as used herein refers to a series of interactions between cellular and optionally extra-cellular components (e.g. proteins, nucleic acids, small molecules, ions, lipids) that conveys a change in one component to one or more other components, which in turn may convey a change to additional components, which is optionally propagated to other signaling pathway components. For example, binding of a Adenosine A2B Receptor or Adenosine A2A Receptor with a compound as described herein may reduce the level of a product of the Adenosine A2B Receptor or Adenosine A2A Receptor catalyzed reaction or the level of a downstream derivative of the product or binding may reduce the interactions between the Adenosine A2B Receptor or Adenosine A2A Receptor or an Adenosine A2B Receptor or Adenosine A2A Receptor reaction product and downstream effectors or signaling pathway components, resulting in changes in cell growth, proliferation, or survival.

### II. Compounds

In an aspect is provided a compound having the formula:

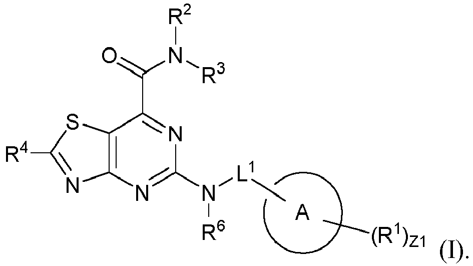

Ring A is a cycloalkyl, heterocycloalkyl, aryl, or heteroaryl.

L¹ is a bond, -SOₙ₅L^{1A}-, -SOᵥ₅NR⁵L^{1A}-, -NHC(O)NR⁵L^{1A}-, -NR⁵L^{1A}-, -C(O)L^{1A}-, -C(O)OL^{1A}-, -C(O)NR⁵L^{1A}-, -OL^{1A}-, -NR⁵SO₂L^{1A}-, -NR⁵C(O)L^{1A}-, -NR⁵C(O)OL^{1A}-, -NR⁵OL^{1A}-, -SL^{1A}-, substituted or unsubstituted alkylene, substituted or unsubstituted heteroalkylene, substituted or unsubstituted cycloalkylene, substituted or unsubstituted heterocycloalkylene, substituted or unsubstituted arylene, or substituted or unsubstituted heteroarylene.

R¹ is halogen, -CX¹₃, -CHX¹₂, -CH₂X¹, -OCX¹₃, -OCH₂X¹, -OCHX¹₂, -N₃, -CN, -SOₙ₁R^{1D}, -SOᵥ₁NR^{1A}R^{1B}, -NHC(O)NR^{1A}R^{1B}, -N(O)ₘ₁, -NR^{1A}R^{1B}, -C(O)R^{1C}, -C(O)-OR^{1C}, -C(O)NR^{1A}R^{1B}, -OR^{1D}, -NR^{1A}SO₂R^{1D}, -NR^{1A}C(O)R^{1C}, -NR^{1A}C(O)OR^{1C}, -NR^{1A}OR^{1C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

R² is hydrogen, -CX²₃, -CHX²₂, -CH₂X², -OCX²₃, -OCH₂X², -OCHX²₂, -C(O)R^{2C}, -C(O)OR^{2C}, -C(O)NR^{2A}R^{2B}, -OR^{2D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

R³ is hydrogen, -CX³₃, -CHX³₂, -CH₂X³, -OCX³₃, -OCH₂X³, -OCHX³₂, -C(O)R^{3C}, -C(O)OR^{3C}, -C(O)NR^{3A}R^{3B}, -OR^{3D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

R² and R³ together with the nitrogen attached thereto may be optionally joined to form a substituted or unsubstituted heterocycloalkyl, or substituted or unsubstituted heteroaryl.

R⁴ is hydrogen, halogen, -CX⁴₃, -CHX⁴₂, -CH₂X⁴, -OCX⁴₃, -OCH₂X⁴, -OCHX⁴₂, -N₃, -CN, -SOₙ₄R^{4D}, -SOᵥ₄NR^{4A}R^{4B}, -NHC(O)NR^{4A}R^{4B}, -N(O)ₘ₄, -NR^{4A}R^{4B}, -C(O)R^{4C}, -C(O)-OR^{4C}, -C(O)NR^{4A}R^{4B}, -OR^{4D}, -NR^{4A}SO₂R^{4D}, -NR^{4A}C(O)R^{4C}, -NR4^{A}C(O)OR^{4C}, -NR^{4A}OR^{4C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

R⁵ is hydrogen, -CX⁵₃, -CHX⁵₂, -CH₂X⁵, -OCX⁵₃, -OCH₂X⁵, -OCHX⁵₂, -C(O)R^{5C}, -C(O)OR^{5C}, -C(O)NR^{5A}R^{5B}, -OR^{5D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

R⁶ is hydrogen, -CX⁶₃, -CHX⁶₂, -CH₂X⁶, -OCX⁶₃, -OCH₂X⁶, -OCHX⁶₂, -C(O)R^{6C}, -C(O)OR^{6C}, -C(O)NR^{6A}R^{6B}, -OR^{6D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

R^{1A}, R^{1B} , R^{1C}, R^{1D}, R^{2A}, R^{2B}, R^{2C}, R^{2D}, R^{3A}, R^{3B}, R^{3C}, R^{3D}, R^{4A}, R^{4B}, R^{4C}, R^{4D}, R^{5A}, R^{5B}, R^{5C}, R^{5D}, R^{6A}, R^{6B}, R^{6C}, and R^{6D} are independently hydrogen, -CX₃, -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

L^{1A} is a bond, substituted or unsubstituted alkylene, substituted or unsubstituted heteroalkylene, substituted or unsubstituted cycloalkylene, substituted or unsubstituted heterocycloalkylene, substituted or unsubstituted arylene, or substituted or unsubstituted heteroarylene. In embodiments, L^{1A} is substituted or unsubstituted alkylene, substituted or unsubstituted heteroalkylene, substituted or unsubstituted cycloalkylene, substituted or unsubstituted heterocycloalkylene, substituted or unsubstituted arylene, or substituted or unsubstituted heteroarylene.

z1 is an integer from 0 to 12. n1 and n4 are independently an integer from 0 to 4. m1, m4, v1, and v4 are independently an integer from 1 to 2. X, X¹, X², X³, X⁴, X⁵ and X⁶ are independently -F, -Cl, -Br, or -I.

In embodiments, the Ring A is a cycloalkyl (e.g., C₃-C₈, C₃-C₆, C₄-C₆, or C₅-C₆ cycloalkyl), heterocycloalkyl (e.g., 3 to 8, 3 to 6, 4 to 6, to 5 to 6 membered heterocycloalkyl), aryl (e.g., C₆-C₁₀, or C₅-C₆ aryl or phenyl) or heteroaryl (e.g., 5 to 10 membered, 5 to 9 membered, or 5 to 6 membered heteroaryl). In embodiments, the Ring A is a cycloalkyl (e.g., C₃-C₈, C₃-C₆, C₄-C₆, or C₅-C₆ cycloalkyl). In embodiments, the Ring A is a heterocycloalkyl (e.g., 3 to 8 membered, 3 to 6 membered, 4 to 6 membered, or 5 to 6 membered heterocycloalkyl). In embodiments, the Ring A is an aryl (e.g., C₆-C₁₀, or C₅-C₆ aryl or phenyl). In embodiments, the Ring A is a heteroaryl (e.g., 5 to 10 membered, 5 to 9 membered, or 5 to 6 membered heteroaryl).

In embodiments, the Ring A is a C₃-C₈ cycloalkyl. In embodiments, the Ring A is a C₃-C₆ cycloalkyl. In embodiments, the Ring A is a C₄-C₆ cycloalkyl. In embodiments, the Ring A is a C₅-C₆ cycloalkyl. In embodiments, the Ring A is a 3 to 8 membered heterocycloalkyl. In embodiments, the Ring A is a 3 to 6 membered heterocycloalkyl. In embodiments, the Ring A is a 4 to 6 membered heterocycloalkyl. In embodiments, the Ring A is a 5 or 6 membered heterocycloalkyl.

In embodiments, the Ring A is a C₆-C₁₀, aryl. In embodiments, the Ring A is a C₅-C₆, aryl. In embodiments, the Ring A is a phenyl. In embodiments, the Ring A is a 5 to 10 membered heteroaryl. In embodiments, the Ring A is a 5 to 9 membered heteroaryl. In embodiments, the Ring A is a 5 to 6 membered heteroaryl (e.g., 5 membered or 6 membered heteroaryl).

In emboduments, L¹ is a bond. In emboduments, L¹ is -SO₂-. In emboduments, L¹ is -NHCH₂-. In emboduments, L¹ is -C(O)CH₂- or -C(O)CH₂CH₂-. In emboduments, L¹ is -C(O)OCH₂-, or -C(O)OCH₂CH₂-. In emboduments, L¹ is -C(O)NHCH₂-, or -C(O)NHCH₂CH₂-. In emboduments, L¹ is -OCH₂- or -OCH₂CH₂-. In emboduments, L¹ is -NHSO₂CH₂-. In emboduments, L¹ is -NHC(O)CH₂-, -NHC(O)-CH=CH-, or -N(CH₃)C(O)CH₂CH₂-. In emboduments, L¹ is NHC(O)OCH₂-, -NHC(O)O-CH=CH-, or -N(CH₃)C(O)OCH₂CH₂-. In emboduments, L¹ is -NHOCH₂-. In emboduments, L¹ is -SCH₂- or -SCH₂CH₂-. In emboduments, L¹ is substituted or unsubstituted alkylene (e.g., C₁-C₈, C₁-C₆, C₁-C₄, or C₁-C₂). In emboduments, L¹ is substituted or unsubstituted heteroalkylene (e.g., 2 to 8 membered, 2 to 6 membered, 4 to 6 membered, 2 to 3 membered, or 4 to 5 membered). In emboduments, L¹ is substituted or unsubstituted cycloalkylene (e.g., C₃-C₈, C₃-C₆, C₄-C₆, or C₅-C₆). In emboduments, L¹ is substituted or unsubstituted heterocycloalkylene (e.g., 3 to 8 membered, 3 to 6 membered, 4 to 6 membered, 4 to 5 membered, or 5 to 6 membered). In emboduments, L¹ is substituted or unsubstituted arylene (e.g., C₆-C₁₀ or phenylene). In emboduments, L¹ is substituted or unsubstituted heteroarylene (e.g., 5 to 10 membered, 5 to 9 membered, or 5 to 6 membered). In embodiments, L¹ is not a bond.

In embodiments, L¹ is substituted or unsubstituted C₁-C₈ alkylene. In embodiments, L¹ is unsubstituted C₁-C₈ alkylene. In embodiments, L¹ is substituted or unsubstituted C₁-C₆ alkylene. In embodiments, L¹ is unsubstituted C₁-C₆ alkylene. In embodiments, L¹ is substituted or unsubstituted C₁-C₄ alkylene. In embodiments, L¹ is unsubstituted C₁-C₄ alkylene. In embodiments, L¹ is substituted or unsubstituted C₁-C₃ alkylene. In embodiments, L¹ is unsubstituted C₁-C₃ alkylene. In embodiments, L¹ is substituted or unsubstituted ethylene. In embodiments, L¹ is substituted or unsubstituted methylene. In embodiments, L¹ is unsubstituted ethylene. In embodiments, L¹ is unsubstituted methylene. In embodiments, L¹ is a substituted or unsubstituted 2 to 8 membered heteroalkylene. In embodiments, L¹ is unsubstituted 2 to 8 membered heteroalkylene. In embodiments, L¹ is a substituted or unsubstituted 2 to 6 membered heteroalkylene. In embodiments, L¹ is unsubstituted 2 to 6 membered heteroalkylene. In embodiments, L¹ is a substituted or unsubstituted 4 to 6 membered heteroalkylene. In embodiments, L¹ is unsubstituted 4 to 6 membered heteroalkylene. In embodiments, L¹ is a substituted or unsubstituted 2 to 3 membered heteroalkylene. In embodiments, L¹ is unsubstituted 2 to 3 membered heteroalkylene.

In embodiments, L¹ is 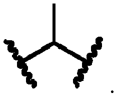 In embodiments, L¹ is 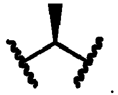 In embodiments, L¹ is 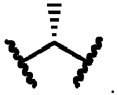 In embodiments, L¹ is 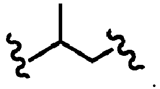 In embodiments, L¹ is 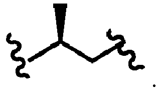 In embodiments, L¹ is 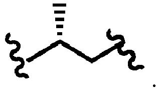 In embodiments, L¹ is 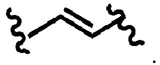 In embodiments, L¹ is 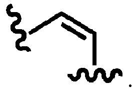 In embodiments, L¹ is 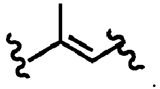 In embodiments, L¹ is

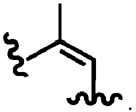

In embodiments, R¹ is independently halogen (e.g., -F, -Cl, Br, or -I). In embodiments, R¹ is independently -F. In embodiments, R¹ is independently -Cl. In embodiments, R¹ is independently -Br. In embodiments, R¹ is independently -I. In embodiments, R¹ is independently -CX¹₃ (e.g., -CF₃, -CCl₃, -CBr₃, or -CI₃). In embodiments, R¹ is independently
-CHX¹₂ (e.g., -CHF₂, -CHCl₂, -CHBr₂, or -CHI₂). In embodiments, R¹ is independently -CH₂X¹ (e.g., -CH₂F,-CH₂Cl, -CH2Br, or -CH₂I). In embodiments, R¹ is independently -OCX¹₃ (e.g., -OCF₃, -OCCl₃, -OCBr₃, or -OCI₃). In embodiments, R¹ is independently -OCH₂X¹ (e.g., -OCH₂F, -OCH₂Cl, -OCH₂Br, or -OCH₂I). In embodiments, R¹ is independently -OCHX¹₂ (e.g., -OCHF₂, -OCHCl₂, -OCHBr₂, or -OCHI₂). In embodiments, R¹ is independently -CN. In embodiments, R¹ is independently -SR^{1D} (e.g., -SH, or -SCH₃). In embodiments, R¹ is independently -NHC(O)NR^{1A}R^{1B} (e.g., -NHC(O)NH₂, or -NHC(O)NHCH₃). In embodiments, R¹ is -NR^{1A}R^{1B} (e.g., -NH₂, or -NHCH₃). In embodiments, R¹ is independently -C(O)-OR^{1C} (e.g., -C(O)OH, or-C(O)OCH₃). In embodiments, R¹ is independently -C(O)R^{1C} (e.g., -C(O)H or -C(O)CH₃). In embodiments, R¹ is independently -C(O)NR^{1A}R^{1B} (e.g., -C(O)NH₂ or -C(O)NHCH₃). In embodiments, R¹ is independently -OR^{1D} (e.g., -OH, or -OCH₃). In embodiments, R¹ is independently -NR^{1A}SO₂R^{1D} (e.g., -NHSO₂H or -NHSO₂CH₃). In embodiments, R¹ is independently -NR^{1A}C(O)R^{1C} (e.g., -NHC(O)H or -NCH₃C(O)H). In embodiments, R¹ is independently -NR^{1A}C(O)OR^{1C} (e.g., -NHC(O)OH or -NCH₃C(O)OH).

In embodiments, R¹ is independently substituted or unsubstituted alkyl (e.g., C₁-C₈, C₁-C₆, C₁-C₄, or C₁-C₂). In embodiments, R¹ is independently substituted or unsubstituted C₁-C₈ alkyl. In embodiments, R¹ is independently unsubstituted C₁-C₈ alkyl. In embodiments, R¹ is independently substituted or unsubstituted C₁-C₆ alkyl. In embodiments, R¹ is independently unsubstituted C₁-C₆ alkyl. In embodiments, R¹ is independently substituted or unsubstituted C₁-C₄ alkyl. In embodiments, R¹ is independently unsubstituted C₁-C₄ alkyl.

In embodiments, R¹ is independently substituted or unsubstituted 2 to 8 membered heteroalkyl. In embodiments, R¹ is independently unsubstituted 2 to 8 membered heteroalkyl. In embodiments, R¹ is independently substituted or unsubstituted 2 to 6 membered heteroalkyl. In embodiments, R¹ is independently unsubstituted 2 to 6 membered heteroalkyl. In embodiments, R¹ is independently substituted or unsubstituted 4 to 6 membered heteroalkyl. In embodiments, R¹ is independently unsubstituted 4 to 6 membered heteroalkyl. In embodiments, R¹ is independently substituted or unsubstituted 2 to 3 membered heteroalkyl. In embodiments, R¹ is independently unsubstituted 2 to 3 membered heteroalkyl.

In embodiments, R⁶ is halogen (e.g., -F, -Cl, Br, or -I). In embodiments, R⁶ is-CX⁶₃ (e.g., -CF₃, -CCl₃, -CBr₃, or -CI₃). In embodiments, R⁶ is -CHX⁶₂ (e.g., -CHF₂,-CHCl₂,
-CHBr₂ or -CHI₂). In embodiments, R⁶ is -CH₂X⁶ (e.g., -CH₂F,-CH₂Cl, -CH₂Br, or -CH₂I). In embodiments, R⁶ is -OCX⁶₃ (e.g., -OCF₃, -OCCl₃, -OCBr₃, or -OCI₃). In embodiments, R⁶ is -OCH₂X⁶ (e.g., -OCH₂F, -OCH₂Cl, -OCH₂Br, or -OCH₂I). In embodiments, R⁶ is-OCHX⁶₂ (e.g., -OCHF₂, -OCHCl₂, -OCHBr₂, or -OCHI₂). In embodiments, R⁶ is -C(O)-OR^{6C} (e.g., -C(O)OH, or -C(O)OCH₃). In embodiments, R¹⁶ is -C(O)R^{6C} (e.g.,-C(O)H or -C(O)CH₃). In embodiments, R⁶ is -C(O)NR^{6A}R^{6B} (e.g., -C(O)NH₂ or -C(O)NHCH₃). In embodiments, R⁶ is -OR^{6D} (e.g., -OH, or -OCH₃). In embodiments, R⁶ is substituted or unsubstituted alkyl (e.g., C₁-C₈, C₁-C₆, C₁-C₄, or C₁-C₂). In embodiments, R⁶ is substituted or unsubstituted C₁-C₈ alkyl. In embodiments, R⁶ is unsubstituted C₁-C₈ alkyl. In embodiments, R⁶ is substituted or unsubstituted C₁-C₆ alkyl. In embodiments, R⁶ is unsubstituted C₁-C₆ alkyl. In embodiments, R⁶ is substituted or unsubstituted C₁-C₄ alkyl. In embodiments, R⁶ is unsubstituted C₁-C₄ alkyl. In embodiments, R⁶ is unsubstituted methyl. In embodiments, R⁶ is hydrogen.

In embodiment, R⁴ is hydrogen. In embodiments, R⁴ is halogen (e.g., -F, -Cl, Br, or -I). In embodiments, R⁴ is -CX⁴₃ (e.g., -CF₃, -CCl₃, -CBr₃, or -CI₃). In embodiments, R⁴ is -CHX⁴₂ (e.g., -CHF₂, -CHCl₂,-CHBr₂, or-CHI₂). In embodiments, R⁴ is -CH₂X⁴ (e.g.,-CH₂F, -CH₂Cl, -CH₂Br, or -CH₂I). In embodiments, R⁴ is -OCX⁴₃ (e.g., -OCF₃, -OCCl₃,-OCBr₃, or -OCI₃). In embodiments, R⁴ is -OCH₂X⁴ (e.g., -OCH₂F, -OCH₂Cl, -OCH₂Br, or-OCH₂I). In embodiments, R⁴ is -OCHX⁴₂ (e.g., -OCHF₂, -OCHCl₂, -OCHBr₂, or -OCHI₂). In embodiments, R⁴ is -CN. In embodiments, R⁴ is -SR^{4D} (e.g., -SH, or -SCH₃). In embodiments, R⁴ is -NHC(O)NR^{4A}R^{4B} (e.g., -NHC(O)NH₂, or -NHC(O)NHCH₃). In embodiments, R⁴ is
-NR^{4A}R^{4B} (e.g., -NH₂, or -NHCH₃). In embodiments, R⁴ is -C(O)-OR^{4C} (e.g., -C(O)OH, or -C(O)OCH₃). In embodiments, R⁴ is -C(O)R^{4C} (e.g., -C(O)H or -C(O)CH₃). In embodiments, R⁴ is -C(O)NR^{4A}R^{4B} (e.g., -C(O)NH₂ or -C(O)NHCH₃). In embodiments, R⁴ is -OR^{4D} (e.g., -OH, or -OCH₃). In embodiments, R⁴ is -NR^{4A}C(O)R^{4C} (e.g., -NHC(O)H or -NCH₃C(O)H). In embodiments, R⁴ is -NR^{4A}C(O)OR^{4C} (e.g., -NHC(O)OH or-NCH₃C(O)OH). In embodiments, R⁴ is -NR^{4A}OR^{4C} (e.g., -NHOH or -NCH₃OH). In embodiments, R⁴ is -NH₂.

In embodiments, R⁴ is substituted or unsubstituted alkyl (e.g., C₁-C₈, C₁-C₆, C₁-C₄, or C₁-C₂). In embodiments, R⁴ is substituted or unsubstituted C₁-C₈ alkyl. In embodiments, R⁴ is unsubstituted C₁-C₈ alkyl. In embodiments, R⁴ is substituted or unsubstituted C₁-C₆ alkyl. In embodiments, R⁴ is unsubstituted C₁-C₆ alkyl. In embodiments, R⁴ is substituted or unsubstituted C₁-C₄ alkyl. In embodiments, R⁴ is unsubstituted C₁-C₄ alkyl. In embodiments, R⁴ is -CH₃. In embodiments, R⁴ is unsubstituted methyl. In embodiments, R⁴ is unsubstituted ethyl. In embodiments, R⁴ is unsubstituted propyl. In embodiments, R⁴ is unsubstituted butyl. In embodiments, R⁴ is unsubstituted tert-butyl.

In embodiments, R⁴ is substituted or unsubstituted 2 to 8 membered heteroalkyl. In embodiments, R⁴ is unsubstituted 2 to 8 membered heteroalkyl. In embodiments, R⁴ is substituted or unsubstituted 2 to 6 membered heteroalkyl. In embodiments, R⁴ is unsubstituted 2 to 6 membered heteroalkyl. In embodiments, R⁴ is substituted or unsubstituted 4 to 6 membered heteroalkyl. In embodiments, R⁴ is unsubstituted 4 to 6 membered heteroalkyl. In embodiments, R⁴ is substituted or unsubstituted 2 to 3 membered heteroalkyl. In embodiments, R⁴ is unsubstituted 2 to 3 membered heteroalkyl.

In embodiments, R⁴ is substituted or unsubstituted cycloalkyl (e.g., C₃-C₈, C₃-C₆, C₄-C₆, or C₅-C₆). In embodiments, R⁴ is substituted or unsubstituted C₃-C₈ cycloalkyl. In embodiments, R⁴ is unsubstituted C₃-C₈ cycloalkyl. In embodiments, R⁴ is substituted or unsubstituted C₃-C₆ cycloalkyl. In embodiments, R⁴ is unsubstituted C₃-C₆ cycloalkyl. In embodiments, R⁴ is substituted or unsubstituted C₅-C₆ cycloalkyl. In embodiments, R⁴ is unsubstituted C₅-C₆ cycloalkyl.

In embodiments, the Ring A is pyridyl. In embodiments, the Ring A is 2-pyridyl. In embodiments, the Ring A is 3-pyridyl. In embodiments, the Ring A is 4-pyridyl.

In embodiments, the compound has the formula: 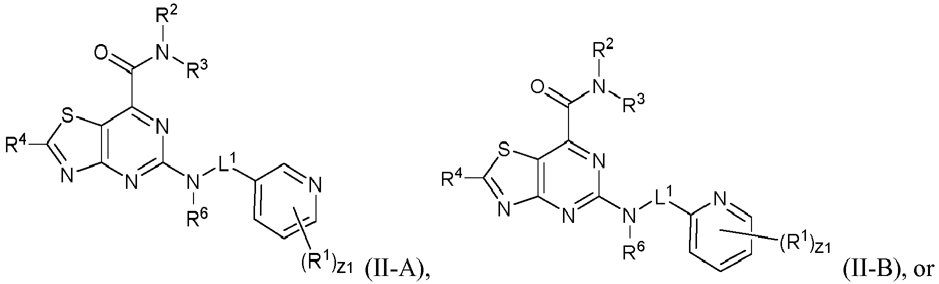 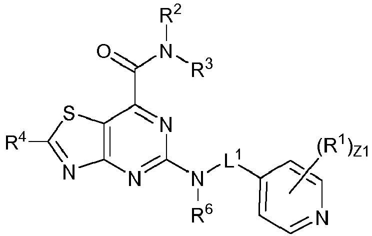 (II-C). R¹, R², R³, R⁴, R⁶, L¹, and z1 are as described herein.

In embodiments, the compound has the formula: 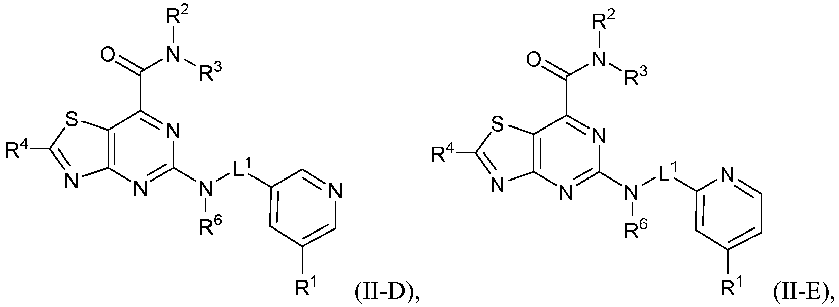 or 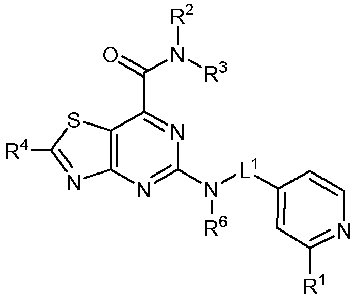 (II-F). R¹, R², R³, R⁴, R⁶, and L¹ are as described herein.

In embodiments, R² and R³ together with the nitrogen attached thereto are joined to form 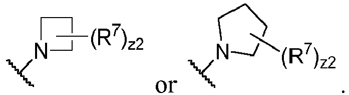 R⁷ is as described herein. z2 is an integer from 0 to 1. In embodiments, z2 is 0. In embodiments, z2 is 1. In embodiments, R⁷ is

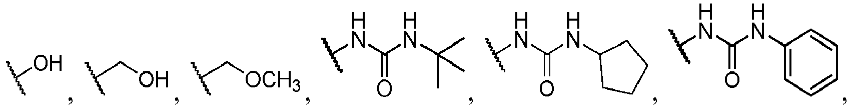

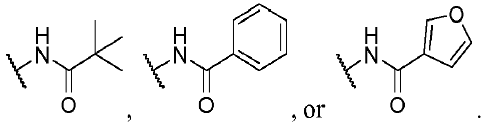

In embodiments, the compound has the formula: 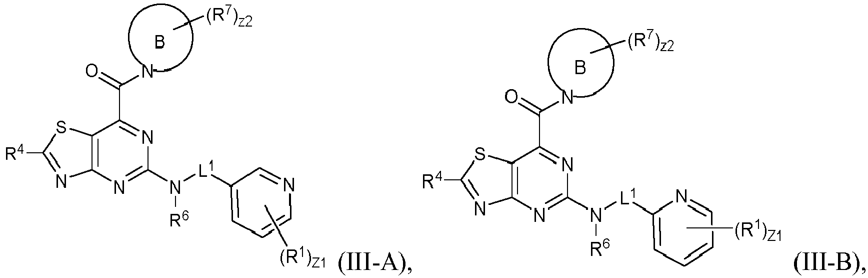 or 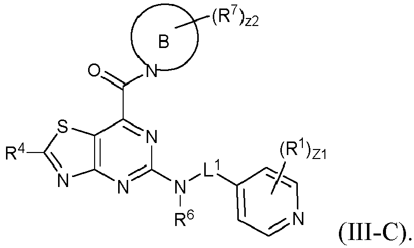 R¹, R⁴, R⁶, L¹, z1, and z2 are as described herein.

In embodiments, the compound has the formula: 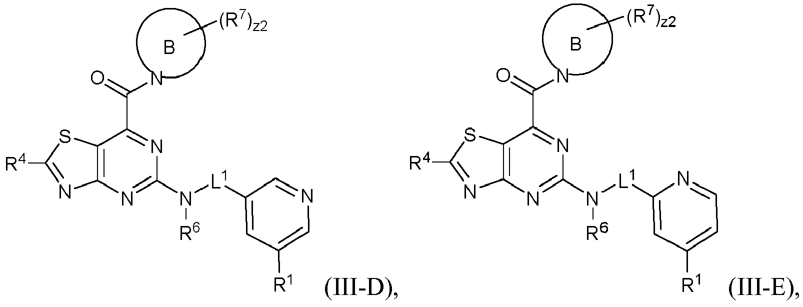 or 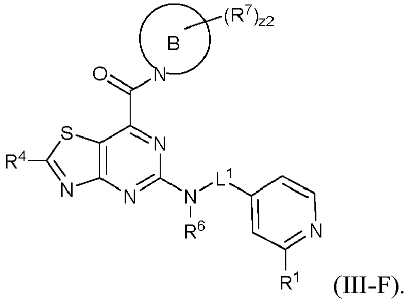 R¹, R⁴, R⁶, L¹, and z2 are as described herein.

Ring B is a heterocycloalkyl or heteroaryl.

R⁷ is independently halogen, -CX⁷₃, -CHX⁷₂, -CH₂X⁷, -OCX⁷₃, -OCH₂X⁷, -OCHX⁷₂, -N₃, -CN, -SOₙ₇R^{7D}, -SOᵥ₇NR^{7A}R^{7B}, -NR^{7A}C(O)NR^{7A}R^{7B}, -NR^{7A}S(O)NR^{7A}R^{7B}, -N(O)ₘ₇, -NR^{7A}R^{7B}, -C(O)R^{7C}, -C(O)-OR^{7C}, -C(O)NR^{7A}R^{7B}, -C(O)NR^{7A}OR^{7B}, -OR^{7D}, -NR^{7A}SO₂R^{7D}, -NR^{7A}C(O)R^{7C}, -NR^{7A}C(O)OR^{7C}, -NR^{7A}OR^{7C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. R^{7A}, R^{7B}, R^{7C}, and R^{7D} are independently hydrogen,-CN, -CX₃, -CHX₂, -CH₂X,
-COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

In embodiments, z1 is an integer from 0 to 4. In embodiments, z1 is 0. In embodiments, z1 is 1. In embodiments, z1 is 2. In embodiments, z1 is 3. In embodiments, z1 is 4. z2 is an integer from 0 to 12. n7 is independently an integer from 0 to 4. m7 and v7 are independently 1 or 2. X⁷ is independently -F, -Cl, -Br, or -I.

In embodiments, the Ring B is a heterocycloalkyl (e.g., 3 to 8 membered, 3 to 7 membered, 4 to 7 membered, or 5 to 6 membered heterocycloalkyl). In embodiments, the Ring B is a 3 to 8 membered heterocycloalkyl. In embodiments, the Ring B is a 3 to 7 membered heterocycloalkyl. In embodiments, the Ring B is a 4 to 7 membered heterocycloalkyl. In embodiments, the Ring B is a 5 to 6 membered heterocycloalkyl. In embodiments, the Ring B is a 3 membered heterocycloalkyl. In embodiments, the Ring B is a 4 membered heterocycloalkyl. In embodiments, the Ring B is a 5 membered heterocycloalkyl. In embodiments, the Ring B is a 6 membered heterocycloalkyl. In embodiments, the Ring B is a 7 membered heterocycloalkyl. In embodiments, the Ring B is a heteroaryl (e.g., 5 to 10 membered, 5 to 9 membered, or 5 to 6 membered heteroaryl). In embodiments, the Ring B is a 5 to 10 membered heteroaryl. In embodiments, the Ring B is a 5 to 9 membered heteroaryl. In embodiments, the Ring B is a 5 to 6 membered heteroaryl. In embodiments, the Ring B is a 5 membered heteroaryl. In embodiments, the Ring B is a 6 membered heteroaryl. In embodiments, Ring B is a 4 to 7 membered heterocycloalkyl or 5 to 6 membered heteroaryl.

In embodiments, R⁷ is independently -NR^{7A}R^{7B} (e.g., -NH₂, or -NHCH₃). In embodiments, R⁷ is independently -C(O)NR^{7A}R^{7B} (e.g., -C(O)NH₂, -C(O)NHCH₃, or -C(O)N(CH₃)₂). In embodiments, R⁷ is independently -NR^{7A}C(O)R^{7C} (e.g., -NHC(O)H, -NCH₃C(O)H, -NCH₃C(O)CH₃, or -NHC(O)CH₃). In embodiments, R⁷ is independently -NR^{7A}C(O)NR^{7A}R^{7B} (e.g., -NHC(O)NH₂, -NCH₃C(O)NH₂, or -NHC(O)NHCH₃). In embodiments, R⁷ is independently -OR^{7D} (e.g., -OH, or -OCH₃). In embodiments, R⁷ is independently -C(O)R^{7C} (e.g., -C(O)H or -C(O)CH₃).

In embodiments, R^{7A}, R^{7B}, R^{7C}, and R^{7D} are independently hydrogen, substituted or unsubstituted C₁-C₆ alkyl, substituted or unsubstituted 2 to 8 membered heteroalkyl, substituted or unsubstituted C₃-C₈ cycloalkyl, substituted or unsubstituted 5 to 6 membered heterocycloalkyl, substituted or unsubstituted phenyl, or substituted or unsubstituted 5 to 6 membered heteroaryl.

In embodiments, R^{7A}, R^{7B}, R^{7C}, and R^{7D} are independently hydrogen, substituted or unsubstituted C₁-C₆ alkyl, substituted or unsubstituted C₃-C₈ cycloalkyl, substituted or unsubstituted 5 to 6 membered heterocycloalkyl, substituted or unsubstituted phenyl, or substituted or unsubstituted 5 to 6 membered heteroaryl.

In embodiments, R^{7A} and R^{7B} are independently hydrogen, or substituted or unsubstituted C₁-C₆ alkyl. In embodiments, R^{7A} and R^{7B} are independently hydrogen, or unsubstituted C₁-C₆ alkyl. In embodiments, R^{7A} is independently hydrogen, or substituted or unsubstituted C₁-C₆ alkyl. In embodiments, R^{7A} is independently hydrogen. In embodiments, R^{7A} is independently substituted or unsubstituted C₁-C₆ alkyl. In embodiments, R^{7A} is independently unsubstituted C₁-C₆ alkyl. In embodiments, R^{7A} is independently substituted or unsubstituted C₁-C₄ alkyl. In embodiments, R^{7A} is independently unsubstituted C₁-C₄ alkyl. In embodiments, R^{7A} is independently substituted butyl. In embodiments, R^{7A} is independently unsubstituted butyl. In embodiments, R^{7A} is independently substituted isobutyl. In embodiments, R^{7A} is independently unsubstituted isobutyl. In embodiments, R^{7A} is independently substituted tert-butyl. In embodiments, R^{7A} is independently unsubstituted tert-butyl. In embodiments, R^{7A} is independently substituted propyl. In embodiments, R^{7A} is independently unsubstituted propyl. In embodiments, R^{7A} is independently substituted isopropyl. In embodiments, R^{7A} is independently unsubstituted isopropyl. In embodiments, R^{7A} is independently substituted ethyl. In embodiments, R^{7A} is independently unsubstituted ethyl. In embodiments, R^{7A} is independently substituted methyl. In embodiments, R^{7A} is independently unsubstituted methyl. In embodiments, R^{7A} is independently substituted neopentyl. In embodiments, R^{7A} is independently unsubstituted neopentyl. In embodiments, R^{7A} is independently substituted pentyl. In embodiments, R^{7A} is independently unsubstituted pentyl. In embodiments, R^{7A} is independently unsubstituted isopentyl. In embodiments, R^{7A} is independently substituted isopentyl. In embodiments, R^{7B} is independently hydrogen, or substituted or unsubstituted C₁-C₆ alkyl. In embodiments, R^{7B} is independently hydrogen. In embodiments, R^{7B} is independently substituted or unsubstituted C₁-C₆ alkyl. In embodiments, R^{7B} is independently unsubstituted C₁-C₆ alkyl. In embodiments, R^{7B} is independently substituted or unsubstituted C₁-C₄ alkyl. In embodiments, R^{7B} is independently unsubstituted C₁-C₄ alkyl. In embodiments, R^{7B} is independently substituted propyl. In embodiments, R^{7B} is independently unsubstituted propyl. In embodiments, R^{7B} is independently substituted isopropyl. In embodiments, R^{7B} is independently unsubstituted isopropyl. In embodiments, R^{7B} is independently substituted ethyl. In embodiments, R^{7B} is independently unsubstituted ethyl. In embodiments, R^{7B} is independently substituted methyl. In embodiments, R^{7B} is independently unsubstituted methyl. In embodiments, R^{7B} is independently substituted neopentyl. In embodiments, R^{7B} is independently unsubstituted neopentyl. In embodiments, R^{7B} is independently substituted pentyl. In embodiments, R^{7B} is independently unsubstituted pentyl. In embodiments, R^{7B} is independently unsubstituted isopentyl. In embodiments, R^{7B} is independently substituted isopentyl.

In embodiments, R^{7C} is independently substituted or unsubstituted alkyl (e.g., C₁-C₁₂, C₁-C₈, C₁-C₆, C₁-C₄, or C₁-C₂). In embodiments, R^{7C} is independently substituted or unsubstituted C₁-C₁₂ alkyl. In embodiments, R^{7C} is independently unsubstituted C₁-C₁₂ alkyl. In embodiments, R^{7C} is independently substituted or unsubstituted C₁-C₈ alkyl. In embodiments, R^{7C} is independently unsubstituted C₁-C₈ alkyl. In embodiments, R^{7C} is independently substituted or unsubstituted C₁-C₆ alkyl. In embodiments, R^{7C} is independently unsubstituted C₁-C₆ alkyl. In embodiments, R^{7C} is independently substituted or unsubstituted C₁-C₆ alkyl. In embodiments, R^{7C} is independently unsubstituted C₁-C₄ alkyl. In embodiments, R^{7C} is independently hydrogen. In embodiments, R^{7C} is independently substituted or unsubstituted C₁-C₆ alkyl. In embodiments, R^{7C} is independently unsubstituted C₁-C₆ alkyl. In embodiments, R^{7C} is independently substituted or unsubstituted C₁-C₄ alkyl. In embodiments, R^{7C} is independently unsubstituted C₁-C₄ alkyl. In embodiments, R^{7C} is independently substituted propyl. In embodiments, R^{7C} is independently unsubstituted propyl. In embodiments, R^{7C} is independently substituted isopropyl. In embodiments, R^{7C} is independently unsubstituted isopropyl. In embodiments, R^{7C} is independently substituted ethyl. In embodiments, R^{7C} is independently unsubstituted ethyl. In embodiments, R^{7C} is independently substituted methyl. In embodiments, R^{7C} is independently unsubstituted methyl. In embodiments, R^{7C} is independently substituted neopentyl. In embodiments, R^{7C} is independently unsubstituted neopentyl. In embodiments, R^{7C} is independently substituted pentyl. In embodiments, R^{7C} is independently unsubstituted pentyl. In embodiments, R^{7C} is independently unsubstituted isopentyl. In embodiments, R^{7C} is independently substituted isopentyl.

In embodiments, R^{7C} is independently substituted or unsubstituted heteroalkyl (e.g., 2 to 8 membered, 2 to 6 membered, 4 to 6 membered, 2 to 3 membered, or 4 to 5 membered). In embodiments, R^{7C} is independently substituted or unsubstituted 2 to 8 membered heteroalkyl. In embodiments, R^{7C} is independently unsubstituted 2 to 8 membered heteroalkyl. In embodiments, R^{7C} is independently substituted or unsubstituted 2 to 6 membered heteroalkyl. In embodiments, R^{7C} is independently unsubstituted 2 to 6 membered heteroalkyl. In embodiments, R^{7C} is independently substituted or unsubstituted 4 to 6 membered heteroalkyl. In embodiments, R^{7C} is independently unsubstituted 4 to 6 membered heteroalkyl. In embodiments, R^{7C} is independently substituted or unsubstituted 2 to 3 membered heteroalkyl. In embodiments, R^{7C} is independently unsubstituted 2 to 3 membered heteroalkyl.

In embodiments, R^{7C} is independently substituted or unsubstituted cycloalkyl (e.g., C₃-C₈, C₃-C₆, C₄-C₆, or C₅-C₆). In embodiments, R^{7C} is independently substituted or unsubstituted C₃-C₈ cycloalkyl. In embodiments, R^{7C} is independently unsubstituted C₃-C₈ cycloalkyl. In embodiments, R^{7C} is independently substituted or unsubstituted C₃-C₆ cycloalkyl. In embodiments, R^{7C} is independently unsubstituted C₃-C₆ cycloalkyl. In embodiments, R^{7C} is independently substituted or unsubstituted C₅-C₆ cycloalkyl. In embodiments, R^{7C} is independently unsubstituted C₅-C₆ cycloalkyl.

In embodiments, R^{7C} is independently substituted or unsubstituted heterocycloalkyl (e.g., 3 to 8 membered, 3 to 6 membered, 4 to 6 membered, 4 to 5 membered, or 5 to 6 membered). In embodiments, R^{7C} is independently substituted or unsubstituted 3 to 8 membered heterocycloalkyl. In embodiments, R^{7C} is independently unsubstituted 3 to 8 membered heterocycloalkyl. In embodiments, R^{7C} is independently substituted or unsubstituted 3 to 6 membered heterocycloalkyl. In embodiments, R^{7C} is independently unsubstituted 3 to 6 membered heterocycloalkyl. In embodiments, R^{7C} is independently substituted or unsubstituted 4 to 6 membered heterocycloalkyl. In embodiments, R^{7C} is independently unsubstituted 4 to 6 membered heterocycloalkyl. In embodiments, R^{7C} is independently substituted or unsubstituted 4 to 5 membered heterocycloalkyl. In embodiments, R^{7C} is independently unsubstituted 4 to 5 membered heterocycloalkyl. In embodiments, R^{7C} is independently substituted or unsubstituted 5 to 6 membered heterocycloalkyl. In embodiments, R^{7C} is independently unsubstituted 5 to 6 membered heterocycloalkyl.

In embodiments, R^{7C} is independently substituted or unsubstituted aryl (e.g., C₆-C₁₂, C₆-C₁₀, or phenyl). In embodiments, R^{7C} is independently substituted or unsubstituted C₆-C₁₂ aryl. In embodiments, R^{7C} is independently unsubstituted C₆-C₁₂ aryl. In embodiments, R^{7C} is independently substituted or unsubstituted C₆-C₁₀ aryl. In embodiments, R^{7C} is independently unsubstituted C₆-C₁₀ aryl. In embodiments, R^{7C} is independently substituted or unsubstituted phenyl. In embodiments, R^{7C} is independently unsubstituted phenyl.

In embodiments, R^{7C} is independently a substituted phenyl. In embodiments, R^{7C} is independently an R^{38C}-substituted phenyl. In embodiments, R^{38C} is independently a halogen. In embodiments, R^{38C} is independently -F. In embodiments, R^{38C} is independently -Cl. In embodiments, R^{38C} is independently -Br. In embodiments, R^{38C} is independently -I. In embodiments, R^{38C} is independently -O-(C₁-C₄ alkyl). In embodiments, R^{38C} is independently unsubstituted methoxy. In embodiments, R^{38C} is independently substituted or unsubstituted C₁-C₄ alkyl. In embodiments, R^{38C} is independently unsubstituted C₁-C₄ alkyl. In embodiments, R^{38C} is independently unsubstituted methyl. In embodiments, R^{38C} is independently unsubstituted ethyl. In embodiments, R^{38C} is independently unsubstituted propyl. In embodiments, R^{38C} is independently unsubstituted isopropyl. In embodiments, R^{38C} is independently unsubstituted butyl. In embodiments, R^{38C} is independently unsubstituted tert-butyl.

In embodiments, R^{7C} is independently substituted or unsubstituted heteroaryl (e.g., 5 to 12 membered, 5 to 10 membered, 5 to 9 membered, or 5 to 6 membered). In embodiments, R^{7C} is independently substituted or unsubstituted 5 to 12 membered heteroaryl. In embodiments, R^{7C} is independently unsubstituted 5 to 12 membered heteroaryl. In embodiments, R^{7C} is independently substituted or unsubstituted 5 to 10 membered heteroaryl. In embodiments, R^{7C} is independently unsubstituted 5 to 10 membered heteroaryl. In embodiments, R^{7C} is independently substituted or unsubstituted 5 to 9 membered heteroaryl. In embodiments, R^{7C} is independently unsubstituted 5 to 9 membered heteroaryl. In embodiments, R^{7C} is independently substituted or unsubstituted 5 to 6 membered heteroaryl. In embodiments, R^{7C} is independently unsubstituted 5 to 6 membered heteroaryl.

In embodiments, R^{7D} is independently substituted or unsubstituted alkyl (e.g., C₁-C₁₂, C₁-C₈, C₁-C₆, C₁-C₄, or C₁-C₂). In embodiments, R^{7D} is independently substituted or unsubstituted C₁-C₁₂ alkyl. In embodiments, R^{7D} is independently unsubstituted C₁-C₁₂ alkyl. In embodiments, R^{7D} is independently substituted or unsubstituted C₁-C₈ alkyl. In embodiments, R^{7D} is independently unsubstituted C₁-C₈ alkyl. In embodiments, R^{7D} is independently substituted or unsubstituted C₁-C₆ alkyl. In embodiments, R^{7D} is independently unsubstituted C₁-C₆ alkyl. In embodiments, R^{7D} is independently substituted or unsubstituted C₁-C₆ alkyl. In embodiments, R^{7D} is independently unsubstituted C₁-C₄ alkyl. In embodiments, R^{7D} is independently hydrogen. In embodiments, R^{7D} is independently substituted or unsubstituted C₁-C₆ alkyl. In embodiments, R^{7D} is independently unsubstituted C₁-C₆ alkyl. In embodiments, R^{7D} is independently substituted or unsubstituted C₁-C₄ alkyl. In embodiments, R^{7D} is independently unsubstituted C₁-C₄ alkyl. In embodiments, R^{7D} is independently substituted propyl. In embodiments, R^{7D} is independently unsubstituted propyl. In embodiments, R^{7D} is independently substituted isopropyl. In embodiments, R^{7D} is independently unsubstituted isopropyl. In embodiments, R^{7D} is independently substituted ethyl. In embodiments, R^{7D} is independently unsubstituted ethyl. In embodiments, R^{7D} is independently substituted methyl. In embodiments, R^{7D} is independently unsubstituted methyl. In embodiments, R^{7D} is independently substituted neopentyl. In embodiments, R^{7D} is independently unsubstituted neopentyl. In embodiments, R^{7D} is independently substituted pentyl. In embodiments, R^{7D} is independently unsubstituted pentyl. In embodiments, R^{7D} is independently unsubstituted isopentyl. In embodiments, R^{7D} is independently substituted isopentyl.

In embodiments, R^{7D} is independently substituted or unsubstituted heteroalkyl (e.g., 2 to 8 membered, 2 to 6 membered, 4 to 6 membered, 2 to 3 membered, or 4 to 5 membered). In embodiments, R^{7D} is independently substituted or unsubstituted 2 to 8 membered heteroalkyl. In embodiments, R^{7D} is independently unsubstituted 2 to 8 membered heteroalkyl. In embodiments, R^{7D} is independently substituted or unsubstituted 2 to 6 membered heteroalkyl. In embodiments, R^{7D} is independently unsubstituted 2 to 6 membered heteroalkyl. In embodiments, R^{7D} is independently substituted or unsubstituted 4 to 6 membered heteroalkyl. In embodiments, R^{7D} is independently unsubstituted 4 to 6 membered heteroalkyl. In embodiments, R^{7D} is independently substituted or unsubstituted 2 to 3 membered heteroalkyl. In embodiments, R^{7D} is independently unsubstituted 2 to 3 membered heteroalkyl.

In embodiments, R^{7D} is independently substituted or unsubstituted cycloalkyl (e.g., C₃-C₈, C₃-C₆, C₄-C₆, or C₅-C₆). In embodiments, R^{7D} is independently substituted or unsubstituted C₃-C₈ cycloalkyl. In embodiments, R^{7D} is independently unsubstituted C₃-C₈ cycloalkyl. In embodiments, R^{7D} is independently substituted or unsubstituted C₃-C₆ cycloalkyl. In embodiments, R^{7D} is independently unsubstituted C₃-C₆ cycloalkyl. In embodiments, R^{7D} is independently substituted or unsubstituted C₅-C₆ cycloalkyl. In embodiments, R^{7D} is independently unsubstituted C₅-C₆ cycloalkyl.

In embodiments, R^{7D} is independently substituted or unsubstituted heterocycloalkyl (e.g., 3 to 8 membered, 3 to 6 membered, 4 to 6 membered, 4 to 5 membered, or 5 to 6 membered). In embodiments, R^{7D} is independently substituted or unsubstituted 3 to 8 membered heterocycloalkyl. In embodiments, R^{7D} is independently unsubstituted 3 to 8 membered heterocycloalkyl. In embodiments, R^{7D} is independently substituted or unsubstituted 3 to 6 membered heterocycloalkyl. In embodiments, R^{7D} is independently unsubstituted 3 to 6 membered heterocycloalkyl. In embodiments, R^{7D} is independently substituted or unsubstituted 4 to 6 membered heterocycloalkyl. In embodiments, R^{7D} is independently unsubstituted 4 to 6 membered heterocycloalkyl. In embodiments, R^{7D} is independently substituted or unsubstituted 4 to 5 membered heterocycloalkyl. In embodiments, R^{7D} is independently unsubstituted 4 to 5 membered heterocycloalkyl. In embodiments, R^{7D} is independently substituted or unsubstituted 5 to 6 membered heterocycloalkyl. In embodiments, R^{7D} is independently unsubstituted 5 to 6 membered heterocycloalkyl.

In embodiments, R^{7D} is independently substituted or unsubstituted aryl (e.g., C₆-C₁₂, C₆-C₁₀, or phenyl). In embodiments, R^{7D} is independently substituted or unsubstituted C₆-C₁₂ aryl. In embodiments, R^{7D} is independently unsubstituted C₆-C₁₂ aryl. In embodiments, R^{7D} is independently substituted or unsubstituted C₆-C₁₀ aryl. In embodiments, R^{7D} is independently unsubstituted C₆-C₁₀ aryl. In embodiments, R^{7D} is independently substituted or unsubstituted phenyl. In embodiments, R^{7D} is independently unsubstituted phenyl.

In embodiments, R^{7D} is independently substituted or unsubstituted heteroaryl (e.g., 5 to 12 membered, 5 to 10 membered, 5 to 9 membered, or 5 to 6 membered). In embodiments, R^{7D} is independently substituted or unsubstituted 5 to 12 membered heteroaryl. In embodiments, R^{7D} is independently unsubstituted 5 to 12 membered heteroaryl. In embodiments, R^{7D} is independently substituted or unsubstituted 5 to 10 membered heteroaryl. In embodiments, R^{7D} is independently unsubstituted 5 to 10 membered heteroaryl. In embodiments, R^{7D} is independently substituted or unsubstituted 5 to 9 membered heteroaryl. In embodiments, R^{7D} is independently unsubstituted 5 to 9 membered heteroaryl. In embodiments, R^{7D} is independently substituted or unsubstituted 5 to 6 membered heteroaryl. In embodiments, R^{7D} is independently unsubstituted 5 to 6 membered heteroaryl.

In embodiments, the compound has the formula: 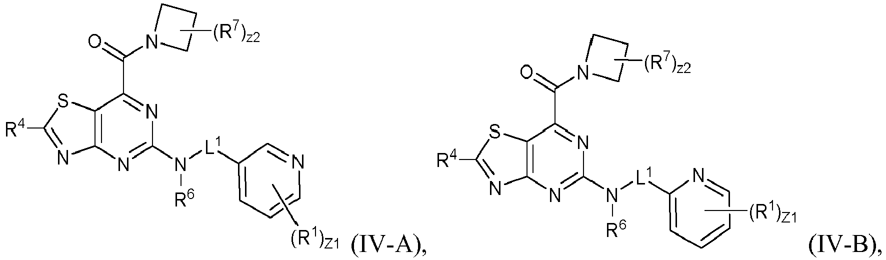 or 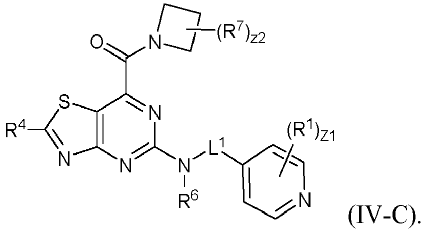 R¹, R⁴, R⁶, L¹, R⁷, z1, and z2 are as described herein, including embodiments.

In embodiments, the compound has the formula: 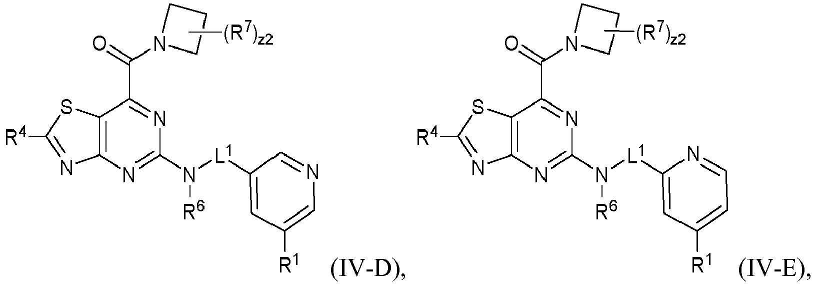 or 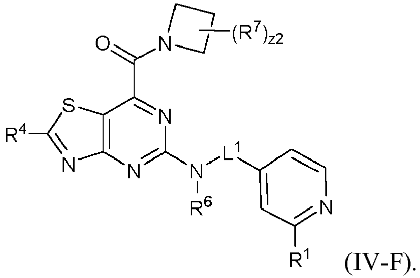 R¹, R⁴, R⁶, L¹, R⁷, and z2 are as described herein, including embodiments.

In embodiments, the compound has the formula: 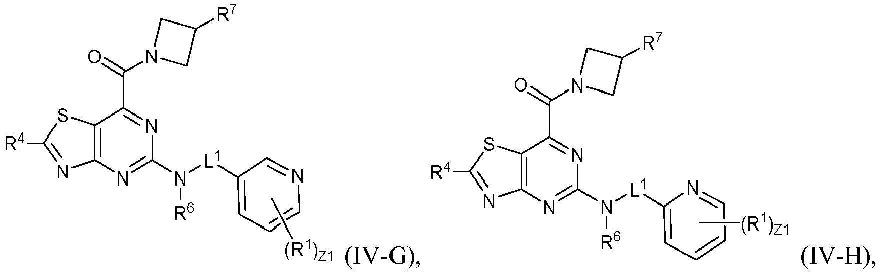 or 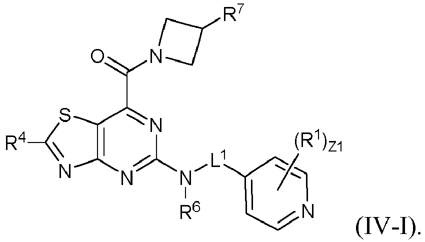 R¹, R⁴, R⁶, L¹, R⁷, and z1 are as described herein, including embodiments.

In embodiments, the compound has the formula: 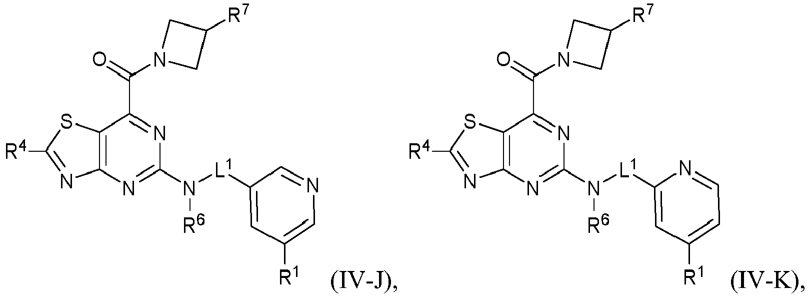 or 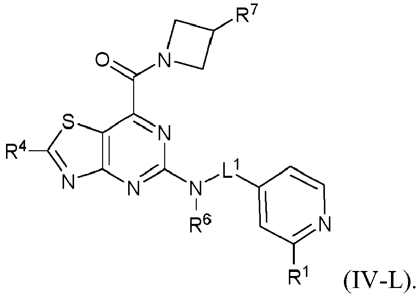 R¹, R⁴, R⁶, L¹, and R⁷ are as described herein, including embodiments.

In embodiments, z2 is 0 to 6. In embodiments, z2 is 0. In embodiments, z2 is 1. In embodiments, z2 is 2. In embodiments, z2 is 3. In embodiments, z2 is 4. In embodiments, z2 is 5. In embodiments, z2 is 6.

In embodiments, R⁷ is independently -NR^{7A}R^{7B} (e.g., -NH₂, or -NHCH₃). In embodiments, R⁷ is independently -C(O)NR^{7A}R^{7B} (e.g., -C(O)NH₂, -C(O)NHCH₃, or -C(O)N(CH₃)₂). In embodiments, R⁷ is independently -NR^{7A}C(O)R^{7C} (e.g., -NHC(O)H, -NCH₃C(O)H, -NCH₃C(O)CH₃, or -NHC(O)CH₃). In embodiments, R⁷ is independently -NR^{7A}C(O)NR^{7A}R^{7B} (e.g., -NHC(O)NH₂, -NCH₃C(O)NH₂, or -NHC(O)NHCH₃). In embodiments, R⁷ is independently -OR^{7D} (e.g., -OH, or -OCH₃). In embodiments, R⁷ is independently -C(O)R^{7C} (e.g., -C(O)H or -C(O)CH₃). Each R^{7A}, R^{7B}, R^{7C}, and R^{7D} is independently as describe above. In embodiments, R^{7A} and R^{7B} are independently hydrogen, or unsubstituted C₁-C₄ alkyl. In embodiments, R^{7C} is independently unsubstituted C₁-C₆ alkyl. In embodiments, R^{7D} is independently hydrogen, or unsubstituted C₁-C₄ alkyl.

In embodiments, R⁷ is independently substituted or unsubstituted alkyl (e.g., C₁-C₁₂, C₁-C₈, C₁-C₆, C₁-C₄, or C₁-C₂). In embodiments, R⁷ is independently substituted or unsubstituted C₁-C₁₂ alkyl. In embodiments, R⁷ is independently unsubstituted C₁-C₁₂ alkyl. In embodiments, R⁷ is independently substituted or unsubstituted C₁-C₈ alkyl. In embodiments, R⁷ is unsubstituted C₁-C₈ alkyl. In embodiments, R⁷ is independently substituted or unsubstituted C₁-C₆ alkyl. In embodiments, R⁷ is independently unsubstituted C₁-C₆ alkyl. In embodiments, R⁷ is independently substituted or unsubstituted C₁-C₆ alkyl. In embodiments, R⁷ is independently unsubstituted C₁-C₄ alkyl. In embodiments, R⁷ is independently substituted or unsubstituted C₁-C₆ alkyl. In embodiments, R⁷ is independently unsubstituted C₁-C₆ alkyl. In embodiments, R⁷ is independently substituted or unsubstituted C₁-C₄ alkyl. In embodiments, R⁷ is independently unsubstituted C₁-C₄ alkyl. In embodiments, R⁷ is independently substituted propyl. In embodiments, R⁷ is independently unsubstituted propyl. In embodiments, R⁷ is independently substituted isopropyl. In embodiments, R⁷ is independently unsubstituted isopropyl. In embodiments, R⁷ is independently substituted ethyl. In embodiments, R⁷ is independently hydroxy-substituted ethyl. In embodiments, R⁷ is independently unsubstituted ethyl. In embodiments, R⁷ is independently substituted methyl. In embodiments, R⁷ is independently hydroxy-substituted methyl. In embodiments, R⁷ is independently unsubstituted methyl.

In embodiments, R⁷ is independently substituted or unsubstituted 2 to 8 membered heteroalkyl. In embodiments, R⁷ is independently unsubstituted 2 to 8 membered heteroalkyl. In embodiments, R⁷ is independently substituted or unsubstituted 2 to 6 membered heteroalkyl. In embodiments, R⁷ is independently unsubstituted 2 to 6 membered heteroalkyl. In embodiments, R⁷ is independently substituted or unsubstituted 4 to 6 membered heteroalkyl. In embodiments, R⁷ is independently unsubstituted 4 to 6 membered heteroalkyl. In embodiments, R⁷ is independently substituted or unsubstituted 2 to 3 membered heteroalkyl. In embodiments, R⁷ is independently unsubstituted 2 to 3 membered heteroalkyl.

In embodiments, R⁷ is independently 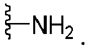 In embodiments, R⁷ is independently 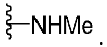 In embodiments, R⁷ is independently 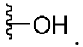 In embodiments, R⁷ is independently 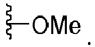 In embodiments, R⁷ is independently 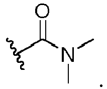 In embodiments, R⁷ is independently 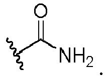 In embodiments, R⁷ is independently 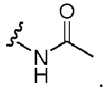 In embodiments, R⁷ is independently 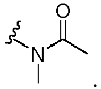 In embodiments, R⁷ is independently 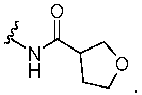 In embodiments, R⁷ is independently 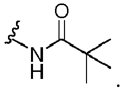 In embodiments, R⁷ is independently 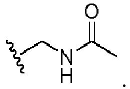 In embodiments, R⁷ is independently 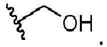 In embodiments, R⁷ is independently 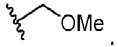 In embodiments, R⁷ is independently 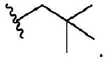 In embodiments, R⁷ is independently 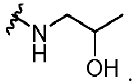 In embodiments, R⁷ is independently

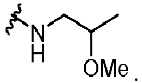

In embodiments, R⁷ is independently -NR^{7A}C(O)R^{7C}. In embodiments, R⁷ is independently -NHC(O)R^{7C}. In embodiments, R^{7C} is independently substituted or unsubstituted C₁-C₄ alkyl. In embodiments, R^{7C} is independently substituted or unsubstituted C₃-C₈ cycloalkyl. In embodiments, R^{7C} is independently substituted C₃-C₈ cycloalkyl. In embodiments, R^{7C} is independently unsubstituted C₃-C₈ cycloalkyl. In embodiments, R^{7C} is independently substituted or unsubstituted 5 to 6 membered heterocycloalkyl. In embodiments, R^{7C} is independently substituted 5 to 6 membered heterocycloalkyl. In embodiments, R^{7C} is independently unsubstituted 5 to 6 membered heterocycloalkyl. In embodiments, R^{7C} is independently substituted or unsubstituted 6 membered heterocycloalkyl. In embodiments, R^{7C} is independently substituted 6 membered heterocycloalkyl. In embodiments, R^{7C} is independently unsubstituted 6 membered heterocycloalkyl. In embodiments, R^{7C} is independently substituted or unsubstituted 5 membered heterocycloalkyl. In embodiments, R^{7C} is independently substituted 5 membered heterocycloalkyl. In embodiments, R^{7C} is independently unsubstituted 5 membered heterocycloalkyl. In embodiments, R^{7C} is independently substituted or unsubstituted tetrahydrofuranyl. In embodiments, R^{7C} is independently substituted tetrahydrofuranyl. In embodiments, R^{7C} is independently unsubstituted tetrahydrofuranyl. In embodiments, R^{7C} is independently substituted or unsubstituted phenyl. In embodiments, R^{7C} is independently substituted phenyl. In embodiments, R^{7C} is independently unsubstituted phenyl. In embodiments, R^{7C} is independently substituted or unsubstituted 5 to 6 membered heteroaryl. In embodiments, R^{7C} is independently substituted 5 to 6 membered heteroaryl. In embodiments, R^{7C} is independently unsubstituted 5 to 6 membered heteroaryl. In embodiments, R^{7C} is independently substituted or unsubstituted 6 membered heteroaryl. In embodiments, R^{7C} is independently substituted 6 membered heteroaryl. In embodiments, R^{7C} is independently unsubstituted 6 membered heteroaryl. In embodiments, R^{7C} is independently substituted or unsubstituted 5 membered heteroaryl. In embodiments, R^{7C} is independently substituted 5 membered heteroaryl. In embodiments, R^{7C} is independently unsubstituted 5 membered heteroaryl.

In embodiments, R^{7C} is independently substituted C₁-C₄ alkyl. In embodiments, R^{7C} is independently unsubstituted C₁-C₄ alkyl. In embodiments, R^{7C} is independently C₁-C₄ haloalkyl. In embodiments, R^{7C} is independently hydroxyl-substituted C₁-C₄ alkyl. In embodiments, R^{7C} is independently alkoxyl-substituted C₁-C₄ alkyl. In embodiments, R^{7C} is independently C₁-C₃ alkoxyl-substituted C₁-C₄ alkyl. In embodiments, R^{7C} is independently methoxyl-substituted C₁-C₄ alkyl. In embodiments, R^{7C} is independently substituted 5 to 6 membered heterocycloalkyl-substituted C₁-C₄ alkyl. In embodiments, R^{7C} is independently unsubstituted 5 to 6 membered heterocycloalkyl-substituted C₁-C₄ alkyl. In embodiments, R^{7C} is independently substituted 5 membered heterocycloalkyl-substituted C₁-C₄ alkyl. In embodiments, R^{7C} is independently unsubstituted 5 membered heterocycloalkyl-substituted C₁-C₄ alkyl. In embodiments, R^{7C} is independently substituted 6 membered heterocycloalkyl-substituted C₁-C₄ alkyl. In embodiments, R^{7C} is independently unsubstituted 6 membered heterocycloalkyl-substituted C₁-C₄ alkyl. In embodiments, R^{7C} is independently substituted morpholinyl-substituted C₁-C₄ alkyl. In embodiments, R^{7C} is independently unsubstituted morpholinyl-substituted C₁-C₄ alkyl. In embodiments, R^{7C} is independently substituted morpholinyl-substituted ethyl. In embodiments, R^{7C} is independently unsubstituted morpholinyl-substituted ethyl. In embodiments, R^{7C} is independently substituted morpholinyl-substituted methyl. In embodiments, R^{7C} is independently unsubstituted morpholinyl-substituted methyl.

In embodiments, R^{7C} is independently substituted C₃-C₈ cycloalkyl. In embodiments, R^{7C} is independently unsubstituted C₃-C₈ cycloalkyl. In embodiments, R^{7C} is independently substituted cyclopropyl. In embodiments, R^{7C} is independently unsubstituted cyclopropyl. In embodiments, R^{7C} is independently substuted or unsubstituted C₁-C₃ alkyl-substituted cyclopropyl. In embodiments, R^{7C} is independently unsubstituted C₁-C₃ alkyl-substituted cyclopropyl. In embodiments, R^{7C} is independently methyl-substituted cyclopropyl. In embodiments, R^{7C} is independently ethyl-substituted cyclopropyl. In embodiments, R^{7C} is independently C₁-C₃ haloalkyl-substituted cyclopropyl. In embodiments, R^{7C} is independently CF₃-substituted cyclopropyl. In embodiments, R^{7C} is independently CN-substituted cyclopropyl. In embodiments, R^{7C} is independently hydroxyl-substituted cyclopropyl. In embodiments, R^{7C} is independently alkoxy-substituted cyclopropyl. In embodiments, R^{7C} is independently C₁-C₃ alkoxy-substituted cyclopropyl. In embodiments, R^{7C} is independently methoxy-substituted cyclopropyl. In embodiments, R^{7C} is independently substituted cyclobutyl. In embodiments, R^{7C} is independently unsubstituted cyclobutyl. In embodiments, R^{7C} is independently substuted or unsubstituted C₁-C₃ alkyl-substituted cyclobutyl. In embodiments, R^{7C} is independently unsubstituted C₁-C₃ alkyl-substituted cyclobutyl. In embodiments, R^{7C} is independently methyl-substituted cyclobutyl. In embodiments, R^{7C} is independently dimethyl-substituted cyclobutyl. In embodiments, R^{7C} is independently ethyl-substituted cyclobutyl. In embodiments, R^{7C} is independently C₁-C₃ haloalkyl-substituted cyclobutyl. In embodiments, R^{7C} is independently CF₃-substituted cyclobutyl. In embodiments, R^{7C} is independently CN-substituted cyclobutyl. In embodiments, R^{7C} is independently hydroxyl-substituted cyclobutyl. In embodiments, R^{7C} is independently C₁-C₃ alkoxy-substituted cyclobutyl. In embodiments, R^{7C} is independently methoxy-substituted cyclobutyl. In embodiments, R^{7C} is independently substituted cyclopentyl. In embodiments, R^{7C} is independently unsubstituted cyclopentyl. In embodiments, R^{7C} is independently substuted or unsubstituted C₁-C₃ alkyl-substituted cyclopentyl. In embodiments, R^{7C} is independently unsubstituted C₁-C₃ alkyl-substituted cyclopentyl. In embodiments, R^{7C} is independently methyl-substituted cyclopentyl. In embodiments, R^{7C} is independently dimethyl-substituted cyclopentyl. In embodiments, R^{7C} is independently ethyl-substituted cyclopentyl. In embodiments, R^{7C} is independently C₁-C₃ haloalkyl-substituted cyclopentyl. In embodiments, R^{7C} is independently CF₃-substituted cyclopentyl. In embodiments, R^{7C} is independently CN-substituted cyclopentyl. In embodiments, R^{7C} is independently hydroxyl-substituted cyclopentyl. In embodiments, R^{7C} is independently C₁-C₃ alkoxy-substituted cyclopentyl. In embodiments, R^{7C} is independently methoxy-substituted cyclopentyl. In embodiments, R^{7C} is independently substituted bicyclo[1.1.1]pentyl. In embodiments, R^{7C} is independently unsubstituted bicyclo[1.1.1]pentyl. In embodiments, R^{7C} is independently substuted or unsubstituted C₁-C₃ alkyl-substituted bicyclo[1.1.1]pentyl. In embodiments, R^{7C} is independently unsubstituted C₁-C₃ alkyl-substituted bicyclo[1.1.1]pentyl. In embodiments, R^{7C} is independently methyl-substituted bicyclo[1.1.1]pentyl. In embodiments, R^{7C} is independently dimethyl-substituted bicyclo[1.1.1]pentyl. In embodiments, R^{7C} is independently ethyl-substituted bicyclo[1.1.1]pentyl. In embodiments, R^{7C} is independently C₁-C₃ haloalkyl-substituted bicyclo[1.1.1]pentyl. In embodiments, R^{7C} is independently CF₃-substituted bicyclo[1.1.1]pentyl. In embodiments, R^{7C} is independently CN-substituted bicyclo[1.1.1]pentyl. In embodiments, R^{7C} is independently hydroxyl-substituted bicyclo[1.1.1]pentyl. In embodiments, R^{7C} is independently C₁-C₃ alkoxy-substituted bicyclo[1.1.1]pentyl. In embodiments, R^{7C} is independently methoxy-substituted bicyclo[1.1.1]pentyl. In embodiments, R^{7C} is independently substituted cyclohexyl. In embodiments, R^{7C} is independently unsubstituted cyclohexyl. In embodiments, R^{7C} is independently substuted or unsubstituted C₁-C₃ alkyl-substituted cyclohexyl. In embodiments, R^{7C} is independently unsubstituted C₁-C₃ alkyl-substituted cyclohexyl. In embodiments, R^{7C} is independently methyl-substituted cyclohexyl. In embodiments, R^{7C} is independently dimethyl-substituted cyclohexyl. In embodiments, R^{7C} is independently ethyl-substituted cyclohexyl. In embodiments, R^{7C} is independently C₁-C₃ haloalkyl-substituted cyclohexyl. In embodiments, R^{7C} is independently CF₃-substituted cyclohexyl. In embodiments, R^{7C} is independently CN-substituted cyclohexyl. In embodiments, R^{7C} is independently hydroxyl-substituted cyclohexyl. In embodiments, R^{7C} is independently C₁-C₃ alkoxy-substituted cyclohexyl. In embodiments, R^{7C} is independently methoxy-substituted cyclohexyl. In embodiments, R^{7C} is independently substituted cycloheptyl. In embodiments, R^{7C} is independently unsubstituted cycloheptyl. In embodiments, R^{7C} is independently substuted or unsubstituted C₁-C₃ alkyl-substituted cycloheptyl. In embodiments, R^{7C} is independently unsubstituted C₁-C₃ alkyl-substituted cycloheptyl. In embodiments, R^{7C} is independently methyl-substituted cycloheptyl. In embodiments, R^{7C} is independently dimethyl-substituted cycloheptyl. In embodiments, R^{7C} is independently ethyl-substituted cycloheptyl. In embodiments, R^{7C} is independently C₁-C₃ haloalkyl-substituted cycloheptyl. In embodiments, R^{7C} is independently CF₃-substituted cycloheptyl. In embodiments, R^{7C} is independently CN-substituted cycloheptyl. In embodiments, R^{7C} is independently hydroxyl-substituted cycloheptyl. In embodiments, R^{7C} is independently C₁-C₃ alkoxy-substituted cycloheptyl. In embodiments, R^{7C} is independently methoxy-substituted cycloheptyl. In embodiments, R^{7C} is independently substituted bicyclo[2.2.2]octyl. In embodiments, R^{7C} is independently unsubstituted bicyclo[2.2.2]octyl. In embodiments, R^{7C} is independently substuted or unsubstituted C₁-C₃ alkyl-substituted bicyclo[2.2.2]octyl. In embodiments, R^{7C} is independently unsubstituted C₁-C₃ alkyl-substituted bicyclo[2.2.2]octyl. In embodiments, R^{7C} is independently methyl-substituted bicyclo[2.2.2]octyl. In embodiments, R^{7C} is independently dimethyl-substituted bicyclo[2.2.2]octyl. In embodiments, R^{7C} is independently ethyl-substituted bicyclo[2.2.2]octyl. In embodiments, R^{7C} is independently C₁-C₃ haloalkyl-substituted bicyclo[2.2.2]octyl. In embodiments, R^{7C} is independently CF₃-substituted bicyclo[2.2.2]octyl. In embodiments, R^{7C} is independently CN-substituted bicyclo[2.2.2]octyl. In embodiments, R^{7C} is independently hydroxyl-substituted bicyclo[2.2.2]octyl. In embodiments, R^{7C} is independently C₁-C₃ alkoxy-substituted bicyclo[2.2.2]octyl. In embodiments, R^{7C} is independently methoxy-substituted bicyclo[2.2.2]octyl.

In embodiments, R^{7C} is independently substituted 4 to 6 membered heterocycloalkyl. In embodiments, R^{7C} is independently unsubstituted 4 to 6 membered heterocycloalkyl. In embodiments, R^{7C} is independently substituted 4 membered heterocycloalkyl. In embodiments, R^{7C} is independently unsubstituted 4 membered heterocycloalkyl. In embodiments, R^{7C} is independently substituted 5 membered heterocycloalkyl. In embodiments, R^{7C} is independently unsubstituted 5 membered heterocycloalkyl. In embodiments, R^{7C} is independently substituted 6 membered heterocycloalkyl. In embodiments, R^{7C} is independently unsubstituted 6 membered heterocycloalkyl. In embodiments, R^{7C} is independently substituted 4 to 6 membered heterocycloalkyl. In embodiments, R^{7C} is independently substituted tetrahydrofuranyl. In embodiments, R^{7C} is independently unsubstituted tetrahydrofuranyl. In embodiments, R^{7C} is independently substuted or unsubstituted C₁-C₃ alkyl-substituted tetrahydrofuranyl. In embodiments, R^{7C} is independently unsubstituted C₁-C₃ alkyl-substituted tetrahydrofuranyl. In embodiments, R^{7C} is independently methyl-substituted tetrahydrofuranyl. In embodiments, R^{7C} is independently ethyl-substituted tetrahydrofuranyl. In embodiments, R^{7C} is independently C₁-C₃ haloalkyl-substituted tetrahydrofuranyl. In embodiments, R^{7C} is independently CF₃-substituted tetrahydrofuranyl. In embodiments, R^{7C} is independently CN-substituted tetrahydrofuranyl. In embodiments, R^{7C} is independently hydroxyl-substituted tetrahydrofuranyl. In embodiments, R^{7C} is independently alkoxy-substituted tetrahydrofuranyl. In embodiments, R^{7C} is independently C₁-C₃ alkoxy-substituted tetrahydrofuranyl. In embodiments, R^{7C} is independently methoxy-substituted tetrahydrofuranyl. In embodiments, R^{7C} is independently substituted tetrahydropyranyl. In embodiments, R^{7C} is independently unsubstituted tetrahydropyranyl. In embodiments, R^{7C} is independently substuted or unsubstituted C₁-C₃ alkyl-substituted tetrahydropyranyl. In embodiments, R^{7C} is independently unsubstituted C₁-C₃ alkyl-substituted tetrahydropyranyl. In embodiments, R^{7C} is independently methyl-substituted tetrahydropyranyl. In embodiments, R^{7C} is independently ethyl-substituted tetrahydropyranyl. In embodiments, R^{7C} is independently C₁-C₃ haloalkyl-substituted tetrahydropyranyl. In embodiments, R^{7C} is independently CF₃-substituted tetrahydropyranyl. In embodiments, R^{7C} is independently CN-substituted tetrahydropyranyl. In embodiments, R^{7C} is independently hydroxyl-substituted tetrahydropyranyl. In embodiments, R^{7C} is independently alkoxy-substituted tetrahydropyranyl. In embodiments, R^{7C} is independently C₁-C₃ alkoxy-substituted tetrahydropyranyl. In embodiments, R^{7C} is independently methoxy-substituted tetrahydropyranyl. In embodiments, R^{7C} is independently substituted morpholinyl. In embodiments, R^{7C} is independently unsubstituted morpholinyl. In embodiments, R^{7C} is independently substituted or unsubstituted C₁-C₃ alkyl-substituted morpholinyl. In embodiments, R^{7C} is independently unsubstituted C₁-C₃ alkyl-substituted morpholinyl. In embodiments, R^{7C} is independently methyl-substituted morpholinyl. In embodiments, R^{7C} is independently ethyl-substituted morpholinyl. In embodiments, R^{7C} is independently C₁-C₃ haloalkyl-substituted morpholinyl. In embodiments, R^{7C} is independently CF₃-substituted morpholinyl. In embodiments, R^{7C} is independently CN-substituted morpholinyl. In embodiments, R^{7C} is independently hydroxyl-substituted morpholinyl. In embodiments, R^{7C} is independently alkoxy-substituted morpholinyl. In embodiments, R^{7C} is independently C₁-C₃ alkoxy-substituted morpholinyl. In embodiments, R^{7C} is independently methoxy-substituted morpholinyl.

In embodiments, R^{7C} is independently substituted C₆ or C₁₀ aryl. In embodiments, R^{7C} is independently unsubstituted C₆ or C₁₀ aryl. In embodiments, R^{7C} is independently substituted phenyl. In embodiments, R^{7C} is independently unsubstituted phenyl. In embodiments, R^{7C} is independently substituted phenyl. In embodiments, R^{7C} is independently unsubstituted phenyl. In embodiments, R^{7C} is independently halogen-substituted phenyl. In embodiments, R^{7C} is independently substituted or unsubstituted C₁-C₃ alkyl-substituted phenyl. In embodiments, R^{7C} is independently unsubstituted C₁-C₃ alkyl-substituted phenyl. In embodiments, R^{7C} is independently methyl-substituted phenyl. In embodiments, R^{7C} is independently ethyl-substituted phenyl. In embodiments, R^{7C} is independently C₁-C₃ haloalkyl-substituted phenyl. In embodiments, R^{7C} is independently CF₃-substituted phenyl. In embodiments, R^{7C} is independently CN-substituted phenyl. In embodiments, R^{7C} is independently hydroxyl-substituted phenyl. In embodiments, R^{7C} is independently alkoxy-substituted phenyl. In embodiments, R^{7C} is independently C₁-C₃ alkoxy-substituted phenyl. In embodiments, R^{7C} is independently methoxy-substituted phenyl.

In embodiments, R^{7C} is independently substituted 5 to 6 membered heteroaryl. In embodiments, R^{7C} is independently unsubstituted 5 to 6 membered heteroaryl. In embodiments, R^{7C} is independently substituted 5 membered heteroaryl. In embodiments, R^{7C} is independently unsubstituted 5 membered heteroaryl. In embodiments, R^{7C} is independently substituted 6 membered heteroaryl. In embodiments, R^{7C} is independently unsubstituted 6 membered heteroaryl. In embodiments, R^{7C} is independently substituted thiazolyl. In embodiments, R^{7C} is independently unsubstituted thiazolyl. In embodiments, R^{7C} is independently halogen-substituted thiazolyl. In embodiments, R^{7C} is independently substuted or unsubstituted C₁-C₃ alkyl-substituted thiazolyl. In embodiments, R^{7C} is independently unsubstituted C₁-C₃ alkyl-substituted thiazolyl. In embodiments, R^{7C} is independently methyl-substituted thiazolyl. In embodiments, R^{7C} is independently ethyl-substituted thiazolyl. In embodiments, R^{7C} is independently C₁-C₃ haloalkyl-substituted thiazolyl. In embodiments, R^{7C} is independently CF₃-substituted thiazolyl. In embodiments, R^{7C} is independently CN-substituted thiazolyl. In embodiments, R^{7C} is independently hydroxyl-substituted thiazolyl. In embodiments, R^{7C} is independently alkoxy-substituted thiazolyl. In embodiments, R^{7C} is independently C₁-C₃ alkoxy-substituted thiazolyl. In embodiments, R^{7C} is independently methoxy-substituted thiazolyl. In embodiments, R^{7C} is independently substituted oxazolyl. In embodiments, R^{7C} is independently unsubstituted oxazolyl. In embodiments, R^{7C} is independently halogen-substituted oxazolyl. In embodiments, R^{7C} is independently substuted or unsubstituted C₁-C₃ alkyl-substituted oxazolyl. In embodiments, R^{7C} is independently unsubstituted C₁-C₃ alkyl-substituted oxazolyl. In embodiments, R^{7C} is independently methyl-substituted oxazolyl. In embodiments, R^{7C} is independently ethyl-substituted oxazolyl. In embodiments, R^{7C} is independently C₁-C₃ haloalkyl-substituted oxazolyl. In embodiments, R^{7C} is independently CF₃-substituted oxazolyl. In embodiments, R^{7C} is independently CN-substituted oxazolyl. In embodiments, R^{7C} is independently hydroxyl-substituted oxazolyl. In embodiments, R^{7C} is independently alkoxy-substituted oxazolyl. In embodiments, R^{7C} is independently C₁-C₃ alkoxy-substituted oxazolyl. In embodiments, R^{7C} is independently methoxy-substituted oxazolyl. In embodiments, R^{7C} is independently substituted 2-pyridyl. In embodiments, R^{7C} is independently unsubstituted 2-pyridyl. In embodiments, R^{7C} is independently substituted 3-pyridyl. In embodiments, R^{7C} is independently unsubstituted 3-pyridyl. In embodiments, R^{7C} is independently substituted 4-pyridyl. In embodiments, R^{7C} is independently unsubstituted 5-pyridyl. In embodiments, R^{7C} is independently substituted pyridyl. In embodiments, R^{7C} is independently unsubstituted pyridyl. In embodiments, R^{7C} is independently halogen-substituted pyridyl. In embodiments, R^{7C} is independently substuted or unsubstituted C₁-C₃ alkyl-substituted pyridyl. In embodiments, R^{7C} is independently unsubstituted C₁-C₃ alkyl-substituted pyridyl. In embodiments, R^{7C} is independently methyl-substituted pyridyl. In embodiments, R^{7C} is independently ethyl-substituted pyridyl. In embodiments, R^{7C} is independently C₁-C₃ haloalkyl-substituted pyridyl. In embodiments, R^{7C} is independently CF₃-substituted pyridyl. In embodiments, R^{7C} is independently CN-substituted pyridyl. In embodiments, R^{7C} is independently hydroxyl-substituted pyridyl. In embodiments, R^{7C} is independently alkoxy-substituted pyridyl. In embodiments, R^{7C} is independently C₁-C₃ alkoxy-substituted pyridyl. In embodiments, R^{7C} is independently methoxy-substituted pyridyl.

In embodiments, R^{7C} is independently 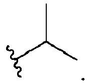 In embodiments, R^{7C} is independently 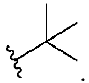 In embodiments, R^{7C} is independently 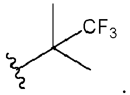 In embodiments, R^{7C} is independently 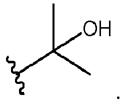 In embodiments, R^{7C} is independently 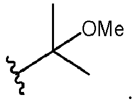 In embodiments, R^{7C} is independently 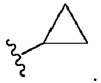 In embodiments, R^{7C} is independently 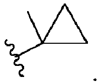 In embodiments, R^{7C} is independently 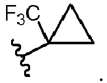 In embodiments, R^{7C} is independently 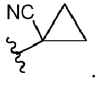 In embodiments, R^{7C} is independently 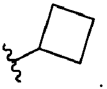 In embodiments, R^{7C} is independently 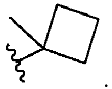 In embodiments, R^{7C} is independently 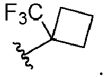 In embodiments, R^{7C} is independently 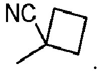 In embodiments, R^{7C} is independently 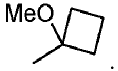 In embodiments, R^{7C} is independently 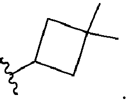 In embodiments, R^{7C} is independently 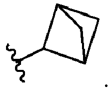 In embodiments, R^{7C} is independently 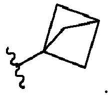 In embodiments, R^{7C} is independently 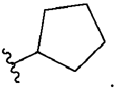 In embodiments, R^{7C} is independently 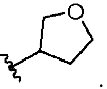 In embodiments, R^{7C} is independently 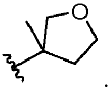 In embodiments, R^{7C} is independently 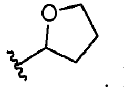 In embodiments, R^{7C} is independently 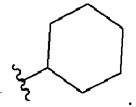 In embodiments, R^{7C} is independently 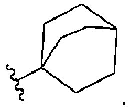 In embodiments, R^{7C} is independently 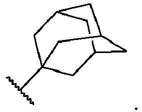 In embodiments, R^{7C} is independently 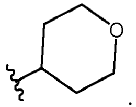 In embodiments, R^{7C} is independently 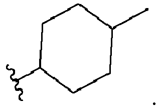 In embodiments, R^{7C} is independently 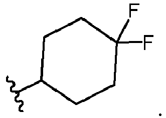 In embodiments, R^{7C} is independently 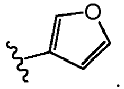 In embodiments, R^{7C} is independently 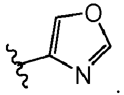 In embodiments, R^{7C} is independently 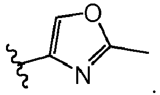 In embodiments, R^{7C} is independently 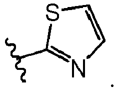 In embodiments, R^{7C} is independently 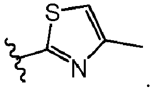 In embodiments, R^{7C} is independently 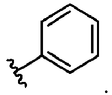 In embodiments, R^{7C} is independently 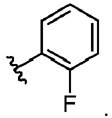 In embodiments, R^{7C} is independently 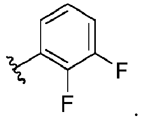 In embodiments, R^{7C} is independently 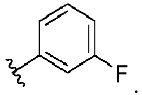 In embodiments, R^{7C} is independently 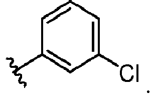 In embodiments, R^{7C} is independently 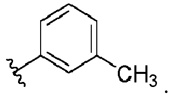 In embodiments, R^{7C} is independently 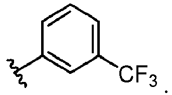 In embodiments, R^{7C} is independently 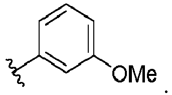 In embodiments, R^{7C} is independently 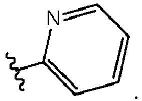 In embodiments, R^{7C} is independently 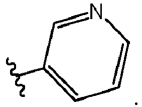 In embodiments, R^{7C} is independently 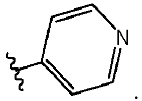 In embodiments, R^{7C} is independently 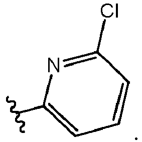 In embodiments, R^{7C} is independently 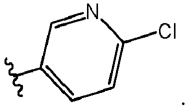 In embodiments, R^{7C} is independently 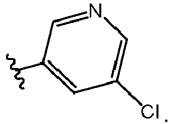 In embodiments, R^{7C} is independently 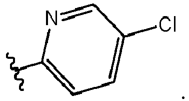 In embodiments, R^{7C} is independently 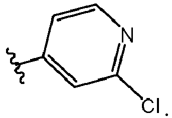 In embodiments, R^{7C} is independently 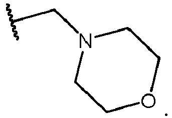 In embodiments, R^{7C} is independently 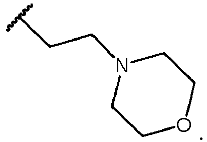 In embodiments, R^{7C} is independently

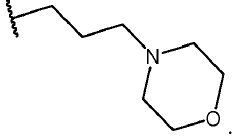

In embodiments, R⁷ is independently: 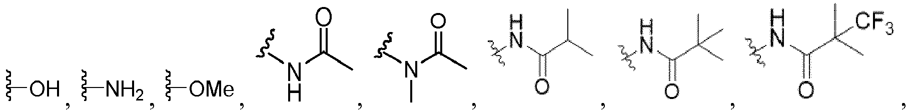 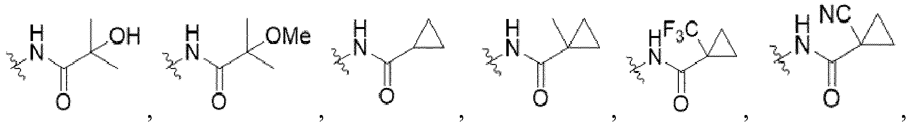 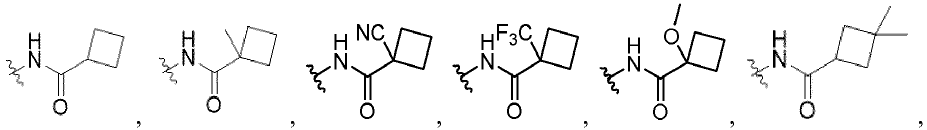 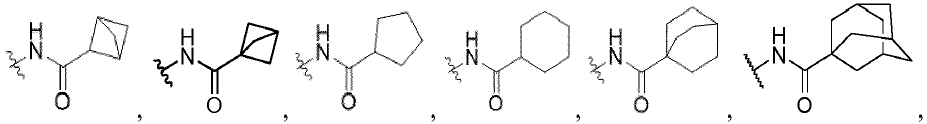 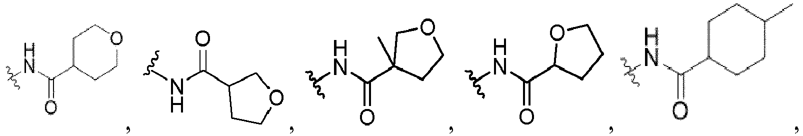 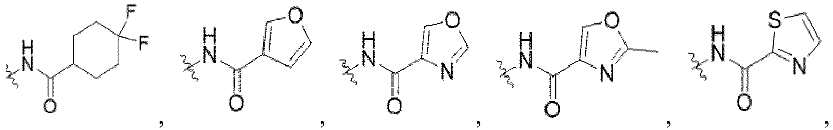 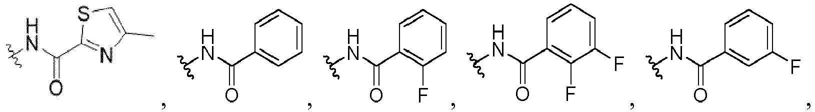 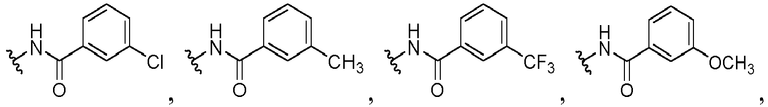 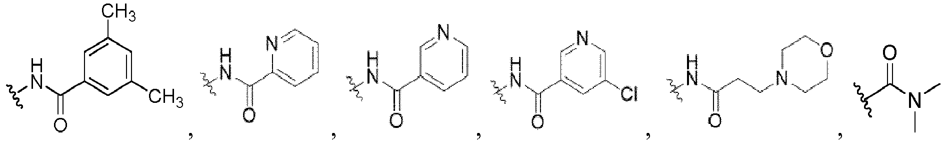 , or

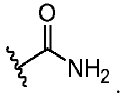

In embodiments, R⁷ is independently:

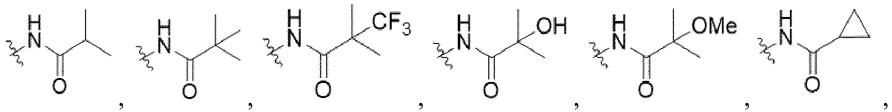

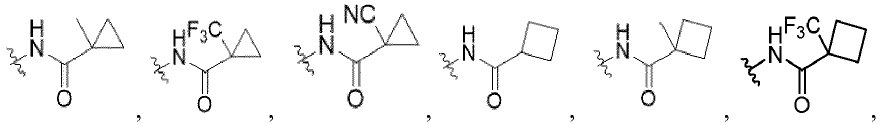

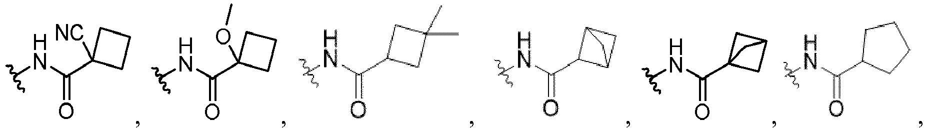

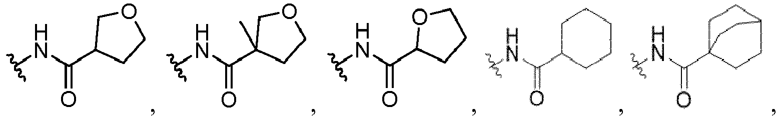

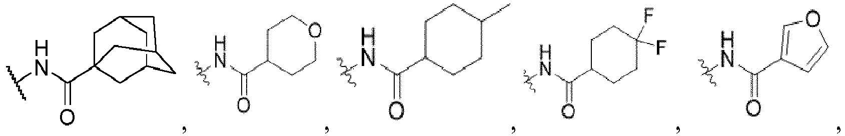

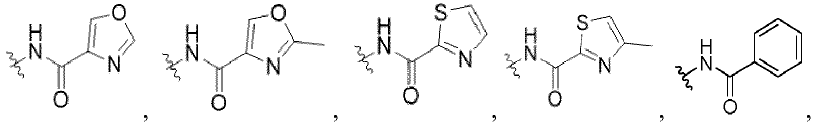

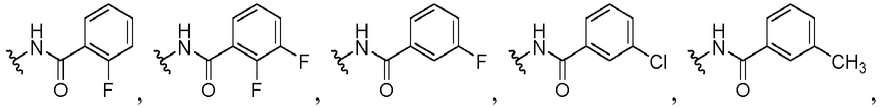

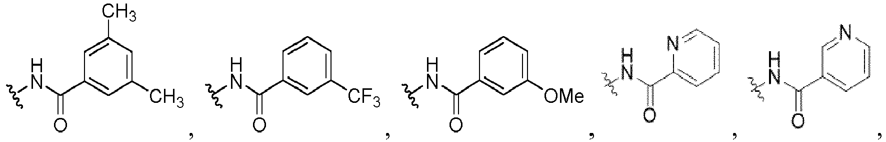

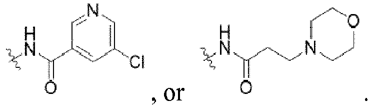

In embodiments, R⁷ is independently:

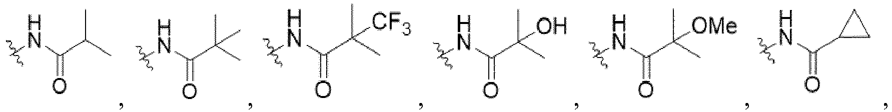

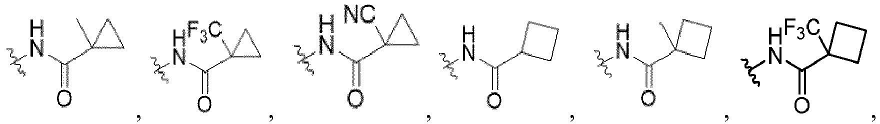

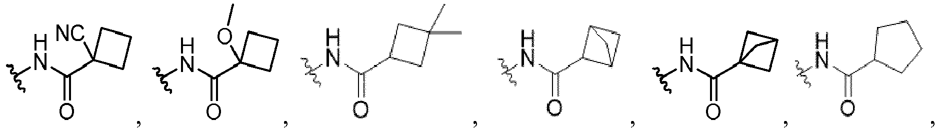

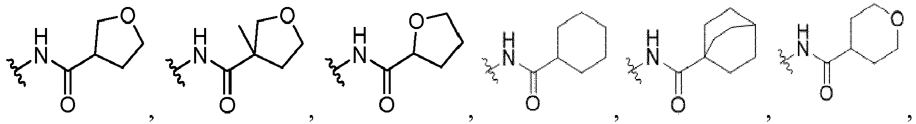

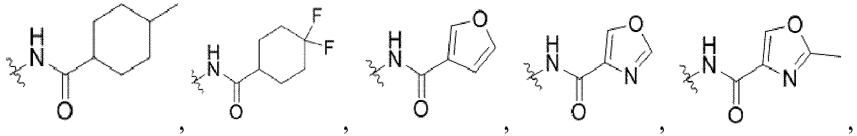

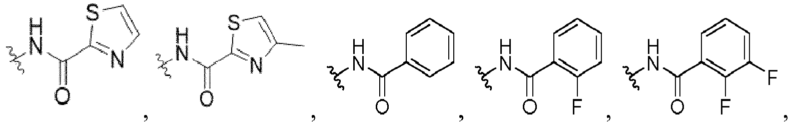

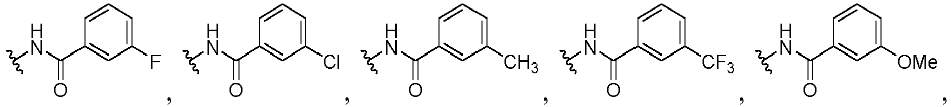

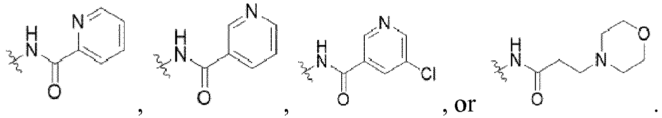

In embodiments, R⁷ is independently -NR^{7A}C(O)NR^{7A}R^{7B}. In embodiments, R^{7A} and R^{7B} are independently hydrogen, substituted or unsubstituted alkyl (e.g., C₁-C₁₂, C₁-C₈, C₁-C₆, C₁-C₄, or C₁-C₂), substituted or unsubstituted heteroalkyl (e.g., 2 to 8 membered, 2 to 6 membered, 4 to 6 membered, 2 to 3 membered, or 4 to 5 membered), substituted or unsubstituted cycloalkyl (e.g., C₃-C₈, C₃-C₆, C₄-C₆, or C₅-C₆), substituted or unsubstituted heterocycloalkyl (e.g., 3 to 8 membered, 3 to 6 membered, 4 to 6 membered, 4 to 5 membered, or 5 to 6 membered), substituted or unsubstituted aryl (e.g., C₆-C₁₂, C₆-C₁₀, or phenyl) or substituted or unsubstituted heteroaryl (e.g., 5 to 12 membered, 5 to 10 membered, 5 to 9 membered, or 5 to 6 membered). In embodiments, R^{7A} is independently hydrogen. In embodiments, R^{7B} is independently hydrogen.

In embodiments, R⁷ is independently -NHC(O)NR^{7A}R^{7B}. In embodiments, R⁷ is independently -NHC(O)NHR^{7B}. In embodiments, R⁷ is independently -NR^{7A}C(O)NHR^{7B}.

In embodiments, R^{7A} is independently substituted or unsubstituted alkyl (e.g., C₁-C₁₂, C₁-C₈, C₁-C₆, C₁-C₄, or C₁-C₂). In embodiments, R^{7A} is independently substituted or unsubstituted C₁-C₁₂ alkyl. In embodiments, R^{7A} is independently unsubstituted C₁-C₁₂ alkyl. In embodiments, R^{7A} is independently substituted or unsubstituted C₁-C₈ alkyl. In embodiments, R^{7A} is independently unsubstituted C₁-C₈ alkyl. In embodiments, R^{7A} is independently substituted or unsubstituted C₁-C₆ alkyl. In embodiments, R^{7A} is independently unsubstituted C₁-C₆ alkyl. In embodiments, R^{7A} is independently substituted or unsubstituted C₁-C₄ alkyl. In embodiments, R^{7A} is independently unsubstituted C₁-C₄ alkyl.

In embodiments, R^{7A} is independently substituted or unsubstituted heteroalkyl (e.g., 2 to 8 membered, 2 to 6 membered, 4 to 6 membered, 2 to 3 membered, or 4 to 5 membered). In embodiments, R^{7A} is independently substituted or unsubstituted 2 to 8 membered heteroalkyl. In embodiments, R^{7A} is independently unsubstituted 2 to 8 membered heteroalkyl. In embodiments, R^{7A} is independently substituted or unsubstituted 2 to 6 membered heteroalkyl. In embodiments, R^{7A} is independently unsubstituted 2 to 6 membered heteroalkyl. In embodiments, R^{7A} is independently substituted or unsubstituted 4 to 6 membered heteroalkyl. In embodiments, R^{7A} is independently unsubstituted 4 to 6 membered heteroalkyl. In embodiments, R^{7A} is independently substituted or unsubstituted 2 to 3 membered heteroalkyl. In embodiments, R^{7A} is independently unsubstituted 2 to 3 membered heteroalkyl.

In embodiments, R^{7A} is independently substituted or unsubstituted cycloalkyl (e.g., C₃-C₈, C₃-C₆, C₄-C₆, or C₅-C₆). In embodiments, R^{7A} is independently substituted or unsubstituted C₃-C₈ cycloalkyl. In embodiments, R^{7A} is independently unsubstituted C₃-C₈ cycloalkyl. In embodiments, R^{7A} is independently substituted or unsubstituted C₃-C₆ cycloalkyl. In embodiments, R^{7A} is independently unsubstituted C₃-C₆ cycloalkyl. In embodiments, R^{7A} is independently substituted or unsubstituted C₅-C₆ cycloalkyl. In embodiments, R^{7A} is independently unsubstituted C₅-C₆ cycloalkyl.

In embodiments, R^{7A} is independently substituted or unsubstituted heterocycloalkyl (e.g., 3 to 8 membered, 3 to 6 membered, 4 to 6 membered, 4 to 5 membered, or 5 to 6 membered). In embodiments, R^{7A} is independently substituted or unsubstituted 3 to 8 membered heterocycloalkyl. In embodiments, R^{7A} is independently unsubstituted 3 to 8 membered heterocycloalkyl. In embodiments, R^{7A} is independently substituted or unsubstituted 3 to 6 membered heterocycloalkyl. In embodiments, R^{7A} is independently unsubstituted 3 to 6 membered heterocycloalkyl. In embodiments, R^{7A} is independently substituted or unsubstituted 4 to 6 membered heterocycloalkyl. In embodiments, R^{7A} is independently unsubstituted 4 to 6 membered heterocycloalkyl. In embodiments, R^{7A} is independently substituted or unsubstituted 4 to 5 membered heterocycloalkyl. In embodiments, R^{7A} is independently unsubstituted 4 to 5 membered heterocycloalkyl. In embodiments, R^{7A} is independently substituted or unsubstituted 5 to 6 membered heterocycloalkyl. In embodiments, R^{7A} is independently unsubstituted 5 to 6 membered heterocycloalkyl.

In embodiments, R^{7A} is independently substituted or unsubstituted aryl (e.g., C₆-C₁₂, C₆-C₁₀, or phenyl). In embodiments, R^{7A} is independently substituted or unsubstituted C₆-C₁₂ aryl. In embodiments, R^{7A} is independently unsubstituted C₆-C₁₂ aryl. In embodiments, R^{7A} is independently substituted or unsubstituted C₆-C₁₀ aryl. In embodiments, R^{7A} is independently unsubstituted C₆-C₁₀ aryl. In embodiments, R^{7A} is independently substituted or unsubstituted phenyl. In embodiments, R^{7A} is independently unsubstituted phenyl.

In embodiments, R^{7A} is independently substituted or unsubstituted heteroaryl (e.g., 5 to 12 membered, 5 to 10 membered, 5 to 9 membered, or 5 to 6 membered). In embodiments, R^{7A} is independently substituted or unsubstituted 5 to 12 membered heteroaryl. In embodiments, R^{7A} is independently unsubstituted 5 to 12 membered heteroaryl. In embodiments, R^{7A} is independently substituted or unsubstituted 5 to 10 membered heteroaryl. In embodiments, R^{7A} is independently unsubstituted 5 to 10 membered heteroaryl. In embodiments, R^{7A} is independently substituted or unsubstituted 5 to 9 membered heteroaryl. In embodiments, R^{7A} is independently unsubstituted 5 to 9 membered heteroaryl. In embodiments, R^{7A} is independently substituted or unsubstituted 5 to 6 membered heteroaryl. In embodiments, R^{7A} is independently unsubstituted 5 to 6 membered heteroaryl.

In embodiments, R^{7A} is independently substituted C₁-C₄ alkyl. In embodiments, R^{7A} is independently unsubstituted C₁-C₄ alkyl. In embodiments, R^{7A} is independently substituted or unsubstituted methyl. In embodiments, R^{7A} is independently unsubstituted methyl. In embodiments, R^{7A} is independently substituted or unsubstituted ethyl. In embodiments, R^{7A} is independently unsubstituted ethyl. In embodiments, R^{7A} is independently substituted or unsubstituted propryl. In embodiments, R^{7A} is independently unsubstituted propyl. In embodiments, R^{7A} is independently substituted or unsubstituted butyl. In embodiments, R^{7A} is independently unsubstituted butyl. In embodiments, R^{7A} is independently substituted or unsubstituted t-butyl. In embodiments, R^{7A} is independently unsubstituted t-butyl. In embodiments, R^{7A} is independently C₁-C₄ haloalkyl. In embodiments, R^{7A} is independently substituted C₃-C₈ cycloalkyl. In embodiments, R^{7A} is independently unsubstituted C₃-C₈ cycloalkyl. In embodiments, R^{7A} is independently substituted cyclopropyl. In embodiments, R^{7A} is independently unsubstituted cyclopropyl. In embodiments, R^{7A} is independently substituted cyclobutyl. In embodiments, R^{7A} is independently unsubstituted cyclobutyl. In embodiments, R^{7A} is independently substituted cyclopentyl. In embodiments, R^{7A} is independently unsubstituted cyclopentyl. In embodiments, R^{7A} is independently substituted cyclohexyl. In embodiments, R^{7A} is independently unsubstituted cyclohexyl. In embodiments, R^{7A} is independently substituted cycloheptyl. In embodiments, R^{7A} is independently unsubstituted cycloheptyl. In embodiments, R^{7A} is independently substituted bicyclo[2.2.2]octyl. In embodiments, R^{7A} is independently unsubstituted bicyclo[2.2.2]octyl. In embodiments, R^{7A} is independently substituted bicyclo[1.1.1]pentyl. In embodiments, R^{7A} is independently unsubstituted bicyclo[1.1.1]pentyl. In embodiments, R^{7A} is independently substituted 2-pyridyl. In embodiments, R^{7A} is independently unsubstituted 2-pyridyl. In embodiments, R^{7A} is independently substituted 3-pyridyl. In embodiments, R^{7A} is independently unsubstituted 3-pyridyl. In embodiments, R^{7A} is independently substituted 4-pyridyl. In embodiments, R^{7A} is independently unsubstituted 5-pyridyl. In embodiments, R^{7A} is independently substituted morpholinyl. In embodiments, R^{7A} is independently unsubstituted morpholinyl.

In embodiments, R^{7B} is independently substituted or unsubstituted alkyl (e.g., C₁-C₁₂, C₁-C₈, C₁-C₆, C₁-C₄, or C₁-C₂). In embodiments, R^{7B} is independently substituted or unsubstituted C₁-C₁₂ alkyl. In embodiments, R^{7B} is independently unsubstituted C₁-C₁₂ alkyl. In embodiments, R^{7B} is independently substituted or unsubstituted C₁-C₈ alkyl. In embodiments, R^{7B} is independently unsubstituted C₁-C₈ alkyl. In embodiments, R^{7B} is independently substituted or unsubstituted C₁-C₆ alkyl. In embodiments, R^{7B} is independently unsubstituted C₁-C₆ alkyl. In embodiments, R^{7B} is independently substituted or unsubstituted C₁-C₄ alkyl. In embodiments, R^{7B} is independently unsubstituted C₁-C₄ alkyl.

In embodiments, R^{7B} is independently substituted or unsubstituted heteroalkyl (e.g., 2 to 8 membered, 2 to 6 membered, 4 to 6 membered, 2 to 3 membered, or 4 to 5 membered). In embodiments, R^{7B} is independently substituted or unsubstituted 2 to 8 membered heteroalkyl. In embodiments, R^{7B} is independently unsubstituted 2 to 8 membered heteroalkyl. In embodiments, R^{7B} is independently substituted or unsubstituted 2 to 6 membered heteroalkyl. In embodiments, R^{7B} is independently unsubstituted 2 to 6 membered heteroalkyl. In embodiments, R^{7B} is independently substituted or unsubstituted 4 to 6 membered heteroalkyl. In embodiments, R^{7B} is independently unsubstituted 4 to 6 membered heteroalkyl. In embodiments, R^{7B} is independently substituted or unsubstituted 2 to 3 membered heteroalkyl. In embodiments, R^{7B} is independently unsubstituted 2 to 3 membered heteroalkyl.

In embodiments, R^{7B} is independently substituted or unsubstituted cycloalkyl (e.g., C₃-C₈, C₃-C₆, C₄-C₆, or C₅-C₆). In embodiments, R^{7B} is independently substituted or unsubstituted C₃-C₈ cycloalkyl. In embodiments, R^{7B} is independently unsubstituted C₃-C₈ cycloalkyl. In embodiments, R^{7B} is independently substituted or unsubstituted C₃-C₆ cycloalkyl. In embodiments, R^{7B} is independently unsubstituted C₃-C₆ cycloalkyl. In embodiments, R^{7B} is independently substituted or unsubstituted C₅-C₆ cycloalkyl. In embodiments, R^{7B} is independently unsubstituted C₅-C₆ cycloalkyl.

In embodiments, R^{7B} is independently substituted or unsubstituted heterocycloalkyl (e.g., 3 to 8 membered, 3 to 6 membered, 4 to 6 membered, 4 to 5 membered, or 5 to 6 membered). In embodiments, R^{7B} is independently substituted or unsubstituted 3 to 8 membered heterocycloalkyl. In embodiments, R^{7B} is independently unsubstituted 3 to 8 membered heterocycloalkyl. In embodiments, R^{7B} is independently substituted or unsubstituted 3 to 6 membered heterocycloalkyl. In embodiments, R^{7B} is independently unsubstituted 3 to 6 membered heterocycloalkyl. In embodiments, R^{7B} is independently substituted or unsubstituted 4 to 6 membered heterocycloalkyl. In embodiments, R^{7B} is independently unsubstituted 4 to 6 membered heterocycloalkyl. In embodiments, R^{7B} is independently substituted or unsubstituted 4 to 5 membered heterocycloalkyl. In embodiments, R^{7B} is independently unsubstituted 4 to 5 membered heterocycloalkyl. In embodiments, R^{7B} is independently substituted or unsubstituted 5 to 6 membered heterocycloalkyl. In embodiments, R^{7B} is independently unsubstituted 5 to 6 membered heterocycloalkyl.

In embodiments, R^{7B} is independently substituted or unsubstituted aryl (e.g., C₆-C₁₂, C₆-C₁₀, or phenyl). In embodiments, R^{7B} is independently substituted or unsubstituted C₆-C₁₂ aryl. In embodiments, R^{7B} is independently unsubstituted C₆-C₁₂ aryl. In embodiments, R^{7B} is independently substituted or unsubstituted C₆-C₁₀ aryl. In embodiments, R^{7B} is independently unsubstituted C₆-C₁₀ aryl. In embodiments, R^{7B} is independently substituted or unsubstituted phenyl. In embodiments, R^{7B} is independently unsubstituted phenyl.

In embodiments, R^{7B} is independently substituted or unsubstituted heteroaryl (e.g., 5 to 12 membered, 5 to 10 membered, 5 to 9 membered, or 5 to 6 membered). In embodiments, R^{7B} is independently substituted or unsubstituted 5 to 12 membered heteroaryl. In embodiments, R^{7B} is independently unsubstituted 5 to 12 membered heteroaryl. In embodiments, R^{7B} is independently substituted or unsubstituted 5 to 10 membered heteroaryl. In embodiments, R^{7B} is independently unsubstituted 5 to 10 membered heteroaryl. In embodiments, R^{7B} is independently substituted or unsubstituted 5 to 9 membered heteroaryl. In embodiments, R^{7B} is independently unsubstituted 5 to 9 membered heteroaryl. In embodiments, R^{7B} is independently substituted or unsubstituted 5 to 6 membered heteroaryl. In embodiments, R^{7B} is independently unsubstituted 5 to 6 membered heteroaryl.

In embodiments, R^{7B} is independently substituted C₁-C₄ alkyl. In embodiments, R^{7B} is independently unsubstituted C₁-C₄ alkyl. In embodiments, R^{7B} is independently substituted or unsubstituted methyl. In embodiments, R^{7B} is independently unsubstituted methyl. In embodiments, R^{7B} is independently substituted or unsubstituted ethyl. In embodiments, R^{7B} is independently unsubstituted ethyl. In embodiments, R^{7B} is independently substituted or unsubstituted propyl. In embodiments, R^{7B} is independently unsubstituted propyl. In embodiments, R^{7B} is independently substituted or unsubstituted butyl. In embodiments, R^{7B} is independently unsubstituted butyl. In embodiments, R^{7B} is independently substituted or unsubstituted t-butyl. In embodiments, R^{7B} is independently unsubstituted t-butyl. In embodiments, R^{7B} is independently C₁-C₄ haloalkyl. In embodiments, R^{7B} is independently substituted C₃-C₈ cycloalkyl. In embodiments, R^{7B} is independently unsubstituted C₃-C₈ cycloalkyl. In embodiments, R^{7B} is independently substituted cyclopropyl. In embodiments, R^{7B} is independently unsubstituted cyclopropyl. In embodiments, R^{7B} is independently substituted cyclobutyl. In embodiments, R^{7B} is independently unsubstituted cyclobutyl. In embodiments, R^{7B} is independently substituted cyclopentyl. In embodiments, R^{7B} is independently unsubstituted cyclopentyl. In embodiments, R^{7B} is independently substituted cyclohexyl. In embodiments, R^{7B} is independently unsubstituted cyclohexyl. In embodiments, R^{7B} is independently substituted cycloheptyl. In embodiments, R^{7B} is independently unsubstituted cycloheptyl. In embodiments, R^{7B} is independently substituted bicyclo[2.2.2]octyl. In embodiments, R^{7B} is independently unsubstituted bicyclo[2.2.2]octyl. In embodiments, R^{7B} is independently substituted bicyclo[1.1.1]pentyl. In embodiments, R^{7B} is independently unsubstituted bicyclo[1.1.1]pentyl. In embodiments, R^{7B} is independently substituted 2-pyridyl. In embodiments, R^{7B} is independently unsubstituted 2-pyridyl. In embodiments, R^{7B} is independently substituted 3-pyridyl. In embodiments, R^{7B} is independently unsubstituted 3-pyridyl. In embodiments, R^{7B} is independently substituted 4-pyridyl. In embodiments, R^{7B} is independently unsubstituted 5-pyridyl. In embodiments, R^{7B} is independently substituted morpholinyl. In embodiments, R^{7B} is independently unsubstituted morpholinyl.

In embodiments, R⁷ is:

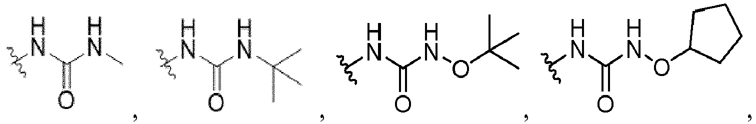

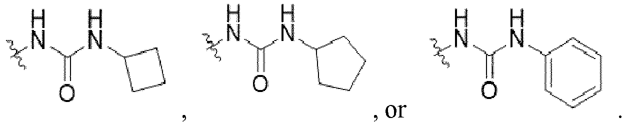

In embodiments, the compound has a formula: 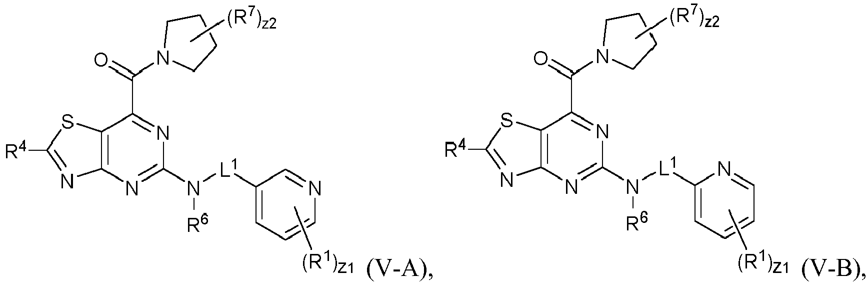 or 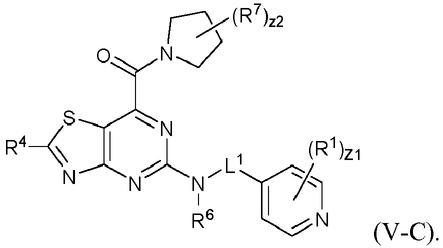 R¹, R⁴, R⁶, R⁷, L¹, z1, and z2 are as described herein, including embodiments.

In embodiments, the compound has a formula: 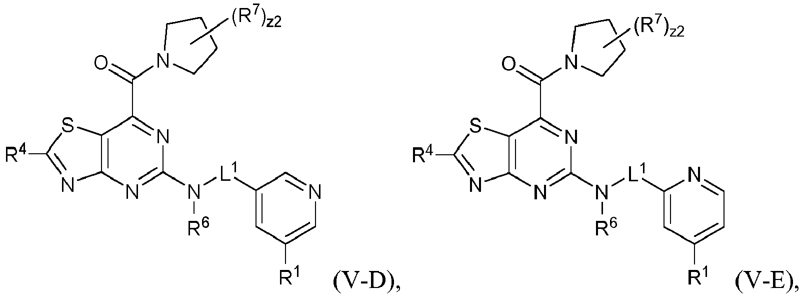 or 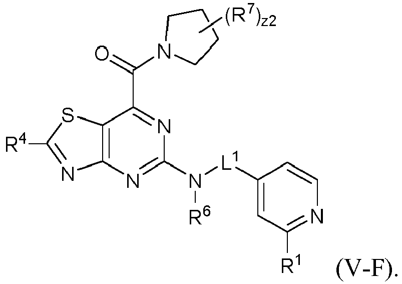 R¹, R⁴, R⁶, R⁷, L¹, and z2 are as described herein, including embodiments.

In embodiments, the compound has a formula: 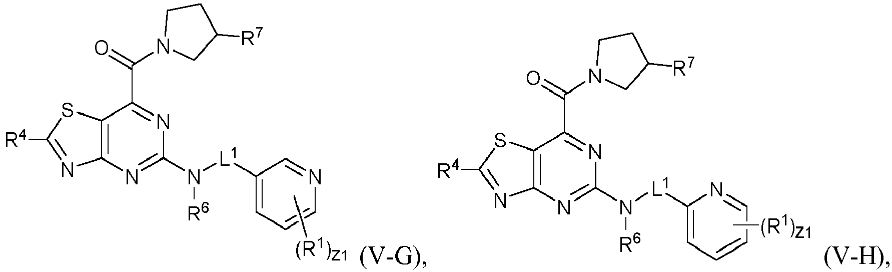 or 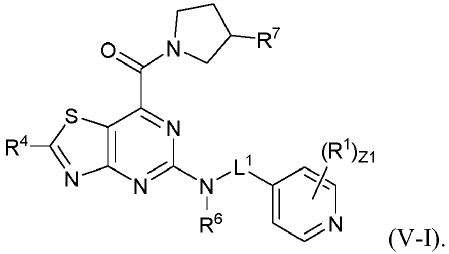 R¹, R⁴, R⁶, R⁷, L¹, and z1 are as described herein, including embodiments.

In embodiments, the compound has a formula: 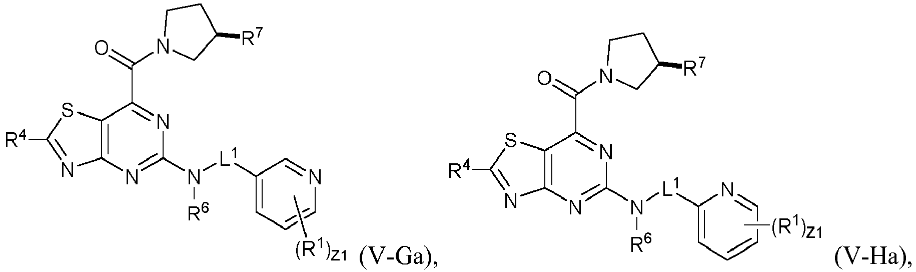 or 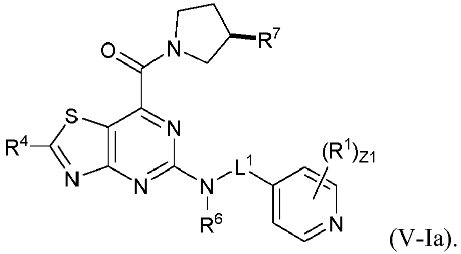 R¹, R⁴, R⁶, R⁷, L¹, and z1 are as described herein, including embodiments.

In embodiments, the compound has a formula: 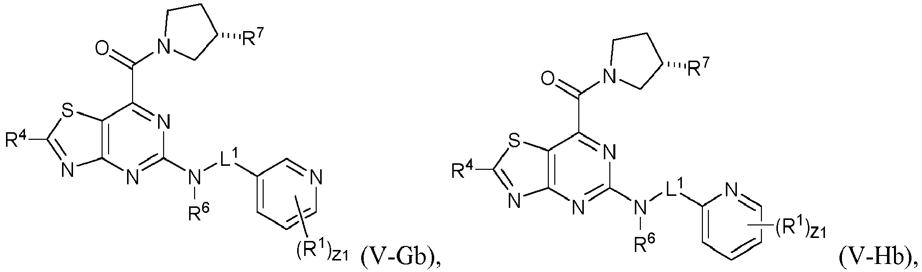 or 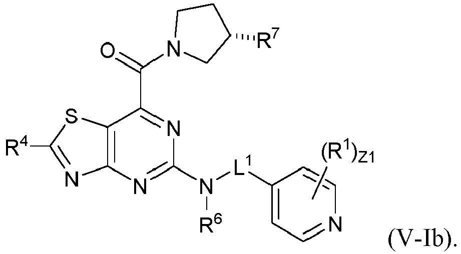 R¹, R⁴, R⁶, R⁷, L¹, and z1 are as described herein, including embodiments.

In embodiments, the compound has a formula: 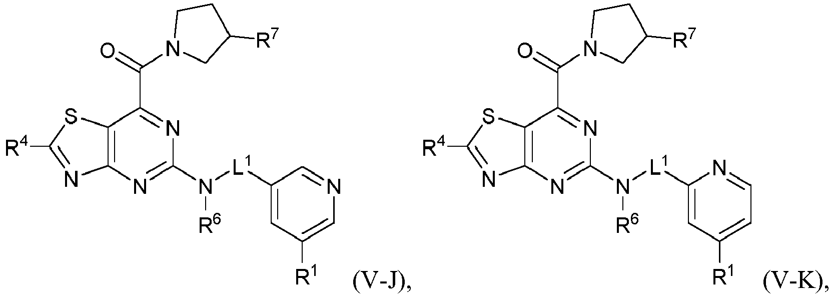 or 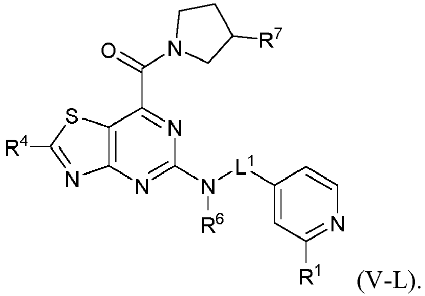 R¹, R⁴, R⁶, R⁷, and L¹ are as described herein, including embodiments.

In embodiments, the compound has a formula: 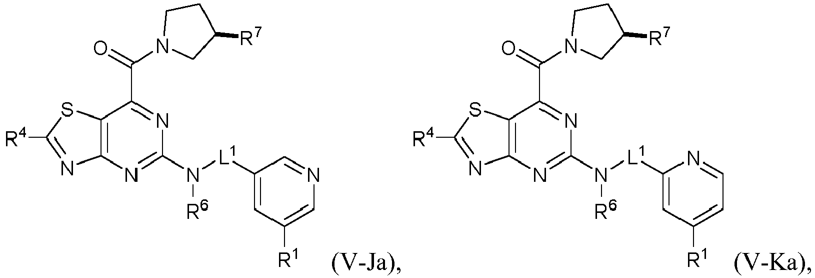 or 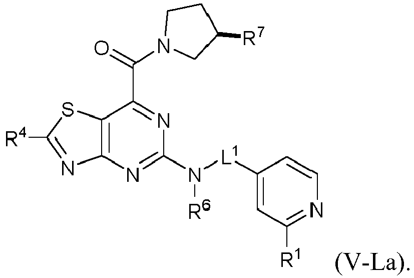 R¹, R⁴, R⁶, R⁷, and L¹ are as described herein, including embodiments.

In embodiments, the compound has a formula: 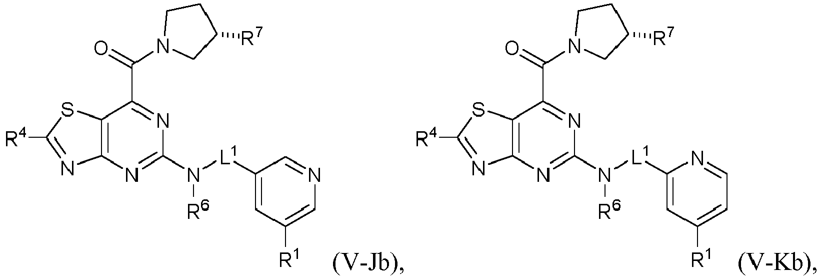 or 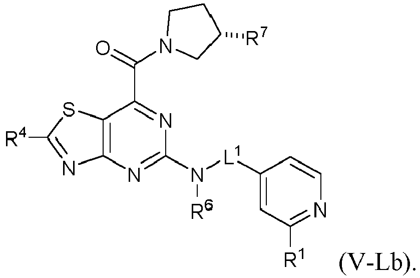 R¹, R⁴, R⁶, R⁷, and L¹ are as described herein, including embodiments.

In embodiments, z2 is an integer from 0 to 8. In embodiments, z2 is 0. In embodiments, z2 is 1. In embodiments, z2 is 2. In embodiments, z2 is 3. In embodiments, z2 is 4. In embodiments, z2 is 5. In embodiments, z2 is 6. In embodiments, z2 is 7. In embodiments, z2 is 8.

In embodiments, z2 is 0 or 1. In embodiments, R⁷ is independently -NR^{7A}R^{7B} (e.g., -NH₂, or -NHCH₃). In embodiments, R⁷ is independently -C(O)NR^{7A}R^{7B} (e.g., -C(O)NH₂, -C(O)NHCH₃ or -C(O)N(CH₃)₂). In embodiments, R⁷ is independently -NR^{7A}C(O)R^{7C} (e.g., -NHC(O)H, -NCH₃C(O)H, -NCH₃C(O)CH₃, or -NHC(O)CH₃). In embodiments, R⁷ is independently -NR^{7A}C(O)NR^{7A}R^{7B} (e.g., -NHC(O)NH₂, -NCH₃C(O)NH₂, or -NHC(O)NHCH₃). In embodiments, R⁷ is independently-OR^{7D} (e.g., -OH, or -OCH₃). In embodiments, R⁷ is independently -C(O)R^{7C} (e.g., -C(O)H or -C(O)CH₃). Each R^{7A}, R^{7B}, R^{7C}, and R^{7D} is as described herein. In embodiments, R^{7A} and R^{7B} are independently hydrogen, or unsubstituted C₁-C₄ alkyl. In embodiments, R^{7C} is independently unsubstituted C₁-C₆ alkyl. In embodiments, R^{7D} is independently hydrogen, or unsubstituted C₁-C₄ alkyl.

In embodiments, R⁷ is independently substituted or unsubstituted alkyl (e.g., C₁-C₁₂, C₁-C₈, C₁-C₆, C₁-C₄, or C₁-C₂). In embodiments, R⁷ is independently substituted or unsubstituted C₁-C₁₂ alkyl. In embodiments, R⁷ is independently unsubstituted C₁-C₁₂ alkyl. In embodiments, R⁷ is independently substituted or unsubstituted C₁-C₈ alkyl. In embodiments, R⁷ is independently unsubstituted C₁-C₈ alkyl. In embodiments, R⁷ is independently substituted or unsubstituted C₁-C₆ alkyl. In embodiments, R⁷ is independently unsubstituted C₁-C₆ alkyl. In embodiments, R⁷ is independently substituted or unsubstituted C₁-C₄ alkyl. In embodiments, R⁷ is independently unsubstituted C₁-C₄ alkyl. In embodiments, R⁷ is independently substituted methyl. In embodiments, R⁷ is independently hydroxyl-substituted methyl. In embodiments, R⁷ is independently alkoxy-substituted methyl. In embodiments, R⁷ is independently methoxy-substituted methyl. In embodiments, R⁷ is independently N-acetyl-substituted methyl.

In embodiments, R⁷ is independently substituted or unsubstituted 2 to 8 membered heteroalkyl. In embodiments, R⁷ is independently unsubstituted 2 to 8 membered heteroalkyl. In embodiments, R⁷ is independently substituted or unsubstituted 2 to 6 membered heteroalkyl. In embodiments, R⁷ is independently unsubstituted 2 to 6 membered heteroalkyl. In embodiments, R⁷ is independently substituted or unsubstituted 4 to 6 membered heteroalkyl. In embodiments, R⁷ is independently unsubstituted 4 to 6 membered heteroalkyl. In embodiments, R⁷ is independently substituted or unsubstituted 2 to 3 membered heteroalkyl. In embodiments, R⁷ is independently unsubstituted 2 to 3 membered heteroalkyl.

In embodiments, R⁷ is independently -NH₂. In embodiments, R⁷ is independently 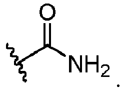 In embodiments, R⁷ is independently 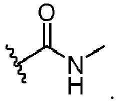 In embodiments, R⁷ is independently 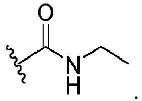 In embodiments, R⁷ is independently 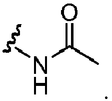 In embodiments, R⁷ is independently 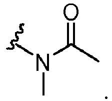 In embodiments, R⁷ is independently 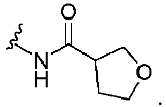 In embodiments, R⁷ is independently 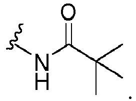 In embodiments, R⁷ is independently 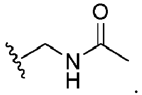 In embodiments, R⁷ is independently 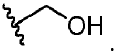 In embodiments, R⁷ is independently 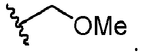 In embodiments, R⁷ is independently -OH. In embodiments, R⁷ is independently -CH₂OH. In embodiments, R⁷ is independently - OCH₃.

In embodiments, the compound has the formula: 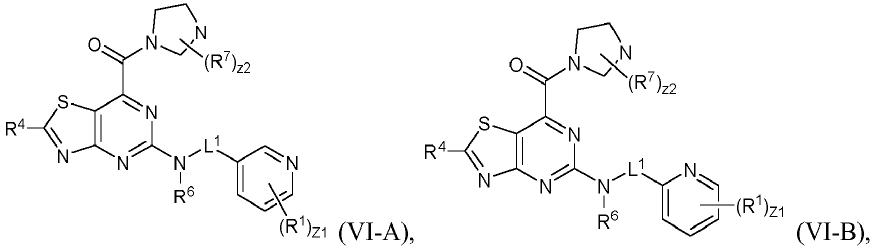 or 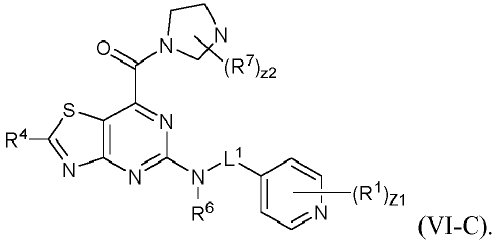 R¹, R⁴, R⁶, L¹, z1, and z2 are as described herein, including embodiments.

In embodiments, the compound has the formula: 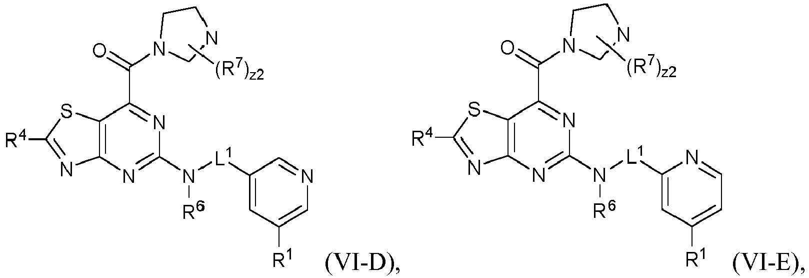 or 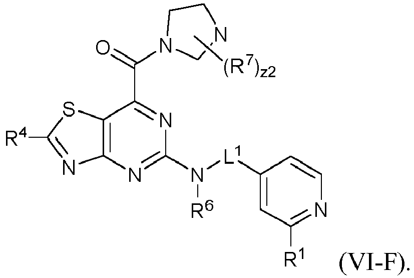 R¹, R⁴, R⁶, L¹, and z2 are as described herein, including embodiments.

In embodiments, the compound has the formula: 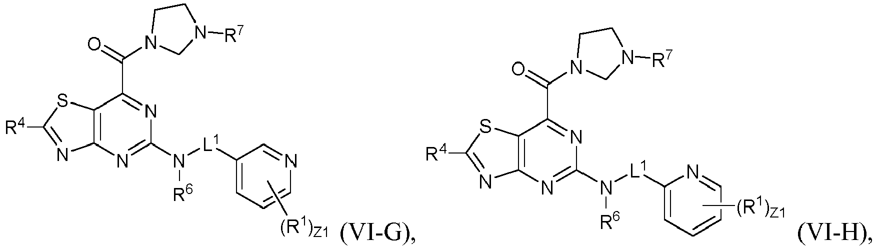 or 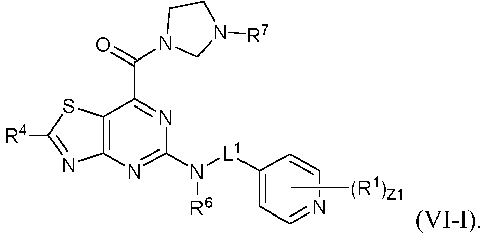 R¹, R⁴, R⁶, L¹, and z1 are as described herein, including embodiments.

In embodiments, the compound has the formula: 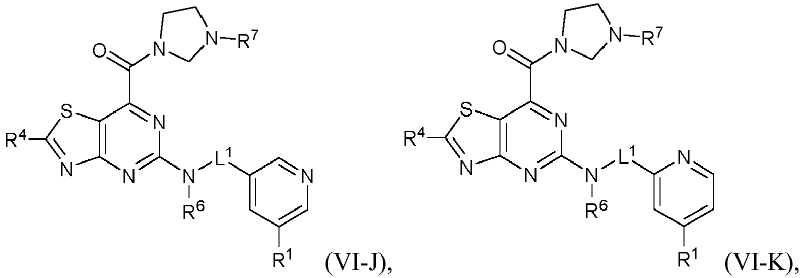 or 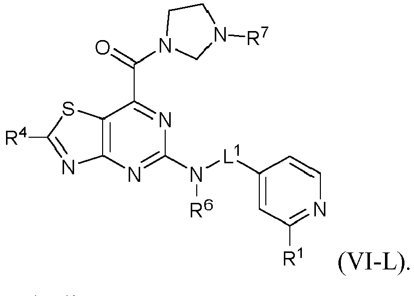 R¹, R⁴, R⁶, and L¹ are as described herein, including embodiments.

In embodiments, z1 is an integer from 0 to 4 and z2 is an integer from 0 to 7. In embodiments, z2 is 0. In embodiments, z2 is 1. In embodiments, z2 is 2. In embodiments, z2 is 3. In embodiments, z2 is 4. In embodiments, z2 is 5. In embodiments, z2 is 6. In embodiments, z2 is 7.

In embodiments, z2 is 0 or 1. In embodiments, R⁷ is independently -NR^{7A}R^{7B} (e.g., -NH₂, or -NHCH₃). In embodiments, R⁷ is independently -C(O)NR^{7A}R^{7B} (e.g., -C(O)NH₂, -C(O)NHCH₃, or -C(O)N(CH₃)₂). In embodiments, R⁷ is independently -NR^{7A}C(O)R^{7C} (e.g., -NHC(O)H, -NCH₃C(O)H, -NCH₃C(O)CH₃, or -NHC(O)CH₃). In embodiments, R⁷ is independently -NR^{7A}C(O)NR^{7A}R^{7B} (e.g., -NHC(O)NH₂, -NCH₃C(O)NH₂, or -NHC(O)NHCH₃). In embodiments, R⁷ is independently-OR^{7D} (e.g., -OH, or -OCH₃). In embodiments, R⁷ is independently -C(O)R^{7C} (e.g., -C(O)H or -C(O)CH₃). Each R^{7A}, R^{7B}, R^{7C}, and R^{7D} is as described herein. In embodiments, R^{7A} and R^{7B} are independently hydrogen, or unsubstituted C₁-C₄ alkyl. In embodiments, R^{7C} is independently unsubstituted C₁-C₆ alkyl. In embodiments, R^{7D} is independently hydrogen, or unsubstituted C₁-C₄ alkyl.

In embodiments, R⁷ is independently -C(O)R^{7C}. In embodiments, R^{7C} is independently unsubstituted C₁-C₆ alkyl. In embodiments, R^{7A} is hydrogen and R^{7B} is unsubstituted C₁-C₄ alkyl. In embodiments, R⁷ is independently 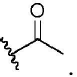 In embodiments, R⁷ is independently 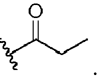 In embodiments, R⁷ is independently 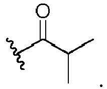 In embodiments, R⁷ is independently 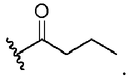 In embodiments, R⁷ is independently 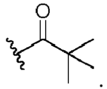 In embodiments, R⁷ is independently 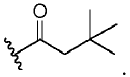 In embodiments, R⁷ is independently 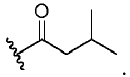 In embodiments, R⁷ is independently

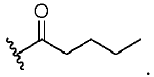

In embodiments, R⁷ is independently -C(O)NR^{7A}R^{7B}. In embodiments, R^{7A} is hydrogen and R^{7B} is unsubstituted C₁-C₄ alkyl. In embodiments, R⁷ is independently 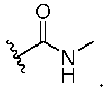 In embodiments, R⁷ is independently 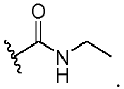 In embodiments, R⁷ is independently 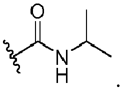 In embodiments, R⁷ is independently 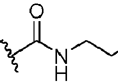 . In embodiments, R⁷ is independently 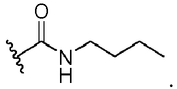 In embodiments, R⁷ is independently 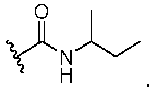 In embodiments, R⁷ is independently

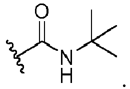

In embodiments, the compound has a formula: 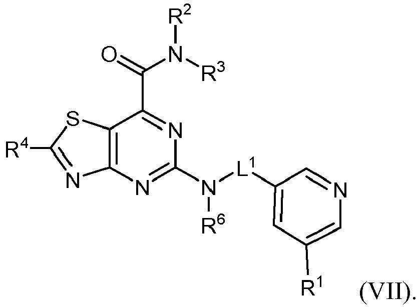 R¹, R², R³, R⁴, R⁶, and L¹ are as described herein, including embodiments. In embodiments, R' is independently hydrogen or halogen. In embodiments of formula (VII), R¹ is hydrogen. In embodiments of formula (VII), R¹ is halogen. In embodiments of formula (VII), R¹ is -F.

In embodiments, the compound has a formula: 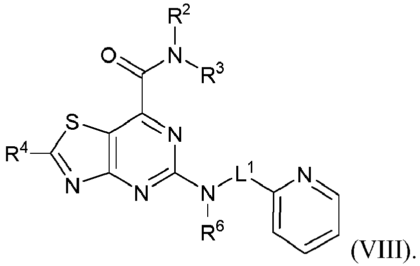 R², R³, R⁴, R⁶, and L¹ are as described herein, including embodiments.

In embodiments, R² and R³ are each independently hydrogen, substituted or unsubstituted C₁-C₆ alkyl, or substituted or unsubstituted 2 to 8 membered heteroalkyl. In embodiments, R² and R³ are each independently hydrogen, substituted or unsubstituted C₁-C₆ alkyl, or substituted or unsubstituted 2 to 6 membered heteroalkyl. In embodiments, R² is hydrogen. In embodiments, R² is substituted or unsubstituted C₁-C₈ alkyl. In embodiments, R² is unsubstituted C₁-C₈ alkyl. In embodiments, R² is substituted or unsubstituted C₁-C₆ alkyl. In embodiments, R² is unsubstituted C₁-C₆ alkyl. In embodiments, R² is substituted or unsubstituted C₁-C₄ alkyl. In embodiments, R² is unsubstituted C₁-C₄ alkyl. In embodiments, R² is unsubstituted methyl. In embodiments, R² is unsubstituted ethyl. In embodiments, R² is unsubstituted propyl. In embodiments, R² is unsubstituted isopropyl. In embodiments, R² is unsubstituted butyl. In embodiments, R² is unsubstituted t-butyl. In embodiments, R² is substituted or unsubstituted 2 to 10 membered heteroalkyl. In embodiments, R² is substituted 2 to 10 membered heteroalkyl. In embodiments, R² is unsubstituted 2 to 10 membered heteroalkyl. In embodiments, R² is substituted or unsubstituted 2 to 8 membered heteroalkyl. In embodiments, R² is substituted 2 to 8 membered heteroalkyl. In embodiments, R² is unsubstituted 2 to 8 membered heteroalkyl. In embodiments, R² is substituted or unsubstituted 2 to 6 membered heteroalkyl. In embodiments, R² is unsubstituted 2 to 6 membered heteroalkyl. In embodiments, R² is substituted or unsubstituted 2 membered heteroalkyl. In embodiments, R² is unsubstituted 2 membered heteroalkyl. In embodiments, R² is substituted or unsubstituted 3 membered heteroalkyl. In embodiments, R² is unsubstituted 3 membered heteroalkyl. In embodiments, R² is substituted or unsubstituted 4 membered heteroalkyl. In embodiments, R² is unsubstituted 4 membered heteroalkyl. In embodiments, R² is substituted or unsubstituted 5 membered heteroalkyl. In embodiments, R² is unsubstituted 5 membered heteroalkyl. In embodiments, R² is substituted or unsubstituted 6 membered heteroalkyl. In embodiments, R² is unsubstituted 6 membered heteroalkyl. In embodiments, R³ is hydrogen. In embodiments, R³ is substituted or unsubstituted C₁-C₈ alkyl. In embodiments, R³ is unsubstituted C₁-C₈ alkyl. In embodiments, R³ is substituted or unsubstituted C₁-C₆ alkyl. In embodiments, R³ is unsubstituted C₁-C₆ alkyl. In embodiments, R³ is substituted or unsubstituted C₁-C₄ alkyl. In embodiments, R³ is unsubstituted C₁-C₄ alkyl. In embodiments, R³ is unsubstituted methyl. In embodiments, R³ is unsubstituted ethyl. In embodiments, R³ is unsubstituted propyl. In embodiments, R³ is unsubstituted isopropyl. In embodiments, R³ is unsubstituted butyl. In embodiments, R³ is unsubstituted t-butyl. In embodiments, R³ is substituted or unsubstituted 2 to 10 membered heteroalkyl. In embodiments, R³ is substituted 2 to 10 membered heteroalkyl. In embodiments, R³ is unsubstituted 2 to 10 membered heteroalkyl. In embodiments, R³ is substituted or unsubstituted 2 to 8 membered heteroalkyl. In embodiments, R³ is substituted 2 to 8 membered heteroalkyl. In embodiments, R³ is unsubstituted 2 to 8 membered heteroalkyl. In embodiments, R³ is substituted or unsubstituted 2 to 6 membered heteroalkyl. In embodiments, R³ is unsubstituted 2 to 6 membered heteroalkyl. In embodiments, R³ is substituted or unsubstituted 2 membered heteroalkyl. In embodiments, R³ is unsubstituted 2 membered heteroalkyl. In embodiments, R³ is substituted or unsubstituted 3 membered heteroalkyl. In embodiments, R³ is unsubstituted 3 membered heteroalkyl. In embodiments, R³ is substituted or unsubstituted 4 membered heteroalkyl. In embodiments, R³ is unsubstituted 4 membered heteroalkyl. In embodiments, R³ is substituted or unsubstituted 5 membered heteroalkyl. In embodiments, R³ is unsubstituted 5 membered heteroalkyl. In embodiments, R³ is substituted or unsubstituted 6 membered heteroalkyl. In embodiments, R³ is unsubstituted 6 membered heteroalkyl.

In embodiments, R² is substituted or unsubstituted C₁-C₈ alkyl, or substituted or unsubstituted 2 to 8 membered heteroalkyl. In embodiments, R² is substituted or unsubstituted C₁-C₄ alkyl, or substituted or unsubstituted 2 to 4 membered heteroalkyl. In embodiments, R² is substituted or unsubstituted C₁-C₁₀ alkyl, or substituted or unsubstituted 2 to 10 membered heteroalkyl. In embodiments, R² is substituted or unsubstituted C₁-C₄ alkyl, or substituted or unsubstituted 2 to 4 membered heteroalkyl. In embodiments, R² is:

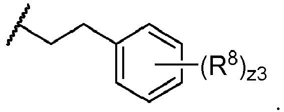

In embodiments, R³ is substituted or unsubstituted C₁-C₈ alkyl, or substituted or unsubstituted 2 to 8 membered heteroalkyl. In embodiments, R³ is substituted or unsubstituted C₁-C₄ alkyl, or substituted or unsubstituted 2 to 4 membered heteroalkyl. In embodiments, R³ is substituted or unsubstituted C₁-C₁₀ alkyl, or substituted or unsubstituted 2 to 10 membered heteroalkyl. In embodiments, R³ is substituted or unsubstituted C₁-C₄ alkyl, or substituted or unsubstituted 2 to 4 membered heteroalkyl. In embodiments, R³ is:

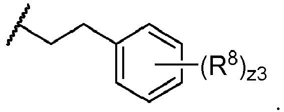

R⁸ is independently halogen, -CX⁸₃, -CHX⁸₂, -CH₂X⁸, -OCX⁸₃, -OCH₂X⁸, -OCHX⁸₂, -N₃, -CN, -SOₙ₈R^{8D}, -SOᵥ₈NR^{8A}R^{8B}, -NHC(O)NR^{8A}R^{8B}, -N(O)ₘ₈, -NR^{8A}R^{8B}, -C(O)R^{8C}, -C(O)-OR^{8C}, -C(O)NR^{8A}R^{8B}, -OR^{8D}, -NR^{8A}SO₂R^{8D}, -NR^{8A}C(O)R^{8C}, -NR^{8A}C(O)OR^{8C}, -NR^{8A}OR^{8C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. z3 is an integer from 0 to 5. X⁸ is independently -F, -Cl, -Br, or -I.

R^{8A}, R^{8B}, R^{8C}, and R^{8D} are independently hydrogen, -CX₃, -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

In embodiments, z3 is 1. In embodiments, z3 is 2. In embodiments, z3 is 3. In embodiments, z3 is 4. In embodiments, z3 is 5. n8 is independently an integer from 0 to 4. In embodiments, n8 is 0. In embodiments, n8 is 1. In embodiments, n8 is 2. In embodiments, n8 is 3. In embodiments, n8 is 4. m8 and v8 are independently an integer from 1 to 2. In embodiments, m8 is 1. In embodiments, m8 is 2. In embodiments, v8 is 1. In embodiments, v8 is 2.

In embodiments, the compound has a formula: 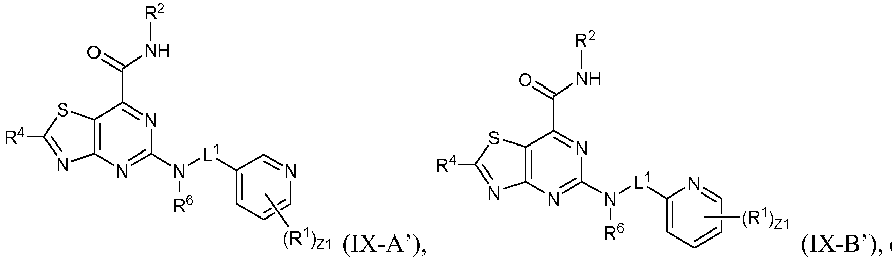 or 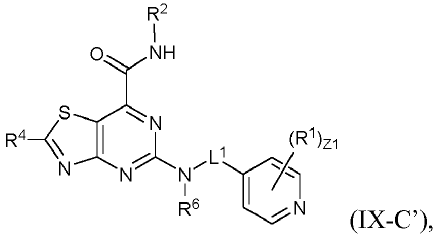 R¹, R², R⁴, R⁶, L¹ and z1 are as described herein, including embodiments.

In embodiments, the compound has a formula: 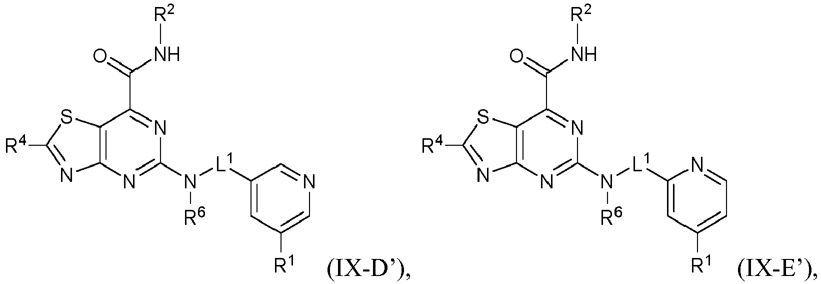 or 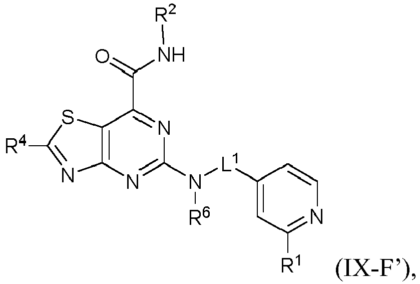 R¹, R², R⁴, R⁶, and L¹ are as described herein, including embodiments.

In embodiments, R² is substituted or unsubstituted C₁-C₈ alkyl, or substituted or unsubstituted 2 to 8 membered heteroalkyl. In embodiments, R² is substituted or unsubstituted C₁-C₄ alkyl, or substituted or unsubstituted 2 to 4 membered heteroalkyl. In embodiments, R² is substituted or unsubstituted C₁-C₄ alkyl. In embodiments, R² is unsubstituted C₁-C₄ alkyl. In embodiments, R² is substituted methyl. In embodiments, R² is unsubstituted methyl. In embodiments, R² is substituted ethyl. In embodiments, R² is unsubstituted ethyl. In embodiments, R² is substituted propyl. In embodiments, R² is unsubstituted propyl. In embodiments, R² is substituted isopropyl. In embodiments, R² is unsubstituted isopropyl. In embodiments, R² is substituted butyl. In embodiments, R² is unsubstituted butyl. In embodiments, R² is substituted t-butyl. In embodiments, R² is unsubstituted t-butyl. In embodiments, R² is substituted phenyl-substituted methyl. In embodiments, R² is unsubstituted phenyl-substituted methyl. In embodiments, R² is substituted tetrahydropyranyl-substituted methyl. In embodiments, R² is unsubstituted tetrahydropyranyl-substituted methyl. In embodiments, R² is substituted pyrrolidinyl-substituted methyl. In embodiments, R² is unsubstituted pyrrolidinyl-substituted methyl. In embodiments, R² is substituted piperidinyl-substituted methyl. In embodiments, R² is unsubstituted piperidinyl-substituted methyl. In embodiments, R² is substituted pyridyl-substituted methyl. In embodiments, R² is unsubstituted pyridyl-substituted methyl. In embodiments, R² is substituted pyrrolidinyl-substituted methyl. In embodiments, R² is unsubstituted pyrrolidinyl-substituted methyl. In embodiments, R² is substituted phenyl-substituted ethyl. In embodiments, R² is unsubstituted phenyl-substituted ethyl. In embodiments, R² is substituted piperidinyl-substituted ethyl. In embodiments, R² is unsubstituted piperidinyl-substituted ethyl. In embodiments, R² is substituted pyridyl-substituted ethyl. In embodiments, R² is unsubstituted pyridyl-substituted ethyl. In embodiments, R² is substituted pyrrolidinyl-substituted ethyl. In embodiments, R² is unsubstituted pyrrolidinyl-substituted ethyl. In embodiments, R² is substituted phenyl-substituted propyl. In embodiments, R² is unsubstituted phenyl-substituted propyl. In embodiments, R² is substituted piperidinyl-substituted propyl. In embodiments, R² is unsubstituted piperidinyl-substituted propyl. In embodiments, R² is substituted pyridyl-substituted propyl. In embodiments, R² is unsubstituted pyridyl-substituted propyl. In embodiments, R² is substituted pyrrolidinyl-substituted propyl. In embodiments, R² is unsubstituted pyrrolidinyl-substituted propyl. In embodiments, R² is substituted phenyl-substituted isopropyl. In embodiments, R² is unsubstituted phenyl-substituted isopropyl. In embodiments, R² is substituted piperidinyl-substituted isopropyl. In embodiments, R² is unsubstituted piperidinyl-substituted isopropyl. In embodiments, R² is substituted pyridyl-substituted isopropyl. In embodiments, R² is unsubstituted pyridyl-substituted isopropyl. In embodiments, R² is substituted pyrrolidinyl-substituted isopropyl. In embodiments, R² is unsubstituted pyrrolidinyl-substituted isopropyl. In embodiments, R² is substituted phenyl-substituted butyl. In embodiments, R² is unsubstituted phenyl-substituted butyl. In embodiments, R² is substituted piperidinyl-substituted butyl. In embodiments, R² is unsubstituted piperidinyl-substituted butyl. In embodiments, R² is substituted pyridyl-substituted butyl. In embodiments, R² is unsubstituted pyridyl-substituted butyl. In embodiments, R² is substituted pyrrolidinyl-substituted butyl. In embodiments, R² is unsubstituted pyrrolidinyl-substituted butyl. In embodiments, R² is substituted phenyl-substituted t-butyl. In embodiments, R² is unsubstituted phenyl-substituted t-butyl. In embodiments, R² is substituted piperidinyl-substituted t-butyl. In embodiments, R² is unsubstituted piperidinyl-substituted t-butyl. In embodiments, R² is substituted pyridyl-substituted t-butyl. In embodiments, R² is unsubstituted pyridyl-substituted t-butyl. In embodiments, R² is substituted pyrrolidinyl-substituted t-butyl. In embodiments, R² is unsubstituted pyrrolidinyl-substituted t-butyl. In embodiments, R² is hydroxyl-methyl. In embodiments, R² is unsubstituted tetrahydrofuranyl-substituted methyl. In embodiments, R² is tetrahydrofuranyl-substituted methyl. In embodiments, R² is unsubstituted tetrahydrofuranyl-substituted ethyl. In embodiments, R² is tetrahydrofuranyl-substituted ethyl. In embodiments, R² is hydroxy ethyl. In embodiments, R² is hydroxy propyl. In embodiments, R² is hydroxy isopropyl. In embodiments, R² is hydroxy butyl. In embodiments, R² is hydroxy t-butyl. In embodiments, R² is methoxy methyl. In embodiments, R² is methoxy ethyl. In embodiments, R² is methoxy propyl. In embodiments, R² is methoxy isopropyl. In embodiments, R² is methoxy butyl. In embodiments, R² is methoxy t-butyl. In embodiments, R² is ethoxy methyl. In embodiments, R² is ethoxy ethyl. In embodiments, R² is ethoxy propyl. In embodiments, R² is ethoxy isopropyl. In embodiments, R² is ethoxy butyl. In embodiments, R² is ethoxy t-butyl. In embodiments, R² is substituted or unsubstituted 2 to 6 membered heteroalkyl. In embodiments, R² is unsubstituted 2 to 6 membered heteroalkyl. In embodiments, R² is substituted or unsubstituted 2 membered heteroalkyl. In embodiments, R² is unsubstituted 2 membered heteroalkyl. In embodiments, R² is substituted or unsubstituted 3 membered heteroalkyl. In embodiments, R² is unsubstituted 3 membered heteroalkyl. In embodiments, R² is substituted or unsubstituted 4 membered heteroalkyl. In embodiments, R² is unsubstituted 4 membered heteroalkyl. In embodiments, R² is substituted or unsubstituted 5 membered heteroalkyl. In embodiments, R² is unsubstituted 5 membered heteroalkyl. In embodiments, R² is substituted or unsubstituted 6 membered heteroalkyl. In embodiments, R² is unsubstituted 6 membered heteroalkyl. In embodiments, R² is substituted or unsubstituted 6 membered heteroalkyl containing substituted urea. In embodiments, R² is substituted or unsubstituted 6 membered heteroalkyl containing unsubstituted urea. In embodiments, R² is unsubstituted 6 membered heteroalkyl containing urea.

In embodiments, R² is: 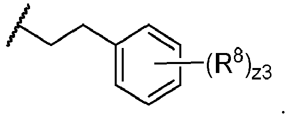 R⁸ is as described herein.

In embodiments, z3 is an integer from 0 to 5. n8 is independently an integer from 0 to 4. m8 and v8 are independently an integer from 1 to 2.

In embodiments, R² is:

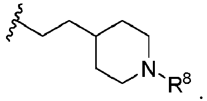

In embodiments, R⁸ is hydrogen, halogen, -CX⁸₃, -CHX⁸₂, -CH₂X⁸, -OCX⁸₃, -OCH₂X⁸, -OCHX⁸₂, -CN, -C(O)R^{8C}, -C(O)-OR^{8C}, -C(O)NR^{8A}R^{8B}, -OR^{8D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. n8, m8, v8, R^{8A}, R^{8B}, R^{8C}, R^{8D} and X⁸ are as described herein.

In embodiments, R² is:

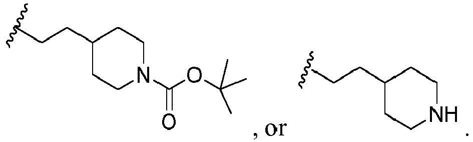

In embodiments, R² is: 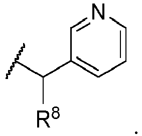 R⁸ is as described herein.

In embodiments, R² is independently 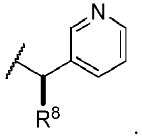 In embodiments, R² is independently 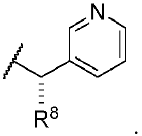 R⁸ is as described herein.

In embodiments, R⁸ is hydrogen, halogen, -CX⁸₃, -CHX⁸₂, -CH₂X⁸, -OCX⁸₃, -OCH₂X⁸, -OCHX⁸₂, -N₃, -CN, -SOₙ₈R^{8D}, -SOᵥ₈NR^{8A}R^{8B}, -NHC(O)NR^{8A}R^{8B}, -N(O)ₘ₈, -NR^{8A}R^{8B}, -C(O)R^{8C}, -C(O)-OR^{8C}, -C(O)NR^{8A}R^{8B}, -OR^{8D}, -NR^{8A}SO₂R^{8D}, -NR^{8A}C(O)R^{8C}, -NR^{8A}C(O)OR^{8C}, -NR^{8A}OR^{8C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. n8, m8, v8, R^{8A}, R^{8B}, R^{8C}, R^{8D} and X⁸ are as described herein.

In embodiments, R² is:

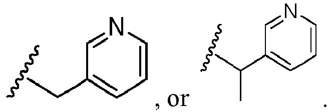

In embodiments, R² is: 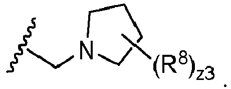 R⁸ is as described herein.

In embodiments, R⁸ is halogen, -CX⁸₃, -CHX⁸₂, -CH₂X⁸, -OCX⁸₃, -OCH₂X⁸, -OCHX⁸₂, -N₃, -CN, -SOₙ₈R^{8D} , -SOᵥ₈NR^{8A}R^{8B} , -NHC(O)NR^{8A}R^{8B}, -N(O)ₘ₈, -NR^{8A}R^{8B}, -C(O)R^{8C}, -C(O)-OR^{8C}, -C(O)NR^{8A}R^{8B}, -OR^{8D}, -NR^{8A}SO₂R^{8D}, -NR^{8A}C(O)R^{8C}, -NR^{8A}C(O)OR^{8C}, -NR^{8A}OR^{8C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. z3 is an integer from 0 to 8. n8, m8, v8, R^{8A}, R^{8B}, R^{8C}, R^{8D} and X⁸ are as described herein.

In embodiments, R² is:

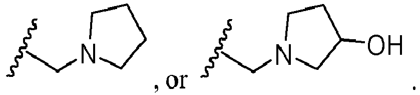

In embodiments R² is independently 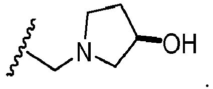 In embodiments, R² is independently

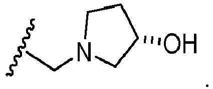

In embodiments, R² is:

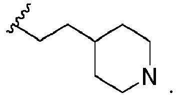

In embodiments, R² is

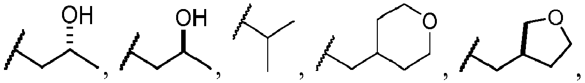

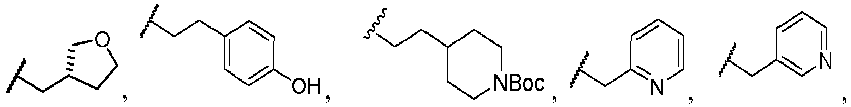

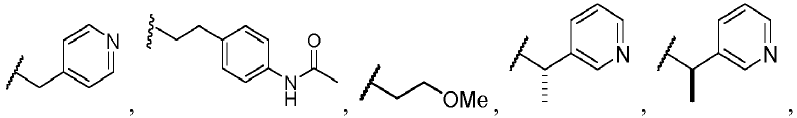

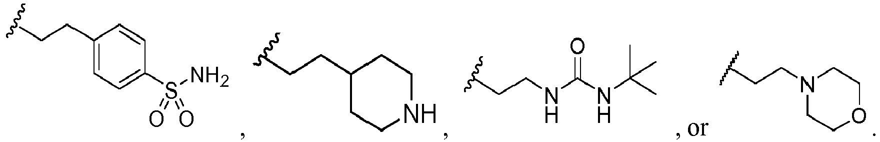

In embodiments, the compound has a formula: 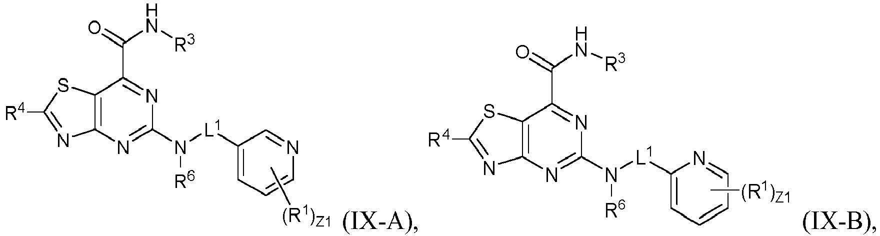 or 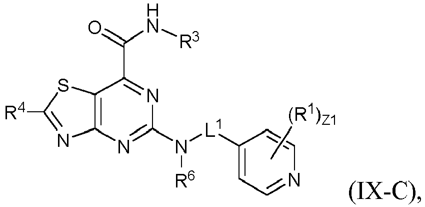 R¹, R³, R⁴, R⁶, L¹ and z1 are as described herein, including embodiments.

In embodiments, the compound has a formula: 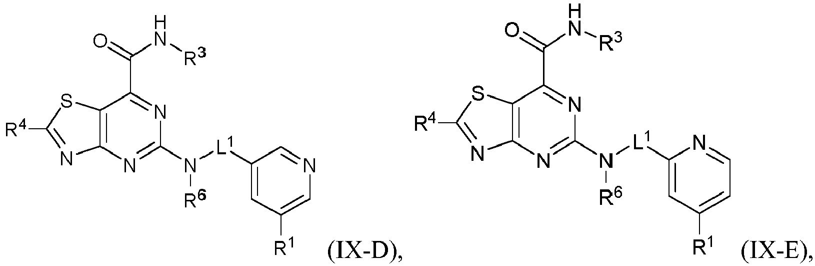 or 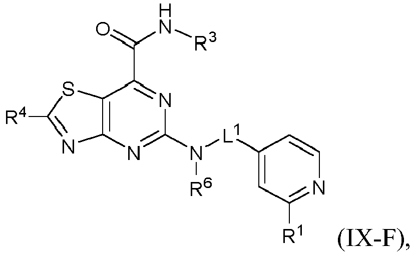 R¹, R³, R⁴, R⁶, and L¹ are as described herein, including embodiments.

In embodiments, R³ is substituted or unsubstituted C₁-C₈ alkyl, or substituted or unsubstituted 2 to 8 membered heteroalkyl. In embodiments, R³ is substituted or unsubstituted C₁-C₄ alkyl, or substituted or unsubstituted 2 to 4 membered heteroalkyl. In embodiments, R³ is substituted or unsubstituted C₁-C₄ alkyl. In embodiments, R³ is unsubstituted C₁-C₄ alkyl. In embodiments, R³ is substituted methyl. In embodiments, R³ is unsubstituted methyl. In embodiments, R³ is substituted ethyl. In embodiments, R³ is unsubstituted ethyl. In embodiments, R³ is substituted propyl. In embodiments, R³ is unsubstituted propyl. In embodiments, R³ is substituted isopropyl. In embodiments, R³ is unsubstituted isopropyl. In embodiments, R³ is substituted butyl. In embodiments, R³ is unsubstituted butyl. In embodiments, R³ is substituted t-butyl. In embodiments, R³ is unsubstituted t-butyl. In embodiments, R³ is substituted phenyl-subsituted methyl. In embodiments, R³ is unsubstituted phenyl-subsituted methyl. In embodiments, R³ is substituted tetrahydropyranyl -subsituted methyl. In embodiments, R³ is unsubstituted tetrahydropyranyl -subsituted methyl. In embodiments, R³ is substituted pyrrolidinyl-subsituted methyl. In embodiments, R³ is unsubstituted pyrrolidinyl-subsituted methyl. In embodiments, R³ is substituted piperidinyl-subsituted methyl. In embodiments, R³ is unsubstituted piperidinyl-subsituted methyl. In embodiments, R³ is substituted pyridyl - subsituted methyl. In embodiments, R³ is unsubstituted pyridyl-subsituted methyl. In embodiments, R³ is substituted pyrrolidinyl-subsituted methyl. In embodiments, R³ is unsubstituted pyrrolidinyl-subsituted methyl. In embodiments, R³ is substituted phenyl-subsituted ethyl. In embodiments, R³ is unsubstituted phenyl-subsituted ethyl. In embodiments, R³ is substituted piperidinyl -subsituted ethyl. In embodiments, R³ is unsubstituted piperidinyl-subsituted ethyl. In embodiments, R³ is substituted pyridyl-subsituted ethyl. In embodiments, R³ is unsubstituted pyridyl-subsituted ethyl. In embodiments, R³ is substituted pyrrolidinyl-subsituted ethyl. In embodiments, R³ is unsubstituted pyrrolidinyl-subsituted ethyl. In embodiments, R³ is substituted phenyl-subsituted propyl. In embodiments, R³ is unsubstituted phenyl-subsituted propyl. In embodiments, R³ is substituted piperidinyl-subsituted propyl. In embodiments, R³ is unsubstituted piperidinyl-subsituted propyl. In embodiments, R³ is substituted pyridyl-subsituted propyl. In embodiments, R³ is unsubstituted pyridyl -subsituted propyl. In embodiments, R³ is substituted pyrrolidinyl-subsituted propyl. In embodiments, R³ is unsubstituted pyrrolidinyl-subsituted propyl. In embodiments, R³ is substituted phenyl-subsituted isopropyl. In embodiments, R³ is unsubstituted phenyl-subsituted isopropyl. In embodiments, R³ is substituted piperidinyl-subsituted isopropyl. In embodiments, R³ is unsubstituted piperidinyl-subsituted isopropyl. In embodiments, R³ is substituted pyridyl-subsituted isopropyl. In embodiments, R³ is unsubstituted pyridyl-subsituted isopropyl. In embodiments, R³ is substituted pyrrolidinyl-subsituted isopropyl. In embodiments, R³ is unsubstituted pyrrolidinyl-subsituted isopropyl. In embodiments, R³ is substituted phenyl-subsituted butyl. In embodiments, R³ is unsubstituted phenyl-subsituted butyl. In embodiments, R³ is substituted piperidinyl-subsituted butyl. In embodiments, R³ is unsubstituted piperidinyl-subsituted butyl. In embodiments, R³ is substituted pyridyl - subsituted butyl. In embodiments, R³ is unsubstituted pyridyl -subsituted butyl. In embodiments, R³ is substituted pyrrolidinyl-subsituted butyl. In embodiments, R³ is unsubstituted pyrrolidinyl-subsituted butyl. In embodiments, R³ is substituted phenyl-subsituted t-butyl. In embodiments, R³ is unsubstituted phenyl-subsituted t-butyl. In embodiments, R³ is substituted piperidinyl-subsituted t-butyl. In embodiments, R³ is unsubstituted piperidinyl-subsituted t-butyl. In embodiments, R³ is substituted pyridyl-subsituted t-butyl. In embodiments, R³ is unsubstituted pyridyl-subsituted t-butyl. In embodiments, R³ is substituted pyrrolidinyl-subsituted t-butyl. In embodiments, R³ is unsubstituted pyrrolidinyl-subsituted t-butyl. In embodiments, R³ is hydroxyl-methyl. In embodiments, R³ is unsubstituted tetrahydrofuranyl-substituted methyl. In embodiments, R³ is tetrahydrofuranyl-substituted methyl. In embodiments, R³ is unsubstituted tetrahydrofuranyl-substituted ethyl. In embodiments, R³ is tetrahydrofuranyl-substituted ethyl. In embodiments, R³ is hydroxy ethyl. In embodiments, R³ is hydroxy propyl. In embodiments, R³ is hydroxy isopropyl. In embodiments, R³ is hydroxy butyl. In embodiments, R³ is hydroxy t-butyl. In embodiments, R³ is methoxy methyl. In embodiments, R³ is methoxy ethyl. In embodiments, R³ is methoxy propyl. In embodiments, R³ is methoxy isopropyl. In embodiments, R³ is methoxy butyl. In embodiments, R³ is methoxy t-butyl. In embodiments, R³ is ethoxy methyl. In embodiments, R³ is ethoxy ethyl. In embodiments, R³ is ethoxy propyl. In embodiments, R³ is ethoxy isopropyl. In embodiments, R³ is ethoxy butyl. In embodiments, R³ is ethoxy t-butyl. In embodiments, R³ is substituted or unsubstituted 2 to 6 membered heteroalkyl. In embodiments, R³ is unsubstituted 2 to 6 membered heteroalkyl. In embodiments, R³ is substituted or unsubstituted 2 membered heteroalkyl. In embodiments, R³ is unsubstituted 2 membered heteroalkyl. In embodiments, R³ is substituted or unsubstituted 3 membered heteroalkyl. In embodiments, R³ is unsubstituted 3 membered heteroalkyl. In embodiments, R³ is substituted or unsubstituted 4 membered heteroalkyl. In embodiments, R³ is unsubstituted 4 membered heteroalkyl. In embodiments, R³ is substituted or unsubstituted 5 membered heteroalkyl. In embodiments, R³ is unsubstituted 5 membered heteroalkyl. In embodiments, R³ is substituted or unsubstituted 6 membered heteroalkyl. In embodiments, R³ is unsubstituted 6 membered heteroalkyl. In embodiments, R³ is substituted or unsubstituted 6 membered heteroalkyl containing subsituted urea. In embodiments, R³ is substituted or unsubstituted 6 membered heteroalkyl containing unsubsituted urea. In embodiments, R³ is unsubstituted 6 membered heteroalkyl containing urea.

In embodiments, R³ is: 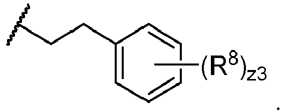 R⁸ is as described herein, including in embodiments.

In embodiments, z3 is an integer from 0 to 5. n8 is independently an integer from 0 to 4. m8 and v8 are independently an integer from 1 to 2.

In embodiments, R³ is:

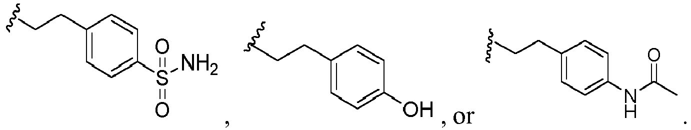

In embodiments, R³ is: 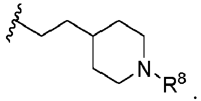 R⁸ is as described herein.

In embodiments, R³ is:

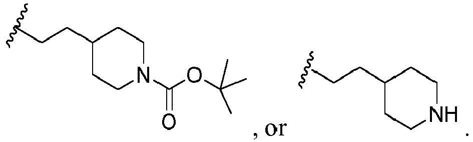

In embodiments, R³ is: 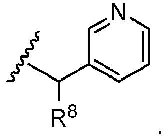 R⁸ is as described herein.

In embodiments, R³ is independently 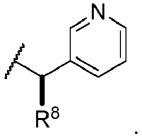 In embodiments, R³ is independently 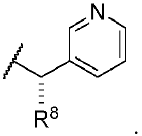 R⁸ is as described herein.

In embodiments, R³ is:

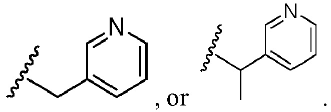

In embodiments, R³ is: 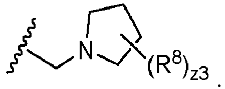 R⁸ is as described herein. z3 is an integer from 0 to 8.

In embodiments, R³ is:

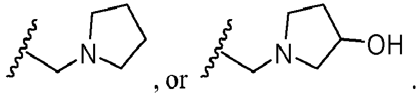

In embodiments, R³ is independently 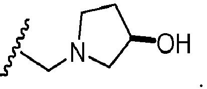 In embodiments, R³ is independently

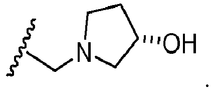

In embodiments, R³ is:

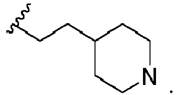

In embodiments, R³ is

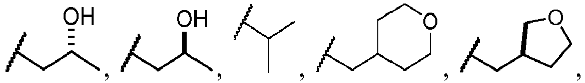

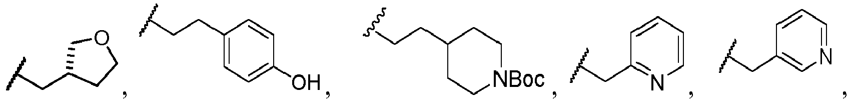

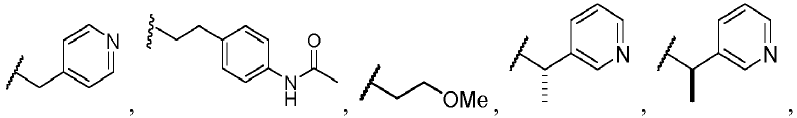

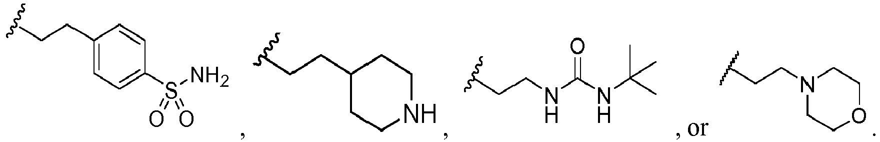

In embodiments, L¹ is substituted or unsubstituted C₁-C₈ alkylene. In embodiments, L¹ is unsubstituted C₁-C₈ alkylene. In embodiments, L¹ is substituted or unsubstituted C₁-C₆ alkylene. In embodiments, L¹ is unsubstituted C₁-C₆ alkylene. In embodiments, L¹ is substituted or unsubstituted C₁-C₃ alkylene. In embodiments, L¹ is unsubstituted C₁-C₃ alkylene. In embodiments, L¹ is substituted or unsubstituted C₁-C₂ alkylene. In embodiments, L¹ is unsubstituted C₁-C₂ alkylene. In embodiments, L¹ is substituted ethylene. In embodiments, L¹ is unsubstituted ethylene. In embodiments, L¹ is substituted methylene. In embodiments, L¹ is unsubstituted methylene.

In embodiments, L¹ is 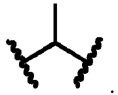 In embodiments, L¹ is 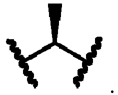 In embodiments, L¹ is 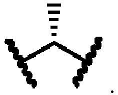 In embodiments, L¹ is 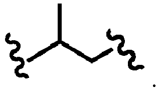 In embodiments, L¹ is 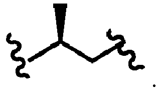 In embodiments, L¹ is 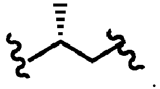 In embodiments, L¹ is 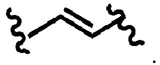 In embodiments, L¹ is 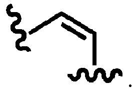 In embodiments, L¹ is 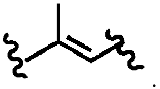 In embodiments, L¹ is

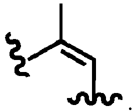

In embodiments, R⁶ is hydrogen. In embodiments, R⁶ is unsubstituted methyl. In embodiments, R⁶ is unsubstituted ethyl. In embodiments, R⁶ is -CH₃. In embodiments, R⁶ is -OH.

In embodiments, R⁴ is hydrogen. In embodiments, R⁴ is substituted or unsubstituted C₁-C₈ alkyl. In embodiments, R⁴ is substituted or unsubstituted 2 to 6 membered heteroalkyl. In embodiments, R⁴ is substituted or unsubstituted C₁-C₄ alkyl, or substituted or unsubstituted 2 to 4 membered heteroalkyl. In embodiments, R⁴ is unsubstituted C₁-C₄ alkyl. In embodiments, R⁴ is unsubstituted methyl. In embodiments, R⁴ is unsubstituted ethyl.

In embodiments, R⁴ is -NR^{4A}R^{4B}. In embodiments, each R^{4A} and R^{4B} is independently hydrogen, or substituted or unsubstituted C₁-C₄ alkyl. In embodiments, R^{4A} is hydrogen. In embodiments, R^{4A} is substituted or unsubstituted C₁-C₄ alkyl. In embodiments, R^{4A} is unsubstituted C₁-C₄ alkyl. In embodiments, R^{4A} is unsubstituted methyl. In embodiments, R^{4A} is unsubstituted ethyl. In embodiments, R^{4B} is hydrogen. In embodiments, R^{4B} is substituted or unsubstituted C₁-C₄ alkyl. In embodiments, R^{4B} is unsubstituted C₁-C₄ alkyl. In embodiments, R^{4B} is unsubstituted methyl. In embodiments, R^{4B} is unsubstituted ethyl. In embodiments, R⁴ is
-NH₂. In embodiments, R⁴ is -NHCH₃. In embodiments, R⁴ is -N(CH₃)₂.

In embodiments, R' is independently oxo, halogen, -CCl₃, -CBr₃, -CF₃, -Cl₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R²⁰-substituted or unsubstituted alkyl (e.g., C₁-C₈ alkyl, C₁-C₆ alkyl, or C₁-C₄ alkyl), R²⁰-substituted or unsubstituted heteroalkyl (e.g., 2 to 8 membered heteroalkyl, 2 to 6 membered heteroalkyl, or 2 to 4 membered heteroalkyl), R²⁰-substituted or unsubstituted cycloalkyl (e.g., C₃-C₈ cycloalkyl, C₃-C₆ cycloalkyl, or C₅-C₆ cycloalkyl), R²⁰-substituted or unsubstituted heterocycloalkyl (e.g., 3 to 8 membered heterocycloalkyl, 3 to 6 membered heterocycloalkyl, or 5 to 6 membered heterocycloalkyl), R²⁰-substituted or unsubstituted aryl (e.g., C₆-C₁₀ aryl, C₁₀ aryl, or phenyl), or R²⁰-substituted or unsubstituted heteroaryl (e.g., 5 to 10 membered heteroaryl, 5 to 9 membered heteroaryl, or 5 to 6 membered heteroaryl).

In embodiments, R¹ is independently R²⁰-substituted or unsubstituted alkyl (e.g., C₁-C₈ alkyl, C₁-C₆ alkyl, or C₁-C₄ alkyl). In embodiments, R¹ is independently R²⁰-substituted alkyl (e.g., C₁-C₈ alkyl, C₁-C₆ alkyl, or C₁-C₄ alkyl). In embodiments, R' is independently an unsubstituted alkyl (e.g., C₁-C₈ alkyl, C₁-C₆ alkyl, or C₁-C₄ alkyl). In embodiments, R' is independently R²⁰-substituted or unsubstituted heteroalkyl (e.g., 2 to 8 membered heteroalkyl, 2 to 6 membered heteroalkyl, or 2 to 4 membered heteroalkyl). In embodiments, R' is independently R²⁰-substituted heteroalkyl (e.g., 2 to 8 membered heteroalkyl, 2 to 6 membered heteroalkyl, or 2 to 4 membered heteroalkyl). In embodiments, R' is independently unsubstituted heteroalkyl (e.g., 2 to 8 membered heteroalkyl, 2 to 6 membered heteroalkyl, or 2 to 4 membered heteroalkyl).

In embodiments, R¹ is independently R²⁰-substituted or unsubstituted cycloalkyl (e.g., C₃-C₈ cycloalkyl, C₃-C₆ cycloalkyl, or C₅-C₆ cycloalkyl). In embodiments, R' is independently R²⁰-substituted cycloalkyl (e.g., C₃-C₈ cycloalkyl, C₃-C₆ cycloalkyl, or C₅-C₆ cycloalkyl). In embodiments, R' is independently unsubstituted cycloalkyl (e.g., C₃-C₈ cycloalkyl, C₃-C₆ cycloalkyl, or C₅-C₆ cycloalkyl). In embodiments, R¹ is independently R²⁰-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R' is independently R²⁰-substituted heterocycloalkyl (e.g., 3 to 8 membered heterocycloalkyl, 3 to 6 membered heterocycloalkyl, or 5 to 6 membered heterocycloalkyl). In embodiments, R' is independently unsubstituted heterocycloalkyl (e.g., 3 to 8 membered heterocycloalkyl, 3 to 6 membered heterocycloalkyl, or 5 to 6 membered heterocycloalkyl).

In embodiments, R' is independently R²⁰-substituted or unsubstituted aryl (e.g., C₆-C₁₀ aryl, C₁₀ aryl, or phenyl). In embodiments, R¹ is independently R²⁰-substituted aryl (e.g., C₆-C₁₀ aryl, C₁₀ aryl, or phenyl). In embodiments, R¹ is independently unsubstituted aryl (e.g., C₆-C₁₀ aryl, C₁₀ aryl, or phenyl). In embodiments, R' is independently R²⁰-substituted or unsubstituted heteroaryl (e.g., 5 to 10 membered heteroaryl, 5 to 9 membered heteroaryl, or 5 to 6 membered heteroaryl). In embodiments, R¹ is independently R²⁰-substituted heteroaryl (e.g., 5 to 10 membered heteroaryl, 5 to 9 membered heteroaryl, or 5 to 6 membered heteroaryl). In embodiments, R' is independently unsubstituted heteroaryl (e.g., 5 to 10 membered heteroaryl, 5 to 9 membered heteroaryl, or 5 to 6 membered heteroaryl).

In embodiments, R^{1A}, R^{1B}, R^{1C}, and R^{1D} are independently hydrogen, halogen, -CF₃, -CBr₃, -CCl₃, -CI₃, -CHF₂, -CHBr₂, -CHCl₂, -CHI₂, -CH₂F, -CH₂Br, -CH₂Cl, -CH₂I, -OCF₃, -OCBr₃, -OCCl₃, -OCI₃, -OCHF₂, -OCHBr₂, -OCHCl₂, -OCHI₂, -OCH₂F, -OCH₂Br, -OCH₂Cl, -OCH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, R²⁰-substituted or unsubstituted alkyl (e.g., C₁-C₈ alkyl, C₁-C₆ alkyl, or C₁-C₄ alkyl), R²⁰-substituted or unsubstituted heteroalkyl (e.g., 2 to 8 membered heteroalkyl, 2 to 6 membered heteroalkyl, or 2 to 4 membered heteroalkyl), R²⁰-substituted or unsubstituted cycloalkyl (e.g., C₃-C₈ cycloalkyl, C₃-C₆ cycloalkyl, or C₅-C₆ cycloalkyl), R²⁰-substituted or unsubstituted heterocycloalkyl (e.g., 3 to 8 membered heterocycloalkyl, 3 to 6 membered heterocycloalkyl, or 5 to 6 membered heterocycloalkyl), R²⁰-substituted or unsubstituted aryl (e.g., C₆-C₁₀ aryl, C₁₀ aryl, or phenyl), or R²⁰-substituted or unsubstituted heteroaryl (e.g., 5 to 10 membered heteroaryl, 5 to 9 membered heteroaryl, or 5 to 6 membered heteroaryl). In embodiments, R^{1A}, R^{1B}, R^{1C}, and R^{1D} are independently hydrogen.

In embodiments, R^{1A}, R^{1B}, R^{1C}, and R^{1D} are independently R²⁰-substituted or unsubstituted alkyl (e.g., C₁-C₈ alkyl, C₁-C₆ alkyl, or C₁-C₄ alkyl). In embodiments, R^{1A}, R^{1B}, R^{1C}, and R^{1D} are independently R²⁰-substituted alkyl (e.g., C₁-C₈ alkyl, C₁-C₆ alkyl, or C₁-C₄ alkyl). In embodiments, R^{1A}, R^{1B}, R^{1C}, and R^{1D} are independently an unsubstituted alkyl (e.g., C₁-C₈ alkyl, C₁-C₆ alkyl, or C₁-C₄ alkyl). In embodiments, R^{1A}, R^{1B}, R^{1C}, R^{1D} are independently R²⁰-substituted or unsubstituted heteroalkyl (e.g., 2 to 8 membered heteroalkyl, 2 to 6 membered heteroalkyl, or 2 to 4 membered heteroalkyl). In embodiments, R^{1A}, R^{1B}, R^{1C}, and R^{1D} are independently R²⁰-substituted heteroalkyl (e.g., 2 to 8 membered heteroalkyl, 2 to 6 membered heteroalkyl, or 2 to 4 membered heteroalkyl). In embodiments, R^{1A}, R^{1B}, R^{1C}, and R^{1D} are independently an unsubstituted heteroalkyl (e.g., 2 to 8 membered heteroalkyl, 2 to 6 membered heteroalkyl, or 2 to 4 membered heteroalkyl).

In embodiments, R^{1A}, R^{1B}, R^{1C}, and R^{1D} are independently R²⁰-substituted or unsubstituted cycloalkyl (e.g., C₃-C₈ cycloalkyl, C₃-C₆ cycloalkyl, or C₅-C₆ cycloalkyl). In embodiments, R^{1A}, R^{1B}, R^{1C}, and R^{1D} are independently R²⁰-substituted cycloalkyl (e.g., C₃-C₈ cycloalkyl, C₃-C₆ cycloalkyl, or C₅-C₆ cycloalkyl). In embodiments, R^{1A}, R^{1B}, R^{1C}, and R^{1D} are independently unsubstituted cycloalkyl (e.g., C₃-C₈ cycloalkyl, C₃-C₆ cycloalkyl, or C₅-C₆ cycloalkyl). In embodiments, R^{1A}, R^{1B}, R^{1C}, and R^{1D} are independently R²⁰-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R^{1A}, R^{1B}, R^{1C}, and R^{1D} are independently R²⁰-substituted heterocycloalkyl (e.g., 3 to 8 membered heterocycloalkyl, 3 to 6 membered heterocycloalkyl, or 5 to 6 membered heterocycloalkyl). In embodiments, R^{1A}, R^{1B}, R^{1C}, and R^{1D} are independently unsubstituted heterocycloalkyl (e.g., 3 to 8 membered heterocycloalkyl, 3 to 6 membered heterocycloalkyl, or 5 to 6 membered heterocycloalkyl).

In embodiments, R^{1A}, R^{1B}, R^{1C}, and R^{1D} are independently R²⁰-substituted or unsubstituted aryl (e.g., C₆-C₁₀ aryl, C₁₀ aryl, or phenyl). In embodiments, R^{1A}, R^{1B}, R^{1C}, and R^{1D} are independently R²⁰-substituted aryl (e.g., C₆-C₁₀ aryl, C₁₀ aryl, or phenyl). In embodiments, R^{1A}, R^{1B}, R^{1C}, and R^{1D} are independently unsubstituted aryl (e.g., C₆-C₁₀ aryl, C₁₀ aryl, or phenyl). In embodiments, R^{1A}, R^{1B}, R^{1C}, and R^{1D} are independently R²⁰-substituted or unsubstituted heteroaryl (e.g., 5 to 10 membered heteroaryl, 5 to 9 membered heteroaryl, or 5 to 6 membered heteroaryl). In embodiments, R^{1A}, R^{1B}, R^{1C}, and R^{1D} are independently R²⁰-substituted heteroaryl (e.g., 5 to 10 membered heteroaryl, 5 to 9 membered heteroaryl, or 5 to 6 membered heteroaryl). In embodiments, R^{1A}, R^{1B}, R^{1C}, and R^{1D} are independently unsubstituted heteroaryl (e.g., 5 to 10 membered heteroaryl, 5 to 9 membered heteroaryl, or 5 to 6 membered heteroaryl).

R²⁰ is independently oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R²¹-substituted or unsubstituted alkyl (e.g., C₁-C₈ alkyl, C₁-C₆ alkyl, or C₁-C₄ alkyl), R²¹⁻substituted or unsubstituted heteroalkyl (e.g., 2 to 8 membered heteroalkyl, 2 to 6 membered heteroalkyl, or 2 to 4 membered heteroalkyl), R²¹-substituted or unsubstituted cycloalkyl (e.g., C₃-C₈ cycloalkyl, C₃-C₆ cycloalkyl, or C₅-C₆ cycloalkyl), R²¹-substituted or unsubstituted heterocycloalkyl (e.g., 3 to 8 membered heterocycloalkyl, 3 to 6 membered heterocycloalkyl, or 5 to 6 membered heterocycloalkyl), R²¹-substituted or unsubstituted aryl (e.g., C₆-C₁₀ aryl, C₁₀ aryl, or phenyl), or R²¹-substituted or unsubstituted heteroaryl (e.g., 5 to 10 membered heteroaryl, 5 to 9 membered heteroaryl, or 5 to 6 membered heteroaryl).

R²¹ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R²²-substituted or unsubstituted alkyl (e.g., C₁-C₈ alkyl, C₁-C₆ alkyl, or C₁-C₄ alkyl), R²²-substituted or unsubstituted heteroalkyl (e.g., 2 to 8 membered heteroalkyl, 2 to 6 membered heteroalkyl, or 2 to 4 membered heteroalkyl), R²²-substituted or unsubstituted cycloalkyl (e.g., C₃-C₈ cycloalkyl, C₃-C₆ cycloalkyl, or C₅-C₆ cycloalkyl), R²²-substituted or unsubstituted heterocycloalkyl (e.g., 3 to 8 membered heterocycloalkyl, 3 to 6 membered heterocycloalkyl, or 5 to 6 membered heterocycloalkyl), R²²-substituted or unsubstituted aryl (e.g., C₆-C₁₀ aryl, C₁₀ aryl, or phenyl), or R²²-substituted or unsubstituted heteroaryl (e.g., 5 to 10 membered heteroaryl, 5 to 9 membered heteroaryl, or 5 to 6 membered heteroaryl).

In embodiments, R' is independently -OH. In embodiments, R' is independently -OCH₃. In embodiments, R¹ is independently -OCH₂CH₃. In embodiments, R¹ is independently unsubstituted C₁-C₄ alkoxy. In embodiments, R' is independently -Cl. In embodiments, R¹ is independently -F. In embodiments, R¹ is independently halogen. In embodiments, R¹ is independently -CH₃. In embodiments, R¹ is independently -CH₂CH₃. In embodiments, R¹ is independently unsubstituted C₁-C₄ alkyl. In embodiments, R¹ is independently -CF₃. In embodiments, R¹ is independently -CX¹₃. In embodiments, R¹ is independently -OCH₃ or -F. In embodiments, R¹ is independently unsubstituted C₁-C₄ alkoxy or halogen. In embodiments, R¹ is independently -CN. In embodiments, R¹ is independently -C(O)NH₂. In embodiments, R¹ is independently -C(O)NR^{1A}R^{1B}. In embodiments, R^{1A} is independently hydrogen. In embodiments, R^{1A} is independently unsubstituted C₁-C₄ alkyl. In embodiments, R^{1A} is independently -CH₃. In embodiments, R^{1A} is independently -CH₂CH₃. In embodiments, R^{1B} is independently hydrogen. In embodiments, R^{1B} is independently unsubstituted C₁-C₄ alkyl. In embodiments, R^{1B} is independently -CH₃. In embodiments, R^{1B} is independently -CH₂CH₃. In embodiments, R^{1C} is independently hydrogen. In embodiments, R^{1C} is independently unsubstituted C₁-C₄ alkyl. In embodiments, R^{1C} is independently -CH₃. In embodiments, R^{1C} is independently-CH₂CH₃. In embodiments, R^{1D} is independently hydrogen. In embodiments, R^{1D} is independently unsubstituted C₁-C₄ alkyl. In embodiments, R^{1D} is independently -CH₃. In embodiments, R^{1D} is independently -CH₂CH₃.

In embodiments, R² is hydrogen, substituted or unsubstituted C₁-C₆ alkyl, substituted or unsubstituted 2 to 6 membered heteroalkyl, substituted or unsubstituted C₃-C₆ cycloalkyl, substituted or unsubstituted 3 to 6 membered heterocycloalkyl, substituted or unsubstituted phenyl, or substituted or unsubstituted 5 to 6 membered heteroaryl.

In embodiments, R² is hydrogen, -CX²₃ (e.g., -CF₃, -CBr₃, -CCl₃, or -CI₃), -CHX²₂ (e.g., -CHF₂, -CHBr₂, -CHCl₂, or -CHI₂), -CH₂X² (e.g., -CH₂F, -CH₂Br, -CH₂Cl, or -CH₂I), -OCX²₃ (e.g., -OCF₃, -OCBr₃, -OCCl₃, or -OCI₃), -OCH₂X² (e.g., -OCH₂F, -OCH₂Br, -OCH₂Cl, or -OCH₂I), -OCHX²₂ (e.g., -OCHF₂, -OCHBr₂, -OCHCl₂, or -OCHI₂), -C(O)R^{2C} (e.g., -COH, -COCH₃, or -COCH₂CH₃), -C(O)OR^{2C} (e.g., -COOH, -COOCH₃, or -COOCH₂CH₃), -C(O)NR^{2A}R^{2B} (e.g., -CONH₂, or -CONHCH₃), -OR^{2D} (e.g., -OH, or -OCH₃), R²³-substituted or unsubstituted alkyl (e.g., C₁-C₈ alkyl, C₁-C₆ alkyl, or C₁-C₄ alkyl), R²³-substituted or unsubstituted heteroalkyl (e.g., 2 to 8 membered heteroalkyl, 2 to 6 membered heteroalkyl, or 2 to 4 membered heteroalkyl), R²³-substituted or unsubstituted cycloalkyl (e.g., C₃-C₈ cycloalkyl, C₃-C₆ cycloalkyl, or C₅-C₆ cycloalkyl), R²³-substituted or unsubstituted heterocycloalkyl (e.g., 3 to 8 membered heterocycloalkyl, 3 to 6 membered heterocycloalkyl, or 5 to 6 membered heterocycloalkyl), R²³-substituted or unsubstituted aryl (e.g., C₆-C₁₀ aryl, C₁₀ aryl, or phenyl), or R²³-substituted or unsubstituted heteroaryl (e.g., 5 to 10 membered heteroaryl, 5 to 9 membered heteroaryl, or 5 to 6 membered heteroaryl). In embodiments, R² is independently hydrogen.

In embodiments, R² is -CX²₃ (e.g., -CF₃, -CBr₃, -CCl₃, or -CI₃). In embodiments, R² is -CHX²₂ (e.g., -CHF₂, -CHBr₂, -CHCl₂, or -CHI₂). In embodiments, R² is -CH₂X² (e.g., -CH₂F, -CH₂Br, -CH₂Cl, or -CH₂I). In embodiments, R² is -OCX²₃ (e.g., -OCF₃, -OCBr₃, -OCCl₃, or -OCI₃). In embodiments, R² is -OCH₂X² (e.g., -OCH₂F, -OCH₂Br, -OCH₂Cl, or -OCH₂I). In embodiments, R² is -OCHX²₂ (e.g., -OCHF₂, -OCHBr₂, -OCHCl₂, or -OCHI₂). In embodiments, R² is -C(O)R^{2C} (e.g., -COH, -COCH₃, or -COCH₂CH₃). In embodiments, R² is -C(O)OR^{2C} (e.g., -COOH, -COOCH₃, or -COOCH₂CH₃). In embodiments, R² is -C(O)NR^{2A}R^{2B} (e.g., -CONH₂, or -CONHCH₃). In embodiments, R² is -OR^{2D} (e.g., -OH, or -OCH₃).

In embodiments, R² is R²³-substituted or unsubstituted alkyl (e.g., C₁-C₈ alkyl, C₁-C₆ alkyl, or C₁-C₄ alkyl). In embodiments, R² is R²³-substituted alkyl (e.g., C₁-C₈ alkyl, C₁-C₆ alkyl, or C₁-C₄ alkyl). In embodiments, R² is an unsubstituted alkyl (e.g., C₁-C₈ alkyl, C₁-C₆ alkyl, or C₁-C₄ alkyl). In embodiments, R² is R²³-substituted or unsubstituted heteroalkyl (e.g., 2 to 8 membered heteroalkyl, 2 to 6 membered heteroalkyl, or 2 to 4 membered heteroalkyl). In embodiments, R² is R²³-substituted heteroalkyl (e.g., 2 to 8 membered heteroalkyl, 2 to 6 membered heteroalkyl, or 2 to 4 membered heteroalkyl). In embodiments, R² is an unsubstituted heteroalkyl (e.g., 2 to 8 membered heteroalkyl, 2 to 6 membered heteroalkyl, or 2 to 4 membered heteroalkyl).

In embodiments, R² is R²³-substituted or unsubstituted cycloalkyl (e.g., C₃-C₈ cycloalkyl, C₃-C₆ cycloalkyl, or C₅-C₆ cycloalkyl). In embodiments, R² is R²³-substituted cycloalkyl (e.g., C₃-C₈ cycloalkyl, C₃-C₆ cycloalkyl, or C₅-C₆ cycloalkyl). In embodiments, R² is an unsubstituted cycloalkyl (e.g., C₃-C₈ cycloalkyl, C₃-C₆ cycloalkyl, or C₅-C₆ cycloalkyl). In embodiments, R² is R²³-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R² is R²³-substituted heterocycloalkyl (e.g., 3 to 8 membered heterocycloalkyl, 3 to 6 membered heterocycloalkyl, or 5 to 6 membered heterocycloalkyl). In embodiments, R² is an unsubstituted heterocycloalkyl (e.g., 3 to 8 membered heterocycloalkyl, 3 to 6 membered heterocycloalkyl, or 5 to 6 membered heterocycloalkyl).

In embodiments, R² is R²³-substituted or unsubstituted aryl (e.g., C₆-C₁₀ aryl, C₁₀ aryl, or phenyl). In embodiments, R² is R²³-substituted aryl (e.g., C₆-C₁₀ aryl, C₁₀ aryl, or phenyl). In embodiments, R² is an unsubstituted aryl (e.g., C₆-C₁₀ aryl, C₁₀ aryl, or phenyl). In embodiments, R² is R²³-substituted or unsubstituted heteroaryl (e.g., 5 to 10 membered heteroaryl, 5 to 9 membered heteroaryl, or 5 to 6 membered heteroaryl). In embodiments, R² is R²³-substituted heteroaryl (e.g., 5 to 10 membered heteroaryl, 5 to 9 membered heteroaryl, or 5 to 6 membered heteroaryl). In embodiments, R² is an unsubstituted heteroaryl (e.g., 5 to 10 membered heteroaryl, 5 to 9 membered heteroaryl, or 5 to 6 membered heteroaryl).

R²³ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R²⁴-substituted or unsubstituted alkyl (e.g., C₁-C₈ alkyl, C₁-C₆ alkyl, or C₁-C₄ alkyl), R²⁴-substituted or unsubstituted heteroalkyl (e.g., 2 to 8 membered heteroalkyl, 2 to 6 membered heteroalkyl, or 2 to 4 membered heteroalkyl), R²⁴-substituted or unsubstituted cycloalkyl (e.g., C₃-C₈ cycloalkyl, C₃-C₆ cycloalkyl, or C₅-C₆ cycloalkyl), R²⁴-substituted or unsubstituted heterocycloalkyl (e.g., 3 to 8 membered heterocycloalkyl, 3 to 6 membered heterocycloalkyl, or 5 to 6 membered heterocycloalkyl), R²⁴-substituted or unsubstituted aryl (e.g., C₆-C₁₀ aryl, C₁₀ aryl, or phenyl), or R²⁴-substituted or unsubstituted heteroaryl (e.g., 5 to 10 membered heteroaryl, 5 to 9 membered heteroaryl, or 5 to 6 membered heteroaryl). In embodiments, when R²³ is R²⁴-substituted or unsubstituted cycloalkyl (e.g., C₃-C₈ cycloalkyl, C₃-C₆ cycloalkyl, or C₅-C₆ cycloalkyl), R²⁴-substituted or unsubstituted heterocycloalkyl (e.g., 3 to 8 membered heterocycloalkyl, 3 to 6 membered heterocycloalkyl, or 5 to 6 membered heterocycloalkyl), R²⁴-substituted or unsubstituted aryl (e.g., C₆-C₁₀ aryl, C₁₀ aryl, or phenyl), or R²⁴-substituted or unsubstituted heteroaryl (e.g., 5 to 10 membered heteroaryl, 5 to 9 membered heteroaryl, or 5 to 6 membered heteroaryl) it may be referred to herein as (R²⁴)_{z4}- substituted Ring C.

In embodiments, R²³ is independently oxo. In embodiments, R²³ is independently halogen. In embodiments, R²³ is independently -CCl₃. In embodiments, R²³ is independently -CBr₃. In embodiments, R²³ is independently -CF₃. In embodiments, R²³ is independently -CI₃. In embodiments, R²³ is independently CHCl₂. In embodiments, R²³ is independently -CHBr₂. In embodiments, R²³ is independently -CHF₂. In embodiments, R²³ is independently -CHI₂. In embodiments, R²³ is independently -CH₂Cl. In embodiments, R²³ is independently -CH₂Br. In embodiments, R²³ is independently -CH₂F. In embodiments, R²³ is independently -CH₂I. In embodiments, R²³ is independently -CN. In embodiments, R²³ is independently -OH. In embodiments, R²³ is independently -NH₂. In embodiments, R²³ is independently -COOH. In embodiments, R²³ is independently -CONH₂. In embodiments, R²³ is independently -NO₂. In embodiments, R²³ is independently -SH. In embodiments, R²³ is independently -SO₃H. In embodiments, R²³ is independently -SO₄H. In embodiments, R²³ is independently -SO₂NH₂. In embodiments, R²³ is independently -NHNH₂. In embodiments, R²³ is independently -ONH₂. In embodiments, R²³ is independently -NHC(O)NHNH₂. In embodiments, R²³ is independently -NHC(O)NH₂. In embodiments, R²³ is independently -NHSO₂H. In embodiments, R²³ is independently -NHC(O)H. In embodiments, R²³ is independently -NHC(O)OH. In embodiments, R²³ is independently -NHOH. In embodiments, R²³ is independently -OCCl₃. In embodiments, R²³ is independently -OCF₃. In embodiments, R²³ is independently -OCBr₃. In embodiments, R²³ is independently -OCI₃. In embodiments, R²³ is independently -OCHCl₂. In embodiments, R²³ is independently -OCHBr₂. In embodiments, R²³ is independently -OCHI₂. In embodiments, R²³ is independently -OCHF₂. In embodiments, R²³ is independently -OCH₂Cl. In embodiments, R²³ is independently -OCH₂Br. In embodiments, R²³ is independently -OCH₂I. In embodiments, R²³ is independently -OCH₂F. In embodiments, R²³ is independently -N₃.

In embodiments, R²³ is R²⁴-substituted or unsubstituted alkyl (e.g., C₁-C₈ alkyl, C₁-C₆ alkyl, or C₁-C₄ alkyl). In embodiments, R²³ is R²⁴-substituted alkyl (e.g., C₁-C₈ alkyl, C₁-C₆ alkyl, or C₁-C₄ alkyl). In embodiments, R²³ is an unsubstituted alkyl (e.g., C₁-C₈ alkyl, C₁-C₆ alkyl, or C₁-C₄ alkyl).

In embodiments, R²³ is R²⁴-substituted or unsubstituted heteroalkyl (e.g., 2 to 8 membered heteroalkyl, 2 to 6 membered heteroalkyl, or 2 to 4 membered heteroalkyl). In embodiments, R²³ is R²⁴-substituted heteroalkyl (e.g., 2 to 8 membered heteroalkyl, 2 to 6 membered heteroalkyl, or 2 to 4 membered heteroalkyl). In embodiments, R²³ is an unsubstituted heteroalkyl (e.g., 2 to 8 membered heteroalkyl, 2 to 6 membered heteroalkyl, or 2 to 4 membered heteroalkyl).

In embodiments, R²³ is R²⁴-substituted or unsubstituted cycloalkyl (e.g., C₃-C₈ cycloalkyl, C₃-C₆ cycloalkyl, or C₅-C₆ cycloalkyl). In embodiments, R²³ is R²⁴-substituted cycloalkyl (e.g., C₃-C₈ cycloalkyl, C₃-C₆ cycloalkyl, or C₅-C₆ cycloalkyl). In embodiments, R²³ is an unsubstituted cycloalkyl (e.g., C₃-C₈ cycloalkyl, C₃-C₆ cycloalkyl, or C₅-C₆ cycloalkyl).

In embodiments, R²³ is R²⁴-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R²³ is R²⁴-substituted heterocycloalkyl (e.g., 3 to 8 membered heterocycloalkyl, 3 to 6 membered heterocycloalkyl, or 5 to 6 membered heterocycloalkyl). In embodiments, R²³ is an unsubstituted heterocycloalkyl (e.g., 3 to 8 membered heterocycloalkyl, 3 to 6 membered heterocycloalkyl, or 5 to 6 membered heterocycloalkyl).

In embodiments, R²³ is R²⁴-substituted or unsubstituted aryl (e.g., C₆-C₁₀ aryl, C₁₀ aryl, or phenyl). In embodiments, R²³ is R²⁴-substituted aryl (e.g., C₆-C₁₀ aryl, C₁₀ aryl, or phenyl). In embodiments, R²³ is an unsubstituted aryl (e.g., C₆-C₁₀ aryl, C₁₀ aryl, or phenyl). In embodiments, R²³ is R²⁴-substituted phenyl. In embodiments, R²³ is unsubstituted phenyl.

In embodiments, R²³ is R²⁴-substituted or unsubstituted heteroaryl (e.g., 5 to 10 membered heteroaryl, 5 to 9 membered heteroaryl, or 5 to 6 membered heteroaryl). In embodiments, R²³ is R²⁴-substituted heteroaryl (e.g., 5 to 10 membered heteroaryl, 5 to 9 membered heteroaryl, or 5 to 6 membered heteroaryl). In embodiments, R²³ is an unsubstituted heteroaryl (e.g., 5 to 10 membered heteroaryl, 5 to 9 membered heteroaryl, or 5 to 6 membered heteroaryl).

In embodiments, R²³ is R²⁴-substituted or unsubstituted pyridyl. In embodiments, R²³ is R²⁴-substituted pyridyl. In embodiments, R²³ is unsubstituted pyridyl. In embodiments, R²³ is R²⁴-substituted or unsubstituted pyrimidinyl. In embodiments, R²³ is R²⁴-substituted pyrimidinyl. In embodiments, R²³ is unsubstituted pyrimidinyl. In embodiments, R²³ is methoxy. In embodiments, R²³ is R²⁴-substituted or unsubstituted phenyl. In embodiments, R²³ is R²⁴-substituted phenyl. In embodiments, R²³ is unsubstituted phenyl. In embodiments, R²³ is R²⁴-substituted or unsubstituted pyridinazinyl. In embodiments, R²³ is R²⁴-substituted pyridinazinyl. In embodiments, R²⁴ is an unsubstituted pyridinazinyl. In embodiments, R²³ is R²⁴-substituted or unsubsituted morpholinyl. In embodiments, R²³ is R²⁴-substituted morpholinyl. In embodiments, R²³ is unsubstituted morpholinyl. In embodiments, R²³ is R²⁴-substituted tetrahydrofuranyl. In embodiments, R²³ is R²⁴-substituted or unsubstituted tetrahydrofuranyl. In embodiments, R²³ is unsubstituted tetrahydrofuranyl. In embodiments, R²³ is R²⁴-substituted or unsubstituted piperidinyl. In embodiments, R²³ is R²⁴-substituted piperidinyl. In embodiments, R²³ is unsubstituted piperidinyl.

R²⁴ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R²⁵-substituted or unsubstituted alkyl (e.g., C₁-C₈ alkyl, C₁-C₆ alkyl, or C₁-C₄ alkyl), R²⁵-substituted or unsubstituted heteroalkyl (e.g., 2 to 8 membered heteroalkyl, 2 to 6 membered heteroalkyl, or 2 to 4 membered heteroalkyl), R²⁵-substituted or unsubstituted cycloalkyl (e.g., C₃-C₈ cycloalkyl, C₃-C₆ cycloalkyl, or C₅-C₆ cycloalkyl), R²⁵-substituted or unsubstituted heterocycloalkyl (e.g., 3 to 8 membered heterocycloalkyl, 3 to 6 membered heterocycloalkyl, or 5 to 6 membered heterocycloalkyl), R²⁵-substituted or unsubstituted aryl (e.g., C₆-C₁₀ aryl, C₁₀ aryl, or phenyl), or R²⁵-substituted or unsubstituted heteroaryl (e.g., 5 to 10 membered heteroaryl, 5 to 9 membered heteroaryl, or 5 to 6 membered heteroaryl). In embodiments, R²⁴ is independently -OH. In embodiments, R²⁴ is independently -OCH₃. In embodiments, R²⁴ is independently -OCH₂CH₃. In embodiments, R²⁴ is independently -F. In embodiments, R²⁴ is independently -NHC(O)CH₃. In embodiments, R²⁴ is independently -COOH. In embodiments, R²⁴ is independently -SO₂NH₂. In embodiments, R²⁴ is independently - C(O)OCCH₃)₃.

In embodiments, R²⁴ is R²⁵-substituted or unsubstituted heteroaryl (e.g., 5 to 10 membered heteroaryl, 5 to 9 membered heteroaryl, or 5 to 6 membered heteroaryl). In embodiments, R²⁴ is substituted or unsubstituted pyridyl. In embodiments, R²⁴ is R²⁵-substituted pyridyl. In embodiments, R²⁴ is unsubstituted pyridyl. In embodiments, R²⁴ is substituted or unsubstituted pyrimidinyl. In embodiments, R²⁴ is R²⁵-substituted pyrimidinyl. In embodiments, R²⁴ is unsubstituted pyrimidinyl. In embodiments, R²⁴ is methoxy. In embodiments, R²⁴ is substituted or unsubstituted phenyl. In embodiments, R²⁴ is R²⁵-substituted phenyl. In embodiments, R²⁴ is unsubstituted phenyl. In embodiments, R²⁴ is substituted or unsubstituted pyridinazinyl. In embodiments, R²⁴ is R²⁵-substituted pyridinazinyl. In embodiments, R²⁴ is unsubstituted pyridinazinyl.

In embodiments, R² is R²³-substituted or unsubstituted alkyl (e.g., C₁-C₈ alkyl, C₁-C₆ alkyl, or C₁-C₄ alkyl). In embodiments, R² is R²³-substituted alkyl (e.g., C₁-C₈ alkyl, C₁-C₆ alkyl, or C₁-C₄ alkyl). In embodiments, R² is an unsubstituted alkyl (e.g., C₁-C₈ alkyl, C₁-C₆ alkyl, or C₁-C₄ alkyl). In embodiments, R² is R²³-substituted or unsubstituted heteroalkyl (e.g., 2 to 8 membered heteroalkyl, 2 to 6 membered heteroalkyl, or 2 to 4 membered heteroalkyl). In embodiments, R² is R²³-substituted heteroalkyl (e.g., 2 to 8 membered heteroalkyl, 2 to 6 membered heteroalkyl, or 2 to 4 membered heteroalkyl). In embodiments, R² is an unsubstituted heteroalkyl (e.g., 2 to 8 membered heteroalkyl, 2 to 6 membered heteroalkyl, or 2 to 4 membered heteroalkyl). In embodiments, R² is R²³-substituted or unsubstituted cycloalkyl (e.g., C₃-C₈ cycloalkyl, C₃-C₆ cycloalkyl, or C₅-C₆ cycloalkyl). In embodiments, R² is R²³-substituted cycloalkyl (e.g., C₃-C₈ cycloalkyl, C₃-C₆ cycloalkyl, or C₅-C₆ cycloalkyl). In embodiments, R² is an unsubstituted cycloalkyl (e.g., C₃-C₈ cycloalkyl, C₃-C₆ cycloalkyl, or C₅-C₆ cycloalkyl). In embodiments, R² is R²³-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R² is R²³-substituted heterocycloalkyl (e.g., 3 to 8 membered heterocycloalkyl, 3 to 6 membered heterocycloalkyl, or 5 to 6 membered heterocycloalkyl). In embodiments, R² is an unsubstituted heterocycloalkyl (e.g., 3 to 8 membered heterocycloalkyl, 3 to 6 membered heterocycloalkyl, or 5 to 6 membered heterocycloalkyl). In embodiments, R² is R²³-substituted or unsubstituted aryl (e.g., C₆-C₁₀ aryl, C₁₀ aryl, or phenyl). In embodiments, R² is R²³-substituted aryl (e.g., C₆-C₁₀ aryl, C₁₀ aryl, or phenyl). In embodiments, R² is an unsubstituted aryl (e.g., C₆-C₁₀ aryl, C₁₀ aryl, or phenyl). In embodiments, R² is R²³-substituted or unsubstituted heteroaryl (e.g., 5 to 10 membered heteroaryl, 5 to 9 membered heteroaryl, or 5 to 6 membered heteroaryl). In embodiments, R² is R²³-substituted heteroaryl (e.g., 5 to 10 membered heteroaryl, 5 to 9 membered heteroaryl, or 5 to 6 membered heteroaryl). In embodiments, R² is an unsubstituted heteroaryl (e.g., 5 to 10 membered heteroaryl, 5 to 9 membered heteroaryl, or 5 to 6 membered heteroaryl). In embodiments, R² is substituted or

In embodiments, R² is substituted or unsubstituted C₁-C₄ alkyl. In embodiments, R² is substituted C₁-C₄ alkyl. In embodiments, R² is unsubstituted C₁-C₄ alkyl. In embodiments, R² is unsubstituted methyl. In embodiments, R² is unsubstituted ethyl. In embodiments, R² is unsubstituted propyl. In embodiments, R² is unsubstituted isopropyl.

In embodiments, R² is hydroxy-substituted methyl. In embodiments, R² is hydroxy-substituted ethyl. In embodiments, R² is hydroxy-substituted propyl. In embodiments, R² is hydroxy-substituted isopropyl.

In embodiments, R² is substituted or unsubstituted phenyl-substituted methyl. In embodiments, R² is substituted or unsubstituted phenyl-substituted ethyl. In embodiments, R² is substituted or unsubstituted phenyl-substituted propyl. In embodiments, R² is substituted or unsubstituted phenyl-substituted isopropyl.

In embodiments, R² is substituted phenyl-substituted methyl. In embodiments, R² is substituted phenyl-substituted ethyl. In embodiments, R² is substituted phenyl-substituted propyl. In embodiments, R² is substituted phenyl-substituted isopropyl.

In embodiments, R² is hydroxy-substituted phenyl-substituted methyl. In embodiments, R² is hydroxy-substituted phenyl-substituted ethyl. In embodiments, R² is hydroxy-substituted phenyl-substituted propyl. In embodiments, R² is hydroxy-substituted phenyl-substituted isopropyl.

In embodiments, R² is acetamide-substituted phenyl-substituted methyl. In embodiments, R² is acetamide-substituted phenyl-substituted ethyl. In embodiments, R² is acetamide-substituted phenyl-substituted propyl. In embodiments, R² is acetamide-substituted phenyl-substituted isopropyl.

In embodiments, R² is CH₃-S(O)₂NH-substituted phenyl-substituted methyl. In embodiments, R² is CH₃-S(O)₂NH-substituted phenyl-substituted ethyl. In embodiments, R² is CH₃-S(O)₂NH-substituted phenyl-substituted propyl. In embodiments, R² is CH₃-S(O)₂NH-substituted phenyl-substituted isopropyl.

In embodiments, R² is unsubstituted phenyl-substituted methyl. In embodiments, R² is unsubstituted phenyl-substituted ethyl. In embodiments, R² is unsubstituted phenyl-substituted propyl. In embodiments, R² is unsubstituted phenyl-substituted isopropyl.

In embodiments, R² is hydroxy-substituted methyl-substituted phenyl-substituted ethyl. In embodiments, R² is hydroxy-substituted methyl-substituted aryl-substituted ethyl. In embodiments, R² is substituted phenyl-substituted ethyl. In embodiments, R² is substituted aryl-substituted ethyl. In embodiments, R² is substituted phenyl-substituted C₁-C₄ alkyl. In embodiments, R² is substituted aryl-substituted C₁-C₄ alkyl.

In embodiments, R² is Cl-substituted phenyl-substituted ethyl. In embodiments, R² is halo-substituted aryl-substituted ethyl. In embodiments, R² is halo-substituted phenyl-substituted ethyl. In embodiments, R² is Cl-substituted phenyl-substituted C₁-C₄ alkyl. In embodiments, R² is halo-substituted phenyl-substituted C₁-C₄ alkyl. In embodiments, R² is halo-substituted aryl-substituted C₁-C₄ alkyl.

In embodiments, R² is unsubstituted azetidinyl. In embodiments, R² is unsubstituted 3 to 6 membered heterocycloalkyl

In embodiments, R² is methoxy-substituted phenoxy-substituted pyridyl-substituted methyl. In embodiments, R² is 2 to 4 membered alkoxy-substituted phenoxy-substituted pyridyl-substituted methyl. In embodiments, R² is 2 to 4 membered alkoxy-substituted aryloxy-substituted pyridyl-substituted methyl. In embodiments, R² is 2 to 4 membered alkoxy-substituted aryloxy-substituted 5 to 6 membered heteroaryl-substituted methyl. In embodiments, R² is 2 to 4 membered alkoxy-substituted aryloxy-substituted 5 to 6 membered heteroaryl-substituted C₁-C₄ alkyl.

In embodiments, R² is methoxy-substituted pyridyl-substituted ethyl. In embodiments, R² is 2 to 4 membered alkoxy-substituted pyridyl-substituted ethyl. In embodiments, R² is 2 to 4 membered alkoxy-substituted 5 to 6 membered heteroaryl-substituted ethyl. In embodiments, R² is 2 to 4 membered alkoxy-substituted 5 to 6 membered heteroaryl-substituted C₁-C₄ alkyl. In embodiments, R² is 2 to 4 membered alkoxy-substituted heteroaryl-substituted C₁-C₄ alkyl.

In embodiments, R² is methoxy-substituted phenyl-substituted ethyl. In embodiments, R² is 2 to 4 membered alkoxy-substituted phenyl-substituted ethyl. In embodiments, R² is 2 to 4 membered alkoxy-substituted aryl-substituted ethyl. In embodiments, R² is 2 to 4 membered alkoxy-substituted aryl-substituted C₁-C₄ alkyl.

In embodiments, R² is oxo-substituted 2 to 4 membered heteroalkyl-substituted phenyl-substituted ethyl. In embodiments, R² is substituted 2 to 4 membered heteroalkyl-substituted phenyl-substituted ethyl. In embodiments, R² is substituted 2 to 4 membered heteroalkyl-substituted aryl-substituted ethyl. In embodiments, R² is substituted 2 to 4 membered heteroalkyl-substituted aryl-substituted C₁-C₄ alkyl.

In embodiments, R² is methoxy-substituted phenyl-substituted propyl. In embodiments, R² is 2 to 4 membered alkoxy-substituted phenyl-substituted propyl. In embodiments, R² is 2 to 4 membered alkoxy-substituted aryl-substituted propyl. In embodiments, R² is 2 to 4 membered alkoxy-substituted aryl-substituted C₁-C₄ alkyl.

In embodiments, R² is oxo-substituted 2 to 4 membered heteroalkyl-substituted phenyl-substituted ethyl. In embodiments, R² is substituted 2 to 4 membered heteroalkyl-substituted phenyl-substituted ethyl. In embodiments, R² is substituted 2 to 4 membered heteroalkyl-substituted aryl-substituted ethyl. In embodiments, R² is substituted 2 to 4 membered heteroalkyl-substituted aryl-substituted C₁-C₄ alkyl.

In embodiments, R² is substituted or unsubstituted 5 to 6 membered heterocycloalkyl-substituted methyl. In embodiments, R² is substituted or unsubstituted 5 to 6 membered heterocycloalkyl-substituted ethyl. In embodiments, R² is substituted or unsubstituted 5 to 6 membered heterocycloalkyl-substituted propyl. In embodiments, R² is substituted or unsubstituted 5 to 6 membered heterocycloalkyl-substituted isopropyl.

In embodiments, R² is substituted or unsubstituted tetrahydropyranyl-substituted methyl. In embodiments, R² is substituted or unsubstituted tetrahydropyranyl-substituted ethyl. In embodiments, R² is substituted or unsubstituted tetrahydropyranyl-substituted propyl. In embodiments, R² is substituted or unsubstituted tetrahydropyranyl-substituted isopropyl. In embodiments, R² is unsubstituted tetrahydropyranyl-substituted methyl. In embodiments, R² is unsubstituted tetrahydropyranyl-substituted ethyl. In embodiments, R² is unsubstituted tetrahydropyranyl-substituted propyl. In embodiments, R² is unsubstituted tetrahydropyranyl -substituted isopropyl.

In embodiments, R² is substituted or unsubstituted tetrahydrofuranyl-substituted methyl. In embodiments, R² is substituted or unsubstituted tetrahydrofuranyl-substituted ethyl. In embodiments, R² is substituted or unsubstituted tetrahydrofuranyl -substituted propyl. In embodiments, R² is substituted or unsubstituted tetrahydrofuranyl-substituted isopropyl. In embodiments, R² is unsubstituted tetrahydrofuranyl-substituted methyl. In embodiments, R² is unsubstituted tetrahydrofuranyl-substituted ethyl. In embodiments, R² is unsubstituted tetrahydrofuranyl-substituted propyl. In embodiments, R² is unsubstituted tetrahydrofuranyl-substituted isopropyl.

In embodiments, R² is unsubstituted tetrahydrofuranyl-substituted methyl. In embodiments, R² is tetrahydrofuranyl-substituted methyl. In embodiments, R² is unsubstituted 3 to 6 membered heterocycloalkyl-substituted methyl. In embodiments, R² is unsubstituted tetrahydrofuranyl-substituted C₁-C₄ alkyl. In embodiments, R² is tetrahydrofuranyl-substituted C₁-C₄ alkyl.

In embodiments, R² is substituted or unsubstituted piperidinyl-substituted methyl. In embodiments, R² is substituted or unsubstituted piperidinyl-substituted ethyl. In embodiments, R² is substituted or unsubstituted piperidinyl-substituted propyl. In embodiments, R² is substituted or unsubstituted piperidinyl-substituted isopropyl.

In embodiments, R² is tert-butoxycarbonyl(Boc)-substituted piperidinyl-substituted methyl. In embodiments, R² is tert-butoxycarbonyl(Boc)-substituted or unsubstituted piperidinyl-substituted ethyl. In embodiments, R² is tert-butoxycarbonyl(Boc)-substituted or unsubstituted piperidinyl-substituted propyl. In embodiments, R² is tert-butoxycarbonyl(Boc)-substituted or unsubstituted piperidinyl-substituted isopropyl.

In embodiments, R² is unsubstituted piperidinyl-substituted methyl. In embodiments, R² is unsubstituted piperidinyl-substituted ethyl. In embodiments, R² is unsubstituted piperidinyl-substituted propyl. In embodiments, R² is unsubstituted piperidinyl-substituted isopropyl.

In embodiments, R² is substituted or unsubstituted 5 to 6 membered heteroaryl-substituted methyl. In embodiments, R² is substituted or unsubstituted 5 to 6 membered heteroaryl-substituted ethyl. In embodiments, R² is substituted or unsubstituted 5 to 6 membered heteroaryl-substituted or unsubstituted pyridyl-substituted propyl. In embodiments, R² is substituted or unsubstituted 5 to 6 membered heteroaryl-substituted or unsubstituted pyridyl-substituted isopropyl.

In embodiments, R² is substituted or unsubstituted pyridyl-substituted methyl. In embodiments, R² is substituted or unsubstituted pyridyl-substituted ethyl. In embodiments, R² is substituted or unsubstituted pyridyl-substituted propyl. In embodiments, R² is substituted or unsubstituted pyridyl-substituted isopropyl.

In embodiments, R² is unsubstituted pyridyl-substituted methyl. In embodiments, R² is unsubstituted pyridyl-substituted ethyl. In embodiments, R² is unsubstituted pyridyl-substituted propyl. In embodiments, R² is unsubstituted pyridyl-substituted isopropyl.

In embodiments, R² is substituted or unsubstituted 2 to 6 membered heteroalkyl-substituted methyl. In embodiments, R² is substituted or unsubstituted 2 to 6 membered heteroalkyl-substituted ethyl. In embodiments, R² is substituted or unsubstituted 2 to 6 membered heteroalkyl-substituted propyl. In embodiments, R² is substituted or unsubstituted 2 to 6 membered heteroalkyl-substituted isopropyl.

In embodiments, R² is substituted or unsubstituted urea-substituted methyl. In embodiments, R² is substituted or unsubstituted urea-substituted ethyl. In embodiments, R² is substituted or unsubstituted urea-substituted propyl. In embodiments, R² is substituted or unsubstituted urea-substituted isopropyl.

In embodiments, R² is tert-butyl-substituted urea-substituted methyl. In embodiments, R² is tert-butyl-substituted urea-substituted ethyl. In embodiments, R² is tert-butyl-substituted urea-substituted propyl. In embodiments, R² is tert-butyl-substituted urea-substituted isopropyl.

In embodiments, R² is unsubstituted urea-substituted methyl. In embodiments, R² is unsubstituted urea-substituted ethyl. In embodiments, R² is unsubstituted urea-substituted propyl. In embodiments, R² is unsubstituted urea-substituted isopropyl.

In embodiments, R² is unsubstituted cyclopentyl-substituted ethyl. In embodiments, R² is unsubstituted C₃-C₆ cycloalkyl-substituted ethyl. In embodiments, R² is unsubstituted C₃-C₆ cycloalkyl-substituted C₁-C₄ alkyl.

In embodiments, R² is tert-butyloxycarbonyl-substituted pyrrolidinyl-substituted ethyl. In embodiments, R² is oxo-substituted 4 to 8 membered heteroalkyl-substituted pyrrolidinyl-substituted ethyl. In embodiments, R² is substituted 4 to 8 membered heteroalkyl-substituted pyrrolidinyl-substituted ethyl. In embodiments, R² is substituted 4 to 8 membered heteroalkyl-substituted 3 to 6 membered heterocycloalkyl-substituted ethyl. In embodiments, R² is substituted 4 to 8 membered heteroalkyl-substituted 3 to 6 membered heterocycloalkyl-substituted C₁-C₄ alkyl.

In embodiments, R² is tert-butyloxycarbonyl-substituted azetidinyl-substituted ethyl. In embodiments, R² is oxo-substituted 4 to 8 membered heteroalkyl-substituted azetidinyl-substituted ethyl. In embodiments, R² is substituted 4 to 8 membered heteroalkyl-substituted azetidinyl-substituted ethyl. In embodiments, R² is substituted 4 to 8 membered heteroalkyl-substituted azetidinyl -substituted C₁-C₄ alkyl.

In embodiments, R² is unsubstituted azetidinyl-substituted ethyl. In embodiments, R² is unsubstituted C₃-C₆ heterocycloalkyl-substituted ethyl. In embodiments, R² is unsubstituted C₃-C₆ heterocycloalkyl-substituted C₁-C₄ alkyl.

In embodiments, R² is CN-substituted phenyl-substituted ethyl. In embodiments, R² is substituted phenyl-substituted ethyl. In embodiments, R² is CN-substituted aryl-substituted ethyl. In embodiments, R² is CN-substituted aryl-substituted C₁-C₄ alkyl. In embodiments, R² is substituted aryl-substituted C₁-C₄ alkyl.

In embodiments, R² is unsubstituted phenyl-substituted ethyl-substituted piperidinyl-substituted methyl. In embodiments, R² is unsubstituted aryl-substituted ethyl-substituted piperidinyl-substituted methyl. In embodiments, R² is unsubstituted aryl-substituted C₁-C₄ alkyl-substituted piperidinyl-substituted methyl. In embodiments, R² is unsubstituted aryl-substituted C₁-C₄ alkyl-substituted 3 to 6 membered heterocycloalkyl-substituted methyl. In embodiments, R² is unsubstituted aryl-substituted C₁-C₄ alkyl-substituted 3 to 6 membered heterocycloalkyl-substituted C₁-C₄ alkyl.

In embodiments, R² is methoxy-substituted pyridyl-substituted methyl. In embodiments, R² is 2 to 4 membered alkoxy-substituted pyridyl-substituted methyl. In embodiments, R² is 2 to 4 membered alkoxy-substituted 5 to 6 membered heteroaryl-substituted methyl. In embodiments, R² is 2 to 4 membered alkoxy-substituted 5 to 6 membered heteroaryl-substituted C₁-C₄ alkyl.

In embodiments, R² is unsubstituted 2 to 4 membered alkoxy-substituted pyridyl-substituted methyl. In embodiments, R² is unsubstituted 2 to 4 membered alkoxy-substituted 5 to 6 membered heteroaryl-substituted methyl. In embodiments, R² is unsubstituted 2 to 4 membered alkoxy-substituted 5 to 6 membered heteroaryl-substituted C₁-C₄ alkyl.

In embodiments, R² is unsubstituted pyridyl-substituted methyl. In embodiments, R² is unsubstituted 5 to 6 membered heteroaryl-substituted methyl. In embodiments, R² is unsubstituted 5 to 6 membered heteroaryl-substituted C₁-C₄ alkyl.

In embodiments, R² is carboxyl-substituted phenyl-substituted ethyl. In embodiments, R² is substituted phenyl-substituted ethyl. In embodiments, R² is carboxyl-substituted aryl-substituted ethyl. In embodiments, R² is carboxyl-substituted aryl-substituted C₁-C₄ alkyl.

In embodiments, R² is SO₂NH₂-substituted phenyl-substituted ethyl. In embodiments, R² is SO₂NH₂-substituted aryl-substituted ethyl. In embodiments, R² is SO₂NH₂-substituted aryl-substituted C₁-C₄ alkyl.

In embodiments, R² is tert-butylcarbamate-substituted phenyl-substituted ethyl. In embodiments, R² is oxo-substituted 6 to 8 membered heteroalkyl-substituted phenyl-substituted ethyl. In embodiments, R² is substituted 6 to 8 membered heteroalkyl-substituted phenyl-substituted ethyl. In embodiments, R² is substituted 6 to 8 membered heteroalkyl-substituted aryl-substituted ethyl. In embodiments, R² is substituted 6 to 8 membered heteroalkyl-substituted aryl-substituted C₁-C₄ alkyl.

In embodiments, R² is tert-butyloxycarbonyl-substituted piperidinyl-substituted ethyl. In embodiments, R² is oxo-substituted 5 to 7 membered heteroalkyl-substituted piperidinyl-substituted ethyl. In embodiments, R² is substituted 5 to 7 membered heteroalkyl-substituted piperidinyl-substituted ethyl. In embodiments, R² is substituted 5 to 7 membered heteroalkyl-substituted 5 to 6 membered heterocycloalkyl-substituted ethyl. In embodiments, R² is substituted 5 to 7 membered heteroalkyl-substituted 5 to 6 membered heterocycloalkyl-substituted C₁-C₄ alkyl.

In embodiments, R² is unsubstituted thiazolyl-substituted methyl. In embodiments, R² is unsubstituted 5 to 6 membered heteroaryl-substituted methyl. In embodiments, R² is unsubstituted 5 to 6 membered heteroaryl-substituted C₁-C₄ alkyl.

In embodiments, R² is NH2-substituted phenyl-substituted ethyl. In embodiments, R² is NH₂-substituted aryl-substituted ethyl. In embodiments, R² is NH2-substituted aryl-substituted C₁-C₄ alkyl. In embodiments, R² is substituted aryl-substituted C₁-C₄ alkyl.

In embodiments, R² is unsubstituted piperidinyl-substituted ethyl. In embodiments, R² is unsubstituted 5 to 6 membered heterocycloalkyl-substituted ethyl. In embodiments, R² is unsubstituted 5 to 6 membered heterocycloalkyl-substituted C₁-C₄ alkyl.

In embodiments, R² is oxo-substituted 2 to 6 membered heteroalkyl-substituted phenyl-substituted ethyl. In embodiments, R² is substituted 2 to 6 membered heteroalkyl-substituted phenyl-substituted ethyl. In embodiments, R² is substituted 2 to 6 membered heteroalkyl-substituted aryl-substituted ethyl. In embodiments, R² is substituted 2 to 6 membered heteroalkyl-substituted aryl-substituted C₁-C₄ alkyl.

In embodiments, R³ is hydrogen, -CX²₃ (e.g., -CF₃, -CBr₃, -CCl₃, or -CI₃), -CHX²₂ (e.g., -CHF₂, -CHBr₂, -CHCl₂, or -CHI₂), -CH₂X² (e.g., -CH₂F, -CH₂Br, -CH₂Cl, or -CH₂I), -OCX²₃ (e.g., -OCF₃, -OCBr₃, -OCCl₃, or -OCI₃), -OCH₂X² (e.g., -OCH₂F, -OCH₂Br, -OCH₂Cl, or -OCH₂I), -OCHX²₂ (e.g., -OCHF₂, -OCHBr₂, -OCHCl₂, or -OCHI₂), -C(O)R^{2C} (e.g., -COH, -COCH₃, or -COCH₂CH₃), -C(O)OR^{2C} (e.g., -COOH, -COOCH₃, or -COOCH₂CH₃), -C(O)NR^{2A}R^{2B} (e.g., -CONH₂, or -CONHCH₃), -OR^{2D} (e.g., -OH, or -OCH₃), R²³-substituted or unsubstituted alkyl (e.g., C₁-C₈ alkyl, C₁-C₆ alkyl, or C₁-C₄ alkyl), R²³-substituted or unsubstituted heteroalkyl (e.g., 2 to 8 membered heteroalkyl, 2 to 6 membered heteroalkyl, or 2 to 4 membered heteroalkyl), R²³-substituted or unsubstituted cycloalkyl (e.g., C₃-C₈ cycloalkyl, C₃-C₆ cycloalkyl, or C₅-C₆ cycloalkyl), R²³-substituted or unsubstituted heterocycloalkyl (e.g., 3 to 8 membered heterocycloalkyl, 3 to 6 membered heterocycloalkyl, or 5 to 6 membered heterocycloalkyl), R²³-substituted or unsubstituted aryl (e.g., C₆-C₁₀ aryl, C₁₀ aryl, or phenyl), or R²³-substituted or unsubstituted heteroaryl (e.g., 5 to 10 membered heteroaryl, 5 to 9 membered heteroaryl, or 5 to 6 membered heteroaryl). In embodiments, R³ is hydrogen.

In embodiments, R³ is -CX²₃ (e.g., -CF₃, -CBr₃, -CCl₃, or -CI₃). In embodiments, R³ is -CHX²₂ (e.g., -CHF₂, -CHBr₂, -CHCl₂, or -CHI₂). In embodiments, R³ is -CH₂X² (e.g., -CH₂F, -CH₂Br, -CH₂Cl, or -CH₂I). In embodiments, R³ is -OCX²₃ (e.g., -OCF₃, -OCBr₃, -OCCl₃, or -OCI₃). In embodiments, R³ is -OCH₂X² (e.g., -OCH₂F, -OCH₂Br, -OCH₂Cl, or -OCH₂I). In embodiments, R³ is -OCHX²₂ (e.g., -OCHF₂, -OCHBr₂, -OCHCl₂, or -OCHI₂). In embodiments, R³ is -C(O)R^{2C} (e.g., -COH, -COCH₃, or -COCH₂CH₃). In embodiments, R³ is -C(O)OR^{2C} (e.g., -COOH, -COOCH₃, or -COOCH₂CH₃). In embodiments, R³ is -C(O)NR^{2A}R^{2B} (e.g., -CONH₂, or -CONHCH₃). In embodiments, R³ is -OR^{2D} (e.g., -OH, or -OCH₃).

In embodiments, R³ is hydrogen, -CX³₃ (e.g., -CF₃, -CBr₃, -CCl₃, or -CI₃), -CHX³₂ (e.g., -CHF₂, -CHBr₂, -CHCl₂, or -CHI₂), -CH₂X³ (e.g., -CH₂F, -CH₂Br, -CH₂Cl, or -CH₂I), -OCX²₃ (e.g., -OCF₃, -OCBr₃, -OCCl₃, or -OCI₃), -OCH₂X³ (e.g., -OCH₂F, -OCH₂Br, -OCH₂Cl, or -OCH₂I), -OCHX²₂ (e.g., -OCHF₂, -OCHBr₂, -OCHCl₂, or -OCHI₂), -C(O)R^{3C} (e.g., -COH, -COCH₃, or -COCH₂CH₃), -C(O)OR^{3C} (e.g., -COOH, -COOCH₃, or -COOCH₂CH₃), -C(O)NR^{3A}R^{3B} (e.g., -CONH₂, or -CONHCH₃), -OR^{3D} (e.g., -OH, or -OCH₃), R²⁶-substituted or unsubstituted alkyl (e.g., C₁-C₈ alkyl, C₁-C₆ alkyl, or C₁-C₄ alkyl), R²⁶-substituted or unsubstituted heteroalkyl (e.g., 2 to 8 membered heteroalkyl, 2 to 6 membered heteroalkyl, or 2 to 4 membered heteroalkyl), R²⁶-substituted or unsubstituted cycloalkyl (e.g., C₃-C₈ cycloalkyl, C₃-C₆ cycloalkyl, or C₅-C₆ cycloalkyl), R²⁶-substituted or unsubstituted heterocycloalkyl (e.g., 3 to 8 membered heterocycloalkyl, 3 to 6 membered heterocycloalkyl, or 5 to 6 membered heterocycloalkyl), R²⁶-substituted or unsubstituted aryl (e.g., C₆-C₁₀ aryl, C₁₀ aryl, or phenyl), or R²⁶-substituted or unsubstituted heteroaryl (e.g., 5 to 10 membered heteroaryl, 5 to 9 membered heteroaryl, or 5 to 6 membered heteroaryl). In embodiments, R³ is hydrogen.

In embodiments, R³ is -CX³₃ (e.g., -CF₃, -CBr₃, -CCl₃, or -CI₃). In embodiments, R³ is -CHX³₂ (e.g., -CHF₂, -CHBr₂, -CHCl₂, or -CHI₂). In embodiments, R³ is -CH₂X³ (e.g., -CH₂F, -CH₂Br, -CH₂Cl, or -CH₂I). In embodiments, R³ is -OCX³₃ (e.g., -OCF₃, -OCBr₃, -OCCl₃, or -OCI₃). In embodiments, R³ is -OCH₂X³ (e.g., -OCH₂F, -OCH₂Br, -OCH₂Cl, or -OCH₂I). In embodiments, R³ is -OCHX³₂ (e.g., -OCHF₂, -OCHBr₂, -OCHCl₂, or -OCHI₂). In embodiments, R³ is -C(O)R^{3C} (e.g., -COH, -COCH₃, or -COCH₂CH₃). In embodiments, R³ is -C(O)OR^{3C} (e.g., -COOH, -COOCH₃, or -COOCH₂CH₃). In embodiments, R³ is -C(O)NR^{3A}R^{3B} (e.g., -CONH₂, or -CONHCH₃). In embodiments, R³ is - OR^{3D} (e.g., -OH, or -OCH₃).

In embodiments, R³ is R²⁶-substituted or unsubstituted alkyl (e.g., C₁-C₈ alkyl, C₁-C₆ alkyl, or C₁-C₄ alkyl). In embodiments, R³ is R²⁶-substituted alkyl (e.g., C₁-C₈ alkyl, C₁-C₆ alkyl, or C₁-C₄ alkyl). In embodiments, R³ is an unsubstituted alkyl (e.g., C₁-C₈ alkyl, C₁-C₆ alkyl, or C₁-C₄ alkyl). In embodiments, R³ is R²⁶-substituted or unsubstituted heteroalkyl (e.g., 2 to 8 membered heteroalkyl, 2 to 6 membered heteroalkyl, or 2 to 4 membered heteroalkyl). In embodiments, R³ is R²⁶-substituted heteroalkyl (e.g., 2 to 8 membered heteroalkyl, 2 to 6 membered heteroalkyl, or 2 to 4 membered heteroalkyl). In embodiments, R³ is an unsubstituted heteroalkyl (e.g., 2 to 8 membered heteroalkyl, 2 to 6 membered heteroalkyl, or 2 to 4 membered heteroalkyl).

In embodiments, R³ is R²⁶-substituted or unsubstituted cycloalkyl (e.g., C₃-C₈ cycloalkyl, C₃-C₆ cycloalkyl, or C₅-C₆ cycloalkyl). In embodiments, R³ is R²⁶-substituted cycloalkyl (e.g., C₃-C₈ cycloalkyl, C₃-C₆ cycloalkyl, or C₅-C₆ cycloalkyl). In embodiments, R³ is an unsubstituted cycloalkyl (e.g., C₃-C₈ cycloalkyl, C₃-C₆ cycloalkyl, or C₅-C₆ cycloalkyl). In embodiments, R³ is R²⁶-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R³ is R²⁶-substituted heterocycloalkyl (e.g., 3 to 8 membered heterocycloalkyl, 3 to 6 membered heterocycloalkyl, or 5 to 6 membered heterocycloalkyl). In embodiments, R³ is an unsubstituted heterocycloalkyl (e.g., 3 to 8 membered heterocycloalkyl, 3 to 6 membered heterocycloalkyl, or 5 to 6 membered heterocycloalkyl).

In embodiments, R³ is R²⁶-substituted or unsubstituted aryl (e.g., C₆-C₁₀ aryl, C₁₀ aryl, or phenyl). In embodiments, R³ is R²⁶-substituted aryl (e.g., C₆-C₁₀ aryl, C₁₀ aryl, or phenyl). In embodiments, R³ is an unsubstituted aryl (e.g., C₆-C₁₀ aryl, C₁₀ aryl, or phenyl). In embodiments, R³ is R²⁶-substituted or unsubstituted heteroaryl (e.g., 5 to 10 membered heteroaryl, 5 to 9 membered heteroaryl, or 5 to 6 membered heteroaryl). In embodiments, R³ is R²⁶-substituted heteroaryl (e.g., 5 to 10 membered heteroaryl, 5 to 9 membered heteroaryl, or 5 to 6 membered heteroaryl). In embodiments, R³ is an unsubstituted heteroaryl (e.g., 5 to 10 membered heteroaryl, 5 to 9 membered heteroaryl, or 5 to 6 membered heteroaryl).

R²⁶ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R²⁷-substituted or unsubstituted alkyl (e.g., C₁-C₈ alkyl, C₁-C₆ alkyl, or C₁-C₄ alkyl), R²⁷-substituted or unsubstituted heteroalkyl (e.g., 2 to 8 membered heteroalkyl, 2 to 6 membered heteroalkyl, or 2 to 4 membered heteroalkyl), R²⁷-substituted or unsubstituted cycloalkyl (e.g., C₃-C₈ cycloalkyl, C₃-C₆ cycloalkyl, or C₅-C₆ cycloalkyl), R²⁷-substituted or unsubstituted heterocycloalkyl (e.g., 3 to 8 membered heterocycloalkyl, 3 to 6 membered heterocycloalkyl, or 5 to 6 membered heterocycloalkyl), R²⁷-substituted or unsubstituted aryl (e.g., C₆-C₁₀ aryl, C₁₀ aryl, or phenyl), or R²⁷-substituted or unsubstituted heteroaryl (e.g., 5 to 10 membered heteroaryl, 5 to 9 membered heteroaryl, or 5 to 6 membered heteroaryl).

In embodiments, R²⁶ is independently oxo. In embodiments, R²⁶ is independently halogen. In embodiments, R²⁶ is independently -CCl₃. In embodiments, R²⁶ is independently -CBr₃. In embodiments, R²⁶ is independently -CF₃. In embodiments, R²⁶ is independently -CI₃. In embodiments, R²⁶ is independently -CHCl₂. In embodiments, R²⁶ is independently -CHBr₂. In embodiments, R²⁶ is independently -CHF₂. In embodiments, R²⁶ is independently -CHI₂. In embodiments, R²⁶ is independently -CH₂Cl. In embodiments, R²⁶ is independently -CH₂Br. In embodiments, R²⁶ is independently -CH₂F. In embodiments, R²⁶ is independently -CH₂I. In embodiments, R²⁶ is independently -CN. In embodiments, R²⁶ is independently -OH. In embodiments, R²⁶ is independently -NH₂. In embodiments, R²⁶ is independently -COOH. In embodiments, R²⁶ is independently -CONH₂. In embodiments, R²⁶ is independently -NO₂. In embodiments, R²⁶ is independently -SH. In embodiments, R²⁶ is independently -SO₃H. In embodiments, R²⁶ is independently -SO₄H. In embodiments, R²⁶ is independently -SO₂NH₂. In embodiments, R²⁶ is independently -NHNH₂. In embodiments, R²⁶ is independently -ONH₂. In embodiments, R²⁶ is independently -NHC(O)NHNH₂. In embodiments, R²⁶ is independently -NHC(O)NH₂. In embodiments, R²⁶ is independently -NHSO₂H. In embodiments, R²⁶ is independently -NHC(O)H. In embodiments, R²⁶ is independently -NHC(O)OH. In embodiments, R²⁶ is independently -NHOH. In embodiments, R²⁶ is independently -OCCl₃. In embodiments, R²⁶ is independently -OCF₃. In embodiments, R²⁶ is independently -OCBr₃. In embodiments, R²⁶ is independently -OCI₃. In embodiments, R²⁶ is independently -OCHCl₂. In embodiments, R²⁶ is independently -OCHBr₂. In embodiments, R²⁶ is independently -OCHI₂. In embodiments, R²⁶ is independently -OCHF₂. In embodiments, R²⁶ is independently -OCH₂Cl. In embodiments, R²⁶ is independently -OCH₂Br. In embodiments, R²⁶ is independently -OCH₂I. In embodiments, R²⁶ is independently -OCH₂F. In embodiments, R²⁶ is independently -N₃.

In embodiments, R²⁶ is R²⁷-substituted or unsubstituted alkyl (e.g., C₁-C₈ alkyl, C₁-C₆ alkyl, or C₁-C₄ alkyl). In embodiments, R²⁶ is R²⁷-substituted alkyl (e.g., C₁-C₈ alkyl, C₁-C₆ alkyl, or C₁-C₄ alkyl). In embodiments, R²⁶ is an unsubstituted alkyl (e.g., C₁-C₈ alkyl, C₁-C₆ alkyl, or C₁-C₄ alkyl).

In embodiments, R²⁶ is R²⁷-substituted or unsubstituted heteroalkyl (e.g., 2 to 8 membered heteroalkyl, 2 to 6 membered heteroalkyl, or 2 to 4 membered heteroalkyl). In embodiments, R²⁶ is R²⁷-substituted heteroalkyl (e.g., 2 to 8 membered heteroalkyl, 2 to 6 membered heteroalkyl, or 2 to 4 membered heteroalkyl). In embodiments, R²⁶ is an unsubstituted heteroalkyl (e.g., 2 to 8 membered heteroalkyl, 2 to 6 membered heteroalkyl, or 2 to 4 membered heteroalkyl).

In embodiments, R²⁶ is R²⁷-substituted or unsubstituted cycloalkyl (e.g., C₃-C₈ cycloalkyl, C₃-C₆ cycloalkyl, or C₅-C₆ cycloalkyl). In embodiments, R²⁶ is R²⁷-substituted cycloalkyl (e.g., C₃-C₈ cycloalkyl, C₃-C₆ cycloalkyl, or C₅-C₆ cycloalkyl). In embodiments, R²⁶ is an unsubstituted cycloalkyl (e.g., C₃-C₈ cycloalkyl, C₃-C₆ cycloalkyl, or C₅-C₆ cycloalkyl).

In embodiments, R²⁶ is R²⁷-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R²⁶ is R²⁷-substituted heterocycloalkyl (e.g., 3 to 8 membered heterocycloalkyl, 3 to 6 membered heterocycloalkyl, or 5 to 6 membered heterocycloalkyl). In embodiments, R²⁶ is an unsubstituted heterocycloalkyl (e.g., 3 to 8 membered heterocycloalkyl, 3 to 6 membered heterocycloalkyl, or 5 to 6 membered heterocycloalkyl).

In embodiments, R²⁶ is R²⁷-substituted or unsubstituted aryl (e.g., C₆-C₁₀ aryl, C₁₀ aryl, or phenyl). In embodiments, R²⁶ is R²⁷-substituted aryl (e.g., C₆-C₁₀ aryl, C₁₀ aryl, or phenyl). In embodiments, R²⁶ is an unsubstituted aryl (e.g., C₆-C₁₀ aryl, C₁₀ aryl, or phenyl).

In embodiments, R²⁶ is R²⁷-substituted or unsubstituted heteroaryl (e.g., 5 to 10 membered heteroaryl, 5 to 9 membered heteroaryl, or 5 to 6 membered heteroaryl). In embodiments, R²⁶ is R²⁷-substituted heteroaryl (e.g., 5 to 10 membered heteroaryl, 5 to 9 membered heteroaryl, or 5 to 6 membered heteroaryl). In embodiments, R²⁶ is an unsubstituted heteroaryl (e.g., 5 to 10 membered heteroaryl, 5 to 9 membered heteroaryl, or 5 to 6 membered heteroaryl).

In embodiments, R²⁶ is R²⁷-substituted or unsubstituted pyridyl. In embodiments, R²⁶ is R²⁷-substituted pyridyl. In embodiments, R²⁶ is unsubstituted pyridyl. In embodiments, R²⁶ is R²⁷-substituted or unsubstituted pyrimidinyl. In embodiments, R²⁶ is R²⁷-substituted pyrimidinyl. In embodiments, R²⁶ is unsubstituted pyrimidinyl. In embodiments, R²⁶ is methoxy. In embodiments, R²⁶ is R²⁷-substituted or unsubstituted phenyl. In embodiments, R²⁶ is R²⁷-substituted phenyl. In embodiments, R²⁶ is unsubstituted phenyl. In embodiments, R²⁶ is R²⁷-substituted or unsubstituted pyridinazinyl. In embodiments, R²⁶ is R²⁷-substituted pyridinazinyl. In embodiments, R²⁷ is an unsubstituted pyridinazinyl. In embodiments, R²⁶ is R²⁷-substituted or unsubsituted morpholinyl. In embodiments, R²⁶ is R²⁷-substituted morpholinyl. In embodiments, R²⁶ is unsubstituted morpholinyl. In embodiments, R²⁶ is R²⁷-substituted tetrahydrofuranyl. In embodiments, R²⁶ is R²⁷-substituted or unsubstituted tetrahydrofuranyl. In embodiments, R²⁶ is unsubstituted tetrahydrofuranyl. In embodiments, R²⁶ is R²⁷-substituted or unsubstituted piperidinyl. In embodiments, R²⁶ is R²⁷-substituted piperidinyl. In embodiments, R²⁶ is unsubstituted piperidinyl.

R²⁷ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R²⁸-substituted or unsubstituted alkyl (e.g., C₁-C₈ alkyl, C₁-C₆ alkyl, or C₁-C₄ alkyl), R²⁸-substituted or unsubstituted heteroalkyl (e.g., 2 to 8 membered heteroalkyl, 2 to 6 membered heteroalkyl, or 2 to 4 membered heteroalkyl), R²⁸-substituted or unsubstituted cycloalkyl (e.g., C₃-C₈ cycloalkyl, C₃-C₆ cycloalkyl, or C₅-C₆ cycloalkyl), R²⁸-substituted or unsubstituted heterocycloalkyl (e.g., 3 to 8 membered heterocycloalkyl, 3 to 6 membered heterocycloalkyl, or 5 to 6 membered heterocycloalkyl), R²⁸-substituted or unsubstituted aryl (e.g., C₆-C₁₀ aryl, C₁₀ aryl, or phenyl), or R²⁸-substituted or unsubstituted heteroaryl (e.g., 5 to 10 membered heteroaryl, 5 to 9 membered heteroaryl, or 5 to 6 membered heteroaryl). In embodiments, R²⁷ is independently -OH. In embodiments, R²⁷ is independently -OCH₃. In embodiments, R²⁷ is independently -OCH₂CH₃. In embodiments, R²⁷ is independently -F. In embodiments, R²⁷ is independently -NHC(O)CH₃. In embodiments, R²⁷ is independently - COOH. In embodiments, R²⁷ is independently -SO₂NH₂.

In embodiments, R² and R³ may optionally be joined to form a substituted or unsubstituted heterocycloalkyl (e.g., 3 to 8 membered heterocycloalkyl, 3 to 6 membered heterocycloalkyl, or 5 to 6 membered heterocycloalkyl) which may be referred to herein as "Ring B". In embodiments, R² and R³ may optionally be joined to form a substituted or unsubstituted 3 to 8 membered heterocycloalkyl. In embodiments, R² and R³ may optionally be joined to form a substituted or unsubstituted 3 membered heterocycloalkyl. In embodiments, R² and R³ may optionally be joined to form a substituted or unsubstituted 4 membered heterocycloalkyl. In embodiments, R² and R³ may optionally be joined to form a substituted or unsubstituted 5 membered heterocycloalkyl. In embodiments, R² and R³ may optionally be joined to form a substituted or unsubstituted 6 membered heterocycloalkyl. In embodiments, R² and R³ may optionally be joined to form a substituted or unsubstituted 7 membered heterocycloalkyl. In embodiments, R² and R³ may optionally be joined to form a substituted or unsubstituted 8 membered heterocycloalkyl.

In embodiments, R² and R³ may optionally be joined to form an R⁷-substituted or unsubstituted heterocycloalkyl (e.g., 3 to 8 membered heterocycloalkyl, 3 to 6 membered heterocycloalkyl, or 5 to 6 membered heterocycloalkyl) which may be referred to herein as "Ring B". In embodiments, R² and R³ may optionally be joined to form an R⁷-substituted or unsubstituted 3 to 8 membered heterocycloalkyl. In embodiments, R² and R³ may optionally be joined to form an R⁷-substituted or unsubstituted 3 membered heterocycloalkyl. In embodiments, R² and R³ may optionally be joined to form an R⁷-substituted or unsubstituted 4 membered heterocycloalkyl. In embodiments, R² and R³ may optionally be joined to form an R⁷-substituted or unsubstituted 5 membered heterocycloalkyl. In embodiments, R² and R³ may optionally be joined to form an R⁷-substituted or unsubstituted 6 membered heterocycloalkyl. In embodiments, R² and R³ may optionally be joined to form an R⁷-substituted or unsubstituted 7 membered heterocycloalkyl. In embodiments, R² and R³ may optionally be joined to form an R⁷-substituted or unsubstituted 8 membered heterocycloalkyl.

In embodiments, R² and R³ may optionally be joined to form an R²³-substituted or unsubstituted heterocycloalkyl (e.g., 3 to 8 membered heterocycloalkyl, 3 to 6 membered heterocycloalkyl, or 5 to 6 membered heterocycloalkyl) which may be referred to herein as "Ring B". In embodiments, R² and R³ may optionally be joined to form an R²³-substituted or unsubstituted 3 to 8 membered heterocycloalkyl. In embodiments, R² and R³ may optionally be joined to form an R²³-substituted or unsubstituted 3 membered heterocycloalkyl. In embodiments, R² and R³ may optionally be joined to form an R²³-substituted or unsubstituted 4 membered heterocycloalkyl. In embodiments, R² and R³ may optionally be joined to form an R²³-substituted or unsubstituted 5 membered heterocycloalkyl. In embodiments, R² and R³ may optionally be joined to form an R²³-substituted or unsubstituted 6 membered heterocycloalkyl. In embodiments, R² and R³ may optionally be joined to form an R²³-substituted or unsubstituted 7 membered heterocycloalkyl. In embodiments, R² and R³ may optionally be joined to form an R²³-substituted or unsubstituted 8 membered heterocycloalkyl.

In embodiments, R² and R³ may optionally be joined to form an R²⁶-substituted or unsubstituted heterocycloalkyl (e.g., 3 to 8 membered heterocycloalkyl, 3 to 6 membered heterocycloalkyl, or 5 to 6 membered heterocycloalkyl) which may be referred to herein as "Ring B". In embodiments, R² and R³ may optionally be joined to form an R²⁶-substituted or unsubstituted 3 to 8 membered heterocycloalkyl. In embodiments, R² and R³ may optionally be joined to form an R²⁶-substituted or unsubstituted 3 membered heterocycloalkyl. In embodiments, R² and R³ may optionally be joined to form an R²⁶-substituted or unsubstituted 4 membered heterocycloalkyl. In embodiments, R² and R³ may optionally be joined to form an R²⁶-substituted or unsubstituted 5 membered heterocycloalkyl. In embodiments, R² and R³ may optionally be joined to form an R²⁶-substituted or unsubstituted 6 membered heterocycloalkyl. In embodiments, R² and R³ may optionally be joined to form an R²⁶-substituted or unsubstituted 7 membered heterocycloalkyl. In embodiments, R² and R³ may optionally be joined to form an R²⁶-substituted or unsubstituted 8 membered heterocycloalkyl.

In embodiments, R³ is substituted or unsubstituted C₁-C₄ alkyl. In embodiments, R³ is substituted C₁-C₄ alkyl. In embodiments, R³ is unsubstituted C₁-C₄ alkyl. In embodiments, R³ is unsubstituted methyl. In embodiments, R³ is unsubstituted ethyl. In embodiments, R³ is unsubstituted propyl. In embodiments, R³ is unsubstituted isopropyl.

In embodiments, R³ is hydroxy-substituted methyl. In embodiments, R³ is hydroxy-substituted ethyl. In embodiments, R³ is hydroxy-substituted propyl. In embodiments, R³ is hydroxy-substituted isopropyl.

In embodiments, R³ is substituted or unsubstituted phenyl-substituted methyl. In embodiments, R³ is substituted or unsubstituted phenyl-substituted ethyl. In embodiments, R³ is substituted or unsubstituted phenyl-substituted propyl. In embodiments, R³ is substituted or unsubstituted phenyl-substituted isopropyl.

In embodiments, R³ is substituted phenyl-substituted methyl. In embodiments, R³ is substituted phenyl-substituted ethyl. In embodiments, R³ is substituted phenyl-substituted propyl. In embodiments, R³ is substituted phenyl-substituted isopropyl.

In embodiments, R³ is hydroxy-substituted phenyl-substituted methyl. In embodiments, R³ is hydroxy-substituted phenyl-substituted ethyl. In embodiments, R³ is hydroxy-substituted phenyl-substituted propyl. In embodiments, R³ is hydroxy-substituted phenyl-substituted isopropyl.

In embodiments, R³ is acetamide-substituted phenyl-substituted methyl. In embodiments, R³ is acetamide-substituted phenyl-substituted ethyl. In embodiments, R³ is acetamide-substituted phenyl-substituted propyl. In embodiments, R³ is acetamide-substituted phenyl-substituted isopropyl.

In embodiments, R³ is CH₃-S(O)₂NH-substituted phenyl-substituted methyl. In embodiments, R³ is CH₃-S(O)₂NH-substituted phenyl-substituted ethyl. In embodiments, R³ is CH₃-S(O)₂NH-substituted phenyl-substituted propyl. In embodiments, R³ is CH₃-S(O)₂NH-substituted phenyl-substituted isopropyl.

In embodiments, R³ is unsubstituted phenyl-substituted methyl. In embodiments, R³ is unsubstituted phenyl-substituted ethyl. In embodiments, R³ is unsubstituted phenyl-substituted propyl. In embodiments, R³ is unsubstituted phenyl-substituted isopropyl.

In embodiments, R³ is hydroxy-substituted methyl-substituted phenyl-substituted ethyl. In embodiments, R³ is hydroxy -substituted methyl-substituted aryl-substituted ethyl. In embodiments, R³ is substituted phenyl-substituted ethyl. In embodiments, R³ is substituted aryl-substituted ethyl. In embodiments, R³ is substituted phenyl-substituted C₁-C₄ alkyl. In embodiments, R³ is substituted aryl-substituted C₁-C₄ alkyl.

In embodiments, R³ is Cl-substituted phenyl-substituted ethyl. In embodiments, R³ is halo-substituted aryl-substituted ethyl. In embodiments, R³ is halo-substituted phenyl-substituted ethyl. In embodiments, R³ is Cl-substituted phenyl-substituted C₁-C₄ alkyl. In embodiments, R³ is halo-substituted phenyl-substituted C₁-C₄ alkyl. In embodiments, R³ is halo-substituted aryl-substituted C₁-C₄ alkyl.

In embodiments, R³ is unsubstituted azetidinyl. In embodiments, R³ is unsubstituted 3 to 6 membered heterocycloalkyl

In embodiments, R³ is methoxy-substituted phenoxy-substituted pyridyl-substituted methyl. In embodiments, R³ is 2 to 4 membered alkoxy-substituted phenoxy-substituted pyridyl-substituted methyl. In embodiments, R³ is 2 to 4 membered alkoxy-substituted aryloxy-substituted pyridyl-substituted methyl. In embodiments, R³ is 2 to 4 membered alkoxy-substituted aryloxy-substituted 5 to 6 membered heteroaryl-substituted methyl. In embodiments, R³ is 2 to 4 membered alkoxy-substituted aryloxy-substituted 5 to 6 membered heteroaryl-substituted C₁-C₄ alkyl.

In embodiments, R³ is methoxy-substituted pyridyl-substituted ethyl. In embodiments, R³ is 2 to 4 membered alkoxy-substituted pyridyl-substituted ethyl. In embodiments, R³ is 2 to 4 membered alkoxy-substituted 5 to 6 membered heteroaryl-substituted ethyl. In embodiments, R³ is 2 to 4 membered alkoxy-substituted 5 to 6 membered heteroaryl-substituted C₁-C₄ alkyl. In embodiments, R³ is 2 to 4 membered alkoxy-substituted heteroaryl-substituted C₁-C₄ alkyl.

In embodiments, R³ is methoxy-substituted phenyl-substituted ethyl. In embodiments, R³ is 2 to 4 membered alkoxy-substituted phenyl-substituted ethyl. In embodiments, R³ is 2 to 4 membered alkoxy-substituted aryl-substituted ethyl. In embodiments, R³ is 2 to 4 membered alkoxy-substituted aryl-substituted C₁-C₄ alkyl.

In embodiments, R³ is oxo-substituted 2 to 4 membered heteroalkyl-substituted phenyl-substituted ethyl. In embodiments, R³ is substituted 2 to 4 membered heteroalkyl-substituted phenyl-substituted ethyl. In embodiments, R³ is substituted 2 to 4 membered heteroalkyl-substituted aryl-substituted ethyl. In embodiments, R³ is substituted 2 to 4 membered heteroalkyl-substituted aryl-substituted C₁-C₄ alkyl.

In embodiments, R³ is methoxy-substituted phenyl-substituted propyl. In embodiments, R³ is 2 to 4 membered alkoxy-substituted phenyl-substituted propyl. In embodiments, R³ is 2 to 4 membered alkoxy-substituted aryl-substituted propyl. In embodiments, R³ is 2 to 4 membered alkoxy-substituted aryl-substituted C₁-C₄ alkyl.

In embodiments, R³ is oxo-substituted 2 to 4 membered heteroalkyl-substituted phenyl-substituted ethyl. In embodiments, R³ is substituted 2 to 4 membered heteroalkyl-substituted phenyl-substituted ethyl. In embodiments, R³ is substituted 2 to 4 membered heteroalkyl-substituted aryl-substituted ethyl. In embodiments, R³ is substituted 2 to 4 membered heteroalkyl-substituted aryl-substituted C₁-C₄ alkyl.

In embodiments, R³ is substituted or unsubstituted 5 to 6 membered heterocycloalkyl-substituted methyl. In embodiments, R³ is substituted or unsubstituted 5 to 6 membered heterocycloalkyl-substituted ethyl. In embodiments, R³ is substituted or unsubstituted 5 to 6 membered heterocycloalkyl-substituted propyl. In embodiments, R³ is substituted or unsubstituted 5 to 6 membered heterocycloalkyl-substituted isopropyl.

In embodiments, R³ is substituted or unsubstituted tetrahydropyranyl-substituted methyl. In embodiments, R³ is substituted or unsubstituted tetrahydropyranyl-substituted ethyl. In embodiments, R³ is substituted or unsubstituted tetrahydropyranyl-substituted propyl. In embodiments, R³ is substituted or unsubstituted tetrahydropyranyl-substituted isopropyl. In embodiments, R³ is unsubstituted tetrahydropyranyl-substituted methyl. In embodiments, R³ is unsubstituted tetrahydropyranyl-substituted ethyl. In embodiments, R³ is unsubstituted tetrahydropyranyl-substituted propyl. In embodiments, R³ is unsubstituted tetrahydropyranyl -substituted isopropyl.

In embodiments, R³ is substituted or unsubstituted tetrahydrofuranyl-substituted methyl. In embodiments, R³ is substituted or unsubstituted tetrahydrofuranyl-substituted ethyl. In embodiments, R³ is substituted or unsubstituted tetrahydrofuranyl -substituted propyl. In embodiments, R³ is substituted or unsubstituted tetrahydrofuranyl-substituted isopropyl. In embodiments, R³ is unsubstituted tetrahydrofuranyl-substituted methyl. In embodiments, R³ is unsubstituted tetrahydrofuranyl-substituted ethyl. In embodiments, R³ is unsubstituted tetrahydrofuranyl-substituted propyl. In embodiments, R³ is unsubstituted tetrahydrofuranyl-substituted isopropyl.

In embodiments, R³ is unsubstituted tetrahydrofuranyl-substituted methyl. In embodiments, R³ is tetrahydrofuranyl-substituted methyl. In embodiments, R³ is unsubstituted 3 to 6 membered heterocycloalkyl-substituted methyl. In embodiments, R³ is unsubstituted tetrahydrofuranyl-substituted C₁-C₄ alkyl. In embodiments, R³ is tetrahydrofuranyl-substituted C₁-C₄ alkyl.

In embodiments, R³ is substituted or unsubstituted piperidinyl-substituted methyl. In embodiments, R³ is substituted or unsubstituted piperidinyl-substituted ethyl. In embodiments, R³ is substituted or unsubstituted piperidinyl-substituted propyl. In embodiments, R³ is substituted or unsubstituted piperidinyl-substituted isopropyl.

In embodiments, R³ is tert-butoxycarbonyl(Boc)-substituted piperidinyl-substituted methyl. In embodiments, R³ is tert-butoxycarbonyl(Boc)-substituted or unsubstituted piperidinyl-substituted ethyl. In embodiments, R³ is tert-butoxycarbonyl(Boc)-substituted or unsubstituted piperidinyl-substituted propyl. In embodiments, R³ is tert-butoxycarbonyl(Boc)-substituted or unsubstituted piperidinyl-substituted isopropyl.

In embodiments, R³ is unsubstituted piperidinyl-substituted methyl. In embodiments, R³ is unsubstituted piperidinyl-substituted ethyl. In embodiments, R³ is unsubstituted piperidinyl-substituted propyl. In embodiments, R³ is unsubstituted piperidinyl-substituted isopropyl.

In embodiments, R³ is substituted or unsubstituted 5 to 6 membered heteroaryl-substituted methyl. In embodiments, R³ is substituted or unsubstituted 5 to 6 membered heteroaryl-substituted ethyl. In embodiments, R³ is substituted or unsubstituted 5 to 6 membered heteroaryl-substituted or unsubstituted pyridyl-substituted propyl. In embodiments, R³ is substituted or unsubstituted 5 to 6 membered heteroaryl-substituted or unsubstituted pyridyl-substituted isopropyl.

In embodiments, R³ is substituted or unsubstituted pyridyl-substituted methyl. In embodiments, R³ is substituted or unsubstituted pyridyl-substituted ethyl. In embodiments, R³ is substituted or unsubstituted pyridyl-substituted propyl. In embodiments, R³ is substituted or unsubstituted pyridyl-substituted isopropyl.

In embodiments, R³ is unsubstituted pyridyl-substituted methyl. In embodiments, R³ is unsubstituted pyridyl-substituted ethyl. In embodiments, R³ is unsubstituted pyridyl-substituted propyl. In embodiments, R³ is unsubstituted pyridyl-substituted isopropyl.

In embodiments, R³ is substituted or unsubstituted 2 to 6 membered heteroalkyl-substituted methyl. In embodiments, R³ is substituted or unsubstituted 2 to 6 membered heteroalkyl-substituted ethyl. In embodiments, R³ is substituted or unsubstituted 2 to 6 membered heteroalkyl-substituted propyl. In embodiments, R³ is substituted or unsubstituted 2 to 6 membered heteroalkyl-substituted isopropyl.

In embodiments, R³ is substituted or unsubstituted urea-substituted methyl. In embodiments, R³ is substituted or unsubstituted urea-substituted ethyl. In embodiments, R³ is substituted or unsubstituted urea-substituted propyl. In embodiments, R³ is substituted or unsubstituted urea-substituted isopropyl.

In embodiments, R³ is tert-butyl-substituted urea-substituted methyl. In embodiments, R³ is tert-butyl-substituted urea-substituted ethyl. In embodiments, R³ is tert-butyl-substituted urea-substituted propyl. In embodiments, R³ is tert-butyl-substituted urea-substituted isopropyl.

In embodiments, R³ is unsubstituted urea-substituted methyl. In embodiments, R³ is unsubstituted urea-substituted ethyl. In embodiments, R³ is unsubstituted urea-substituted propyl. In embodiments, R³ is unsubstituted urea-substituted isopropyl.

In embodiments, R³ is unsubstituted cyclopentyl-substituted ethyl. In embodiments, R³ is unsubstituted C₃-C₆ cycloalkyl-substituted ethyl. In embodiments, R³ is unsubstituted C₃-C₆ cycloalkyl-substituted C₁-C₄ alkyl.

In embodiments, R³ is tert-butyloxycarbonyl-substituted pyrrolidinyl-substituted ethyl. In embodiments, R³ is oxo-substituted 4 to 8 membered heteroalkyl-substituted pyrrolidinyl-substituted ethyl. In embodiments, R³ is substituted 4 to 8 membered heteroalkyl-substituted pyrrolidinyl-substituted ethyl. In embodiments, R³ is substituted 4 to 8 membered heteroalkyl-substituted 3 to 6 membered heterocycloalkyl-substituted ethyl. In embodiments, R³ is substituted 4 to 8 membered heteroalkyl-substituted 3 to 6 membered heterocycloalkyl-substituted C₁-C₄ alkyl.

In embodiments, R³ is tert-butyloxycarbonyl-substituted azetidinyl-substituted ethyl. In embodiments, R³ is oxo-substituted 4 to 8 membered heteroalkyl-substituted azetidinyl-substituted ethyl. In embodiments, R³ is substituted 4 to 8 membered heteroalkyl-substituted azetidinyl-substituted ethyl. In embodiments, R³ is substituted 4 to 8 membered heteroalkyl-substituted azetidinyl-substituted C₁-C₄ alkyl.

In embodiments, R³ is unsubstituted azetidinyl-substituted ethyl. In embodiments, R³ is unsubstituted 3 to 6 membered heterocycloalkyl-substituted ethyl. In embodiments, R³ is unsubstituted 3 to 6 membered heterocycloalkyl-substituted C₁-C₄ alkyl.

In embodiments, R³ is unsubstituted azetidinyl-substituted ethyl. In embodiments, R³ is unsubstituted C₃-C₆ heterocycloalkyl-substituted ethyl. In embodiments, R³ is unsubstituted C₃-C₆ heterocycloalkyl-substituted C₁-C₄ alkyl.

In embodiments, R³ is CN-substituted phenyl-substituted ethyl. In embodiments, R³ is substituted phenyl-substituted ethyl. In embodiments, R³ is CN-substituted aryl-substituted ethyl. In embodiments, R³ is CN-substituted aryl-substituted C₁-C₄ alkyl. In embodiments, R³ is substituted aryl-substituted C₁-C₄ alkyl.

In embodiments, R³ is unsubstituted phenyl-substituted ethyl-substituted piperidinyl-substituted methyl. In embodiments, R³ is unsubstituted aryl-substituted ethyl-substituted piperidinyl-substituted methyl. In embodiments, R³ is unsubstituted aryl-substituted C₁-C₄ alkyl-substituted piperidinyl-substituted methyl. In embodiments, R³ is unsubstituted aryl-substituted C₁-C₄ alkyl-substituted 3 to 6 membered heterocycloalkyl-substituted methyl. In embodiments, R³ is unsubstituted aryl-substituted C₁-C₄ alkyl-substituted 3 to 6 membered heterocycloalkyl-substituted C₁-C₄ alkyl.

In embodiments, R³ is methoxy-substituted pyridyl-substituted methyl. In embodiments, R³ is 2 to 4 membered alkoxy-substituted pyridyl-substituted methyl. In embodiments, R³ is 2 to 4 membered alkoxy-substituted 5 to 6 membered heteroaryl-substituted methyl. In embodiments, R³ is 2 to 4 membered alkoxy-substituted 5 to 6 membered heteroaryl-substituted C₁-C₄ alkyl.

In embodiments, R³ is unsubstituted 2 to 4 membered alkoxy-substituted pyridyl-substituted methyl. In embodiments, R³ is unsubstituted 2 to 4 membered alkoxy-substituted 5 to 6 membered heteroaryl-substituted methyl. In embodiments, R³ is unsubstituted 2 to 4 membered alkoxy-substituted 5 to 6 membered heteroaryl-substituted C₁-C₄ alkyl.

In embodiments, R³ is unsubstituted pyridyl-substituted methyl. In embodiments, R³ is unsubstituted 5 to 6 membered heteroaryl-substituted methyl. In embodiments, R³ is unsubstituted 5 to 6 membered heteroaryl-substituted C₁-C₄ alkyl.

In embodiments, R³ is carboxyl-substituted phenyl-substituted ethyl. In embodiments, R³ is substituted phenyl-substituted ethyl. In embodiments, R³ is carboxyl-substituted aryl-substituted ethyl. In embodiments, R³ is carboxyl-substituted aryl-substituted C₁-C₄ alkyl.

In embodiments, R³ is SO₂NH₂-substituted phenyl-substituted ethyl. In embodiments, R³ is SO₂NH₂-substituted aryl-substituted ethyl. In embodiments, R³ is SO₂NH₂-substituted aryl-substituted C₁-C₄ alkyl.

In embodiments, R³ is tert-butylcarbamate-substituted phenyl-substituted ethyl. In embodiments, R³ is oxo-substituted 6 to 8 membered heteroalkyl-substituted phenyl-substituted ethyl. In embodiments, R³ is substituted 6 to 8 membered heteroalkyl-substituted phenyl-substituted ethyl. In embodiments, R³ is substituted 6 to 8 membered heteroalkyl-substituted aryl-substituted ethyl. In embodiments, R³ is substituted 6 to 8 membered heteroalkyl-substituted aryl-substituted C₁-C₄ alkyl.

In embodiments, R³ is tert-butyloxycarbonyl-substituted piperidinyl-substituted ethyl. In embodiments, R³ is oxo-substituted 5 to 7 membered heteroalkyl-substituted piperidinyl-substituted ethyl. In embodiments, R³ is substituted 5 to 7 membered heteroalkyl-substituted piperidinyl-substituted ethyl. In embodiments, R³ is substituted 5 to 7 membered heteroalkyl-substituted 5 to 6 membered heterocycloalkyl-substituted ethyl. In embodiments, R³ is substituted 5 to 7 membered heteroalkyl-substituted 5 to 6 membered heterocycloalkyl-substituted C₁-C₄ alkyl.

In embodiments, R³ is unsubstituted thiazolyl-substituted methyl. In embodiments, R³ is unsubstituted 5 to 6 membered heteroaryl-substituted methyl. In embodiments, R³ is unsubstituted 5 to 6 membered heteroaryl-substituted C₁-C₄ alkyl.

In embodiments, R³ is NH₂-substituted phenyl-substituted ethyl. In embodiments, R³ is NH₂-substituted aryl-substituted ethyl. In embodiments, R³ is NH₂-substituted aryl-substituted C₁-C₄ alkyl. In embodiments, R³ is substituted aryl-substituted C₁-C₄ alkyl.

In embodiments, R³ is unsubstituted piperidinyl-substituted ethyl. In embodiments, R³ is unsubstituted 5 to 6 membered heterocycloalkyl-substituted ethyl. In embodiments, R³ is unsubstituted 5 to 6 membered heterocycloalkyl-substituted C₁-C₄ alkyl.

In embodiments, R³ is oxo-substituted 2 to 6 membered heteroalkyl-substituted phenyl-substituted ethyl. In embodiments, R³ is substituted 2 to 6 membered heteroalkyl-substituted phenyl-substituted ethyl. In embodiments, R³ is substituted 2 to 6 membered heteroalkyl-substituted aryl-substituted ethyl. In embodiments, R³ is substituted 2 to 6 membered heteroalkyl-substituted aryl-substituted C₁-C₄ alkyl.

In embodiments, R⁴ is hydrogen, halogen (e.g., -F, -Cl, -Br, or -I), -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂,
-NHC(O)NH₂, -NHSO₂H, -NHC(O)H, -NHC(O)OH, -NHOH, -OCCl₃, -OCF₃, -OCBr₃, -OC I₃,
-OCHCl₂, -OCHBr₂, -OCHI₂, -OCHF₂, -OCH₂Cl, -OCH₂Br, -OCH₂I, -OCH₂F, -N₃, R²⁹-substituted or unsubstituted alkyl (e.g., C₁-C₈ alkyl, C₁-C₆ alkyl, or C₁-C₄ alkyl), R²⁹-substituted or unsubstituted heteroalkyl (e.g., 2 to 8 membered heteroalkyl, 2 to 6 membered heteroalkyl, or 2 to 4 membered heteroalkyl), R²⁹-substituted or unsubstituted cycloalkyl (e.g., C₃-C₈ cycloalkyl, C₃-C₆ cycloalkyl, or C₅-C₆ cycloalkyl), R²⁹-substituted or unsubstituted heterocycloalkyl (e.g., 3 to 8 membered heterocycloalkyl, 3 to 6 membered heterocycloalkyl, or 5 to 6 membered heterocycloalkyl), R²⁹-substituted or unsubstituted aryl (e.g., C₆-C₁₀ aryl, C₁₀ aryl, or phenyl), or R²⁹-substituted or unsubstituted heteroaryl (e.g., 5 to 10 membered heteroaryl, 5 to 9 membered heteroaryl, or 5 to 6 membered heteroaryl). In embodiments, R⁴ is hydrogen.

In embodiments, R⁴ is R²⁹-substituted or unsubstituted alkyl (e.g., C₁-C₈ alkyl, C₁-C₆ alkyl, or C₁-C₄ alkyl). In embodiments, R⁴ is R²⁹-substituted alkyl (e.g., C₁-C₈ alkyl, C₁-C₆ alkyl, or C₁-C₄ alkyl). In embodiments, R⁴ is an unsubstituted alkyl (e.g., C₁-C₈ alkyl, C₁-C₆ alkyl, or C₁-C₄ alkyl). In embodiments, R⁴ is R²⁹-substituted or unsubstituted heteroalkyl (e.g., 2 to 8 membered heteroalkyl, 2 to 6 membered heteroalkyl, or 2 to 4 membered heteroalkyl). In embodiments, R⁴ is R²⁹-substituted heteroalkyl (e.g., 2 to 8 membered heteroalkyl, 2 to 6 membered heteroalkyl, or 2 to 4 membered heteroalkyl). In embodiments, R⁴ is unsubstituted heteroalkyl (e.g., 2 to 8 membered heteroalkyl, 2 to 6 membered heteroalkyl, or 2 to 4 membered heteroalkyl).

In embodiments, R⁴ is R²⁹-substituted or unsubstituted cycloalkyl (e.g., C₃-C₈ cycloalkyl, C₃-C₆ cycloalkyl, or C₅-C₆ cycloalkyl). In embodiments, R⁴ is R²⁹-substituted cycloalkyl (e.g., C₃-C₈ cycloalkyl, C₃-C₆ cycloalkyl, or C₅-C₆ cycloalkyl). In embodiments, R⁴ is unsubstituted cycloalkyl (e.g., C₃-C₈ cycloalkyl, C₃-C₆ cycloalkyl, or C₅-C₆ cycloalkyl). In embodiments, R⁴ is R²⁹-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R⁴ is R²⁹-substituted heterocycloalkyl (e.g., 3 to 8 membered heterocycloalkyl, 3 to 6 membered heterocycloalkyl, or 5 to 6 membered heterocycloalkyl). In embodiments, R⁴ is unsubstituted heterocycloalkyl (e.g., 3 to 8 membered heterocycloalkyl, 3 to 6 membered heterocycloalkyl, or 5 to 6 membered heterocycloalkyl).

In embodiments, R⁴ is R²⁹-substituted or unsubstituted aryl (e.g., C₆-C₁₀ aryl, C₁₀ aryl, or phenyl). In embodiments, R⁴ is R²⁹-substituted aryl (e.g., C₆-C₁₀ aryl, C₁₀ aryl, or phenyl). In embodiments, R⁴ is unsubstituted aryl (e.g., C₆-C₁₀ aryl, C₁₀ aryl, or phenyl). In embodiments, R⁴ is R²⁹-substituted or unsubstituted heteroaryl (e.g., 5 to 10 membered heteroaryl, 5 to 9 membered heteroaryl, or 5 to 6 membered heteroaryl). In embodiments, R⁴ is R²⁹-substituted heteroaryl (e.g., 5 to 10 membered heteroaryl, 5 to 9 membered heteroaryl, or 5 to 6 membered heteroaryl). In embodiments, R⁴ is unsubstituted heteroaryl (e.g., 5 to 10 membered heteroaryl, 5 to 9 membered heteroaryl, or 5 to 6 membered heteroaryl).

In embodiments, R^{4A}, R^{4B}, R^{4C}, and R^{4D} are independently hydrogen, halogen, -CF₃, -CBr₃, -CCl₃, -CI₃, -CHF₂, -CHBr₂, -CHCl₂, -CHI₂, -CH₂F, -CH₂Br, -CH₂Cl, -CH₂I, -OCF₃, -OCBr₃, -OCCl₃, -OCI₃, -OCHF₂, -OCHBr₂, -OCHCl₂, -OCHI₂, -OCH₂F, -OCH₂Br, -OCH₂Cl, -OCH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, R²⁹-substituted or unsubstituted alkyl (e.g., C₁-C₈ alkyl, C₁-C₆ alkyl, or C₁-C₄ alkyl), R²⁹-substituted or unsubstituted heteroalkyl (e.g., 2 to 8 membered heteroalkyl, 2 to 6 membered heteroalkyl, or 2 to 4 membered heteroalkyl), R²⁹-substituted or unsubstituted cycloalkyl (e.g., C₃-C₈ cycloalkyl, C₃-C₆ cycloalkyl, or C₅-C₆ cycloalkyl), R²⁹-substituted or unsubstituted heterocycloalkyl (e.g., 3 to 8 membered heterocycloalkyl, 3 to 6 membered heterocycloalkyl, or 5 to 6 membered heterocycloalkyl), R²⁹-substituted or unsubstituted aryl (e.g., C₆-C₁₀ aryl, C₁₀ aryl, or phenyl), or R²⁹-substituted or unsubstituted heteroaryl (e.g., 5 to 10 membered heteroaryl, 5 to 9 membered heteroaryl, or 5 to 6 membered heteroaryl). In embodiments, R^{4A}, R^{4B}, R^{4C}, and R^{4D} are independently hydrogen.

In embodiments, R^{4A}, R^{4B}, R^{4C}, and R^{4D} are independently R²⁹-substituted or unsubstituted alkyl (e.g., C₁-C₈ alkyl, C₁-C₆ alkyl, or C₁-C₄ alkyl). In embodiments, R^{4A}, R^{4B}, R^{4C}, and R^{4D} are independently R²⁹-substituted alkyl (e.g., C₁-C₈ alkyl, C₁-C₆ alkyl, or C₁-C₄ alkyl). In embodiments, R^{4A}, R^{4B}, R^{4C}, and R^{4D} are independently an unsubstituted alkyl (e.g., C₁-C₈ alkyl, C₁-C₆ alkyl, or C₁-C₄ alkyl). In embodiments, R^{4A}, R^{4B}, R^{4C}, R^{4D} are independently R²⁹-substituted or unsubstituted heteroalkyl (e.g., 2 to 8 membered heteroalkyl, 2 to 6 membered heteroalkyl, or 2 to 4 membered heteroalkyl). In embodiments, R^{4A}, R^{4B}, R^{4C}, and R^{4D} are independently R²⁹-substituted heteroalkyl (e.g., 2 to 8 membered heteroalkyl, 2 to 6 membered heteroalkyl, or 2 to 4 membered heteroalkyl). In embodiments, R^{4A}, R^{4B}, R^{4C}, and R^{4D} are independently an unsubstituted heteroalkyl (e.g., 2 to 8 membered heteroalkyl, 2 to 6 membered heteroalkyl, or 2 to 4 membered heteroalkyl).

In embodiments, R^{4A}, R^{4B}, R^{4C}, and R^{4D} are independently R²⁹-substituted or unsubstituted cycloalkyl (e.g., C₃-C₈ cycloalkyl, C₃-C₆ cycloalkyl, or C₅-C₆ cycloalkyl). In embodiments, R^{4A}, R^{4B}, R^{4C}, and R^{4D} are independently R²⁹-substituted cycloalkyl (e.g., C₃-C₈ cycloalkyl, C₃-C₆ cycloalkyl, or C₅-C₆ cycloalkyl). In embodiments, R^{4A}, R^{4B}, R^{4C}, and R^{4D} are independently unsubstituted cycloalkyl (e.g., C₃-C₈ cycloalkyl, C₃-C₆ cycloalkyl, or C₅-C₆ cycloalkyl). In embodiments, R^{4A}, R^{4B}, R^{4C}, and R^{4D} are independently R²⁹-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R^{4A}, R^{4B}, R^{4C}, and R^{4D} are independently R²⁹-substituted heterocycloalkyl (e.g., 3 to 8 membered heterocycloalkyl, 3 to 6 membered heterocycloalkyl, or 5 to 6 membered heterocycloalkyl). In embodiments, R^{4A}, R^{4B}, R^{4C}, and R^{4D} are independently unsubstituted heterocycloalkyl (e.g., 3 to 8 membered heterocycloalkyl, 3 to 6 membered heterocycloalkyl, or 5 to 6 membered heterocycloalkyl).

In embodiments, R^{4A}, R^{4B}, R^{4C}, and R^{4D} are independently R²⁹-substituted or unsubstituted aryl (e.g., C₆-C₁₀ aryl, C₁₀ aryl, or phenyl). In embodiments, R^{4A}, R^{4B}, R^{4C}, and R^{4D} are independently R²⁹-substituted aryl (e.g., C₆-C₁₀ aryl, C₁₀ aryl, or phenyl). In embodiments, R^{4A}, R^{4B}, R^{4C}, and R^{4D} are independently unsubstituted aryl (e.g., C₆-C₁₀ aryl, C₁₀ aryl, or phenyl). In embodiments, R^{4A}, R^{4B}, R^{4C}, and R^{4D} are independently R¹⁹-substituted or unsubstituted heteroaryl (e.g., 5 to 10 membered heteroaryl, 5 to 9 membered heteroaryl, or 5 to 6 membered heteroaryl). In embodiments, R^{4A}, R^{4B}, R^{4C}, and R^{4D} are independently R²⁹-substituted heteroaryl (e.g., 5 to 10 membered heteroaryl, 5 to 9 membered heteroaryl, or 5 to 6 membered heteroaryl). In embodiments, R^{4A}, R^{4B}, R^{4C}, and R^{4D} are independently unsubstituted heteroaryl (e.g., 5 to 10 membered heteroaryl, 5 to 9 membered heteroaryl, or 5 to 6 membered heteroaryl).

R²⁹ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R³⁰-substituted or unsubstituted alkyl (e.g., C₁-C₈ alkyl, C₁-C₆ alkyl, or C₁-C₄ alkyl), R³⁰-substituted or unsubstituted heteroalkyl (e.g., 2 to 8 membered heteroalkyl, 2 to 6 membered heteroalkyl, or 2 to 4 membered heteroalkyl), R³⁰-substituted or unsubstituted cycloalkyl (e.g., C₃-C₈ cycloalkyl, C₃-C₆ cycloalkyl, or C₅-C₆ cycloalkyl), R³⁰-substituted or unsubstituted heterocycloalkyl (e.g., 3 to 8 membered heterocycloalkyl, 3 to 6 membered heterocycloalkyl, or 5 to 6 membered heterocycloalkyl), R³⁰-substituted or unsubstituted aryl (e.g., C₆-C₁₀ aryl, C₁₀ aryl, or phenyl), or R³⁰-substituted or unsubstituted heteroaryl (e.g., 5 to 10 membered heteroaryl, 5 to 9 membered heteroaryl, or 5 to 6 membered heteroaryl).

R³⁰ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R³¹-substituted or unsubstituted alkyl (e.g., C₁-C₈ alkyl, C₁-C₆ alkyl, or C₁-C₄ alkyl), R³¹-substituted or unsubstituted heteroalkyl (e.g., 2 to 8 membered heteroalkyl, 2 to 6 membered heteroalkyl, or 2 to 4 membered heteroalkyl), R³¹-substituted or unsubstituted cycloalkyl (e.g., C₃-C₈ cycloalkyl, C₃-C₆ cycloalkyl, or C₅-C₆ cycloalkyl), R³¹-substituted or unsubstituted heterocycloalkyl (e.g., 3 to 8 membered heterocycloalkyl, 3 to 6 membered heterocycloalkyl, or 5 to 6 membered heterocycloalkyl), R³¹-substituted or unsubstituted aryl (e.g., C₆-C₁₀ aryl, C₁₀ aryl, or phenyl), or R³¹-substituted or unsubstituted heteroaryl (e.g., 5 to 10 membered heteroaryl, 5 to 9 membered heteroaryl, or 5 to 6 membered heteroaryl).

In embodiments, R⁴ is hydrogen. In embodiments, R⁴ is -NH(CH₃). In embodiments, R⁴ is unsubstituted 2 to 3 membered heteroalkyl. In embodiments, R⁴ is 2 to 3 membered heteroalkyl. In embodiments, R⁴ is -NH₂. In embodiments, R⁴ is -NH(R^{4A}). In embodiments, R⁴ is -NR^{4A}R^{4B}.

In embodiments, R^{4A} is R²⁹-substituted or unsubstituted alkyl (e.g., C₁-C₈ alkyl, C₁-C₆ alkyl, or C₁-C₄ alkyl). In embodiments, R^{4A} is R²⁹-substituted alkyl (e.g., C₁-C₈ alkyl, C₁-C₆ alkyl, or C₁-C₄ alkyl). In embodiments, R^{4A} is an unsubstituted alkyl (e.g., C₁-C₈ alkyl, C₁-C₆ alkyl, or C₁-C₄ alkyl). In embodiments, R^{4A} is an unsubstituted C₁-C₄ alkyl. In embodiments, R^{4A} is an unsubstituted C₁-C₂ alkyl. In embodiments, R^{4A} is hydrogen.

In embodiments, R^{4B} is R²⁹-substituted or unsubstituted alkyl (e.g., C₁-C₈ alkyl, C₁-C₆ alkyl, or C₁-C₄ alkyl). In embodiments, R^{4B} is R²⁹-substituted alkyl (e.g., C₁-C₈ alkyl, C₁-C₆ alkyl, or C₁-C₄ alkyl). In embodiments, R^{4B} is an unsubstituted alkyl (e.g., C₁-C₈ alkyl, C₁-C₆ alkyl, or C₁-C₄ alkyl). In embodiments, R^{4B} is an unsubstituted C₁-C₄ alkyl. In embodiments, R^{4B} is an unsubstituted C₁-C₂ alkyl. In embodiments, R^{4B} is hydrogen.

In embodiments, R^{4C} is R²⁹-substituted or unsubstituted alkyl (e.g., C₁-C₈ alkyl, C₁-C₆ alkyl, or C₁-C₄ alkyl). In embodiments, R^{4C} is R²⁹-substituted alkyl (e.g., C₁-C₈ alkyl, C₁-C₆ alkyl, or C₁-C₄ alkyl). In embodiments, R^{4C} is an unsubstituted alkyl (e.g., C₁-C₈ alkyl, C₁-C₆ alkyl, or C₁-C₄ alkyl). In embodiments, R^{4C} is an unsubstituted C₁-C₄ alkyl. In embodiments, R^{4C} is an unsubstituted C₁-C₂ alkyl. In embodiments, R^{4C} is hydrogen.

In embodiments, R^{4D} is R²⁹-substituted or unsubstituted alkyl (e.g., C₁-C₈ alkyl, C₁-C₆ alkyl, or C₁-C₄ alkyl). In embodiments, R^{4D} is R²⁹-substituted alkyl (e.g., C₁-C₈ alkyl, C₁-C₆ alkyl, or C₁-C₄ alkyl). In embodiments, R^{4D} is an unsubstituted alkyl (e.g., C₁-C₈ alkyl, C₁-C₆ alkyl, or C₁-C₄ alkyl). In embodiments, R^{4D} is an unsubstituted C₁-C₄ alkyl. In embodiments, R^{4D} is an unsubstituted C₁-C₂ alkyl. In embodiments, R^{4D} is hydrogen.

In embodiments, R⁴ is unsubstituted methyl. In embodiments, R⁴ is unsubstituted C₁-C₃ alkyl. In embodiments, R⁴ is unsubstituted ethyl.

In embodiments, R⁴ is -CF₃. In embodiments, R⁴ is -CHF₂. In embodiments, R⁴ is -CH₂F. In embodiments, R⁴ is -CX⁴₃. In embodiments, R⁴ is -CHX⁴₂. In embodiments, R⁴ is -CH₂X⁴. X⁴ is independently -F, -Cl, -Br, or -I.

In embodiments, R⁵ is hydrogen, -CX⁵₃, -CHX⁵ ₅, -CH₅X⁵, -OCX⁵₃, -OCH₅X⁵, -OCHX⁵₅, -C(O)R^{5C}, -C(O)OR^{5C}, -C(O)NR^{5A}R^{5B}, -OR^{5D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. X⁵ is independently -F, -Cl, -Br, or -I.

In embodiments, R⁵ is independently hydrogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -C(O)H, -C(O)CH₃, -COOH, -C(O)OCH₃, -CONH₂, -CONCH₃, -OH, -OCH₃, R³²-substituted or unsubstituted alkyl (e.g., C₁-C₈ alkyl, C₁-C₆ alkyl, or C₁-C₄ alkyl), or R³²-substituted or unsubstituted heteroalkyl (e.g., 2 to 8 membered heteroalkyl, 2 to 6 membered heteroalkyl, or 2 to 4 membered heteroalkyl), R³²-substituted or unsubstituted cycloalkyl (e.g., C₃-C₈ cycloalkyl, C₃-C₆ cycloalkyl, or C₅-C₆ cycloalkyl), R³²-substituted or unsubstituted heterocycloalkyl (e.g., 3 to 8 membered heterocycloalkyl, 3 to 6 membered heterocycloalkyl, or 5 to 6 membered heterocycloalkyl), R³²-substituted or unsubstituted aryl (e.g., C₆-C₁₀ aryl, C₁₀ aryl, or phenyl), or R³²-substituted or unsubstituted heteroaryl (e.g., 5 to 10 membered heteroaryl, 5 to 9 membered heteroaryl, or 5 to 6 membered heteroaryl).

In embodiments, R⁵ is R³²-substituted or unsubstituted alkyl (e.g., C₁-C₈ alkyl, C₁-C₆ alkyl, or C₁-C₄ alkyl). In embodiments, R⁵ is R³²-substituted alkyl (e.g., C₁-C₈ alkyl, C₁-C₆ alkyl, or C₁-C₄ alkyl). In embodiments, R⁵ is an unsubstituted alkyl (e.g., C₁-C₈ alkyl, C₁-C₆ alkyl, or C₁-C₄ alkyl). In embodiments, R⁵ is R³²-substituted or unsubstituted heteroalkyl (e.g., 2 to 8 membered heteroalkyl, 2 to 6 membered heteroalkyl, or 2 to 4 membered heteroalkyl). In embodiments, R⁵ is R³²-substituted heteroalkyl (e.g., 2 to 8 membered heteroalkyl, 2 to 6 membered heteroalkyl, or 2 to 4 membered heteroalkyl). In embodiments, R⁵ is an unsubstituted heteroalkyl (e.g., 2 to 8 membered heteroalkyl, 2 to 6 membered heteroalkyl, or 2 to 4 membered heteroalkyl).

In embodiments, R⁵ is R³²-substituted or unsubstituted cycloalkyl (e.g., C₃-C₈ cycloalkyl, C₃-C₆ cycloalkyl, or C₅-C₆ cycloalkyl). In embodiments, R⁵ is R³²-substituted cycloalkyl (e.g., C₃-C₈ cycloalkyl, C₃-C₆ cycloalkyl, or C₅-C₆ cycloalkyl). In embodiments, R⁵ is unsubstituted cycloalkyl (e.g., C₃-C₈ cycloalkyl, C₃-C₆ cycloalkyl, or C₅-C₆ cycloalkyl). In embodiments, R⁵ is R³²-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R⁵ is R³²-substituted heterocycloalkyl (e.g., 3 to 8 membered heterocycloalkyl, 3 to 6 membered heterocycloalkyl, or 5 to 6 membered heterocycloalkyl). In embodiments, R⁵ is unsubstituted heterocycloalkyl (e.g., 3 to 8 membered heterocycloalkyl, 3 to 6 membered heterocycloalkyl, or 5 to 6 membered heterocycloalkyl).

In embodiments, R⁵ is R³²-substituted or unsubstituted aryl (e.g., C₆-C₁₀ aryl, C₁₀ aryl, or phenyl). In embodiments, R⁵ is R³²-substituted aryl (e.g., C₆-C₁₀ aryl, C₁₀ aryl, or phenyl). In embodiments, R⁵ is unsubstituted aryl (e.g., C₆-C₁₀ aryl, C₁₀ aryl, or phenyl). In embodiments, R⁵ is R³²-substituted or unsubstituted heteroaryl (e.g., 5 to 10 membered heteroaryl, 5 to 9 membered heteroaryl, or 5 to 6 membered heteroaryl). In embodiments, R⁵ is R³²-substituted heteroaryl (e.g., 5 to 10 membered heteroaryl, 5 to 9 membered heteroaryl, or 5 to 6 membered heteroaryl). In embodiments, R⁵ is unsubstituted heteroaryl (e.g., 5 to 10 membered heteroaryl, 5 to 9 membered heteroaryl, or 5 to 6 membered heteroaryl).

R³² is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R³³-substituted or unsubstituted alkyl (e.g., C₁-C₈ alkyl, C₁-C₆ alkyl, or C₁-C₄ alkyl), R³³-substituted or unsubstituted heteroalkyl (e.g., 2 to 8 membered heteroalkyl, 2 to 6 membered heteroalkyl, or 2 to 4 membered heteroalkyl), R³³-substituted or unsubstituted cycloalkyl (e.g., C₃-C₈ cycloalkyl, C₃-C₆ cycloalkyl, or C₅-C₆ cycloalkyl), R³³-substituted or unsubstituted heterocycloalkyl (e.g., 3 to 8 membered heterocycloalkyl, 3 to 6 membered heterocycloalkyl, or 5 to 6 membered heterocycloalkyl), R³³-substituted or unsubstituted aryl (e.g., C₆-C₁₀ aryl, C₁₀ aryl, or phenyl), or R³³-substituted or unsubstituted heteroaryl (e.g., 5 to 10 membered heteroaryl, 5 to 9 membered heteroaryl, or 5 to 6 membered heteroaryl).

R³³ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R³⁴-substituted or unsubstituted alkyl (e.g., C₁-C₈ alkyl, C₁-C₆ alkyl, or C₁-C₄ alkyl), R³⁴-substituted or unsubstituted heteroalkyl (e.g., 2 to 8 membered heteroalkyl, 2 to 6 membered heteroalkyl, or 2 to 4 membered heteroalkyl), R³⁴-substituted or unsubstituted cycloalkyl (e.g., C₃-C₈ cycloalkyl, C₃-C₆ cycloalkyl, or C₅-C₆ cycloalkyl), R³⁴-substituted or unsubstituted heterocycloalkyl (e.g., 3 to 8 membered heterocycloalkyl, 3 to 6 membered heterocycloalkyl, or 5 to 6 membered heterocycloalkyl), R³⁴-substituted or unsubstituted aryl (e.g., C₆-C₁₀ aryl, C₁₀ aryl, or phenyl), or R³⁴-substituted or unsubstituted heteroaryl (e.g., 5 to 10 membered heteroaryl, 5 to 9 membered heteroaryl, or 5 to 6 membered heteroaryl).

In embodiments, R⁵ is unsubstituted methyl. In embodiments, R⁵ is unsubstituted C₁-C₂ alkyl. In embodiments, R⁵ is unsubstituted C₁-C₃ alkyl. In embodiments, R⁵ is hydrogen.

In embodiments, R⁶ is hydrogen, -CX⁶₃, -CHX⁶₂, -CH₂X⁶, -OCX⁶₃, -OCH₂X⁶, -OCHX⁶₂, -C(O)R^{6C}, -C(O)OR^{6C}, -C(O)NR^{6A}R^{6B}, -OR^{6D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

In embodiments, R⁶ is independently hydrogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -C(O)H, -C(O)CH₃, -COOH, -C(O)OCH₃, -CONH₂, -CONCH₃, -OH, -OCH₃, R³⁵-substituted or unsubstituted alkyl (e.g., C₁-C₈ alkyl, C₁-C₆ alkyl, or C₁-C₄ alkyl), or R³⁵-substituted or unsubstituted heteroalkyl (e.g., 2 to 8 membered heteroalkyl, 2 to 6 membered heteroalkyl, or 2 to 4 membered heteroalkyl), R³⁵-substituted or unsubstituted cycloalkyl (e.g., C₃-C₈ cycloalkyl, C₃-C₆ cycloalkyl, or C₅-C₆ cycloalkyl), R³⁵-substituted or unsubstituted heterocycloalkyl (e.g., 3 to 8 membered heterocycloalkyl, 3 to 6 membered heterocycloalkyl, or 5 to 6 membered heterocycloalkyl), R³⁵-substituted or unsubstituted aryl (e.g., C₆-C₁₀ aryl, C₁₀ aryl, or phenyl), or R³⁵-substituted or unsubstituted heteroaryl (e.g., 5 to 10 membered heteroaryl, 5 to 9 membered heteroaryl, or 5 to 6 membered heteroaryl).

In embodiments, R⁶ is R³⁵-substituted or unsubstituted alkyl (e.g., C₁-C₈ alkyl, C₁-C₆ alkyl, or C₁-C₄ alkyl). In embodiments, R⁶ is R³⁵-substituted alkyl (e.g., C₁-C₈ alkyl, C₁-C₆ alkyl, or C₁-C₄ alkyl). In embodiments, R⁶ is an unsubstituted alkyl (e.g., C₁-C₈ alkyl, C₁-C₆ alkyl, or C₁-C₄ alkyl). In embodiments, R⁶ is R³⁵-substituted or unsubstituted heteroalkyl (e.g., 2 to 8 membered heteroalkyl, 2 to 6 membered heteroalkyl, or 2 to 4 membered heteroalkyl). In embodiments, R⁶ is R³⁵-substituted heteroalkyl (e.g., 2 to 8 membered heteroalkyl, 2 to 6 membered heteroalkyl, or 2 to 4 membered heteroalkyl). In embodiments, R⁶ is an unsubstituted heteroalkyl (e.g., 2 to 8 membered heteroalkyl, 2 to 6 membered heteroalkyl, or 2 to 4 membered heteroalkyl).

In embodiments, R⁶ is R³⁵-substituted or unsubstituted cycloalkyl (e.g., C₃-C₈ cycloalkyl, C₃-C₆ cycloalkyl, or C₅-C₆ cycloalkyl). In embodiments, R⁶ is R³⁵-substituted cycloalkyl (e.g., C₃-C₈ cycloalkyl, C₃-C₆ cycloalkyl, or C₅-C₆ cycloalkyl). In embodiments, R⁶ is unsubstituted cycloalkyl (e.g., C₃-C₈ cycloalkyl, C₃-C₆ cycloalkyl, or C₅-C₆ cycloalkyl). In embodiments, R⁶ is R³⁵-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R⁶ is R³⁵-substituted heterocycloalkyl (e.g., 3 to 8 membered heterocycloalkyl, 3 to 6 membered heterocycloalkyl, or 5 to 6 membered heterocycloalkyl). In embodiments, R⁶ is unsubstituted heterocycloalkyl (e.g., 3 to 8 membered heterocycloalkyl, 3 to 6 membered heterocycloalkyl, or 5 to 6 membered heterocycloalkyl).

In embodiments, R⁶ is R³⁵-substituted or unsubstituted aryl (e.g., C₆-C₁₀ aryl, C₁₀ aryl, or phenyl). In embodiments, R⁶ is R³⁵-substituted aryl (e.g., C₆-C₁₀ aryl, C₁₀ aryl, or phenyl). In embodiments, R⁶ is unsubstituted aryl (e.g., C₆-C₁₀ aryl, C₁₀ aryl, or phenyl). In embodiments, R⁶ is R³⁵-substituted or unsubstituted heteroaryl (e.g., 5 to 10 membered heteroaryl, 5 to 9 membered heteroaryl, or 5 to 6 membered heteroaryl). In embodiments, R⁶ is R³⁵-substituted heteroaryl (e.g., 5 to 10 membered heteroaryl, 5 to 9 membered heteroaryl, or 5 to 6 membered heteroaryl). In embodiments, R⁶ is unsubstituted heteroaryl (e.g., 5 to 10 membered heteroaryl, 5 to 9 membered heteroaryl, or 5 to 6 membered heteroaryl).

R³⁵ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R³⁶-substituted or unsubstituted alkyl (e.g., C₁-C₈ alkyl, C₁-C₆ alkyl, or C₁-C₄ alkyl), R³⁶-substituted or unsubstituted heteroalkyl (e.g., 2 to 8 membered heteroalkyl, 2 to 6 membered heteroalkyl, or 2 to 4 membered heteroalkyl), R³⁶-substituted or unsubstituted cycloalkyl (e.g., C₃-C₈ cycloalkyl, C₃-C₆ cycloalkyl, or C₅-C₆ cycloalkyl), R³⁶-substituted or unsubstituted heterocycloalkyl (e.g., 3 to 8 membered heterocycloalkyl, 3 to 6 membered heterocycloalkyl, or 5 to 6 membered heterocycloalkyl), R³⁶-substituted or unsubstituted aryl (e.g., C₆-C₁₀ aryl, C₁₀ aryl, or phenyl), or R³⁶-substituted or unsubstituted heteroaryl (e.g., 5 to 10 membered heteroaryl, 5 to 9 membered heteroaryl, or 5 to 6 membered heteroaryl).

R³⁶ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R³⁷-substituted or unsubstituted alkyl (e.g., C₁-C₈ alkyl, C₁-C₆ alkyl, or C₁-C₄ alkyl), R³⁷-substituted or unsubstituted heteroalkyl (e.g., 2 to 8 membered heteroalkyl, 2 to 6 membered heteroalkyl, or 2 to 4 membered heteroalkyl), R³⁷-substituted or unsubstituted cycloalkyl (e.g., C₃-C₈ cycloalkyl, C₃-C₆ cycloalkyl, or C₅-C₆ cycloalkyl), R³⁷-substituted or unsubstituted heterocycloalkyl (e.g., 3 to 8 membered heterocycloalkyl, 3 to 6 membered heterocycloalkyl, or 5 to 6 membered heterocycloalkyl), R³⁷-substituted or unsubstituted aryl (e.g., C₆-C₁₀ aryl, C₁₀ aryl, or phenyl), or R³⁷-substituted or unsubstituted heteroaryl (e.g., 5 to 10 membered heteroaryl, 5 to 9 membered heteroaryl, or 5 to 6 membered heteroaryl).

In embodiments, R⁶ is unsubstituted methyl. In embodiments, R⁶ is unsubstituted ethyl. In embodiments, R⁶ is unsubstituted propyl. In embodiments, R⁶ is unsubstituted C₁-C₂ alkyl. In embodiments, R⁶ is unsubstituted C₁-C₃ alkyl. In embodiments, R⁶ is hydrogen.

In embodiments, R⁷ is independently halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COH, -COCH₃, -COOH, -CONH₂, -CONHOH, -CONHOCH₃, -COCH₃,-NO₂, -SH, -SO₃H, -SO₄H, -SO₂NH₂, -NHNH₂, -ONH₂, -NHC(O)NHNH₂, -NHC(O)NH₂, -NHS(O)NH₂, -NHS(O)NHCH₃, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R³⁸-substituted or unsubstituted alkyl (e.g., C₁-C₈ alkyl, C₁-C₆ alkyl, or C₁-C₄ alkyl), R³⁸-substituted or unsubstituted heteroalkyl (e.g., 2 to 8 membered heteroalkyl, 2 to 6 membered heteroalkyl, or 2 to 4 membered heteroalkyl), R³⁸-substituted or unsubstituted cycloalkyl (e.g., C₃-C₈ cycloalkyl, C₃-C₆ cycloalkyl, or C₅-C₆ cycloalkyl), R³⁸-substituted or unsubstituted heterocycloalkyl (e.g., 3 to 8 membered heterocycloalkyl, 3 to 6 membered heterocycloalkyl, or 5 to 6 membered heterocycloalkyl), R³⁸-substituted or unsubstituted aryl (e.g., C₆-C₁₀ aryl, C₁₀ aryl, or phenyl), or R³⁸-substituted or unsubstituted heteroaryl (e.g., 5 to 10 membered heteroaryl, 5 to 9 membered heteroaryl, or 5 to 6 membered heteroaryl).

In embodiments, R⁷ is independently R³⁸-substituted or unsubstituted alkyl (e.g., C₁-C₈ alkyl, C₁-C₆ alkyl, or C₁-C₄ alkyl). In embodiments, R⁷ is independently R³⁸-substituted alkyl (e.g., C₁-C₈ alkyl, C₁-C₆ alkyl, or C₁-C₄ alkyl). In embodiments, R⁷ is independently an unsubstituted alkyl (e.g., C₁-C₈ alkyl, C₁-C₆ alkyl, or C₁-C₄ alkyl). In embodiments, R⁷ is independently R³⁸-substituted or unsubstituted heteroalkyl (e.g., 2 to 8 membered heteroalkyl, 2 to 6 membered heteroalkyl, or 2 to 4 membered heteroalkyl). In embodiments, R⁷ is independently R³⁸-substituted heteroalkyl (e.g., 2 to 8 membered heteroalkyl, 2 to 6 membered heteroalkyl, or 2 to 4 membered heteroalkyl). In embodiments, R⁷ is independently unsubstituted heteroalkyl (e.g., 2 to 8 membered heteroalkyl, 2 to 6 membered heteroalkyl, or 2 to 4 membered heteroalkyl).

In embodiments, R⁷ is independently R³⁸-substituted or unsubstituted cycloalkyl (e.g., C₃-C₈ cycloalkyl, C₃-C₆ cycloalkyl, or C₅-C₆ cycloalkyl). In embodiments, R⁷ is independently R³⁸-substituted cycloalkyl (e.g., C₃-C₈ cycloalkyl, C₃-C₆ cycloalkyl, or C₅-C₆ cycloalkyl). In embodiments, R⁷ is independently unsubstituted cycloalkyl (e.g., C₃-C₈ cycloalkyl, C₃-C₆ cycloalkyl, or C₅-C₆ cycloalkyl). In embodiments, R⁷ is independently R³⁸-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R⁷ is independently R³⁸-substituted heterocycloalkyl (e.g., 3 to 8 membered heterocycloalkyl, 3 to 6 membered heterocycloalkyl, or 5 to 6 membered heterocycloalkyl). In embodiments, R⁷ is independently unsubstituted heterocycloalkyl (e.g., 3 to 8 membered heterocycloalkyl, 3 to 6 membered heterocycloalkyl, or 5 to 6 membered heterocycloalkyl).

In embodiments, R⁷ is independently R³⁸-substituted or unsubstituted aryl (e.g., C₆-C₁₀ aryl, C₁₀ aryl, or phenyl). In embodiments, R⁷ is independently R³⁸-substituted aryl (e.g., C₆-C₁₀ aryl, C₁₀ aryl, or phenyl). In embodiments, R⁷ is independently unsubstituted aryl (e.g., C₆-C₁₀ aryl, C₁₀ aryl, or phenyl). In embodiments, R⁷ is independently R³⁸-substituted or unsubstituted heteroaryl (e.g., 5 to 10 membered heteroaryl, 5 to 9 membered heteroaryl, or 5 to 6 membered heteroaryl). In embodiments, R⁷ is independently R³⁸-substituted heteroaryl (e.g., 5 to 10 membered heteroaryl, 5 to 9 membered heteroaryl, or 5 to 6 membered heteroaryl). In embodiments, R⁷ is independently unsubstituted heteroaryl (e.g., 5 to 10 membered heteroaryl, 5 to 9 membered heteroaryl, or 5 to 6 membered heteroaryl).

In embodiments, R^{7A}, R^{7B}, R^{7C}, and R^{7D} are independently hydrogen, halogen, -CF₃, -CBr₃, -CCl₃, -CI₃, -CHF₂, -CHBr₂, -CHCl₂, -CHI₂, -CH₂F, -CH₂Br, -CH₂Cl, -CH₂I, -OCF₃, -OCBr₃, -OCCl₃, -OCI₃, -OCHF₂, -OCHBr₂, -OCHCl₂, -OCHI₂, -OCH₂F, -OCH₂Br, -OCH₂Cl, -OCH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, R³⁸-substituted or unsubstituted alkyl (e.g., C₁-C₈ alkyl, C₁-C₆ alkyl, or C₁-C₄ alkyl), R³⁸-substituted or unsubstituted heteroalkyl (e.g., 2 to 8 membered heteroalkyl, 2 to 6 membered heteroalkyl, or 2 to 4 membered heteroalkyl), R³⁸-substituted or unsubstituted cycloalkyl (e.g., C₃-C₈ cycloalkyl, C₃-C₆ cycloalkyl, or C₅-C₆ cycloalkyl), R³⁸-substituted or unsubstituted heterocycloalkyl (e.g., 3 to 8 membered heterocycloalkyl, 3 to 6 membered heterocycloalkyl, or 5 to 6 membered heterocycloalkyl), R³⁸-substituted or unsubstituted aryl (e.g., C₆-C₁₀ aryl, C₁₀ aryl, or phenyl), or R³⁸-substituted or unsubstituted heteroaryl (e.g., 5 to 10 membered heteroaryl, 5 to 9 membered heteroaryl, or 5 to 6 membered heteroaryl). In embodiments, R^{7A}, R^{7B}, R^{7C}, and R^{7D} are independently hydrogen.

In embodiments, R^{7A}, R^{7B}, R^{7C}, and R^{7D} are independently R³⁸-substituted or unsubstituted alkyl (e.g., C₁-C₈ alkyl, C₁-C₆ alkyl, or C₁-C₄ alkyl). In embodiments, R^{7A} , R^{7B}, R^{7C}, and R^{7D} are independently R³⁸-substituted alkyl (e.g., C₁-C₈ alkyl, C₁-C₆ alkyl, or C₁-C₄ alkyl). In embodiments, R^{7A}, R^{7B}, R^{7C}, and R^{7D} are independently an unsubstituted alkyl (e.g., C₁-C₈ alkyl, C₁-C₆ alkyl, or C₁-C₄ alkyl). In embodiments, R^{7A}, R^{7B}, R^{7C}, R^{7D} are independently R³⁸-substituted or unsubstituted heteroalkyl (e.g., 2 to 8 membered heteroalkyl, 2 to 6 membered heteroalkyl, or 2 to 4 membered heteroalkyl). In embodiments, R^{7A}, R^{7B}, R^{7C}, and R^{7D} are independently R³⁸-substituted heteroalkyl (e.g., 2 to 8 membered heteroalkyl, 2 to 6 membered heteroalkyl, or 2 to 4 membered heteroalkyl). In embodiments, R^{7A}, R^{7B}, R^{7C}, and R^{7D} are independently an unsubstituted heteroalkyl (e.g., 2 to 8 membered heteroalkyl, 2 to 6 membered heteroalkyl, or 2 to 4 membered heteroalkyl).

In embodiments, R^{7A}, R^{7B}, R^{7C}, and R^{7D} are independently R³⁸-substituted or unsubstituted cycloalkyl (e.g., C₃-C₈ cycloalkyl, C₃-C₆ cycloalkyl, or C₅-C₆ cycloalkyl). In embodiments, R^{7A}, R^{7B}, R^{7C}, and R^{7D} are independently R³⁸-substituted cycloalkyl (e.g., C₃-C₈ cycloalkyl, C₃-C₆ cycloalkyl, or C₅-C₆ cycloalkyl). In embodiments, R^{7A}, R^{7B}, R^{7C}, and R^{7D} are independently unsubstituted cycloalkyl (e.g., C₃-C₈ cycloalkyl, C₃-C₆ cycloalkyl, or C₅-C₆ cycloalkyl). In embodiments, R^{7A}, R^{7B}, R^{7C}, and R^{7D} are independently R³⁸-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R^{7A}, R^{7B}, R^{7C}, and R^{7D} are independently R³⁸-substituted heterocycloalkyl (e.g., 3 to 8 membered heterocycloalkyl, 3 to 6 membered heterocycloalkyl, or 5 to 6 membered heterocycloalkyl). In embodiments, R^{7A}, R^{7B}, R^{7C}, and R^{7D} are independently unsubstituted heterocycloalkyl (e.g., 3 to 8 membered heterocycloalkyl, 3 to 6 membered heterocycloalkyl, or 5 to 6 membered heterocycloalkyl).

In embodiments, R^{7A}, R^{7B}, R^{7C}, and R^{7D} are independently R³⁸-substituted or unsubstituted aryl (e.g., C₆-C₁₀ aryl, C₁₀ aryl, or phenyl). In embodiments, R^{7A}, R^{7B}, R^{7C}, and R^{7D} are independently R³⁸-substituted aryl (e.g., C₆-C₁₀ aryl, C₁₀ aryl, or phenyl). In embodiments, R^{7A}, R^{7B}, R^{7C}, and R^{7D} are independently unsubstituted aryl (e.g., C₆-C₁₀ aryl, C₁₀ aryl, or phenyl). In embodiments, R^{7A}, R^{7B}, R^{7C}, and R^{7D} are independently R³⁸-substituted or unsubstituted heteroaryl (e.g., 5 to 10 membered heteroaryl, 5 to 9 membered heteroaryl, or 5 to 6 membered heteroaryl). In embodiments, R^{7A}, R^{7B}, R^{7C}, and R^{7D} are independently R³⁸-substituted heteroaryl (e.g., 5 to 10 membered heteroaryl, 5 to 9 membered heteroaryl, or 5 to 6 membered heteroaryl). In embodiments, R^{7A}, R^{7B}, R^{7C}, and R^{7D} are independently unsubstituted heteroaryl (e.g., 5 to 10 membered heteroaryl, 5 to 9 membered heteroaryl, or 5 to 6 membered heteroaryl).

In embodiments, R⁷ is independently halogen. In embodiments, R⁷ is independently -CX⁷₃ (e.g., -CF₃, -CBr₃, -CCl₃, or -CI₃). In embodiments, R⁷ is independently -CHX⁷₂ (e.g., -CHF₂, -CHBr₂, -CHCl₂, or -CHI₂). In embodiments, R⁷ is independently-CH₂X⁷ (e.g., -CH₂F, -CH₂Br, -CH₂Cl, or -CH₂I). In embodiments, R⁷ is independently -OCX⁷₃ (e.g., -OCF₃, -OCBr₃, -OCCl₃, or -OCI₃). In embodiments, R⁷ is independently -OCH₁X⁷ (e.g., -OCH₂F, -OCH₂Br, -OCH₂Cl, or -OCH₂I). In embodiments, R⁷ is independently -OCHX⁷₂ (e.g., -OCHF₂, -OCHBr₂, -OCHCl₂, or -OCHI₂). In embodiments, R⁷ is independently -C(O)R^{7C} (e.g., -COH, -COCH₃, or -COCH₂CH₃). In embodiments, R⁷ is independently -C(O)OR^{7C} (e.g.,
-COOH, -COOCH₃, or -COOCH₂CH₃). In embodiments, R⁷ is independently -C(O)NR^{7A}R^{7B} (e.g., -CONH₂, or -CONHCH₃). In embodiments, R⁷ is independently -OR^{7D} (e.g., -OH, or -OCH₃). In embodiments, R⁷ is independently -N₃. In embodiments, R⁷ is independently-CN. In embodiments, R⁷ is independently -SOₙ₇R^{7D} (e.g., -SH, -SO₃H, -SO₄H). In embodiments, R⁷ is independently -SOᵥ₇NR^{7A}R^{7B} (e.g., -SO₂NH₂). In embodiments, R⁷ is independently
-NR^{7A}C(O)NR^{7A}R^{7B} (e.g., -NHC(O)NH₂). In embodiments, R⁷ is independently -NR^{7A}S(O)NR^{7A}R^{7B} (e.g., -NHS(O)NH₂, or -NHS(O)NHCH₃). In embodiments, R⁷ is independently -N(O)ₘ₇ (e.g., -NO₂). In embodiments, R⁷ is independently -NR^{7A}R^{7B} (e.g., -NH₂ or -NHCH₃). In embodiments, R⁷ is independently -C(O)NR^{7A}OR^{7B} (e.g.,-CONHOH, or
-CONHOCH₃). In embodiments, R⁷ is independently -NR^{7A}SO₂R^{7D} (e.g., -NHSO₂H). In embodiments, R⁷ is independently -NR^{7A}C(O)R^{7C} (e.g., -NHC(O)H). In embodiments, R⁷ is independently -NR^{7A}C(O)OR^{7C} (e.g., -NHC(O)H). In embodiments, R⁷ is independently -NR^{7A}OR^{7C} (e.g., -NHOH).

R³⁸ is oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In embodiments, R³⁸ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂,
-NHC(O)NHNH₂,
-NHC(O)NH₂, -NHSO₂H, -NHC(O)H, -NHC(O)OH, -NHOH, -OCCl₃, -OCF₃, -OCBr₃, -OC I₃,
-OCHCl₂, -OCHBr₂, -OCHI₂, -OCHF₂, -OCH₂Cl, -OCH₂Br, -OCH₂I, -OCH₂F, -N₃, R³⁹-substituted or unsubstituted alkyl (e.g., C₁-C₈ alkyl, C₁-C₆ alkyl, or C₁-C₄ alkyl), R³⁹-substituted or unsubstituted heteroalkyl (e.g., 2 to 8 membered heteroalkyl, 2 to 6 membered heteroalkyl, or 2 to 4 membered heteroalkyl), R³⁹-substituted or unsubstituted cycloalkyl (e.g., C₃-C₈ cycloalkyl, C₃-C₆ cycloalkyl, or C₅-C₆ cycloalkyl), R³⁹-substituted or unsubstituted heterocycloalkyl (e.g., 3 to 8 membered heterocycloalkyl, 3 to 6 membered heterocycloalkyl, or 5 to 6 membered heterocycloalkyl), R³⁹-substituted or unsubstituted aryl (e.g., C₆-C₁₀ aryl, C₁₀ aryl, or phenyl), or R³⁹-substituted or unsubstituted heteroaryl (e.g., 5 to 10 membered heteroaryl, 5 to 9 membered heteroaryl, or 5 to 6 membered heteroaryl).

In embodiments, R³⁸ is independently unsubstituted phenyl. In embodiments, R³⁸ is independently unsubstituted methyl. In embodiments, R³⁸ is independently -CF₃. In embodiments, R³⁸ is independently -NH₂. In embodiments, R³⁸ is independently unsubstituted benzyl. In embodiments, R³⁸ is independently oxo. In embodiments, R³⁸ is independently halogen. In embodiments, R³⁸ is independently -CCl₃. In embodiments, R³⁸ is independently -CBr₃. In embodiments, R³⁸ is independently -CF₃. In embodiments, R³⁸ is independently -CI₃. In embodiments, R³⁸ is independently -CHCl₂. In embodiments, R³⁸ is independently -CHBr₂. In embodiments, R³⁸ is independently -CHF₂. In embodiments, R³⁸ is independently -CHI₂. In embodiments, R³⁸ is independently -CH₂Cl. In embodiments, R³⁸ is independently -CH₂Br. In embodiments, R³⁸ is independently -CH₂F. In embodiments, R³⁸ is independently -CH₂I. In embodiments, R³⁸ is independently -CN. In embodiments, R³⁸ is independently -OH. In embodiments, R³⁸ is independently -NH₂. In embodiments, R³⁸ is independently -COOH. In embodiments, R³⁸ is independently -CONH₂. In embodiments, R³⁸ is independently -NO₂. In embodiments, R³⁸ is independently -SH. In embodiments, R³⁸ is independently -SO₃H. In embodiments, R³⁸ is independently -SO₄H. In embodiments, R³⁸ is independently -SO₂NH₂. In embodiments, R³⁸ is independently -NHNH₂. In embodiments, R³⁸ is independently -ONH₂. In embodiments, R³⁸ is independently -NHC(O)NHNH₂. In embodiments, R³⁸ is independently -NHC(O)NH₂. In embodiments, R³⁸ is independently -NHSO₂H. In embodiments, R³⁸ is independently -NHC(O)H. In embodiments, R³⁸ is independently -NHC(O)OH. In embodiments, R³⁸ is independently -NHOH. In embodiments, R³⁸ is independently -OCCl₃. In embodiments, R³⁸ is independently -OCF₃. In embodiments, R³⁸ is independently -OCBr₃. In embodiments, R³⁸ is independently -OCI₃. In embodiments, R³⁸ is independently -OCHCl₂. In embodiments, R³⁸ is independently -OCHBr₂. In embodiments, R³⁸ is independently -OCHI₂. In embodiments, R³⁸ is independently -OCHF₂. In embodiments, R³⁸ is independently -OCH₂Cl. In embodiments, R³⁸ is independently -OCH₂Br. In embodiments, R³⁸ is independently -OCH₂I. In embodiments, R³⁸ is independently -OCH₂F.

In embodiments, R³⁸ is R³⁹-substituted or unsubstituted alkyl (e.g., C₁-C₈ alkyl, C₁-C₆ alkyl, or C₁-C₄ alkyl). In embodiments, R³⁸ is R³⁹-substituted alkyl (e.g., C₁-C₈ alkyl, C₁-C₆ alkyl, or C₁-C₄ alkyl). In embodiments, R³⁸ is an unsubstituted alkyl (e.g., C₁-C₈ alkyl, C₁-C₆ alkyl, or C₁-C₄ alkyl).

In embodiments, R³⁸ is R³⁹-substituted or unsubstituted heteroalkyl (e.g., 2 to 8 membered heteroalkyl, 2 to 6 membered heteroalkyl, or 2 to 4 membered heteroalkyl). In embodiments, R³⁸ is R³⁹-substituted heteroalkyl (e.g., 2 to 8 membered heteroalkyl, 2 to 6 membered heteroalkyl, or 2 to 4 membered heteroalkyl). In embodiments, R³⁸ is an unsubstituted heteroalkyl (e.g., 2 to 8 membered heteroalkyl, 2 to 6 membered heteroalkyl, or 2 to 4 membered heteroalkyl).

In embodiments, R³⁸ is R³⁹-substituted or unsubstituted cycloalkyl (e.g., C₃-C₈ cycloalkyl, C₃-C₆ cycloalkyl, or C₅-C₆ cycloalkyl). In embodiments, R³⁸ is R³⁹-substituted cycloalkyl (e.g., C₃-C₈ cycloalkyl, C₃-C₆ cycloalkyl, or C₅-C₆ cycloalkyl). In embodiments, R³⁸ is an unsubstituted cycloalkyl (e.g., C₃-C₈ cycloalkyl, C₃-C₆ cycloalkyl, or C₅-C₆ cycloalkyl).

In embodiments, R³⁸ is R³⁹-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R³⁸ is R³⁹-substituted heterocycloalkyl (e.g., 3 to 8 membered heterocycloalkyl, 3 to 6 membered heterocycloalkyl, or 5 to 6 membered heterocycloalkyl). In embodiments, R³⁸ is an unsubstituted heterocycloalkyl (e.g., 3 to 8 membered heterocycloalkyl, 3 to 6 membered heterocycloalkyl, or 5 to 6 membered heterocycloalkyl).

In embodiments, R³⁸ is R³⁹-substituted or unsubstituted aryl (e.g., C₆-C₁₀ aryl, C₁₀ aryl, or phenyl). In embodiments, R³⁸ is R³⁹-substituted aryl (e.g., C₆-C₁₀ aryl, C₁₀ aryl, or phenyl). In embodiments, R³⁸ is an unsubstituted aryl (e.g., C₆-C₁₀ aryl, C₁₀ aryl, or phenyl).

In embodiments, R³⁸ is R³⁹-substituted or unsubstituted heteroaryl (e.g., 5 to 10 membered heteroaryl, 5 to 9 membered heteroaryl, or 5 to 6 membered heteroaryl). In embodiments, R³⁸ is R³⁹-substituted heteroaryl (e.g., 5 to 10 membered heteroaryl, 5 to 9 membered heteroaryl, or 5 to 6 membered heteroaryl). In embodiments, R³⁸ is an unsubstituted heteroaryl (e.g., 5 to 10 membered heteroaryl, 5 to 9 membered heteroaryl, or 5 to 6 membered heteroaryl).

R³⁹ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R⁴⁴-substituted or unsubstituted alkyl (e.g., C₁-C₈ alkyl, C₁-C₆ alkyl, or C₁-C₄ alkyl), R⁴⁴-substituted or unsubstituted heteroalkyl (e.g., 2 to 8 membered heteroalkyl, 2 to 6 membered heteroalkyl, or 2 to 4 membered heteroalkyl), R⁴⁴-substituted or unsubstituted cycloalkyl (e.g., C₃-C₈ cycloalkyl, C₃-C₆ cycloalkyl, or C₅-C₆ cycloalkyl), R⁴⁴-substituted or unsubstituted heterocycloalkyl (e.g., 3 to 8 membered heterocycloalkyl, 3 to 6 membered heterocycloalkyl, or 5 to 6 membered heterocycloalkyl), R⁴⁴-substituted or unsubstituted aryl (e.g., C₆-C₁₀ aryl, C₁₀ aryl, or phenyl), or R⁴⁴-substituted or unsubstituted heteroaryl (e.g., 5 to 10 membered heteroaryl, 5 to 9 membered heteroaryl, or 5 to 6 membered heteroaryl).

In embodiments, R³⁹ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂,
-NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

R⁴⁴ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

In embodiments, R⁷ is independently halogen, -CX⁷₃, -CHX⁷₂, -CH₂X⁷, -OCX⁷₃, -OCH₁X⁷, -OCHX⁷₂, -NR^{7A}R^{7B}, -C(O)R^{7C}, substituted or unsubstituted C₁-C₆ alkyl, substituted or unsubstituted 2 to 6 membered heteroalkyl. In embodiments, R⁷ is independently halogen, -CX⁷₃, -CHX⁷₂, -CH₂X⁷, -OCX⁷₃, - OCH₂X⁷, -OCHX⁷₂, -CN, -SR^{7D}, -SO₂R^{7D},
-NR^{7A}R^{7B}, -C(O)R^{7C}, -C(O)OR^{7C}, -C(O)NR^{7A}R^{7B}, -OR^{7D}, -NR^{7A}C(O)R^{7C}, -NR^{7A}C(O)OR^{7C}, substituted or unsubstituted C₁-C₆ alkyl, substituted or unsubstituted 2 to 6 membered heteroalkyl. In embodiments, R⁷ is independently -C(O)R^{7C}, -C(O)OR^{7C},
-C(O)NR^{7A}R^{7B}, -OR^{7D}, -NR^{7A}C(O)R^{7C}, -NR^{7A}C(O)OR^{7C}, substituted or unsubstituted C₁-C₆ alkyl, substituted or unsubstituted 2 to 6 membered heteroalkyl. In embodiments, R⁷ is independently -F, -OH, -OCH₃, -OCH₂CH₃, -CH₂OH, -CH₂OCH₃, -NHC(O)CH₃, -CH₃, -N(CH₃)₂, -CH₂NH₂, -CH₂N(CH₃)₂, -NH₂, -NHCH₃, -COOH, or -SO₂CH₃. In embodiments, R⁷ is independently -OH, -OCH₃, -OCH₂CH₃, -CH₂OH, or -CH₂OCH₃. In embodiments, R⁷ is independently substituted or unsubstituted C₁-C₆ alkyl, or substituted or unsubstituted 2 to 6 membered heteroalkyl. In embodiments, R⁷ is independently R³⁸-substituted or unsubstituted C₁-C₆ alkyl, or R³⁸-substituted or unsubstituted 2 to 6 membered heteroalkyl.

In embodiments, R^{7A} is independently hydrogen, halogen, -CF₃, -CBr₃, -CCl₃, -CI₃, -CHF₂, -CHBr₂, -CHCl₂, -CHI₂, -CH₂F, -CH₂Br, -CH₂Cl, -CH₂I, -OCF₃, -OCBr₃, -OCCl₃, -OCI₃, -OCHF₂, -OCHBr₂, -OCHCl₂, -OCHI₂, -OCH₂F, -OCH₂Br, -OCH₂Cl, -OCH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, R^{38A}-substituted or unsubstituted alkyl (e.g., C₁-C₈ alkyl, C₁-C₆ alkyl, or C₁-C₄ alkyl), R^{38A}-substituted or unsubstituted heteroalkyl (e.g., 2 to 8 membered heteroalkyl, 2 to 6 membered heteroalkyl, or 2 to 4 membered heteroalkyl), R^{38A}-substituted or unsubstituted cycloalkyl (e.g., C₃-C₈ cycloalkyl, C₃-C₆ cycloalkyl, or C₅-C₆ cycloalkyl), R^{38A}-substituted or unsubstituted heterocycloalkyl (e.g., 3 to 8 membered heterocycloalkyl, 3 to 6 membered heterocycloalkyl, or 5 to 6 membered heterocycloalkyl), R^{38A}-substituted or unsubstituted aryl (e.g., C₆-C₁₀ aryl, C₁₀ aryl, or phenyl), or R^{38A}-substituted or unsubstituted heteroaryl (e.g., 5 to 10 membered heteroaryl, 5 to 9 membered heteroaryl, or 5 to 6 membered heteroaryl).

In embodiments, R^{7A} is independently R^{38A}-substituted or unsubstituted alkyl (e.g., C₁-C₈ alkyl, C₁-C₆ alkyl, or C₁-C₄ alkyl). In embodiments, R^{7A} is independently R^{38A}-substituted alkyl (e.g., C₁-C₈ alkyl, C₁-C₆ alkyl, or C₁-C₄ alkyl). In embodiments, R^{7A} is independently an unsubstituted alkyl (e.g., C₁-C₈ alkyl, C₁-C₆ alkyl, or C₁-C₄ alkyl). In embodiments, R^{7A} is independently R^{38A}-substituted or unsubstituted heteroalkyl (e.g., 2 to 8 membered heteroalkyl, 2 to 6 membered heteroalkyl, or 2 to 4 membered heteroalkyl). In embodiments, R^{7A} is independently R^{38A}-substituted heteroalkyl (e.g., 2 to 8 membered heteroalkyl, 2 to 6 membered heteroalkyl, or 2 to 4 membered heteroalkyl). In embodiments, R^{7A} is independently an unsubstituted heteroalkyl (e.g., 2 to 8 membered heteroalkyl, 2 to 6 membered heteroalkyl, or 2 to 4 membered heteroalkyl).

In embodiments, R^{7A} is independently R^{38A}-substituted or unsubstituted cycloalkyl (e.g., C₃-C₈ cycloalkyl, C₃-C₆ cycloalkyl, or C₅-C₆ cycloalkyl). In embodiments, R^{7A} is independently R^{38A}-substituted cycloalkyl (e.g., C₃-C₈ cycloalkyl, C₃-C₆ cycloalkyl, or C₅-C₆ cycloalkyl). In embodiments, R^{7A} is independently an unsubstituted cycloalkyl (e.g., C₃-C₈ cycloalkyl, C₃-C₆ cycloalkyl, or C₅-C₆ cycloalkyl).

In embodiments, R^{7A} is independently R^{38A}-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R^{7A} is independently R^{38A}-substituted heterocycloalkyl (e.g., 3 to 8 membered heterocycloalkyl, 3 to 6 membered heterocycloalkyl, or 5 to 6 membered heterocycloalkyl). In embodiments, R^{7A} is independently unsubstituted heterocycloalkyl (e.g., 3 to 8 membered heterocycloalkyl, 3 to 6 membered heterocycloalkyl, or 5 to 6 membered heterocycloalkyl).

In embodiments, R^{7A} is independently R^{38A}-substituted or unsubstituted aryl (e.g., C₆-C₁₀ aryl, C₁₀ aryl, or phenyl). In embodiments, R^{7A} is independently R^{38A}-substituted aryl (e.g., C₆-C₁₀ aryl, C₁₀ aryl, or phenyl). In embodiments, R^{7A} is independently an unsubstituted aryl (e.g., C₆-C₁₀ aryl, C₁₀ aryl, or phenyl).

In embodiments, R^{7A} is independently R^{38A}-substituted or unsubstituted heteroaryl (e.g., 5 to 10 membered heteroaryl, 5 to 9 membered heteroaryl, or 5 to 6 membered heteroaryl). In embodiments, R^{7A} is independently R^{38A}-substituted heteroaryl (e.g., 5 to 10 membered heteroaryl, 5 to 9 membered heteroaryl, or 5 to 6 membered heteroaryl). In embodiments, R^{7A} is independently an unsubstituted heteroaryl (e.g., 5 to 10 membered heteroaryl, 5 to 9 membered heteroaryl, or 5 to 6 membered heteroaryl).

R^{38A} is oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In embodiments, R^{38A} is independently oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂,
-NHC(O)NH₂, -NHSO₂H, -NHC(O)H, -NHC(O)OH, -NHOH, -OCCl₃, -OCF₃, -OCBr₃, -OC I₃,
-OCHCl₂, -OCHBr₂, -OCHI₂, -OCHF₂, -OCH₂Cl, -OCH₂Br, -OCH₂I, -OCH₂F, -N₃, R^{39A}-substituted or unsubstituted alkyl (e.g., C₁-C₈ alkyl, C₁-C₆ alkyl, or C₁-C₄ alkyl), R^{39A}-substituted or unsubstituted heteroalkyl (e.g., 2 to 8 membered heteroalkyl, 2 to 6 membered heteroalkyl, or 2 to 4 membered heteroalkyl), R^{39A}-substituted or unsubstituted cycloalkyl (e.g., C₃-C₈ cycloalkyl, C₃-C₆ cycloalkyl, or C₅-C₆ cycloalkyl), R^{39A}-substituted or unsubstituted heterocycloalkyl (e.g., 3 to 8 membered heterocycloalkyl, 3 to 6 membered heterocycloalkyl, or 5 to 6 membered heterocycloalkyl), R^{39A}-substituted or unsubstituted aryl (e.g., C₆-C₁₀ aryl, C₁₀ aryl, or phenyl), or R^{39A}-substituted or unsubstituted heteroaryl (e.g., 5 to 10 membered heteroaryl, 5 to 9 membered heteroaryl, or 5 to 6 membered heteroaryl).

In embodiments, R^{38A} is independently unsubstituted phenyl. In embodiments, R^{38A} is independently unsubstituted methyl. In embodiments, R^{38A} is independently -CF₃. In embodiments, R^{38A} is independently -NH₂. In embodiments, R^{38A} is independently unsubstituted benzyl. In embodiments, R^{38A} is independently oxo. In embodiments, R^{38A} is independently halogen. In embodiments, R^{38A} is independently -CCl₃. In embodiments, R^{38A} is independently -CBr₃. In embodiments, R^{38A} is independently -CF₃. In embodiments, R^{38A} is independently -CI₃. In embodiments, R^{38A} is independently -CHCl₂. In embodiments, R^{38A} is independently -CHBr₂. In embodiments, R^{38A} is independently -CHF₂. In embodiments, R^{38A} is independently -CHI₂. In embodiments, R^{38A} is independently -CH₂Cl. In embodiments, R^{38A} is independently -CH₂Br. In embodiments, R^{38A} is independently -CH₂F. In embodiments, R^{38A} is independently -CH₂I. In embodiments, R^{38A} is independently -CN. In embodiments, R^{38A} is independently -OH. In embodiments, R^{38A} is independently -NH₂. In embodiments, R^{38A} is independently -COOH. In embodiments, R^{38A} is independently -CONH₂. In embodiments, R^{38A} is independently -NO₂. In embodiments, R^{38A} is independently -SH. In embodiments, R^{38A} is independently -SO₃H. In embodiments, R^{38A} is independently -SO₄H. In embodiments, R^{38A} is independently -SO₂NH₂. In embodiments, R^{38A} is independently -NHNH₂. In embodiments, R^{38A} is independently -ONH₂. In embodiments, R^{38A} is independently -NHC(O)NHNH₂. In embodiments, R^{38A} is independently -NHC(O)NH₂. In embodiments, R^{38A} is independently -NHSO₂H. In embodiments, R^{38A} is independently -NHC(O)H. In embodiments, R^{38A} is independently -NHC(O)OH. In embodiments, R^{38A} is independently -NHOH. In embodiments, R^{38A} is independently -OCCl₃. In embodiments, R^{38A} is independently -OCF₃. In embodiments, R^{38A} is independently -OCBr₃. In embodiments, R^{38A} is independently -OCI₃. In embodiments, R^{38A} is independently -OCHCl₂. In embodiments, R^{38A} is independently -OCHBr₂. In embodiments, R^{38A} is independently -OCHI₂. In embodiments, R^{38A} is independently -OCHF₂. In embodiments, R^{38A} is independently -OCH₂Cl. In embodiments, R^{38A} is independently -OCH₂Br. In embodiments, R^{38A} is independently -OCH₂I. In embodiments, R^{38A} is independently -OCH₂F.

R^{39A} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R^{44A}-substituted or unsubstituted alkyl (e.g., C₁-C₈ alkyl, C₁-C₆ alkyl, or C₁-C₄ alkyl), R^{44A}-substituted or unsubstituted heteroalkyl (e.g., 2 to 8 membered heteroalkyl, 2 to 6 membered heteroalkyl, or 2 to 4 membered heteroalkyl), R^{44A}-substituted or unsubstituted cycloalkyl (e.g., C₃-C₈ cycloalkyl, C₃-C₆ cycloalkyl, or C₅-C₆ cycloalkyl), R^{44A}-substituted or unsubstituted heterocycloalkyl (e.g., 3 to 8 membered heterocycloalkyl, 3 to 6 membered heterocycloalkyl, or 5 to 6 membered heterocycloalkyl), R^{44A}-substituted or unsubstituted aryl (e.g., C₆-C₁₀ aryl, C₁₀ aryl, or phenyl), or R^{44A}-substituted or unsubstituted heteroaryl (e.g., 5 to 10 membered heteroaryl, 5 to 9 membered heteroaryl, or 5 to 6 membered heteroaryl).

In embodiments, R^{39A} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂,
-NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

R^{44A} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

In embodiments, R^{7B} is independently hydrogen, halogen, -CF₃, -CBr₃, -CCl₃, -CI₃, -CHF₂, -CHBr₂, -CHCl₂, -CHI₂, -CH₂F, -CH₂Br, -CH₂Cl, -CH₂I, -OCF₃, -OCBr₃, -OCCl₃, -OCI₃, -OCHF₂, -OCHBr₂, -OCHCl₂, -OCHI₂, -OCH₂F, -OCH₂Br, -OCH₂Cl, -OCH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, R^{38B}-substituted or unsubstituted alkyl (e.g., C₁-C₈ alkyl, C₁-C₆ alkyl, or C₁-C₄ alkyl), R^{31B}-substituted or unsubstituted heteroalkyl (e.g., 2 to 8 membered heteroalkyl, 2 to 6 membered heteroalkyl, or 2 to 4 membered heteroalkyl), R^{38B}-substituted or unsubstituted cycloalkyl (e.g., C₃-C₈ cycloalkyl, C₃-C₆ cycloalkyl, or C₅-C₆ cycloalkyl), R^{38B}-substituted or unsubstituted heterocycloalkyl (e.g., 3 to 8 membered heterocycloalkyl, 3 to 6 membered heterocycloalkyl, or 5 to 6 membered heterocycloalkyl), R^{38B}-substituted or unsubstituted aryl (e.g., C₆-C₁₀ aryl, C₁₀ aryl, or phenyl), or R^{38B}-substituted or unsubstituted heteroaryl (e.g., 5 to 10 membered heteroaryl, 5 to 9 membered heteroaryl, or 5 to 6 membered heteroaryl).

In embodiments, R^{7B} is independently R^{38B}-substituted or unsubstituted alkyl (e.g., C₁-C₈ alkyl, C₁-C₆ alkyl, or C₁-C₄ alkyl). In embodiments, R^{7B} is independently R^{38B}-substituted alkyl (e.g., C₁-C₈ alkyl, C₁-C₆ alkyl, or C₁-C₄ alkyl). In embodiments, R^{7B} is independently an unsubstituted alkyl (e.g., C₁-C₈ alkyl, C₁-C₆ alkyl, or C₁-C₄ alkyl). In embodiments, R^{7B} is independently R^{38B}-substituted or unsubstituted heteroalkyl (e.g., 2 to 8 membered heteroalkyl, 2 to 6 membered heteroalkyl, or 2 to 4 membered heteroalkyl). In embodiments, R^{7B} is independently R^{38B}-substituted heteroalkyl (e.g., 2 to 8 membered heteroalkyl, 2 to 6 membered heteroalkyl, or 2 to 4 membered heteroalkyl). In embodiments, R^{7B} is independently an unsubstituted heteroalkyl (e.g., 2 to 8 membered heteroalkyl, 2 to 6 membered heteroalkyl, or 2 to 4 membered heteroalkyl).

In embodiments, R^{7B} is independently R^{38B}-substituted or unsubstituted cycloalkyl (e.g., C₃-C₈ cycloalkyl, C₃-C₆ cycloalkyl, or C₅-C₆ cycloalkyl). In embodiments, R^{7B} is independently R^{38B}-substituted cycloalkyl (e.g., C₃-C₈ cycloalkyl, C₃-C₆ cycloalkyl, or C₅-C₆ cycloalkyl). In embodiments, R^{7B} is independently an unsubstituted cycloalkyl (e.g., C₃-C₈ cycloalkyl, C₃-C₆ cycloalkyl, or C₅-C₆ cycloalkyl).

In embodiments, R^{7B} is independently R³⁸-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R^{7B} is independently R^{38B}-substituted heterocycloalkyl (e.g., 3 to 8 membered heterocycloalkyl, 3 to 6 membered heterocycloalkyl, or 5 to 6 membered heterocycloalkyl). In embodiments, R^{7B} is independently an unsubstituted heterocycloalkyl (e.g., 3 to 8 membered heterocycloalkyl, 3 to 6 membered heterocycloalkyl, or 5 to 6 membered heterocycloalkyl).

In embodiments, R^{7B} is independently R^{38B}-substituted or unsubstituted aryl (e.g., C₆-C₁₀ aryl, C₁₀ aryl, or phenyl). In embodiments, R^{7B} is independently R^{38B}-substituted aryl (e.g., C₆-C₁₀ aryl, C₁₀ aryl, or phenyl). In embodiments, R^{7B} is independently an unsubstituted aryl (e.g., C₆-C₁₀ aryl, C₁₀ aryl, or phenyl).

In embodiments, R^{7B} is independently R^{38B}-substituted or unsubstituted heteroaryl (e.g., 5 to 10 membered heteroaryl, 5 to 9 membered heteroaryl, or 5 to 6 membered heteroaryl). In embodiments, R^{7B} is independently R^{38B}-substituted heteroaryl (e.g., 5 to 10 membered heteroaryl, 5 to 9 membered heteroaryl, or 5 to 6 membered heteroaryl). In embodiments, R^{7B} is independently an unsubstituted heteroaryl (e.g., 5 to 10 membered heteroaryl, 5 to 9 membered heteroaryl, or 5 to 6 membered heteroaryl).

R^{38B} is oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In embodiments, R^{38B} is independently oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R^{39B}-substituted or unsubstituted alkyl (e.g., C₁-C₈ alkyl, C₁-C₆ alkyl, or C₁-C₄ alkyl), R^{39B}-substituted or unsubstituted heteroalkyl (e.g., 2 to 8 membered heteroalkyl, 2 to 6 membered heteroalkyl, or 2 to 4 membered heteroalkyl), R^{39B}-substituted or unsubstituted cycloalkyl (e.g., C₃-C₈ cycloalkyl, C₃-C₆ cycloalkyl, or C₅-C₆ cycloalkyl), R^{39B}-substituted or unsubstituted heterocycloalkyl (e.g., 3 to 8 membered heterocycloalkyl, 3 to 6 membered heterocycloalkyl, or 5 to 6 membered heterocycloalkyl), R^{39B}-substituted or unsubstituted aryl (e.g., C₆-C₁₀ aryl, C₁₀ aryl, or phenyl), or R^{39B}-substituted or unsubstituted heteroaryl (e.g., 5 to 10 membered heteroaryl, 5 to 9 membered heteroaryl, or 5 to 6 membered heteroaryl).

In embodiments, R^{38B} is independently unsubstituted phenyl. In embodiments, R^{38B} is independently unsubstituted methyl. In embodiments, R^{38B} is independently -CF₃. In embodiments, R^{38B} is independently -NH₂. In embodiments, R^{38B} is independently unsubstituted benzyl. In embodiments, R^{38B} is independently oxo. In embodiments, R^{38B} is independently halogen. In embodiments, R^{38B} is independently -CCl₃. In embodiments, R^{38B} is independently -CBr₃. In embodiments, R^{38B} is independently -CF₃. In embodiments, R^{38B} is independently -CI₃. In embodiments, R^{38B} is independently -CHCl₂. In embodiments, R^{38B} is independently -CHBr₂. In embodiments, R^{38B} is independently -CHF₂. In embodiments, R^{38B} is independently -CHI₂. In embodiments, R^{38B} is independently -CH₂Cl. In embodiments, R^{38B} is independently -CH₂Br. In embodiments, R^{38B} is independently -CH₂F. In embodiments, R^{38B} is independently -CH₂I. In embodiments, R^{38B} is independently -CN. In embodiments, R^{38B} is independently -OH. In embodiments, R^{38B} is independently -NH₂. In embodiments, R^{38B} is independently -COOH. In embodiments, R^{38B} is independently -CONH₂. In embodiments, R^{38B} is independently -NO₂. In embodiments, R^{38B} is independently -SH. In embodiments, R^{38B} is independently -SO₃H. In embodiments, R^{38B} is independently -SO₄H. In embodiments, R^{38B} is independently -SO₂NH₂. In embodiments, R^{38B} is independently -NHNH₂. In embodiments, R^{38B} is independently -ONH₂. In embodiments, R^{38B} is independently -NHC(O)NHNH₂. In embodiments, R^{38B} is independently -NHC(O)NH₂. In embodiments, R^{38B} is independently -NHSO₂H. In embodiments, R^{38B} is independently -NHC(O)H. In embodiments, R^{38B} is independently -NHC(O)OH. In embodiments, R^{38B} is independently -NHOH. In embodiments, R^{38B} is independently -OCCl₃. In embodiments, R^{38B} is independently -OCF₃. In embodiments, R^{38B} is independently -OCBr₃. In embodiments, R^{38B} is independently -OCI₃. In embodiments, R^{38B} is independently -OCHCl₂. In embodiments, R^{38B} is independently -OCHBr₂. In embodiments, R^{38B} is independently -OCHI₂. In embodiments, R^{38B} is independently -OCHF₂. In embodiments, R^{38B} is independently -OCH₂Cl. In embodiments, R^{38B} is independently -OCH₂Br. In embodiments, R^{38B} is independently -OCH₂I. In embodiments, R^{38B} is independently -OCH₂F.

R^{39B} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R^{44B}-substituted or unsubstituted alkyl (e.g., C₁-C₈ alkyl, C₁-C₆ alkyl, or C₁-C₄ alkyl), R^{44B}-substituted or unsubstituted heteroalkyl (e.g., 2 to 8 membered heteroalkyl, 2 to 6 membered heteroalkyl, or 2 to 4 membered heteroalkyl), R^{44B}-substituted or unsubstituted cycloalkyl (e.g., C₃-C₈ cycloalkyl, C₃-C₆ cycloalkyl, or C₅-C₆ cycloalkyl), R^{44B}-substituted or unsubstituted heterocycloalkyl (e.g., 3 to 8 membered heterocycloalkyl, 3 to 6 membered heterocycloalkyl, or 5 to 6 membered heterocycloalkyl), R^{44B}-substituted or unsubstituted aryl (e.g., C₆-C₁₀ aryl, C₁₀ aryl, or phenyl), or R^{44B}-substituted or unsubstituted heteroaryl (e.g., 5 to 10 membered heteroaryl, 5 to 9 membered heteroaryl, or 5 to 6 membered heteroaryl).

In embodiments, R^{39B} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂,
-NHC(O)NHNH₂,
-NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

R^{44B} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

In embodiments, R^{7C} is independently hydrogen, halogen, -CF₃, -CBr₃, -CCl₃, -CI₃, -CHF₂, -CHBr₂, -CHCl₂, -CHI₂, -CH₂F, -CH₂Br, -CH₂Cl, -CH₂I, -OCF₃, -OCBr₃, -OCCl₃, -OCI₃, -OCHF₂, -OCHBr₂, -OCHCl₂, -OCHI₂, -OCH₂F, -OCH₂Br, -OCH₂Cl, -OCH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, R^{38C}-substituted or unsubstituted alkyl (e.g., C₁-C₈ alkyl, C₁-C₆ alkyl, or C₁-C₄ alkyl), R^{38C}-substituted or unsubstituted heteroalkyl (e.g., 2 to 8 membered heteroalkyl, 2 to 6 membered heteroalkyl, or 2 to 4 membered heteroalkyl), R^{38C}-substituted or unsubstituted cycloalkyl (e.g., C₃-C₈ cycloalkyl, C₃-C₆ cycloalkyl, or C₅-C₆ cycloalkyl), R^{38C}-substituted or unsubstituted heterocycloalkyl (e.g., 3 to 8 membered heterocycloalkyl, 3 to 6 membered heterocycloalkyl, or 5 to 6 membered heterocycloalkyl), R^{38C}-substituted or unsubstituted aryl (e.g., C₆-C₁₀ aryl, C₁₀ aryl, or phenyl), or R^{38C}-substituted or unsubstituted heteroaryl (e.g., 5 to 10 membered heteroaryl, 5 to 9 membered heteroaryl, or 5 to 6 membered heteroaryl).

In embodiments, R^{7C} is independently R^{38C}-substituted or unsubstituted alkyl (e.g., C₁-C₈ alkyl, C₁-C₆ alkyl, or C₁-C₄ alkyl). In embodiments, R^{7C} is independently R^{38C}-substituted alkyl (e.g., C₁-C₈ alkyl, C₁-C₆ alkyl, or C₁-C₄ alkyl). In embodiments, R^{7C} is independently an unsubstituted alkyl (e.g., C₁-C₈ alkyl, C₁-C₆ alkyl, or C₁-C₄ alkyl). In embodiments, R^{7C} is independently R^{38C}-substituted or unsubstituted heteroalkyl (e.g., 2 to 8 membered heteroalkyl, 2 to 6 membered heteroalkyl, or 2 to 4 membered heteroalkyl). In embodiments, R^{7C} is independently R³⁸-substituted heteroalkyl (e.g., 2 to 8 membered heteroalkyl, 2 to 6 membered heteroalkyl, or 2 to 4 membered heteroalkyl). In embodiments, R^{7C} is independently an unsubstituted heteroalkyl (e.g., 2 to 8 membered heteroalkyl, 2 to 6 membered heteroalkyl, or 2 to 4 membered heteroalkyl).

In embodiments, R^{7C} is independently R^{38C}-substituted or unsubstituted cycloalkyl (e.g., C₃-C₈ cycloalkyl, C₃-C₆ cycloalkyl, or C₅-C₆ cycloalkyl). In embodiments, R^{7C} is independently R^{38C}-substituted cycloalkyl (e.g., C₃-C₈ cycloalkyl, C₃-C₆ cycloalkyl, or C₅-C₆ cycloalkyl). In embodiments, R^{7C} is independently an unsubstituted cycloalkyl (e.g., C₃-C₈ cycloalkyl, C₃-C₆ cycloalkyl, or C₅-C₆ cycloalkyl). In embodiments, R^{7C} is independently R^{38C}-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R^{7C} is independently R^{38C}-substituted heterocycloalkyl (e.g., 3 to 8 membered heterocycloalkyl, 3 to 6 membered heterocycloalkyl, or 5 to 6 membered heterocycloalkyl). In embodiments, R^{7C} is independently an unsubstituted heterocycloalkyl (e.g., 3 to 8 membered heterocycloalkyl, 3 to 6 membered heterocycloalkyl, or 5 to 6 membered heterocycloalkyl).

In embodiments, R^{7C} is independently R^{38C}-substituted or unsubstituted aryl (e.g., C₆-C₁₀ aryl, C₁₀ aryl, or phenyl). In embodiments, R^{7C} is independently R^{38C}-substituted aryl (e.g., C₆-C₁₀ aryl, C₁₀ aryl, or phenyl). In embodiments, R^{7C} is independently an unsubstituted aryl (e.g., C₆-C₁₀ aryl, C₁₀ aryl, or phenyl). In embodiments, R^{7C} is independently R^{38C}-substituted or unsubstituted heteroaryl (e.g., 5 to 10 membered heteroaryl, 5 to 9 membered heteroaryl, or 5 to 6 membered heteroaryl). In embodiments, R^{7C} is independently R^{38C}-substituted heteroaryl (e.g., 5 to 10 membered heteroaryl, 5 to 9 membered heteroaryl, or 5 to 6 membered heteroaryl). In embodiments, R^{7C} is independently an unsubstituted heteroaryl (e.g., 5 to 10 membered heteroaryl, 5 to 9 membered heteroaryl, or 5 to 6 membered heteroaryl).

R^{38C} is oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In embodiments, R^{38C} is independently oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂,
-NHC(O)NHNH₂,
-NHC(O)NH₂, -NHSO₂H, -NHC(O)H, -NHC(O)OH, -NHOH, -OCCl₃, -OCF₃, -OCBr₃, -OC I₃,
-OCHCl₂, -OCHBr₂, -OCHI₂, -OCHF₂, -OCH₂Cl, -OCH₂Br, -OCH₂I, -OCH₂F, -N₃, R^{39C}-substituted or unsubstituted alkyl (e.g., C₁-C₈ alkyl, C₁-C₆ alkyl, or C₁-C₄ alkyl), R^{39C}-substituted or unsubstituted heteroalkyl (e.g., 2 to 8 membered heteroalkyl, 2 to 6 membered heteroalkyl, or 2 to 4 membered heteroalkyl), R^{39C}-substituted or unsubstituted cycloalkyl (e.g., C₃-C₈ cycloalkyl, C₃-C₆ cycloalkyl, or C₅-C₆ cycloalkyl), R^{39C}-substituted or unsubstituted heterocycloalkyl (e.g., 3 to 8 membered heterocycloalkyl, 3 to 6 membered heterocycloalkyl, or 5 to 6 membered heterocycloalkyl), R^{39C}-substituted or unsubstituted aryl (e.g., C₆-C₁₀ aryl, C₁₀ aryl, or phenyl), or R^{39C}-substituted or unsubstituted heteroaryl (e.g., 5 to 10 membered heteroaryl, 5 to 9 membered heteroaryl, or 5 to 6 membered heteroaryl).

In embodiments, R^{38C} is independently unsubstituted phenyl. In embodiments, R^{38C} is independently unsubstituted methyl. In embodiments, R^{38C} is independently -CF₃. In embodiments, R^{38C} is independently -NH₂. In embodiments, R^{38C} is independently unsubstituted benzyl. In embodiments, R^{38C} is independently oxo. In embodiments, R^{38C} is independently halogen. In embodiments, R^{38C} is independently -CCl₃. In embodiments, R^{38C} is independently -CBr₃. In embodiments, R^{38C} is independently -CF₃. In embodiments, R^{38C} is independently -CI₃. In embodiments, R^{38C} is independently -CHCl₂. In embodiments, R^{38C} is independently -CHBr₂. In embodiments, R^{38C} is independently -CHF₂. In embodiments, R^{38C} is independently -CHI₂. In embodiments, R^{38C} is independently -CH₂Cl. In embodiments, R^{38C} is independently -CH₂Br. In embodiments, R^{38C} is independently -CH₂F. In embodiments, R^{38C} is independently -CH₂I. In embodiments, R^{38C} is independently -CN. In embodiments, R^{38C} is independently -OH. In embodiments, R^{38C} is independently -NH₂. In embodiments, R^{38C} is independently -COOH. In embodiments, R^{38C} is independently -CONH₂. In embodiments, R^{38C} is independently -NO₂. In embodiments, R^{38C} is independently -SH. In embodiments, R^{38C} is independently -SO₃H. In embodiments, R^{38C} is independently -SO₄H. In embodiments, R^{38C} is independently -SO₂NH₂. In embodiments, R^{38C} is independently -NHNH₂. In embodiments, R^{38C} is independently -ONH₂. In embodiments, R^{38C} is independently -NHC(O)NHNH₂. In embodiments, R^{38C} is independently -NHC(O)NH₂. In embodiments, R^{38C} is independently -NHSO₂H. In embodiments, R^{38C} is independently -NHC(O)H. In embodiments, R^{38C} is independently -NHC(O)OH. In embodiments, R^{38C} is independently -NHOH. In embodiments, R^{38C} is independently -OCCl₃. In embodiments, R^{38C} is independently -OCF₃. In embodiments, R^{38C} is independently -OCBr₃. In embodiments, R^{38C} is independently -OCI₃. In embodiments, R^{38C} is independently -OCHCl₂. In embodiments, R^{38C} is independently -OCHBr₂. In embodiments, R^{38C} is independently -OCHI₂. In embodiments, R^{38C} is independently -OCHF₂. In embodiments, R^{38C} is independently -OCH₂Cl. In embodiments, R^{38C} is independently -OCH₂Br. In embodiments, R^{38C} is independently -OCH₂I. In embodiments, R^{38C} is independently -OCH₂F.

R^{39C} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R^{44C}-substituted or unsubstituted alkyl (e.g., C₁-C₈ alkyl, C₁-C₆ alkyl, or C₁-C₄ alkyl), R^{44C}-substituted or unsubstituted heteroalkyl (e.g., 2 to 8 membered heteroalkyl, 2 to 6 membered heteroalkyl, or 2 to 4 membered heteroalkyl), R^{44C}-substituted or unsubstituted cycloalkyl (e.g., C₃-C₈ cycloalkyl, C₃-C₆ cycloalkyl, or C₅-C₆ cycloalkyl), R^{44C}-substituted or unsubstituted heterocycloalkyl (e.g., 3 to 8 membered heterocycloalkyl, 3 to 6 membered heterocycloalkyl, or 5 to 6 membered heterocycloalkyl), R^{44C}-substituted or unsubstituted aryl (e.g., C₆-C₁₀ aryl, C₁₀ aryl, or phenyl), or R^{44C}-substituted or unsubstituted heteroaryl (e.g., 5 to 10 membered heteroaryl, 5 to 9 membered heteroaryl, or 5 to 6 membered heteroaryl).

In embodiments, R^{39C} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂,
-NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

R^{44C} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

In embodiments, R^{7D} is independently hydrogen, halogen, -CF₃, -CBr₃, -CCl₃, -CI₃, -CHF₂, -CHBr₂, -CHCl₂, -CHI₂, -CH₂F, -CH₂Br, -CH₂Cl, -CH₂I, -OCF₃, -OCBr₃, -OCCl₃, -OCI₃, -OCHF₂, -OCHBr₂, -OCHCl₂, -OCHI₂, -OCH₂F, -OCH₂Br, -OCH₂Cl, -OCH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, R^{38D}-substituted or unsubstituted alkyl (e.g., C₁-C₈ alkyl, C₁-C₆ alkyl, or C₁-C₄ alkyl), R^{38D}-substituted or unsubstituted heteroalkyl (e.g., 2 to 8 membered heteroalkyl, 2 to 6 membered heteroalkyl, or 2 to 4 membered heteroalkyl), R^{38D}-substituted or unsubstituted cycloalkyl (e.g., C₃-C₈ cycloalkyl, C₃-C₆ cycloalkyl, or C₅-C₆ cycloalkyl), R^{38D}-substituted or unsubstituted heterocycloalkyl (e.g., 3 to 8 membered heterocycloalkyl, 3 to 6 membered heterocycloalkyl, or 5 to 6 membered heterocycloalkyl), R^{38D}-substituted or unsubstituted aryl (e.g., C₆-C₁₀ aryl, C₁₀ aryl, or phenyl), or R^{38D}-substituted or unsubstituted heteroaryl (e.g., 5 to 10 membered heteroaryl, 5 to 9 membered heteroaryl, or 5 to 6 membered heteroaryl).

In embodiments, R^{7D} is independently R^{38D}-substituted or unsubstituted alkyl (e.g., C₁-C₈ alkyl, C₁-C₆ alkyl, or C₁-C₄ alkyl). In embodiments, R^{7D} is independently R^{38D}-substituted alkyl (e.g., C₁-C₈ alkyl, C₁-C₆ alkyl, or C₁-C₄ alkyl). In embodiments, R^{7D} is independently an unsubstituted alkyl (e.g., C₁-C₈ alkyl, C₁-C₆ alkyl, or C₁-C₄ alkyl). In embodiments, R^{7D} is independently R^{38D}-substituted or unsubstituted heteroalkyl (e.g., 2 to 8 membered heteroalkyl, 2 to 6 membered heteroalkyl, or 2 to 4 membered heteroalkyl). In embodiments, R^{7D} is independently R^{38D}-substituted heteroalkyl (e.g., 2 to 8 membered heteroalkyl, 2 to 6 membered heteroalkyl, or 2 to 4 membered heteroalkyl). In embodiments, R^{7D} is independently an unsubstituted heteroalkyl (e.g., 2 to 8 membered heteroalkyl, 2 to 6 membered heteroalkyl, or 2 to 4 membered heteroalkyl).

In embodiments, R^{7D} is independently R^{38D}-substituted or unsubstituted cycloalkyl (e.g., C₃-C₈ cycloalkyl, C₃-C₆ cycloalkyl, or C₅-C₆ cycloalkyl). In embodiments, R^{7D} is independently R^{38D}-substituted cycloalkyl (e.g., C₃-C₈ cycloalkyl, C₃-C₆ cycloalkyl, or C₅-C₆ cycloalkyl). In embodiments, R^{7D} is independently an unsubstituted cycloalkyl (e.g., C₃-C₈ cycloalkyl, C₃-C₆ cycloalkyl, or C₅-C₆ cycloalkyl). In embodiments, R^{7D} is independently R^{38D}-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R^{7D} is independently R^{38D}-substituted heterocycloalkyl (e.g., 3 to 8 membered heterocycloalkyl, 3 to 6 membered heterocycloalkyl, or 5 to 6 membered heterocycloalkyl). In embodiments, R^{7D} is independently an unsubstituted heterocycloalkyl (e.g., 3 to 8 membered heterocycloalkyl, 3 to 6 membered heterocycloalkyl, or 5 to 6 membered heterocycloalkyl).

In embodiments, R^{7D} is independently R^{38D}-substituted or unsubstituted aryl (e.g., C₆-C₁₀ aryl, C₁₀ aryl, or phenyl). In embodiments, R^{7D} is independently R^{38D}-substituted aryl (e.g., C₆-C₁₀ aryl, C₁₀ aryl, or phenyl). In embodiments, R^{7D} is independently an unsubstituted aryl (e.g., C₆-C₁₀ aryl, C₁₀ aryl, or phenyl). In embodiments, R^{7D} is independently R^{38D}-substituted or unsubstituted heteroaryl (e.g., 5 to 10 membered heteroaryl, 5 to 9 membered heteroaryl, or 5 to 6 membered heteroaryl). In embodiments, R^{7D} is independently R^{38D}-substituted heteroaryl (e.g., 5 to 10 membered heteroaryl, 5 to 9 membered heteroaryl, or 5 to 6 membered heteroaryl). In embodiments, R^{7D} is independently an unsubstituted heteroaryl (e.g., 5 to 10 membered heteroaryl, 5 to 9 membered heteroaryl, or 5 to 6 membered heteroaryl).

R^{38D} is oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In embodiments, R^{38D} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R^{39D}-substituted or unsubstituted alkyl (e.g., C₁-C₈ alkyl, C₁-C₆ alkyl, or C₁-C₄ alkyl), R^{39D}-substituted or unsubstituted heteroalkyl (e.g., 2 to 8 membered heteroalkyl, 2 to 6 membered heteroalkyl, or 2 to 4 membered heteroalkyl), R^{39D}-substituted or unsubstituted cycloalkyl (e.g., C₃-C₈ cycloalkyl, C₃-C₆ cycloalkyl, or C₅-C₆ cycloalkyl), R^{39D}-substituted or unsubstituted heterocycloalkyl (e.g., 3 to 8 membered heterocycloalkyl, 3 to 6 membered heterocycloalkyl, or 5 to 6 membered heterocycloalkyl), R^{39D}-substituted or unsubstituted aryl (e.g., C₆-C₁₀ aryl, C₁₀ aryl, or phenyl), or R^{39D}-substituted or unsubstituted heteroaryl (e.g., 5 to 10 membered heteroaryl, 5 to 9 membered heteroaryl, or 5 to 6 membered heteroaryl).

In embodiments, R^{38D} is unsubstituted phenyl. In embodiments, R^{38D} is unsubstituted methyl. In embodiments, R^{38D} is -CF₃. In embodiments, R^{38D} is -NH₂. In embodiments, R^{38D} is unsubstituted benzyl. In embodiments, R^{38D} is oxo. In embodiments, R^{38D} is halogen. In embodiments, R^{38D} is -CCl₃. In embodiments, R^{38D} is -CBr₃. In embodiments, R^{38D} is -CF₃. In embodiments, R^{38D} is -CI₃. In embodiments, R^{38D} is CHCl₂. In embodiments, R^{38D} is -CHBr₂. In embodiments, R^{38D} is -CHF₂. In embodiments, R^{38D} is -CHI₂. In embodiments, R^{38D} is -CH₂Cl. In embodiments, R^{38D} is -CH₂Br. In embodiments, R^{38D} is -CH₂F. In embodiments, R^{38D} is -CH₂I. In embodiments, R^{38D} is -CN. In embodiments, R^{38D} is -OH. In embodiments, R^{38D} is -NH₂. In embodiments, R^{38D} is -COOH. In embodiments, R^{38D} is -CONH₂. In embodiments, R^{38D} is -NO₂. In embodiments, R^{38D} is -SH. In embodiments, R^{38D} is -SO₃H. In embodiments, R^{38D} is -SO₄H. In embodiments, R^{38D} is -SO₂NH₂. In embodiments, R^{38D} is -NHNH₂. In embodiments, R^{38D} is -ONH₂. In embodiments, R^{38D} is -NHC(O)NHNH₂. In embodiments, R^{38D} is -NHC(O)NH₂. In embodiments, R^{38D} is -NHSO₂H. In embodiments, R^{38D} is -NHC(O)H. In embodiments, R^{38D} is -NHC(O)OH. In embodiments, R^{38D} is -NHOH. In embodiments, R^{38D} is -OCCl₃. In embodiments, R^{38D} is -OCF₃. In embodiments, R^{38D} is -OCBr₃. In embodiments, R^{38D} is -OCI₃. In embodiments, R^{38D} is -OCHCl₂. In embodiments, R^{38D} is -OCHBr₂. In embodiments, R^{38D} is -OCHI₂. In embodiments, R^{38D} is -OCHF₂. In embodiments, R^{38D} is -OCH₂Cl. In embodiments, R^{38D} is -OCH₂Br. In embodiments, R^{38D} is -OCH₂I. In embodiments, R^{38D} is -OCH₂F.

R^{39D} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R^{44D}-substituted or unsubstituted alkyl (e.g., C₁-C₈ alkyl, C₁-C₆ alkyl, or C₁-C₄ alkyl), R^{44D}-substituted or unsubstituted heteroalkyl (e.g., 2 to 8 membered heteroalkyl, 2 to 6 membered heteroalkyl, or 2 to 4 membered heteroalkyl), R^{44D}-substituted or unsubstituted cycloalkyl (e.g., C₃-C₈ cycloalkyl, C₃-C₆ cycloalkyl, or C₅-C₆ cycloalkyl), R^{44D}-substituted or unsubstituted heterocycloalkyl (e.g., 3 to 8 membered heterocycloalkyl, 3 to 6 membered heterocycloalkyl, or 5 to 6 membered heterocycloalkyl), R^{44D}-substituted or unsubstituted aryl (e.g., C₆-C₁₀ aryl, C₁₀ aryl, or phenyl), or R^{44D}-substituted or unsubstituted heteroaryl (e.g., 5 to 10 membered heteroaryl, 5 to 9 membered heteroaryl, or 5 to 6 membered heteroaryl).

In embodiments, R^{39D} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂,
-NHC(O)NHNH₂,
-NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

R^{44D} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

In embodiments, R⁸ is independently hydrogen, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R⁴⁰-substituted or unsubstituted alkyl (e.g., C₁-C₈ alkyl, C₁-C₆ alkyl, or C₁-C₄ alkyl), R⁴⁰-substituted or unsubstituted heteroalkyl (e.g., 2 to 8 membered heteroalkyl, 2 to 6 membered heteroalkyl, or 2 to 4 membered heteroalkyl), R⁴⁰-substituted or unsubstituted cycloalkyl (e.g., C₃-C₈ cycloalkyl, C₃-C₆ cycloalkyl, or C₅-C₆ cycloalkyl), R⁴⁰-substituted or unsubstituted heterocycloalkyl (e.g., 3 to 8 membered heterocycloalkyl, 3 to 6 membered heterocycloalkyl, or 5 to 6 membered heterocycloalkyl), R⁴⁰-substituted or unsubstituted aryl (e.g., C₆-C₁₀ aryl, C₁₀ aryl, or phenyl), or R⁴⁰-substituted or unsubstituted heteroaryl (e.g., 5 to 10 membered heteroaryl, 5 to 9 membered heteroaryl, or 5 to 6 membered heteroaryl). In embodiments, R⁸ is independently
halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R⁴⁰-substituted or unsubstituted alkyl (e.g., C₁-C₈ alkyl, C₁-C₆ alkyl, or C₁-C₄ alkyl), R⁴⁰-substituted or unsubstituted heteroalkyl (e.g., 2 to 8 membered heteroalkyl, 2 to 6 membered heteroalkyl, or 2 to 4 membered heteroalkyl), R⁴⁰-substituted or unsubstituted cycloalkyl (e.g., C₃-C₈ cycloalkyl, C₃-C₆ cycloalkyl, or C₅-C₆ cycloalkyl), R⁴⁰-substituted or unsubstituted heterocycloalkyl (e.g., 3 to 8 membered heterocycloalkyl, 3 to 6 membered heterocycloalkyl, or 5 to 6 membered heterocycloalkyl), R⁴⁰-substituted or unsubstituted aryl (e.g., C₆-C₁₀ aryl, C₁₀ aryl, or phenyl), or R⁴⁰-substituted or unsubstituted heteroaryl (e.g., 5 to 10 membered heteroaryl, 5 to 9 membered heteroaryl, or 5 to 6 membered heteroaryl). In embodiments, R⁸ is independently hydrogen,
halogen, -CCl₃, -CBr₃, -CF₃, -CI₃, -CHCl₂, -CHBr₂,
-CHF₂, -CHI₂, -CH₂Cl, -CH₂Br, -CH₂F, -CH₂I, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -CN, -COH, -COCH₃, -COOH, -COOCH₃, -CONH₂, -COCH₃, -OH, -OCH₃, R⁴⁰-substituted or unsubstituted alkyl, R⁴⁰-substituted or unsubstituted heteroalkyl, R⁴⁰-substituted or unsubstituted cycloalkyl, R⁴⁰-substituted or unsubstituted heterocycloalkyl, R⁴⁰-substituted or unsubstituted aryl, or R⁴⁰-substituted or unsubstituted heteroaryl.

In embodiments, R⁸ is independently R⁴⁰-substituted or unsubstituted alkyl (e.g., C₁-C₈ alkyl, C₁-C₆ alkyl, or C₁-C₄ alkyl). In embodiments, R⁸ is independently R⁴⁰-substituted alkyl (e.g., C₁-C₈ alkyl, C₁-C₆ alkyl, or C₁-C₄ alkyl). In embodiments, R⁸ is independently an unsubstituted alkyl (e.g., C₁-C₈ alkyl, C₁-C₆ alkyl, or C₁-C₄ alkyl). In embodiments, R⁸ is independently R⁴⁰-substituted or unsubstituted heteroalkyl (e.g., 2 to 8 membered heteroalkyl, 2 to 6 membered heteroalkyl, or 2 to 4 membered heteroalkyl). In embodiments, R⁸ is independently R⁴⁰-substituted heteroalkyl (e.g., 2 to 8 membered heteroalkyl, 2 to 6 membered heteroalkyl, or 2 to 4 membered heteroalkyl). In embodiments, R⁸ is independently unsubstituted heteroalkyl (e.g., 2 to 8 membered heteroalkyl, 2 to 6 membered heteroalkyl, or 2 to 4 membered heteroalkyl).

In embodiments, R⁸ is independently 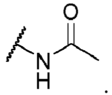 In embodiments, R⁸ is independently 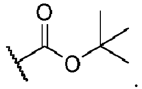 In embodiments, R⁸ is independently -SO₂NH₂.

In embodiments, R⁸ is independently R⁴⁰-substituted or unsubstituted cycloalkyl (e.g., C₃-C₈ cycloalkyl, C₃-C₆ cycloalkyl, or C₅-C₆ cycloalkyl). In embodiments, R⁸ is independently R⁴⁰-substituted cycloalkyl (e.g., C₃-C₈ cycloalkyl, C₃-C₆ cycloalkyl, or C₅-C₆ cycloalkyl). In embodiments, R⁸ is independently unsubstituted cycloalkyl (e.g., C₃-C₈ cycloalkyl, C₃-C₆ cycloalkyl, or C₅-C₆ cycloalkyl). In embodiments, R⁸ is independently R⁴⁰-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R⁸ is independently R⁴⁰-substituted heterocycloalkyl (e.g., 3 to 8 membered heterocycloalkyl, 3 to 6 membered heterocycloalkyl, or 5 to 6 membered heterocycloalkyl). In embodiments, R⁸ is independently unsubstituted heterocycloalkyl (e.g., 3 to 8 membered heterocycloalkyl, 3 to 6 membered heterocycloalkyl, or 5 to 6 membered heterocycloalkyl).

In embodiments, R⁸ is independently R⁴⁰-substituted or unsubstituted aryl (e.g., C₆-C₁₀ aryl, C₁₀ aryl, or phenyl). In embodiments, R⁸ is independently R⁴⁰-substituted aryl (e.g., C₆-C₁₀ aryl, C₁₀ aryl, or phenyl). In embodiments, R⁸ is independently unsubstituted aryl (e.g., C₆-C₁₀ aryl, C₁₀ aryl, or phenyl). In embodiments, R⁸ is independently R⁴⁰-substituted or unsubstituted heteroaryl (e.g., 5 to 10 membered heteroaryl, 5 to 9 membered heteroaryl, or 5 to 6 membered heteroaryl). In embodiments, R⁸ is independently R⁴⁰-substituted heteroaryl (e.g., 5 to 10 membered heteroaryl, 5 to 9 membered heteroaryl, or 5 to 6 membered heteroaryl). In embodiments, R⁸ is independently unsubstituted heteroaryl (e.g., 5 to 10 membered heteroaryl, 5 to 9 membered heteroaryl, or 5 to 6 membered heteroaryl).

In embodiments, R^{8A}, R^{8B}, R^{8C}, and R^{8D} are independently hydrogen, halogen, -CF₃, -CBr₃, -CCl₃, -CI₃, -CHF₂, -CHBr₂, -CHCl₂, -CHI₂, -CH₂F, -CH₂Br, -CH₂Cl, -CH₂I, -OCF₃, -OCBr₃, -OCCl₃, -OCI₃, -OCHF₂, -OCHBr₂, -OCHCl₂, -OCHI₂, -OCH₂F, -OCH₂Br, -OCH₂Cl, -OCH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, R⁴⁰-substituted or unsubstituted alkyl (e.g., C₁-C₈ alkyl, C₁-C₆ alkyl, or C₁-C₄ alkyl), R⁴⁰-substituted or unsubstituted heteroalkyl (e.g., 2 to 8 membered heteroalkyl, 2 to 6 membered heteroalkyl, or 2 to 4 membered heteroalkyl), R⁴⁰-substituted or unsubstituted cycloalkyl (e.g., C₃-C₈ cycloalkyl, C₃-C₆ cycloalkyl, or C₅-C₆ cycloalkyl), R⁴⁰-substituted or unsubstituted heterocycloalkyl (e.g., 3 to 8 membered heterocycloalkyl, 3 to 6 membered heterocycloalkyl, or 5 to 6 membered heterocycloalkyl), R⁴⁰-substituted or unsubstituted aryl (e.g., C₆-C₁₀ aryl, C₁₀ aryl, or phenyl), or R⁴⁰-substituted or unsubstituted heteroaryl (e.g., 5 to 10 membered heteroaryl, 5 to 9 membered heteroaryl, or 5 to 6 membered heteroaryl). In embodiments, R^{8A}, R^{8B}, R^{8C}, and R^{8D} are independently hydrogen.

In embodiments, R^{8A}, R^{8B}, R^{8C}, and R^{8D} are independently R⁴⁰-substituted or unsubstituted alkyl (e.g., C₁-C₈ alkyl, C₁-C₆ alkyl, or C₁-C₄ alkyl). In embodiments, R^{8A}, R^{8B}, R^{8C}, and R^{8D} are independently R⁴⁰-substituted alkyl (e.g., C₁-C₈ alkyl, C₁-C₆ alkyl, or C₁-C₄ alkyl). In embodiments, R^{8A}, R^{8B}, R^{8C}, and R^{8D} are independently an unsubstituted alkyl (e.g., C₁-C₈ alkyl, C₁-C₆ alkyl, or C₁-C₄ alkyl). In embodiments, R^{8A}, R^{8B}, R^{8C}, R^{8D} are independently R⁴⁰-substituted or unsubstituted heteroalkyl (e.g., 2 to 8 membered heteroalkyl, 2 to 6 membered heteroalkyl, or 2 to 4 membered heteroalkyl). In embodiments, R^{8A}, R^{8B}, R^{8C}, and R^{8D} are independently R⁴⁰-substituted heteroalkyl (e.g., 2 to 8 membered heteroalkyl, 2 to 6 membered heteroalkyl, or 2 to 4 membered heteroalkyl). In embodiments, R^{8A}, R^{8B}, R^{8C}, and R^{8D} are independently an unsubstituted heteroalkyl (e.g., 2 to 8 membered heteroalkyl, 2 to 6 membered heteroalkyl, or 2 to 4 membered heteroalkyl).

In embodiments, R^{8A}, R^{8B}, R^{8C}, and R^{8D} are independently R⁴⁰-substituted or unsubstituted cycloalkyl (e.g., C₃-C₈ cycloalkyl, C₃-C₆ cycloalkyl, or C₅-C₆ cycloalkyl). In embodiments, R^{8A}, R^{8B}, R^{8C}, and R^{8D} are independently R⁴⁰-substituted cycloalkyl (e.g., C₃-Cs cycloalkyl, C₃-C₆ cycloalkyl, or C₅-C₆ cycloalkyl). In embodiments, R^{8A}, R^{8B}, R^{8C}, and R^{8D} are independently unsubstituted cycloalkyl (e.g., C₃-C₈ cycloalkyl, C₃-C₆ cycloalkyl, or C₅-C₆ cycloalkyl). In embodiments, R^{8A}, R^{8B}, R^{8C}, and R^{8D} are independently R⁴⁰-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R^{8A}, R^{8B}, R^{8C}, and R^{8D} are independently R⁴⁰-substituted heterocycloalkyl (e.g., 3 to 8 membered heterocycloalkyl, 3 to 6 membered heterocycloalkyl, or 5 to 6 membered heterocycloalkyl). In embodiments, R^{8A}, R^{8B}, R^{8C}, and R^{8D} are independently unsubstituted heterocycloalkyl (e.g., 3 to 8 membered heterocycloalkyl, 3 to 6 membered heterocycloalkyl, or 5 to 6 membered heterocycloalkyl).

In embodiments, R^{8A}, R^{8B}, R^{8C}, and R^{8D} are independently R⁴⁰-substituted or unsubstituted aryl (e.g., C₆-C₁₀ aryl, C₁₀ aryl, or phenyl). In embodiments, R^{8A}, R^{8B}, R^{8C}, and R^{8D} are independently R⁴⁰-substituted aryl (e.g., C₆-C₁₀ aryl, C₁₀ aryl, or phenyl). In embodiments, R^{8A}, R^{8B}, R^{8C}, and R^{8D} are independently unsubstituted aryl (e.g., C₆-C₁₀ aryl, C₁₀ aryl, or phenyl). In embodiments, R^{8A}, R^{8B}, R^{8C}, and R^{8D} are independently R⁴⁰-substituted or unsubstituted heteroaryl (e.g., 5 to 10 membered heteroaryl, 5 to 9 membered heteroaryl, or 5 to 6 membered heteroaryl). In embodiments, R^{8A}, R^{8B}, R^{8C}, and R^{8D} are independently R⁴⁰-substituted heteroaryl (e.g., 5 to 10 membered heteroaryl, 5 to 9 membered heteroaryl, or 5 to 6 membered heteroaryl). In embodiments, R^{8A}, R^{8B}, R^{8C}, and R^{8D} are independently unsubstituted heteroaryl (e.g., 5 to 10 membered heteroaryl, 5 to 9 membered heteroaryl, or 5 to 6 membered heteroaryl).

In embodiments, R⁸ is independently halogen. In embodiments, R⁸ is independently -CX⁸₃ (e.g., -CF₃, -CBr₃, -CCl₃, or -CI₃). In embodiments, R⁸ is independently -CHX⁸₂ (e.g., -CHF₂, -CHBr₂, -CHCl₂, or -CHI₂). In embodiments, R⁸ is independently -CH₂X⁸ (e.g., -CH₂F, -CH₂Br, -CH₂Cl, or -CH₂I). In embodiments, R⁸ is independently -OCX⁸₃ (e.g., -OCF₃, -OCBr₃, -OCCl₃, or -OCI₃). In embodiments, R⁸ is independently -OCH₂X⁸ (e.g., -OCH₂F, -OCH₂Br, -OCH₂Cl, or -OCH₂I). In embodiments, R⁸ is independently -OCHX⁸₂ (e.g., -OCHF₂, -OCHBr₂, -OCHCl₂, or -OCHI₂). In embodiments, R⁸ is independently -C(O)R^{8C} (e.g., -COH, -COCH₃, or -COCH₂CH₃). In embodiments, R⁸ is independently -C(O)OR^{8C} (e.g., -COOH, -COOCH₃, or -COOCH₂CH₃). In embodiments, R⁸ is independently -C(O)NR^{8A}R^{8B} (e.g., -CONH₂, or -CONHCH₃). In embodiments, R⁸ is independently -OR^{8D} (e.g., -OH, or -OCH₃),

R⁴⁰ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In embodiments, R⁴⁰ is independently oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R⁴¹-substituted or unsubstituted alkyl (e.g., C₁-C₈ alkyl, C₁-C₆ alkyl, or C₁-C₄ alkyl), R⁴¹-substituted or unsubstituted heteroalkyl (e.g., 2 to 8 membered heteroalkyl, 2 to 6 membered heteroalkyl, or 2 to 4 membered heteroalkyl), R⁴¹-substituted or unsubstituted cycloalkyl (e.g., C₃-C₈ cycloalkyl, C₃-C₆ cycloalkyl, or C₅-C₆ cycloalkyl), R⁴¹-substituted or unsubstituted heterocycloalkyl (e.g., 3 to 8 membered heterocycloalkyl, 3 to 6 membered heterocycloalkyl, or 5 to 6 membered heterocycloalkyl), R⁴¹-substituted or unsubstituted aryl (e.g., C₆-C₁₀ aryl, C₁₀ aryl, or phenyl), or R⁴¹-substituted or unsubstituted heteroaryl (e.g., 5 to 10 membered heteroaryl, 5 to 9 membered heteroaryl, or 5 to 6 membered heteroaryl).

In embodiments, R⁴⁰ is independently unsubstituted phenyl. In embodiments, R⁴⁰ is independently unsubstituted methyl. In embodiments, R⁴⁰ is independently -CF₃. In embodiments, R⁴⁰ is independently -NH₂. In embodiments, R⁴⁰ is independently unsubstituted benzyl. In embodiments, R⁴⁰ is independently oxo. In embodiments, R⁴⁰ is independently halogen. In embodiments, R⁴⁰ is independently -CCl₃. In embodiments, R⁴⁰ is independently -CBr₃. In embodiments, R⁴⁰ is independently -CF₃. In embodiments, R⁴⁰ is independently -CI₃. In embodiments, R⁴⁰ is independently -CHCl₂. In embodiments, R⁴⁰ is independently -CHBr₂. In embodiments, R⁴⁰ is independently -CHF₂. In embodiments, R⁴⁰ is independently -CHI₂. In embodiments, R⁴⁰ is independently -CH₂Cl. In embodiments, R⁴⁰ is independently -CH₂Br. In embodiments, R⁴⁰ is independently -CH₂F. In embodiments, R⁴⁰ is independently -CH₂I. In embodiments, R⁴⁰ is independently -CN. In embodiments, R⁴⁰ is independently -OH. In embodiments, R⁴⁰ is independently -NH₂. In embodiments, R⁴⁰ is independently -COOH. In embodiments, R⁴⁰ is independently -CONH₂. In embodiments, R⁴⁰ is independently -NO₂. In embodiments, R⁴⁰ is independently -SH. In embodiments, R⁴⁰ is independently -SO₃H. In embodiments, R⁴⁰ is independently -SO₄H. In embodiments, R⁴⁰ is independently -SO₂NH₂. In embodiments, R⁴⁰ is independently -NHNH₂. In embodiments, R⁴⁰ is independently -ONH₂. In embodiments, R⁴⁰ is independently -NHC(O)NHNH₂. In embodiments, R⁴⁰ is independently -NHC(O)NH₂. In embodiments, R⁴⁰ is independently -NHSO₂H. In embodiments, R⁴⁰ is independently -NHC(O)H. In embodiments, R⁴⁰ is independently -NHC(O)OH. In embodiments, R⁴⁰ is independently -NHOH. In embodiments, R⁴⁰ is independently -OCCl₃. In embodiments, R⁴⁰ is independently -OCF₃. In embodiments, R⁴⁰ is independently -OCBr₃. In embodiments, R⁴⁰ is independently -OCI₃. In embodiments, R⁴⁰ is independently -OCHCl₂. In embodiments, R⁴⁰ is independently -OCHBr₂. In embodiments, R⁴⁰ is independently -OCHI₂. In embodiments, R⁴⁰ is independently -OCHF₂. In embodiments, R⁴⁰ is independently -OCH₂Cl. In embodiments, R⁴⁰ is independently -OCH₂Br. In embodiments, R⁴⁰ is independently -OCH₂I. In embodiments, R⁴⁰ is independently -OCH₂F.

In embodiments, R⁴⁰ is independently R⁴¹-substituted or unsubstituted alkyl (e.g., C₁-C₈ alkyl, C₁-C₆ alkyl, or C₁-C₄ alkyl). In embodiments, R⁴⁰ is independently R⁴¹-substituted alkyl (e.g., C₁-C₈ alkyl, C₁-C₆ alkyl, or C₁-C₄ alkyl). In embodiments, R⁴⁰ is independently an unsubstituted alkyl (e.g., C₁-C₈ alkyl, C₁-C₆ alkyl, or C₁-C₄ alkyl).

In embodiments, R⁴⁰ is independently R⁴¹-substituted or unsubstituted heteroalkyl (e.g., 2 to 8 membered heteroalkyl, 2 to 6 membered heteroalkyl, or 2 to 4 membered heteroalkyl). In embodiments, R⁴⁰ is independently R⁴¹-substituted heteroalkyl (e.g., 2 to 8 membered heteroalkyl, 2 to 6 membered heteroalkyl, or 2 to 4 membered heteroalkyl). In embodiments, R⁴⁰ is independently an unsubstituted heteroalkyl (e.g., 2 to 8 membered heteroalkyl, 2 to 6 membered heteroalkyl, or 2 to 4 membered heteroalkyl).

In embodiments, R⁴⁰ is independently R⁴¹-substituted or unsubstituted cycloalkyl (e.g., C₃-C₈ cycloalkyl, C₃-C₆ cycloalkyl, or C₅-C₆ cycloalkyl). In embodiments, R⁴⁰ is independently R⁴¹-substituted cycloalkyl (e.g., C₃-C₈ cycloalkyl, C₃-C₆ cycloalkyl, or C₅-C₆ cycloalkyl). In embodiments, R⁴⁰ is independently an unsubstituted cycloalkyl (e.g., C₃-C₈ cycloalkyl, C₃-C₆ cycloalkyl, or C₅-C₆ cycloalkyl).

In embodiments, R⁴⁰ is independently R⁴¹-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R⁴⁰ is independently R⁴¹-substituted heterocycloalkyl (e.g., 3 to 8 membered heterocycloalkyl, 3 to 6 membered heterocycloalkyl, or 5 to 6 membered heterocycloalkyl). In embodiments, R⁴⁰ is independently an unsubstituted heterocycloalkyl (e.g., 3 to 8 membered heterocycloalkyl, 3 to 6 membered heterocycloalkyl, or 5 to 6 membered heterocycloalkyl).

In embodiments, R⁴⁰ is independently R⁴¹-substituted or unsubstituted aryl (e.g., C₆-C₁₀ aryl, C₁₀ aryl, or phenyl). In embodiments, R⁴⁰ is independently R⁴¹-substituted aryl (e.g., C₆-C₁₀ aryl, C₁₀ aryl, or phenyl). In embodiments, R⁴⁰ is independently an unsubstituted aryl (e.g., C₆-C₁₀ aryl, C₁₀ aryl, or phenyl).

In embodiments, R⁴⁰ is independently R⁴¹-substituted or unsubstituted heteroaryl (e.g., 5 to 10 membered heteroaryl, 5 to 9 membered heteroaryl, or 5 to 6 membered heteroaryl). In embodiments, R⁴⁰ is independently R⁴¹-substituted heteroaryl (e.g., 5 to 10 membered heteroaryl, 5 to 9 membered heteroaryl, or 5 to 6 membered heteroaryl). In embodiments, R⁴⁰ is independently an unsubstituted heteroaryl (e.g., 5 to 10 membered heteroaryl, 5 to 9 membered heteroaryl, or 5 to 6 membered heteroaryl).

R⁴¹ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R⁴⁵-substituted or unsubstituted alkyl (e.g., C₁-C₈ alkyl, C₁-C₆ alkyl, or C₁-C₄ alkyl), R⁴⁵-substituted or unsubstituted heteroalkyl (e.g., 2 to 8 membered heteroalkyl, 2 to 6 membered heteroalkyl, or 2 to 4 membered heteroalkyl), R⁴⁵-substituted or unsubstituted cycloalkyl (e.g., C₃-C₈ cycloalkyl, C₃-C₆ cycloalkyl, or C₅-C₆ cycloalkyl), R⁴⁵-substituted or unsubstituted heterocycloalkyl (e.g., 3 to 8 membered heterocycloalkyl, 3 to 6 membered heterocycloalkyl, or 5 to 6 membered heterocycloalkyl), R⁴⁵-substituted or unsubstituted aryl (e.g., C₆-C₁₀ aryl, C₁₀ aryl, or phenyl), or R⁴⁵-substituted or unsubstituted heteroaryl (e.g., 5 to 10 membered heteroaryl, 5 to 9 membered heteroaryl, or 5 to 6 membered heteroaryl).

In embodiments, R⁴¹ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

R⁴⁵ is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

In embodiments, R⁸ is independently halogen, -CX⁸₃, -CHX⁸₂, -CH₂X⁸, -OCX⁸₃, -OCH₂X⁸, -OCHX⁸₂, -NR^{8A}R^{8B}, -C(O)R^{8C}, substituted or unsubstituted C₁-C₆ alkyl, substituted or unsubstituted 2 to 6 membered heteroalkyl. In embodiments, R⁸ is independently halogen, -CX⁸₃, -CHX⁸₂, -CH₂X⁸, -OCX⁸₃, -OCH₂X⁸, -OCHX⁸₂, -CN, -SR^{8D}, -SO₂R^{8D}, -NR^{8A}R^{8B}, -C(O)R^{8C}, -C(O)OR^{8C}, -C(O)NR^{8A}R^{8B}, -OR^{8D}, -NR^{8A}C(O)R^{8C}, -NR^{8A}C(O)OR^{8C}, substituted or unsubstituted C₁-C₆ alkyl, substituted or unsubstituted 2 to 6 membered heteroalkyl. In embodiments, R⁸ is independently -C(O)R^{8C}, -C(O)OR^{8C}, -C(O)NR^{8A}R^{8B}, -OR^{8D}, -NR^{8A}C(O)R^{8C}, -NR^{8A}C(O)OR^{8C}, substituted or unsubstituted C₁-C₆ alkyl, substituted or unsubstituted 2 to 6 membered heteroalkyl. In embodiments, R⁸ is independently -F, -OH, -OCH₃, -OCH₂CH₃, -CH₂OH, -CH₂OCH₃, -NHC(O)CH₃, -CH₃, -N(CH₃)₂, -CH₂NH₂, -CH₂N(CH₃)₂, -NH₂, -NHCH₃, -COOH, or -SO₂CH₃. In embodiments, R⁸ is independently -OH, -OCH₃, -OCH₂CH₃, -CH₂OH, or -CH₂OCH₃. In embodiments, R⁸ is independently -OH. In embodiments, R⁸ is independently substituted or unsubstituted C₁-C₆ alkyl, or substituted or unsubstituted 2 to 6 membered heteroalkyl. In embodiments, R⁸ is independently R⁴⁰-substituted or unsubstituted C₁-C₆ alkyl, or R⁴⁰-substituted or unsubstituted 2 to 6 membered heteroalkyl. In embodiments, R⁸ is independently unsubstituted C1-C4 alkyl. In embodiments, R⁸ is independently unsubstituted methyl.

In embodiments, R^{8A} is independently hydrogen, halogen, -CF₃, -CBr₃, -CCl₃, -CI₃, -CHF₂, -CHBr₂, -CHCl₂, -CHI₂, -CH₂F, -CH₂Br, -CH₂Cl, -CH₂I, -OCF₃, -OCBr₃, -OCCl₃, -OCI₃, -OCHF₂, -OCHBr₂, -OCHCl₂, -OCHI₂, -OCH₂F, -OCH₂Br, -OCH₂Cl, -OCH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, R^{40A}-substituted or unsubstituted alkyl (e.g., C₁-C₈ alkyl, C₁-C₆ alkyl, or C₁-C₄ alkyl), R^{40A}-substituted or unsubstituted heteroalkyl (e.g., 2 to 8 membered heteroalkyl, 2 to 6 membered heteroalkyl, or 2 to 4 membered heteroalkyl), R^{40A}-substituted or unsubstituted cycloalkyl (e.g., C₃-C₈ cycloalkyl, C₃-C₆ cycloalkyl, or C₅-C₆ cycloalkyl), R^{40A}-substituted or unsubstituted heterocycloalkyl (e.g., 3 to 8 membered heterocycloalkyl, 3 to 6 membered heterocycloalkyl, or 5 to 6 membered heterocycloalkyl), R^{40A}-substituted or unsubstituted aryl (e.g., C₆-C₁₀ aryl, C₁₀ aryl, or phenyl), or R^{40A}-substituted or unsubstituted heteroaryl (e.g., 5 to 10 membered heteroaryl, 5 to 9 membered heteroaryl, or 5 to 6 membered heteroaryl).

In embodiments, R^{8A} is independently R^{40A}-substituted or unsubstituted alkyl (e.g., C₁-C₈ alkyl, C₁-C₆ alkyl, or C₁-C₄ alkyl). In embodiments, R^{8A} is independently R^{40A}-substituted alkyl (e.g., C₁-C₈ alkyl, C₁-C₆ alkyl, or C₁-C₄ alkyl). In embodiments, R^{8A} is independently an unsubstituted alkyl (e.g., C₁-C₈ alkyl, C₁-C₆ alkyl, or C₁-C₄ alkyl). In embodiments, R^{8A} is independently R^{40A}-substituted or unsubstituted heteroalkyl (e.g., 2 to 8 membered heteroalkyl, 2 to 6 membered heteroalkyl, or 2 to 4 membered heteroalkyl). In embodiments, R^{8A} is independently R^{40A}-substituted heteroalkyl (e.g., 2 to 8 membered heteroalkyl, 2 to 6 membered heteroalkyl, or 2 to 4 membered heteroalkyl). In embodiments, R^{8A} is independently an unsubstituted heteroalkyl (e.g., 2 to 8 membered heteroalkyl, 2 to 6 membered heteroalkyl, or 2 to 4 membered heteroalkyl).

In embodiments, R^{8A} is independently R^{40A}-substituted or unsubstituted cycloalkyl (e.g., C₃-C₈ cycloalkyl, C₃-C₆ cycloalkyl, or C₅-C₆ cycloalkyl). In embodiments, R^{8A} is independently R^{40A}-substituted cycloalkyl (e.g., C₃-C₈ cycloalkyl, C₃-C₆ cycloalkyl, or C₅-C₆ cycloalkyl). In embodiments, R^{8A} is independently an unsubstituted cycloalkyl (e.g., C₃-C₈ cycloalkyl, C₃-C₆ cycloalkyl, or C₅-C₆ cycloalkyl). In embodiments, R^{8A} is independently R^{40A}-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R^{8A} is independently R^{40A}-substituted heterocycloalkyl (e.g., 3 to 8 membered heterocycloalkyl, 3 to 6 membered heterocycloalkyl, or 5 to 6 membered heterocycloalkyl). In embodiments, R^{8A} is independently unsubstituted heterocycloalkyl (e.g., 3 to 8 membered heterocycloalkyl, 3 to 6 membered heterocycloalkyl, or 5 to 6 membered heterocycloalkyl).

In embodiments, R^{8A} is independently R^{40A}-substituted or unsubstituted aryl (e.g., C₆-C₁₀ aryl, C₁₀ aryl, or phenyl). In embodiments, R^{8A} is independently R^{40A}-substituted aryl (e.g., C₆-C₁₀ aryl, C₁₀ aryl, or phenyl). In embodiments, R^{8A} is independently an unsubstituted aryl (e.g., C₆-C₁₀ aryl, C₁₀ aryl, or phenyl). In embodiments, R^{8A} is independently R^{40A}-substituted or unsubstituted heteroaryl (e.g., 5 to 10 membered heteroaryl, 5 to 9 membered heteroaryl, or 5 to 6 membered heteroaryl). In embodiments, R^{8A} is independently R^{40A}-substituted heteroaryl (e.g., 5 to 10 membered heteroaryl, 5 to 9 membered heteroaryl, or 5 to 6 membered heteroaryl). In embodiments, R^{8A} is independently an unsubstituted heteroaryl (e.g., 5 to 10 membered heteroaryl, 5 to 9 membered heteroaryl, or 5 to 6 membered heteroaryl).

R^{40A} is oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In embodiments, R^{40A} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R^{41A}-substituted or unsubstituted alkyl (e.g., C₁-C₈ alkyl, C₁-C₆ alkyl, or C₁-C₄ alkyl), R^{41A}-substituted or unsubstituted heteroalkyl (e.g., 2 to 8 membered heteroalkyl, 2 to 6 membered heteroalkyl, or 2 to 4 membered heteroalkyl), R^{41A}-substituted or unsubstituted cycloalkyl (e.g., C₃-C₈ cycloalkyl, C₃-C₆ cycloalkyl, or C₅-C₆ cycloalkyl), R^{41A}-substituted or unsubstituted heterocycloalkyl (e.g., 3 to 8 membered heterocycloalkyl, 3 to 6 membered heterocycloalkyl, or 5 to 6 membered heterocycloalkyl), R^{41A}-substituted or unsubstituted aryl (e.g., C₆-C₁₀ aryl, C₁₀ aryl, or phenyl), or R^{41A}-substituted or unsubstituted heteroaryl (e.g., 5 to 10 membered heteroaryl, 5 to 9 membered heteroaryl, or 5 to 6 membered heteroaryl).

In embodiments, R^{40A} is independently unsubstituted phenyl. In embodiments, R^{40A} is independently unsubstituted methyl. In embodiments, R^{40A} is independently -CF₃. In embodiments, R^{40A} is independently -NH₂. In embodiments, R^{40A} is independently unsubstituted benzyl. In embodiments, R^{40A} is independently oxo. In embodiments, R^{40A} is independently halogen. In embodiments, R^{40A} is independently -CCl₃. In embodiments, R^{40A} is independently -CBr₃. In embodiments, R^{40A} is independently -CF₃. In embodiments, R^{40A} is independently -CI₃. In embodiments, R^{40A} is independently -CHCl₂. In embodiments, R^{40A} is independently -CHBr₂. In embodiments, R^{40A} is independently -CHF₂. In embodiments, R^{40A} is independently -CHI₂. In embodiments, R^{40A} is independently -CH₂Cl. In embodiments, R^{40A} is independently -CH₂Br. In embodiments, R^{40A} is independently -CH₂F. In embodiments, R^{40A} is independently -CH₂I. In embodiments, R^{40A} is independently -CN. In embodiments, R^{40A} is independently -OH. In embodiments, R^{40A} is independently -NH₂. In embodiments, R^{40A} is independently -COOH. In embodiments, R^{40A} is independently -CONH₂. In embodiments, R^{40A} is independently -NO₂. In embodiments, R^{40A} is independently -SH. In embodiments, R^{40A} is independently -SO₃H. In embodiments, R^{40A} is independently -SO₄H. In embodiments, R^{40A} is independently -SO₂NH₂. In embodiments, R^{40A} is independently -NHNH₂. In embodiments, R^{40A} is independently -ONH₂. In embodiments, R^{40A} is independently -NHC(O)NHNH₂. In embodiments, R^{40A} is independently -NHC(O)NH₂. In embodiments, R^{40A} is independently -NHSO₂H. In embodiments, R^{40A} is independently -NHC(O)H. In embodiments, R^{40A} is independently -NHC(O)OH. In embodiments, R^{40A} is independently -NHOH. In embodiments, R^{40A} is independently -OCCl₃. In embodiments, R^{40A} is independently -OCF₃. In embodiments, R^{40A} is independently -OCBr₃. In embodiments, R^{40A} is independently -OCI₃. In embodiments, R^{40A} is independently -OCHCl₂. In embodiments, R^{40A} is independently -OCHBr₂. In embodiments, R^{40A} is independently -OCHI₂. In embodiments, R^{40A} is independently -OCHF₂. In embodiments, R^{40A} is independently -OCH₂Cl. In embodiments, R^{40A} is independently -OCH₂Br. In embodiments, R^{40A} is independently -OCH₂I. In embodiments, R^{40A} is independently -OCH₂F.

R^{41A} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R^{45A}-substituted or unsubstituted alkyl (e.g., C₁-C₈ alkyl, C₁-C₆ alkyl, or C₁-C₄ alkyl), R^{45A}-substituted or unsubstituted heteroalkyl (e.g., 2 to 8 membered heteroalkyl, 2 to 6 membered heteroalkyl, or 2 to 4 membered heteroalkyl), R^{45A}-substituted or unsubstituted cycloalkyl (e.g., C₃-C₈ cycloalkyl, C₃-C₆ cycloalkyl, or C₅-C₆ cycloalkyl), R^{45A}-substituted or unsubstituted heterocycloalkyl (e.g., 3 to 8 membered heterocycloalkyl, 3 to 6 membered heterocycloalkyl, or 5 to 6 membered heterocycloalkyl), R^{45A}-substituted or unsubstituted aryl (e.g., C₆-C₁₀ aryl, C₁₀ aryl, or phenyl), or R^{45A}-substituted or unsubstituted heteroaryl (e.g., 5 to 10 membered heteroaryl, 5 to 9 membered heteroaryl, or 5 to 6 membered heteroaryl).

In embodiments, R^{41A} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

R^{45A} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

In embodiments, R^{8B} is independently hydrogen, halogen, -CF₃, -CBr₃, -CCl₃, -CI₃, -CHF₂, -CHBr₂, -CHCl₂, -CHI₂, -CH₂F, -CH₂Br, -CH₂Cl, -CH₂I, -OCF₃, -OCBr₃, -OCCl₃, -OCI₃, -OCHF₂, -OCHBr₂, -OCHCl₂, -OCHI₂, -OCH₂F, -OCH₂Br, -OCH₂Cl, -OCH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, R^{40B}-substituted or unsubstituted alkyl (e.g., C₁-C₈ alkyl, C₁-C₆ alkyl, or C₁-C₄ alkyl), R^{40B}-substituted or unsubstituted heteroalkyl (e.g., 2 to 8 membered heteroalkyl, 2 to 6 membered heteroalkyl, or 2 to 4 membered heteroalkyl), R^{40B}-substituted or unsubstituted cycloalkyl (e.g., C₃-C₈ cycloalkyl, C₃-C₆ cycloalkyl, or C₅-C₆ cycloalkyl), R^{40B}-substituted or unsubstituted heterocycloalkyl (e.g., 3 to 8 membered heterocycloalkyl, 3 to 6 membered heterocycloalkyl, or 5 to 6 membered heterocycloalkyl), R^{40B}-substituted or unsubstituted aryl (e.g., C₆-C₁₀ aryl, C₁₀ aryl, or phenyl), or R^{40B}-substituted or unsubstituted heteroaryl (e.g., 5 to 10 membered heteroaryl, 5 to 9 membered heteroaryl, or 5 to 6 membered heteroaryl).

In embodiments, R^{8B} is independently R^{40B}-substituted or unsubstituted alkyl (e.g., C₁-C₈ alkyl, C₁-C₆ alkyl, or C₁-C₄ alkyl). In embodiments, R^{8B} is independently R^{40B}-substituted alkyl (e.g., C₁-C₈ alkyl, C₁-C₆ alkyl, or C₁-C₄ alkyl). In embodiments, R^{8B} is independently an unsubstituted alkyl (e.g., C₁-C₈ alkyl, C₁-C₆ alkyl, or C₁-C₄ alkyl). In embodiments, R^{8B} is independently R^{40B}-substituted or unsubstituted heteroalkyl (e.g., 2 to 8 membered heteroalkyl, 2 to 6 membered heteroalkyl, or 2 to 4 membered heteroalkyl). In embodiments, R^{8B} is independently R^{40B}-substituted heteroalkyl (e.g., 2 to 8 membered heteroalkyl, 2 to 6 membered heteroalkyl, or 2 to 4 membered heteroalkyl). In embodiments, R^{8B} is independently an unsubstituted heteroalkyl (e.g., 2 to 8 membered heteroalkyl, 2 to 6 membered heteroalkyl, or 2 to 4 membered heteroalkyl).

In embodiments, R^{8B} is independently R^{40B}-substituted or unsubstituted cycloalkyl (e.g., C₃-C₈ cycloalkyl, C₃-C₆ cycloalkyl, or C₅-C₆ cycloalkyl). In embodiments, R^{8B} is independently R^{40B}-substituted cycloalkyl (e.g., C₃-C₈ cycloalkyl, C₃-C₆ cycloalkyl, or C₅-C₆ cycloalkyl). In embodiments, R^{8B} is independently an unsubstituted cycloalkyl (e.g., C₃-C₈ cycloalkyl, C₃-C₆ cycloalkyl, or C₅-C₆ cycloalkyl). In embodiments, R^{8B} is independently R^{40B}-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R^{8B} is independently R^{40B}-substituted heterocycloalkyl (e.g., 3 to 8 membered heterocycloalkyl, 3 to 6 membered heterocycloalkyl, or 5 to 6 membered heterocycloalkyl). In embodiments, R^{8B} is independently an unsubstituted heterocycloalkyl (e.g., 3 to 8 membered heterocycloalkyl, 3 to 6 membered heterocycloalkyl, or 5 to 6 membered heterocycloalkyl).

In embodiments, R^{8B} is independently R^{40B}-substituted or unsubstituted aryl (e.g., C₆-C₁₀ aryl, C₁₀ aryl, or phenyl). In embodiments, R^{8B} is independently R^{40B}-substituted aryl (e.g., C₆-C₁₀ aryl, C₁₀ aryl, or phenyl). In embodiments, R^{8B} is independently an unsubstituted aryl (e.g., C₆-C₁₀ aryl, C₁₀ aryl, or phenyl). In embodiments, R^{8B} is independently R^{40B}-substituted or unsubstituted heteroaryl (e.g., 5 to 10 membered heteroaryl, 5 to 9 membered heteroaryl, or 5 to 6 membered heteroaryl). In embodiments, R^{8B} is independently R^{40B}-substituted heteroaryl (e.g., 5 to 10 membered heteroaryl, 5 to 9 membered heteroaryl, or 5 to 6 membered heteroaryl). In embodiments, R^{8B} is independently an unsubstituted heteroaryl (e.g., 5 to 10 membered heteroaryl, 5 to 9 membered heteroaryl, or 5 to 6 membered heteroaryl).

R^{40B} is oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In embodiments, R^{40B} is independently oxo, halogen, -CCl₃, -CBr₃, -CF₃,
-CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂,
-NHC(O)NHNH₂,
-NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R^{41B}-substituted or unsubstituted alkyl (e.g., C₁-C₈ alkyl, C₁-C₆ alkyl, or C₁-C₄ alkyl), R^{41B}-substituted or unsubstituted heteroalkyl (e.g., 2 to 8 membered heteroalkyl, 2 to 6 membered heteroalkyl, or 2 to 4 membered heteroalkyl), R^{41B}-substituted or unsubstituted cycloalkyl (e.g., C₃-C₈ cycloalkyl, C₃-C₆ cycloalkyl, or C₅-C₆ cycloalkyl), R^{41B}-substituted or unsubstituted heterocycloalkyl (e.g., 3 to 8 membered heterocycloalkyl, 3 to 6 membered heterocycloalkyl, or 5 to 6 membered heterocycloalkyl), R^{41B}-substituted or unsubstituted aryl (e.g., C₆-C₁₀ aryl, C₁₀ aryl, or phenyl), or R^{41B}-substituted or unsubstituted heteroaryl (e.g., 5 to 10 membered heteroaryl, 5 to 9 membered heteroaryl, or 5 to 6 membered heteroaryl).

In embodiments, R^{40B} is independently unsubstituted phenyl. In embodiments, R^{40B} is independently unsubstituted methyl. In embodiments, R^{40B} is independently -CF₃. In embodiments, R^{40B} is independently -NH₂. In embodiments, R^{40B} is independently unsubstituted benzyl. In embodiments, R^{40B} is independently oxo. In embodiments, R^{40B} is independently halogen. In embodiments, R^{40B} is independently -CCl₃. In embodiments, R^{40B} is independently -CBr₃. In embodiments, R^{40B} is independently -CF₃. In embodiments, R^{40B} is independently -CI₃. In embodiments, R^{40B} is independently -CHCl₂. In embodiments, R^{40B} is independently -CHBr₂. In embodiments, R^{40B} is independently -CHF₂. In embodiments, R^{40B} is independently -CHI₂. In embodiments, R^{40B} is independently -CH₂Cl. In embodiments, R^{40B} is independently -CH₂Br. In embodiments, R^{40B} is independently -CH₂F. In embodiments, R^{40B} is independently -CH₂I. In embodiments, R^{40B} is independently -CN. In embodiments, R^{40B} is independently -OH. In embodiments, R^{40B} is independently -NH₂. In embodiments, R^{40B} is independently -COOH. In embodiments, R^{40B} is independently -CONH₂. In embodiments, R^{40B} is independently -NO₂. In embodiments, R^{40B} is independently -SH. In embodiments, R^{40B} is independently -SO₃H. In embodiments, R^{40B} is independently -SO₄H. In embodiments, R^{40B} is independently -SO₂NH₂. In embodiments, R^{40B} is independently -NHNH₂. In embodiments, R^{40B} is independently -ONH₂. In embodiments, R^{40B} is independently -NHC(O)NHNH₂. In embodiments, R^{40B} is independently -NHC(O)NH₂. In embodiments, R^{40B} is independently -NHSO₂H. In embodiments, R^{40B} is independently -NHC(O)H. In embodiments, R^{40B} is independently -NHC(O)OH. In embodiments, R^{40B} is independently -NHOH. In embodiments, R^{40B} is independently -OCCl₃. In embodiments, R^{40B} is independently -OCF₃. In embodiments, R^{40B} is independently -OCBr₃. In embodiments, R^{40B} is independently -OCI₃. In embodiments, R^{40B} is independently -OCHCl₂. In embodiments, R^{40B} is independently -OCHBr₂. In embodiments, R^{40B} is independently -OCHI₂. In embodiments, R^{40B} is independently -OCHF₂. In embodiments, R^{40B} is independently -OCH₂Cl. In embodiments, R^{40B} is independently -OCH₂Br. In embodiments, R^{40B} is independently -OCH₂I. In embodiments, R^{40B} is independently -OCH₂F.

R^{41B} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R^{45B}-substituted or unsubstituted alkyl (e.g., C₁-C₈ alkyl, C₁-C₆ alkyl, or C₁-C₄ alkyl), R^{45B}-substituted or unsubstituted heteroalkyl (e.g., 2 to 8 membered heteroalkyl, 2 to 6 membered heteroalkyl, or 2 to 4 membered heteroalkyl), R^{45B}-substituted or unsubstituted cycloalkyl (e.g., C₃-C₈ cycloalkyl, C₃-C₆ cycloalkyl, or C₅-C₆ cycloalkyl), R^{45B}-substituted or unsubstituted heterocycloalkyl (e.g., 3 to 8 membered heterocycloalkyl, 3 to 6 membered heterocycloalkyl, or 5 to 6 membered heterocycloalkyl), R^{45B}-substituted or unsubstituted aryl (e.g., C₆-C₁₀ aryl, C₁₀ aryl, or phenyl), or R^{45B}-substituted or unsubstituted heteroaryl (e.g., 5 to 10 membered heteroaryl, 5 to 9 membered heteroaryl, or 5 to 6 membered heteroaryl).

In embodiments, R^{41B} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

R^{45B} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

In embodiments, R^{8C} is independently hydrogen, halogen, -CF₃, -CBr₃, -CCl₃, -CI₃, -CHF₂, -CHBr₂, -CHCl₂, -CHI₂, -CH₂F, -CH₂Br, -CH₂Cl, -CH₂I, -OCF₃, -OCBr₃, -OCCl₃, -OCI₃, -OCHF₂, -OCHBr₂, -OCHCl₂, -OCHI₂, -OCH₂F, -OCH₂Br, -OCH₂Cl, -OCH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, R^{40C}-substituted or unsubstituted alkyl (e.g., C₁-C₈ alkyl, C₁-C₆ alkyl, or C₁-C₄ alkyl), R^{40C}-substituted or unsubstituted heteroalkyl (e.g., 2 to 8 membered heteroalkyl, 2 to 6 membered heteroalkyl, or 2 to 4 membered heteroalkyl), R^{40C}-substituted or unsubstituted cycloalkyl (e.g., C₃-C₈ cycloalkyl, C₃-C₆ cycloalkyl, or C₅-C₆ cycloalkyl), R^{40C}-substituted or unsubstituted heterocycloalkyl (e.g., 3 to 8 membered heterocycloalkyl, 3 to 6 membered heterocycloalkyl, or 5 to 6 membered heterocycloalkyl), R^{40C}-substituted or unsubstituted aryl (e.g., C₆-C₁₀ aryl, C₁₀ aryl, or phenyl), or R^{40C}-substituted or unsubstituted heteroaryl (e.g., 5 to 10 membered heteroaryl, 5 to 9 membered heteroaryl, or 5 to 6 membered heteroaryl).

In embodiments, R^{8C} is independently R^{40C}-substituted or unsubstituted alkyl (e.g., C₁-C₈ alkyl, C₁-C₆ alkyl, or C₁-C₄ alkyl). In embodiments, R^{8C} is independently R^{40C}-substituted alkyl (e.g., C₁-C₈ alkyl, C₁-C₆ alkyl, or C₁-C₄ alkyl). In embodiments, R^{8C} is independently an unsubstituted alkyl (e.g., C₁-C₈ alkyl, C₁-C₆ alkyl, or C₁-C₄ alkyl). In embodiments, R^{8C} is independently R^{40C}-substituted or unsubstituted heteroalkyl (e.g., 2 to 8 membered heteroalkyl, 2 to 6 membered heteroalkyl, or 2 to 4 membered heteroalkyl). In embodiments, R^{8C} is independently R^{40C}-substituted heteroalkyl (e.g., 2 to 8 membered heteroalkyl, 2 to 6 membered heteroalkyl, or 2 to 4 membered heteroalkyl). In embodiments, R^{8C} is independently an unsubstituted heteroalkyl (e.g., 2 to 8 membered heteroalkyl, 2 to 6 membered heteroalkyl, or 2 to 4 membered heteroalkyl).

In embodiments, R^{8C} is independently R^{40C}-substituted or unsubstituted cycloalkyl (e.g., C₃-C₈ cycloalkyl, C₃-C₆ cycloalkyl, or C₅-C₆ cycloalkyl). In embodiments, R^{8C} is independently R^{40C}-substituted cycloalkyl (e.g., C₃-C₈ cycloalkyl, C₃-C₆ cycloalkyl, or C₅-C₆ cycloalkyl). In embodiments, R^{8C} is independently an unsubstituted cycloalkyl (e.g., C₃-C₈ cycloalkyl, C₃-C₆ cycloalkyl, or C₅-C₆ cycloalkyl). In embodiments, R^{8C} is independently R^{40C}-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R^{8C} is independently R^{40C}-substituted heterocycloalkyl (e.g., 3 to 8 membered heterocycloalkyl, 3 to 6 membered heterocycloalkyl, or 5 to 6 membered heterocycloalkyl). In embodiments, R^{8C} is independently an unsubstituted heterocycloalkyl (e.g., 3 to 8 membered heterocycloalkyl, 3 to 6 membered heterocycloalkyl, or 5 to 6 membered heterocycloalkyl).

In embodiments, R^{8C} is independently R^{40C}-substituted or unsubstituted aryl (e.g., C₆-C₁₀ aryl, C₁₀ aryl, or phenyl). In embodiments, R^{8C} is independently R^{40C}-substituted aryl (e.g., C₆-C₁₀ aryl, C₁₀ aryl, or phenyl). In embodiments, R^{8C} is independently an unsubstituted aryl (e.g., C₆-C₁₀ aryl, C₁₀ aryl, or phenyl). In embodiments, R^{8C} is independently R^{40C}-substituted or unsubstituted heteroaryl (e.g., 5 to 10 membered heteroaryl, 5 to 9 membered heteroaryl, or 5 to 6 membered heteroaryl). In embodiments, R^{8C} is independently R^{40C}-substituted heteroaryl (e.g., 5 to 10 membered heteroaryl, 5 to 9 membered heteroaryl, or 5 to 6 membered heteroaryl). In embodiments, R^{8C} is independently an unsubstituted heteroaryl (e.g., 5 to 10 membered heteroaryl, 5 to 9 membered heteroaryl, or 5 to 6 membered heteroaryl).

R^{40C} is oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In embodiments, R^{40C} is independently oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂,
-NHC(O)NHNH₂,
-NHC(O)NH₂, -NHSO₂H, -NHC(O)H, -NHC(O)OH, -NHOH, -OCCl₃, -OCF₃, -OCBr₃, -OC I₃,
-OCHCl₂, -OCHBr₂, -OCHI₂, -OCHF₂, -OCH₂Cl, -OCH₂Br, -OCH₂I, -OCH₂F, -N₃, R^{41C}-substituted or unsubstituted alkyl (e.g., C₁-C₈ alkyl, C₁-C₆ alkyl, or C₁-C₄ alkyl), R^{41C}-substituted or unsubstituted heteroalkyl (e.g., 2 to 8 membered heteroalkyl, 2 to 6 membered heteroalkyl, or 2 to 4 membered heteroalkyl), R^{41C}-substituted or unsubstituted cycloalkyl (e.g., C₃-C₈ cycloalkyl, C₃-C₆ cycloalkyl, or C₅-C₆ cycloalkyl), R^{41C}-substituted or unsubstituted heterocycloalkyl (e.g., 3 to 8 membered heterocycloalkyl, 3 to 6 membered heterocycloalkyl, or 5 to 6 membered heterocycloalkyl), R^{41C}-substituted or unsubstituted aryl (e.g., C₆-C₁₀ aryl, C₁₀ aryl, or phenyl), or R^{41C}-substituted or unsubstituted heteroaryl (e.g., 5 to 10 membered heteroaryl, 5 to 9 membered heteroaryl, or 5 to 6 membered heteroaryl).

In embodiments, R^{40C} is independently unsubstituted phenyl. In embodiments, R^{40C} is independently unsubstituted methyl. In embodiments, R^{40C} is independently -CF₃. In embodiments, R^{40C} is independently -NH₂. In embodiments, R^{40C} is independently unsubstituted benzyl. In embodiments, R^{40C} is independently oxo. In embodiments, R^{40C} is independently halogen. In embodiments, R^{40C} is independently -CCl₃. In embodiments, R^{40C} is independently -CBr₃. In embodiments, R^{40C} is independently -CF₃. In embodiments, R^{40C} is independently -CI₃. In embodiments, R^{40C} is independently -CHCl₂. In embodiments, R^{40C} is independently -CHBr₂. In embodiments, R^{40C} is independently -CHF₂. In embodiments, R^{40C} is independently -CHI₂. In embodiments, R^{40C} is independently -CH₂Cl. In embodiments, R^{40C} is independently -CH₂Br. In embodiments, R^{40C} is independently -CH₂F. In embodiments, R^{40C} is independently -CH₂I. In embodiments, R^{40C} is independently -CN. In embodiments, R^{40C} is independently -OH. In embodiments, R^{40C} is independently -NH₂. In embodiments, R^{40C} is independently -COOH. In embodiments, R^{40C} is independently -CONH₂. In embodiments, R^{40C} is independently -NO₂. In embodiments, R^{40C} is independently -SH. In embodiments, R^{40C} is independently -SO₃H. In embodiments, R^{40C} is independently -SO₄H. In embodiments, R^{40C} is independently -SO₂NH₂. In embodiments, R^{40C} is independently -NHNH₂. In embodiments, R^{40C} is independently -ONH₂. In embodiments, R^{40C} is independently -NHC(O)NHNH₂. In embodiments, R^{40C} is independently -NHC(O)NH₂. In embodiments, R^{40C} is independently -NHSO₂H. In embodiments, R^{40C} is independently -NHC(O)H. In embodiments, R^{40C} is independently -NHC(O)OH. In embodiments, R^{40C} is independently -NHOH. In embodiments, R^{40C} is independently -OCCl₃. In embodiments, R^{40C} is independently -OCF₃. In embodiments, R^{40C} is independently -OCBr₃. In embodiments, R^{40C} is independently -OCI₃. In embodiments, R^{40C} is independently -OCHCl₂. In embodiments, R^{40C} is independently -OCHBr₂. In embodiments, R^{40C} is independently -OCHI₂. In embodiments, R^{40C} is independently -OCHF₂. In embodiments, R^{40C} is independently -OCH₂Cl. In embodiments, R^{40C} is independently -OCH₂Br. In embodiments, R^{40C} is independently -OCH₂I. In embodiments, R^{40C} is independently -OCH₂F.

R^{41C} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R^{45C}-substituted or unsubstituted alkyl (e.g., C₁-C₈ alkyl, C₁-C₆ alkyl, or C₁-C₄ alkyl), R^{45C}-substituted or unsubstituted heteroalkyl (e.g., 2 to 8 membered heteroalkyl, 2 to 6 membered heteroalkyl, or 2 to 4 membered heteroalkyl), R^{45C}-substituted or unsubstituted cycloalkyl (e.g., C₃-C₈ cycloalkyl, C₃-C₆ cycloalkyl, or C₅-C₆ cycloalkyl), R^{45C}-substituted or unsubstituted heterocycloalkyl (e.g., 3 to 8 membered heterocycloalkyl, 3 to 6 membered heterocycloalkyl, or 5 to 6 membered heterocycloalkyl), R^{45C}-substituted or unsubstituted aryl (e.g., C₆-C₁₀ aryl, C₁₀ aryl, or phenyl), or R^{45C}-substituted or unsubstituted heteroaryl (e.g., 5 to 10 membered heteroaryl, 5 to 9 membered heteroaryl, or 5 to 6 membered heteroaryl).

In embodiments, R^{41C} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

R^{45C} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

In embodiments, R^{8D} is independently hydrogen, halogen, -CF₃, -CBr₃, -CCl₃, -CI₃, -CHF₂, -CHBr₂, -CHCl₂, -CHI₂, -CH₂F, -CH₂Br, -CH₂Cl, -CH₂I, -OCF₃, -OCBr₃, -OCCl₃, -OCI₃, -OCHF₂, -OCHBr₂, -OCHCl₂, -OCHI₂, -OCH₂F, -OCH₂Br, -OCH₂Cl, -OCH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, R^{40D}-substituted or unsubstituted alkyl (e.g., C₁-C₈ alkyl, C₁-C₆ alkyl, or C₁-C₄ alkyl), R^{40D}-substituted or unsubstituted heteroalkyl (e.g., 2 to 8 membered heteroalkyl, 2 to 6 membered heteroalkyl, or 2 to 4 membered heteroalkyl), R^{40D}-substituted or unsubstituted cycloalkyl (e.g., C₃-C₈ cycloalkyl, C₃-C₆ cycloalkyl, or C₅-C₆ cycloalkyl), R^{40D}-substituted or unsubstituted heterocycloalkyl (e.g., 3 to 8 membered heterocycloalkyl, 3 to 6 membered heterocycloalkyl, or 5 to 6 membered heterocycloalkyl), R^{40D}-substituted or unsubstituted aryl (e.g., C₆-C₁₀ aryl, C₁₀ aryl, or phenyl), or R^{40D}-substituted or unsubstituted heteroaryl (e.g., 5 to 10 membered heteroaryl, 5 to 9 membered heteroaryl, or 5 to 6 membered heteroaryl). In embodiments, R^{8D} is independently hydrogen.

In embodiments, R^{8D} is independently R^{40D}-substituted or unsubstituted alkyl (e.g., C₁-C₈ alkyl, C₁-C₆ alkyl, or C₁-C₄ alkyl). In embodiments, R^{8D} is independently R^{40D}-substituted alkyl (e.g., C₁-C₈ alkyl, C₁-C₆ alkyl, or C₁-C₄ alkyl). In embodiments, R^{8D} is independently an unsubstituted alkyl (e.g., C₁-C₈ alkyl, C₁-C₆ alkyl, or C₁-C₄ alkyl). In embodiments, R^{8D} is independently R^{40D}-substituted or unsubstituted heteroalkyl (e.g., 2 to 8 membered heteroalkyl, 2 to 6 membered heteroalkyl, or 2 to 4 membered heteroalkyl). In embodiments, R^{8D} is independently R^{40D}-substituted heteroalkyl (e.g., 2 to 8 membered heteroalkyl, 2 to 6 membered heteroalkyl, or 2 to 4 membered heteroalkyl). In embodiments, R^{8D} is independently an unsubstituted heteroalkyl (e.g., 2 to 8 membered heteroalkyl, 2 to 6 membered heteroalkyl, or 2 to 4 membered heteroalkyl).

In embodiments, R^{8D} is independently R^{40D}-substituted or unsubstituted cycloalkyl (e.g., C₃-C₈ cycloalkyl, C₃-C₆ cycloalkyl, or C₅-C₆ cycloalkyl). In embodiments, R^{8D} is independently R^{40D}-substituted cycloalkyl (e.g., C₃-C₈ cycloalkyl, C₃-C₆ cycloalkyl, or C₅-C₆ cycloalkyl). In embodiments, R^{8D} is independently an unsubstituted cycloalkyl (e.g., C₃-C₈ cycloalkyl, C₃-C₆ cycloalkyl, or C₅-C₆ cycloalkyl). In embodiments, R^{8D} is independently R^{40D}-substituted or unsubstituted heterocycloalkyl (e.g., 3 to 8 membered heterocycloalkyl, 3 to 6 membered heterocycloalkyl, or 5 to 6 membered heterocycloalkyl). In embodiments, R^{8D} is independently R^{40D}-substituted heterocycloalkyl (e.g., 3 to 8 membered heterocycloalkyl, 3 to 6 membered heterocycloalkyl, or 5 to 6 membered heterocycloalkyl). In embodiments, R^{8D} is independently an unsubstituted heterocycloalkyl (e.g., 3 to 8 membered heterocycloalkyl, 3 to 6 membered heterocycloalkyl, or 5 to 6 membered heterocycloalkyl).

In embodiments, R^{8D} is independently R^{40D}-substituted or unsubstituted aryl (e.g., C₆-C₁₀ aryl, C₁₀ aryl, or phenyl). In embodiments, R^{8D} is independently R^{40D}-substituted aryl (e.g., C₆-C₁₀ aryl, C₁₀ aryl, or phenyl). In embodiments, R^{8D} is independently an unsubstituted aryl (e.g., C₆-C₁₀ aryl, C₁₀ aryl, or phenyl). In embodiments, R^{8D} is independently R^{40D}-substituted or unsubstituted heteroaryl (e.g., 5 to 10 membered heteroaryl, 5 to 9 membered heteroaryl, or 5 to 6 membered heteroaryl). In embodiments, R^{8D} is independently R^{40D}-substituted heteroaryl (e.g., 5 to 10 membered heteroaryl, 5 to 9 membered heteroaryl, or 5 to 6 membered heteroaryl). In embodiments, R^{8D} is independently an unsubstituted heteroaryl (e.g., 5 to 10 membered heteroaryl, 5 to 9 membered heteroaryl, or 5 to 6 membered heteroaryl).

R^{40D} is oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In embodiments, R^{40D} is independently oxo, halogen, -CCl₃, -CBr₃, -CF₃, -CI₃, CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂,
-NHC(O)NHNH₂,
-NHC(O)NH₂, -NHSO₂H, -NHC(O)H, -NHC(O)OH, -NHOH, -OCCl₃, -OCF₃, -OCBr₃, -O CI₃, -OCHCl₂, -OCHBr₂, -OCHI₂, -OCHF₂, -OCH₂Cl, -OCH₂Br, -OCH₂I, -OCH₂F, -N₃, R^{41D}-substituted or unsubstituted alkyl (e.g., C₁-C₈ alkyl, C₁-C₆ alkyl, or C₁-C₄ alkyl), R^{41D}-substituted or unsubstituted heteroalkyl (e.g., 2 to 8 membered heteroalkyl, 2 to 6 membered heteroalkyl, or 2 to 4 membered heteroalkyl), R^{41D}-substituted or unsubstituted cycloalkyl (e.g., C₃-C₈ cycloalkyl, C₃-C₆ cycloalkyl, or C₅-C₆ cycloalkyl), R^{41D}-substituted or unsubstituted heterocycloalkyl (e.g., 3 to 8 membered heterocycloalkyl, 3 to 6 membered heterocycloalkyl, or 5 to 6 membered heterocycloalkyl), R^{41D}-substituted or unsubstituted aryl (e.g., C₆-C₁₀ aryl, C₁₀ aryl, or phenyl), or R^{41D}-substituted or unsubstituted heteroaryl (e.g., 5 to 10 membered heteroaryl, 5 to 9 membered heteroaryl, or 5 to 6 membered heteroaryl).

In embodiments, R^{40D} is unsubstituted phenyl. In embodiments, R^{40D} is unsubstituted methyl. In embodiments, R^{40D} is -CF₃. In embodiments, R^{40D} is -NH₂. In embodiments, R^{40D} is unsubstituted benzyl. In embodiments, R^{40D} is oxo. In embodiments, R^{40D} is halogen. In embodiments, R^{40D} is -CCl₃. In embodiments, R^{40D} is -CBr₃. In embodiments, R^{40D} is -CF₃. In embodiments, R^{40D} is -CI₃. In embodiments, R^{40D} is -CHCl₂. In embodiments, R^{40D} is -CHBr₂. In embodiments, R^{40D} is -CHF₂. In embodiments, R^{40D} is -CHI₂. In embodiments, R^{40D} is -CH₂Cl. In embodiments, R^{40D} is -CH₂Br. In embodiments, R^{40D} is -CH₂F. In embodiments, R^{40D} is -CH₂I. In embodiments, R^{40D} is -CN. In embodiments, R^{40D} is -OH. In embodiments, R^{40D} is -NH₂. In embodiments, R^{40D} is -COOH. In embodiments, R^{40D} is -CONH₂. In embodiments, R^{40D} is -NO₂. In embodiments, R^{40D} is -SH. In embodiments, R^{40D} is -SO₃H. In embodiments, R^{40D} is -SO₄H. In embodiments, R^{40D} is -SO₂NH₂. In embodiments, R^{40D} is -NHNH₂. In embodiments, R^{40D} is -ONH₂. In embodiments, R^{40D} is -NHC(O)NHNH₂. In embodiments, R^{40D} is -NHC(O)NH₂. In embodiments, R^{40D} is -NHSO₂H. In embodiments, R^{40D} is -NHC(O)H. In embodiments, R^{40D} is -NHC(O)OH. In embodiments, R^{40D} is -NHOH. In embodiments, R^{40D} is -OCCl₃. In embodiments, R^{40D} is -OCF₃. In embodiments, R^{40D} is -OCBr₃. In embodiments, R^{40D} is -OCI₃. In embodiments, R^{40D} is -OCHCl₂. In embodiments, R^{40D} is -OCHBr₂. In embodiments, R^{40D} is -OCHI₂. In embodiments, R^{40D} is -OCHF₂. In embodiments, R^{40D} is -OCH₂Cl. In embodiments, R^{40D} is -OCH₂Br. In embodiments, R^{40D} is -OCH₂I. In embodiments, R^{40D} is -OCH₂F.

R^{41D} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R^{45D}-substituted or unsubstituted alkyl (e.g., C₁-C₈ alkyl, C₁-C₆ alkyl, or C₁-C₄ alkyl), R^{45D}-substituted or unsubstituted heteroalkyl (e.g., 2 to 8 membered heteroalkyl, 2 to 6 membered heteroalkyl, or 2 to 4 membered heteroalkyl), R^{45D}-substituted or unsubstituted cycloalkyl (e.g., C₃-C₈ cycloalkyl, C₃-C₆ cycloalkyl, or C₅-C₆ cycloalkyl), R^{45D}-substituted or unsubstituted heterocycloalkyl (e.g., 3 to 8 membered heterocycloalkyl, 3 to 6 membered heterocycloalkyl, or 5 to 6 membered heterocycloalkyl), R^{45D}-substituted or unsubstituted aryl (e.g., C₆-C₁₀ aryl, C₁₀ aryl, or phenyl), or R^{45D}-substituted or unsubstituted heteroaryl (e.g., 5 to 10 membered heteroaryl, 5 to 9 membered heteroaryl, or 5 to 6 membered heteroaryl).

In embodiments, R^{41D} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

R^{45D} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂,-SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

In embodiments, L¹ is a bond, -SOₙ₅L^{1A}- (e.g., -SO₂-, -SO₃-, -SO₄-, -SO₂CH₂-, or -SO₂CH₂CH₂-), -SOᵥ₅NR⁵L^{1A}- (e.g.,-SO₂NHCH-, -SO₂N(CH₃)CH₂-, -SO₂N(CH₃)-CH=CH-, -SO₂N(CH₃)-N=CH-), -NHC(O)NR⁵L^{1A}- (e.g., -NHC(O)NHCH₂-, or -NHC(O)N(CH₃)CH₂-), -NR⁵L^{1A}- (e.g., -NHCH₂- or -NHCH₂CH₂-), -C(O)L^{1A}- (e.g., -C(O)CH₂-, -C(O)CH₂CH₂-, or -C(O)C₆C₄-), -C(O)OL^{1A}- (e.g.,-C(O)OCH₂-, -C(O)OCH₂CH₂-, or -C(O)OC₆C₄-), -C(O)NR⁵L^{1A}- (e.g., -C(O)NHCH₂-, -C(O)NHCH₂CH₂-, or -C(O)NHC₆C₄-), -OL^{1A}- (e.g., -OCH₂- or -OCH₂CH₂-), -NR⁵SO₂L^{1A}- (e.g., -NHSO₂CH₂-, or -N(CH₃)SO₂CH₂-), -NR⁵C(O)L^{1A}- (e.g., -NHC(O)CH₂-, -NHC(O)-CH=CH-, or -N(CH₃)C(O)CH₂CH₂-), -NR⁵C(O)OL^{1A}- (e.g., -NHC(O)OCH₂-, -NHC(O)O-CH=CH-, or -N(CH₃)C(O)OCH₂CH₂-), -NR⁵OL^{1A}- (e.g., -NHOCH₂-, -NHO-CH=CH-, or -N(CH₃)OCH₂CH₂-), -SL^{1A}- (e.g.,-SCH₂- or -SCH₂CH₂-), R⁴²-substituted or unsubstituted alkylene (e.g., C₁-C₈, C₁-C₆, C₁-C₄, or C₁-C₂), R⁴²-substituted or unsubstituted heteroalkylene (e.g., 2 to 8 membered, 2 to 6 membered, 4 to 6 membered, 2 to 3 membered, or 4 to 5 membered), R⁴²-substituted or unsubstituted cycloalkylene (e.g., C₃-C₈, C₃-C₆, C₄-C₆, or C₅-C₆), R⁴²-substituted or unsubstituted heterocycloalkylene (e.g., 3 to 8 membered, 3 to 6 membered, 4 to 6 membered, 4 to 5 membered, or 5 to 6 membered), R⁴²-substituted or unsubstituted arylene (e.g., C₆-C₁₀, C₁₀ or phenylene), or R⁴²-substituted or unsubstituted heteroarylene (e.g., 5 to 10 membered, 5 to 9 membered, or 5 to 6 membered). In embodiments, L¹ is -SOₙ₅L^{1A}- (e.g., -SO₂-, -SO₃-, -SO₄-, -SO₂CH₂-, or -SO₂CH₂CH₂-), -SOᵥ₅NR⁵L^{1A}- (e.g.,-SO₂NHCH-, -SO₂N(CH₃)CH₂-, -SO₂N(CH₃)-CH=CH-, -SO₂N(CH₃)-N=CH-), -NHC(O)NR⁵L^{1A}- (e.g., -NHC(O)NHCH₂-, or -NHC(O)N(CH₃)CH₂-), -NR⁵L^{1A}- (e.g., -NHCH₂- or -NHCH₂CH₂-), -C(O)L^{1A}- (e.g., -C(O)CH₂-, -C(O)CH₂CH₂-, or -C(O)C₆C₄-), -C(O)OL^{1A}- (e.g.,-C(O)OCH₂-, -C(O)OCH₂CH₂-, or -C(O)OC₆C₄-), -C(O)NR⁵L^{1A}- (e.g., -C(O)NHCH₂-, -C(O)NHCH₂CH₂-, or -C(O)NHC₆C₄-), -OL^{1A}- (e.g.,-OCH₂- or -OCH₂CH₂-), -NR⁵SO₂L^{1A}- (e.g., -NHSO₂CH₂-, or -N(CH₃)SO₂CH₂-), -NR⁵C(O)L^{1A}- (e.g., -NHC(O)CH₂-, -NHC(O)-CH=CH-, or -N(CH₃)C(O)CH₂CH₂-), -NR⁵C(O)OL^{1A}- (e.g., -NHC(O)OCH₂-, -NHC(O)O-CH=CH-, or -N(CH₃)C(O)OCH₂CH₂-), -NR⁵OL^{1A}- (e.g., -NHOCH₂-, -NHO-CH=CH-, or -N(CH₃)OCH₂CH₂-), -SL^{1A}- (e.g.,-SCH₂- or -SCH₂CH₂-), R⁴²-substituted or unsubstituted alkylene (e.g., C₁-C₈, C₁-C₆, C₁-C₄, or C₁-C₂), R⁴²-substituted or unsubstituted heteroalkylene (e.g., 2 to 8 membered, 2 to 6 membered, 4 to 6 membered, 2 to 3 membered, or 4 to 5 membered), R⁴²-substituted or unsubstituted cycloalkylene (e.g., C₃-C₈, C₃-C₆, C₄-C₆, or C₅-C₆), R⁴²-substituted or unsubstituted heterocycloalkylene (e.g., 3 to 8 membered, 3 to 6 membered, 4 to 6 membered, 4 to 5 membered, or 5 to 6 membered), R⁴²-substituted or unsubstituted arylene (e.g., C₆-C₁₀, C₁₀ or phenylene), or R⁴²-substituted or unsubstituted heteroarylene (e.g., 5 to 10 membered, 5 to 9 membered, or 5 to 6 membered).

In embodiments, L¹ is a bond, -SO₂-, -SO₃-, -SO₄-, -SO₂CH₂-, -SO₂CH₂CH₂-, -SO₂NHCH-, -SO₂N(CH₃)CH₂-, -SO₂N(CH₃)-CH=CH-,-SO₂N(CH₃)-N=CH-, -NHC(O)NHCH₂-, -NHC(O)N(CH₃)CH₂-, -NHCH₂-, -NHCH₂CH₂-, -C(O)CH₂-, -C(O)CH₂CH₂-, -C(O)C₆C₄-,-C(O)OCH₂-, -C(O)OCH₂CH₂-,-C(O)OC₆C₄-, -C(O)NHCH₂-, -C(O)NHCH₂CH₂-, -C(O)NHC₆C₄-,-OCH₂-, -OCH₂CH₂-, -NHSO₂CH₂-, -N(CH₃)SO₂CH₂-, -NHC(O)CH₂-, -NHC(O)-CH=CH-,-N(CH₃)C(O)CH₂CH₂-, -NHC(O)OCH₂-, -NHC(O)O-CH=CH-, -N(CH₃)C(O)OCH₂CH₂-, -NHOCH₂-, -NHO-CH=CH-, -N(CH₃)OCH₂CH₂-, -SCH₂-, -SCH₂CH₂-, R⁴²-substituted or unsubstituted alkylene (e.g., C₁-C₈, C₁-C₆, C₁-C₄, or C₁-C₂), R⁴²-substituted or unsubstituted heteroalkylene (e.g., 2 to 8 membered, 2 to 6 membered, 4 to 6 membered, 2 to 3 membered, or 4 to 5 membered), R⁴²-substituted or unsubstituted cycloalkylene (e.g., C₃-C₈, C₃-C₆, C₄-C₆, or C₅-C₆), R⁴²-substituted or unsubstituted heterocycloalkylene (e.g., 3 to 8 membered, 3 to 6 membered, 4 to 6 membered, 4 to 5 membered, or 5 to 6 membered), R⁴²-substituted or unsubstituted arylene (e.g., C₆-C₁₀, C₁₀ or phenylene), or R⁴²-substituted or unsubstituted heteroarylene (e.g., 5 to 10 membered, 5 to 9 membered, or 5 to 6 membered). In embodiments, L¹ is -SO₂-, -SO₃-, -SO₄-, -SO₂CH₂-,
-SO₂CH₂CH₂-, -SO₂NHCH-, -SO₂N(CH₃)CH₂-, -SO₂N(CH₃)-CH=CH-, -SO₂N(CH₃)-N=CH-,
-NHC(O)NHCH₂-, -NHC(O)N(CH₃)CH₂-, -NHCH₂-, -NHCH₂CH₂-, -C(O)CH₂-, -C(O)CH₂CH₂-, -C(O)C₆C₄-,-C(O)OCH₂-, -C(O)OCH₂CH₂-,-C(O)OC₆C₄-, -C(O)NHCH₂-, -C(O)NHCH₂CH₂-, -C(O)NHC₆C₄-, -OCH₂-, -OCH₂CH₂-, -NHSO₂CH₂-, -N(CH₃)SO₂CH₂-, -NHC(O)CH₂-, -NHC(O)-CH=CH-, -N(CH₃)C(O)CH₂CH₂-, -NHC(O)OCH₂-, -NHC(O)O-CH=CH-,-N(CH₃)C(O)OCH₂CH₂-, -NHOCH₂-, -NHO-CH=CH-, -N(CH₃)OCH₂CH₂-, -SCH₂-, or -SCH₂CH₂-. In embodiments, L¹ is -SO₂-, -SO₃-, -SO₄-, -SO₂CH₂-, -SO₂CH₂CH₂-, -SO₂NHCH-, -SO₂N(CH₃)CH₂-, -SO₂N(CH₃)-CH=CH-, -SO₂N(CH₃)-N=CH-, -NHC(O)NHCH₂-, -NHC(O)N(CH₃)CH₂-, -NHCH₂-, -NHCH₂CH₂-, -C(O)CH₂-, -C(O)CH₂CH₂-, -C(O)C₆C₄-,-C(O)OCH₂-, -C(O)OCH₂CH₂-, -C(O)OC₆C₄-, -C(O)NHCH₂-, -C(O)NHCH₂CH₂-, -C(O)NHC₆C₄-, -OCH₂-, -OCH₂CH₂-, -NHSO₂CH₂-, -N(CH₃)SO₂CH₂-,-NHC(O)CH₂-, -NHC(O)-CH=CH-, -N(CH₃)C(O)CH₂CH₂-, -NHC(O)OCH₂-, -NHC(O)O-CH=CH-, -N(CH₃)C(O)OCH₂CH₂-, -NHOCH₂-, -NHO-CH=CH-, -N(CH₃)OCH₂CH₂-,-SCH₂-, or -SCH₂CH₂-. In embodiments, L¹ is R⁴²-substituted or unsubstituted alkylene (e.g., C₁-C₈, C₁-C₆, C₁-C₄, or C₁-C₂), R⁴²-substituted or unsubstituted heteroalkylene (e.g., 2 to 8 membered, 2 to 6 membered, 4 to 6 membered, 2 to 3 membered, or 4 to 5 membered), R⁴²-substituted or unsubstituted cycloalkylene (e.g., C₃-C₈, C₃-C₆, C₄-C₆, or C₅-C₆), R⁴²-substituted or unsubstituted heterocycloalkylene (e.g., 3 to 8 membered, 3 to 6 membered, 4 to 6 membered, 4 to 5 membered, or 5 to 6 membered), R⁴²-substituted or unsubstituted arylene (e.g., C₆-C₁₀, C₁₀ or phenylene), or R⁴²-substituted or unsubstituted heteroarylene (e.g., 5 to 10 membered, 5 to 9 membered, or 5 to 6 membered). In embodiments, L¹ is unsubstituted alkylene (e.g., C₁-C₈, C₁-C₆, C₁-C₄, or C₁-C₂), unsubstituted heteroalkylene (e.g., 2 to 8 membered, 2 to 6 membered, 4 to 6 membered, 2 to 3 membered, or 4 to 5 membered), unsubstituted cycloalkylene (e.g., C₃-C₈, C₃-C₆, C₄-C₆, or C₅-C₆), unsubstituted heterocycloalkylene (e.g., 3 to 8 membered, 3 to 6 membered, 4 to 6 membered, 4 to 5 membered, or 5 to 6 membered), unsubstituted arylene (e.g., C₆-C₁₀, C₁₀ or phenylene), or unsubstituted heteroarylene (e.g., 5 to 10 membered, 5 to 9 membered, or 5 to 6 membered).

R⁴² is oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In embodiments, R⁴² is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂,-SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R⁴³-substituted or unsubstituted alkyl (e.g., C₁-C₈ alkyl, C₁-C₆ alkyl, or C₁-C₄ alkyl), R⁴³-substituted or unsubstituted heteroalkyl (e.g., 2 to 8 membered heteroalkyl, 2 to 6 membered heteroalkyl, or 2 to 4 membered heteroalkyl), R⁴³-substituted or unsubstituted cycloalkyl (e.g., C₃-C₈ cycloalkyl, C₃-C₆ cycloalkyl, or C₅-C₆ cycloalkyl), R⁴³-substituted or unsubstituted heterocycloalkyl (e.g., 3 to 8 membered heterocycloalkyl, 3 to 6 membered heterocycloalkyl, or 5 to 6 membered heterocycloalkyl), R⁴³-substituted or unsubstituted aryl (e.g., C₆-C₁₀ aryl, C₁₀ aryl, or phenyl), or R⁴³-substituted or unsubstituted heteroaryl (e.g., 5 to 10 membered heteroaryl, 5 to 9 membered heteroaryl, or 5 to 6 membered heteroaryl).

In embodiments, L¹ is -CH(CH₃)-. In embodiments, L¹ is unsubstituted ethyl. In embodiments, L¹ is unsubstituted C₁-C₃ alkylene. In embodiments, L¹ is unsubstituted methylene. In embodiments, L¹ is unsubstituted C₂-C₃ alkylene. In embodiments, L¹ is -CH(CH₂CH₃)-. In embodiments, L¹ is -C(CH₃)₂-. In embodiments, L¹ is -CH₂CH₂-

In embodiments, L¹ is -CH(CH₂OCH₃)-. In embodiments, L¹ is unsubstituted 3 membered heteroalkyl-substituted methylene. In embodiments, L¹ is unsubstituted 2 to 4 membered heteroalkyl-substituted methylene. In embodiments, L¹ is -CH(CH₂OH)-. In embodiments, L¹ is OH-substituted ethylene. In embodiments, L¹ is OH-substituted C₁-C₃ alkylene. In embodiments, L¹ is substituted ethylene. In embodiments, L¹ is substituted C₁-C₃ alkylene. In embodiments, L¹ is 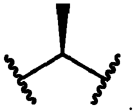 In embodiments, L¹ is

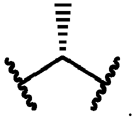

In embodiments, L^{1A} is a bond, R^{42A}-substituted or unsubstituted alkylene (e.g., C₁-C₈, C₁-C₆, C₁-C₄, or C₁-C₂), R⁴²-substituted or unsubstituted heteroalkylene (e.g., 2 to 8 membered, 2 to 6 membered, 4 to 6 membered, 2 to 3 membered, or 4 to 5 membered), R^{42A}-substituted or unsubstituted cycloalkylene (e.g., C₃-C₈, C₃-C₆, C₄-C₆, or C₅-C₆), R^{42A}-substituted or unsubstituted heterocycloalkylene (e.g., 3 to 8 membered, 3 to 6 membered, 4 to 6 membered, 4 to 5 membered, or 5 to 6 membered), R^{42A}-substituted or unsubstituted arylene (e.g., C₆-C₁₀, C₁₀ or phenylene), or R^{42A}-substituted or unsubstituted heteroarylene (e.g., 5 to 10 membered, 5 to 9 membered, or 5 to 6 membered). In embodiments, L^{1A} is a bond. In embodiments, L^{1A} is R^{42A}-substituted alkylene (e.g., C₁-C₈, C₁-C₆, C₁-C₄, or C₁-C₂), R⁴²-substituted heteroalkylene (e.g., 2 to 8 membered, 2 to 6 membered, 4 to 6 membered, 2 to 3 membered, or 4 to 5 membered), R^{42A}-substituted cycloalkylene (e.g., C₃-C₈, C₃-C₆, C₄-C₆, or C₅-C₆), R^{42A}-substituted heterocycloalkylene (e.g., 3 to 8 membered, 3 to 6 membered, 4 to 6 membered, 4 to 5 membered, or 5 to 6 membered), R^{42A}-substituted arylene (e.g., C₆-C₁₀, C₁₀ or phenylene), or R^{42A}-substituted heteroarylene (e.g., 5 to 10 membered, 5 to 9 membered, or 5 to 6 membered). In embodiments, L^{1A} is unsubstituted alkylene (e.g., C₁-C₈, C₁-C₆, C₁-C₄, or C₁-C₂), unsubstituted heteroalkylene (e.g., 2 to 8 membered, 2 to 6 membered, 4 to 6 membered, 2 to 3 membered, or 4 to 5 membered), unsubstituted cycloalkylene (e.g., C₃-C₈, C₃-C₆, C₄-C₆, or C₅-C₆), unsubstituted heterocycloalkylene (e.g., 3 to 8 membered, 3 to 6 membered, 4 to 6 membered, 4 to 5 membered, or 5 to 6 membered), unsubstituted arylene (e.g., C₆-C₁₀, C₁₀ or phenylene), or unsubstituted heteroarylene (e.g., 5 to 10 membered, 5 to 9 membered, or 5 to 6 membered).

R^{42A} is oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl. In embodiments, R^{42A} is independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂,-SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, R^{43A}-substituted or unsubstituted alkyl (e.g., C₁-C₈ alkyl, C₁-C₆ alkyl, or C₁-C₄ alkyl), R^{43A}-substituted or unsubstituted heteroalkyl (e.g., 2 to 8 membered heteroalkyl, 2 to 6 membered heteroalkyl, or 2 to 4 membered heteroalkyl), R^{43A}-substituted or unsubstituted cycloalkyl (e.g., C₃-C₈ cycloalkyl, C₃-C₆ cycloalkyl, or C₅-C₆ cycloalkyl), R^{43A}-substituted or unsubstituted heterocycloalkyl (e.g., 3 to 8 membered heterocycloalkyl, 3 to 6 membered heterocycloalkyl, or 5 to 6 membered heterocycloalkyl), R^{43A}-substituted or unsubstituted aryl (e.g., C₆-C₁₀ aryl, C₁₀ aryl, or phenyl), or R^{43A}-substituted or unsubstituted heteroaryl (e.g., 5 to 10 membered heteroaryl, 5 to 9 membered heteroaryl, or 5 to 6 membered heteroaryl).

Each R²², R²⁵, R²⁸, R³¹, R³⁴, R³⁷, R⁴³ and R^{43A} are independently oxo, halogen, -CCl₃, -CBr₃, -CF₃, -CI₃, -CHCl₂, -CHBr₂, -CHF₂, -CHI₂, -CH₂Cl, -CH₂Br, -CH₂F, -CH₂I, -CN, -OH, -NH₂, -COOH, -CONH₂, -NO₂, -SH, -SO₃H, -SO₄H, -SO₂NH₂, -NHNH₂, -ONH₂, -NHC(O)NHNH₂, -NHC(O)NH₂, -NHSO₂H, -NHC(O)H, -NHC(O)OH, -NHOH, -OCCl₃, -OCF₃, -OCBr₃, -OCI₃, -OCHCl₂, -OCHBr₂, -OCHI₂, -OCHF₂, -OCH₂Cl, -OCH₂Br, -OCH₂I, -OCH₂F, -N₃, unsubstituted alkyl (e.g., C₁-C₈ alkyl, C₁-C₆ alkyl, or C₁-C₄ alkyl), unsubstituted heteroalkyl (e.g., 2 to 8 membered heteroalkyl, 2 to 6 membered heteroalkyl, or 2 to 4 membered heteroalkyl), unsubstituted cycloalkyl (e.g., C₃-C₈ cycloalkyl, C₃-C₆ cycloalkyl, or C₅-C₆ cycloalkyl), unsubstituted heterocycloalkyl (e.g., 3 to 8 membered heterocycloalkyl, 3 to 6 membered heterocycloalkyl, or 5 to 6 membered heterocycloalkyl), unsubstituted aryl (e.g., C₆-C₁₀ aryl, C₁₀ aryl, or phenyl), or unsubstituted heteroaryl (e.g., 5 to 10 membered heteroaryl, 5 to 9 membered heteroaryl, or 5 to 6 membered heteroaryl).

In embodiments, Ring A is C₆-C₁₀ aryl or 5 to 10 membered heteroaryl. In embodiments, Ring A is C₆-C₁₀ aryl or 5 to 10 membered heteroaryl. In embodiments, Ring A is C₆-C₁₀ aryl. In embodiments, Ring A is phenyl. In embodiments, Ring A is naphthyl. In embodiments, Ring A is 5 to 10 membered heteroaryl. In embodiments, Ring A is 5 to 6 membered heteroaryl. In embodiments, Ring A is thienyl. In embodiments, Ring A is furanyl. In embodiments, Ring A is pyrrolyl. In embodiments, Ring A is imidazolyl. In embodiments, Ring A is pyrazolyl. In embodiments, Ring A is oxazolyl. In embodiments, Ring A is isoxazolyl. In embodiments, Ring A is thiazolyl. In embodiments, Ring A is pyridyl. In embodiments, Ring A is pyridinyl. In embodiments, Ring A is pyrazinyl. In embodiments, Ring A is pyrimidinyl. In embodiments, Ring A is pyridazinyl. In embodiments, Ring A is 1,2,3-triazinyl. In embodiments, Ring A is 1,2,4-triazinyl. In embodiments, Ring A is 1,3,5-triazinyl.

When Ring A is substituted (e.g., substituted C₆-C₁₀ aryl) it is understood z1 is not 0 (i.e., Ring A is R¹-substituted). In embodiments, Ring A is substituted C₆-C₁₀ aryl or substituted 5 to 10 membered heteroaryl. In embodiments, Ring A is substituted C₆-C₁₀ aryl or substituted 5 to 10 membered heteroaryl. In embodiments, Ring A is substituted C₆-C₁₀ aryl. In embodiments, Ring A is substituted phenyl. In embodiments, Ring A is substituted naphthyl. In embodiments, Ring A is substituted 5 to 10 membered heteroaryl. In embodiments, Ring A is substituted 5 to 6 membered heteroaryl. In embodiments, Ring A is substituted thienyl. In embodiments, Ring A is substituted furanyl. In embodiments, Ring A is substituted pyrrolyl. In embodiments, Ring A is substituted imidazolyl. In embodiments, Ring A is substituted pyrazolyl. In embodiments, Ring A is substituted oxazolyl. In embodiments, Ring A is substituted isoxazolyl. In embodiments, Ring A is substituted thiazolyl. In embodiments, Ring A is substituted pyridyl. In embodiments, Ring A is substituted pyridinyl. In embodiments, Ring A is substituted pyrazinyl. In embodiments, Ring A is substituted pyrimidinyl. In embodiments, Ring A is substituted pyridazinyl. In embodiments, Ring A is substituted 1,2,3-triazinyl. In embodiments, Ring A is substituted 1,2,4-triazinyl. In embodiments, Ring A is substituted 1,3,5-triazinyl.

In embodiments, Ring A is an unsubstituted C₆-C₁₀ aryl or unsubstituted 5 to 10 membered heteroaryl. In embodiments, Ring A is an unsubstituted C₆-C₁₀ aryl or unsubstituted 5 to 10 membered heteroaryl. In embodiments, Ring A is an unsubstituted C₆-C₁₀ aryl. In embodiments, Ring A is an unsubstituted phenyl. In embodiments, Ring A is an unsubstituted naphthyl. In embodiments, Ring A is an unsubstituted 5 to 10 membered heteroaryl. In embodiments, Ring A is an unsubstituted 5 to 6 membered heteroaryl. In embodiments, Ring A is an unsubstituted thienyl. In embodiments, Ring A is an unsubstituted furanyl. In embodiments, Ring A is an unsubstituted pyrrolyl. In embodiments, Ring A is an unsubstituted imidazolyl. In embodiments, Ring A is an unsubstituted pyrazolyl. In embodiments, Ring A is an unsubstituted oxazolyl. In embodiments, Ring A is an unsubstituted isoxazolyl. In embodiments, Ring A is an unsubstituted thiazolyl. In embodiments, Ring A is an unsubstituted pyridyl. In embodiments, Ring A is an unsubstituted pyridinyl. In embodiments, Ring A is an unsubstituted pyrazinyl. In embodiments, Ring A is an unsubstituted pyrimidinyl. In embodiments, Ring A is an unsubstituted pyridazinyl. In embodiments, Ring A is an unsubstituted 1,2,3-triazinyl. In embodiments, Ring A is an unsubstituted 1,2,4-triazinyl. In embodiments, Ring A is an unsubstituted 1,3,5-triazinyl.

In embodiments, Ring A is substituted with one R¹. In embodiments, Ring A is substituted with two optionally different R¹ substituents. In embodiments, Ring A is substituted with three optionally different R¹ substituents. In embodiments, Ring A is substituted with four optionally different R¹ substituents. In embodiments, Ring A is substituted with five optionally different R¹ substituents.

In embodiments, Ring A is phenyl, or 5 to 6 membered heteroaryl. In embodiments, Ring A is phenyl. In embodiments, Ring A is 5 to 6 membered heteroaryl. In embodiments, Ring A is a 5 membered heteroaryl. In embodiments, Ring A is a 5 membered heteroaryl.

In embodiments, Ring A is phenyl, pyridyl, pyrazolyl, imidazolyl, oxazolyl, isoxazolyl, thiazolyl, furanyl, pyrrolyl, thienyl, pyrazinyl, pyridazinyl, or pyrimidinyl. In embodiments, Ring A is phenyl. In embodiments, Ring A is pyridyl. In embodiments, Ring A is pyrazolyl. In embodiments, Ring A is imidazolyl. In embodiments, Ring A is oxazolyl. In embodiments, Ring A is isoxazolyl. In embodiments, Ring A is thiazolyl. In embodiments, Ring A is furanyl. In embodiments, Ring A is pyrrolyl. In embodiments, Ring A is thienyl. In embodiments, Ring A is pyrazinyl. In embodiments, Ring A is pyridazinyl. In embodiments, Ring A is pyrimidinyl. In embodiments, Ring A is phenyl or pyridyl. In embodiments, Ring A is pyridyl.

In embodiments, Ring A is phenyl.

In embodiments, Ring A is pyridin-3-yl. In embodiments, Ring A is pyridyl. In embodiments, Ring A is a 6 membered heteroaryl. In embodiments, Ring A is pyridin-2-yl. In embodiments, Ring A is pyridin-4-yl.

In embodiments, Ring A is pyrimidin-5-yl. In embodiments, Ring A is pyrimidinyl.

In embodiments, Ring A is pyridazin-3-yl. In embodiments, Ring A is pyridazinyl.

In embodiments, Ring A is pyrazin-2-yl. In embodiments, Ring A is pyrazinyl.

In embodiments, Ring A is imidazol-1-yl. In embodiments, Ring A is imidazolyl. In embodiments, Ring A is a 5 membered heteroaryl.

In embodiments, -(Ring A)-(R¹)_{z1} is 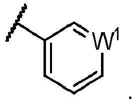 In embodiments, -(Ring A)-(R¹)_{z1} is 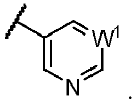 In embodiments, -(Ring A)-(R¹)_{z1} is 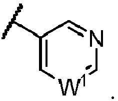 In embodiments, -(Ring A)-(R¹)_{z1} is 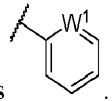 In embodiments, -(Ring A)-(R¹)_{z1} is 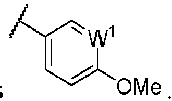 In embodiments, - (Ring A)-(R¹)_{z1} is 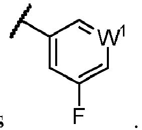 In embodiments, -(Ring A)-(R¹)_{z1} is 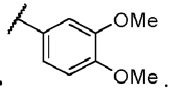 In embodiments, -(Ring A)-(R¹)_{z1} is 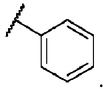 In embodiments, -(Ring A)-(R¹)_{z1} is 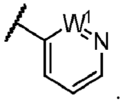 In embodiments, -(Ring A)-(R¹)_{z1} is 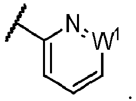 In embodiments, -(Ring A)-(R¹)_{z1} is 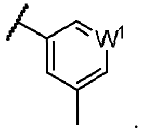 In embodiments, -(Ring A)-(R¹)_{z1} is 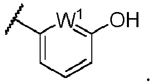 In embodiments, -(Ring A)-(R¹)_{z1} is 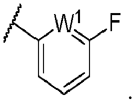 In embodiments, -(Ring A)-(R¹)_{z1} is 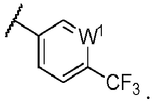 In embodiments, - (Ring A)-(R¹)_{z1} is 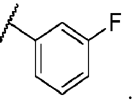 In embodiments, -(Ring A)-(R¹)_{z1} is 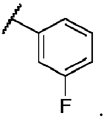 In embodiments, -(Ring A)-(R¹)_{z1} is 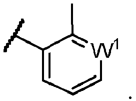 In embodiments, -(Ring A)-(R¹)_{z1} is 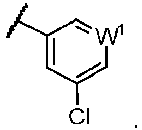 In embodiments, -(Ring A)-(R¹)_{z1} is 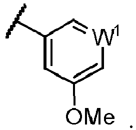 In embodiments, -(Ring A)-(R¹)_{z1} is 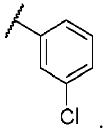 In embodiments, -(Ring A)-(R¹)_{z1} is 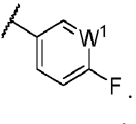 In embodiments, -(Ring A)-(R¹)_{z1} is 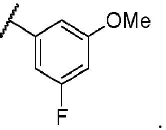 In embodiments, -(Ring A)-(R¹)_{z1} is 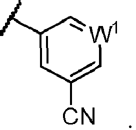 In embodiments, -(Ring A)-(R¹)_{z1} is 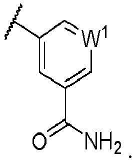 In embodiments, -(Ring A)-(R¹)_{z1} is 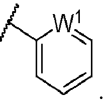 In embodiments, -(Ring A)-(R¹)_{z1} is 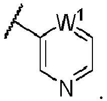 In embodiments, -(Ring A)-(R¹)_{z1} is 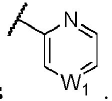 In embodiments, -(Ring A)-(R¹)_{z1} is 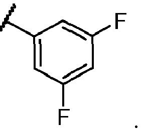 In embodiments, -(Ring A)-(R¹)_{z1} is 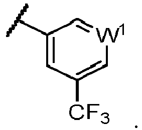 In embodiments, -(Ring A)-(R¹)_{z1} is 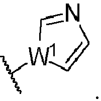 In embodiments, -(Ring A)-(R¹)_{z1} is 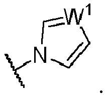 In embodiments, -(Ring A)-(R¹)_{z1} is 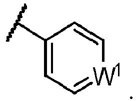 In embodiments, -(Ring A)-(R¹)_{z1} is 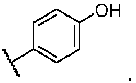 In embodiments, -(Ring A)-(R¹)_{z1} is 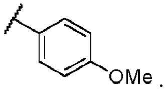 In embodiments, -(Ring A)-(R¹)_{z1} is 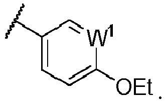 In embodiments, W¹ is -C= or -N=. In embodiments, W¹ is -C=. In embodiments, W¹ is -N=.

In embodiments, -(Ring A)-(R¹)_{z1} is 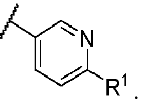 In embodiments, -(Ring A)-(R¹)_{z1} is 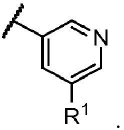 In embodiments, -(Ring A)-(R¹)_{z1} is 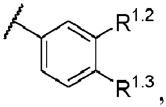 wherein R^{1.2} and R^{1.3} are each R¹ at a fixed position on the attached ring. R^{1.2} and R^{1.3} may independently be any substituent of R¹ described herein, including in any aspect, embodiment, example, figure, or claim. In embodiments, -(Ring A)-(R¹)_{z1} is 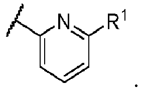 In embodiments, -(Ring A)-(R¹)_{z1} is 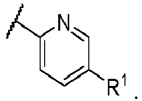 In embodiments -(Ring A)-(R¹)_{z1} is 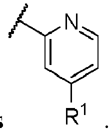 In embodiments, -(Ring A)-(R¹)_{z1} is 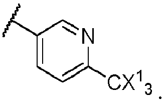 In embodiments, -(Ring A)-(R¹)_{z1} is 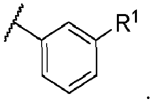 In embodiments, -(Ring A)-(R¹)_{z1} is 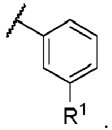 In embodiments, -(Ring A)-(R¹)_{z1} is 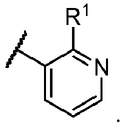 In embodiments, - (Ring A)-(R¹)_{z1} is 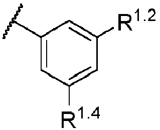 wherein R^{1.2} and R^{1.4} are each R¹ at a fixed position on the attached ring. R^{1.2} and R^{1.4} may independently be any substituent of R¹ described herein, including in any aspect, embodiment, example, figure, or claim. In embodiments, -(Ring A)-(R¹)_{z1} is

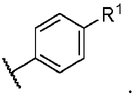

In embodiments, -(Ring A)-(R¹)_{z1} is 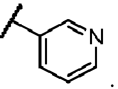 In embodiments, -(Ring A)-(R¹)_{z1} is 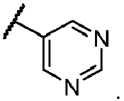 In embodiments, -(Ring A)-(R¹)_{z1} is 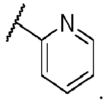 In embodiments, -(Ring A)-(R¹)_{z1} is 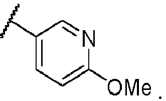 In embodiments, -(Ring A)-(R¹)_{z1} is 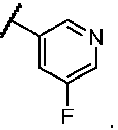 In embodiments, - (Ring A)-(R¹)_{z1} is 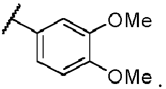 In embodiments, -(Ring A)-(R¹)_{z1} is 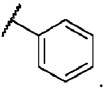 In embodiments, -(Ring A)-(R¹)_{z1} is 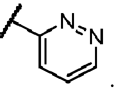 In embodiments, -(Ring A)-(R¹)_{z1} is 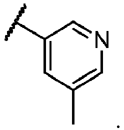 In embodiments, -(Ring A)-(R¹)_{z1} is 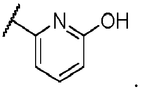 In embodiments, -(Ring A)-(R¹)_{z1} is 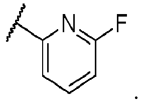 In embodiments, -(Ring A)-(R¹)_{z1} is 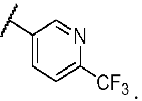 In embodiments, -(Ring A)-(R¹)_{z1} is 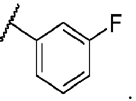 In embodiments, -(Ring A)-(R¹)_{z1} is 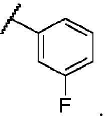 In embodiments, -(Ring A)-(R¹)_{z1} is 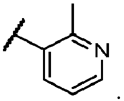 In embodiments, -(Ring A)-(R¹)_{z1} is 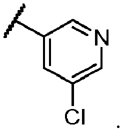 In embodiments, - (Ring A)-(R¹)_{z1} is 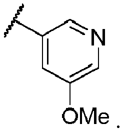 In embodiments, -(Ring A)-(R¹)_{z1} is 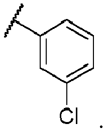 In embodiments, -(Ring A)-(R¹)_{z1} is 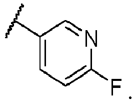 In embodiments, -(Ring A)-(R¹)_{z1} is 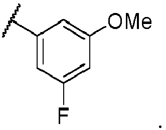 In embodiments, -(Ring A)-(R¹)_{z1} is 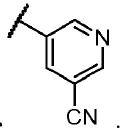 In embodiments, -(Ring A)-(R¹)_{z1} is 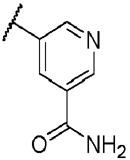 . In embodiments, -(Ring A)-(R¹)_{z1} is 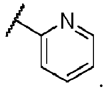 In embodiments, -(Ring A)-(R¹)_{z1} is 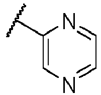 . In embodiments, -(Ring A)-(R¹)_{z1} is 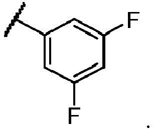 In embodiments, -(Ring A)-(R¹)_{z1} is 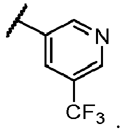 In embodiments, -(Ring A)-(R¹)_{z1} is 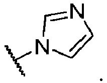 In embodiments, -(Ring A)-(R¹)_{z1} is 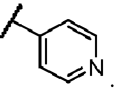 In embodiments, -(Ring A)-(R¹)_{z1} is 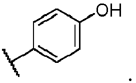 In embodiments, -(Ring A)-(R¹)_{z1} is 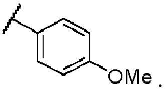 In embodiments, -(Ring A)-(R¹)_{z1} is

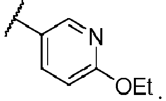

In embodiments, Ring B is a 4 to 8 membered heterocycloalkyl. In embodiments, Ring B is a 4 membered heterocycloalkyl. In embodiments, Ring B is a 5 membered heterocycloalkyl. In embodiments, Ring B is a 6 membered heterocycloalkyl. In embodiments, Ring B is a 7 membered heterocycloalkyl. In embodiments, Ring B is an 8 membered heterocycloalkyl. In embodiments, Ring B is a 4 to 6 membered heterocycloalkyl. In embodiments, Ring B is azetidinyl. When Ring B is substituted (e.g., substituted 4 to 8 membered heterocycloalkyl) it is understood z1 is not 0 (i.e., Ring B is R⁷-substituted Ring B).

In embodiments, Ring B is a substituted or unsubstituted heterocycloalkyl. In embodiments, Ring B is a substituted heterocycloalkyl. In embodiments, Ring B is an unsubstituted heterocycloalkyl. In embodiments, Ring B is a substituted or unsubstituted 3 to 10 membered heterocycloalkyl. In embodiments, Ring B is a substituted 3 to 10 membered heterocycloalkyl. In embodiments, Ring B is an unsubstituted 3 to 10 membered heterocycloalkyl. In embodiments, Ring B is a R⁷-substituted or unsubstituted 5 to 10 membered heterocycloalkyl. In embodiments, Ring B is a R⁷-substituted 5 to 10 membered heterocycloalkyl. In embodiments, Ring B is an unsubstituted 5 to 10 membered heterocycloalkyl.

In embodiments, Ring B is a substituted or unsubstituted 3 membered heterocycloalkyl. In embodiments, Ring B is a substituted or unsubstituted 4 membered heterocycloalkyl. In embodiments, Ring B is a substituted or unsubstituted 5 membered heterocycloalkyl. In embodiments, Ring B is a substituted or unsubstituted 6 membered heterocycloalkyl. In embodiments, Ring B is a substituted 3 membered heterocycloalkyl. In embodiments, Ring B is a substituted 4 membered heterocycloalkyl. In embodiments, Ring B is a substituted 5 membered heterocycloalkyl. In embodiments, Ring B is a substituted 6 membered heterocycloalkyl. In embodiments, Ring B is an unsubstituted 3 membered heterocycloalkyl. In embodiments, Ring B is an unsubstituted 4 membered heterocycloalkyl. In embodiments, Ring B is an unsubstituted 5 membered heterocycloalkyl. In embodiments, Ring B is an unsubstituted 6 membered heterocycloalkyl.

In embodiments, Ring B is a substituted or unsubstituted aziridinyl, azirinyl, azetidinyl, dihydroazetyl, diazetidinyl, azetyl, pyrrolidinyl, pyrrolinyl, pyrrolyl, pyrazolidinyl, imidazolidinyl, pyrazolinyl, pyrazolyl, thiazolidinyl, thiazolyl, isothiazolyl, piperidinyl, piperazinyl, morpholinyl, oxazinyl, thiomorpholinyl, thiazinyl, decahydroquinolinyl, dihydroazepinyl, azepanyl, or azocanyl. In embodiments, Ring B is a substituted aziridinyl, azirinyl, azetidinyl, dihydroazetyl, diazetidinyl, azetyl, pyrrolidinyl, pyrrolinyl, pyrrolyl, pyrazolidinyl, imidazolidinyl, pyrazolinyl, pyrazolyl, thiazolidinyl, thiazolyl, isothiazolyl, piperidinyl, piperazinyl, morpholinyl, oxazinyl, thiomorpholinyl, thiazinyl, decahydroquinolinyl, dihydroazepinyl, azepanyl, or azocanyl. In embodiments, Ring B is an unsubstituted aziridinyl, azirinyl, azetidinyl, dihydroazetyl, diazetidinyl, azetyl, pyrrolidinyl, pyrrolinyl, pyrrolyl, pyrazolidinyl, imidazolidinyl, pyrazolinyl, pyrazolyl, thiazolidinyl, thiazolyl, isothiazolyl, piperidinyl, piperazinyl, morpholinyl, oxazinyl, thiomorpholinyl, thiazinyl, decahydroquinolinyl, dihydroazepinyl, azepanyl, or azocanyl.

In embodiments, Ring B is substituted with one R⁷. In embodiments, Ring B is substituted with two optionally different R⁷ substituents. In embodiments, Ring B is substituted with three optionally different R⁷ substituents. In embodiments, Ring B is substituted with four optionally different R⁷ substituents. In embodiments, Ring B is substituted with five optionally different R⁷ substituents. In embodiments, Ring B is substituted with six optionally different R⁷ substituents. In embodiments, Ring B is substituted with seven optionally different R⁷ substituents. In embodiments, Ring B is substituted with eight optionally different R⁷ substituents. In embodiments, Ring B is substituted with nine optionally different R⁷ substituents. In embodiments, Ring B is substituted with ten optionally different R⁷ substituents.

In embodiments, Ring B is azetidinyl. In embodiments, Ring B is 4 membered heterocycloalkyl. In embodiments, Ring B is 3 to 5 membered heterocycloalkyl. In embodiments, Ring B is 3 to 6 membered heterocycloalkyl. In embodiments, Ring B is pyrrolidinyl. In embodiments, Ring B is 5 membered heterocycloalkyl. In embodiments, Ring B is 4 to 6 membered heterocycloalkyl. In embodiments, Ring B is morpholinyl. In embodiments, Ring B is 6 membered heterocycloalkyl. In embodiments, Ring B is 5 to 7 membered heterocycloalkyl. In embodiments, Ring B is 2-oxa-6-azaspiro[3.3]heptanyl. In embodiments, Ring B is 2-oxa-6-azaspiro[3.3]heptan-6-yl. In embodiments, Ring B is 7 membered heterocycloalkyl. In embodiments, Ring B is 6 to 8 membered heterocycloalkyl. In embodiments, Ring B is 7 membered spirocyclic heterocycloalkyl. In embodiments, Ring B is 6 to 8 membered spirocyclic heterocycloalkyl. In embodiments, Ring B is piperidin-1-yl. In embodiments, Ring B is piperidinyl. In embodiments, Ring B is piperazin-1-yl. In embodiments, Ring B is piperazinyl. In embodiments, Ring B is oxazolidin-3-yl. In embodiments, Ring B is oxazolidinyl. In embodiments, Ring B is imidazolidinyl. In embodiments, Ring B is 1,3-oxazinanyl. In embodiments, Ring B is oxazinanyl.

In embodiments, -(Ring B)-(R⁷)_{z2} is 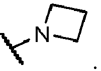 In embodiments, -(Ring B)-(R⁷)_{z2} is 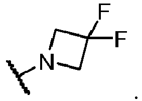 In embodiments, -(Ring B)-(R⁷)_{z2} is 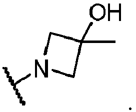 In embodiments, -(Ring B)-(R⁷)_{z2} is 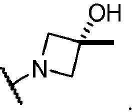 In embodiments, -(Ring B)-(R⁷)_{z2} is 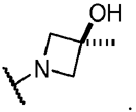 In embodiments, - (Ring B)-(R⁷)_{z2} is 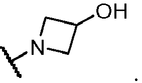 In embodiments, -(Ring B)-(R⁷)_{z2} is 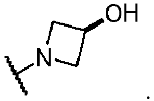 In embodiments, -(Ring B)-(R⁷)_{z2} is 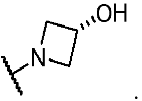 In embodiments, -(Ring B)-(R⁷)_{z2} is 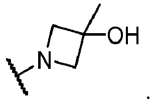 In embodiments, -(Ring B)-(R⁷)_{z2} is 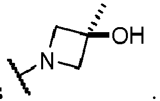 In embodiments, -(Ring B)-(R⁷)_{z2} is 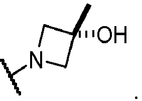 In embodiments, -(Ring B)-(R⁷)_{z2} is 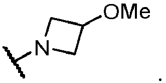 In embodiments, -(Ring B)-(R⁷)_{z2} is 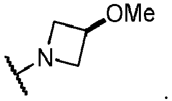 In embodiments, -(Ring B)-(R⁷)_{z2} is 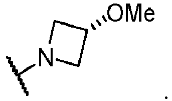 In embodiments, -(Ring B)-(R⁷)_{z2} is 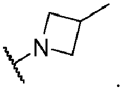 In embodiments, -(Ring B)-(R⁷)_{z2} is 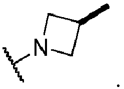 In embodiments, -(Ring B)-(R⁷)_{z2} is 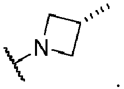 In embodiments, -(Ring B)-(R⁷)_{z2} is 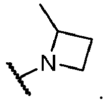 In embodiments, -(Ring B)-(R⁷)_{z2} is 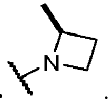 In embodiments, -(Ring B)-(R⁷)_{z2} is 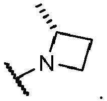 In embodiments, -(Ring B)-(R⁷)_{z2} is 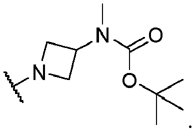 In embodiments, -(Ring B)-(R⁷)_{z2} is 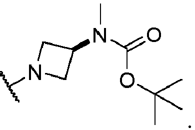 In embodiments, -(Ring B)-(R⁷)_{z2} is 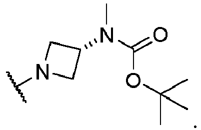 In embodiments, -(Ring B)-(R⁷)_{z2} is 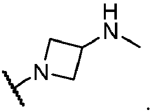 In embodiments,-(Ring B)-(R⁷)_{z2} is 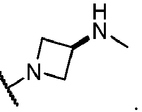 In embodiments, -(Ring B)-(R⁷)_{z2} is 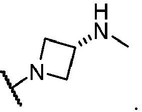 In embodiments, -(Ring B)-(R⁷)_{z2} is 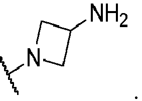 In embodiments, -(Ring B)-(R⁷)_{z2} is 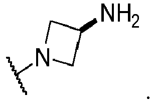 In embodiments, -(Ring B)-(R⁷)_{z2} is 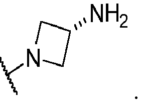 In embodiments, -(Ring B)-(R⁷)_{z2} is 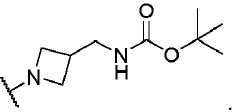 In embodiments, -(Ring B)-(R⁷)_{z2} is 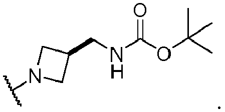 In embodiments, -(Ring B)-(R⁷)_{z2} is 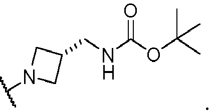 In embodiments, -(Ring B)-(R⁷)_{z2} 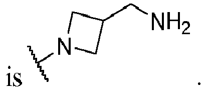 In embodiments, -(Ring B)-(R⁷)_{z2} is 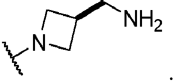 In embodiments, - (Ring B)-(R⁷)_{z2} is 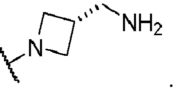 In embodiments, -(Ring B)-(R⁷)_{z2} is 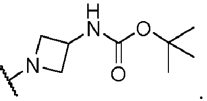 In embodiments, -(Ring B)-(R⁷)_{z2} is 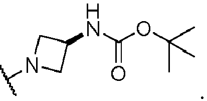 In embodiments, -(Ring B)-(R⁷)_{z2} is 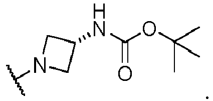 In embodiments, -(Ring B)-(R⁷)_{z2} is 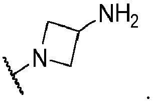 In embodiments, - (Ring B)-(R⁷)_{z2} is 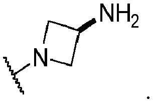 In embodiments, -(Ring B)-(R⁷)_{z2} is 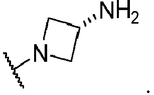 In embodiments, -(Ring B)-(R⁷)_{z2} is 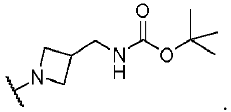 In embodiments, -(Ring B)-(R⁷)_{z2} is 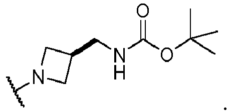 In embodiments, -(Ring B)-(R⁷)_{z2} is 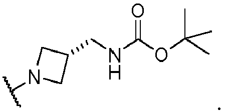 In embodiments, -(Ring B)-(R⁷)_{z2} is 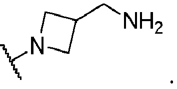 In embodiments, -(Ring B)-(R⁷)_{z2} is 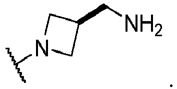 In embodiments, -(Ring B)-(R⁷)_{z2} is 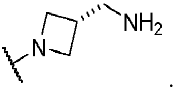 In embodiments, - (Ring B)-(R⁷)_{z2} is 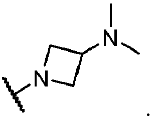 In embodiments, -(Ring B)-(R⁷)_{z2} is 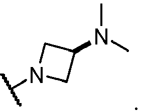 In embodiments, -(Ring B)-(R⁷)_{z2} is 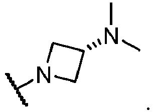 In embodiments, -(Ring B)-(R⁷)_{z2} is 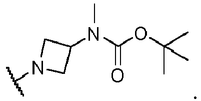 In embodiments, -(Ring B)-(R⁷)_{z2} is 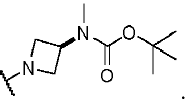 In embodiments, - (Ring B)-(R⁷)_{z2} is 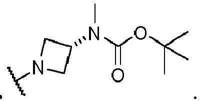 In embodiments, -(Ring B)-(R⁷)_{z2} is 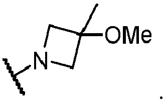 In embodiments, -(Ring B)-(R⁷)_{z2} is 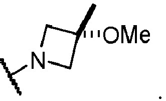 In embodiments, -(Ring B)-(R⁷)_{z2} is 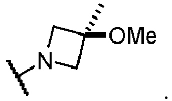 In embodiments, -(Ring B)-(R⁷)_{z2} is 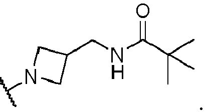 In embodiments, - (Ring B)-(R⁷)_{z2} is 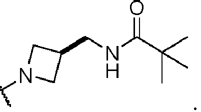 In embodiments, -(Ring B)-(R⁷)_{z2} is 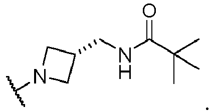 In embodiments, -(Ring B)-(R⁷)_{z2} is 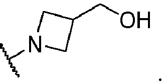 In embodiments, - (Ring B)-(R⁷)_{z2} is 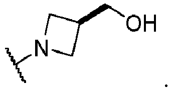 In embodiments, -(Ring B)-(R⁷)_{z2} is 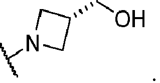 In embodiments, -(Ring B)-(R⁷)_{z2} is 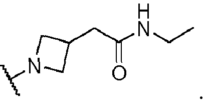 In embodiments, -(Ring B)-(R⁷)_{z2} is 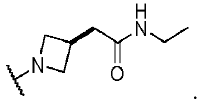 In embodiments, -(Ring B)-(R⁷)_{z2} is 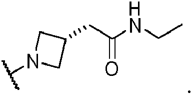 In embodiments, - (Ring B)-(R⁷)_{z2} is 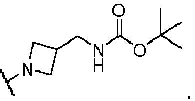 In embodiments, -(Ring B)-(R⁷)_{z2} is 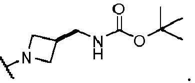 In embodiments, -(Ring B)-(R⁷)_{z2} is 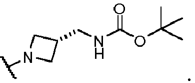 In embodiments, -(Ring B)-(R⁷)_{z2} is 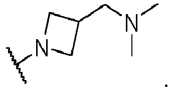 In embodiments, -(Ring B)-(R⁷)_{z2} is 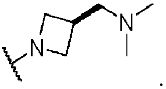 In embodiments, -(Ring B)-(R⁷)_{z2} is 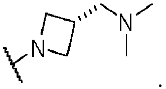 In embodiments, -(Ring B)-(R⁷)_{z2} is 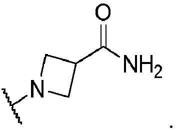 In embodiments, -(Ring B)-(R⁷)_{z2} is 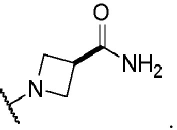 In embodiments, -(Ring B)-(R⁷)_{z2} is 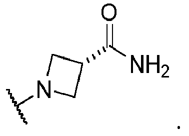 n embodiments, -(Ring B)-(R⁷)_{z2} is 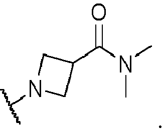 In embodiments, - (Ring B)-(R⁷)_{z2} is 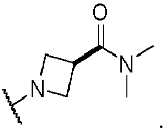 In embodiments, -(Ring B)-(R⁷)_{z2} is 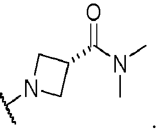 In embodiments, -(Ring B)-(R⁷)_{z2} is 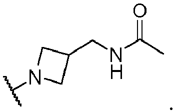 In embodiments, -(Ring B)-(R⁷)_{z2} is 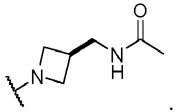 In embodiments, -(Ring B)-(R⁷)_{z2} is 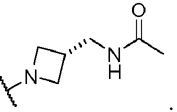 In embodiments, - (Ring B)-(R⁷)_{z2} is 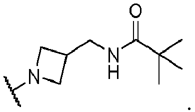 In embodiments, -(Ring B)-(R⁷)_{z2} is 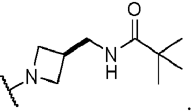 In embodiments, -(Ring B)-(R⁷)_{z2} is 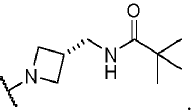 In embodiments, -(Ring B)-(R⁷)_{z2} is 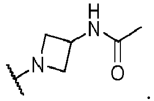 In embodiments, -(Ring B)-(R⁷)_{z2} is 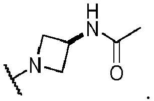 In embodiments, -(Ring B)-(R⁷)_{z2} is 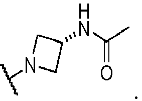 In embodiments, -(Ring B)-(R⁷)_{z2} is 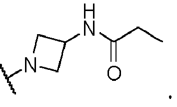 In embodiments, -(Ring B)-(R⁷)_{z2} is 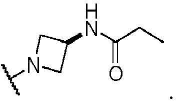 In embodiments, -(Ring B)-(R⁷)_{z2} is

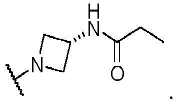

In embodiments, -(Ring B)-(R⁷)_{z2} is 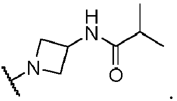 In embodiments, -(Ring B)-(R⁷)_{z2} is 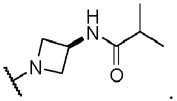 In embodiments, -(Ring B)-(R⁷)_{z2} is 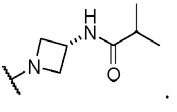 In embodiments, -(Ring B)-(R⁷)_{z2} is 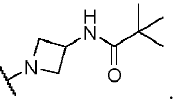 In embodiments, -(Ring B)-(R⁷)_{z2} is 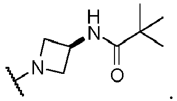 In embodiments, -(Ring B)-(R⁷)_{z2} is 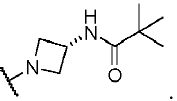 In embodiments, - (Ring B)-(R⁷)_{z2} is 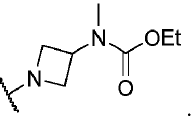 In embodiments, -(Ring B)-(R⁷)_{z2} is 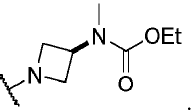 In embodiments, -(Ring B)-(R⁷)_{z2} is 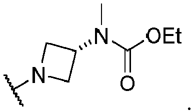 In embodiments, -(Ring B)-(R⁷)_{z2} is 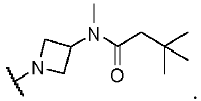 In embodiments, -(Ring B)-(R⁷)_{z2} is 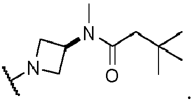 In embodiments, - (Ring B)-(R⁷)_{z2} is 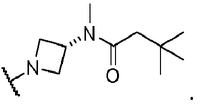 In embodiments, -(Ring B)-(R⁷)_{z2} is 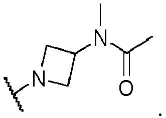 In embodiments, -(Ring B)-(R⁷)_{z2} is 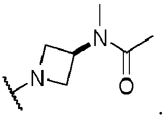 In embodiments, -(Ring B)-(R⁷)_{z2} is 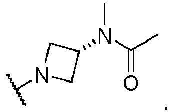 In embodiments, -(Ring B)-(R⁷)_{z2} is 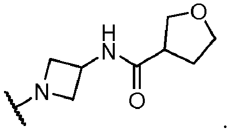 In embodiments,-(Ring B)-(R⁷)_{z2} is 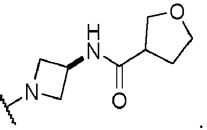 In embodiments, -(Ring B)-(R⁷)_{z2} is 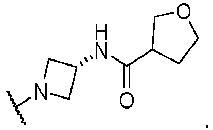 In embodiments, -(Ring B)-(R⁷)_{z2} is 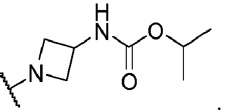 In embodiments, -(Ring B)-(R⁷)_{z2} is 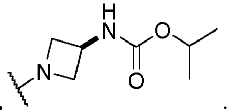 In embodiments, -(Ring B)-(R⁷)_{z2} is 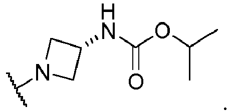 In embodiments, -(Ring B)-(R⁷)_{z2} is 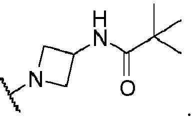 In embodiments, -(Ring B)-(R⁷)_{z2} is 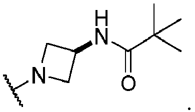 In embodiments, -(Ring B)-(R⁷)_{z2} is 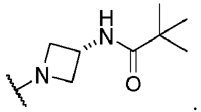 In embodiments, -(Ring B)-(R⁷)_{z2} is 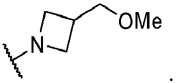 In embodiments,-(Ring B)-(R⁷)_{z2} is 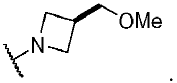 In embodiments, -(Ring B)-(R⁷)_{z2} is 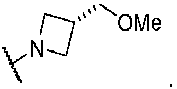 In embodiments, -(Ring B)-(R⁷)_{z2} is 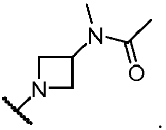 In embodiments, -(Ring B)-(R⁷)_{z2} is 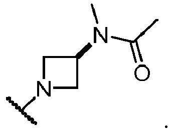 In embodiments, -(Ring B)-(R⁷)_{z2} is 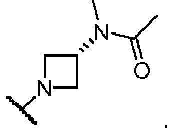 In embodiments,-(Ring B)-(R⁷)_{z2} is 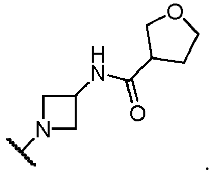 In embodiments, -(Ring B)-(R⁷)_{z2} is 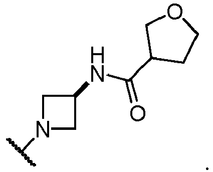 In embodiments, -(Ring B)-(R⁷)_{z2} is

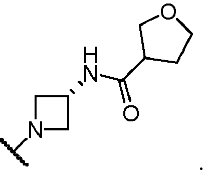

In embodiments, -(Ring B)-(R⁷)_{z2} is 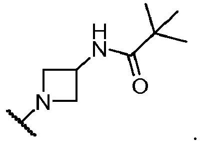 In embodiments, -(Ring B)-(R⁷)_{z2} is 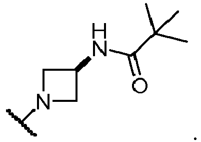 In embodiments, -(Ring B)-(R⁷)_{z2} is 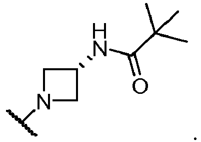 In embodiments, -(Ring B)-(R⁷)_{z2} is 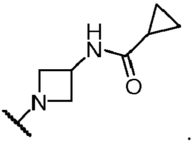 In embodiments, -(Ring B)-(R⁷)_{z2} is 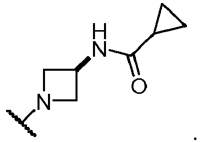 In embodiments, -(Ring B)-(R⁷)_{z2} is 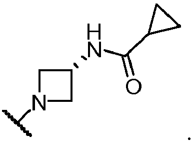 In embodiments, -(Ring B)-(R⁷)_{z2} is 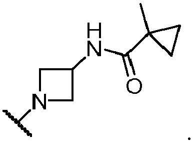 In embodiments, -(Ring B)-(R⁷)_{z2} is 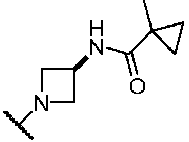 . In embodiments, -(Ring B)-(R⁷)_{z2} is 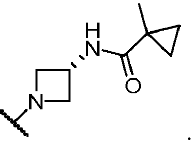 In embodiments, -(Ring B)-(R⁷)_{z2} 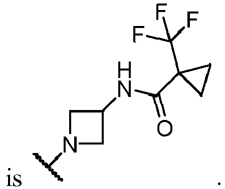 In embodiments, -(Ring B)-(R⁷)_{z2} is 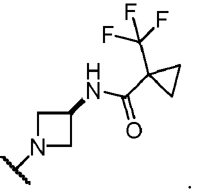 In embodiments, -(Ring B)-(R⁷)_{z2} is 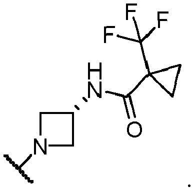 In embodiments, -(Ring B)-(R⁷)_{z2} is 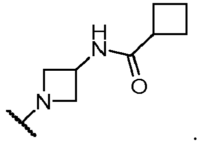 In embodiments, -(Ring B)-(R⁷)_{z2} is 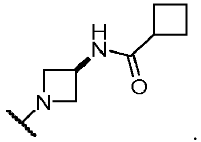 In embodiments, -(Ring B)-(R⁷)_{z2} is 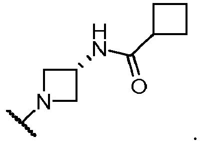 In embodiments, -(Ring B)-(R⁷)_{z2} is 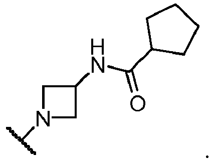 In embodiments, -(Ring B)-(R⁷)_{z2} is 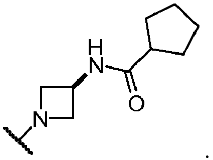 In embodiments, -(Ring B)-(R⁷)_{z2} is 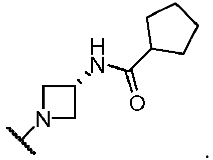 In embodiments, -(Ring B)-(R⁷)_{z2} is 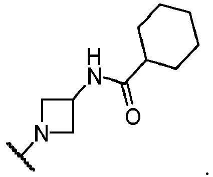 In embodiments, -(Ring B)-(R⁷)_{z2} is 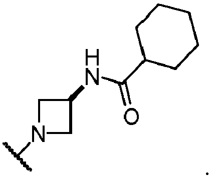 In embodiments, -(Ring B)-(R⁷)_{z2} is 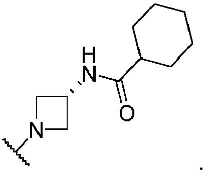 In embodiments, -(Ring B)-(R⁷)_{z2} is 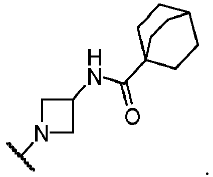 In embodiments, -(Ring B)-(R⁷)_{z2} is 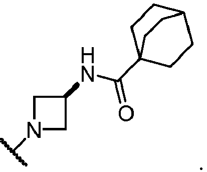 In embodiments, -(Ring B)-(R⁷)_{z2} is 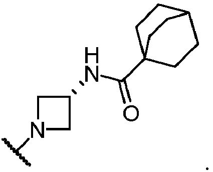 In embodiments, -(Ring B)-(R⁷)_{z2} is 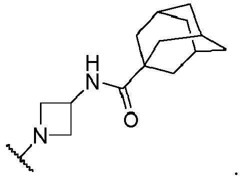 In embodiments, -(Ring B)-(R⁷)_{z2} is 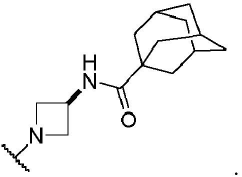 In embodiments, -(Ring B)-(R⁷)_{z2} is 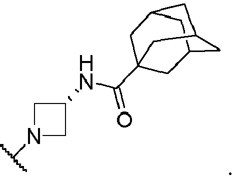 In embodiments, -(Ring B)-(R⁷)_{z2} 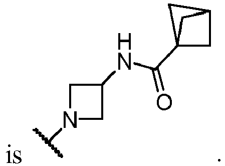 In embodiments, -(Ring B)-(R⁷)_{z2} is 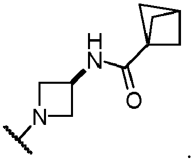 In embodiments, -(Ring B)-(R⁷)_{z2} is 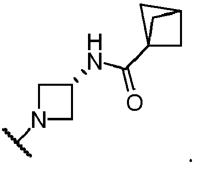 In embodiments, -(Ring B)-(R⁷)_{z2} is 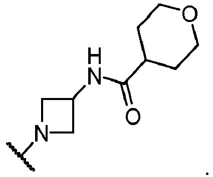 In embodiments, -(Ring B)-(R⁷)_{z2} is 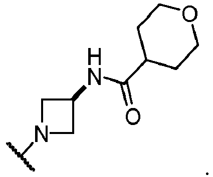 In embodiments, -(Ring B)-(R⁷)_{z2} is 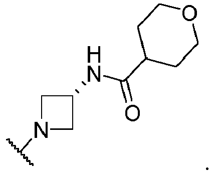 In embodiments, -(Ring B)-(R⁷)_{z2} is 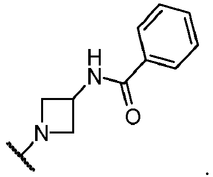 In embodiments, -(Ring B)-(R⁷)_{z2} is 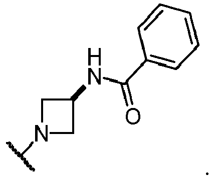 In embodiments, -(Ring B)-(R⁷)_{z2} is 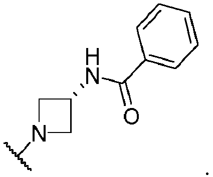 In embodiments, -(Ring B)-(R⁷)_{z2} is 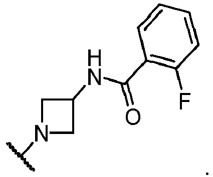 In embodiments, -(Ring B)-(R⁷)_{z2} is 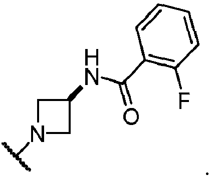 In embodiments, -(Ring B)-(R⁷)_{z2} is 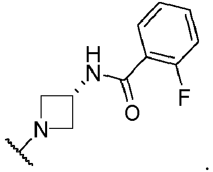 In embodiments, -(Ring B)-(R⁷)_{z2} is 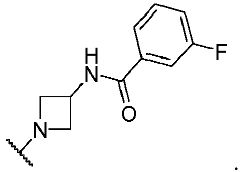 In embodiments, -(Ring B)-(R⁷)_{z2} is 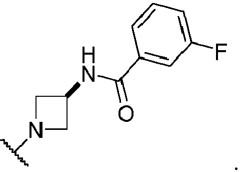 In embodiments, -(Ring B)-(R⁷)_{z2} is 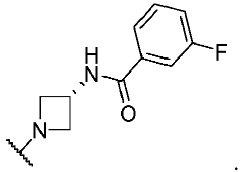 In embodiments, -(Ring B)-(R⁷)_{z2} 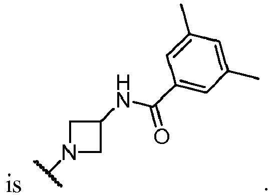 In embodiments, -(Ring B)-(R⁷)_{z2} is 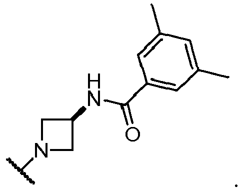 In embodiments, -(Ring B)-(R⁷)_{z2} is 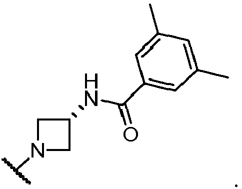 In embodiments, -(Ring B)-(R⁷)_{z2} 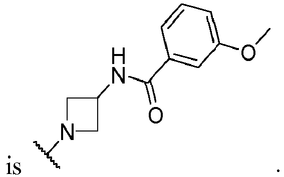 In embodiments, -(Ring B)-(R⁷)_{z2} is 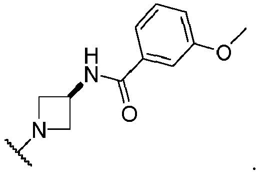 In embodiments, -(Ring B)-(R⁷)_{z2} is 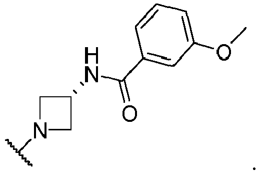 In embodiments, -(Ring B)-(R⁷)_{z2} 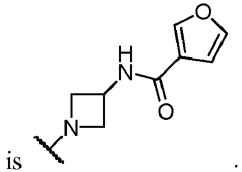 In embodiments, -(Ring B)-(R⁷)_{z2} is 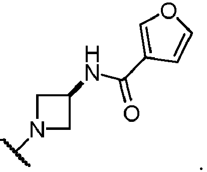 In embodiments, -(Ring B)-(R⁷)_{z2} is

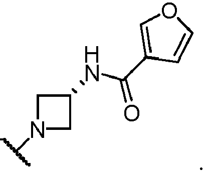

In embodiments, -(Ring B)-(R⁷)_{z2} is 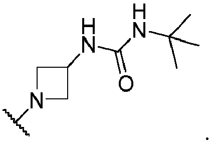 In embodiments, -(Ring B)-(R⁷)_{z2} is 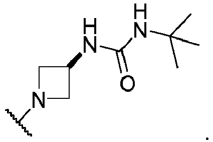 In embodiments, -(Ring B)-(R⁷)_{z2} is 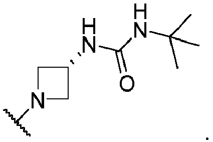 In embodiments, -(Ring B)-(R⁷)_{z2} is 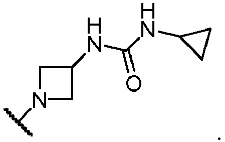 In embodiments, -(Ring B)-(R⁷)_{z2} is 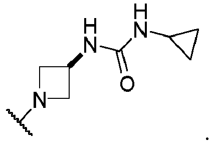 In embodiments, -(Ring B)-(R⁷)_{z2} is 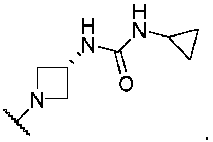 In embodiments, -(Ring B)-(R⁷)_{z2} is 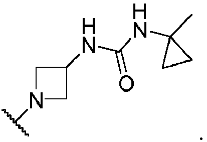 In embodiments, -(Ring B)-(R⁷)_{z2} is 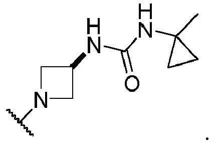 In embodiments, -(Ring B)-(R⁷)_{z2} is 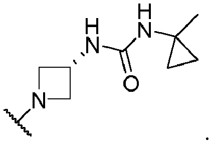 In embodiments, -(Ring B)-(R⁷)_{z2} is 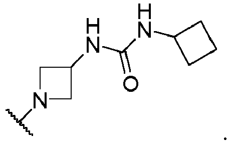 In embodiments, -(Ring B)-(R⁷)_{z2} is 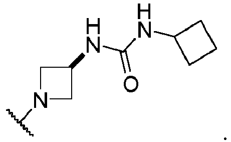 In embodiments, -(Ring B)-(R⁷)_{z2} is 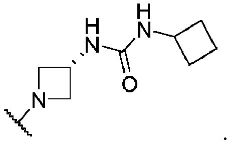 In embodiments, -(Ring B)-(R⁷)_{z2} is 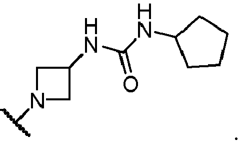 In embodiments, -(Ring B)-(R⁷)_{z2} 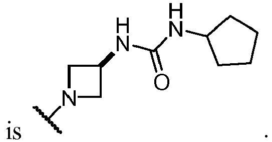 In embodiments, -(Ring B)-(R⁷)_{z2} is 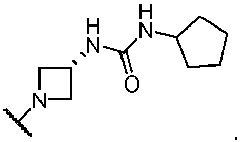 In embodiments, -(Ring B)-(R⁷)_{z2} is 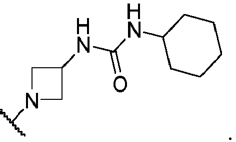 In embodiments, -(Ring B)-(R⁷)_{z2} 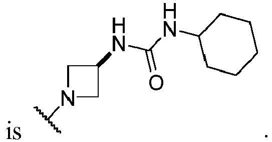 In embodiments, -(Ring B)-(R⁷)_{z2} is 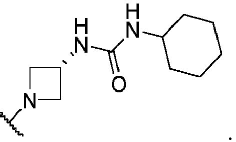 In embodiments, -(Ring B)-(R⁷)_{z2} is 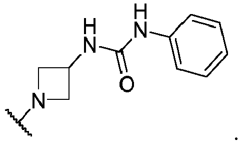 In embodiments, -(Ring B)-(R)_{z2} 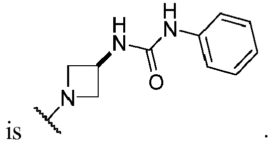 In embodiments, -(Ring B)-(R⁷)_{z2} is 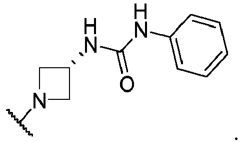 In embodiments, -(Ring B)-(R⁷)_{z2} is 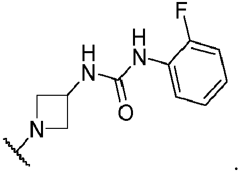 In embodiments, -(Ring B)-(R⁷)_{z2} 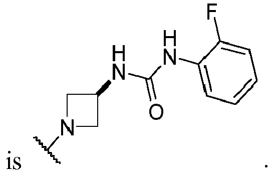 In embodiments, -(Ring B)-(R⁷)_{z2} is 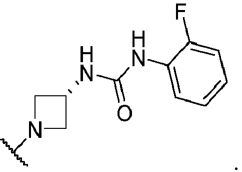 In embodiments, -(Ring B)-(R⁷)_{z2} is 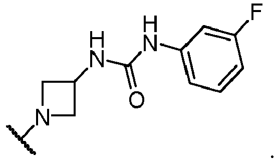 In embodiments, -(Ring B)-(R⁷)_{z2} is 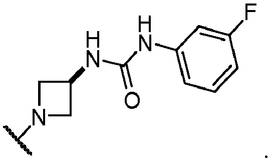 In embodiments, -(Ring B)-(R⁷)_{z2} is 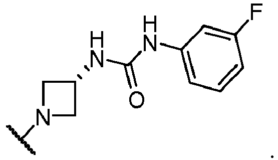 In embodiments, -(Ring B)-(R⁷)_{z2} is 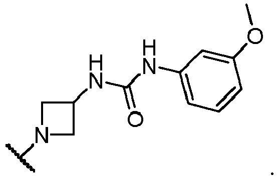 In embodiments, -(Ring B)-(R⁷)_{z2} is 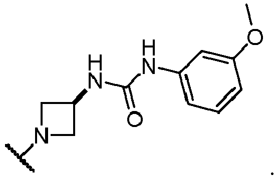 In embodiments, -(Ring B)-(R⁷)_{z2} is 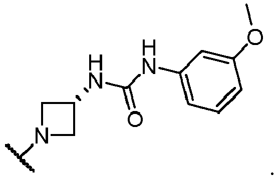 In embodiments, -(Ring B)-(R⁷)_{z2} is 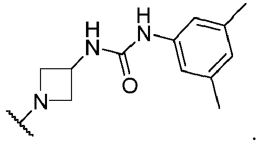 In embodiments, -(Ring B)-(R⁷)_{z2} is 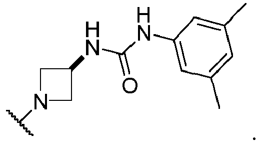 In embodiments, -(Ring B)-(R⁷)_{z2} is

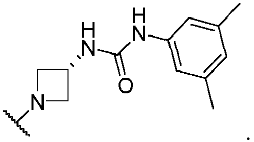

In embodiments, -(Ring B)-(R⁷)_{z2} is 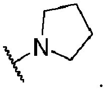 In embodiments, -(Ring B)-(R⁷)_{z2} is 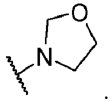 In embodiments, -(Ring B)-(R⁷)_{z2} is 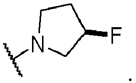 In embodiments, -(Ring B)-(R⁷)_{z2} is 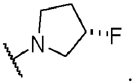 In embodiments, -(Ring B)-(R⁷)_{z2} is 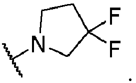 In embodiments, - (Ring B)-(R⁷)_{z2} is 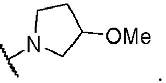 In embodiments, -(Ring B)-(R⁷)_{z2} is 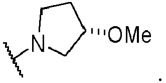 In embodiments, -(Ring B)-(R⁷)_{z2} is 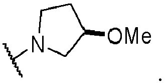 In embodiments, -(Ring B)-(R⁷)_{z2} is 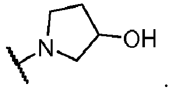 In embodiments, -(Ring B)-(R⁷)_{z2} is 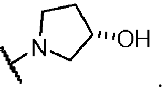 In embodiments, -(Ring B)-(R⁷)_{z2} is 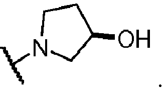 In embodiments, -(Ring B)-(R⁷)_{z2} is 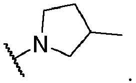 In embodiments, -(Ring B)-(R⁷)_{z2} is 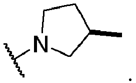 In embodiments, -(Ring B)-(R⁷)_{z2} is 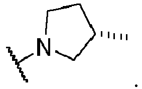 In embodiments, -(Ring B)-(R⁷)_{z2} is 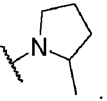 In embodiments, -(Ring B)-(R⁷)_{z2} is 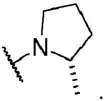 In embodiments, -(Ring B)-(R⁷)_{z2} is 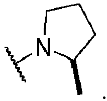 In embodiments, -(Ring B)-(R⁷)_{z2} is 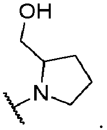 In embodiments, -(Ring B)-(R⁷)_{z2} is 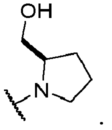 In embodiments, -(Ring B)-(R⁷)_{z2} is 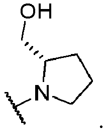 In embodiments, -(Ring B)-(R⁷)_{z2} is 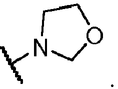 In embodiments, -(Ring B)-(R⁷)_{z2} is 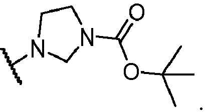 In embodiments, -(Ring B)-(R⁷)_{z2} is 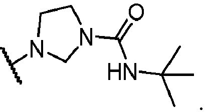 In embodiments, -(Ring B)-(R⁷)_{z2} is 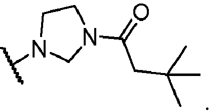 In embodiments, -(Ring B)-(R⁷)_{z2} is 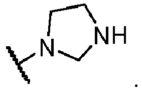 In embodiments, -(Ring B)-(R⁷)_{z2} is 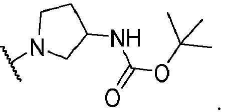 In embodiments,-(Ring B)-(R⁷)_{z2} is 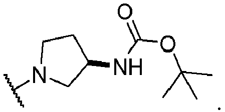 In embodiments, -(Ring B)-(R⁷)_{z2} is 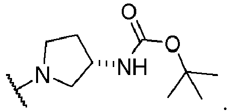 In embodiments, -(Ring B)-(R⁷)_{z2} is 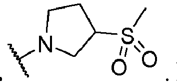 In embodiments, - (Ring B)-(R⁷)_{z2} is 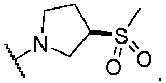 In embodiments, -(Ring B)-(R⁷)_{z2} is 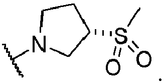 In embodiments, -(Ring B)-(R⁷)_{z2} is 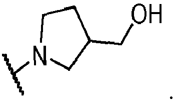 In embodiments, -(Ring B)-(R⁷)_{z2} is 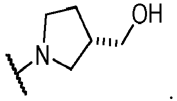 In embodiments, -(Ring B)-(R⁷)_{z2} is 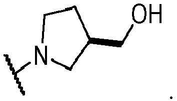 In embodiments, - (Ring B)-(R⁷)_{z2} is 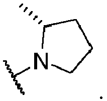 In embodiments, -(Ring B)-(R⁷)_{z2} is 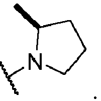 In embodiments,-(Ring B)-(R⁷)_{z2} is 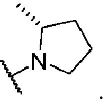 In embodiments, -(Ring B)-(R⁷)_{z2} is 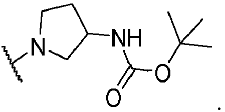 In embodiments, -(Ring B)-(R⁷)_{z2} is 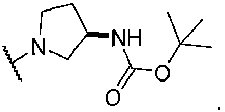 In embodiments, -(Ring B)-(R⁷)_{z2} is 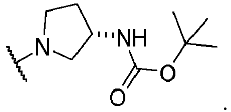 In embodiments, -(Ring B)-(R⁷)_{z2} is 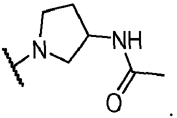 In embodiments, -(Ring B)-(R⁷)_{z2} is 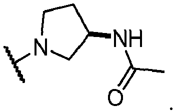 In embodiments, -(Ring B)-(R⁷)_{z2} is 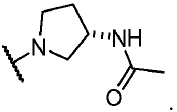 In embodiments, -(Ring B)-(R⁷)_{z2} is 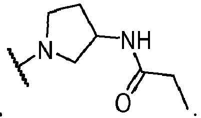 In embodiments, -(Ring B)-(R⁷)_{z2} is 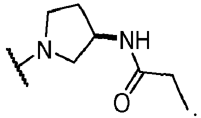 In embodiments, -(Ring B)-(R⁷)_{z2} is 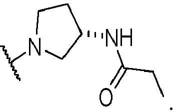 In embodiments, - (Ring B)-(R⁷)_{z2} is 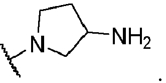 In embodiments, -(Ring B)-(R⁷)_{z2} is 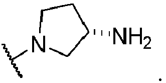 In embodiments, -(Ring B)-(R⁷)_{z2} is 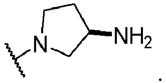 In embodiments, -(Ring B)-(R⁷)_{z2} is 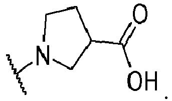 In embodiments, -(Ring B)-(R⁷)_{z2} is 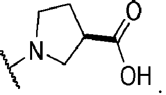 In embodiments, -(Ring B)-(R⁷)_{z2} is 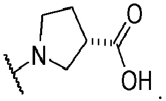 In embodiments, -(Ring B)-(R⁷)_{z2} is 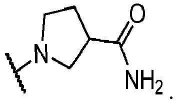 In embodiments, -(Ring B)-(R⁷)_{z2} is 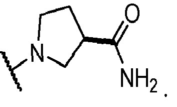 In embodiments, -(Ring B)-(R⁷)_{z2} is 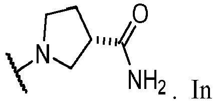 embodiments, -(Ring B)-(R⁷)_{z2} is 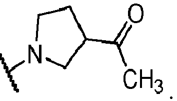 In embodiments, - (Ring B)-(R⁷)_{z2} is 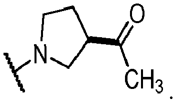 In embodiments, -(Ring B)-(R⁷)_{z2} is 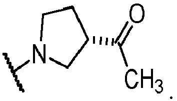 In embodiments, -(Ring B)-(R⁷)_{z2} is 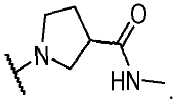 In embodiments, -(Ring B)-(R⁷)_{z2} is 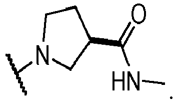 In embodiments, -(Ring B)-(R⁷)_{z2} is

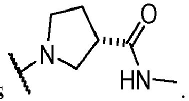

In embodiments, -(Ring B)-(R⁷)_{z2} is 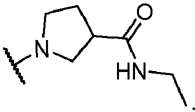 In embodiments, -(Ring B)-(R⁷)_{z2} is 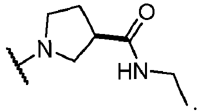 In embodiments, -(Ring B)-(R⁷)_{z2} is 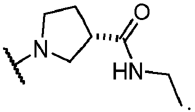 In embodiments, -(Ring B)-(R⁷)_{z2} is 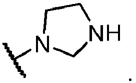 In embodiments, -(Ring B)-(R⁷)_{z2} is 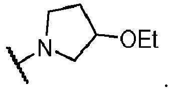 In embodiments, -(Ring B)-(R⁷)_{z2} is 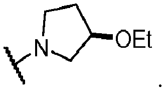 In embodiments, -(Ring B)-(R⁷)_{z2} is 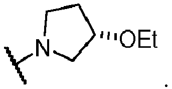 In embodiments, -(Ring B)-(R⁷)_{z2} is 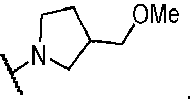 In embodiments, -(Ring B)-(R⁷)_{z2} is 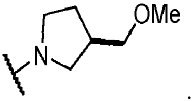 In embodiments, -(Ring B)-(R⁷)_{z2} is 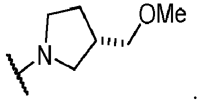 In embodiments, -(Ring B)-(R⁷)z₂ is 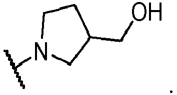 In embodiments,-(Ring B)-(R⁷)z₂ is 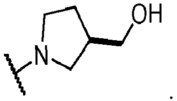 In embodiments, -(Ring B)-(R⁷)z₂ is 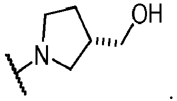 In embodiments, -(Ring B)-(R⁷)z₂ is 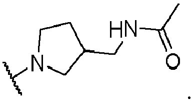 In embodiments, -(Ring B)-(R⁷)z₂ is 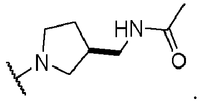 In embodiments, -(Ring B)-(R⁷)z₂ is

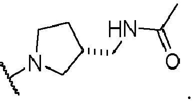

In embodiments, the compound is: 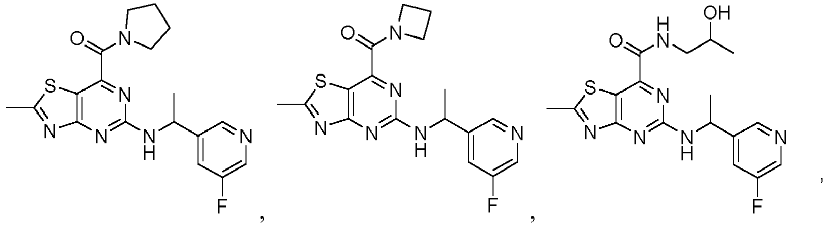 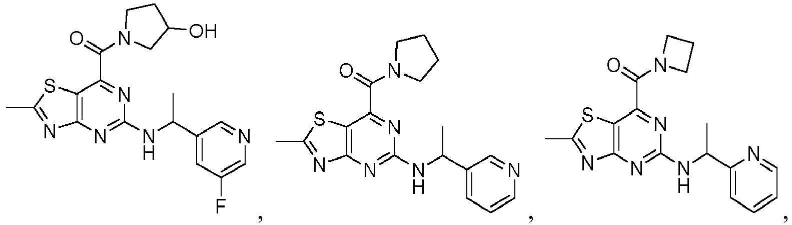 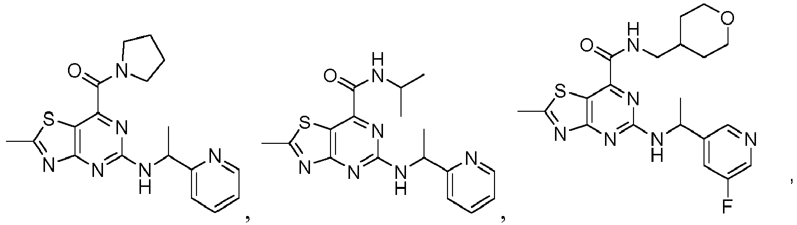 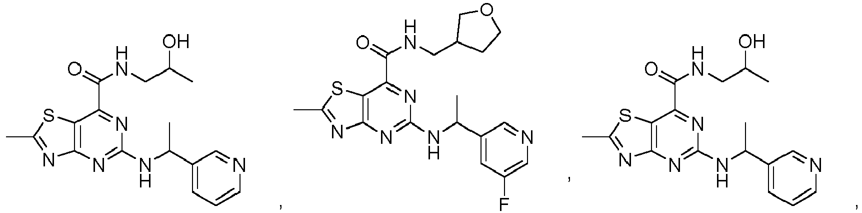 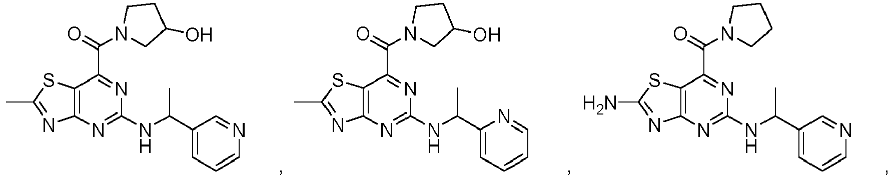 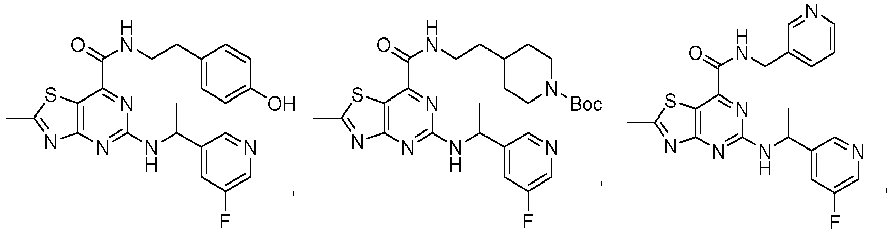 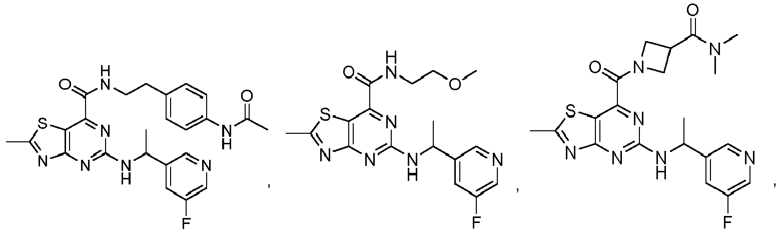 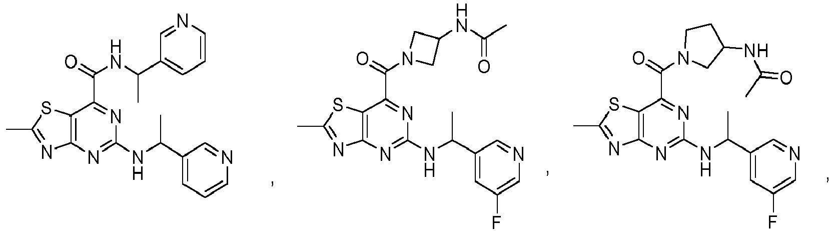 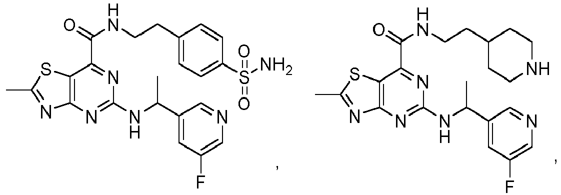 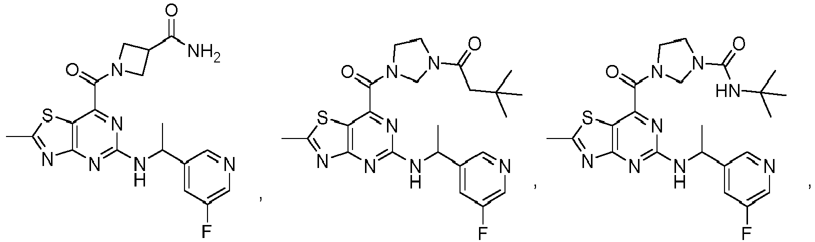 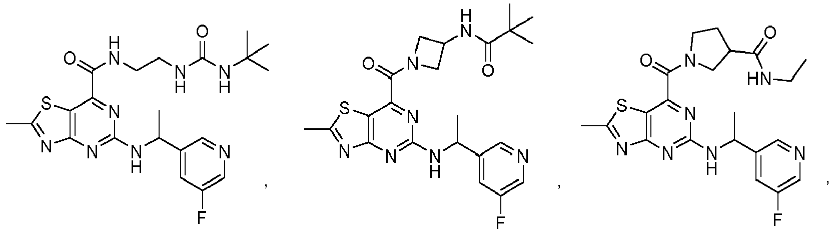 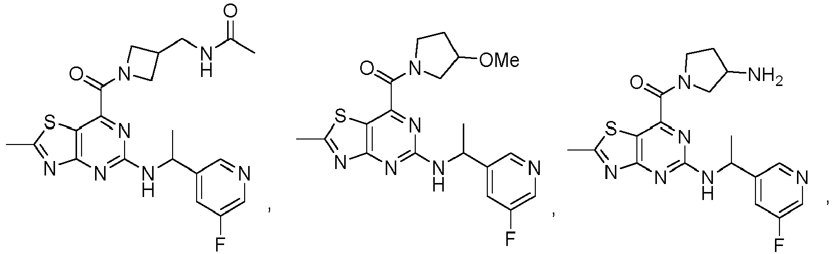 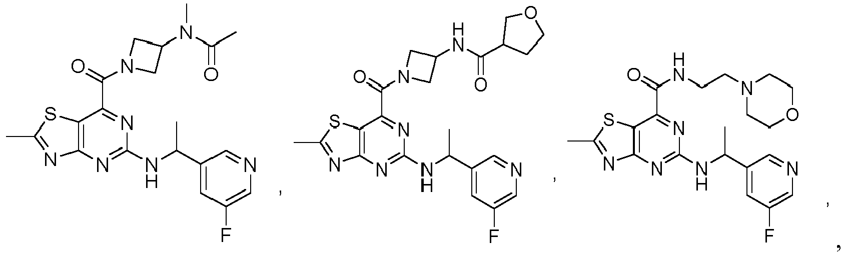 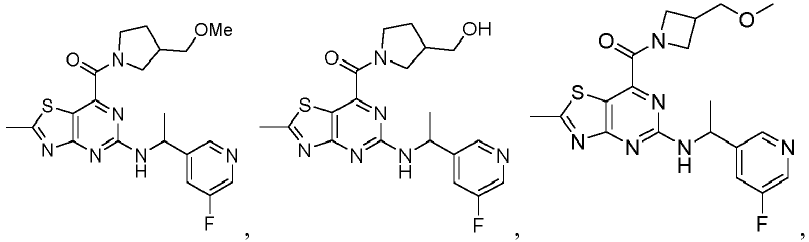 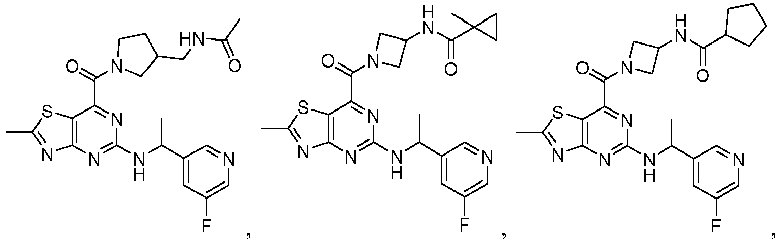 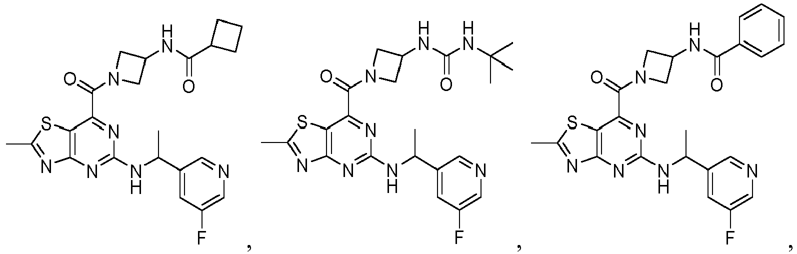 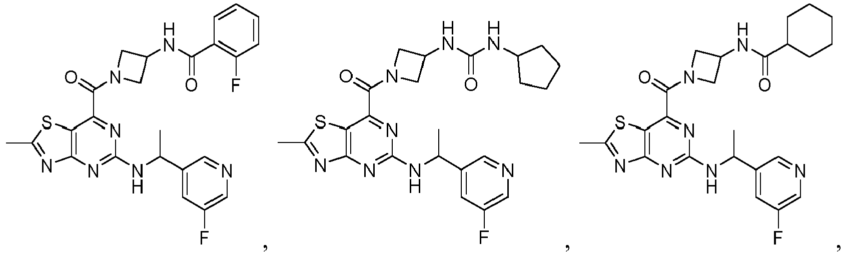 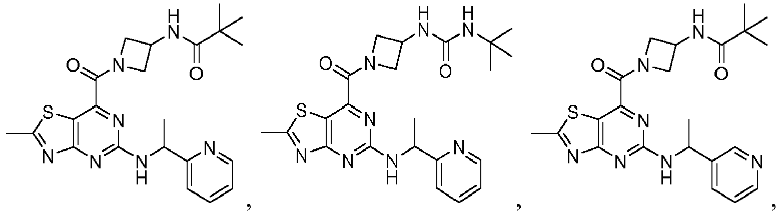 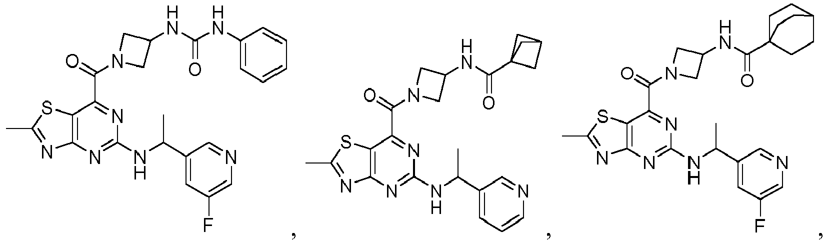 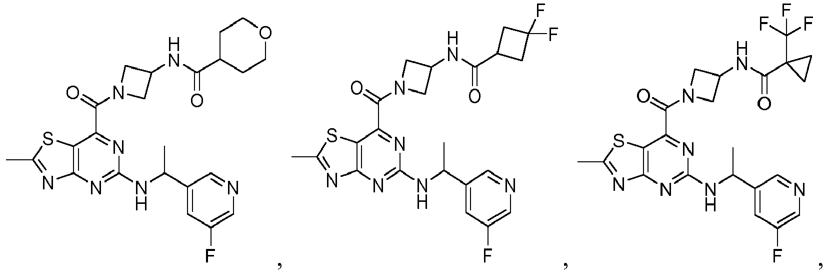 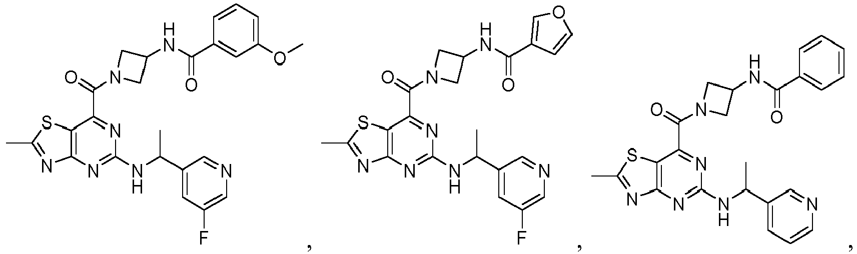 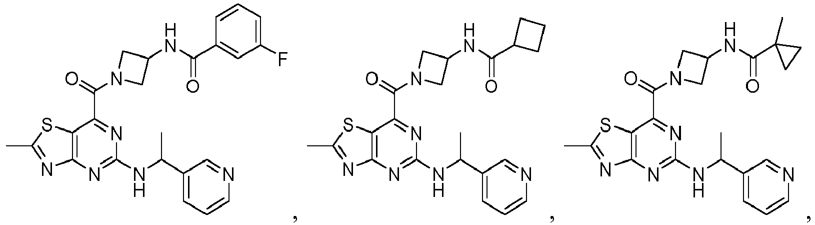 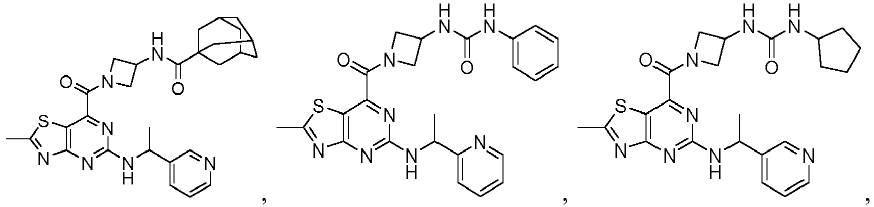 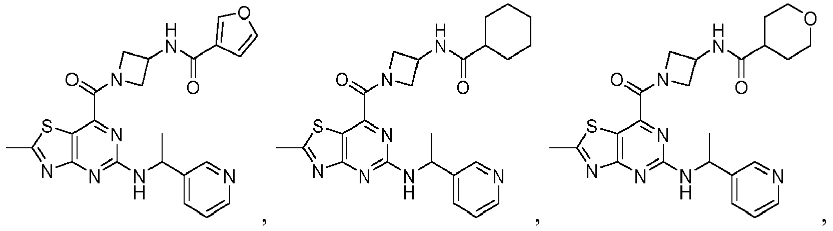 or

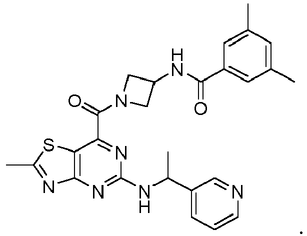

In embodiments, the compound is: 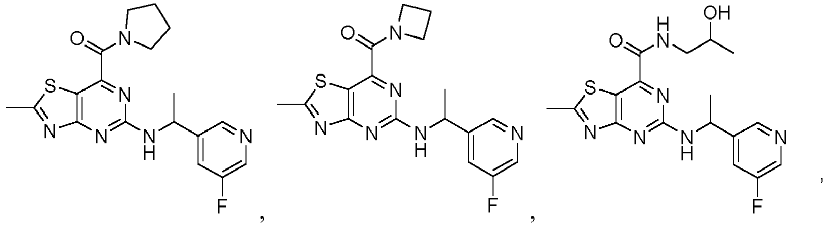 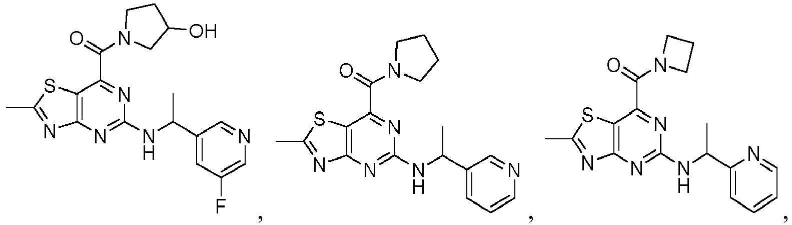 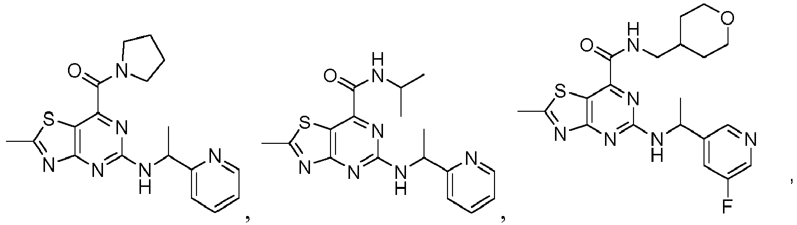 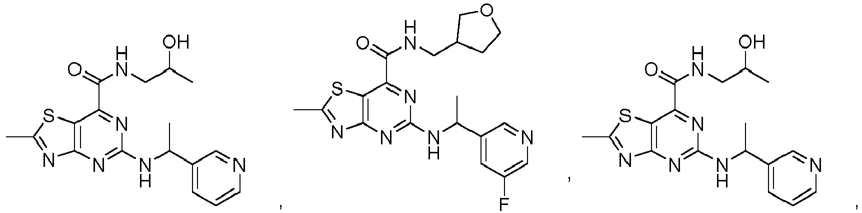 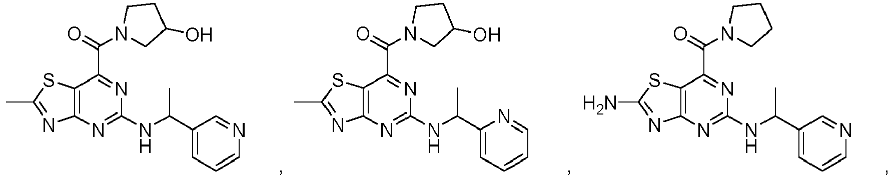 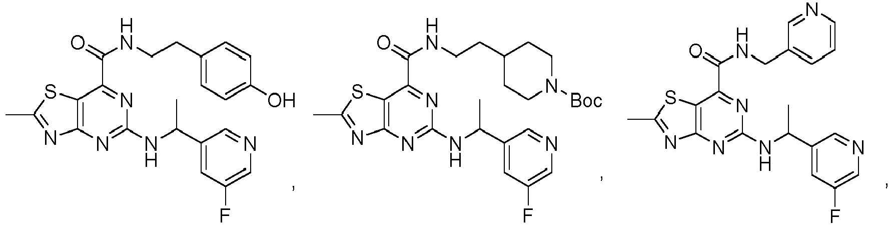 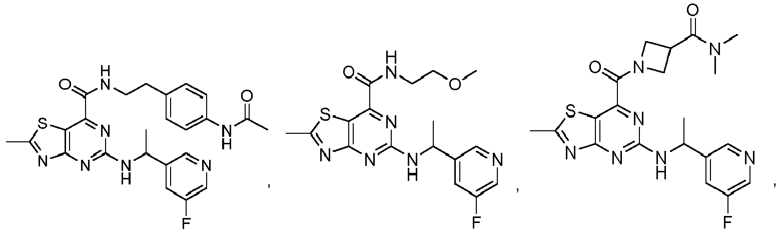 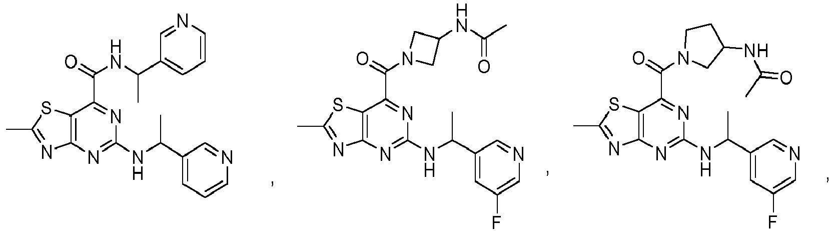 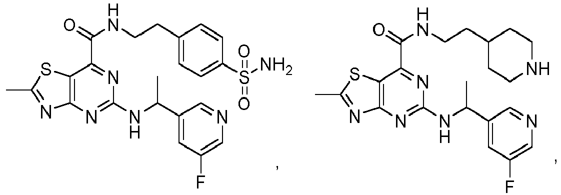 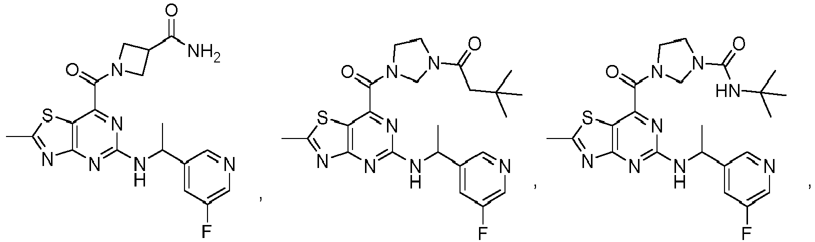 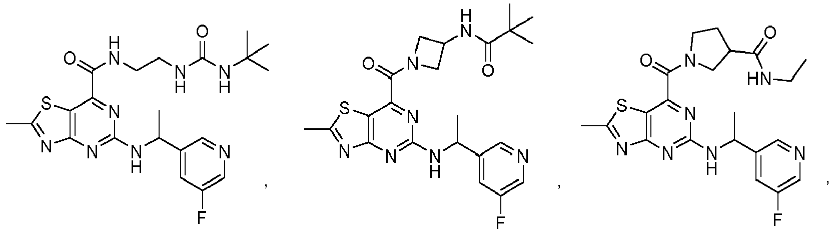 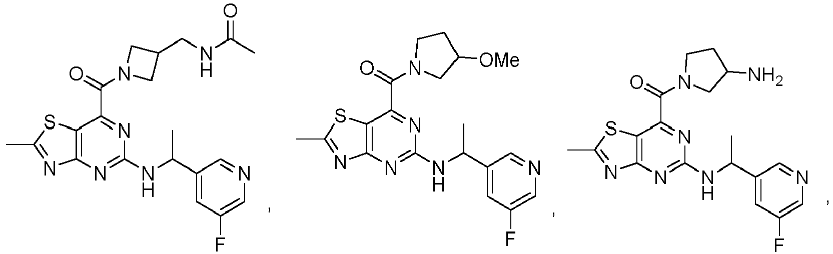 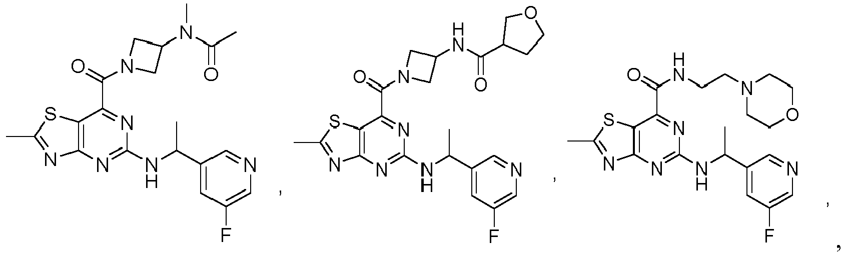 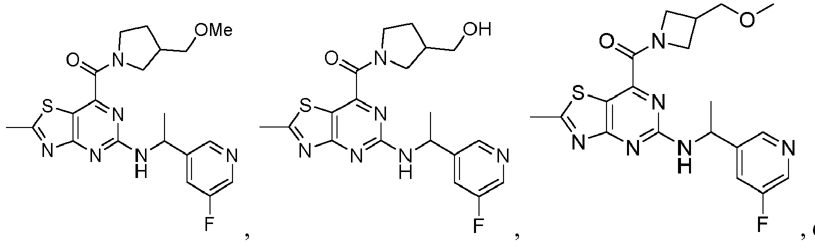 or

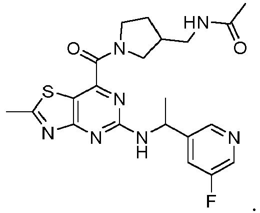

In embodiments, the compound is:

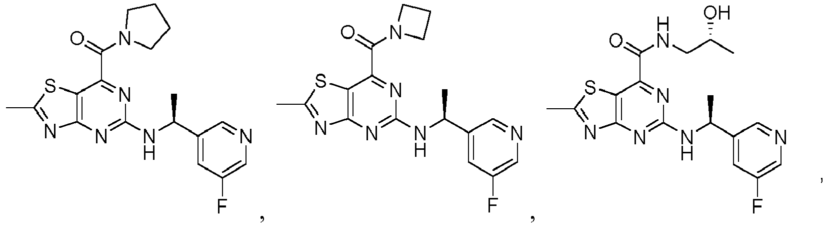

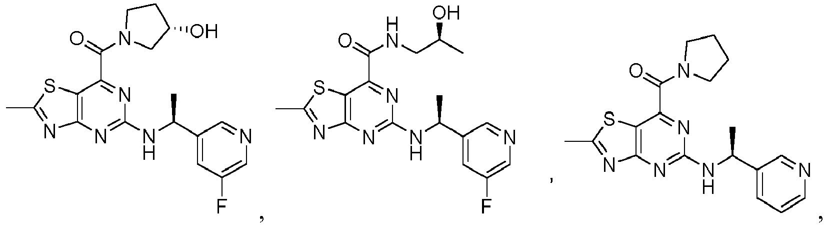

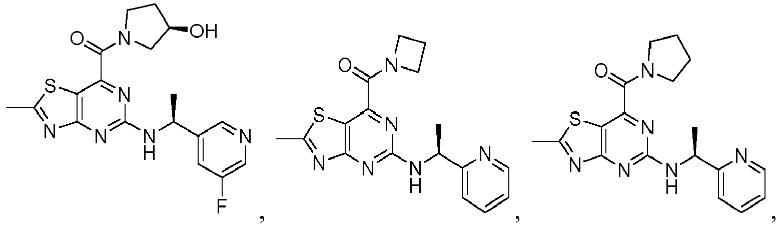

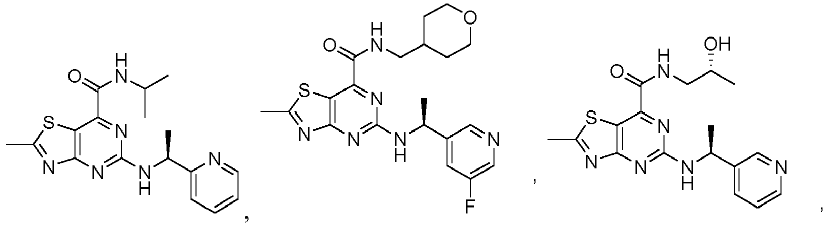

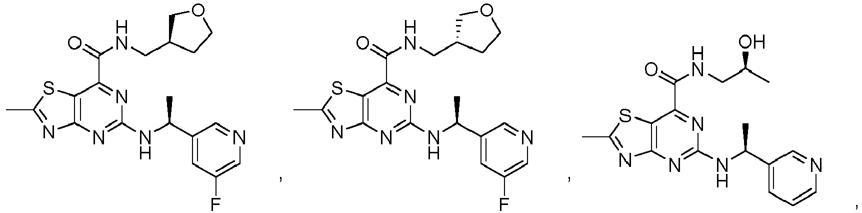

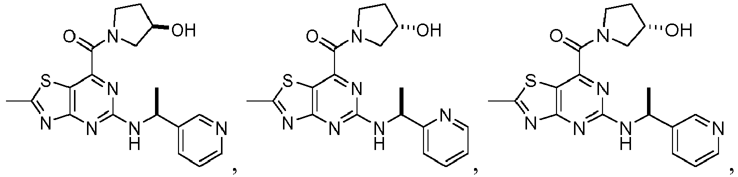

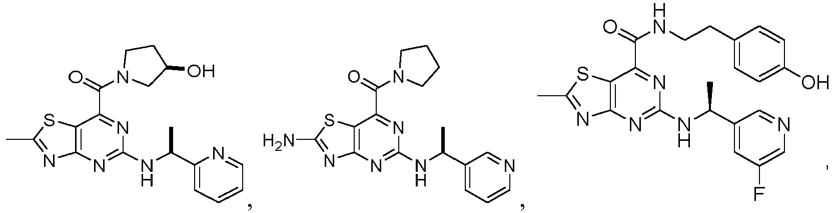

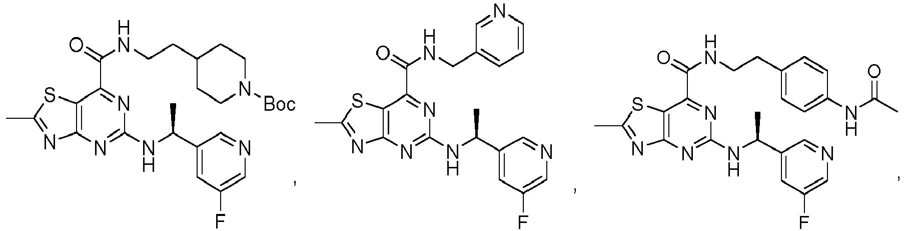

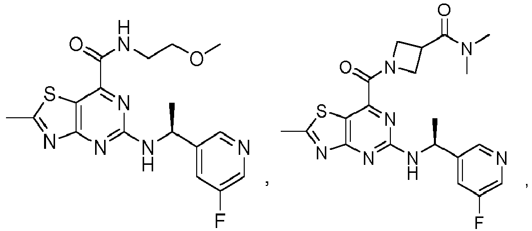

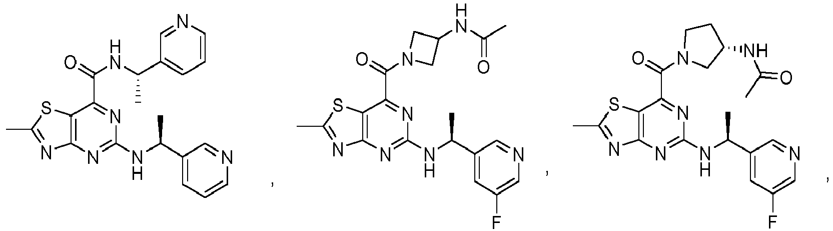

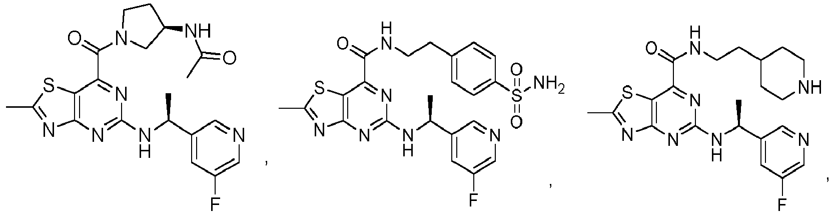

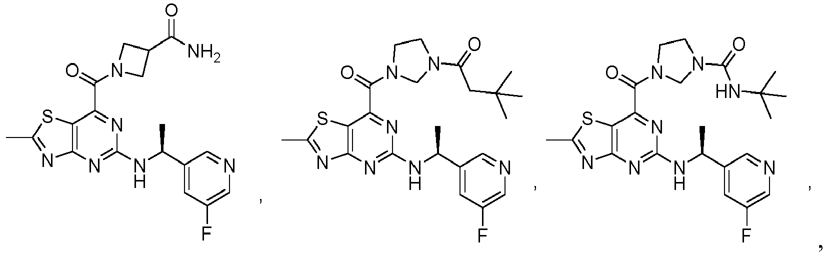

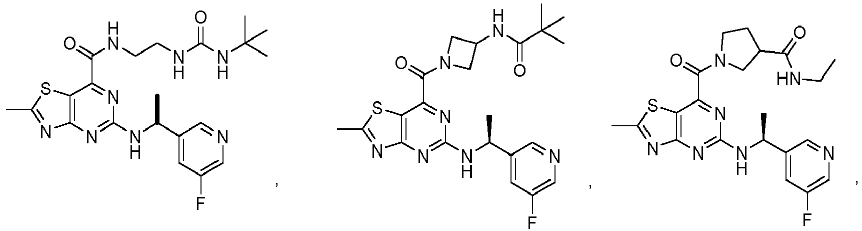

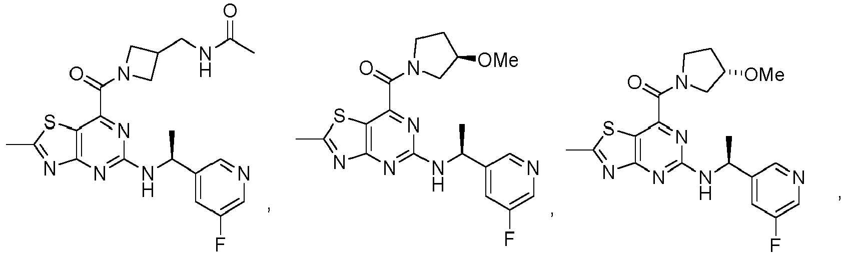

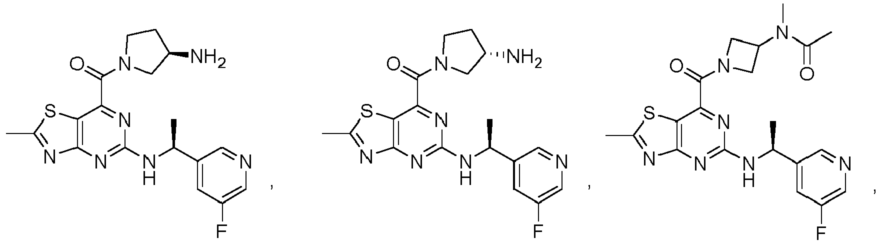

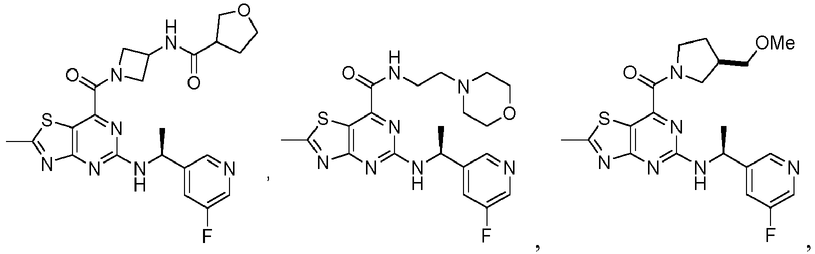

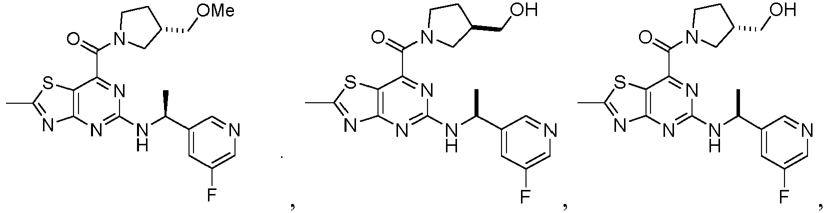

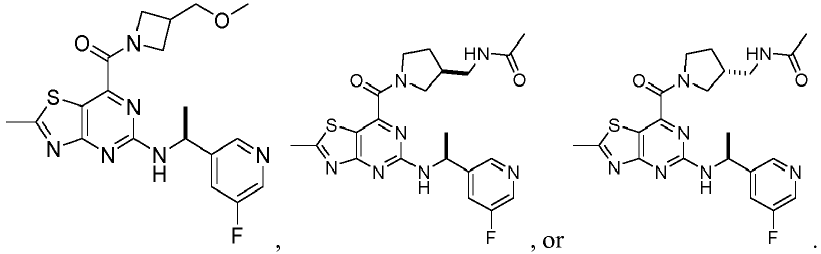

In embodiments, the compound is:

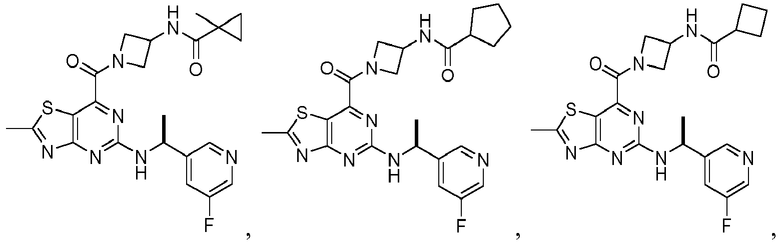

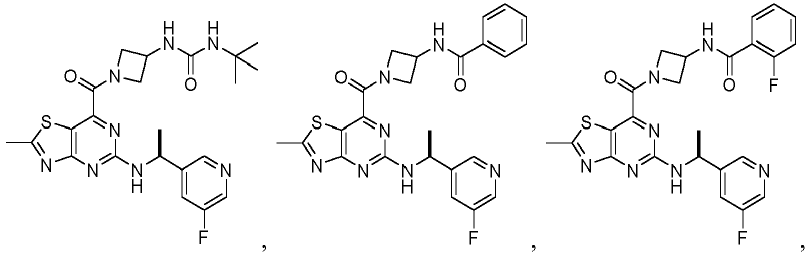

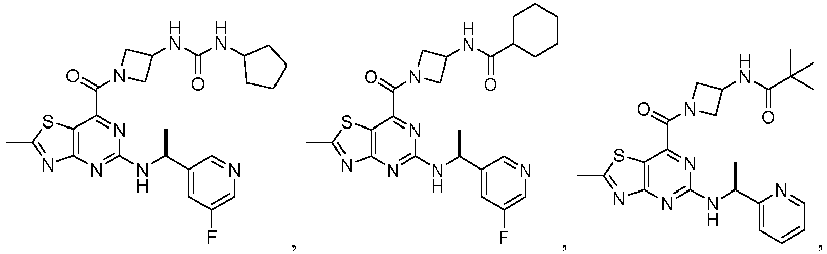

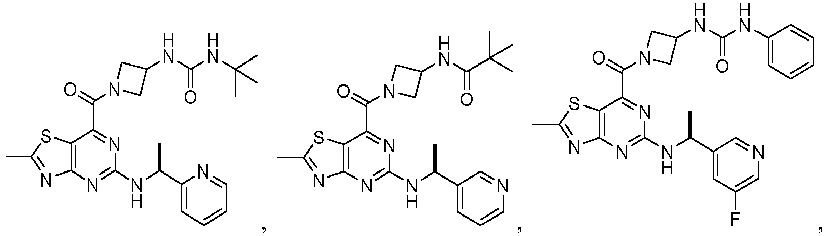

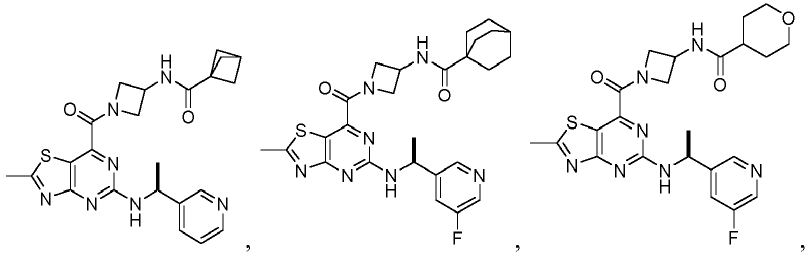

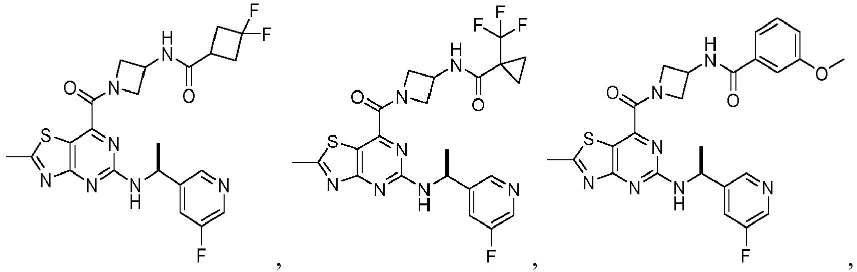

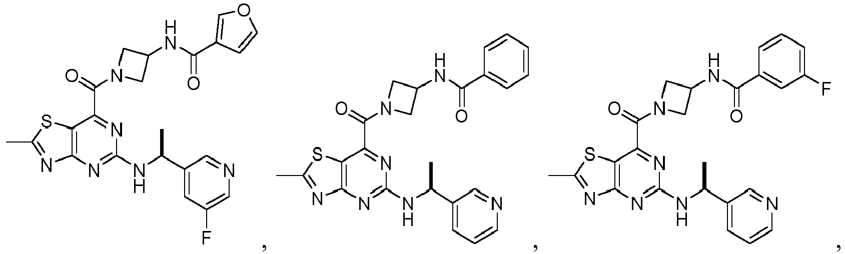

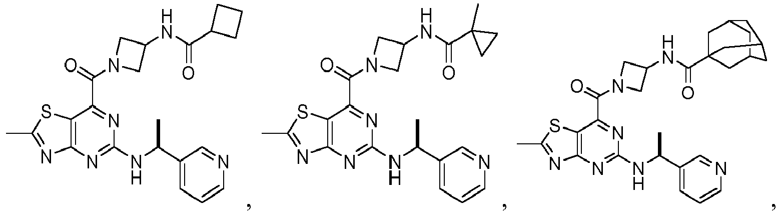

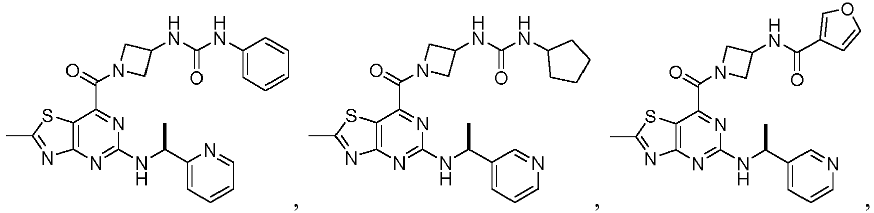

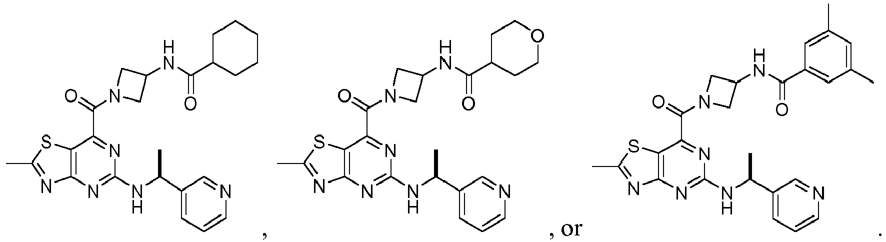

In embodiments, the compound is 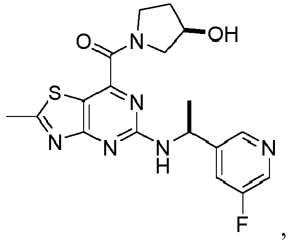 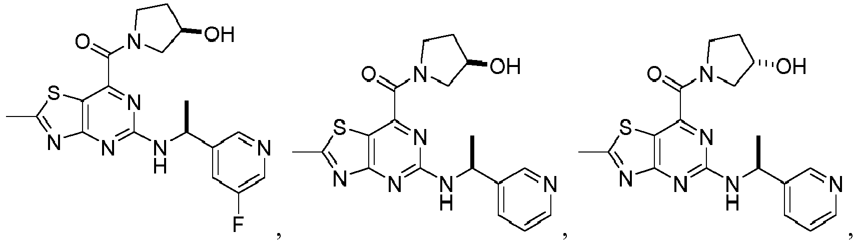 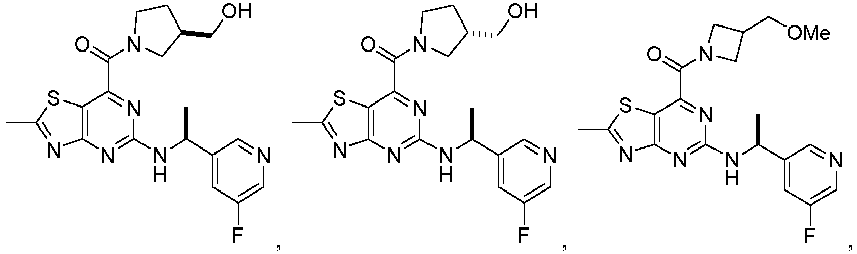 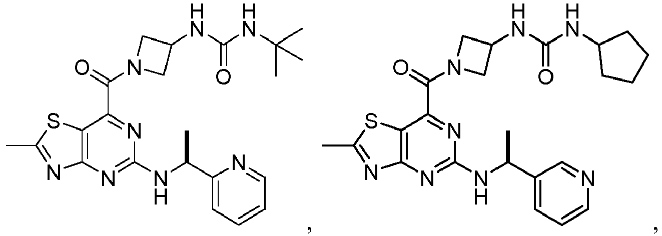 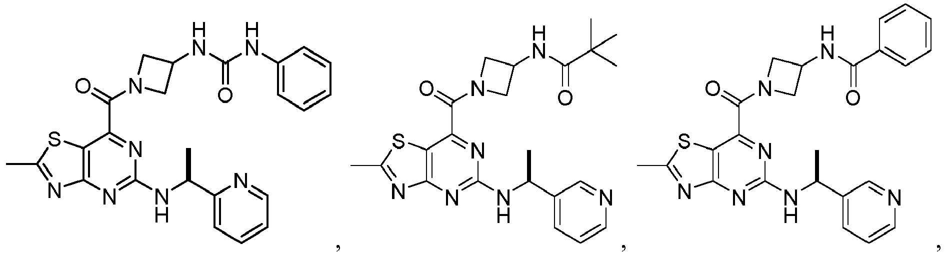 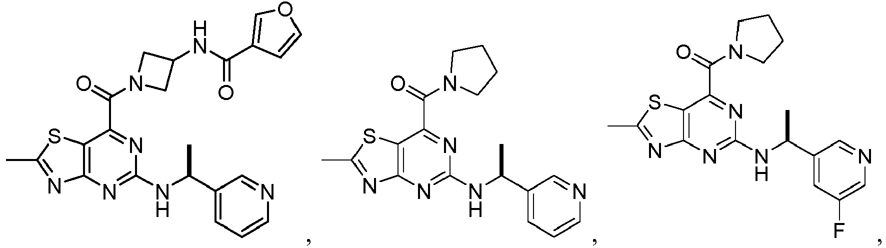 or 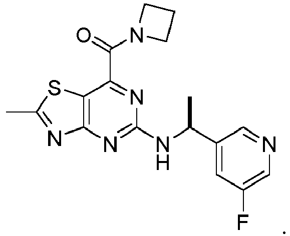 In embodiments, the compound is 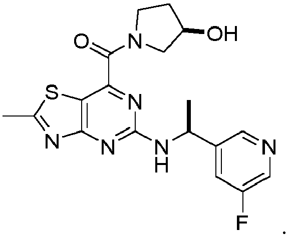 In embodiments, the compound is 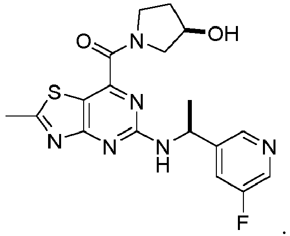 In embodiments, the compound is 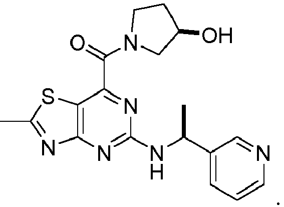 In embodiments, the compound is 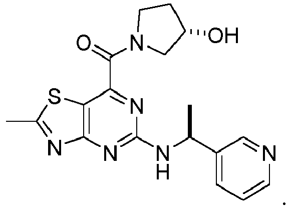 In embodiments, the compound is 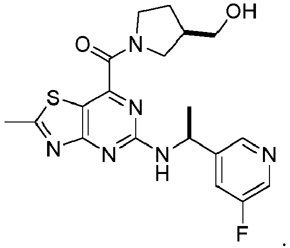 In embodiments, the compound is 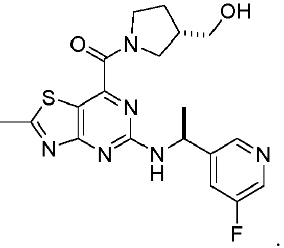 In embodiments, the compound is 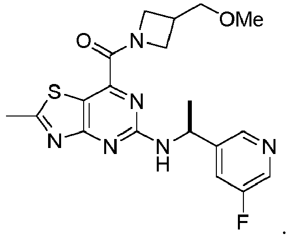 In embodiments, the compound is 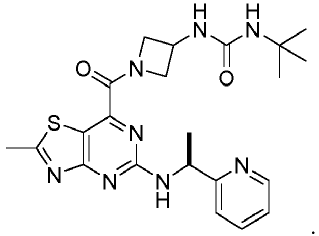 In embodiments, the compound is 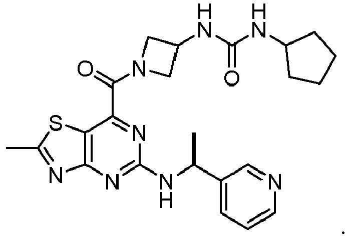 In embodiments, the compound is 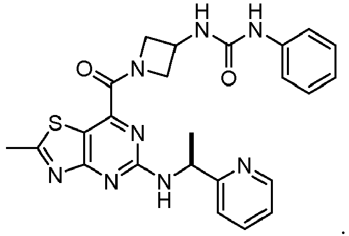 In embodiments, the compound is 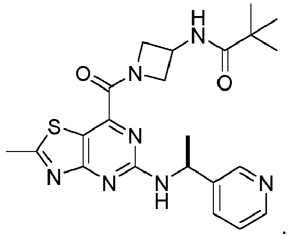 In embodiments, the compound is 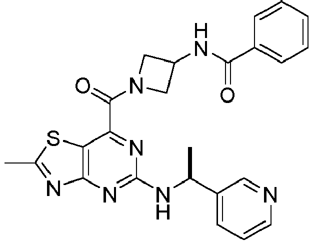 . In embodiments, the compound is 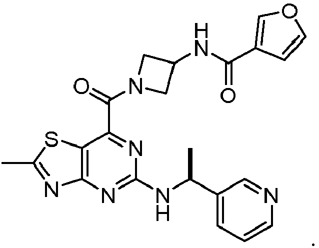 In embodiments, the compound is 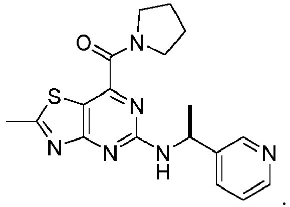 In embodiments, the compound is 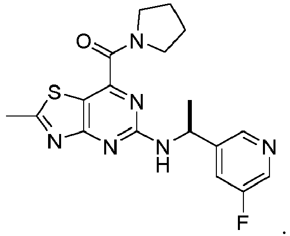 In embodiments, the compound is

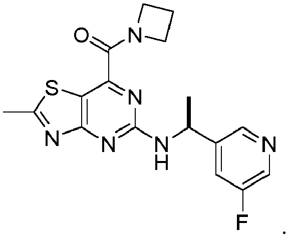

### III. Pharmaceutical compositions

In an aspect is provided a pharmaceutical composition including a compound as described herein, and a pharmaceutically acceptable excipient.

In embodiments of the pharmaceutical compositions, the compound, or pharmaceutically acceptable salt thereof, is included in a therapeutically effective amount.

In embodiments of the pharmaceutical compositions, the pharmaceutical composition includes a second agent (e.g., therapeutic agent). In embodiments of the pharmaceutical compositions, the pharmaceutical composition includes a second agent (e.g., therapeutic agent) in a therapeutically effective amount. In embodiments of the pharmaceutical compositions, the second agent is an agent for treating cancer. In embodiments, the second agent is an anti-cancer agent. In embodiments, the second agent is a chemotherapeutic. In embodiments, the second agent is an anti-inflammatory agent. In embodiments, the second agent is an anti-autoimmune disease agent. In embodiments, the second agent is an anti-infectious disease agent. In embodiments, the second agent is an anti-fibrotic agent. In embodiments, the second agent is an anti-neurodegenerative disease agent. In embodiments, the second agent is an agent for treating fibrosis. In embodiments, the second agent is an agent for treating a neurodegenerative disease. In embodiments, the administering does not include administration of any active agent other than the recited active agent (e.g., a compound described herein).

### IV. Methods of use

In an aspect is provided a method of inhibiting Adenosine A2B Receptor activity and Adenosine A2A Receptor activity, the method including contacting the Adenosine A2B Receptor and Adenosine A2A Receptor with a compound as described herein, including embodiments. In embodiments, the compound contacts the Adenosine A2B Receptor. In embodiments, the compound contacts the Adenosine A2A Receptor. In embodiments, the compound is capable of independently contacting the Adenosine A2A Receptor and the Adenosine A2B Receptor. In embodiments, the compound does not contact the Adenosine A2A Receptor and the Adenosine A2B Receptor simultaneously.

In an aspect is provided a method of inhibiting Adenosine A2B Receptor activity and Adenosine A2A Receptor activity, the method including contacting the Adenosine A2B Receptor or Adenosine A2A Receptor with a compound as described herein, including embodiments.

In embodiments, the method inhibits Adenosine A2B Receptor activity to Adenosine A2A Receptor activity at a ratio of about 1 to about 1. In embodiments, the method inhibits Adenosine A2B Receptor activity to Adenosine A2A Receptor activity at a ratio of about 2 to about 1. In embodiments, the method inhibits Adenosine A2B Receptor activity to Adenosine A2A Receptor activity at a ratio of about 3 to about 1. In embodiments, the method inhibits Adenosine A2B Receptor activity to Adenosine A2A Receptor activity at a ratio of about 4 to about 1. In embodiments, the method inhibits Adenosine A2B Receptor activity to Adenosine A2A Receptor activity at a ratio of about 5 to about 1. In embodiments, the method inhibits Adenosine A2B Receptor activity to Adenosine A2A Receptor activity at a ratio of about 6 to about 1. In embodiments, the method inhibits Adenosine A2B Receptor activity to Adenosine A2A Receptor activity at a ratio of about 7 to about 1. In embodiments, the method inhibits Adenosine A2B Receptor activity to Adenosine A2A Receptor activity at a ratio of about 8 to about 1. In embodiments, the method inhibits Adenosine A2B Receptor activity to Adenosine A2A Receptor activity at a ratio of about 9 to about 1. In embodiments, the method inhibits Adenosine A2B Receptor activity to Adenosine A2A Receptor activity at a ratio of about 10 to about 1. In embodiments, the method inhibits Adenosine A2B Receptor activity to Adenosine A2A Receptor activity at a ratio of about 20 to about 1. In embodiments, the method inhibits Adenosine A2B Receptor activity to Adenosine A2A Receptor activity at a ratio of about 25 to about 1. In embodiments, the method inhibits Adenosine A2B Receptor activity to Adenosine A2A Receptor activity at a ratio of about 30 to about 1. In embodiments, the method inhibits Adenosine A2B Receptor activity to Adenosine A2A Receptor activity at a ratio of about 40 to about 1. In embodiments, the method inhibits Adenosine A2B Receptor activity to Adenosine A2A Receptor activity at a ratio of about 50 to about 1. In embodiments, the method inhibits Adenosine A2B Receptor activity to Adenosine A2A Receptor activity at a ratio of about 100 to about 1. In embodiments, the method inhibits Adenosine A2B Receptor activity to Adenosine A2A Receptor activity at a ratio of about 200 to about 1. In embodiments, the method inhibits Adenosine A2B Receptor activity to Adenosine A2A Receptor activity at a ratio of about 300 to about 1. In embodiments, the method inhibits Adenosine A2B Receptor activity to Adenosine A2A Receptor activity at a ratio of about 400 to about 1. In embodiments, the method inhibits Adenosine A2B Receptor activity to Adenosine A2A Receptor activity at a ratio of about 500 to about 1. In embodiments, the method inhibits Adenosine A2B Receptor activity to Adenosine A2A Receptor activity at a ratio of about 600 to about 1. In embodiments, the method inhibits Adenosine A2B Receptor activity to Adenosine A2A Receptor activity at a ratio of about 700 to about 1. In embodiments, the method inhibits Adenosine A2B Receptor activity to Adenosine A2A Receptor activity at a ratio of about 800 to about 1. In embodiments, the method inhibits Adenosine A2B Receptor activity to Adenosine A2A Receptor activity at a ratio of about 900 to about 1. In embodiments, the method inhibits Adenosine A2B Receptor activity to Adenosine A2A Receptor activity at a ratio of about 1000 to about 1.

In embodiments, the method inhibits Adenosine A2B Receptor activity to Adenosine A2A Receptor activity at a ratio equal to or greater than 2 to 1. In embodiments, the method inhibits Adenosine A2B Receptor activity to Adenosine A2A Receptor activity at a ratio equal to or greater than 3 to 1. In embodiments, the method inhibits Adenosine A2B Receptor activity to Adenosine A2A Receptor activity at a ratio equal to or greater than 4 to 1. In embodiments, the method inhibits Adenosine A2B Receptor activity to Adenosine A2A Receptor activity at a ratio equal to or greater than 5 to 1. In embodiments, the method inhibits Adenosine A2B Receptor activity to Adenosine A2A Receptor activity at a ratio equal to or greater than 6 to 1. In embodiments, the method inhibits Adenosine A2B Receptor activity to Adenosine A2A Receptor activity at a ratio equal to or greater than 7 to 1. In embodiments, the method inhibits Adenosine A2B Receptor activity to Adenosine A2A Receptor activity at a ratio equal to or greater than 8 to 1. In embodiments, the method inhibits Adenosine A2B Receptor activity to Adenosine A2A Receptor activity at a ratio equal to or greater than 9 to 1. In embodiments, the method inhibits Adenosine A2B Receptor activity to Adenosine A2A Receptor activity at a ratio equal to or greater than 10 to 1. In embodiments, the method inhibits Adenosine A2B Receptor activity to Adenosine A2A Receptor activity at a ratio equal to or greater than 20 to 1. In embodiments, the method inhibits Adenosine A2B Receptor activity to Adenosine A2A Receptor activity at a ratio equal to or greater than 25 to 1. In embodiments, the method inhibits Adenosine A2B Receptor activity to Adenosine A2A Receptor activity at a ratio equal to or greater than 30 to 1. In embodiments, the method inhibits Adenosine A2B Receptor activity to Adenosine A2A Receptor activity at a ratio equal to or greater than 40 to 1. In embodiments, the method inhibits Adenosine A2B Receptor activity to Adenosine A2A Receptor activity at a ratio equal to or greater than 50 to 1. In embodiments, the method inhibits Adenosine A2B Receptor activity to Adenosine A2A Receptor activity at a ratio equal to or greater than 100 to 1. In embodiments, the method inhibits Adenosine A2B Receptor activity to Adenosine A2A Receptor activity at a ratio equal to or greater than 200 to 1. In embodiments, the method inhibits Adenosine A2B Receptor activity to Adenosine A2A Receptor activity at a ratio equal to or greater than 300 to 1. In embodiments, the method inhibits Adenosine A2B Receptor activity to Adenosine A2A Receptor activity at a ratio equal to or greater than 400 to 1. In embodiments, the method inhibits Adenosine A2B Receptor activity to Adenosine A2A Receptor activity at a ratio equal to or greater than 500 to 1. In embodiments, the method inhibits Adenosine A2B Receptor activity to Adenosine A2A Receptor activity at a ratio equal to or greater than 600 to 1. In embodiments, the method inhibits Adenosine A2B Receptor activity to Adenosine A2A Receptor activity at a ratio equal to or greater than 700 to 1. In embodiments, the method inhibits Adenosine A2B Receptor activity to Adenosine A2A Receptor activity at a ratio equal to or greater than 800 to 1. In embodiments, the method inhibits Adenosine A2B Receptor activity to Adenosine A2A Receptor activity at a ratio equal to or greater than 900 to 1. In embodiments, the method inhibits Adenosine A2B Receptor activity to Adenosine A2A Receptor activity at a ratio equal to or greater than 1000 to 1.

In another aspect is provided a method of inhibiting Adenosine A2B Receptor activity, said method comprising contacting the Adenosine A2B Receptor with a compound as described herein, including embodiments.

In an aspect is provided a method of treating cancer, said method comprising administering to a subject in need thereof an effective amount of a compound as described herein, including embodiments. In embodiments, the cancer is breast cancer, lung cancer, colon cancer, bladder cancer, kidney cancer, liver cancer, pancreatic cancer, melanoma, leukemia, lymphoma, or prostate cancer. In embodiments, the cancer is breast cancer. In embodiments, the cancer is lung cancer. In embodiments, the cancer is colon cancer. In embodiments, the cancer is bladder cancer. In embodiments, the cancer is kidney cancer. In embodiments, the cancer is liver cancer. In embodiments, the cancer is pancreatic cancer. In embodiments, the cancer is melanoma. In embodiments, the cancer is leukemia. In embodiments, the cancer is lymphoma. In embodiments, the cancer is prostate cancer.

In an aspect is provided a method of treating an inflammatory disease, said method comprising administering to a subject in need thereof an effective amount of a compound as described herein, including embodiments.

In an aspect is provided a method of treating a neurodegenerative disorder, said method comprising administering to a subject in need thereof an effective amount of a compound as described herein, including embodiments. In embodiments, the neurodegenerative disorder is Alzheimer's disease, Huntington's disease, Multiple sclerosis, Parkinson's disease, retinitis pigmentosa, amyotrophic lateral sclerosis, retinal degeneration, macular degeneration, Prion Disease, Creutzfeldt-Jakob Disease, or Kuru. In embodiments, the neurodegenerative disorder is Alzheimer's disease. In embodiments, the neurodegenerative disease is Huntington's disease. In embodiments, the neurodegenerative disorder is Multiple sclerosis. In embodiments, the neurodegenerative disorder is Parkinson's disease. In embodiments, the neurodegenerative disorder is Amyotrophic lateral sclerosis.

In an aspect is provided a method of treating a fibrotic disease, said method comprising administering to a subject in need thereof an effective amount of a compound as described herein, including embodiments. In embodiments, the fibrotic disease is pulmonary fibrosis. In embodiments, the fibrotic disease is idiopathic pulmonary fibrosis (IPF). In embodiments, the fibrotic disease is idiopathic pulmonary fibrosis (IPF), myocardial infarction, cardiac hypertrophy, heart failure, cirrhosis, acetominophen (Tylenol) liver toxicity, hepatitis C liver disease, hepatosteatosis (fatty liver disease), or hepatic fibrosis. In embodiments, the fibrotic disease is nonalcoholic steatohepatitis (NASH). In embodiments, the fibrotic disease is nonalcoholic fatty liver disease (NAFLD).

In an aspect is provided a method of treating a disease or disorder associated with Adenosine A2B Receptor activity and/or Adenosine A2A Receptor activity, said method comprising administering to a subject in need thereof an effective amount of a compound as described herein, including embodiments.

In embodiments, the disease or disorder is a cancer. In embodiments, the cancer is breast cancer, lung cancer, colon cancer, bladder cancer, kidney cancer, liver cancer, pancreatic cancer, melanoma, leukemia, lymphoma, or prostate cancer. In embodiments, the cancer is breast cancer. In embodiments, the cancer is lung cancer. In embodiments, the cancer is colon cancer. In embodiments, the cancer is bladder cancer. In embodiments, the cancer is kidney cancer. In embodiments, the cancer is liver cancer. In embodiments, the cancer is pancreatic cancer. In embodiments, the cancer is melanoma. In embodiments, the cancer is leukemia. In embodiments, the cancer is lymphoma. In embodiments, the cancer is prostate cancer.

In embodiments, the disease or disorder is a neurodegenerative disorder. In embodiments, the neurodegenerative disorder is Alexander's disease. In embodiments, the neurodegenerative disorder is Alper's disease. In embodiments, the neurodegenerative disorder is Alzheimer's disease. In embodiments, the neurodegenerative disorder is Amyotrophic lateral sclerosis. In embodiments, the neurodegenerative disorder is Ataxia telangiectasia. In embodiments, the neurodegenerative disorder is Batten disease (also known as Spielmeyer-Vogt-Sjogren-Batten disease). In embodiments, the neurodegenerative disorder is Bovine spongiform encephalopathy (BSE). In embodiments, the neurodegenerative disorder is Canavan disease. In embodiments, the neurodegenerative disorder is chronic fatigue syndrome. In embodiments, the neurodegenerative disorder is Cockayne syndrome. In embodiments, the neurodegenerative disorder is Corticobasal degeneration. In embodiments, the neurodegenerative disorder is Creutzfeldt-Jakob disease. In embodiments, the neurodegenerative disorder is frontotemporal dementia. In embodiments, the neurodegenerative disorder is Gerstmann-Sträussler-Scheinker syndrome. In embodiments, the neurodegenerative disorder is Huntington's disease. In embodiments, the neurodegenerative disorder is HIV-associated dementia. In embodiments, the neurodegenerative disorder is Kennedy's disease. In embodiments, the neurodegenerative disorder is Krabbe's disease. In embodiments, the neurodegenerative disorder is kuru. In embodiments, the neurodegenerative disorder is Lewy body dementia. In embodiments, the neurodegenerative disorder is Machado-Joseph disease (Spinocerebellar ataxia type 3). In embodiments, the neurodegenerative disorder is Multiple sclerosis. In embodiments, the neurodegenerative disorder is Multiple System Atrophy. In embodiments, the neurodegenerative disorder is myalgic encephalomyelitis. In embodiments, the neurodegenerative disorder is Narcolepsy. In embodiments, the neurodegenerative disorder is Neuroborreliosis. In embodiments, the neurodegenerative disorder is Parkinson's disease. In embodiments, the neurodegenerative disorder is Pelizaeus-Merzbacher Disease. In embodiments, the neurodegenerative disorder is Pick's disease. In embodiments, the neurodegenerative disorder is Primary lateral sclerosis. In embodiments, the neurodegenerative disorder is Prion diseases. In embodiments, the neurodegenerative disorder is Refsum's disease. In embodiments, the neurodegenerative disorder is Sandhoffs disease. In embodiments, the neurodegenerative disorder is Schilder's disease. In embodiments, the neurodegenerative disorder is Subacute combined degeneration of spinal cord secondary to Pernicious Anaemia. In embodiments, the neurodegenerative disorder is Schizophrenia. In embodiments, the neurodegenerative disorder is Spinocerebellar ataxia (multiple types with varying characteristics). In embodiments, the neurodegenerative disorder is Spinal muscular atrophy, Steele-Richardson-Olszewski disease. In embodiments, the neurodegenerative disorder is progressive supranuclear palsy. In embodiments, the neurodegenerative disorder is Tabes dorsalis.

In embodiments, the disease or disorder is a fibrotic disorder. In embodiments, the fibrotic disorder is rheumatoid arthritis. In embodiments, the fibrotic disorder is surgical adhesions. In embodiments, the fibrotic disorder is osteoarthritis. In embodiments, the fibrotic disorder is visible skin scars. In embodiments, the fibrotic disorder is a cardiovascular fibrotic disorder. In embodiments, the fibrotic disorder is a liver fibrotic disorder. In embodiments, the fibrotic disorder is a kidney fibrotic disorder. In embodiments, the fibrotic disorder is a lung fibrotic disorder. In embodiments, the fibrotic disorder is periodontal fibrotic disorder. In embodiments, the fibrotic disorder is diseases entailing excess collagen and/or elastin deposition. In embodiments, the fibrotic disorder is cutaneous keloid formation. In embodiments, the fibrotic disorder is progressive systemic sclerosis. In embodiments, the fibrotic disorder is liver cirrhosis. In embodiments, the fibrotic disorder is idiopathic and pharmacologically induced pulmonary fibrosis. In embodiments, the fibrotic disorder is chronic graft-versus-host disease. In embodiments, the fibrotic disorder is scleroderma (local and systemic). In embodiments, the fibrotic disorder is Peyronie's disease. In embodiments, the fibrotic disorder is pharmacologically induced fibrosis of the penis. In embodiments, the fibrotic disorder is post-cystoscopic urethral stenosis. In embodiments, the fibrotic disorder is post-surgical internal adhesions. In embodiments, the fibrotic disorder is myelofibrosis. In embodiments, the fibrotic disorder is idiopathic and pharmacologically induced retroperitoneal fibrosis.

In embodiments, the methods include administering a second agent (e.g. therapeutic agent). In embodiments, the method includes administering a second agent (e.g. therapeutic agent) in a therapeutically effective amount. In embodiments, the second agent is an agent for treating cancer. In embodiments, the second agent is an anti-cancer agent. In embodiments, the second agent is a chemotherapeutic. In embodiments, the second agent is an anti-inflammatory agent.

In an aspect is provided a method of inhibiting a Adenosine Receptor activity, the method including contacting the Adenosine Receptor with a compound as described herein, including embodiments.

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

### V. Embodiments

Embodiment P1. A compound having the formula: 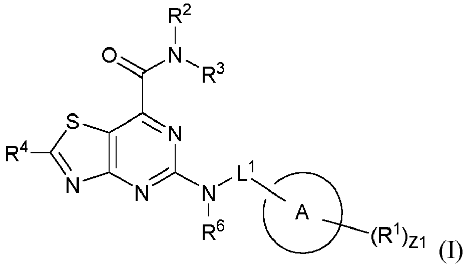
   Ring A is a cycloalkyl, heterocycloalkyl, aryl, or heteroaryl;
   L¹ is a bond, -SOₙ₅L^{1A}-, -SOᵥ₅NR⁵L^{1A}-, -NHC(O)NR⁵L^{1A}-, -NR⁵L^{1A}-, -C(O)L^{1A}-, -C(O)OL^{1A}- , -C(O)NR⁵L^{1A}-, -OL^{1A}-, -NR⁵SO₂L^{1A}-, -NR⁵C(O)L^{1A}-, -NR⁵C(O)OL^{1A}-, -NR⁵OL^{1A}-, -SL^{1A}-, substituted or unsubstituted alkylene, substituted or unsubstituted heteroalkylene, substituted or unsubstituted cycloalkylene, substituted or unsubstituted heterocycloalkylene, substituted or unsubstituted arylene, or substituted or unsubstituted heteroarylene;
   R¹ is halogen, -CX¹₃, -CHX¹₂, -CH₂X¹, -OCX¹₃, -OCH₂X¹, -OCHX¹₂, -N₃, -CN, -SOₙ₁R^{1D}, -SOᵥ₁NR^{1A}R^{1B}, -NHC(O)NR^{1A}R^{1B}, -N(O)ₘ₁, -NR^{1A}R^{1B}, -C(O)R^{1C}, -C(O)-OR^{1C}, -C(O)NR^{1A}R^{1B}, -OR^{1D}, -NR^{1A}SO₂R^{1D}, -NR^{1A}C(O)R^{1C}, -NR^{1A}C(O)OR^{1C}, -NR^{1A}OR^{1C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R² is hydrogen, -CX²₃, -CHX²₂, -CH₂X², -OCX²₃, -OCH₂X², -OCHX²₂, -C(O)R^{2C}, -C(O)OR^{2C}, -C(O)NR^{2A}R^{2B}, -OR^{2D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R³ is hydrogen, -CX³₃, -CHX³₂, -CH₂X³, -OCX³₃, -OCH₂X³, -OCHX³₂, -C(O)R^{3C}, -C(O)OR^{3C}, -C(O)NR^{3A}R^{3B}, -OR^{3D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R² and R³ together with the nitrogen attached thereto may be optionally joined to form a substituted or unsubstituted heterocycloalkyl, or substituted or unsubstituted heteroaryl;
   R⁴ is hydrogen, halogen, -CX⁴₃, -CHX⁴₂, -CH₂X⁴, -OCX⁴₃, -OCH₂X⁴, -OCHX⁴₂, -N₃, -CN, -SOₙ₄R^{4D}, -SOᵥ₄NR^{4A}R^{4B}, -NHC(O)NR^{4A}R^{4B}, -N(O)ₘ₄, -NR^{4A}R^{4B}, -C(O)R^{4C}, -C(O)-OR^{4C}, -C(O)NR^{4A}R^{4B}, -OR^{4D}, -NR^{4A}SO₂R^{4D}, -NR^{4A}C(O)R^{4C}, -NR^{4A}C(O)OR^{4C}, -NR^{4A}OR^{4C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R⁵ is hydrogen, -CX⁵₃, -CHX⁵₂, -CH₂X⁵, -OCX⁵₃, -OCH₂X⁵, -OCHX⁵₂, -C(O)R^{5C}, -C(O)OR^{5C}, -C(O)NR^{5A}R^{5B}, -OR^{5D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R⁶ is hydrogen, -CX⁶₃, -CHX⁶₂, -CH₂X⁶, -OCX⁶₃, -OCH₂X⁶, -OCHX⁶₂, -C(O)R^{6C}, -C(O)OR^{6C}, -C(O)NR^{6A}R^{6B}, -OR^{6D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   wherein R^{1A}, R^{1B}, R^{1C} , R^{1D}, R^{2A}, R^{2B}, R^{2C}, R^{2D}, R^{3A}, R^{3B}, R^{3C}, R^{3D}, R^{4A}, R^{4B}, R^{4C}, R^{4D} , R^{5A}, R^{5B}, R^{5C}, R^{5D}, R^{6A}, R^{6B}, R^{6C}, and R^{6D} are independently hydrogen, -CX₃, -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   L^{1A} is a bond, substituted or unsubstituted alkylene, substituted or unsubstituted heteroalkylene, substituted or unsubstituted cycloalkylene, substituted or unsubstituted heterocycloalkylene, substituted or unsubstituted arylene, or substituted or unsubstituted heteroarylene;
   z1 is an integer from 0-12;
   n1 and n4 are independently an integer from 0 to 4;
   m1, m4, v1, andv4 are independently an integer from 1 to 2; and
   X, X¹, X², X³, X⁴, X⁵ and X⁶ are independently -F, -Cl, -Br, or -I.
Embodiment P2. The compound of embodiment P1, wherein the Ring A is C₆-C₁₀ aryl or 5-10 membered heteroaryl.
Embodiment P3. The compound of embodiment P1, wherein the Ring A is pyridyl.
Embodiment P4. The compound of any one of embodiments P1 to P3, wherein L¹ is a bond, substituted or unsubstituted alkylene, or substituted or unsubstituted heteroalkylene.
Embodiment P5. The compound of any one of embodiments P1 to P4, wherein R¹ is halogen, -CX¹₃, -CHX¹₂, -CH₂X¹, -OCX¹₃, -OCH₂X¹, -OCHX¹₂, -CN, -SR^{1D}, -NHC(O)NR^{1A}R^{1B}, -NR^{1A}R^{1B}, -C(O)R^{1C}, -C(O)-OR^{1C}, -C(O)NR^{1A}R^{1B}, -OR^{1D}, -NR^{1A}C(O)R^{1C}, -NR^{1A}C(O)OR^{1C}, substituted or unsubstituted alkyl, or substituted or unsubstituted heteroalkyl.
Embodiment P6. The compound of any one of embodiments P1 to P5, wherein R⁶ is hydrogen, -CX⁶₃, -CHX⁶₆, -CH₆X⁶, -OCX⁶₃, -OCH₆X⁶, -OCHX⁶₆, -C(O)R^{6C}, -C(O)OR^{6C}, -C(O)NR^{6A}R^{6B}, -OR^{6D}, or substituted or unsubstituted alkyl.
Embodiment P7. The compound of any one of embodiments P1 to P6, wherein R⁴ is hydrogen, halogen, -CX⁴₃, -CHX⁴₂, -CH₂X⁴, -OCX⁴₃, -OCH₂X⁴, -OCHX⁴₂, -CN, -SR^{4D}, -NHC(O)NR^{4A}R^{4B}, -NR^{4A}R^{4B}, -C(O)R^{4C}, -C(O)-OR^{4C}, -C(O)NR^{4A}R^{4B}, -OR^{4D}, -NR^{4A}C(O)R^{4C}, -NR^{4A}C(O)OR^{4C}, -NR^{4A}OR^{4C}, substituted or unsubstituted alkyl, substituted or unsubstituted heteroalkyl, or substituted or unsubstituted cycloalkyl.
Embodiment P8. The compound of any one of embodiments P1 to P7, wherein the compound has a formula: 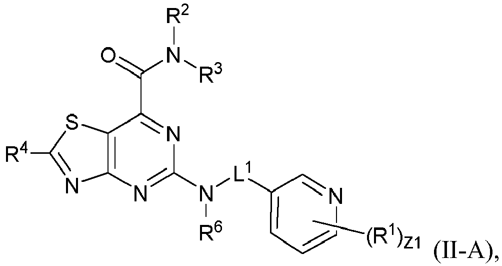 wherein z1 is an integer from 0 to 4.
Embodiment P9. The compound of any one of embodiments P1 to P7, wherein the compound has a formula: 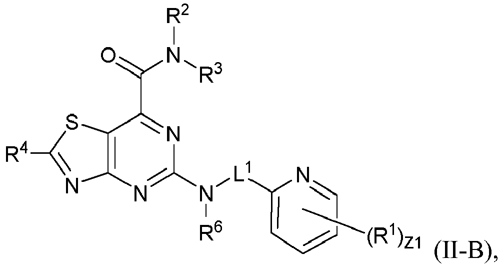 wherein z1 is an integer from 0 to 4.
Embodiment P10. The compound of any one of embodiments P1 to P7, wherein the compound has the formula: 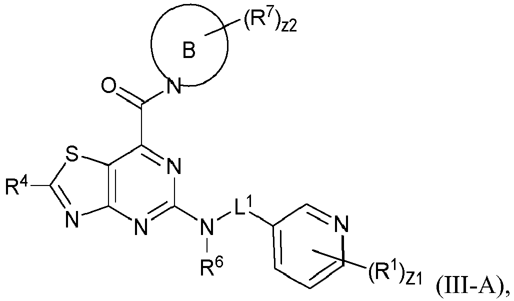 or 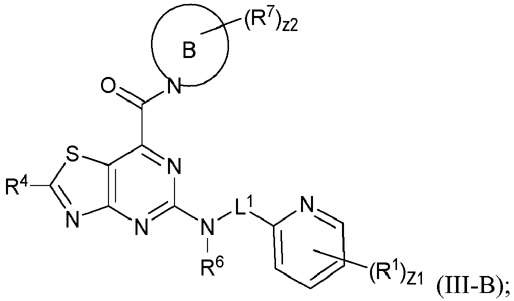 wherein:
   Ring B is a 4 to 7 membered heterocycloalkyl;
   R⁷ is independently halogen, -CX⁷₃, -CHX⁷₂, -CH₂X⁷, -OCX⁷₃, -OCH₂X⁷, -OCHX⁷₂, -N₃, -CN, -SOₙ₇R^{7D}, -SOᵥ₇NR^{7A}R^{7B}, -NR^{7A}C(O)NR^{7A}R^{7B}, -NR^{7A}S(O)NR^{7A}R^{7B}, -N(O)ₘ₇, -NR^{7A}R^{7B}, -C(O)R^{7C}, -C(O)-OR^{7C}, -C(O)NR^{7A}R^{7B}, -C(O)NR^{7A}OR^{7B}, -OR^{7D}, -NR^{7A}SO₂R^{7D}, -NR^{7A}C(O)R^{7C}, -NR^{7A}C(O)OR^{7C}, -NR^{7A}OR^{7C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{7A}, R^{7B}, R^{7C}, and R^{7D} are independently hydrogen, -CN, -CX₃, -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   z1 is an integer from 0 to 4;
   z2 is an integer from 0 to 12;
   n7 is independently an integer from 0 to 4;
   m7 and v7 are independently an integer from 1 to 2; and
   X⁷ is independently -F, -Cl, -Br, or -I.
Embodiment P11. The compound of embodiment P10, wherein the compound has a formula 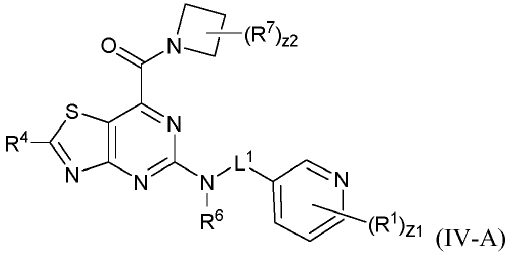 or 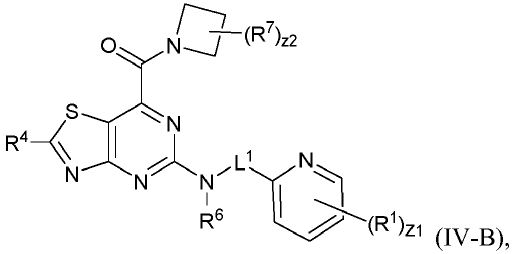 wherein z2 is an integer from 0 to 6.
Embodiment P12. The compound of embodiment P11, wherein:
   z2 is 0 or 1;
   R⁷ is independently -NR^{7A}R^{7B}, -C(O)NR^{7A}R^{7B}, -C(O)NR^{7A}OR^{7B}, -NR^{7A}C(O)R^{7C}, -NR^{7A}C(O)NR^{7A}R^{7B}, -OR^{7D}, -C(O)R^{7C}, or substituted or unsubstituted 2 to 6 membered heteroalkyl;
   R^{7A} and R^{7B} are independently hydrogen, or unsubstituted C₁-C₄ alkyl;
   R^{7C} is independently substituted or unsubstituted C₁-C₁₂ alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{7D} is independently hydrogen, or unsubstituted C₁-C₄ alkyl.
Embodiment P13. The compound of embodiment P12, wherein R⁷ is independently -NR^{7A}C(O)R^{7C}, or -C(O)NR^{7A}R^{7B}-.
Embodiment P14. The compound of embodiment P10, wherein R^{7A}, R^{7B} and R^{7C} are independently hydrogen, substituted or unsubstituted C₁-C₄ alkyl, substituted or unsubstituted 3 to 8-membered cycloalkyl, substituted or unsubstituted 5 to 6 membered heterocycloalkyl, substituted or unsubstituted phenyl, or substituted or unsubstituted 5 to 6 membered heteroaryl.
Embodiment P15. The compound of embodiment P14, wherein R⁷ is: 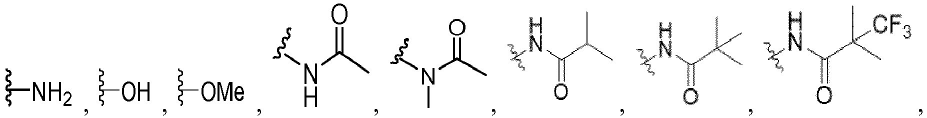 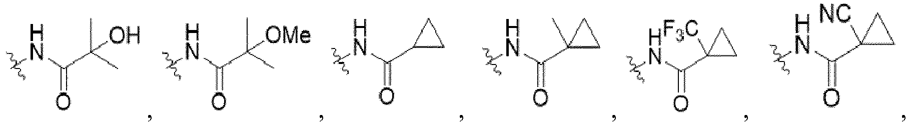 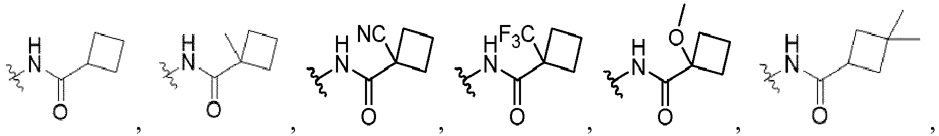 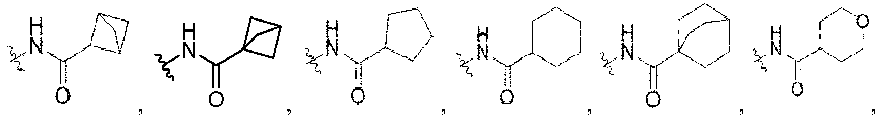 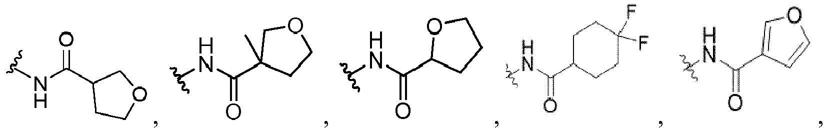 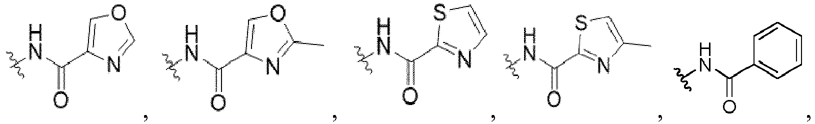 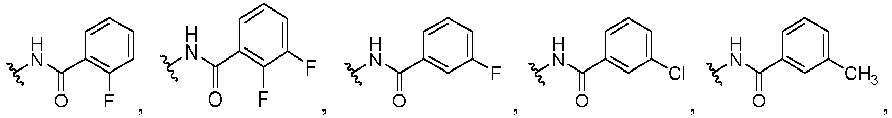 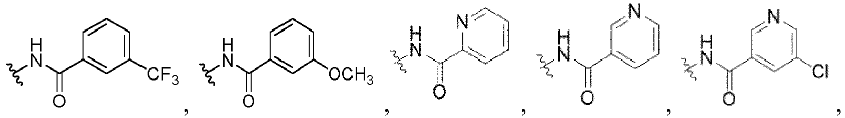 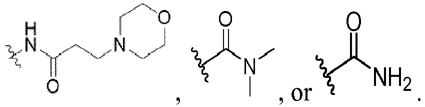
Embodiment P16. The compound of embodiment P10, wherein R⁷ is independently -NHC(O)NHR^{7B}.
Embodiment P17. The compound of embodiment P16, wherein R^{7B} is independently substituted or unsubstituted C₁-C₄ alkyl, substituted or unsubstituted 3 to 8-membered cycloalkyl, substituted or unsubstituted 5 to 6 membered heterocycloalkyl, substituted or unsubstituted phenyl, or substituted or unsubstituted 5 to 6 membered heteroaryl.
Embodiment P18. The compound of embodiment P17, wherein R⁷ is: 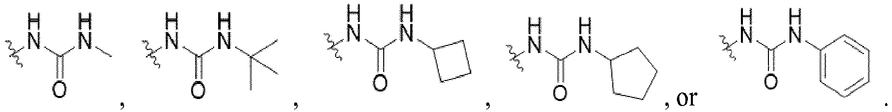
Embodiment P19. The compound of embodiment P12, wherein R⁷ is independently substituted or unsubstituted C₁-C₄ alkyl or substituted or unsubstituted 2 to 6 membered heteroalkyl.
Embodiment P20. The compound of embodiment P19, wherein R⁷ is independently 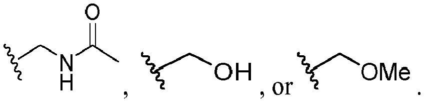
Embodiment P21. The compound of embodiment P10, wherein the compound has a formula:. 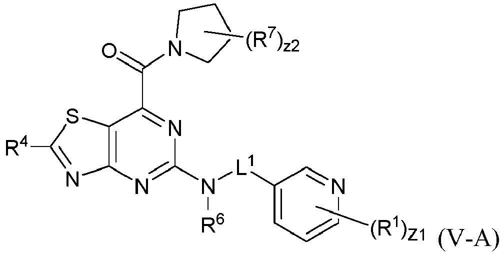 or 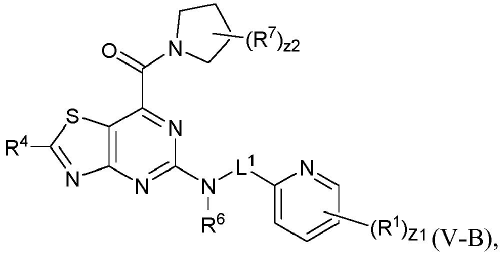 wherein z2 is an integer 0 to 8.
Embodiment P22. The compound of embodiment P21, wherein:
   z2 is 0 or 1;
   R⁷ is independently -NR^{7A}R^{7B}, - C(O)NR^{7A}R^{7B}, -C(O)NR^{7A}OR^{7B}, -NR^{7A}C(O)R^{7C}, -OR^{7D}, -C(O)R^{7C}, or substituted or unsubstituted 2 to 6 membered heteroalkyl;
   R^{7A} and R^{7B} are independently hydrogen, or unsubstituted C₁-C₄ alkyl;
   R^{7C} is independently unsubstituted C₁-C₆ alkyl; and
   R^{7D} is independently hydrogen, or unsubstituted C₁-C₄ alkyl.
Embodiment P23. The compound of embodiment P10, wherein the compound has the formula: 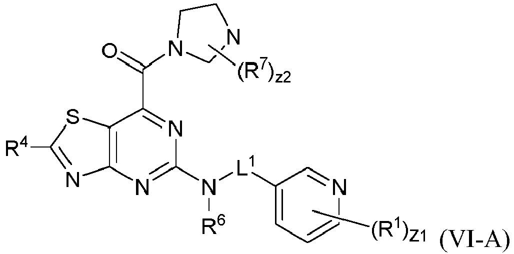 or 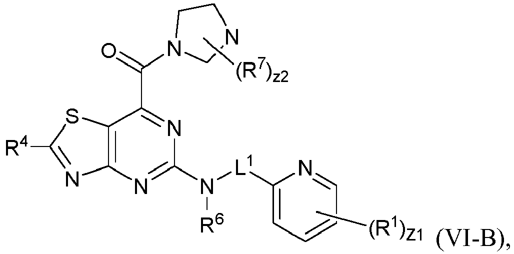 wherein z2 is an integer from 0 to 7.
Embodiment P24. The compound of embodiment P23, wherein:
   z2 is 0 or 1;
   R⁷ is independently -C(O)R^{7C}, or - C(O)NR^{7A}R^{7B};
   R^{7A} and R^{7B} are independently hydrogen, or unsubstituted C₁-C₄ alkyl; and
   R^{7C} is independently unsubstituted C₁-C₆ alkyl.
Embodiment P25. The compound of any one of embodiments P1 to P7, wherein the compound has a formula: 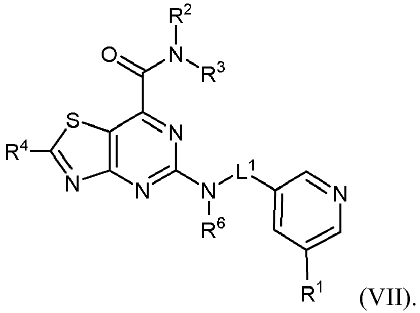
Embodiment P26. The compound of embodiment P25, wherein R¹ is halogen.
Embodiment P27. The compound of any of embodiments P1-P7, wherein the compound has a formula: 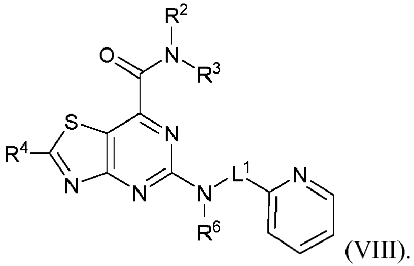
Embodiment P28. The compound of any one of embodiments P1 to P9 and P25-P27, wherein R² and R³ are each independently hydrogen, substituted or unsubstituted C₁-C₁₂ alkyl, or substituted or unsubstituted 2 to 12 membered heteroalkyl.
Embodiment P29. The compound of any one of embodiments P1 to P9 and P25-P27, wherein the compound has a formula: 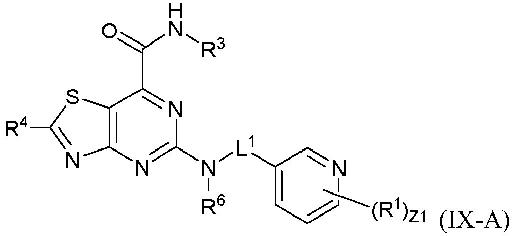 or 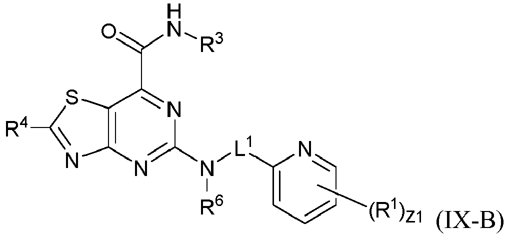
Embodiment P30. The compound of embodiment P29, wherein R³ is substituted or unsubstituted C₁-C₁₀ alkyl, or substituted or unsubstituted 2 to 10 membered heteroalkyl.
Embodiment P31. The compound of embodiment P29, wherein R³ is substituted or unsubstituted C₁-C₄ alkyl, or substituted or unsubstituted 2 to 4 membered heteroalkyl.
Embodiment P32. The compound of embodiment P30, wherein R³ is: 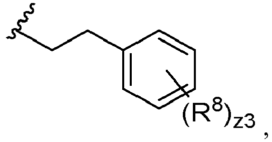 wherein
   R⁸ is independently halogen, -CX⁸₃, -CHX⁸₂, -CH₂X⁸, -OCX⁸₃, - OCH₂X⁸, -OCHX⁸₂, -N₃, -CN,
      -SOₙ₈R^{8D}, -SOᵥ₈NR^{8A}R^{8B}, -NHC(O)NR^{8A}R^{8B}, -N(O)ₘ₈, -NR^{8A}R^{8B}, -C(O)R^{8C}, -C(O)-OR^{8C}, -C(O)NR^{8A}R^{8B}, -OR^{8D}, -NR^{1A}SO₂R^{1D}, -NR^{8A}C(O)R^{8C}, -NR^{8A}C(O)OR^{8C}, -NR^{8A}OR^{8C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
      (IIIB);
   R^{8A}, R^{8B}, R^{8C}, and R^{8D} are independently hydrogen, -CX₃, -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   z3 is an integer from 0 to 5;
   n8 is independently an integer from 0 to 4;
   m8 and v8 are independently an integer from 1 to 2; and
   X⁸ is independently -F, -Cl, -Br, or -I.
Embodiment P33. The compound of embodiment P32, wherein R³ is: 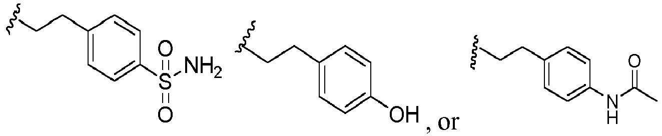
Embodiment P34. The compound of embodiment P30, wherein R³ is: 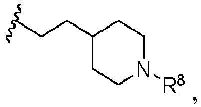 wherein:
   R⁸ is hydrogen, halogen, -CX⁸₃, -CHX⁸₂, -CH₂X⁸, -OCX⁸₃, -OCH₂X⁸, -OCHX⁸₂, -CN, , -C(O)R^{8C}, -C(O)-OR^{8C}, -C(O)NR^{8A} R^{8B}, -OR^{5D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{8A}, R^{8B}, R^{8C}, and R^{8D} are independently hydrogen, -CX₃, -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   n8 is independently an integer from 0 to 4;
   m8 and v8 are independently an integer from 1 to 2; and
   X⁸ is independently -F, -Cl, -Br, or -I.
Embodiment P35. The compound of embodiment P34, wherein R³ is: 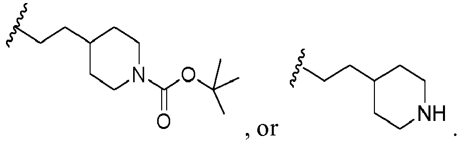
Embodiment P36. The compound of embodiment P30, wherein R³ is: 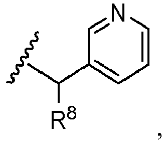 wherein:
   R⁸ is hydrogen, halogen, -CX⁸₃, -CHX⁸₂, -CH₂X⁸, -OCX⁸₃, -OCH₂X⁸, -OCHX⁸₂, -N₃, -CN, -SOₙ₈R^{8D}, -SOᵥ₈NR^{1A}R^{1B}, -NHC(O)NR^{8A}R^{8B}, -N(O)ₘ₈, -NR^{8A}R^{8B}, -C(O)R^{8C}, -C(O)-OR^{8C}, -C(O)NR^{8A}R^{8B}, -OR^{8D}, -NR^{1A}SO₂R^{1D}, -NR^{8A}C(O)R^{8C}, -NR^{8A}C(O)OR^{8C}, -NR^{8A}OR^{8C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
      (IIIB);
   R^{8A}, R^{8B}, R^{8C}, and R^{8D} are independently hydrogen, -CX₃, -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   n8 is independently an integer from 0 to 4;
   m8 and v8 are independently an integer from 1 to 2; and
   X⁸ is independently -F, -Cl, -Br, or -I.
Embodiment P37. The compound of embodiment P36, wherein R³ is: 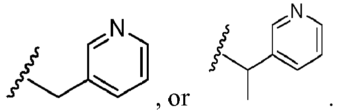
Embodiment P38. The compound of embodiment P30, wherein R³ is: 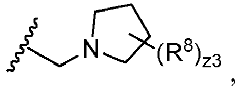 wherein:
   R⁸ is halogen, -CX⁸₃, -CHX⁸₂, -CH₂X⁸, -OCX⁸₃, -OCH₂X⁸, -OCHX⁸₂, -N₃, -CN, -SOₙ₈R^{8D}, -SOᵥ₈NR^{1A}R^{1B}, -NHC(O)NR^{8A}R^{8B}, -N(O)ₘ₈, -NR^{8A}R^{8B}, -C(O)R^{8C}, -C(O)-OR^{8C}, -C(O)NR^{8A} R^{8B}, -OR^{8D}, -NR^{8A}SO₂R^{8D}, -NR^{8A}C(O)R^{8C}, -NR^{8A}C(O)OR^{8C}, -NR^{8A}OR^{8C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
      (IIIB);
   R^{8A}, R^{8B}, R^{8C}, and R^{8D} are independently hydrogen, -CX₃, -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   z3 is an integer from 0 to 8;
   n8 is independently an integer from 0 to 4;
   m8 and v8 are independently an integer from 1 to 2; and
   X⁸ is independently -F, -Cl, -Br, or -I.
Embodiment P39. The compound of embodiment P38, wherein R³ is: 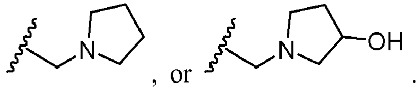
Embodiment P40. The compound of embodiment P30, wherein R³ is: 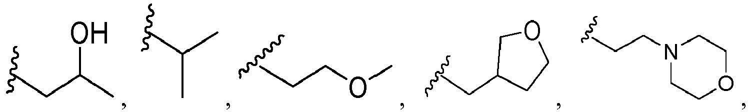 or 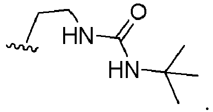
Embodiment P41. The compound of embodiment P30, wherein R³ is: 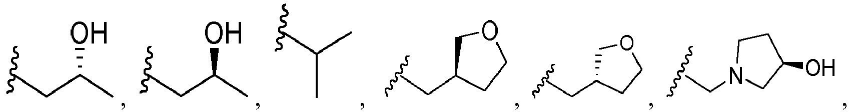 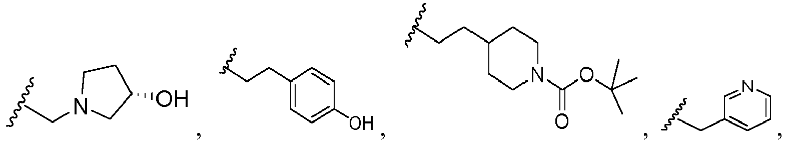 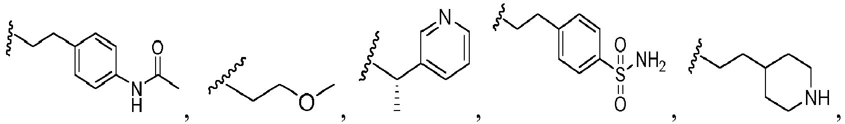 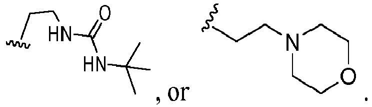
Embodiment P42. The compound of any one of embodiments P1-P41, wherein L¹ is substituted or unsubstituted C₁-C₄ alkylene.
Embodiment P43. The compound of embodiment P42, wherein L¹ is 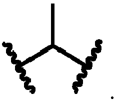
Embodiment P44. The compound of any one of embodiments P1-P43, wherein R⁶ is hydrogen.
Embodiment P45. The compound of any one of embodiments P1-P44, wherein R⁴ is hydrogen, substituted or unsubstituted C₁-C₈ alkyl, or substituted or unsubstituted 2 to 6 membered heteroalkyl.
Embodiment P46. The compound of embodiment P45, wherein R⁴ is substituted or unsubstituted C₁-C₄ alkyl, or substituted or unsubstituted 2 to 4 membered heteroalkyl.
Embodiment P47. The compound of embodiment P46, wherein R⁴ is unsubstituted C₁-C₄ alkyl.
Embodiment P48. The compound of embodiment P47, wherein R⁴ is unsubstituted methyl.
Embodiment P49. The compound of any one of embodiments P1-P44, wherein R⁴ is -NR^{4A}R^{4B}, and each R^{4A} and R^{4B} is independently hydrogen, or substituted or unsubstituted C₁-C₄ alkyl.
Embodiment P50. The compound of embodiment P49, wherein R⁴ is -NH₂.
Embodiment P51. The compound of embodiment P1, wherein the compound is: 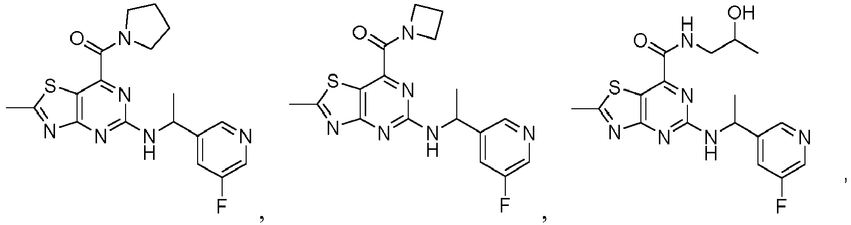 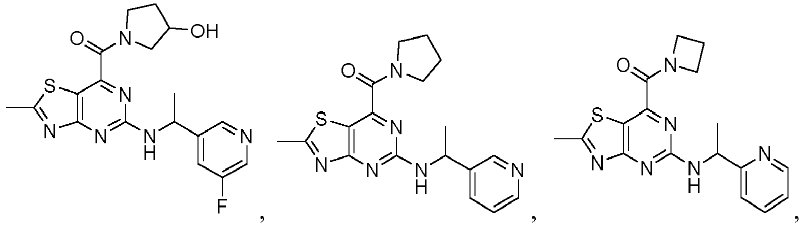 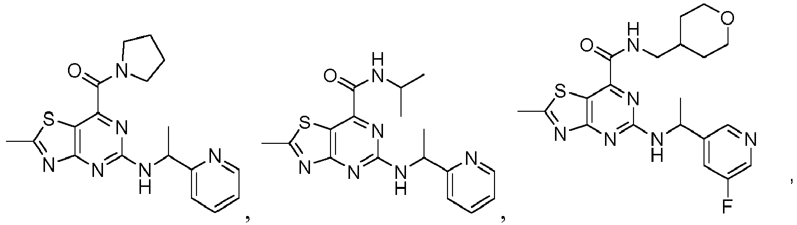 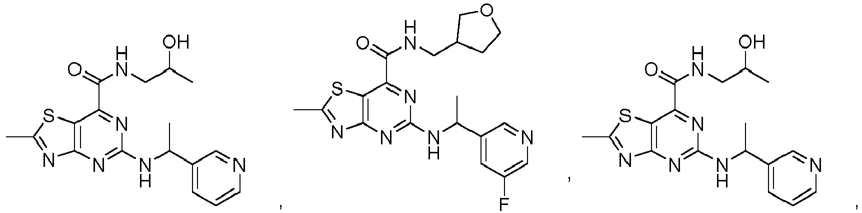 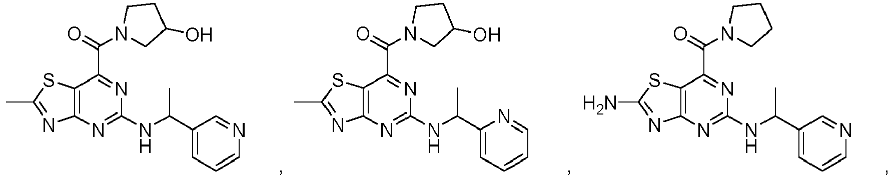 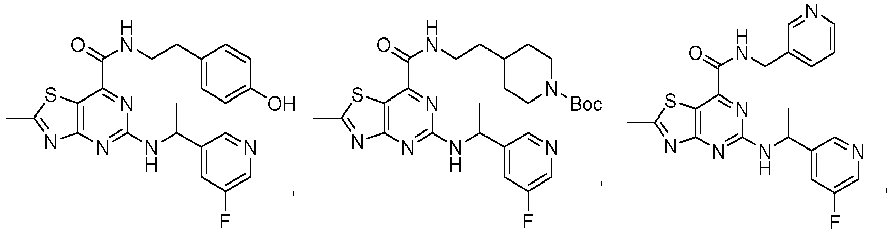 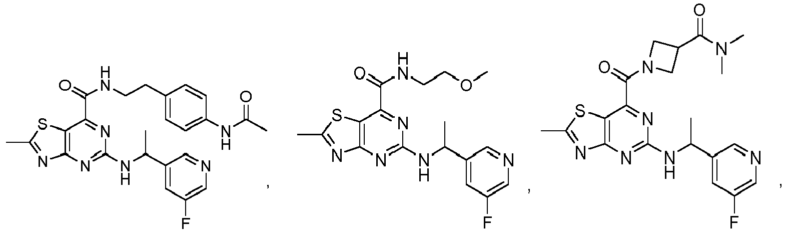 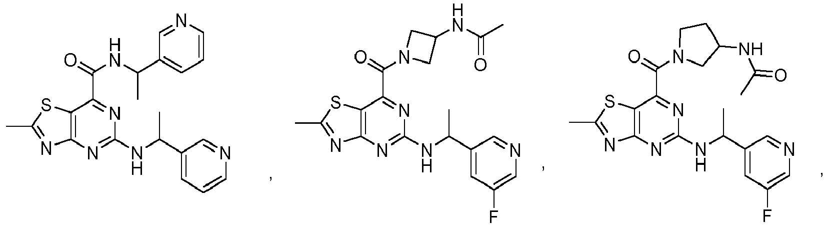 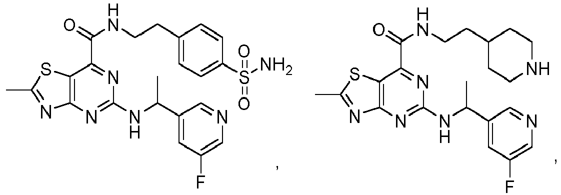 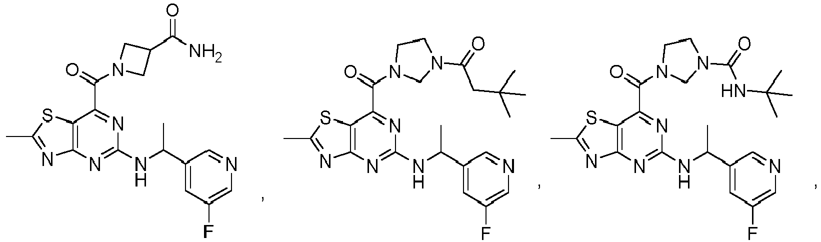 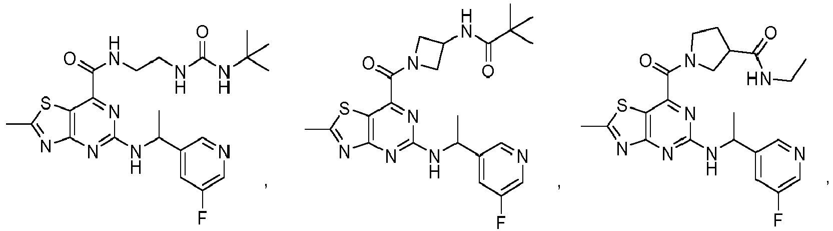 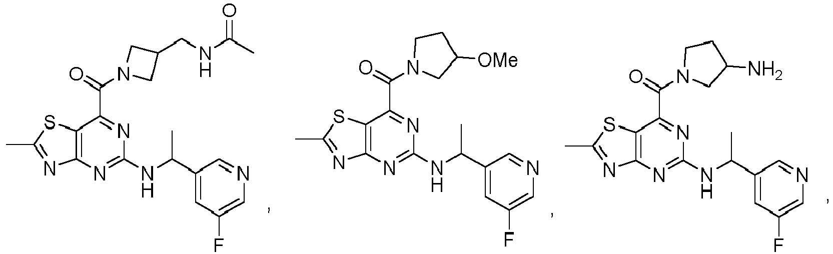 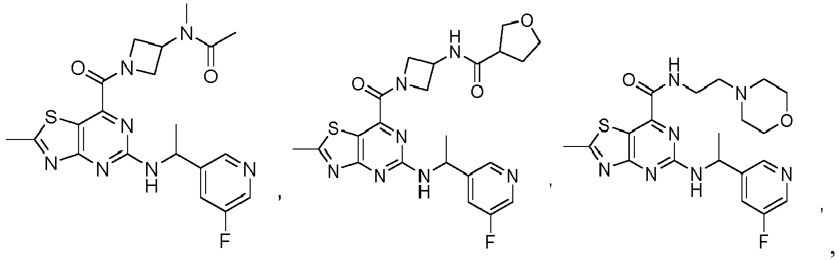 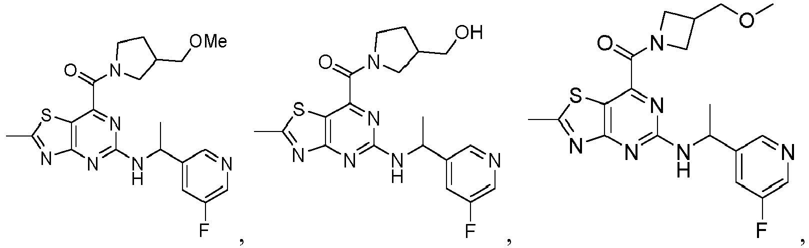 or 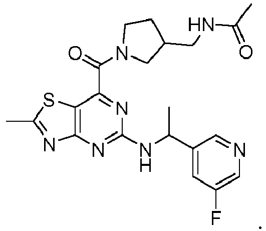
Embodiment P52. The compound of embodiment P1, wherein the compound is: 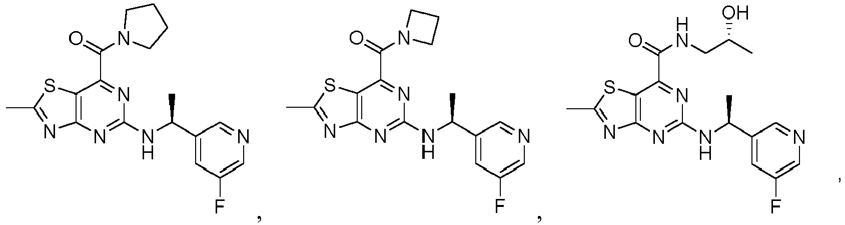 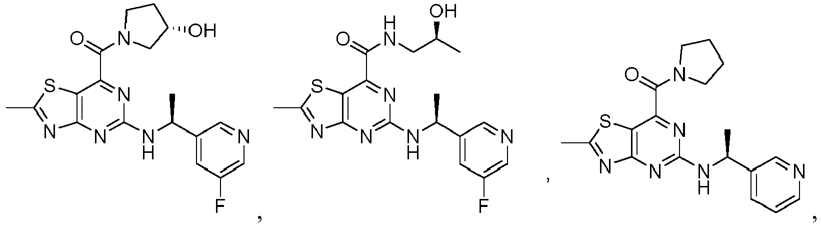 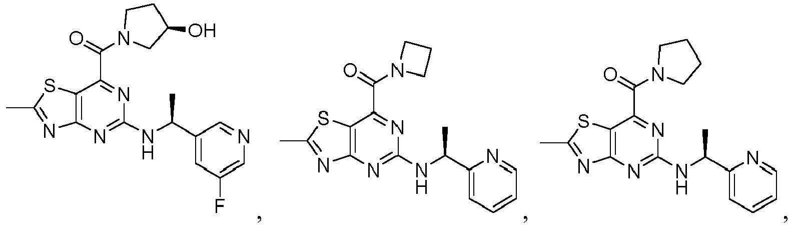 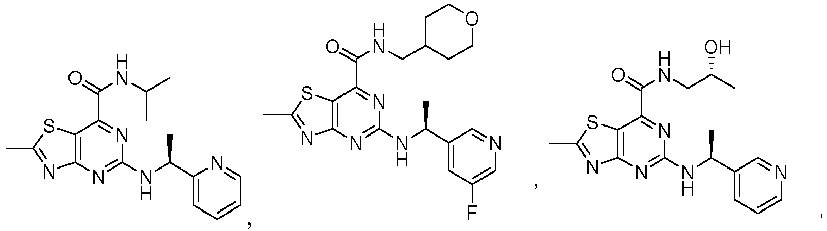 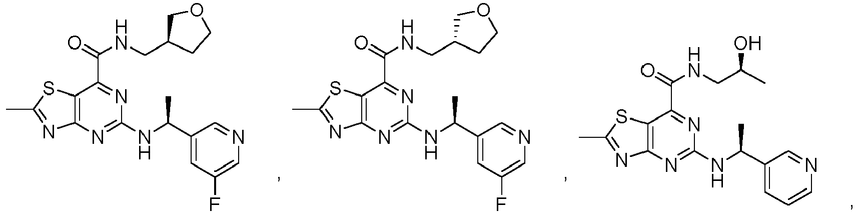 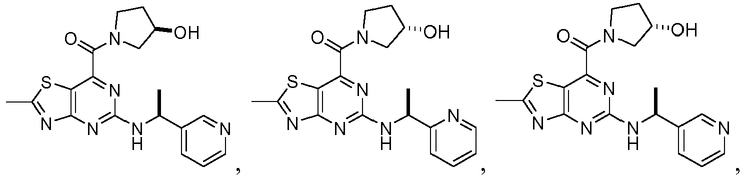 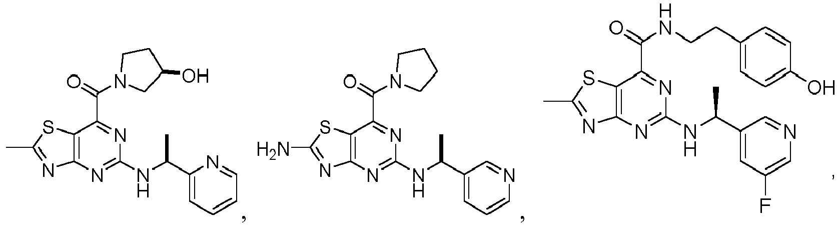 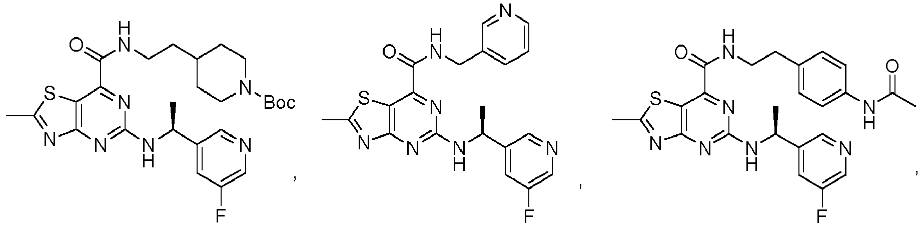 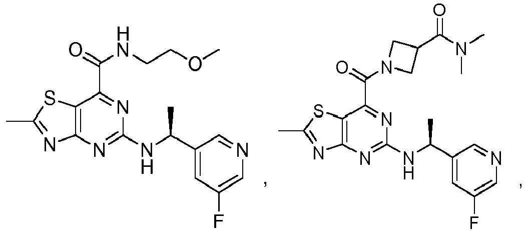 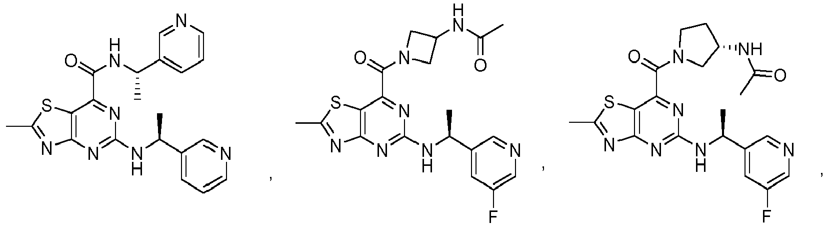 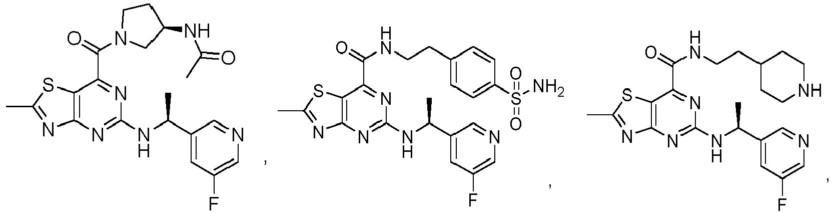 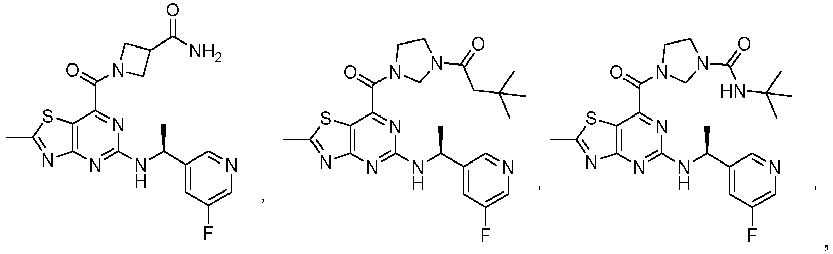 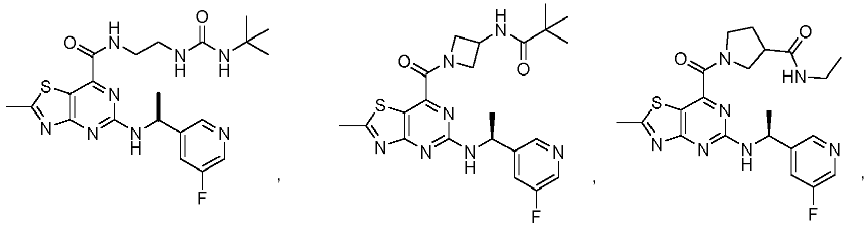 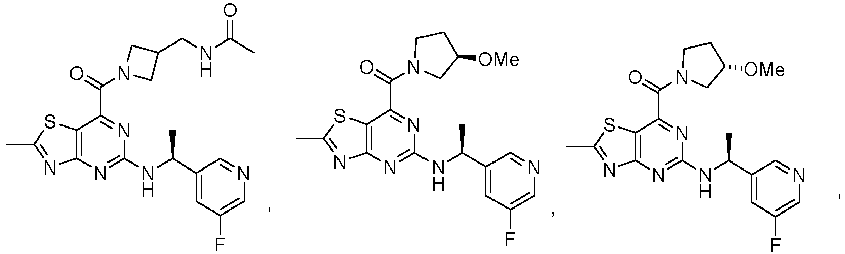 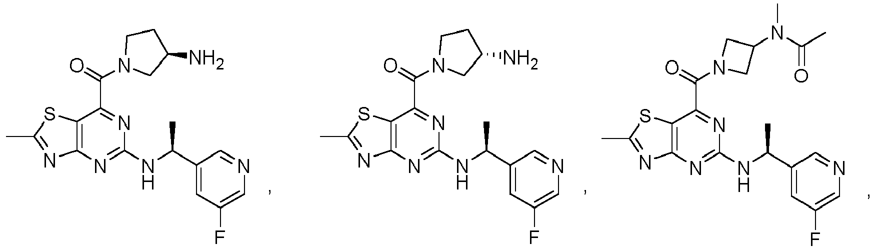 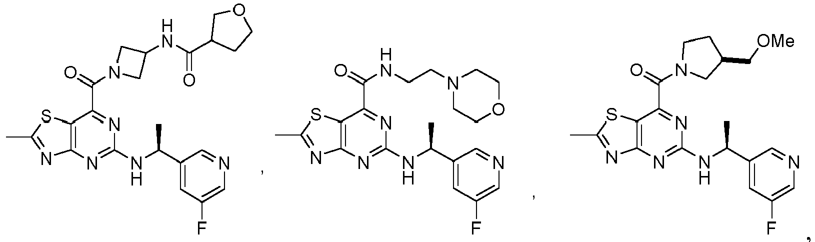 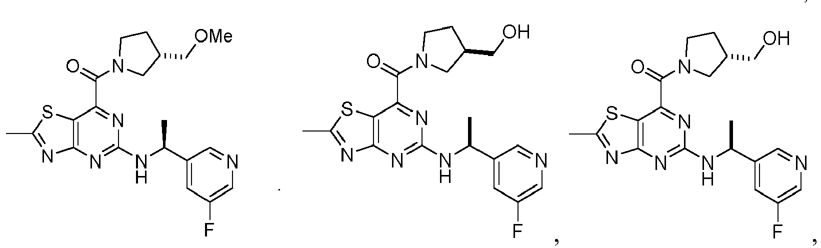 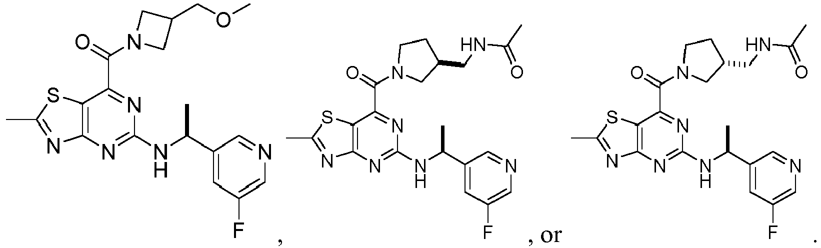
Embodiment P53. A pharmaceutical composition comprising the compound of any one of embodiments P1 to P52, and a pharmaceutically acceptable excipient.
Embodiment P54. A method of inhibiting Adenosine A2B Receptor activity and Adenosine A2A Receptor activity, said method comprising contacting the Adenosine A2B Receptor and/or Adenosine A2A Receptor with a compound of any one of embodiments P1 to P52.
Embodiment P55. A method of inhibiting Adenosine A2B Receptor activity, said method comprising contacting the Adenosine A2B Receptor with a compound of any one of embodiments P1 to P52.
Embodiment P56. A method of treating cancer, said method comprising administering to a subject in need thereof an effective amount of a compound of any one of embodiments P1 to P52.
Embodiment P57. The method of embodiment P56, wherein the cancer is breast cancer, lung cancer, colon cancer, bladder cancer, kidney cancer, liver cancer, pancreatic cancer, melanoma, leukemia, lymphoma, or prostate cancer.
Embodiment P58. A method of treating a disease or disorder associated with Adenosine A2B Receptor activity and/or Adenosine A2A Receptor activity, said method comprising administering to a subject in need thereof an effective amount of a compound of any one of embodiments P1 to P52.
Embodiment P59. The method of embodiment P58, wherein the disease or disorder is a cancer.
Embodiment P60. The method of embodiment P59, wherein the cancer is breast cancer, lung cancer, colon cancer, bladder cancer, kidney cancer, liver cancer, pancreatic cancer, melanoma, leukemia, lymphoma, or prostate cancer.
Embodiment P61. The method of embodiment P58, wherein the disease or disorder is a neurodegenerative disorder.
Embodiment P62. The method of embodiment P57, wherein the disease or disorder is a fibrotic disorder.

### VI. Additional embodiments

Embodiment 1. A compound having the formula: 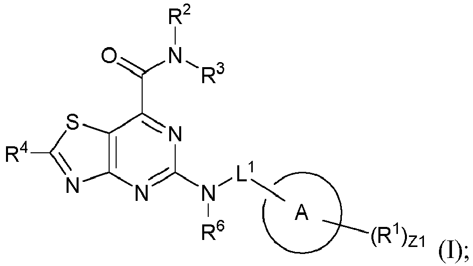
   Ring A is a heteroaryl, cycloalkyl, heterocycloalkyl, or aryl;
   L¹ is an unsubstituted or substituted alkylene, a bond, -SOₙ₅L^{1A}-,
      -SOᵥ₅NR⁵L^{1A}-, -NHC(O)NR⁵L^{1A}-, -NR⁵L^{1A}-, -C(O)L^{1A}-, -C(O)OL^{1A}-, -C(O)NR⁵L^{1A}-, -OL^{1A}-, -NR⁵SO₂L^{1A}- , -NR⁵C(O)L^{1A}-, -NR⁵C(O)OL^{1A}-, -NR⁵OL^{1A}-, -SL^{1A}-, substituted or unsubstituted heteroalkylene, substituted or unsubstituted cycloalkylene, substituted or unsubstituted heterocycloalkylene, substituted or unsubstituted arylene, or substituted or unsubstituted heteroarylene;
   R¹ is halogen, -CX¹₃, -CHX¹₂, -CH₂X¹, -OCX¹₃, -OCH₂X¹, -OCHX¹₂, -N₃,
      -CN, -SOₙ₁R^{1D}, -SOᵥ₁NR^{1A}R^{1B}, -NHC(O)NR^{1A}R^{1B}, -N(O)ₘ₁, -NR^{1A}R^{1B}, -C(O)R^{1C}, -C(O)-OR^{1C}, -C(O)NR^{1A}R^{1B}, -OR^{1D}, -NR^{1A}SO₂R^{1D}, -NR^{1A}C(O)R^{1C}, -NR^{1A}C(O)OR^{1C}, -NR^{1A}OR^{1C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R² is hydrogen, -CX²₃, -CHX²₂, -CH₂X², -OCX²₃, -OCH₂X², -OCHX²₂,
      -C(O)R^{2C}, -C(O)OR^{2C}, -C(O)NR^{2A}R^{2B}, -OR^{2D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R³ is a substituted or unsubstituted alkyl, hydrogen, -CX³₃, -CHX³₂, -CH₂X³,
      -OCX³₃, -OCH₂X³, -OCHX³₂, -C(O)R^{3C}, -C(O)OR^{3C}, -C(O)NR^{3A}R^{3B}, -OR^{3D}, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R² and R³ together with the nitrogen attached thereto may be optionally joined to form a substituted or unsubstituted heterocycloalkyl, or substituted or unsubstituted heteroaryl;
   R⁴ is an unsubstituted or substituted alkyl, hydrogen, halogen, -CX⁴₃, -CHX⁴₂,-CH₂X⁴, -OCX⁴₃, -OCH₂X⁴, -OCHX⁴₂, -N₃, -CN, -SOₙ₄R^{4D}, -SOᵥ₄NR^{4A}R^{4B}, -NHC(O)NR^{4A}R^{4B}, -N(O)ₘ₄, -NR^{4A}R^{4B}, -C(O)R^{4C}, -C(O)-OR^{4C}, -C(O)NR^{4A}R^{4B}, -OR^{4D}, -NR^{4A}SO₂R^{4D}, -NR^{4A}C(O)R^{4C}, -NR^{4A}C(O)OR^{4C}, -NR^{4A}OR^{4C}, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R⁵ is hydrogen, -CX⁵₃, -CHX⁵₂, -CH₂X⁵, -OCX⁵₃, -OCH₂X⁵, -OCHX⁵₂,
      -C(O)R^{5C}, -C(O)OR^{5C}, -C(O)NR^{5A}R^{5B}, -OR^{5D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R⁶ is hydrogen, -CX⁶₃, -CHX⁶₂, -CH₂X⁶, -OCX⁶₃, -OCH₂X⁶, -OCHX⁶₂,
      -C(O)R^{6C}, -C(O)OR^{6C}, -C(O)NR^{6A}R^{6B}, -OR^{6D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   wherein R^{1A}, R^{1B}, R^{1C}, R^{1D}, R^{2A}, R^{2B}, R^{2C}, R^{2D}, R^{3A}, R^{3B}, R^{3C}, R^{3D}, R^{4A}, R^{4B}, R^{4C}, R^{4D}, R^{5A}, R^{5B}, R^{5C}, R^{5D}, R^{6A}, R^{6B}, R^{6C}, and R^{6D} are independently hydrogen, -CX₃,
      -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   L^{1A} is a bond, substituted or unsubstituted alkylene, substituted or unsubstituted heteroalkylene, substituted or unsubstituted cycloalkylene, substituted or unsubstituted heterocycloalkylene, substituted or unsubstituted arylene, or substituted or unsubstituted heteroarylene;
   z1 is an integer from 0 to 12;
   n1 and n4 are independently an integer from 0 to 4;
   m1, m4, v1, and v4 are independently an integer from 1 to 2; and
   X, X¹, X², X³, X⁴, X⁵ and X⁶ are independently -F, -Cl, -Br, or -I.
Embodiment 2. The compound of embodiment 1, wherein the Ring A is 5 to 10 membered heteroaryl or C₆-C₁₀ aryl.
Embodiment 3. The compound of embodiment 1, wherein the Ring A is pyridyl.
Embodiment 4. The compound of any one of embodiments 1 to 3, wherein L¹ is an unsubstituted or substituted alkylene, a bond, or substituted or unsubstituted heteroalkylene.
Embodiment 5. The compound of any one of embodiments 1 to 4, wherein R¹ is halogen, -CX¹₃, -CHX¹₂, -CH₂X¹, -OCX¹₃, -OCH₂X¹, -OCHX¹₂, -CN, -SR^{1D}, -NHC(O)NR^{1A}R^{1B}, -NR^{1A}R^{1B}, -C(O)R^{1C}, -C(O)-OR^{1C}, -C(O)NR^{1A}R^{1B}, -OR^{1D}, -NR^{1A}C(O)R^{1 C} ,
   -NR^{1A}C(O)OR^{1C}, substituted or unsubstituted alkyl, or substituted or unsubstituted heteroalkyl.
Embodiment 6. The compound of any one of embodiments 1 to 5, wherein R⁶ is hydrogen, -CX⁶₃, -CHX⁶₆, -CH₆X⁶, -OCX⁶₃, -OCH₆X⁶, -OCHX⁶₆, -C(O)R^{6C}, -C(O)OR^{6C}, -C(O)NR^{6A}R^{6B}, -OR^{6D}, or substituted or unsubstituted alkyl.
Embodiment 7. The compound of any one of embodiments 1 to 6, wherein R⁴ is unsubstituted or substituted alkyl, hydrogen, halogen, -CX⁴₃, -CHX⁴₂, -CH₂X⁴, -OCX⁴₃, -OCH₂X⁴, -OCHX⁴₂, -CN, -SR^{4D}, -NHC(O)NR^{4A}R^{4B}, -NR^{4A}R^{4B}, -C(O)R^{4C}, -C(O)-OR^{4C}, -C(O)NR^{4A}R^{4B}, -OR^{4D}, -NR^{4A}C(O)R^{4C}, -NR^{4A}C(O)OR^{4C}, -NR^{4A}OR^{4C}, substituted or unsubstituted heteroalkyl, or substituted or unsubstituted cycloalkyl.
Embodiment 8. The compound of any one of embodiments 1 to 7, wherein the compound has the formula: 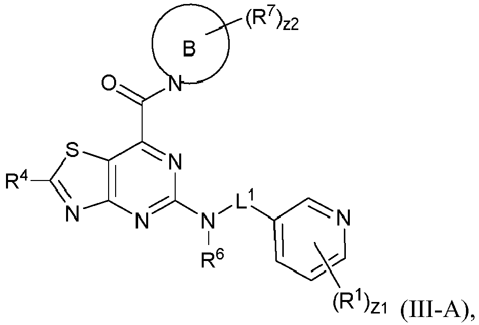 or 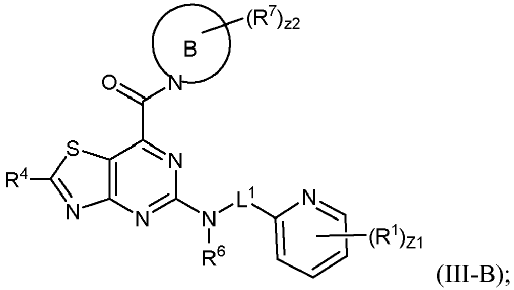 wherein
   Ring B is a 4 to 7 membered heterocycloalkyl;
   R⁷ is independently halogen, -CX⁷₃, -CHX⁷₂, -CH₂X⁷, -OCX⁷₃, -OCH₂X⁷,
      -OCHX⁷₂, -N₃, -CN, -SOₙ₇R^{7D}, -SOᵥ₇NR^{7A}R^{7B}, -NR^{7A}C(O)NR^{7A}R^{7B}, -NR^{7A}S(O)NR^{7A}R^{7B}, -N(O)m7, -NR^{7A}R^{7B}, -C(O)R^{7C}, -C(O)-OR^{7C}, -C(O)NR^{7A}R^{7B}, -C(O)NR^{7A}OR^{7B}, -OR^{7D}, -NR^{7A}SO₂R^{7D}, -NR^{7A}C(O)R^{7C}, -NR^{7A}C(O)OR^{7C}, -NR^{7A}OR^{7C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{7A}, R^{7B}, R^{7C}, and R^{7D} are independently hydrogen, -CN, -CX₃, -CHX₂,
      -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   z1 is an integer from 0 to 4;
   z2 is an integer from 0 to 12;
   n7 is independently an integer from 0 to 4;
   m7 and v7 are independently an integer from 1 to 2; and
   X⁷ is independently -F, -Cl, -Br, or -I.
Embodiment 9. The compound of any one of embodiments 1 to 7, wherein Ring A is 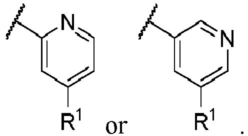
Embodiment 10. The compound of embodiment 8, wherein the compound has a formula 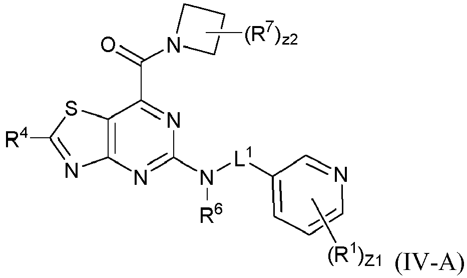 or 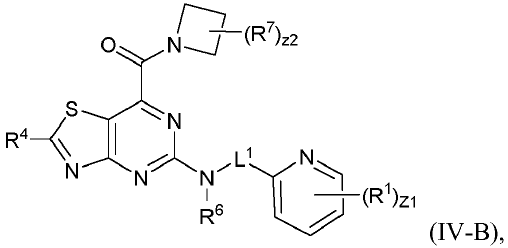 wherein z2 is an integer from 0 to 6.
Embodiment 11. The compound of embodiment 10, wherein:
   z2 is 0 or 1;
   R⁷ is independently -OR^{7D}, -NR^{7A}R^{7B}, -C(O)NR^{7A}R^{7B}, -C(O)NR^{7A}OR^{7B}, -NR^{7A}C(O)R^{7C}, - NR^{7A}C(O)NR^{7A}R^{7B}, -C(O)R^{7C}, or substituted or unsubstituted 2 to 6 membered heteroalkyl;
   R^{7A} and R^{7B} are independently hydrogen, or unsubstituted C₁-C₄ alkyl;
   R^{7C} is independently substituted or unsubstituted C₁-C₁₂ alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl; and
   R^{7D} is independently hydrogen, or unsubstituted C₁-C₄ alkyl.
Embodiment 12. The compound of embodiment 11, wherein R⁷ is independently -NR^{7A}C(O)R^{7C}, or -C(O)NR^{7A}R^{7B}-.
Embodiment 13. The compound of embodiment 8, wherein R^{7A}, R^{7B}, and R^{7C} are independently hydrogen, substituted or unsubstituted C₁-C₄ alkyl, substituted or unsubstituted 3 to 8-membered cycloalkyl, substituted or unsubstituted 5 to 6 membered heterocycloalkyl, substituted or unsubstituted phenyl, or substituted or unsubstituted 5 to 6 membered heteroaryl.
Embodiment 14. The compound of embodiment 13, wherein R⁷ is independently: 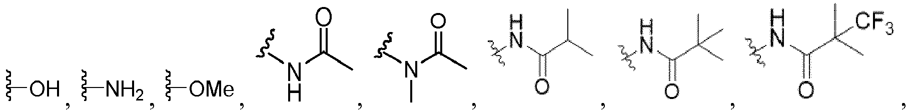 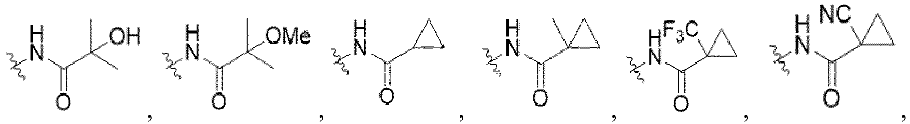 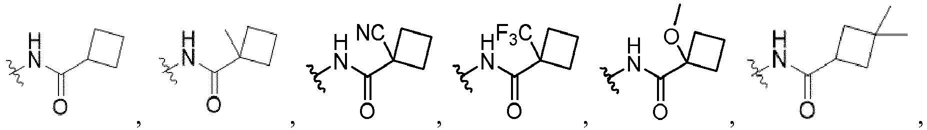 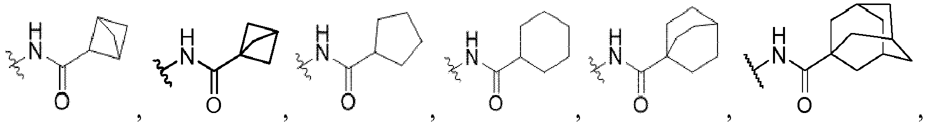 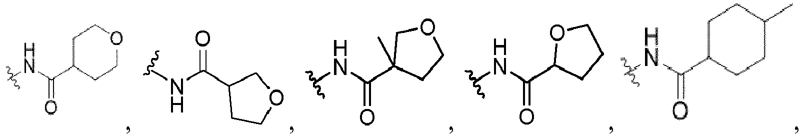 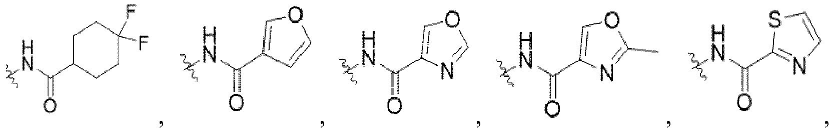 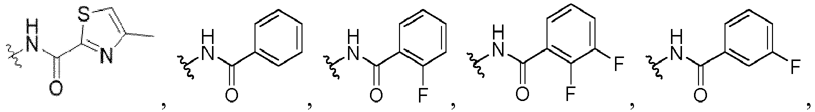 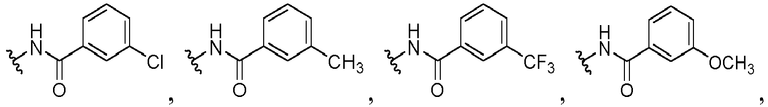 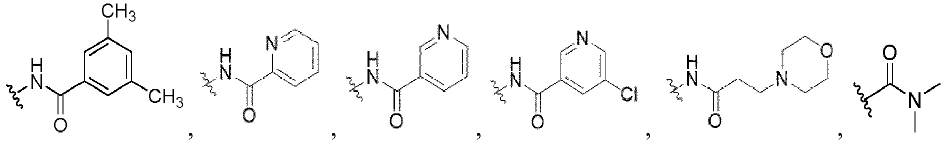 , or 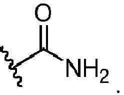
Embodiment 15. The compound of embodiment 14, wherein R⁷ is independently -OH.
Embodiment 16. The compound of embodiment 8, wherein R⁷ is independently -NHC(O)NHR^{7B}.
Embodiment 17. The compound of embodiment 16, wherein R^{7B} is independently substituted or unsubstituted C₁-C₄ alkyl, substituted or unsubstituted 3 to 8-membered cycloalkyl, substituted or unsubstituted 5 to 6 membered heterocycloalkyl, substituted or unsubstituted phenyl, or substituted or unsubstituted 5 to 6 membered heteroaryl.
Embodiment 18. The compound of embodiment 17, wherein R⁷ is independently: 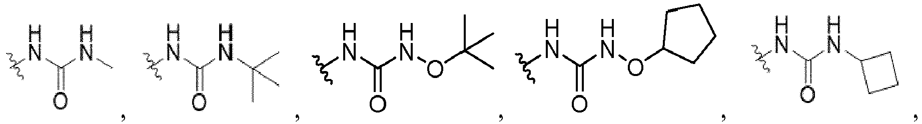 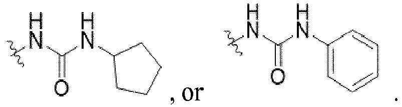
Embodiment 19. The compound of embodiment 11, wherein R⁷ is independently substituted or unsubstituted C₁-C₄ alkyl or substituted or unsubstituted 2 to 6 membered heteroalkyl.
Embodiment 20. The compound of embodiment 19, wherein R⁷ is independently 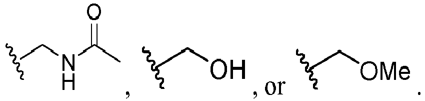
Embodiment 21. The compound of embodiment 8, wherein the compound has a formula: 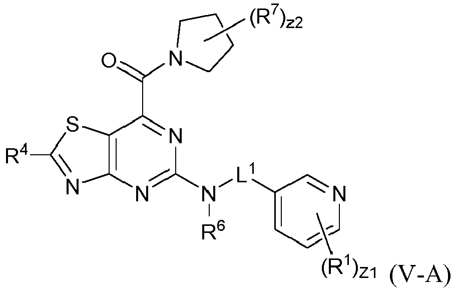 or 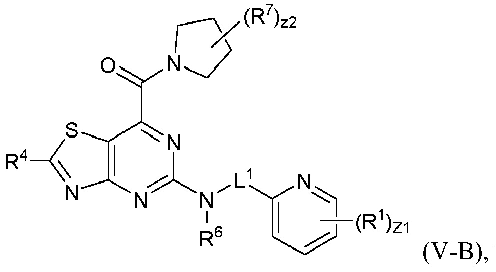 wherein z2 is an integer 0 to 8.
Embodiment 22. The compound of embodiment 21, wherein
   z2 is 0 or 1;
   R⁷ is independently -NR^{7A}R^{7B}, -C(O)NR^{7A}R^{7B}, -C(O)NR^{7A}OR^{7B},
      -NR^{7A}C(O)R^{7C}, -OR^{7D}, -C(O)R^{7C}, or substituted or unsubstituted 2 to 6 membered heteroalkyl;
   R^{7A} and R^{7B} are independently hydrogen, or unsubstituted C₁-C₄ alkyl;
   R^{7C} is independently unsubstituted C₁-C₆ alkyl; and
   R^{7D} is independently hydrogen, or unsubstituted C₁-C₄ alkyl.
Embodiment 23. The compound of embodiment 21, wherein z2 is 1 and R⁷ is independently -OH.
Embodiment 24. The compound of embodiment 21, wherein z1 is 0 or 1, and R¹ is independently a halogen.
Embodiment 25. The compound of embodiment 8, wherein the compound has the formula: 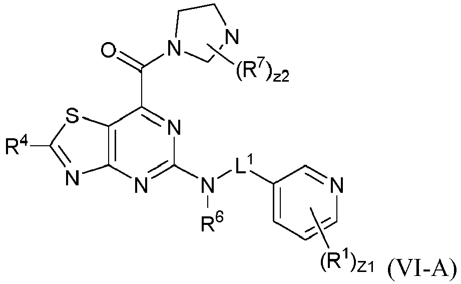 or 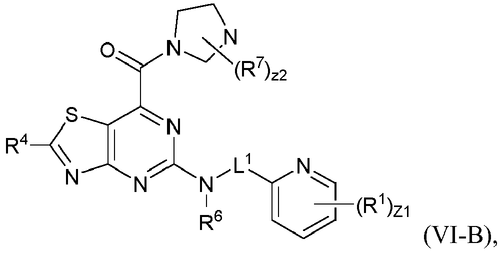 wherein z2 is an integer from 0 to 7.
Embodiment 26. The compound of embodiment 25, wherein:
   z2 is 0 or 1;
   R⁷ is independently -C(O)R^{7C}, or -C(O)NR^{7A}R^{7B};
   R^{7A} and R^{7B} are independently hydrogen, or unsubstituted C₁-C₄ alkyl; and
   R^{7C} is independently unsubstituted C₁-C₆ alkyl.
Embodiment 27. The compound of any one of embodiments 1 to 7, wherein the compound has a formula: 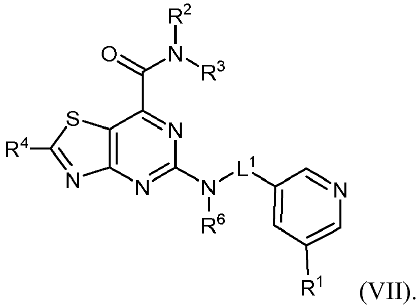
Embodiment 28. The compound of embodiment 27, wherein R¹ is halogen.
Embodiment 29. The compound of any one of embodiments 1 to 7, wherein the compound has the formula: 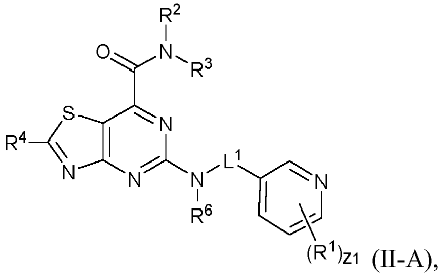 wherein z1 is an integer from 0 to 4.
Embodiment 30. The compound of any one of embodiments 1 to 7, wherein the compound has the formula: 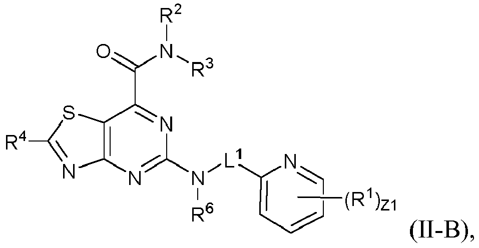 wherein z1 is an integer from 0 to 4.
Embodiment 31. The compound of any one of embodiments 1 to 7, wherein the compound has a formula: 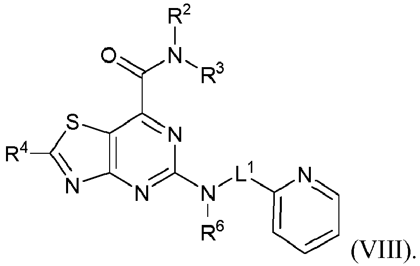
Embodiment 32. The compound of any one of embodiments 27 to 31, wherein R² and R³ together with the nitrogen attached thereto are joined to form 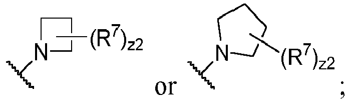 wherein
   R⁷ is independently halogen, -CX⁷₃, -CHX⁷₂, -CH₂X⁷, -OCX⁷₃, -OCH₂X⁷,
      -OCHX⁷₂, -N₃, -CN, -SOₙ₇R^{7D}, -SOᵥ₇NR^{7A}R^{7B}, -NR^{7A}C(O)NR^{7A}R^{7B}, -NR^{7A}S(O)NR^{7A}R^{7B}, -N(O)ₘ₇, -NR^{7A}R^{7B}, -C(O)R^{7C}, -C(O)-OR^{7C}, -C(O)NR^{7A}R^{7B}, -C(O)NR^{7A}OR^{7B}, -OR^{7D}, -NR^{7A}SO₂R^{7D}, -NR^{7A}C(O)R^{7C}, -NR^{7A}C(O)OR^{7C}, -NR^{7A}OR^{7C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{7A}, R^{7B}, R^{7C}, and R^{7D} are independently hydrogen, -CN, -CX₃, -CHX₂,
      -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   z2 is an integer from 0 to 1;
   n7 is independently an integer from 0 to 4;
   m7 and v7 are independently an integer from 1 to 2; and
   X⁷ is independently -F, -Cl, -Br, or -I.
Embodiment 33. The compound of embodiment 32, where z2 is 0.
Embodiment 34. The compound of embodiment 32, wherein z2 is 1.
Embodiment 35. The compound of embodiment 34, wherein R⁷ is 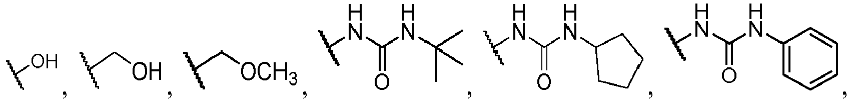 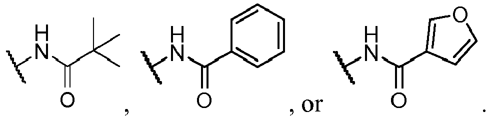
Embodiment 36. The compound of any one of embodiments 1 to 7 and 27 to 31, wherein R² and R³ are each independently hydrogen, substituted or unsubstituted C₁-C₁₂ alkyl, or substituted or unsubstituted 2 to 12 membered heteroalkyl.
Embodiment 37. The compound of any one of embodiments 1 to 7 and 27 to 31, wherein the compound has a formula: 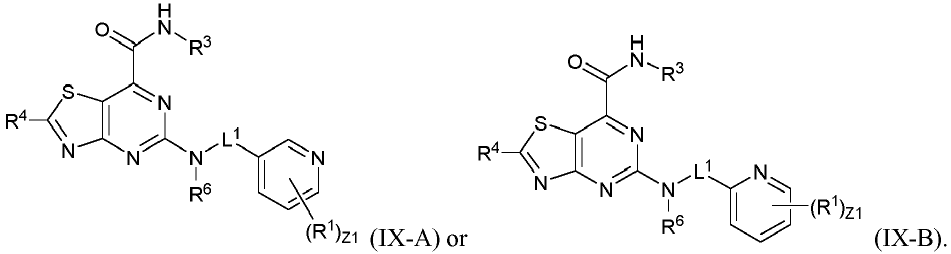
Embodiment 38. The compound of embodiment 37, wherein R³ is substituted or unsubstituted C₁-C₁₀ alkyl, or substituted or unsubstituted 2 to 10 membered heteroalkyl.
Embodiment 39. The compound of embodiment 37, wherein R³ is substituted or unsubstituted C₁-C₄ alkyl, or substituted or unsubstituted 2 to 4 membered heteroalkyl.
Embodiment 40. The compound of embodiment 38, wherein R³ is: 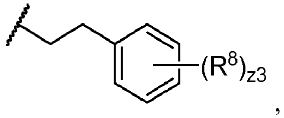 wherein
   R⁸ is independently -OR^{8D}, halogen, -CX⁸₃, -CHX⁸₂, -CH₂X⁸, -OCX⁸₃,
      -OCH₂X⁸, -OCHX⁸₂, -N₃, -CN, -SOₙ₈R^{8D}, -SOᵥ₈NR^{8A}R^{8B}, -NHC(O)NR^{8A}R^{8B}, -N(O)ₘ₈, -NR^{8A}R^{8B}, -C(O)R^{8C}, -C(O)-OR^{8C}, -C(O)NR^{8A}R^{8B}, -NR^{8A}SO₂R^{8D}, -NR^{8A}C(O)R^{8C}, -NR^{8A}C(O)OR^{8C}, -NR^{8A}OR^{8C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{8A}, R^{8B}, R^{8C}, and R^{8D} are independently hydrogen, -CX₃, -CHX₂, -CH₂X,
      -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   z3 is an integer from 0 to 5;
   n8 is independently an integer from 0 to 4;
   m8 and v8 are independently an integer from 1 to 2; and
   X⁸ is independently -F, -Cl, -Br, or -I.
Embodiment 41. The compound of embodiment 40, wherein R³ is: 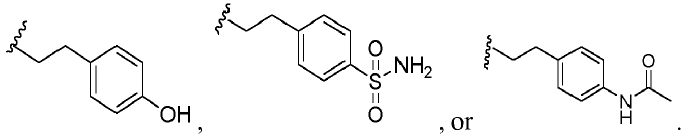
Embodiment 42. The compound of embodiment 38, wherein R³ is: 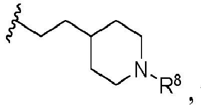 wherein:
   R⁸ is hydrogen, halogen, -CX⁸₃, -CHX⁸₂, -CH₂X⁸, -OCX⁸₃, -OCH₂X⁸,
      -OCHX⁸₂, -CN, -C(O)R^{8C}, -C(O)-OR^{8C}, -C(O)NR^{8A}R^{8B}, -OR^{8D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{8A}, R^{8B}, R^{8C}, and R^{8D} are independently hydrogen, -CX₃, -CHX₂, -CH₂X,
      -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   n8 is independently an integer from 0 to 4;
   m8 and v8 are independently an integer from 1 to 2; and
   X⁸ is independently -F, -Cl, -Br, or -I.
Embodiment 43. The compound of embodiment 42, wherein R³ is: 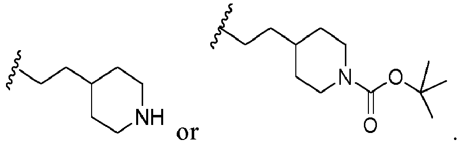
Embodiment 44. The compound of embodiment 38, wherein R³ is: 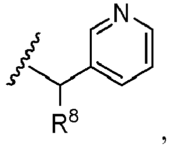 wherein
   R⁸ is hydrogen, unsubstituted or substituted alkyl, halogen, -CX⁸₃, -CHX⁸₂,
      -CH₂X⁸, -OCX⁸₃, -OCH₂X⁸, -OCHX⁸₂, -N₃, -CN, -SOₙ₈R^{8D}, -SOᵥ₈NR^{8A}R^{8B}, -NHC(O)NR^{8A}R^{8B}, -N(O)ₘ₈, -NR^{8A}R^{8B}, -C(O)R^{8C}, -C(O)-OR^{8C}, -C(O)NR^{8A}R^{8B}, -OR^{8D}, -NR^{8A}SO₂R^{8D}, -NR^{8A}C(O)R^{8C}, -NR^{8A}C(O)OR^{8C}, -NR^{8A}OR^{8C}, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{8A}, R^{8B}, R^{8C}, and R^{8D} are independently hydrogen, -CX₃, -CHX₂, -CH₂X,
      -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   n8 is independently an integer from 0 to 4;
   m8 and v8 are independently an integer from 1 to 2; and
   X⁸ is independently -F, -Cl, -Br, or -I.
Embodiment 45. The compound of embodiment 44, wherein R³ is: 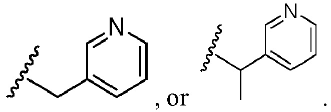
Embodiment 46. The compound of embodiment 38, wherein R³ is: 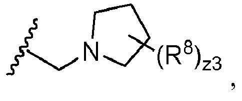 wherein:
   R⁸ is -OR^{8D}, halogen, -CX⁸₃, -CHX⁸₂, -CH₂X⁸, -OCX⁸₃, -OCH₂X⁸, -OCHX⁸₂,
      -N₃, -CN, -SOₙ₈R^{8D}, -SOᵥ₈NR^{8A}R^{8B}, -NHC(O)NR^{8A}R^{8B}, -N(O)ₘ₈, -NR^{8A} R^{8B}, -C(O)R^{8C}, -C(O)-OR^{8C}, -C(O)NR^{8A}R^{8B}, -NR^{8A}SO₂R^{8D}, -NR^{8A}C(O)R^{8C}, -NR^{8A}C(O)OR^{8C}, -NR^{8A}OR^{8C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{8A}, R^{8B}, R^{8C}, and R^{8D} are independently hydrogen, -CX₃, -CHX₂, -CH₂X,
      -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   z3 is an integer from 0 to 8;
   n8 is independently an integer from 0 to 4;
   m8 and v8 are independently an integer from 1 to 2; and
   X⁸ is independently -F, -Cl, -Br, or -I.
Embodiment 47. The compound of embodiment 46, wherein R³ is: 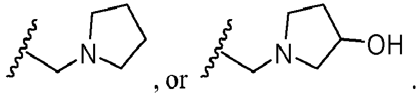
Embodiment 48. The compound of embodiment 38, wherein R³ is: 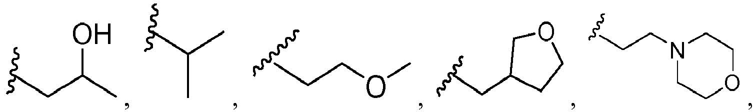 or 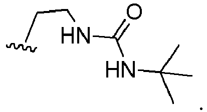
Embodiment 49. The compound of embodiment 38, wherein R³ is: 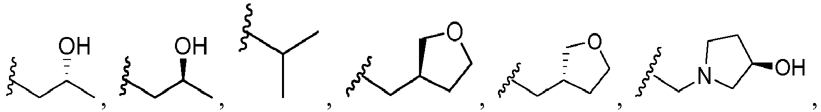 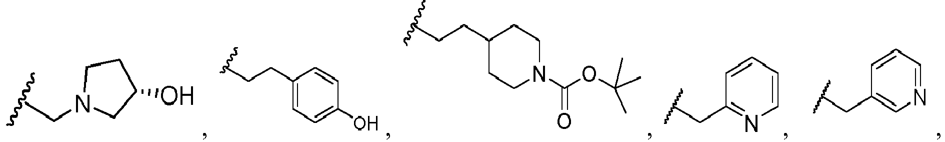 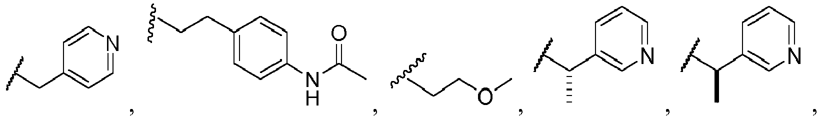 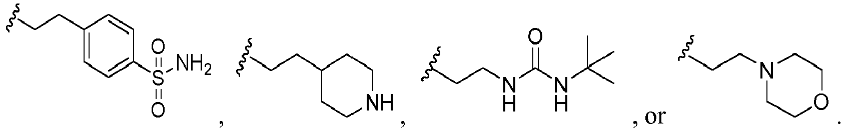
Embodiment 50. The compound of any one of embodiments 1 to 49, wherein L¹ is substituted or unsubstituted C₁-C₄ alkylene.
Embodiment 51. The compound of embodiment 50, wherein L¹ is 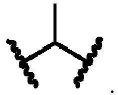
Embodiment 52. The compound of any one of embodiments 1 to 51, wherein R⁶ is hydrogen.
Embodiment 53. The compound of any one of embodiments 1 to 52, wherein R⁴ is hydrogen, substituted or unsubstituted C₁-C₈ alkyl, or substituted or unsubstituted 2 to 6 membered heteroalkyl.
Embodiment 54. The compound of embodiment 53, wherein R⁴ is substituted or unsubstituted C₁-C₄ alkyl, or substituted or unsubstituted 2 to 4 membered heteroalkyl.
Embodiment 55. The compound of embodiment 54, wherein R⁴ is unsubstituted C₁-C₄ alkyl.
Embodiment 56. The compound of embodiment 55, wherein R⁴ is unsubstituted methyl.
Embodiment 57. The compound of any one of embodiments 1 to 52, wherein R⁴ is -NR^{4A}R^{4B}, and each R^{4A} and R^{4B} is independently hydrogen, or substituted or unsubstituted C₁-C₄ alkyl.
Embodiment 58. The compound of embodiment 57, wherein R⁴ is -NH₂.
Embodiment 59. The compound of embodiment 1, wherein the compound is: 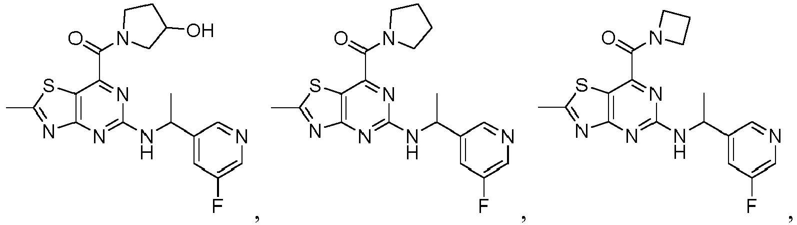 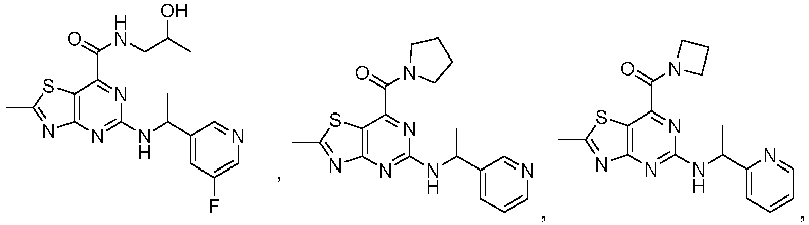 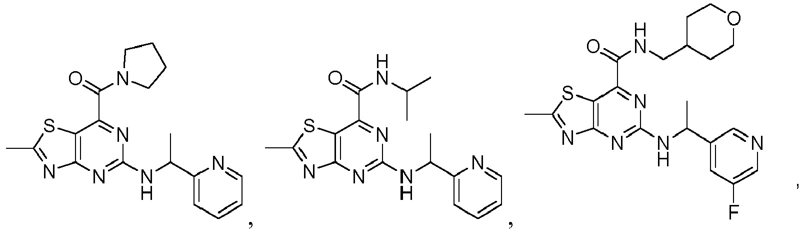 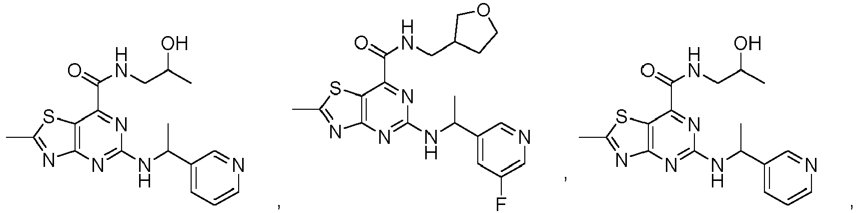 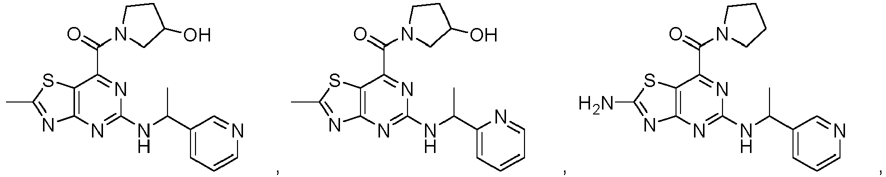 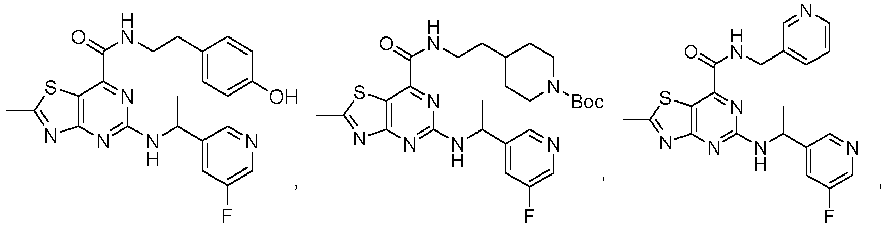 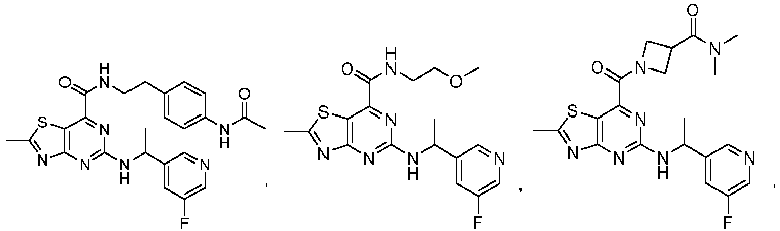 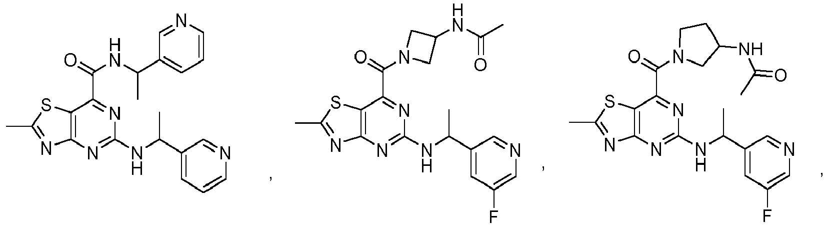 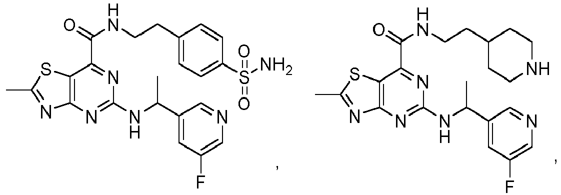 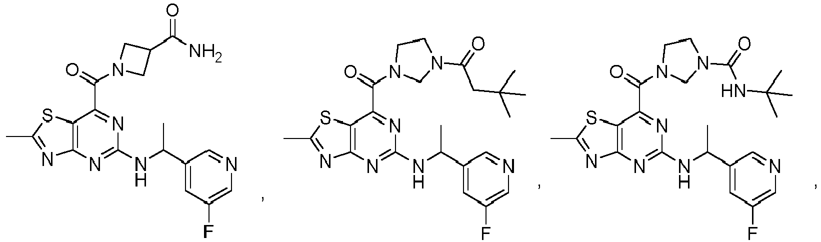 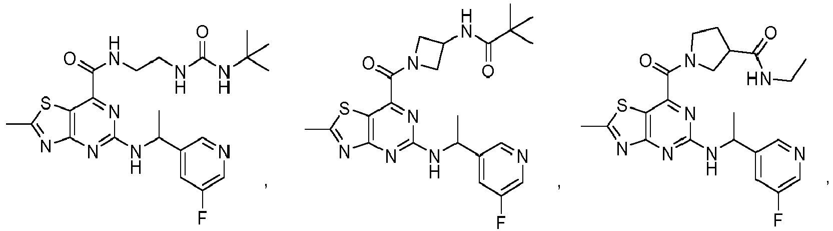 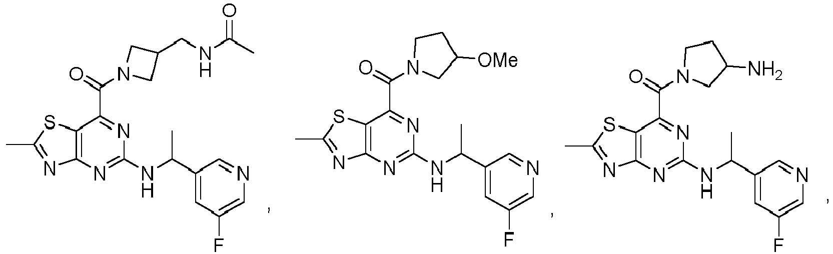 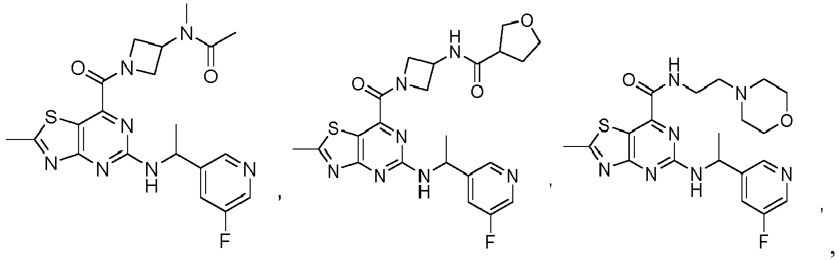 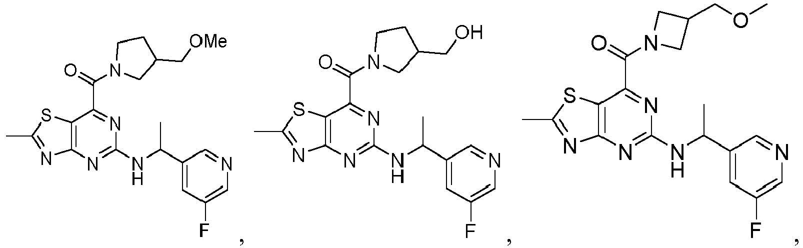 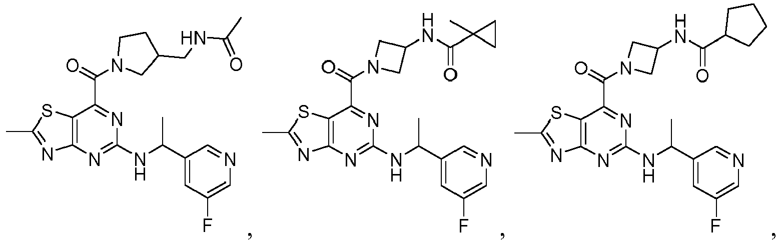 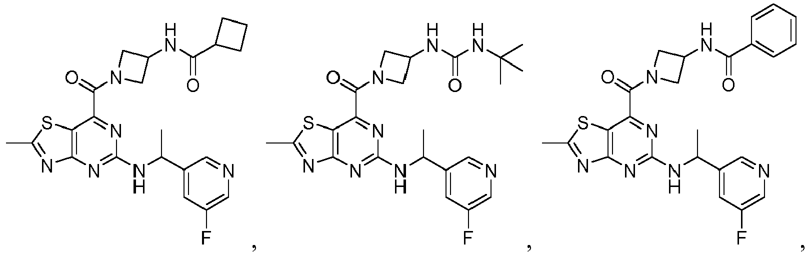 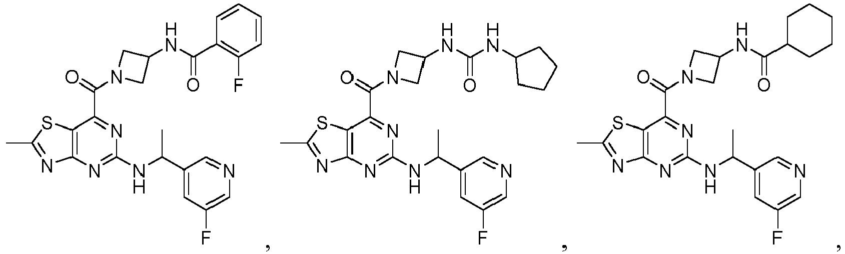 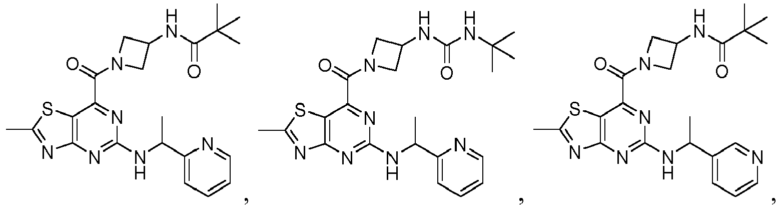 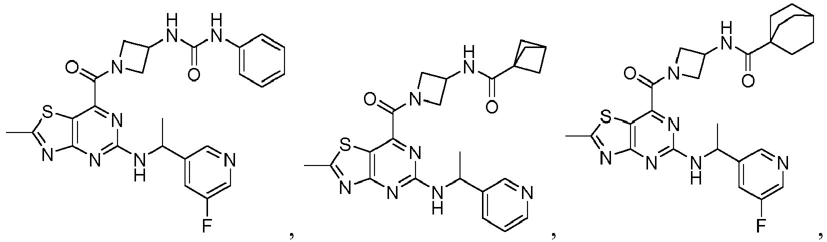 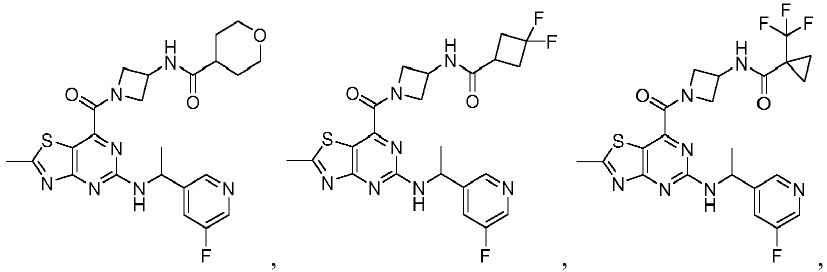 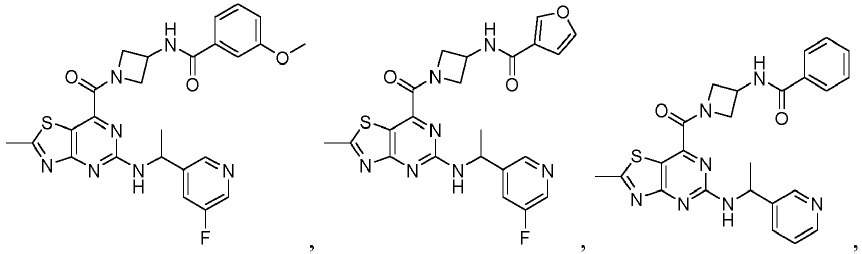 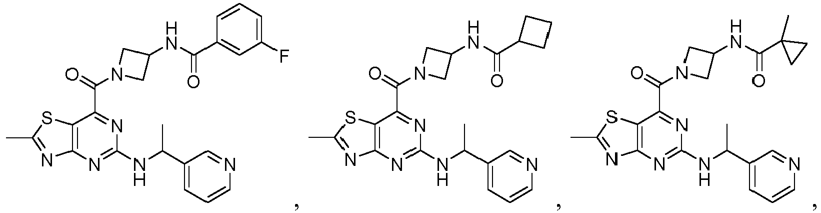 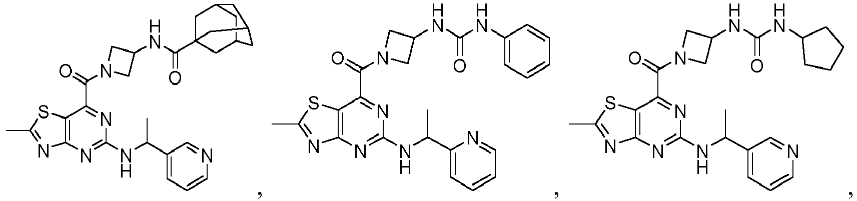 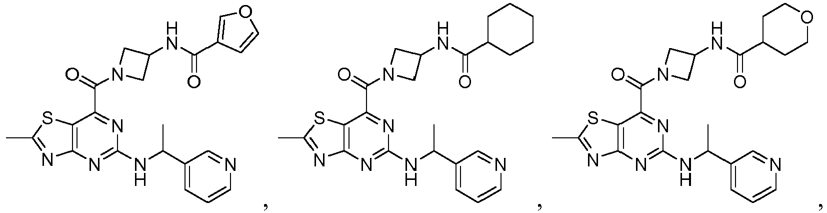 or 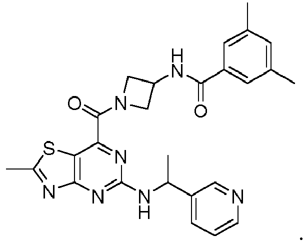
Embodiment 60. The compound of embodiment 1, wherein the compound is: 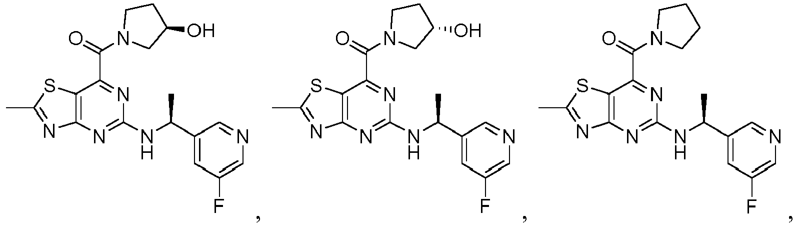 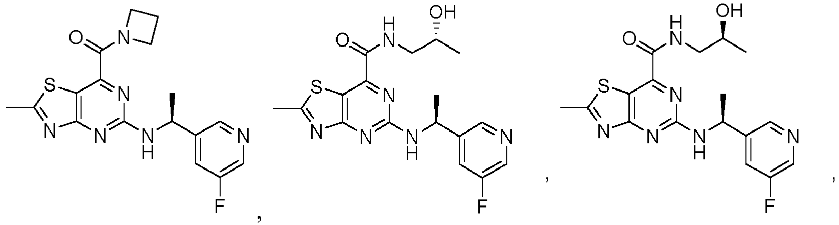 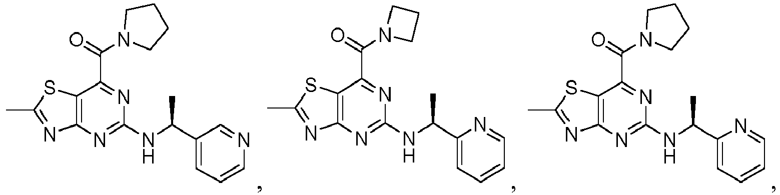 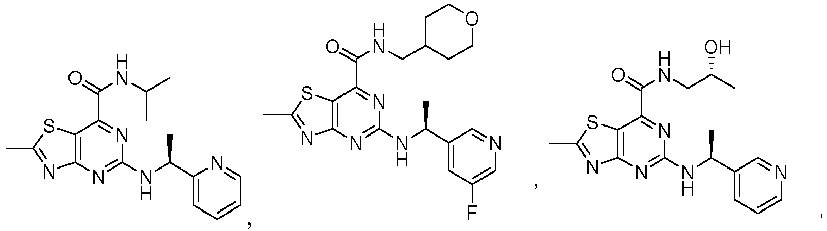 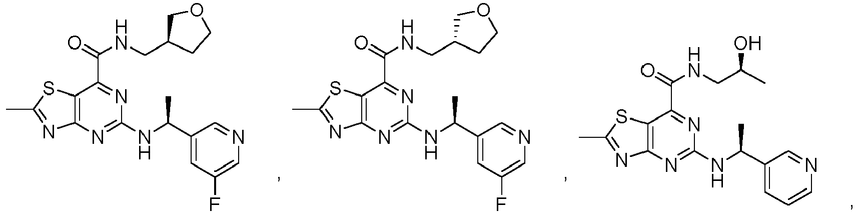 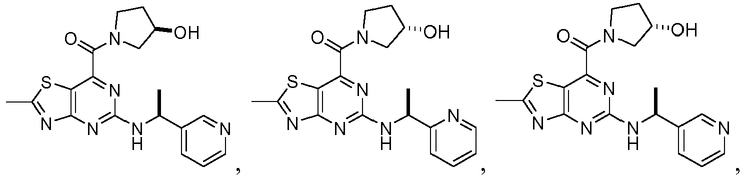 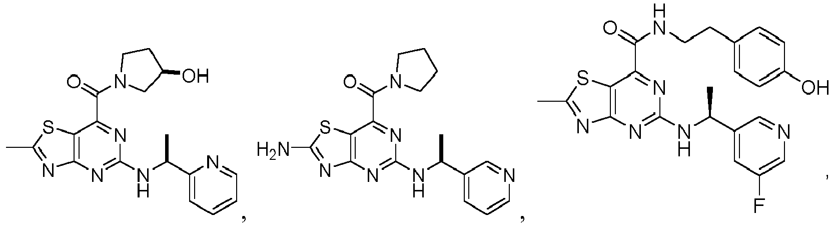 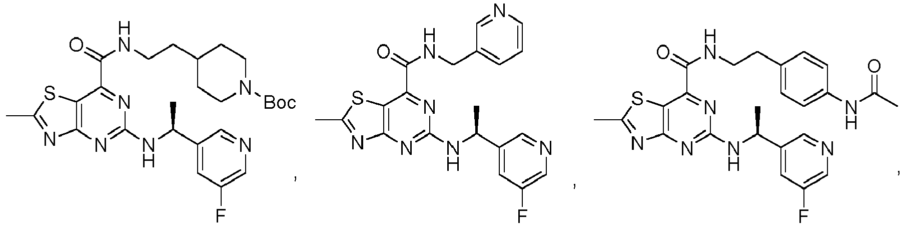 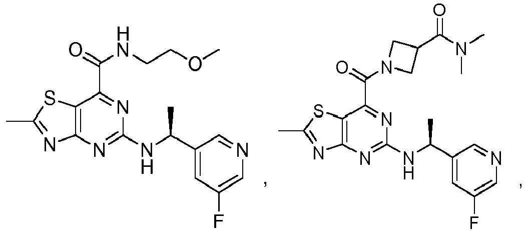 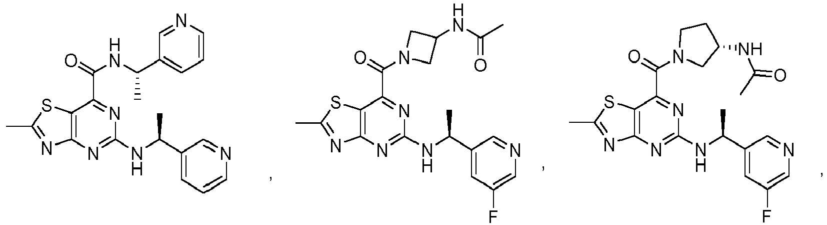 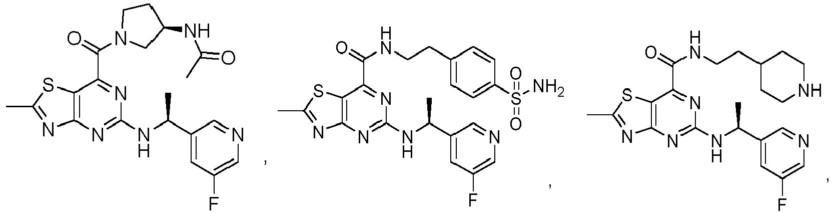 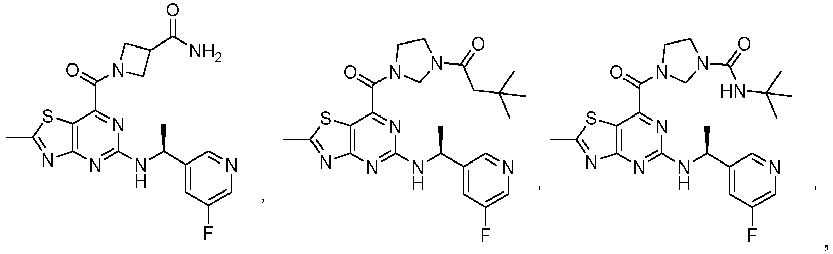 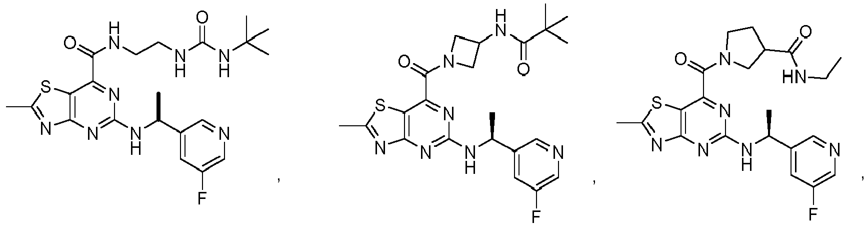 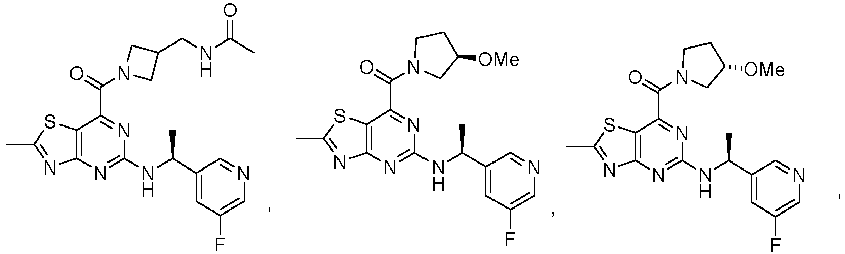 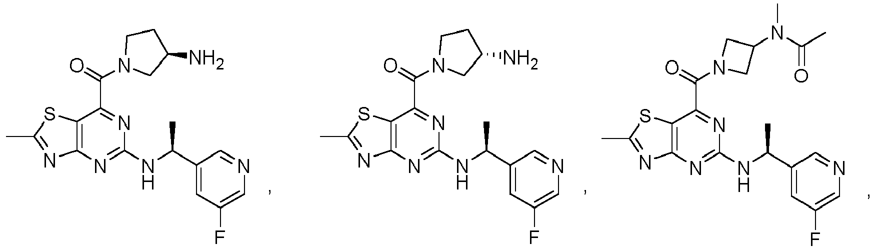 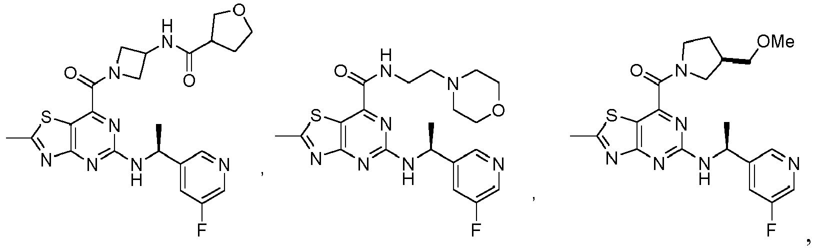 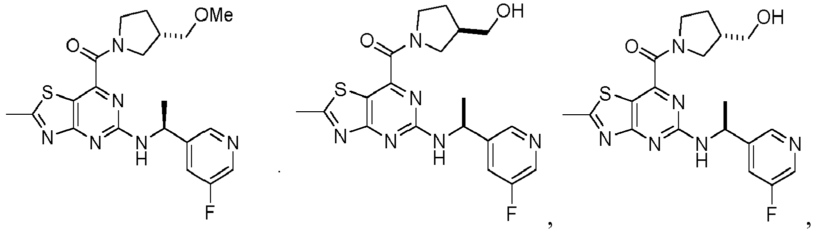 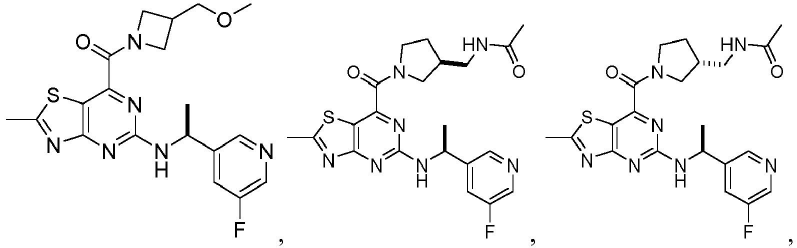 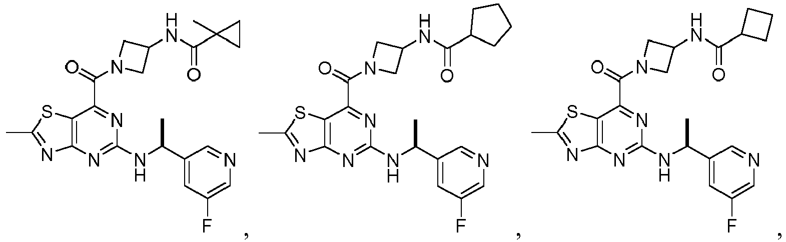 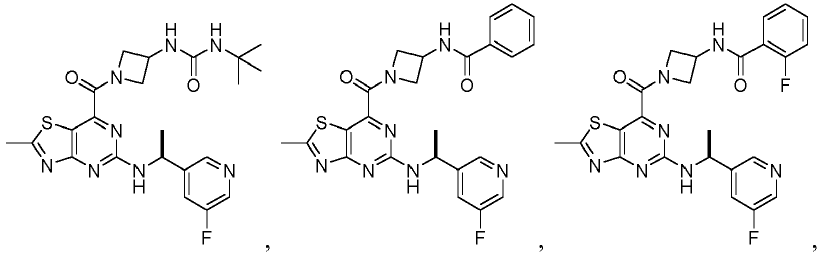 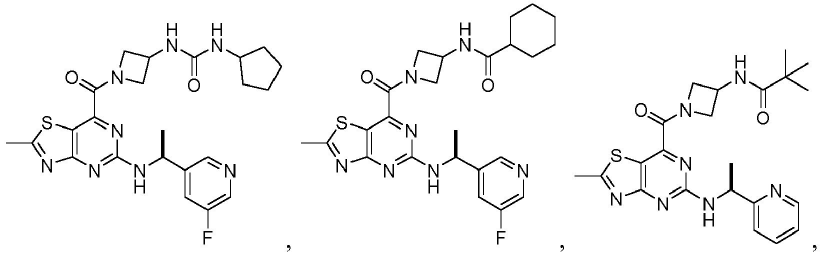 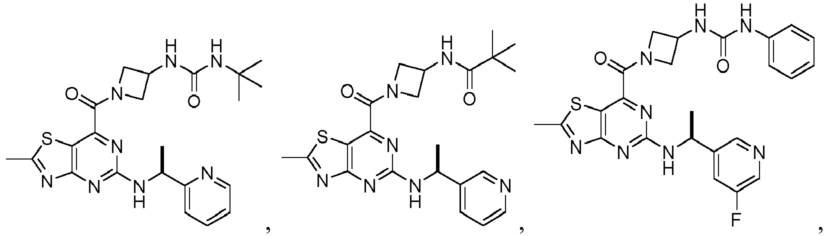 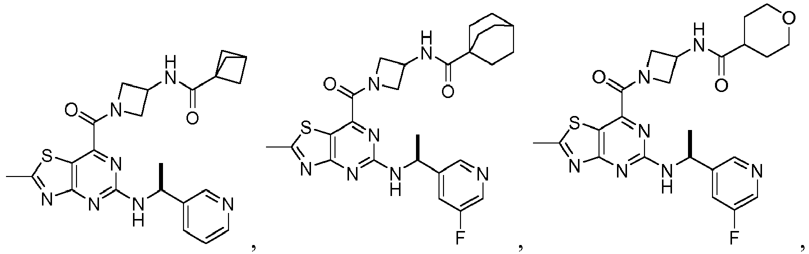 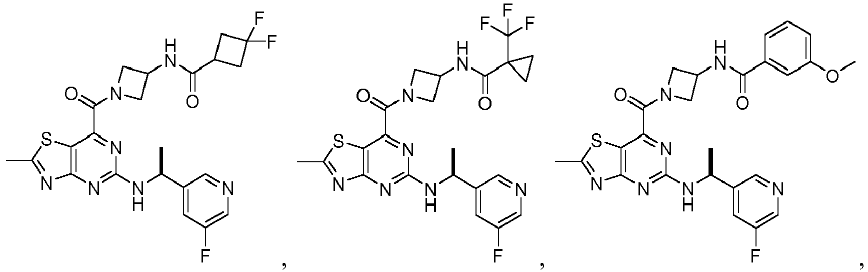 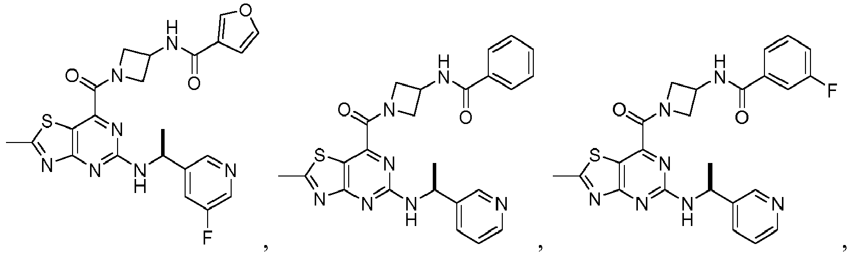 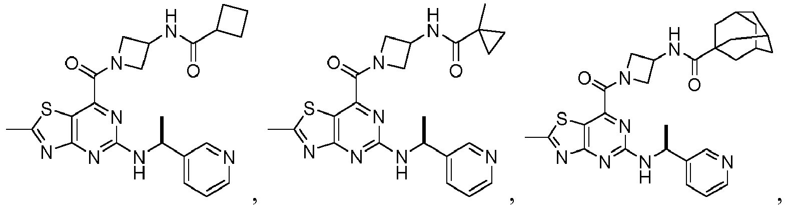 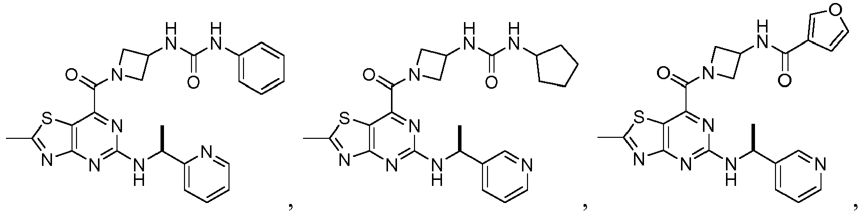 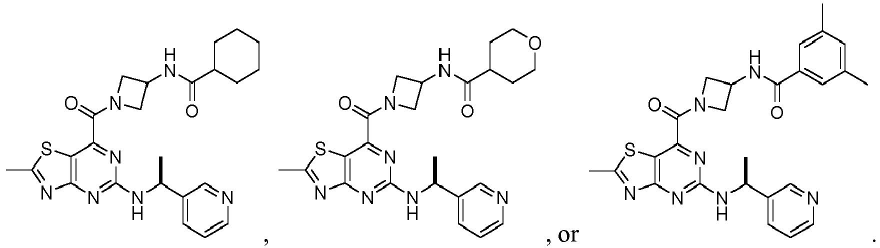
Embodiment 61. The compound of embodiment 1, wherein the compound is: 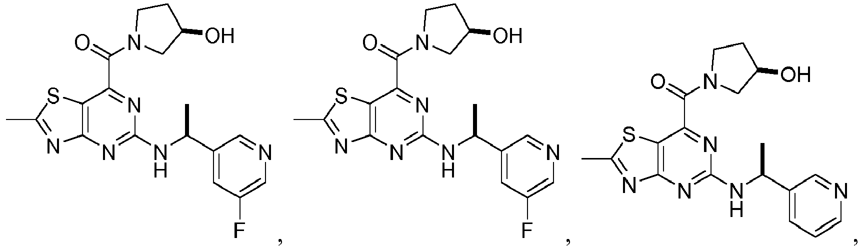 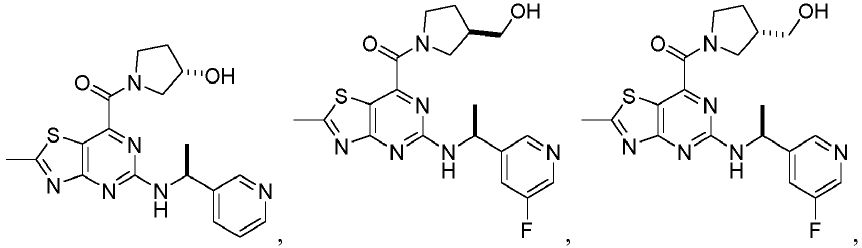 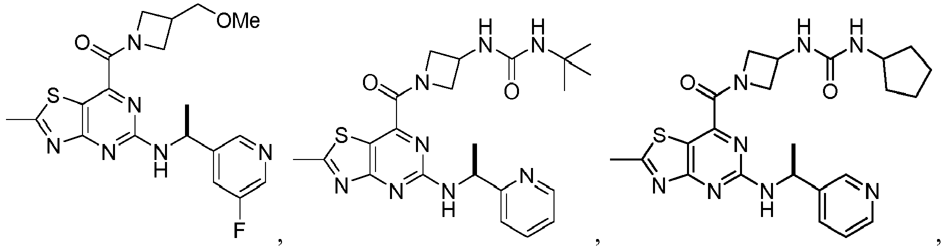 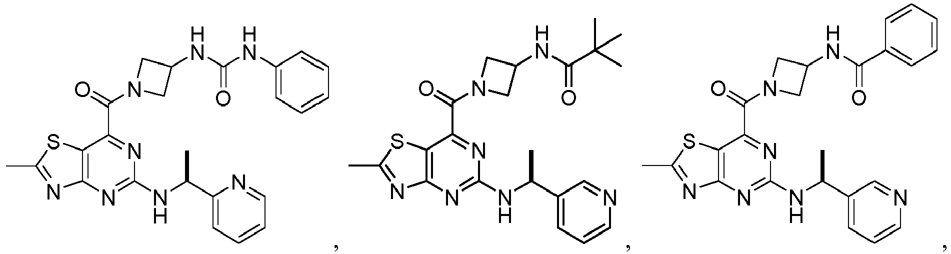 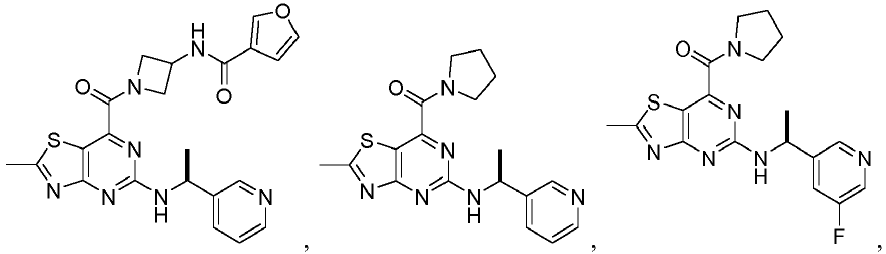 or 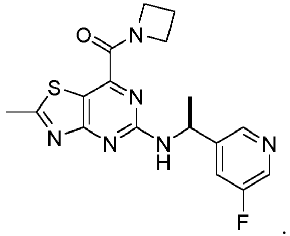
Embodiment 62. The compound of embodiment 1, wherein the compound is: 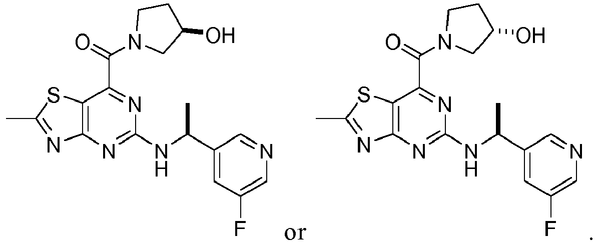
Embodiment 63. A pharmaceutical composition comprising the compound of any one of embodiments 1 to 62, and a pharmaceutically acceptable excipient.
Embodiment 64. A method of inhibiting Adenosine A2B Receptor activity and Adenosine A2A Receptor activity, said method comprising contacting the Adenosine A2B Receptor and/or Adenosine A2A Receptor with a compound of any one of embodiments 1 to 62.
Embodiment 65. A method of inhibiting Adenosine A2B Receptor activity, said method comprising contacting the Adenosine A2B Receptor with a compound of any one of embodiments 1 to 62.
Embodiment 66. A method of treating cancer, said method comprising administering to a subject in need thereof an effective amount of a compound of any one of embodiments 1 to 62.
Embodiment 67. The method of embodiment 66, wherein the cancer is breast cancer, lung cancer, colon cancer, bladder cancer, kidney cancer, liver cancer, pancreatic cancer, melanoma, leukemia, lymphoma, or prostate cancer.
Embodiment 68. A method of treating a disease or disorder associated with Adenosine A2B Receptor activity and/or Adenosine A2A Receptor activity, said method comprising administering to a subject in need thereof an effective amount of a compound of any one of embodiments 1 to 62.
Embodiment 69. The method of embodiment 68, wherein the disease or disorder is a cancer.
Embodiment 70. The method of embodiment 69, wherein the cancer is breast cancer, lung cancer, colon cancer, bladder cancer, kidney cancer, liver cancer, pancreatic cancer, melanoma, leukemia, lymphoma, or prostate cancer.
Embodiment 71. The method of embodiment 68, wherein the disease or disorder is a neurodegenerative disorder.
Embodiment 72. The method of embodiment 68, wherein the disease or disorder is a fibrotic disorder.

Further embodiments include the following numbered clauses:
Clause 1. A compound having the formula: 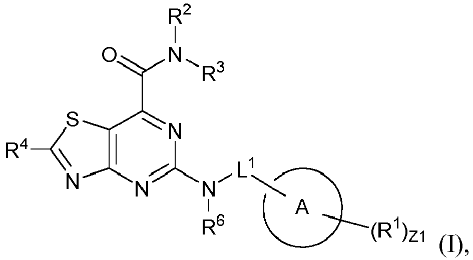 or a pharmaceutically acceptable salt thereof,
   wherein
   Ring A is a heteroaryl, cycloalkyl, heterocycloalkyl, or aryl;
   L¹ is an unsubstituted or substituted alkylene, a bond, -SOₙ₅L^{1A}-,
      -SOᵥ₅NR⁵L^{1A}- , -NHC(O)NR⁵L^{1A}-, -NR⁵L^{1A}-, -C(O)L^{1A}-, -C(O)OL^{1A}-, -C(O)NR⁵L^{1A}-, -OL^{1A}-, -NR⁵SO₂L^{1A}-, -NR⁵C(O)L^{1A}-, -NR⁵C(O)OL^{1A}-, -NR⁵OL^{1A}-, -SL^{1A}-, substituted or unsubstituted heteroalkylene, substituted or unsubstituted cycloalkylene, substituted or unsubstituted heterocycloalkylene, substituted or unsubstituted arylene, or substituted or unsubstituted heteroarylene;
   R¹ is halogen, -CX¹₃, -CHX¹₂, -CH₂X¹, -OCX¹₃, -OCH₂X¹, -OCHX¹₂, -N₃, -CN, -SOₙ₁R^{1D}, -SOᵥ₁NR^{1A}R^{1B}, -NHC(O)NR^{1A}R^{1B}, -N(O)ₘ₁, -NR^{1A}R^{1B}, -C(O)R^{1C}, -C(O)-OR^{1C}, -C(O)NR^{1A}R^{1B}, -OR^{1D}, -NR^{1A}SO₂R^{1D}, -NR^{1A}C(O)R^{1C}, -NR^{1A}C(O)OR^{1C}, -NR^{1A}OR^{1C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R² is hydrogen, -CX²₃, -CHX²₂, -CH₂X², -OCX²₃, -OCH₂X², -OCHX²₂,
      -C(O)R^{2C}, -C(O)OR^{2C}, -C(O)NR^{2A}R^{2B}, -OR^{2D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R³ is a substituted or unsubstituted alkyl, hydrogen, -CX³₃, -CHX³₂, -CH₂X³,
      -OCX³₃, -OCH₂X³, -OCHX³₂, -C(O)R^{3C}, -C(O)OR^{3C}, -C(O)NR^{3A}R^{3B}, -OR^{3D}, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R² and R³ together with the nitrogen attached thereto may be optionally joined to form a substituted or unsubstituted heterocycloalkyl, or substituted or unsubstituted heteroaryl;
   R⁴ is an unsubstituted or substituted alkyl, hydrogen, halogen, -CX⁴₃, -CHX⁴₂,-CH₂X⁴, -OCX⁴₃, -OCH₂X⁴, -OCHX⁴₂, -N₃, -CN, -SOₙ₄R^{4D}, -SOᵥ₄NR^{4A}R^{4B}, -NHC(O)NR^{4A}R^{4B}, -N(O)ₘ₄, -NR^{4A}R^{4B}, -C(O)R^{4C}, -C(O)-OR^{4C}, -C(O)NR^{4A}R^{4B}, -OR^{4D}, -NR^{4A}SO₂R^{4D}, -NR^{4A}C(O)R^{4C}, -NR^{4A}C(O)OR^{4C}, -NR^{4A}OR^{4C}, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R⁵ is hydrogen, -CX⁵₃, -CHX⁵₂, -CH₂X⁵, -OCX⁵₃, -OCH₂X⁵, -OCHX⁵₂,
      -C(O)R^{5C}, -C(O)OR^{5C}, -C(O)NR^{5A}R^{5B}, -OR^{5D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R⁶ is hydrogen, -CX⁶₃, -CHX⁶₂, -CH₂X⁶, -OCX⁶₃, -OCH₂X⁶, -OCHX⁶₂,
      -C(O)R^{6C}, -C(O)OR^{6C}, -C(O)NR^{6A}R^{6B}, -OR^{6D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   wherein R^{1A}, R^{1B}, R^{1C}, R^{1D}, R^{2A}, R^{2B}, R^{2C}, R^{2D}, R^{3A}, R^{3B}, R^{3C}, R^{3D}, R^{4A}, R^{4B}, R^{4C}, R^{4D}, R^{5A}, R^{5B}, R^{5C}, R^{5D}, R^{6A}, R^{6B}, R^{6C}, and R^{6D} are independently hydrogen, -CX₃,
      -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   L^{1A} is a bond, substituted or unsubstituted alkylene, substituted or unsubstituted heteroalkylene, substituted or unsubstituted cycloalkylene, substituted or unsubstituted heterocycloalkylene, substituted or unsubstituted arylene, or substituted or unsubstituted heteroarylene;
   z1 is an integer from 0 to 12;
   n1 and n4 are independently an integer from 0 to 4;
   m1, m4, v1, and v4 are independently an integer from 1 to 2; and
   X, X¹, X², X³, X⁴, X⁵ and X⁶ are independently -F, -Cl, -Br, or -I.
Clause 2. The compound of clause 1, wherein the Ring A is 5 to 10 membered heteroaryl or C₆-C₁₀ aryl.
Clause 3. The compound of clause 1, wherein the Ring A is pyridyl.
Clause 4. The compound of any one of clauses 1 to 3, wherein L¹ is an unsubstituted or substituted alkylene, a bond, or substituted or unsubstituted heteroalkylene.
Clause 5. The compound of any one of clauses 1 to 4, wherein R¹ is halogen, -CX¹₃,
   -CHX¹₂, -CH₂X¹, -OCX¹₃, -OCH₂X¹, -OCHX¹₂, -CN, -SR^{1D}, -NHC(O)NR^{1A}R^{1B}, -NR^{1A}R^{1B}, -C(O)R^{1C}, -C(O)-OR^{1C}, -C(O)NR^{1A}R^{1B}, -OR^{1D}, -NR^{1A}C(O)R^{1C}, -NR^{1A}C(O)OR^{1C}, substituted or unsubstituted alkyl, or substituted or unsubstituted heteroalkyl.
Clause 6. The compound of any one of clauses 1 to 5, wherein R⁶ is hydrogen, -CX⁶₃, -CHX⁶₆, -CH₆X⁶, -OCX⁶₃, -OCH₆X⁶, -OCHX⁶₆, -C(O)R^{6C}, -C(O)OR^{6C}, -C(O)NR^{6A}R^{6B}, -OR^{6D}, or substituted or unsubstituted alkyl.
Clause 7. The compound of any one of clauses 1 to 6, wherein R⁴ is unsubstituted or substituted alkyl, hydrogen, halogen, -CX⁴₃, -CHX⁴₂, -CH₂X⁴, -OCX⁴₃,-OCH₂X⁴, -OCHX⁴₂, -CN, -SR^{4D}, -NHC(O)NR^{4A}R^{4B}, -NR^{4A}R^{4B}, -C(O)R^{4C}, -C(O)-OR^{4C}, -C(O)NR^{4A}R^{4B}, -OR^{4D}, -NR^{4A}C(O)R^{4C}, -NR^{4A}C(O)OR^{4C}, -NR^{4A}OR^{4C}, substituted or unsubstituted heteroalkyl, or substituted or unsubstituted cycloalkyl.
Clause 8. The compound of any one of clauses 1 to 7, wherein the compound has the formula: 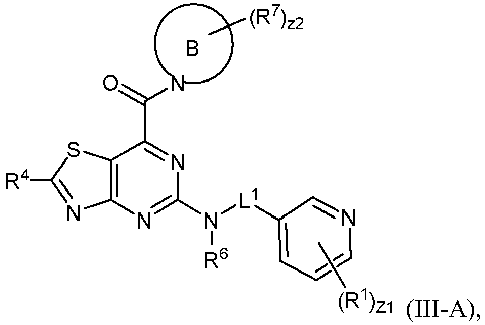 or 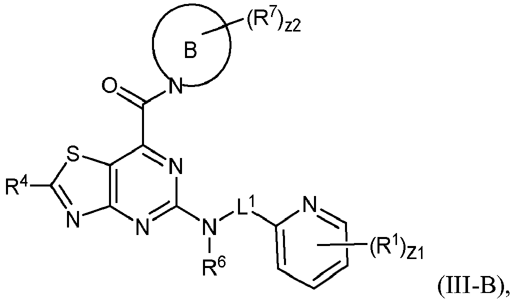 or a pharmaceutically acceptable salt thereof;
   wherein
   Ring B is a 4 to 7 membered heterocycloalkyl;
   R⁷ is independently halogen, -CX⁷₃, -CHX⁷₂, -CH₂X⁷, -OCX⁷₃, -OCH₂X⁷,
      -OCHX⁷₂, -N₃, -CN, -SOₙ₇R^{7D}, -SOᵥ₇NR^{7A}R^{7B}, -NR^{7A}C(O)NR^{7A}R^{7B}, -NR7^{A}S(O)NR7^{A}R7^{B}, -N(O)ₘ₇, -NR^{7A}R^{7B}, -C(O)R^{7C}, -C(O)-OR^{7C}, -C(O)NR^{7A}R^{7B}, -C(O)NR^{7A}OR^{7B}, -OR^{7D}, -NR^{7A}SO₂R^{7D}, -NR^{7A}C(O)R^{7C}, -NR^{7A}C(O)OR^{7C}, -NR^{7A}OR^{7C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{7A}, R^{7B}, R^{7C}, and R^{7D} are independently hydrogen, -CN, -CX₃, -CHX₂,
      -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   z1 is an integer from 0 to 4;
   z2 is an integer from 0 to 12;
   n7 is independently an integer from 0 to 4;
   m7 and v7 are independently an integer from 1 to 2; and
   X⁷ is independently -F, -Cl, -Br, or -I.
Clause 9. The compound of any one of clauses 1 to 7, wherein Ring A is 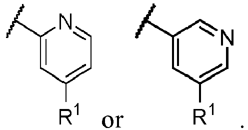
Clause 10. The compound of clause 8, wherein the compound has a formula 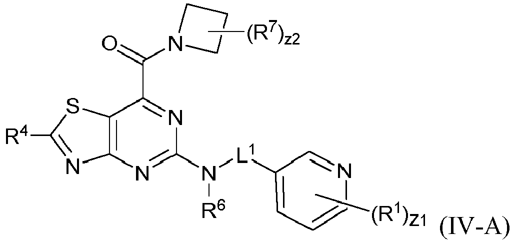 or 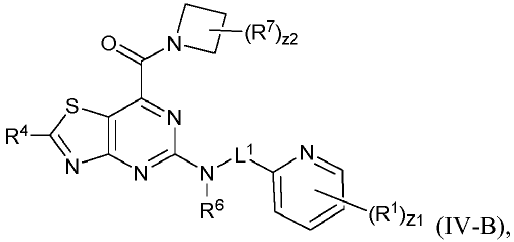 or a pharmaceutically acceptable salt thereof, wherein z2 is an integer from 0 to 6.
Clause 11. The compound of clause 10, wherein:
   z2 is 0 or 1;
   R⁷ is independently -OR^{7D}, -NR^{7A}R^{7B}, -C(O)NR^{7A}R^{7B}, -C(O)NR^{7A}OR^{7B},
      -NR^{7A}C(O)R^{7C}, -NR^{7A}C(O)NR^{7A}R^{7B}, -C(O)R^{7C}, or substituted or unsubstituted 2 to 6 membered heteroalkyl;
   R^{7A} and R^{7B} are independently hydrogen, or unsubstituted C₁-C₄ alkyl;
   R^{7C} is independently substituted or unsubstituted C₁-C₁₂ alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{7D} is independently hydrogen, or unsubstituted C₁-C₄ alkyl.
Clause 12. The compound of clause 11, wherein R⁷ is independently -NR^{7A}C(O)R^{7C}, or -C(O)NR^{7A}R^{7B}-.
Clause 13. The compound of clause 8, wherein R^{7A}, R^{7B}, and R^{7C} are independently hydrogen, substituted or unsubstituted C₁-C₄ alkyl, substituted or unsubstituted 3 to 8-membered cycloalkyl, substituted or unsubstituted 5 to 6 membered heterocycloalkyl, substituted or unsubstituted phenyl, or substituted or unsubstituted 5 to 6 membered heteroaryl.
Clause 14. The compound of clause 13, wherein R⁷ is independently: 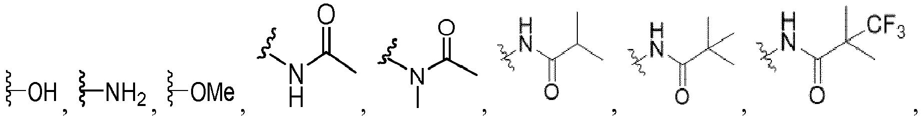 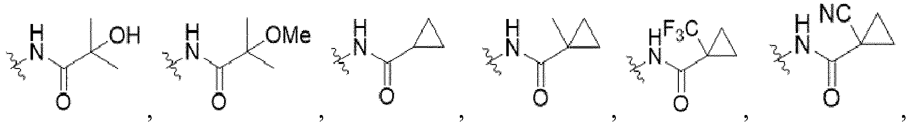 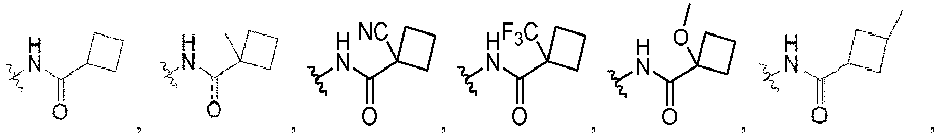 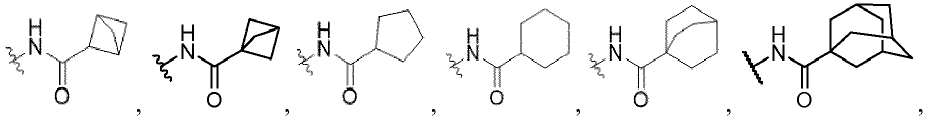 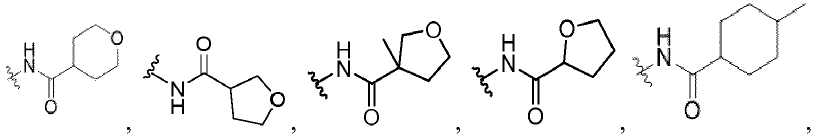 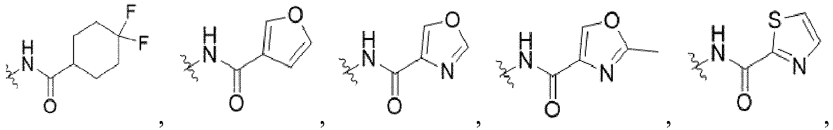 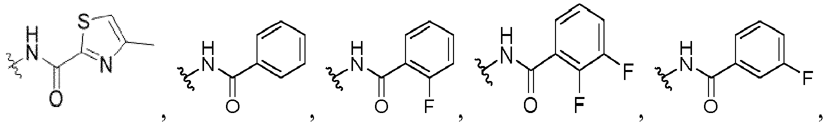 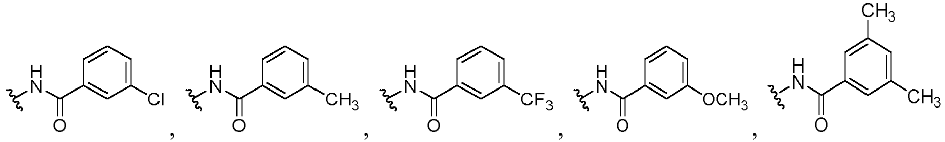 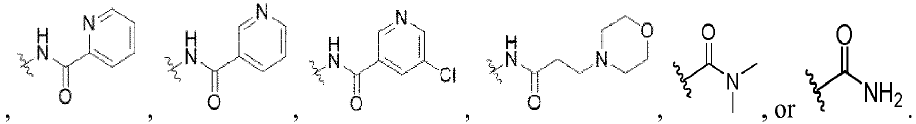
Clause 15. The compound of clause 14, wherein R⁷ is independently -OH.
Clause 16. The compound of clause 8, wherein R⁷ is independently - NHC(O)NHR^{7B}.
Clause 17. The compound of clause 16, wherein R^{7B} is independently substituted or unsubstituted C₁-C₄ alkyl, substituted or unsubstituted 3 to 8-membered cycloalkyl, substituted or unsubstituted 5 to 6 membered heterocycloalkyl, substituted or unsubstituted phenyl, or substituted or unsubstituted 5 to 6 membered heteroaryl.
Clause 18. The compound of clause 17, wherein R⁷ is independently: 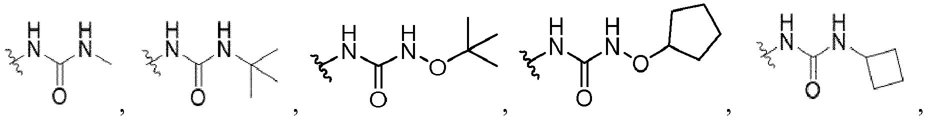 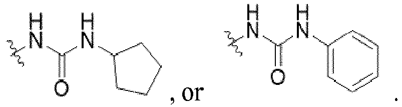
Clause 19. The compound of clause 11, wherein R⁷ is independently substituted or unsubstituted C₁-C₄ alkyl or substituted or unsubstituted 2 to 6 membered heteroalkyl.
Clause 20. The compound of clause 19, wherein R⁷ is independently 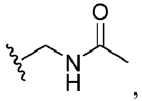 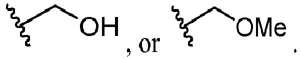
Clause 21. The compound of clause 8, wherein the compound has a formula: 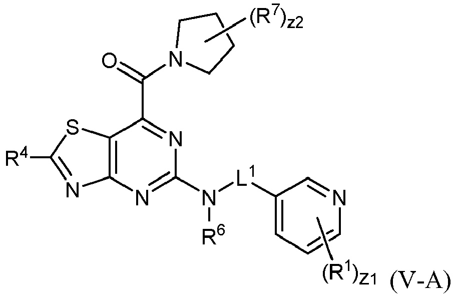 or 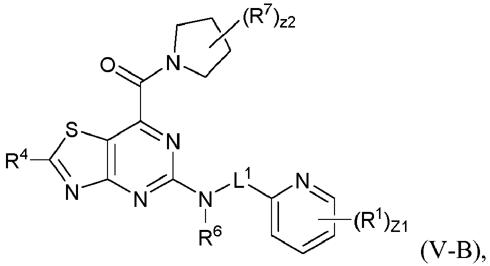 or a pharmaceutically acceptable salt thereof, wherein z2 is an integer 0 to 8.
Clause 22. The compound of clause 21, wherein
   z2 is 0 or 1;
   R⁷ is independently -NR^{7A}R^{7B}, -C(O)NR^{7A}R^{7B}, -C(O)NR^{7A}OR^{7B},
      -NR^{7A}C(O)R^{7C}, -OR^{7D}, -C(O)R^{7C}, or substituted or unsubstituted 2 to 6 membered heteroalkyl;
   R^{7A} and R^{7B} are independently hydrogen, or unsubstituted C₁-C₄ alkyl;
   R^{7C} is independently unsubstituted C₁-C₆ alkyl; and
   R^{7D} is independently hydrogen, or unsubstituted C₁-C₄ alkyl.
Clause 23. The compound of clause 21, wherein z2 is 1 and R⁷ is independently -OH.
Clause 24. The compound of clause 21, wherein z1 is 0 or 1, and R¹ is independently a halogen.
Clause 25. The compound of clause 8, wherein the compound has the formula: 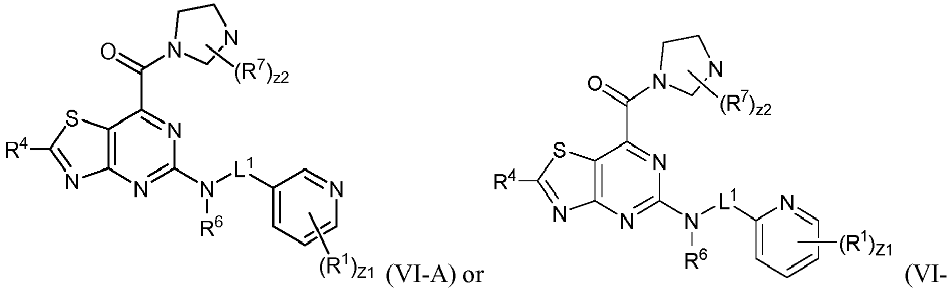 B), or a pharmaceutically acceptable salt thereof, wherein z2 is an integer from 0 to 7.
Clause 26. The compound of clause 25, wherein:
   z2 is 0 or 1;
   R⁷ is independently -C(O)R^{7C}, or -C(O)NR^{7A}R^{7B};
   R^{7A} and R^{7B} are independently hydrogen, or unsubstituted C₁-C₄ alkyl; and
   R^{7C} is independently unsubstituted C₁-C₆ alkyl.
Clause 27. The compound of any one of clauses 1 to 7, wherein the compound has a formula: 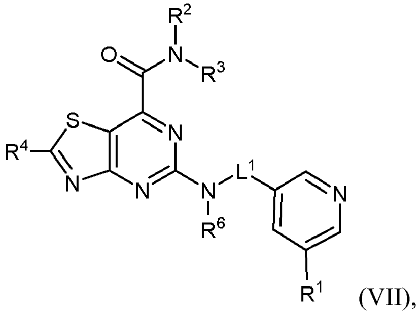 or a pharmaceutically acceptable salt thereof.
Clause 28. The compound of clause 27, wherein R¹ is halogen.
Clause 29. The compound of any one of clauses 1 to 7, wherein the compound has the formula: 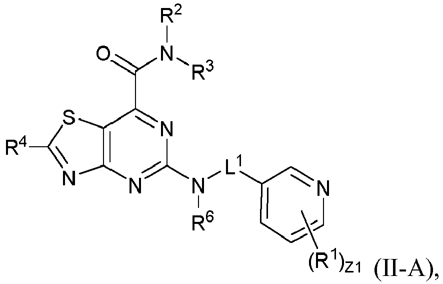 or a pharmaceutically acceptable salt thereof, wherein z1 is an integer from 0 to 4.
Clause 30. The compound of any one of clauses 1 to 7, wherein the compound has the formula: 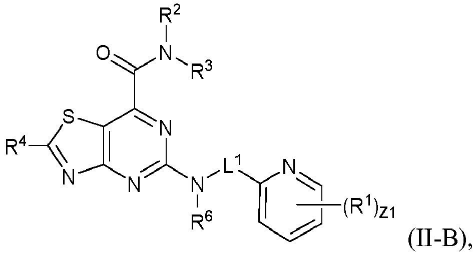 or a pharmaceutically acceptable salt thereof wherein z1 is an integer from 0 to 4.
Clause 31. The compound of any one of clauses 1 to 7, wherein the compound has a formula: 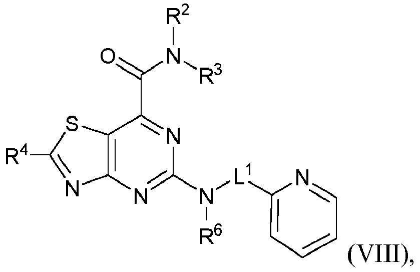 or a pharmaceutically acceptable salt thereof
Clause 32. The compound of any one of clauses 27 to 31, wherein R² and R³ together with the nitrogen attached thereto are joined to form 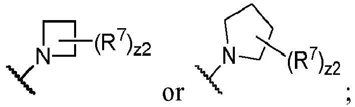 wherein
   R⁷ is independently halogen, -CX⁷₃, -CHX⁷₂, -CH₂X⁷, -OCX⁷₃, -OCH₂X⁷,
      -OCHX⁷₂, -N₃, -CN, -SOₙ₇R^{7D}, -SOᵥ₇NR^{7A}R^{7B}, -NR^{7A}C(O)NR^{7A}R^{7B}, -NR^{7A}S(O)NR^{7A}R^{7B}, -N(O)ₘ₇, -NR^{7A}R^{7B}, -C(O)R^{7C}, -C(O)-OR^{7C}, -C(O)NR^{7A}R^{7B}, -C(O)NR^{7A}OR^{7B}, -OR^{7D}, -NR^{7A}SO₂R^{7D}, -NR^{7A}C(O)R^{7C}, -NR^{7A}C(O)OR^{7C}, -NR^{7A}OR^{7C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{7A}, R^{7B}, R^{7C}, and R^{7D} are independently hydrogen, -CN, -CX₃, -CHX₂,
      -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   z2 is an integer from 0 to 1;
   n7 is independently an integer from 0 to 4;
   m7 and v7 are independently an integer from 1 to 2; and
   X⁷ is independently -F, -Cl, -Br, or -I.
Clause 33. The compound of clause 32, where z2 is 0.
Clause 34. The compound of clause 32, wherein z2 is 1.
Clause 35. The compound of clause 34, wherein R⁷ is 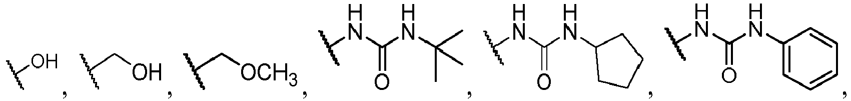 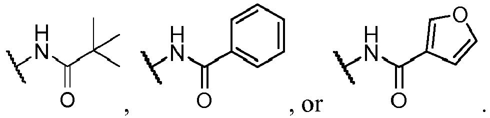
Clause 36. The compound of any one of clauses 1 to 7 and 27 to 31, wherein R² and R³ are each independently hydrogen, substituted or unsubstituted C₁-C₁₂ alkyl, or substituted or unsubstituted 2 to 12 membered heteroalkyl.
Clause 37. The compound of any one of clauses 1 to 7 and 27 to 31, wherein the compound has a formula: 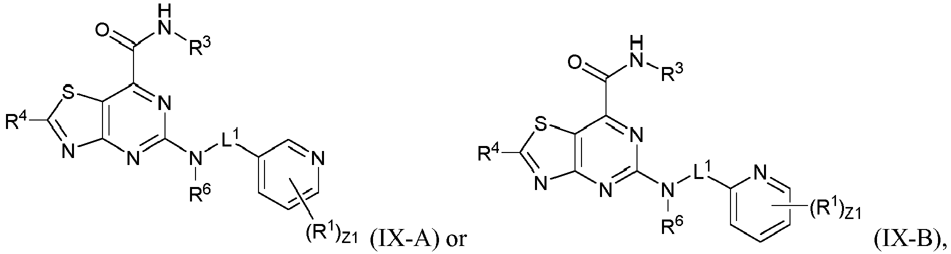 or a pharmaceutically acceptable salt thereof.
Clause 38. The compound of clause 37, wherein R³ is substituted or unsubstituted C₁-C₁₀ alkyl, or substituted or unsubstituted 2 to 10 membered heteroalkyl.
Clause 39. The compound of clause 37, wherein R³ is substituted or unsubstituted C₁-C₄ alkyl, or substituted or unsubstituted 2 to 4 membered heteroalkyl.
Clause 40. The compound of clause 38, wherein R³ is: 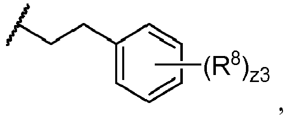 wherein
   R⁸ is independently -OR^{8D}, halogen, -CX⁸₃, -CHX⁸₂, -CH₂X⁸, -OCX⁸₃,
      -OCH₂X⁸, -OCHX⁸₂, -N₃, -CN, -SOₙ₈R^{8D}, -SOᵥ₈NR^{8A}R^{8B}, -NHC(O)NR^{8A}R^{8B}, -N(O)ₘ₈, -NR^{8A}R^{8B}, -C(O)R^{8C}, -C(O)-OR^{8C}, -C(O)NR^{8A}R^{8B}, -NR^{8A}SO₂R^{8D}, -NR^{8A}C(O)R^{8C}, -NR^{8A}C(O)OR^{8C}, -NR^{8A}OR^{8C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{8A}, R^{8B}, R^{8C}, and R^{8D} are independently hydrogen, -CX₃, -CHX₂, -CH₂X,
      -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   z3 is an integer from 0 to 5;
   n8 is independently an integer from 0 to 4;
   m8 and v8 are independently an integer from 1 to 2; and
   X⁸ is independently -F, -Cl, -Br, or -I.
Clause 41. The compound of clause 40, wherein R³ is: 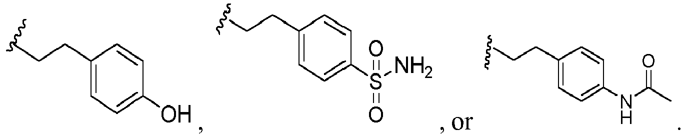
Clause 42. The compound of clause 38, wherein R³ is: 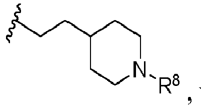 wherein:
   R⁸ is hydrogen, halogen, -CX⁸₃, -CHX⁸₂, -CH₂X⁸, -OCX⁸₃, -OCH₂X⁸,
      -OCHX⁸₂, -CN, -C(O)R^{8C}, -C(O)-OR^{8C}, -C(O)NR^{8A}R^{8B}, -OR^{8D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{8A}, R^{8B}, R^{8C}, and R^{8D} are independently hydrogen, -CX₃, -CHX₂, -CH₂X,
      -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   n8 is independently an integer from 0 to 4;
   m8 and v8 are independently an integer from 1 to 2; and
   X⁸ is independently -F, -Cl, -Br, or -I.
Clause 43. The compound of clause 42, wherein R³ is: 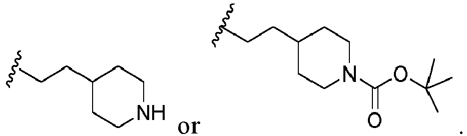
Clause 44. The compound of clause 38, wherein R³ is: 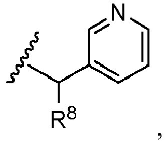 wherein
   R⁸ is hydrogen, unsubstituted or substituted alkyl, halogen, -CX⁸₃, -CHX⁸₂,
      -CH₂X⁸, -OCX⁸₃, -OCH₂X⁸, -OCHX⁸₂, -N₃, -CN, -SOᵥ₈R^{8D}, -SOᵥ₈NR^{8A}R^{8B} -NHC(O)NR^{8A}R^{8B}, -N(O)ₘ₈, -NR^{8A}R^{8B}, -C(O)R^{8C}, -C(O)-OR^{8C}, -C(O)NR^{8A}R^{8B}, -OR^{8D}, -NR^{8A}SO₂R^{8D}, -NR^{8A}C(O)R^{8C}, -NR^{8A}C(O)OR^{8C}, -NR^{8A}OR^{8C}, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{8A}, R^{8B}, R^{8C}, and R^{8D} are independently hydrogen, -CX₃, -CHX₂, -CH₂X,
      -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   n8 is independently an integer from 0 to 4;
   m8 and v8 are independently an integer from 1 to 2; and
   X⁸ is independently -F, -Cl, -Br, or -I.
Clause 45. The compound of clause 44, wherein R³ is: 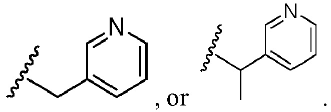
Clause 46. The compound of clause 38, wherein R³ is: 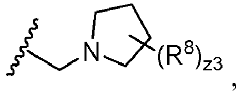 wherein:
   R⁸ is -OR^{8D}, halogen, -CX⁸₃, -CHX⁸₂, -CH₂X⁸, -OCX⁸₃, -OCH₂X⁸, -OCHX⁸₂,
      -N₃, -CN, -SOₙ₈R^{8D}, -SOᵥ₈NR^{8A}R^{8B}, -NHC(O)NR^{8A}R^{8B}, -N(O)ₘ₈, -NR^{8A}R^{8B}, -C(O)R^{8C}, -C(O)-OR^{8C}, -C(O)NR^{8A}R^{8B}, -NR^{8A}SO₂R^{8D}, -NR^{8A}C(O)R^{8C}, -NR^{8A}C(O)OR^{8C}, -NR^{8A}OR^{8C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   R^{8A}, R^{8B}, R^{8C}, and R^{8D} are independently hydrogen, -CX₃, -CHX₂, -CH₂X,
      -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
   z3 is an integer from 0 to 8;
   n8 is independently an integer from 0 to 4;
   m8 and v8 are independently an integer from 1 to 2; and
   X⁸ is independently -F, -Cl, -Br, or -I.
Clause 47. The compound of clause 46, wherein R³ is: 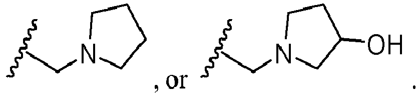
Clause 47. The compound of clause 38, wherein R³ is: 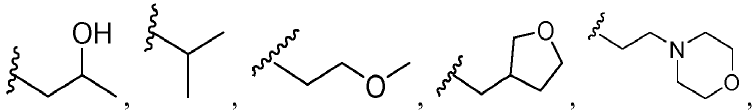 or 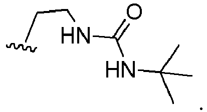
Clause 49. The compound of clause 38, wherein R³ is: 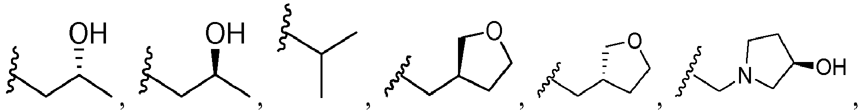 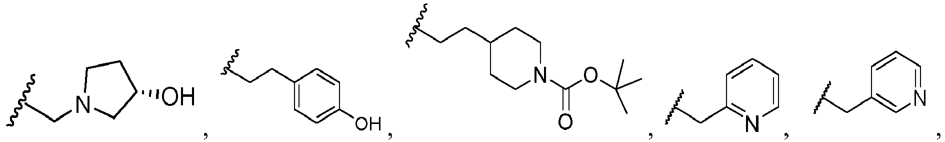 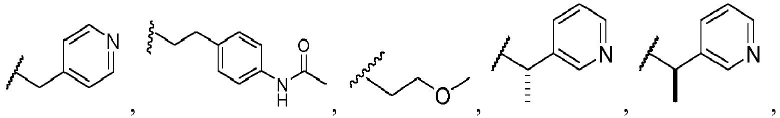 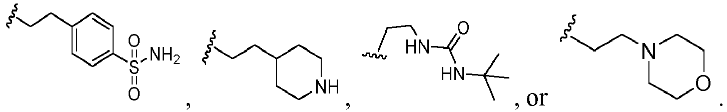
Clause 50. The compound of any one of clauses 1 to 49, wherein L¹ is substituted or unsubstituted C₁-C₄ alkylene.
Clause 51. The compound of clause 50, wherein L¹ is 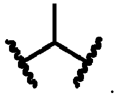
Clause 52. The compound of any one of clauses 1 to 51, wherein R⁶ is hydrogen.
Clause 53. The compound of any one of clauses 1 to 52, wherein R⁴ is hydrogen, substituted or unsubstituted C₁-C₈ alkyl, or substituted or unsubstituted 2 to 6 membered heteroalkyl.
Clause 54. The compound of clause 53, wherein R⁴ is substituted or unsubstituted C₁-C₄ alkyl, or substituted or unsubstituted 2 to 4 membered heteroalkyl.
Clause 55. The compound of clause 54, wherein R⁴ is unsubstituted C₁-C₄ alkyl.
Clause 56. The compound of clause 55, wherein R⁴ is unsubstituted methyl.
Clause 57. The compound of any one of clauses 1 to 52, wherein R⁴ is -NR^{4A}R^{4B}, and each R^{4A} and R^{4B} is independently hydrogen, or substituted or unsubstituted C₁-C₄ alkyl.
Clause 58. The compound of clause 57, wherein R⁴ is -NH₂.
Clause 59. The compound of clause 1, wherein the compound is: 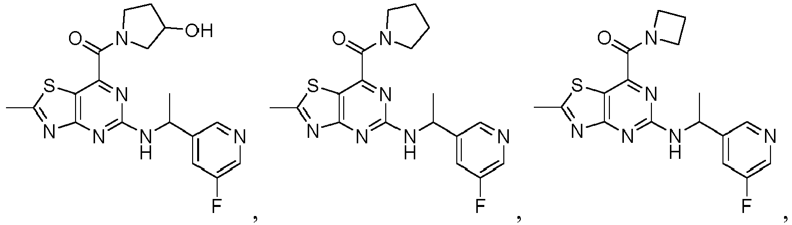 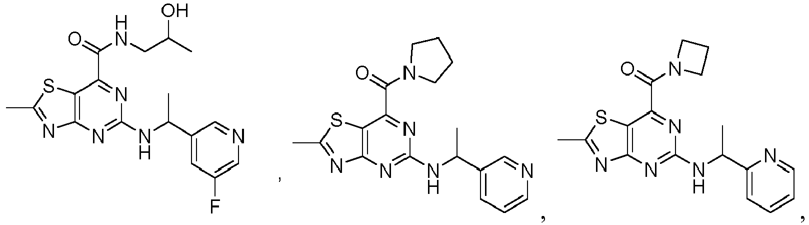 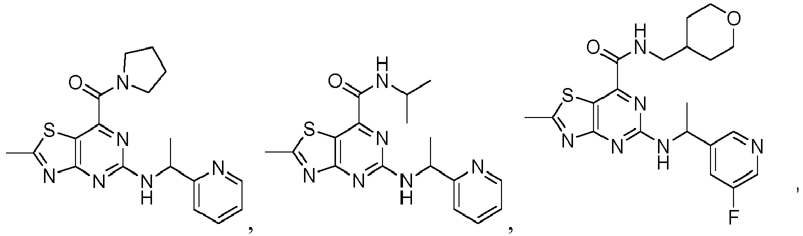 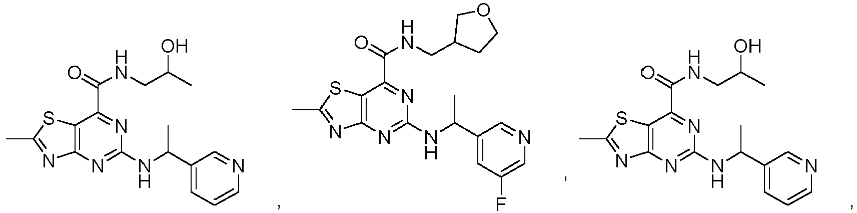 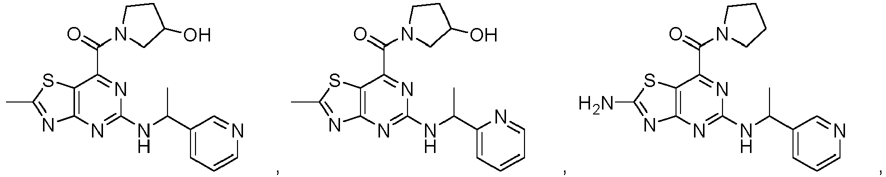 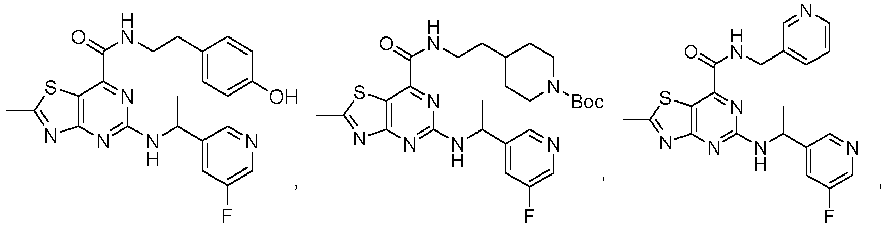 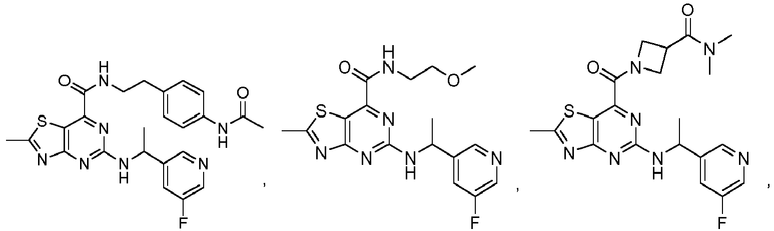 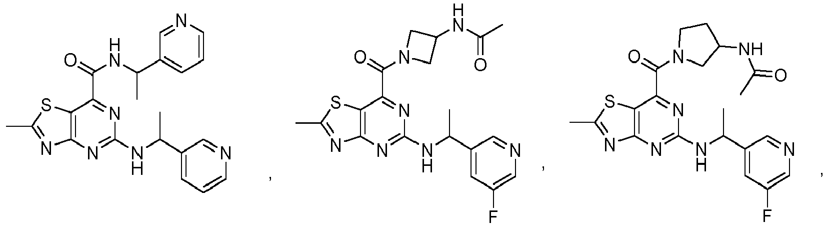 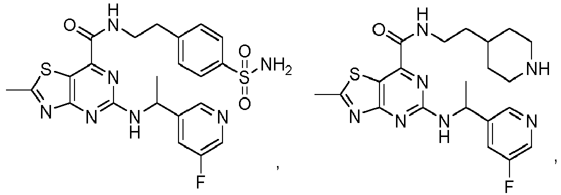 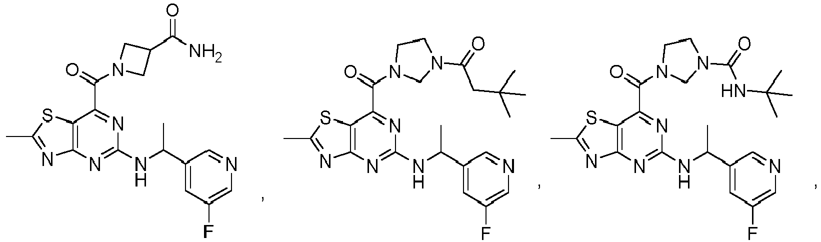 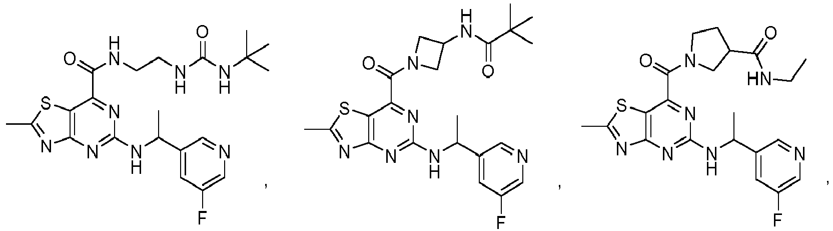 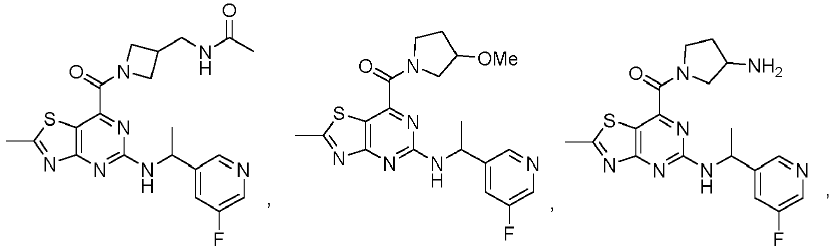 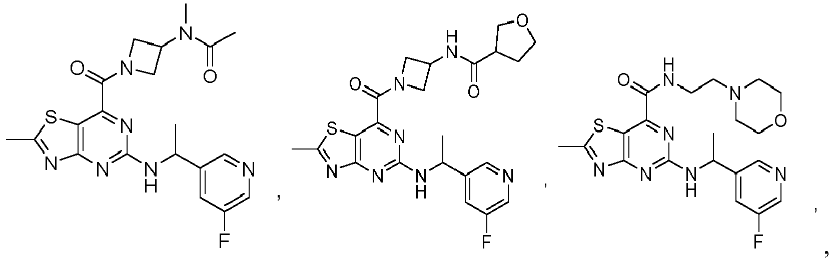 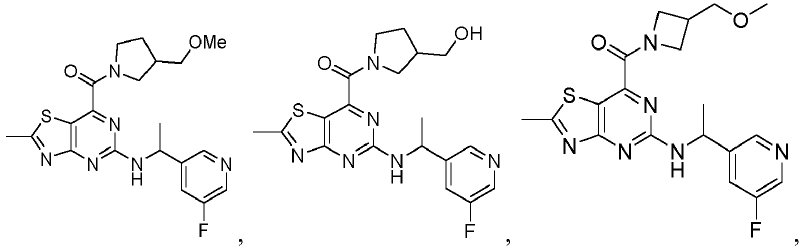 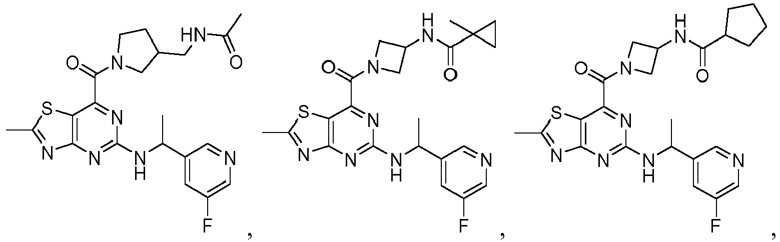 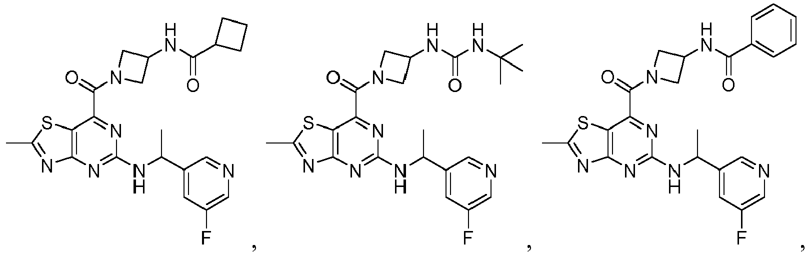 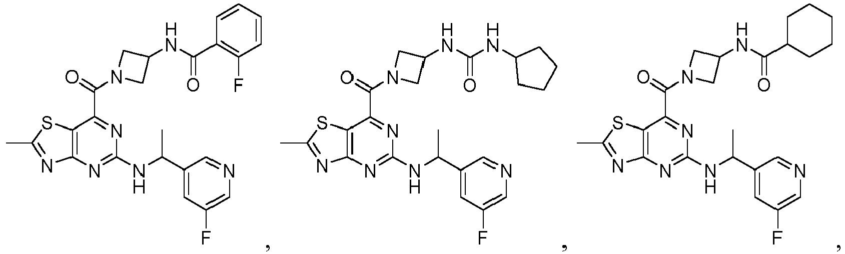 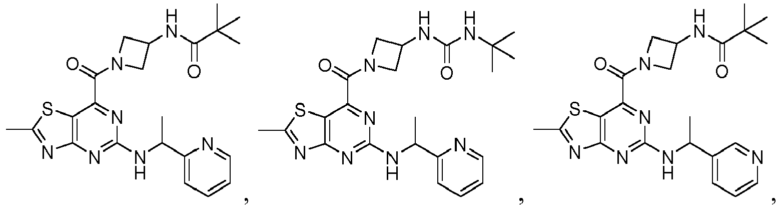 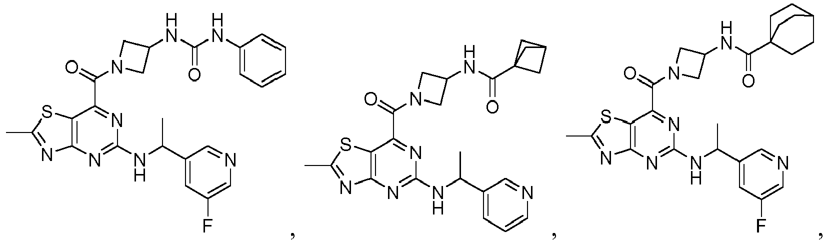 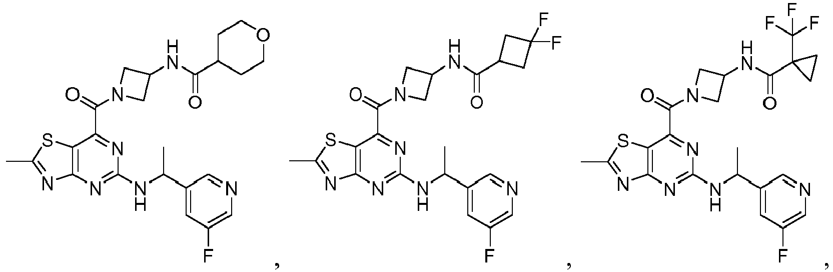 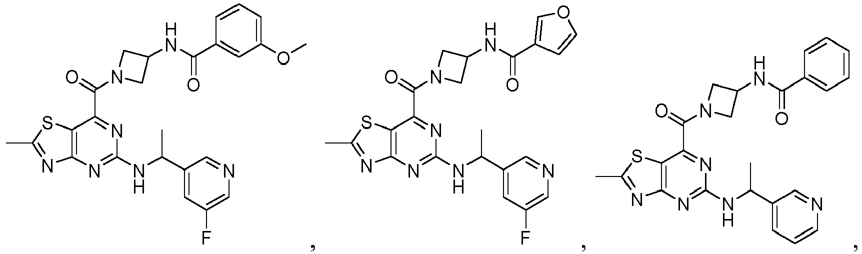 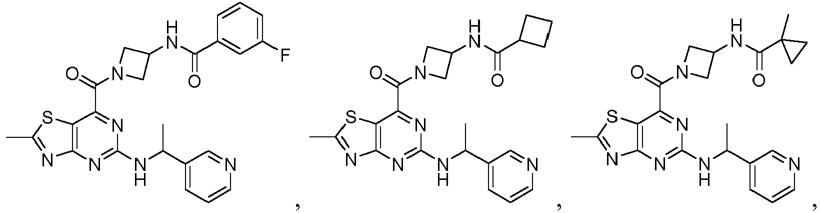 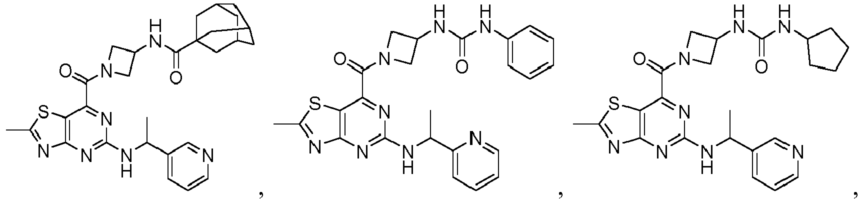 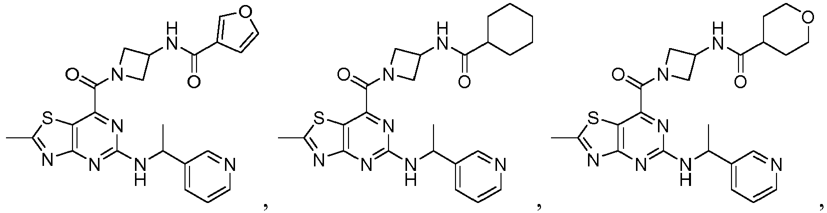 or 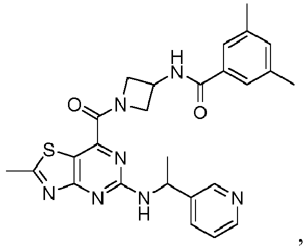 or a pharmaceutically acceptable salt thereof.
Clause 60. The compound of clause 1, wherein the compound is: 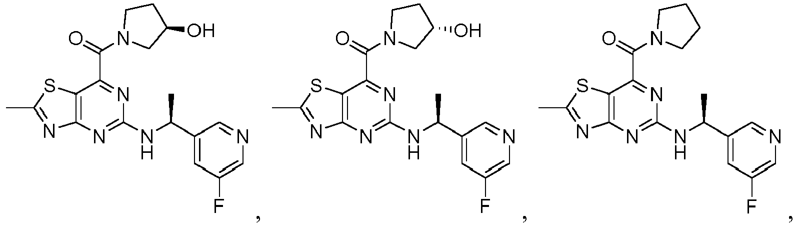 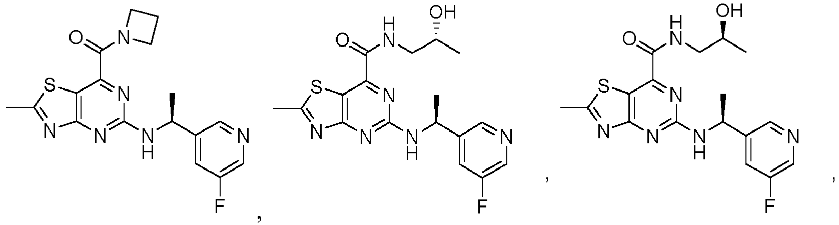 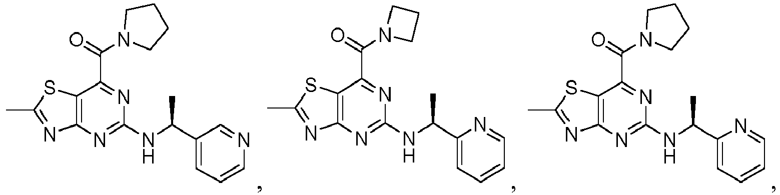 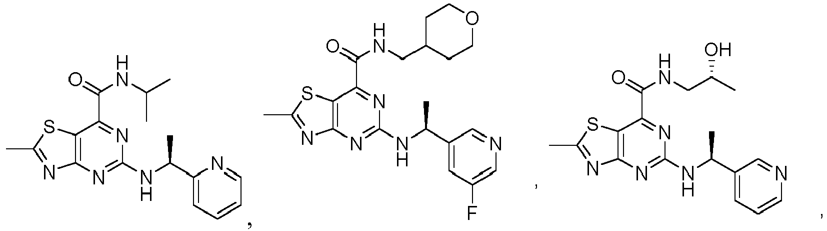 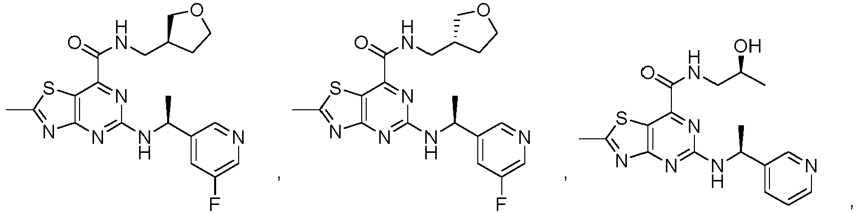 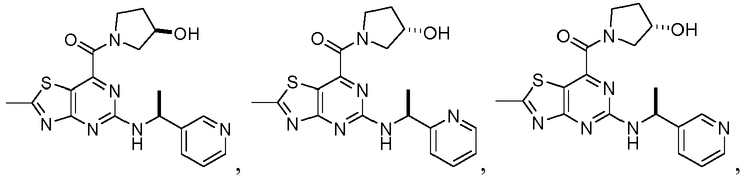 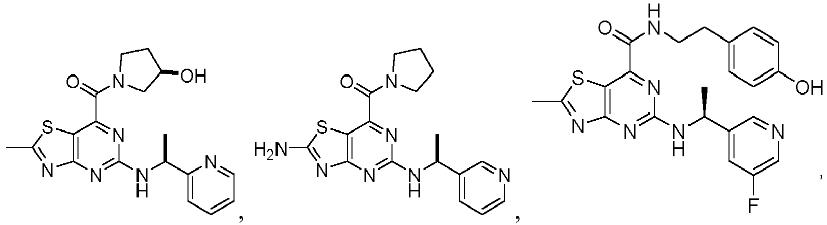 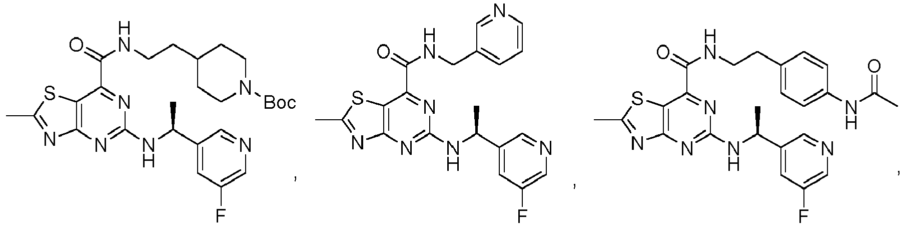 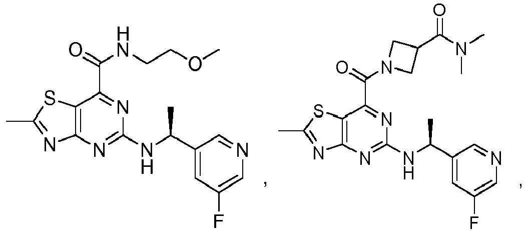 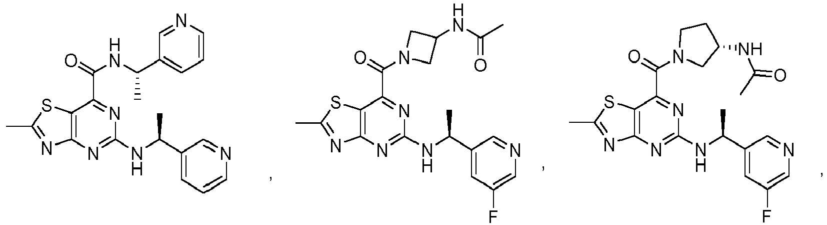 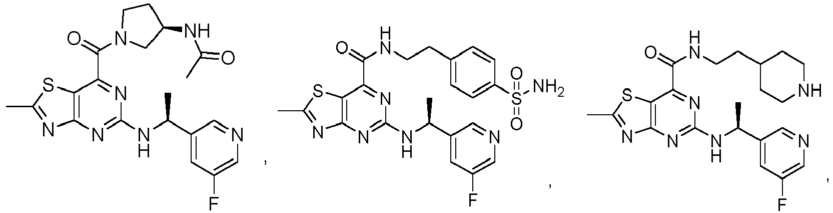 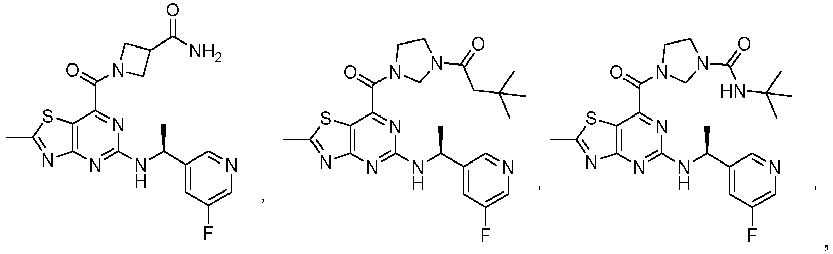 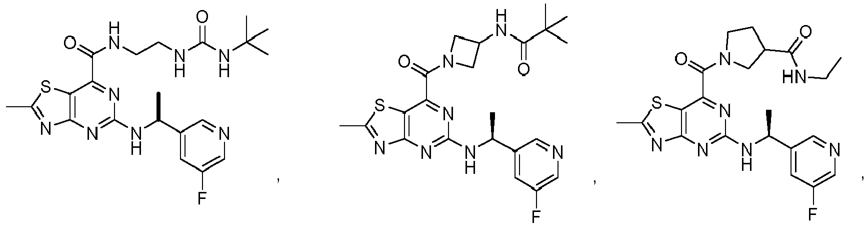 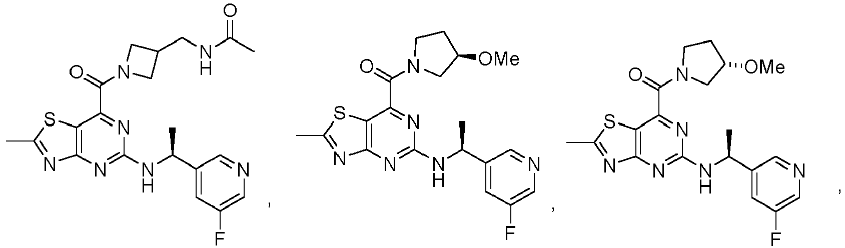 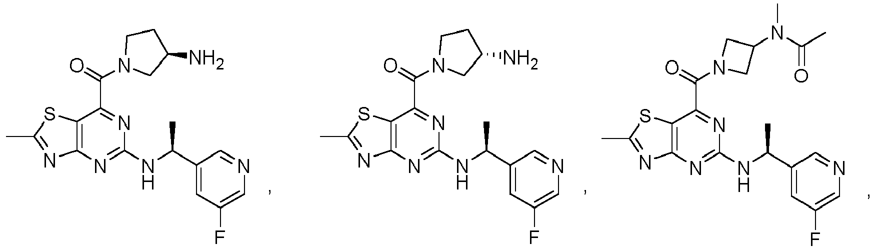 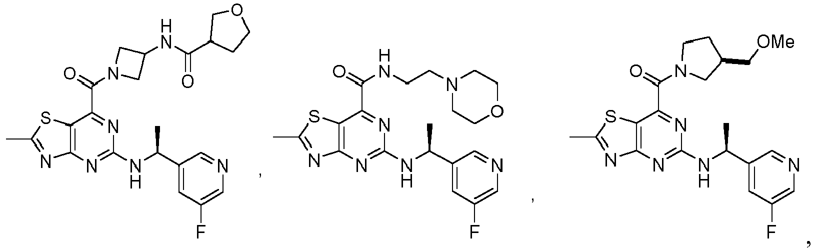 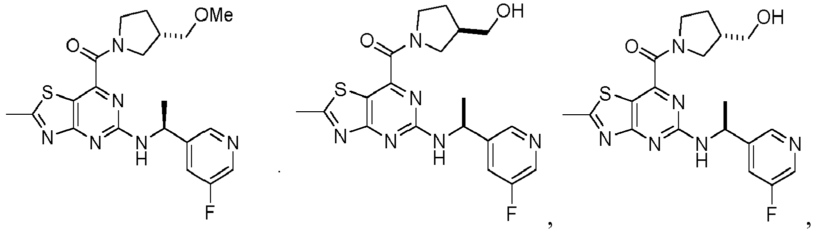 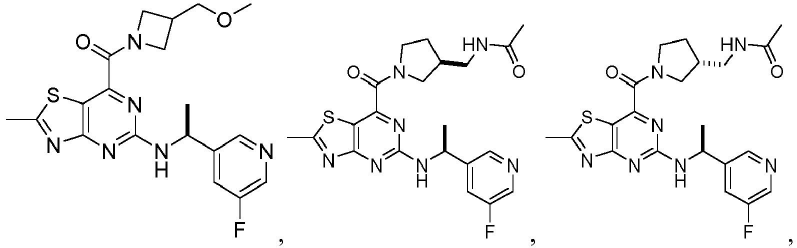 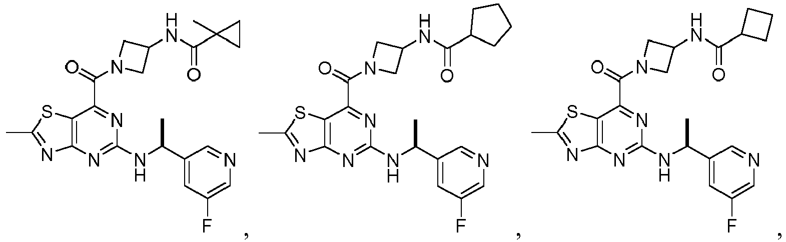 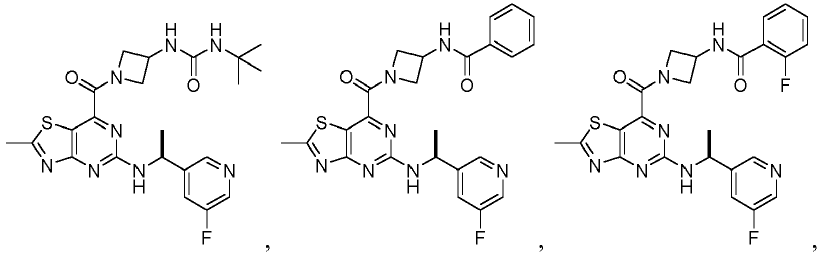 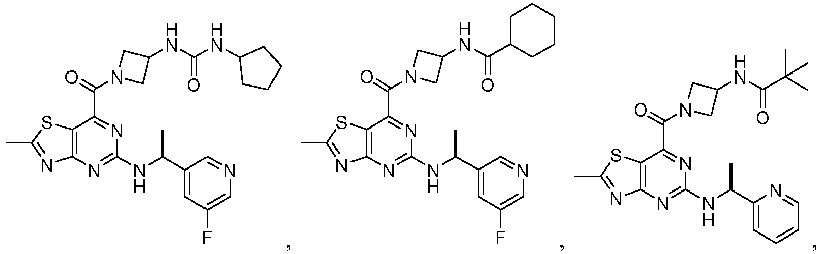 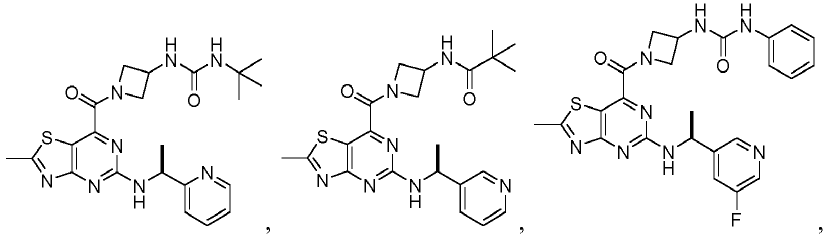 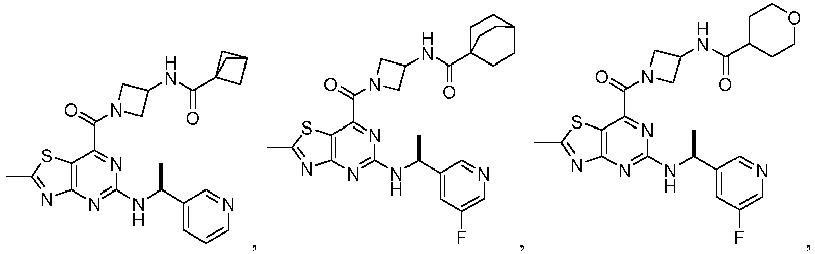 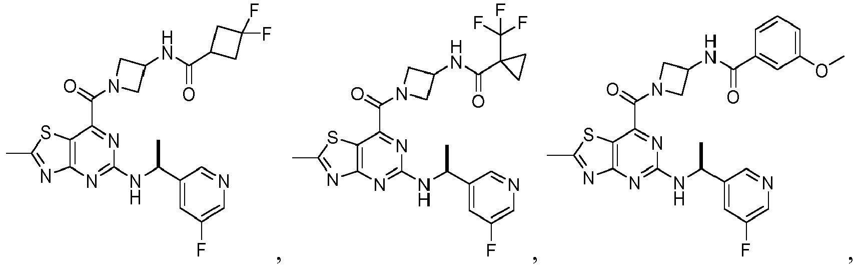 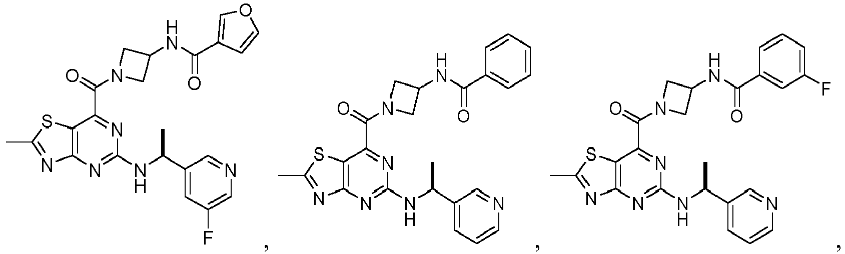 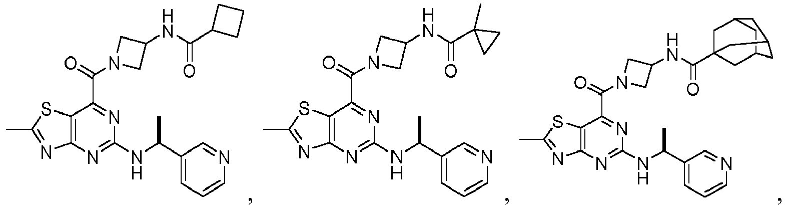 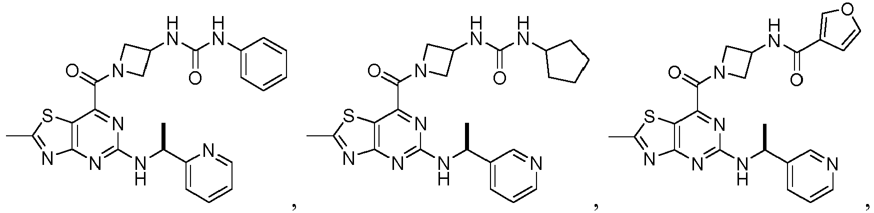 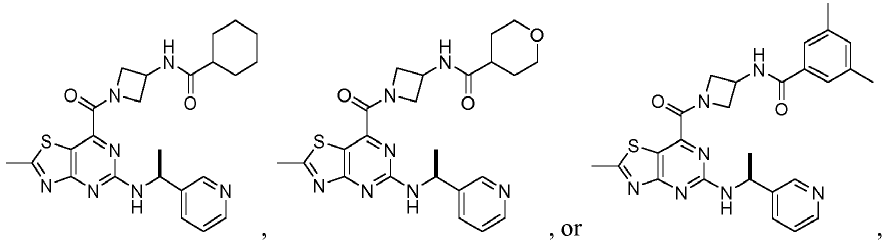 or a pharmaceutically acceptable salt thereof.
Clause 61. The compound of clause 1, wherein the compound is: 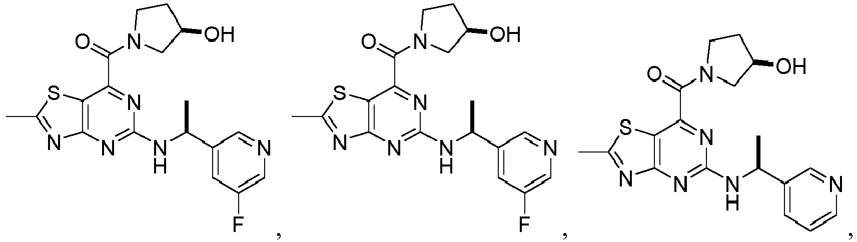 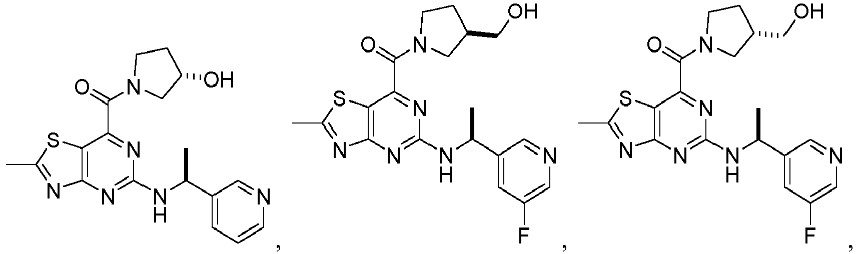 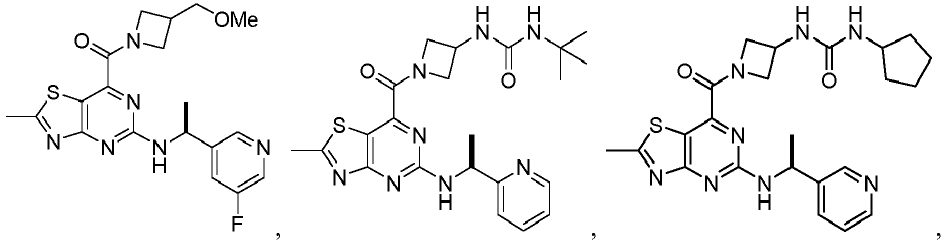 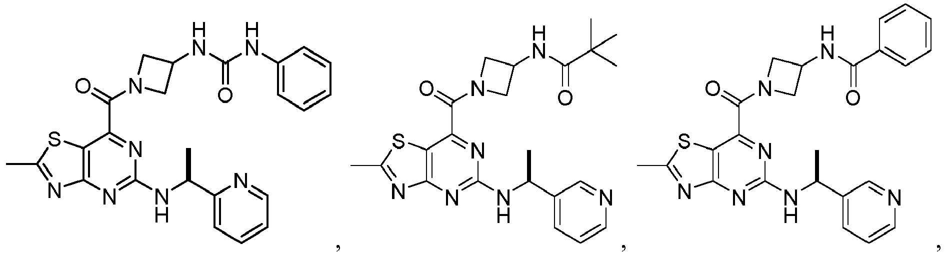 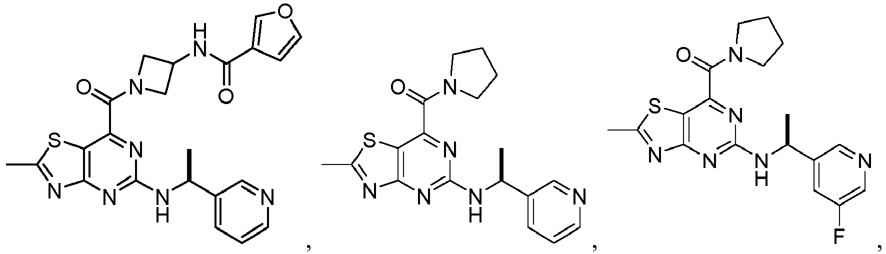 or 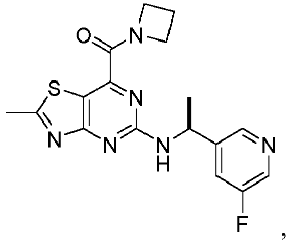 or a pharmaceutically acceptable salt thereof.
Clause 62. The compound of clause 1, wherein the compound is: 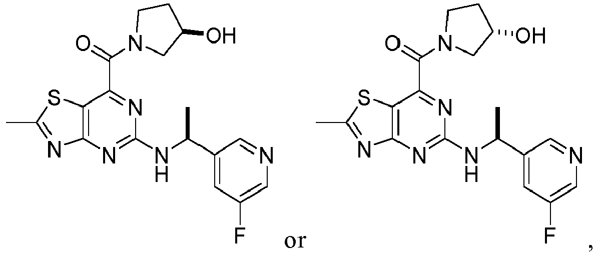 or a pharmaceutically acceptable salt thereof.
Clause 63. A pharmaceutical composition comprising the compound of any one of clauses 1 to 62, or a pharmaceutically acceptable salt thereof, and a pharmaceutically acceptable excipient.
Clause 64. A compound of any one of clauses 1 to 62, or a pharmaceutically acceptable salt thereof, for use as a medicament.
Clause 65. A method of inhibiting Adenosine A2B Receptor activity and Adenosine A2A Receptor activity, said method comprising contacting the Adenosine A2B Receptor and/or Adenosine A2A Receptor with a compound of any one of clauses 1 to 62, or a pharmaceutically acceptable salt thereof;
   optionally wherein said method is an *ex-vivo* method.
Clause 66. A method of inhibiting Adenosine A2B Receptor activity, said method comprising contacting the Adenosine A2B Receptor with a compound of any one of clauses 1 to 62, or a pharmaceutically acceptable salt thereof;
   optionally wherein said method is an *ex-vivo* method.
Clause 67. A compound of any one of clauses 1 to 62, or a pharmaceutically acceptable salt thereof, for use in the treatment of a cancer.
Clause 68. The compound for the use of claim 67, wherein the cancer is breast cancer, lung cancer, colon cancer, bladder cancer, kidney cancer, liver cancer, pancreatic cancer, melanoma, leukemia, lymphoma, or prostate cancer.
Clause 69. A compound of any one of clauses 1 to 62, or a pharmaceutically acceptable salt thereof, for use a disease or disorder associated with Adenosine A2B Receptor activity and/or Adenosine A2A Receptor activity.
Clause 70. The compound for the use of clause 69, wherein the disease or disorder is a cancer.
Clause 71. The compound for the use of clause 70, wherein the cancer is breast cancer, lung cancer, colon cancer, bladder cancer, kidney cancer, liver cancer, pancreatic cancer, melanoma, leukemia, lymphoma, or prostate cancer.
Clause 72. The compound for the use of clause 69, wherein the disease or disorder is a neurodegenerative disorder.
Clause 73. The compound for the use of clause 69, wherein the disease or disorder is a fibrotic disorder.
Clause 74. A compound of any one of clauses 1 to 62, or a pharmaceutically acceptable salt thereof, for use in the treatment of a cancer, neurodegenerative disorder, or fibrotic disorder; optionally wherein the cancer is breast cancer, lung cancer, colon cancer, bladder cancer, kidney cancer, liver cancer, pancreatic cancer, melanoma, leukemia, lymphoma, or prostate cancer.

### EXAMPLES

Unless otherwise stated, all compounds were obtained from commercial sources or were prepared using the methods and experimental procedures described herein. The following Abbreviations are used to refer to various reagents and solvents:
AcOH Acetic acid
n-BuOH n-Butanol
DCM Dichloromethane
DIEA *N*,*N*-Diisopropylethylamine
DMF *N*,*N-*Dimethylformamide
DMSO Dimethylsulfoxide
ESI MS Electrospray ionization mass spectrometry
HATU 1-[Bis(dimethylamino)methylene]-1*H*-1,2,3-triazo[4,5-*b*]pyridinium 3-oxid hexafluorophosphate
KMnO₄ Potassium permanganate
MeOH Methanol
NMP *N*-Methyl-2-pyrrolidone
NMR Nuclear magnetic resonance
NaIO₄ Sodium periodate
Pd(PPh₃)₂Cl₂ Bis(triphenylphosphine)palladium(II) dichloride
TEA Triethylamine
TFA Trifluoroacetic acid
THF Tetrahydrofuran
TLC Thin Layer Chromatography
T3P 2,4,6-Tripropyl-1,3,5,2,4,6-trioxatriphosphorinane-2,4,6-trioxide

### EXPERIMENTAL SYNTHESIS AND CHARACTERIZATION

### Example 1. (S)-(5-((1-(5-Fluoropyridin-3-yl)ethyl)amino)-2-methylthiazolo[4,5-d]pyrimidin-7-yl)(pyrrolidin-1-yl)methanone

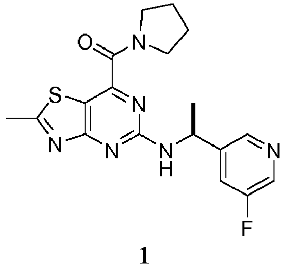

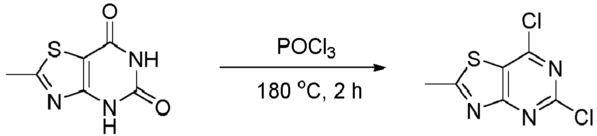

**Synthesis of 5,7-dichloro-2-methylthiazolo[4,5-*d*]pyrimidine:** A solution of 2-methylthiazolo[4,5-*d*]pyrimidine-5,7(4*H*,6*H*)-dione (3.7 g, 20.2 mmol) (commercially obtained from Ark Pharm, Arlington Heights, IL) in phosphoryl trichloride (40 mL) was stirred for 2 h at 180 °C. The reaction was then quenched by addition of 80 mL of water. The resulting solution was extracted with ethyl acetate and the combined organic layer was dried over anhydrous sodium sulfate and concentrated under vacuum. The residue was purified by flash chromatography on silica gel eluting with 20% ethyl acetate in petroleum ether to provide 5,7-dichloro-2-methylthiazolo[4,5-d]pyrimidine (3.5 g, 79% yield) as an yellow solid. ¹H NMR (400 MHz, DMSO-*d*₆) δ 3.00 (3H, s) ppm. ESI MS: m/z [M+H⁺] for C₆H₃Cl₂N₃S, calcd 220.0, found 220.0.

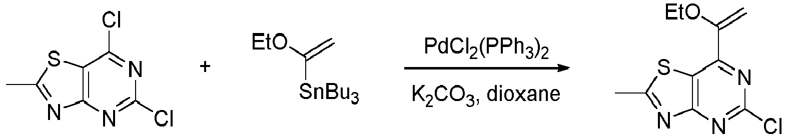

**Synthesis of 5-chloro-7-(1-ethoxyvinyl)-2-methylthiazolo[4,5-*d*]pyrimidine:** To a solution of 5,7-dichloro-2-methylthiazolo[4,5-*d*]pyrimidine (600 mg, 2.73 mmol) in dioxane (20 mL) were added tributyl(1-ethoxyethenyl)stannane (1 g, 2.77 mmol)) (commercially obtained from Sigma-Aldrich, St. Louis, MO), a solution of potassium carbonate (756 mg, 5.47 mmol) in water (4 mL), and Pd(PPh₃)₂Cl₂ (96 mg, 0.14 mmol). The reaction mixture thus obtained was stirred for 1 h at 100 °C. The reaction mixture was concentrated and extracted with ethyl acetate. The combined organic layer was dried over anhydrous sodium sulfate and concentrated under vacuum. The residue was purified by flash chromatography on silica gel eluting with 10% ethyl acetate in petroleum ether to provide 5-chloro-7-(1-ethoxyvinyl)-2-methylthiazolo[4,5-*d*]pyrimidine (300 mg, 43% yield) as a red solid. ¹H NMR (400 MHz, DMSO-*d*₆) δ 1.47 (3H, t, *J* = 8.0 Hz), 2.93 (3H, s), 4.09 (2H, q, *J* = 8.0 Hz), 4.92 (1H, d, *J* = 4.0 Hz), 5.78 (1H, *J* = 4.0 Hz) ppm. ESI MS: m/z [M+H⁺] for C₁₀H₁₀ClN₃OS, calcd 256.0, found 256.0.

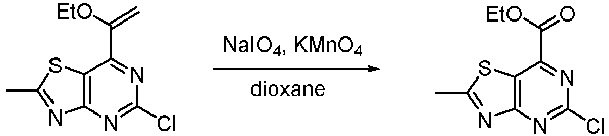

### Synthesis of ethyl 5-chloro-2-methylthiazolo[4,5-d]pyrimidine-7-carboxylate:

To a solution of 5-chloro-7-(1-ethoxyvinyl)-2-methylthiazolo[4,5-*d*]pyrimidine (650 mg, 2.54 mmol) in 1,4-dioxane (24 mL) were added a solution of NaIO₄ (1.09 g, 5.08 mmol) in water(12 mL) and KMnO₄ (80.5 mg, 0.51 mmol). The reaction mixture was stirred at 50 °C for 2 h. The precipitate was filtered off and the residue was rinsed thoroughly with DCM (4 x 20 mL). The combined filtrates were washed with water, dried over MgSO₄ and concentrated in vacuo. The residue was purified by flash chromatography on silica gel eluting with 30% ethyl acetate in petroleum ether to provide ethyl 5-chloro-2-methylthiazolo[4,5-*d*]pyrimidine-7-carboxylate as a white solid (250 mg, 38% yield). ¹H NMR (400 MHz, DMSO-*d*₆) δ 1.41 (3H, t, *J* = 8.0 Hz), 3.01 (3H, s), 4.49 (2H, *q, J =* 8.0 Hz) ppm. ESI MS: m/z [M+H⁺] for C₉H₈ClN₃O₂S, calcd 258.0, found 258.1.

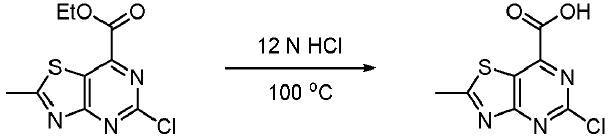

### Synthesis of 5-chloro-2-methylthiazolo[4,5-d]pyrimidine-7-carboxylic acid:

Ethyl 5-chloro-2-methyl-thiazolo[4,5-*d*]pyrimidine-7-carboxylate (1.29 g, 5.0 mmol) was dissolved in 12N HCl (8.3 mL) and stirred at 100 °C for 30 min. The reaction mixture was evaporated to dryness in vacuo at a bath temperature of 60 °C and the residue was dried under high vacuum to provide 5-chloro-2-methylthiazolo[4,5-*d*]pyrimidine-7-carboxylic acid as a beige solid (1.18 g, 100% yield). ¹H NMR (400 MHz, DMSO-*d*₆) δ 2.99 (3H, s) ppm. ESI MS: m/z [M+H⁺] for C₇H₄CN₃O₂S, calcd 230.0, found 230.0.

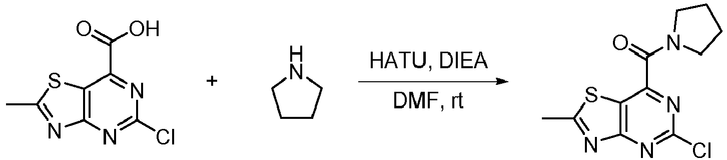

**Synthesis of (5-chloro-2-methylthiazolo[4,5-*d*]pyrimidin-7-yl)(pyrrolidin-1-yl)methanone:** To solution of 5-chloro-2-methylthiazolo[4,5-*d*]pyrimidine-7-carboxylic acid (200 mg, 0.87 mmol) in DMF (10 mL) were added pyrrolidine (62 mg, 0.87 mmol), HATU (661 mg, 1.74 mmol), and DIEA (337 mg, 2.61 mmol). The reaction mixture thus obtained was stirred at room temperature for 2 h. The reaction mixture was diluted with ethyl acetate and washed with 5% NaHCO₃, water, and brine, then dried over MgSO₄. The solvent was evaporated in vacuo and the residue was purified flash chromatography on silica gel eluting with 10% to 50% solvent A (DCM/MeOH/NH4OH, 100/10/1) in DCM to provide (5-chloro-2-methylthiazolo[4,5-*d*]pyrimidin-7-yl)(pyrrolidin-1-yl)methanone as an yellow solid (220 mg, 89% yield). ESI MS: m/z [M+H⁺] for C₁₁H₁₁ClN₄OS, calcd 283.0, found 283.0.

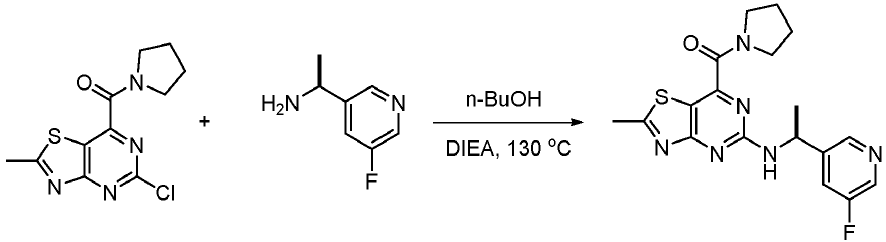

**Synthesis of (S)-(5-((1-(5-fluoropyridin-3-yl)ethyl)amino)-2-methylthiazolo[4,5-*d*]pyrimidin-7-yl)(pyrrolidin-1-yl)methanone (1):** To a solution of (5-chloro-2-methylthiazolo[4,5-*d*]pyrimidin-7-yl)(pyrrolidin-1-yl)methanone (100 mg, 0.35 mmol) in n-butanol (5 mL) were added (1*S*)-1-(5-fluoropyridin-3-yl)ethan-1-amine dihydrochloride (commercially obtained from AA Chempharm, Valley Cottage, NY) (226 mg, 1.06 mmol) and DIEA (229 mg, 1.77 mmol). The reaction mixture thus obtained was stirred at 130 °C for 4 h under microwave irradiations and then was diluted with ethyl acetate and washed with 5% NaHCO₃, water, and brine, then dried over MgSO₄. The solvent was evaporated in vacuo and the residue was purified by prep-HPLC (C-18 OBD column and a gradient elution system of water-acetonitrile) to provide (*S*)-(5-((1-(5-fluoropyridin-3-yl)ethyl)amino)-2-methylthiazolo[4,5-*d*]pyrimidin-7-yl)(pyrrolidin-1-yl)methanone as a white solid (15 mg, 11% yield). ¹H NMR (300 MHz, methanol-*d*₄) δ 1.64 (3H, d, *J* = 7.2 Hz), 1.85-1.98 (4H, m), 2.84 (3H, s), 3.60-3.66 (2H, m), 3.98-4.04 (2H, m), 5.28 (1H, q, *J* = 7.2 Hz), 7.43 (1H, dt, *J* = 9.6, 2.0 Hz), 8.33 (1H, d, *J* = 2.7 Hz), 8.33 (1H, s) ppm. ESI MS: m/z [M+H⁺] for C₁₈H₁₉FN₆OS, calcd 387.1, found 387.1.

### Example 2. (S)-Azetidin-1-yl(5-((1-(5-fluoropyridin-3-yl)ethyl)amino)-2-methylthiazolo[4,5-d]pyrimidin-7-yl)methanone

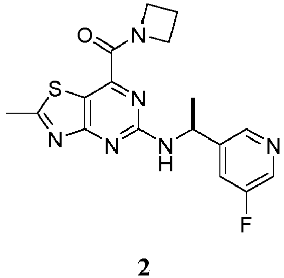

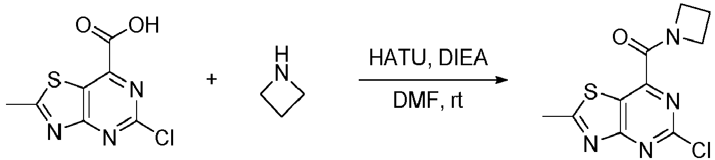

**Synthesis of azetidin-1-yl(5-chloro-2-methylthiazolo[4,5-*d*]pyrimidin-7-yl)methanone:** To a solution of 5-chloro-2-methylthiazolo[4,5-*d*]pyrimidine-7-carboxylic acid (200 mg, 0.87 mmol) in DMF (10 mL) were added azetidine hydrochloride (121.4 mg, 1.30 mmol), HATU (661.2 mg, 1.74 mmol), and DIEA (336.7 mg, 2.61 mmol). The resulting solution was stirred for 1 h at room temperature. The reaction mixture was extracted with ethyl acetate and the combined organic layer was dried over anhydrous sodium sulfate and then concentrated under vacuum to provide azetidin-1-yl(5-chloro-2-methylthiazolo[4,5-*d*]pyrimidin-7-yl)methanone as an yellow solid (220 mg, 94% yield). ESI MS: m/z [M+H⁺] for C₁₀H₉ClN₄OS, calcd 269.0, found 269.0.

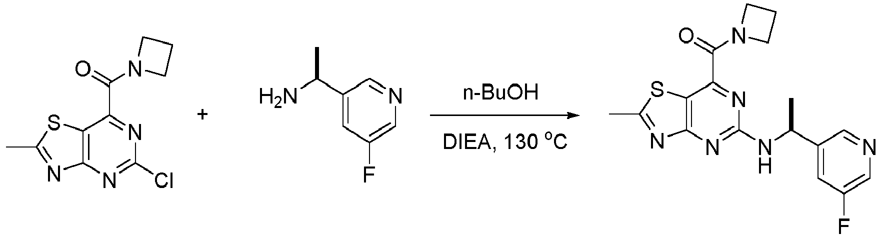

**Synthesis of (*S*)-azetidin-1-yl(5-((1-(5-fluoropyridin-3-yl)ethyl)amino)-2-methylthiazolo[4,5-*d*]pyrimidin-7-yl)methanone (2):** The title compound (**2**) was prepared from azetidin-1-yl(5-chloro-2-methylthiazolo[4,5-*d*]pyrimidin-7-yl)methanone (100 mg, 0.37 mmol) using chemistry similar to that described in Example 1 (17 mg, 12% yield). ¹H NMR (300 MHz, methanol-*d*₄) δ 1.65 (3H, d, *J* = 7.2 Hz), 2.41-2.48 (2H, m), 2.83 (3H, s), 4.24 (2H, t, *J* = 7.8 Hz), 4.88 (2H, m), 5.31 (1H, q, *J* = 7.2 Hz), 7.73 (1H, dt, *J* = 9.6, 2.4 Hz), 8.33 (1H, d, *J* = 2.4 Hz), 8.52 (1H, s) ppm. ESI MS: m/z [M+H⁺] for C₁₇H₁₇FN₆OS, calcd 373.1, found 373.1.

### Example 3. 5-(((S)-1-(5-Fluoropyridin-3-yl)ethyl)amino)-N-((R)-2-hydroxypropyl)-2-methylthiazolo[4,5-d]pyrimidine-7-carboxamide

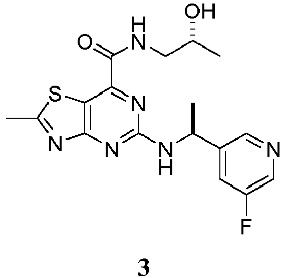

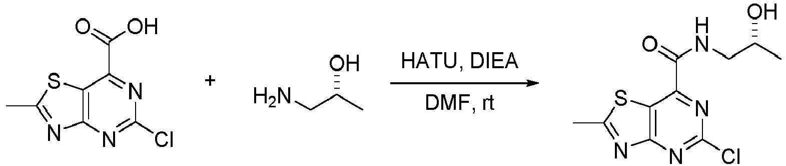

**Synthesis of (*R*)-5-chloro-N-(2-hydroxypropyl)-2-methylthiazolo[4,5-*d*]pyrimidine-7-carboxamide:** To a solution of 5-chloro-2-methylthiazolo[4,5-d]pyrimidine-7-carboxylic acid (240 mg, 1.05 mmol) in DMF (4 mL) were added (2*R*)-1-aminopropan-2-ol (94.4 mg, 1.26 mmol) (commercially obtained from AK Scientific, Union City, CA), HATU (796.5 mg, 2.09 mmol), and DIEA (405.6 mg, 3.14 mmol). The resulting solution was stirred for 2 h at room temperature. The reaction mixture was diluted with ethyl acetate and washed with 5% NaHCO₃, water, and brine, then dried over anhydrous sodium sulfate and concentrated under vacuum. The residue was purified by flash column chromatography on silica gel eluting with 30% ethyl acetate in petroleum ether to provide (*R*)-5-chloro-*N*-(2-hydroxypropyl)-2-methylthiazolo[4,5-*d*]pyrimidine-7-carboxamide as a yellow solid (120 mg, 40% yield). ESI MS: m/z [M+H⁺] for C₁₀H₁₁ClN₄O₂S, calcd 287.0, found 287.0.

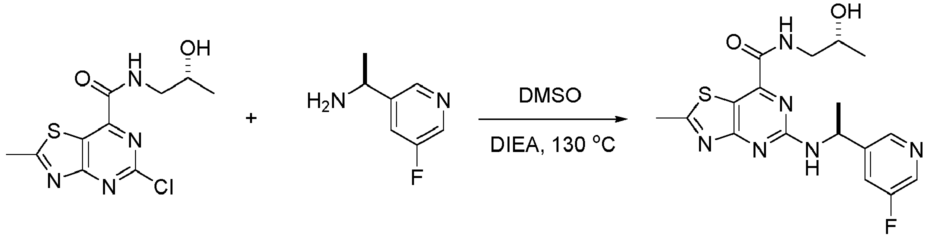

**Synthesis of 5-(((*S*)-1-(5-fluoropyridin-3-yl)ethyl)amino)-*N*-((*R*)-2-hydroxypropyl)-2-methylthiazolo[4,5-*d*]pyrimidine-7-carboxamide (3):** To a solution of (*R*)-5-chloro-*N*-(2-hydroxypropyl)-2-methylthiazolo[4,5-*d*]pyrimidine-7-carboxamide (120 mg, 0.42 mmol) in DMSO (2 mL) where added (1*S*)-1-(5-fluoropyridin-3-yl)ethan-1-amine dihydrochloride (264 mg, 1.24 mmol) and DIEA (276 mg, 2.14 mmol) and the resulting solution was stirred for 2 h at 130 °C. The reaction mixture was diluted with ethyl acetate and washed with 5% NaHCO₃, water, and brine, then dried over anhydrous sodium sulfate and concentrated under vacuum. The residue was purified by prep-HPLC (C-18 OBD column and a gradient elution system of water-acetonitrile) to provide 5-(((*S*)-1-(5-fluoropyridin-3-yl)ethyl)amino)-*N*-((*R*)-2-hydroxypropyl)-2-methylthiazolo[4,5-*d*]pyrimidine-7-carboxamide as a light yellow solid (11 mg, 7% yield). ¹H NMR (300 MHz, MeOD) δ 1.23 (3H, d, *J* = 6.3 Hz), 1.65 (3H, d, *J* = 7.2 Hz), 2.85 (3H, s), 3.34 (1H, m), 3.53 (1H, dd, *J* = 8.4, 4.2 Hz), 3.97 (1H, m), 5.36 (1H, q, *J* = 7.2 Hz), 7.76 (1H, dt, *J* = 9.2, 2.1 Hz), 8.32 (1H, d, *J* = 2.7 Hz), 8.56 (1H, s) ppm. ESI MS: m/z [M+H⁺] for C₁₇H₁₉FN₆O₂S, calcd 391.1, found 391.2.

### Example 4. (5-(((S)-1-(5-Fluoropyridin-3-yl)ethyl)amino)-2-methylthiazolo[4,5-d]pyrimidin-7-yl)((S)-3-hydroxypyrrolidin-1-yl)methanone

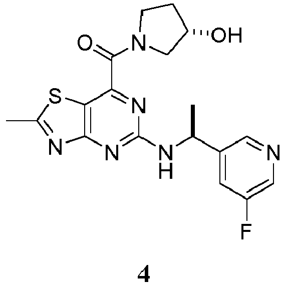

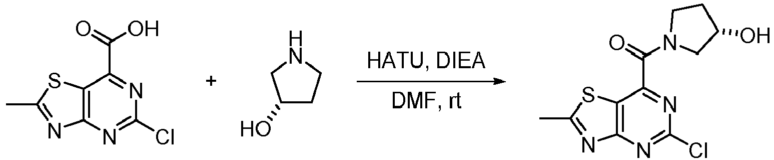

**Synthesis of (*S*)-(5-chloro-2-methylthiazolo[4,5-*d*]pyrimidin-7-yl)(3-hydroxypyrrolidin-1-yl)methanone:** To a solution of 5-chloro-2-methylthiazolo[4,5-d]pyrimidine-7-carboxylic acid (300 mg, 1.31 mmol) in DMF (15 mL) were added (3*S*)-pyrrolidin-3-ol hydrochloride (195 mg, 1.58 mmol) (commercially obtained from AK Scientific, Union City, CA), HATU (996 mg, 2.62 mmol), and DIEA (510 mg, 3.95 mmol). The resulting solution was stirred for 2 h at room temperature and the reaction mixture was extracted with ethyl acetate. The combined organic layer was dried over anhydrous sodium sulfate and concentrated under vacuum to provide (*S*)-(5-chloro-2-methylthiazolo[4,5-*d*]pyrimidin-7-yl)(3-hydroxypyrrolidin-1-yl)methanone (250 mg, 64% yield) as a brown oil which was used in next step without further purification. ESI MS: m/z [M+H⁺] for C₁₁H₁₁ClN₄O₂S, calcd 299.0, found 299.0.

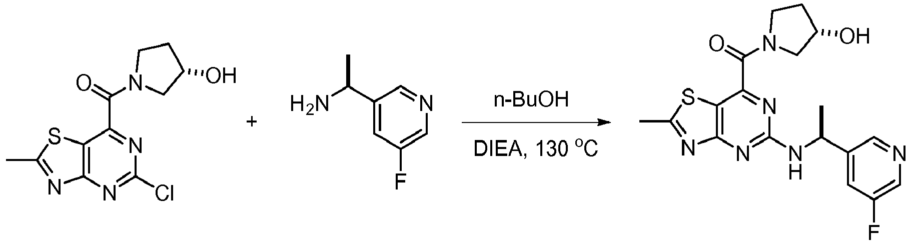

**Synthesis of (5-(((S)-1-(5-fluoropyridin-3-yl)ethyl)amino)-2-methylthiazolo[4,5-*d*]pyrimidin-7-yl)((*S*)-3-hydroxypyrrolidin-1-yl)methanone (4):** The title compound (**4**) was prepared from (S)-(5-chloro-2-methylthiazolo[4,5-*d*]pyrimidin-7-yl)(3-hydroxypyrrolidin-1-yl)methanone (100 mg, 0.33 mmol) using chemistry similar to that described in Example 1 (11 mg, 11% yield). ¹H NMR (300 MHz, methanol-*d*₄) δ 1.65 (3H, d, *J=* 7.2 Hz), 1.98-2.08 (2H, m), 2.84 (3H, s), 3.50 (1H, m), 3.64-3.78 (2H, m), 3.97-4.06 (2H, m), 4.45 (1H, m), 5.30 (1H, q, *J* = 7.2 Hz), 7.74 (1H, dt, *J* = 9.6, 2.0 Hz), 8.33 (1H, d, *J* = 2.0 Hz), 8.53 (1H, d, *J* = 2.0 Hz) ppm. ESI MS: m/z [M+H⁺] for C₁₈H₁₉FN₆O₂S, calcd 403.1, found 403.3.

### Example 5.5-(((S)-1-(5-Fluoropyridin-3-yl)ethyl)amino)-N-((S)-2-hydroxypropyl)-2-methylthiazolo[4,5-d]pyrimidine-7-carboxamide

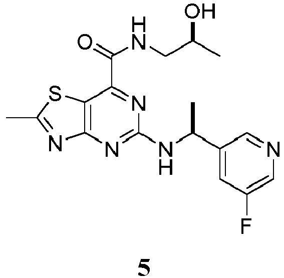

**Synthesis of 5-(((*S*)-1-(5-fluoropyridin-3-yl)ethyl)amino)-*N*-((*S*)-2-hydroxypropyl)-2-methylthiazolo[4,5-*d*]pyrimidine-7-carboxamide (5):** The title compound (5) was prepared using chemistry similar to that described in Example 3. ¹H NMR (300 MHz, MeOD) 8 1.23 (3H, d, *J* = 6.3 Hz), 1.65 (3H, d, *J* = 7.2 Hz), 2.85 (3H, s), 3.36 (1H, m), 3.53 (1H, dd, *J* = 8.4, 4.2 Hz), 3.97 (1H, m), 5.36 (1H, q, *J* = 7.2 Hz), 7.76 (1H, dt, *J* = 9.2, 2.1 Hz), 8.32 (1H, d, *J* = 2.1 Hz), 8.56 (1H, d, *J* = 2.1 Hz) ppm. ESI MS: m/z [M+H⁺] for C₁₇H₁₉FN₆O₂S, calcd 391.1, found 391.1.

### Example 6. (S)-(2-Methyl-5-((1-(pyridin-3-yl)ethyl)amino)thiazolo[4,5-d]pyrimidin-7-yl)(pyrrolidin-1-yl)methanone

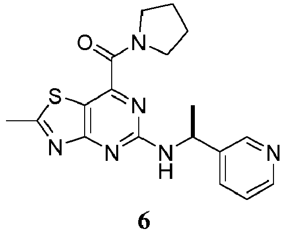

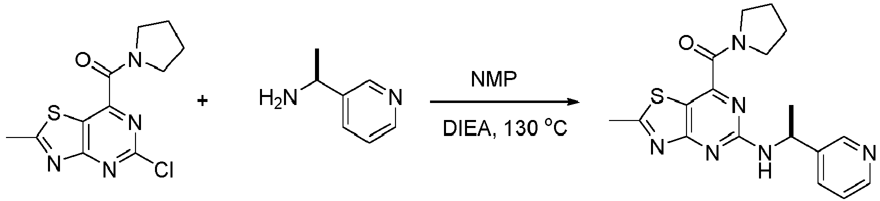

**Synthesis of (*S*)-(2-methyl-5-((1-(pyridin-3-yl)ethyl)amino)thiazolo[4,5-*d*]pyrimidin-7-yl)(pyrrolidin-1-yl)methanone (6):** The title compound (**6**) was prepared from (5-chloro-2-methylthiazolo[4,5-*d*]pyrimidin-7-yl)(pyrrolidin-1-yl)methanone (100 mg, 0.35 mmol) using chemistry similar to that described in Example 1 (13 mg, 10% yield). ¹H NMR (300 MHz, methanol-*d*₄) δ 1.64 (3H, d, *J* = 7.2 Hz), 1.85-1.98 (4H, m), 2.84 (3H, s), 3.60-3.66 (2H, m), 3.98-4.04 (2H, m), 5.28 (1H, q, *J* = 7.2 Hz), 7.42 (1H, m), 7.95 (1H, m), 8.41 (1H, m), 8.66 (1H, m) ppm. ESI MS: m/z [M+H⁺] for C₁₈H₂₀N₆OS, calcd 369.1, found 369.2.

### Example 7. (5-(((S)-1-(5-Fluoropyridin-3-yl)ethyl)amino)-2-methylthiazolo[4,5-d]pyrimidin-7-yl)((R)-3-hydroxypyrrolidin-1-yl)methanone

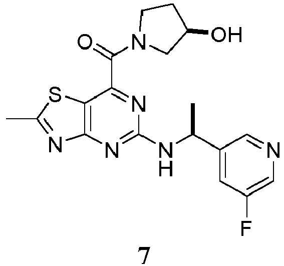

**Synthesis of (5-(((*S*)-1-(5-fluoropyridin-3-yl)ethyl)amino)-2-methylthiazolo[4,5-*d*]pyrimidin-7-yl)((*R*)-3-hydroxypyrrolidin-1-yl)methanone (7):** The title compound (**7**) was prepared using chemistry similar to that described in Example 4. ¹H NMR (300 MHz, methanol-*d*₄) δ 1.65 (3H, d, *J* = 7.2 Hz), 1.98-2.08 (2H, m), 2.83 (3H, s), 3.70 (1H, m), 3.79 (1H, m), 4.22 (1H, m), 4.66 (1H, m), 5.30 (1H, q, *J* = 7.2 Hz), 7.74 (1H, dt, *J* = 9.6, 2.0 Hz), 8.33 (1H, d, *J* = 2.0 Hz), 8.53 (1H, d, *J* = 2.0 Hz) ppm. ESI MS: m/z [M+H⁺] for C₁₈H₁₉FN₆O₂S, calcd 403.1, found 403.0.

### Example 8. (S)-Azetidin-1-yl(2-methyl-5-((1-(pyridin-2-yl)ethyl)amino)thiazolo[4,5-d]pyrimidin-7-yl)methanone

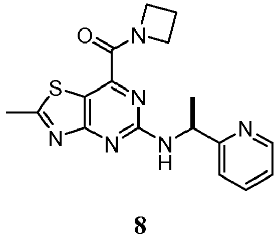

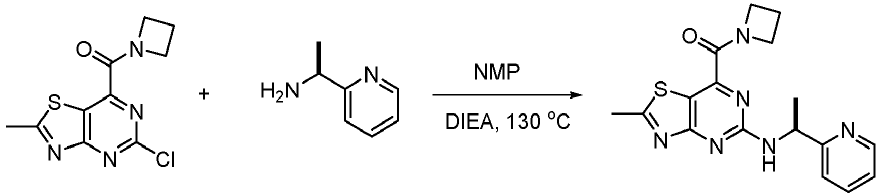

**Synthesis of (*S*)-azetidin-1-yl(2-methyl-5-((1-(pyridin-2-yl)ethyl)amino)thiazolo[4,5-*d*]pyrimidin-7-yl)methanone (8):** The title compound (**8**) was prepared from azetidin-1-yl(5-chloro-2-methylthiazolo[4,5-*d*]pyrimidin-7-yl)methanone (100 mg, 0.37 mmol) using chemistry similar to that described in Example 1 (40 mg, 30% yield). ¹H NMR (300 MHz, methanol-*d*₄) δ 1.63 (3H, d, *J* = 7.2 Hz), 2.37-2.42 (2H, m), 2.83 (3H, s), 4.18-4.22 (2H, m), 4.65-4.88 (2H, m), 5.20 (1H, q, *J* = 7.2 Hz), 7.29 (1H, m), 7.51 (1H, d, *J* = 7.8 Hz), 7.78 (1H, td, *J* = 7.8, 1.8 Hz), 8.51 (1H, m) ppm. ESI MS: m/z [M+H⁺] for C₁₇H₁₈N₆OS, calcd 355.1, found 355.1.

### Example 9. (S)-(2-Methyl-5-((1-(pyridin-2-yl)ethyl)amino)thiazolo[4,5-d]pyrimidin-7-yl)(pyrrolidin-1-yl)methanone

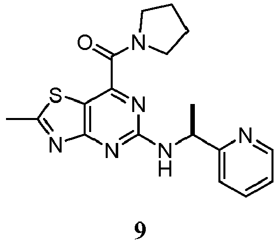

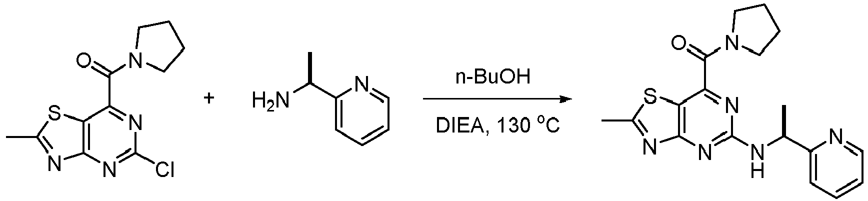

**Synthesis of (*S*)-(2-methyl-5-((1-(pyridin-2-yl)ethyl)amino)thiazolo[4,5-*d*]pyrimidin-7-yl)(pyrrolidin-1-yl)methanone (9):** The title compound (**9**) was prepared from (5-chloro-2-methylthiazolo[4,5-*d*]pyrimidin-7-yl)(pyrrolidin-1-yl)methanone (100 mg, 0.35 mmol) using chemistry similar to that described in Example 1 using (*S*)-1-(pyridin-2-yl)ethan-1-amine (commercially obtained from Ark Pharm, Arlington Heights, IL) in place of (*S*)-1-(5-fluoropyridin-3-yl)ethan-1-amine (41 mg, 31% yield). ¹H NMR (300 MHz, methanol-*d*₄) δ 1.63 (3H, d, *J* = 6.8 Hz), 1.80-2.00 (4H, m), 2.83 (3H, s), 3.56-3.61 (4H, m), 5.18 (1H, q, *J* = 6.8 Hz), 7.29 (1H, m), 7.52 (1H, d, *J* = 7.8 Hz), 7.77 (1H, td, *J* = 7.8, 1.8 Hz), 8.52 (1H, m) ppm. ESI MS: m/z [M+H⁺] for C₁₈H₂₀N₆OS, calcd 369.1, found 369.1.

### Example 10. (S)-N-Isopropyl-2-methyl-5-((1-(pyridin-2-yl)ethyl)amino)thiazolo[4,5-d]pyrimidine-7-carboxamide

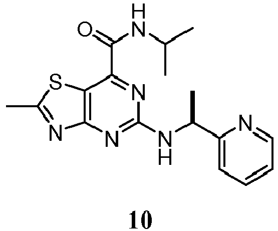

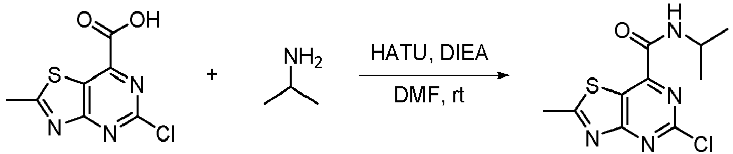

**Synthesis of 5-chloro-N-isopropyl-2-methylthiazolo[4,5-*d*]pyrimidine-7-carboxamide:** To a solution of 5-chloro-2-methylthiazolo[4,5-*d*]pyrimidine-7-carboxylic acid (100 mg, 0.44 mmol) in DMF (4 mL) were added propan-2-amine hydrochloride (68.6 mg, 0.72 mmol), HATU (332 mg, 0.87 mmol), DIEA (170 mg, 1.32 mmol) and the reaction mixture was stirred for 2 h at room temperature. The reaction mixture was extracted with ethyl acetate and the combined organic layer was dried over anhydrous sodium sulfate and concentrated under vacuum to provide 5-chloro-*N*-isopropyl-2-methylthiazolo[4,5-*d*]pyrimidine-7-carboxamide as a brown solid (100 mg, 85% yield). ESI MS: m/z [M+H⁺] for C₁₀H₁₁ClN₄OS, calcd 271.0, found 271.2.

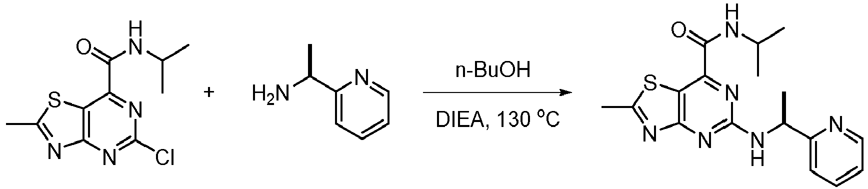

**Synthesis of (*S*)-*N*-isopropyl-2-methyl-5-((1-(pyridin-2-yl)ethyl)amino)thiazolo[4,5-*d*]pyrimidine-7-carboxamide (10):** The title compound (**10**) was prepared from 5-chloro-*N*-isopropyl-2-methylthiazolo[4,5-*d*]pyrimidine-7-carboxamide (90 mg, 0.33 mmol) using chemistry similar to that described in Example 1 using (*S*)-1-(pyridin-2-yl)ethan-1-amine in place of (*S*)-1-(5-fluoropyridin-3-yl)ethan-1-amine (38 mg, 32% yield). ¹H NMR (400 MHz, methanol-*d*₄) δ 1.25 (3H, d, J = 6.8 Hz), 1.31 (3H, d, J = 6.8 Hz), 1.63 (3H, d, *J* = 6.8 Hz), 2.83 (3H, s), 4.17 (1H, m), 5.23 (1H, q, *J* = 6.8 Hz), 7.29 (1H, m), 7.52 (1H, dd, *J* = 6.8, 5.2 Hz), 7.54 (1H, d, J = 8.0 Hz), 7.78 (1H, td, *J* = 9.6, 1.6 Hz), 8.52 (1H, m) ppm. ESI MS: m/z [M+H⁺] for C₁₇H₂₀N₆OS, calcd 357.1, found 357.1.

### Example 11. (S)-5-((1-(5-Fluoropyridin-3-yl)ethyl)amino)-2-methyl-N-((tetrahydro-2H-pyran-4-yl)methyl)thiazolo[4,5-d]pyrimidine-7-carboxamide

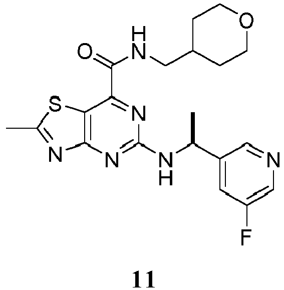

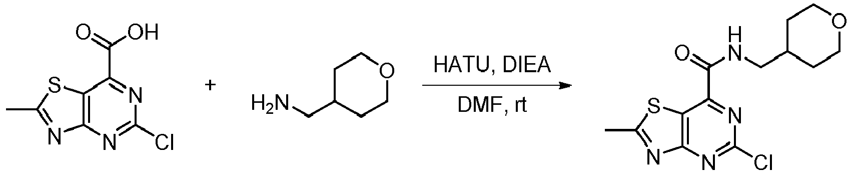

**Synthesis of 5-chloro-2-methyl-*N*-((tetrahydro-2*H*-pyran-4-yl)methyl)thiazolo[4,5-*d*]pyrimidine-7-carboxamide:** To a solution of 5-chloro-2-methylthiazolo[4,5-*d*]pyrimidine-7-carboxylic acid (200 mg, 0.87 mmol) in DMF (10 mL) were added oxan-4-ylmethanamine (120 mg, 1.04 mmol) (commercially obtained from AK Scientific, Union City, CA), HATU (664 mg, 1.75 mmol), and DIEA (338 mg, 2.62 mmol), and the reaction mixture was stirred for 2 h at room temperature. The reaction mixture was extracted with ethyl acetate and the combined organic layer was dried over anhydrous sodium sulfate and concentrated under vacuum. The residue was purified by flash chromatography on silica gel eluting with ethyl acetate/petroleum ether to provide 5-chloro-2-methyl-*N-*((tetrahydro-2*H*-pyran-4-yl)methyl)thiazolo[4,5-*d*]pyrimidine-7-carboxamide as an yellow solid (65 mg, 23% yield). ESI MS: m/z [M+H⁺] for C₁₃H₁₅ClN₄O₂S, calcd 327.1, found 327.2.

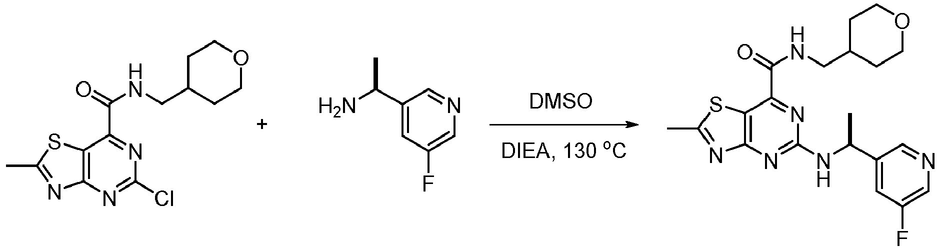

**Synthesis of (*S*)-5-((1-(5-fluoropyridin-3-yl)ethyl)amino)-2-methyl-*N-*((tetrahydro-2*H*-pyran-4-yl)methyl)thiazolo[4,5-*d*]pyrimidine-7-carboxamide (11):** The title compound (**11**) was prepared from 5-chloro-2-methyl-*N*-((tetrahydro-2H-pyran-4-yl)methyl)thiazolo[4,5-*d*]pyrimidine-7-carboxamide (65 mg, 0.20 mmol) using chemistry similar to that described in Example 1 (13 mg, 14% yield). ¹H NMR (300 MHz, methanol-*d*₄) δ 1.31-1.47 (2H, m), 1.60-1.68 (2H, m), 1.65 (3H, d, *J* = 6.8 Hz), 1.92 (1H, m), 2.85 (3H, s), 3.32-3.47 (4H, m), 3.95-4.00 (2H, m), 5.38 (1H, q, *J* = 6.8 Hz), 7.75 (1H, td, *J* = 9.6, 2.8 Hz), 8.33 (1H, d, *J=* 2.8 Hz), 8.56 (1H, m) ppm. ESI MS: m/z [M+H⁺] for C₂₀H₂₃FN₆O₂S, calcd 431.2, found 431.1.

### Example 12. N-((R)-2-Hydroxypropyl)-2-methyl-5-(((S)-1-(pyridin-3-yl)ethyl)amino)thiazolo[4,5-d]pyrimidine-7-carboxamide

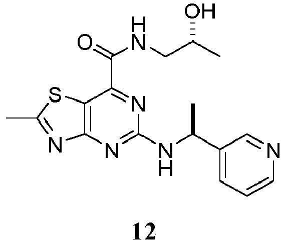

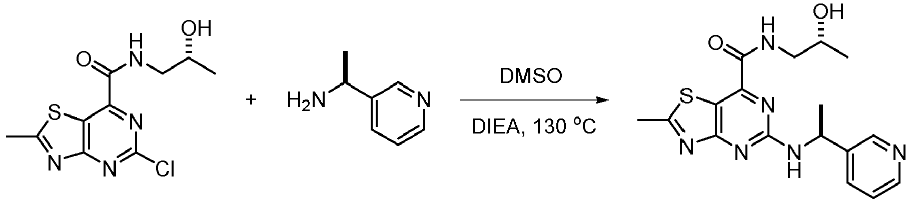

**Synthesis of *N*-((*R*)-2-hydroxypropyl)-2-methyl-5-(((*S*)-1-(pyridin-3-yl)ethyl)amino)thiazolo[4,5-*d*]pyrimidine-7-carboxamide (12):** The title compound **(12)** was prepared from (*R*)-5-chloro-*N*-(2-hydroxypropyl)-2-methylthiazolo[4,5-*d*]pyrimidine-7-carboxamide (100 mg, 0.32 mmol) using chemistry similar to that described in Example 3 using (*S*)-1-(pyridin-3-yl)ethan-1-amine in place of (*S*)-1-(5-fluoropyridin-3-yl)ethan-1-amine (commercially obtained from J&W Pharmlab, Levittown, PA) (17 mg, 14% yield). ¹H NMR (300 MHz, methanol-*d*₄) δ 1.23 (3H, d, *J* = 6.2 Hz), 1.65 (3H, d, *J* = 6.8 Hz), 2.84 (3H, s), 3.33 (1H, m), 3.52 (1H, dd, *J* = 13.5, 4.2 Hz), 3.98 (1H, m), 5.32 (1H, q, *J* = 6.8 Hz), 7.40 (1H, dd, *J* = 8.0, 4.8 Hz), 7.98 (1H, dt, *J* = 8.0, 2.0 Hz), 8.40 (1H, dd, *J* = 4.8, 2.0 Hz), 8.69 (1H, m) ppm. ESI MS: m/z [M+H⁺] for C₁₇H₂₀N₆O₂S, calcd 373.1, found 373.1.

### Example 13. 5-(((S)-1-(5-Fluoropyridin-3-yl)ethyl)amino)-2-methyl-N-(((S)-tetrahydrofuran-3-yl)methyl)thiazolo[4,5-d]pyrimidine-7-carboxamide

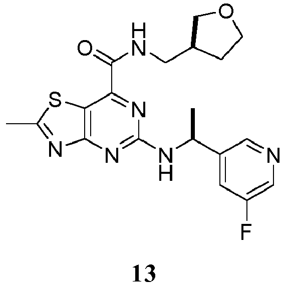

**Synthesis of 5-(((*S*)-1-(5-fluoropyridin-3-yl)ethyl)amino)-2-methyl-*N*-(((*S*)-tetrahydrofuran-3-yl)methyl)thiazolo[4,5-*d*]pyrimidine-7-carboxamide (13):** The title compound (13) was synthesized in similar fashion to example 1. ¹H NMR (300 MHz, methanol-*d*₄) δ 1.66 (3H, d, *J* = 6.8 Hz), 1.74 (1H, m), 2.09 (1H, m), 2.64 (1H, m), 2.84 (3H, s), 3.39-3.48 (2H, m), 3.58 (1H, m), 3.73-3.95 (3H, m), 5.39 (1H, q, *J* = 6.8 Hz), 7.76 (1H, dt, *J* = 9.6, 2.4 Hz), 8.32 (1H, d, *J* = 2.4 Hz), 8.56 (1H, m) ppm. ESI MS: m/z [M+H⁺] for C₁₉H₂₁FN₆O₂S, calcd 417.1, found 417.1.

### Example 14. 5-(((S)-1-(5-Fluoropyridin-3-yl)ethyl)amino)-2-methyl-N-(((R)-tetrahydrofuran-3-yl)methyl)thiazolo[4,5-d]pyrimidine-7-carboxamide

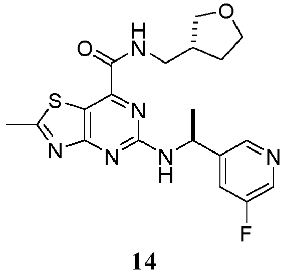

**Synthesis of 5-(((*S*)-1-(5-fluoropyridin-3-yl)ethyl)amino)-2-methyl-*N*-(((*R*)-tetrahydrofuran-3-yl)methyl)thiazolo[4,5-*d*]pyrimidine-7-carboxamide (14):** The title compound **(14)** was synthesized in similar fashion to example 1. ¹H NMR (300 MHz, methanol-*d*₄) δ 1.66 (3H, d, *J* = 6.8 Hz), 1.71 (1H, m), 2.09 (1H, m), 2.63 (1H, m), 2.85 (3H, s), 3.39-3.52 (2H, m), 3.61 (1H, m), 3.73-3.86 (2H, m), 3.92 (1H, m), 5.39 (1H, q, *J* = 6.8 Hz), 7.75 (1H, dt, *J* = 9.6, 2.4 Hz), 8.33 (1H, d, *J* = 2.4 Hz), 8.56 (1H, m) ppm. ESI MS: m/z [M+H⁺] for C₁₉H₂₁FN₆O₂S, calcd 417.1, found 417.1.

### Example 15. N-((S)-2-Hydroxypropyl)-2-methyl-5-(((S)-1-(pyridin-3-yl)ethyl)amino)thiazolo[4,5-d]pyrimidine-7-carboxamide

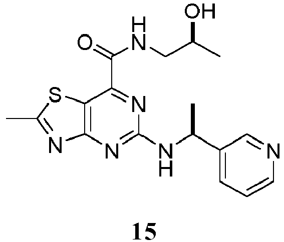

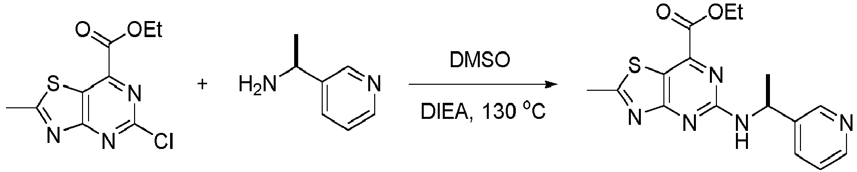

**Synthesis of ethyl (*S*)-2-methyl-5-((1-(pyridin-3-yl)ethyl)amino)thiazolo[4,5-*d*]pyrimidine-7-carboxylate:** To a solution of ethyl 5-chloro-2-methylthiazolo[4,5-d]pyrimidine-7-carboxylate (1 g, 3.88 mmol) in DMSO (15 mL) were added (1*S*)-1-(pyridin-3-yl)ethan-1-amine (712 mg, 5.83 mmol) and DIEA (1.5 g, 11.61 mmol), and the resulting solution was stirred for 1.5 h at 130 °C. The reaction mixture was diluted with ethyl acetate and washed with brine four times. The organic layer was dried over anhydrous sodium sulfate and concentrated under vacuum. The residue was purified by flash chromatography on silica gel eluting with ethyl acetate/petroleum ether to provide ethyl (*S*)-2-methyl-5-((1-(pyridin-3-yl)ethyl)amino)thiazolo[4,5-*d*]pyrimidine-7-carboxylate as an yellow solid (600 mg, 45% yield). ¹H NMR (400 MHz, DMSO-*d*₆) δ 1.37 (3H, t, *J* = 8.0 Hz), 1.52 (3H, d, *J* = 8.0 Hz), 2.80 (3H, s), 4.42 (2H, q, *J* = 8.0 Hz), 5.16 (1H, quintet, *J* = 8.0 Hz), 7.33 (1H, m), 7.83 (1H, m), 8.40 (1H, m), 8.45 (1H, m), 8.66 (1H, m) ppm. ESI MS: m/z [M+H⁺] for C₁₆H₁₇N₅O₂S, calcd 344.1, found 344.0.

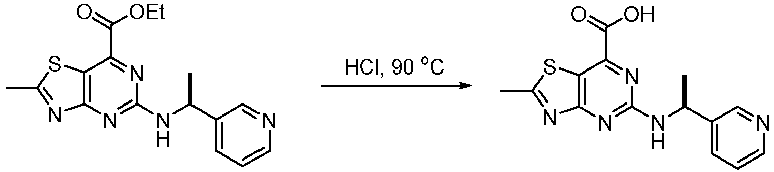

**Synthesis of (*S*)-2-methyl-5-((1-(pyridin-3-yl)ethyl)amino)thiazolo[4,5-*d*]pyrimidine-7-carboxylic acid hydrochloride:** A solution of ethyl (*S*)-2-methyl-5-((1-(pyridin-3-yl)ethyl)amino)thiazolo[4,5-*d*]pyrimidine-7-carboxylate (600 mg, 1.75 mmol) in con. HCl (15 mL) was stirred for 30 min at 90 °C. The reaction mixture was evaporated to dryness in vacuo at a bath temperature of 60 °C and the residue was dried under high vacuum to provide (S)-2-methyl-5-((1-(pyridin-3-yl)ethyl)amino)thiazolo[4,5-*d*]pyrimidine-7-carboxylic acid hydrochloride salt a dark yellow solid (560 mg, 91% yield). ESI MS: m/z [M+H⁺] for C₁₄H₁₃N₅O₂S, calcd 316.0, found 316.0.

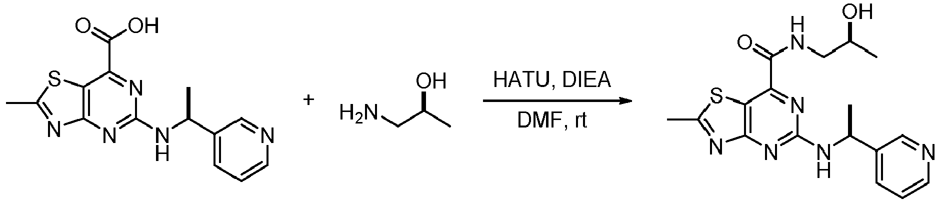

**Synthesis of *N*-((*S*)-2-hydroxypropyl)-2-methyl-5-(((*S*)-1-(pyridin-3-yl)ethyl)amino)thiazolo[4,5-*d*]pyrimidine-7-carboxamide (15):** To a solution of (S)-2-methyl-5-((1-(pyridin-3-yl)ethyl)amino)thiazolo[4,5-*d*]pyrimidine-7-carboxylic acid hydrochloride (100 mg, 0.28 mmol) in DMF (5 mL) were added (2*S*)-1-aminopropan-2-ol (28.6 mg, 0.38 mmol), HATU (241.2 mg, 0.63 mmol), and DIEA (124 mg, 0.96 mmol). The resulting solution was stirred for 2 h at room temperature and was extracted with ethyl acetate and the organic layer was dried over anhydrous sodium sulfate and concentrated under vacuum. The residue was purified by flash chromatography on silica gel (12 g HP silica, Teledyne Isco) eluting with 10% to 50% solvent A (DCM/MeOH/NH₄OH, 100/10/1) in DCM to provide *N*-((*S*)-2-hydroxypropyl)-2-methyl-5-(((*S*)-1-(pyridin-3-yl)ethyl)amino)thiazolo[4,5-d]pyrimidine-7-carboxamide as a light yellow solid (42 mg, 40% yield). ¹H NMR (300 MHz, methanol-*d*₄) δ 1.23 (3H, d, *J* = 6.2 Hz), 1.65 (3H, d, *J* = 7.2 Hz), 2.84 (3H, s), 3.33 (1H, m), 3.53 (1H, dd, *J* = 13.5, 4.2 Hz), 3.97 (1H, m), 5.33 (1H, q, *J* = 7.2 Hz), 7.40 (1H, dd, *J* = 8.0, 4.8 Hz), 7.97 (1H, dt, *J* = 8.0, 2.0 Hz), 8.40 (1H, dd, *J* = 4.8, 2.0 Hz), 8.68 (1H, m) ppm. ESI MS: m/z [M+H⁺] for C₁₇H₂₀N₆O₂S, calcd 373.1, found 373.1.

### Example 16. ((R)-3-Hydroxypyrrolidin-1-yl)(2-methyl-5-(((S)-1-(pyridin-3-yl)ethyl)amino)thiazolo[4,5-d]pyrimidin-7-yl)methanone

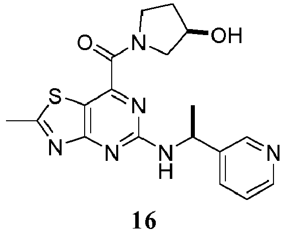

**Synthesis of ((*R*)-3-hydroxypyrrolidin-1-yl)(2-methyl-5-(((*S*)-1-(pyridin-3-yl)ethyl)amino)thiazolo[4,5-*d*]pyrimidin-7-yl)methanone (16):** The title compound **(16)** was synthesized in similar fashion to example 4. ¹H NMR (300 MHz, DMSO-*d*₆) δ 1.52 (3H, d, *J* = 6.8 Hz), 1.75-1.95 (2H, m), 2.76 (3H, s), 3.48-3.64 (3H, m), 4.05 (1H, m), 4.30 (1H, m), 5.13 (1H, quintet, *J* = 6.8 Hz), 7.32 (1H, m), 7.80 (1H, m), 8.22 (1H, m), 8.40 (1H, m), 8.62 (1H, m) ppm. ESI MS: m/z [M+H⁺] for C₁₈H₂₀N₆O₂S, calcd 385.1, found 385.1.

### Example 17. ((S)-3-Hydroxypyrrolidin-1-yl)(2-methyl-5-(((S)-1-(pyridin-2-yl)ethyl)amino)thiazolo[4,5-d]pyrimidin-7-yl)methanone

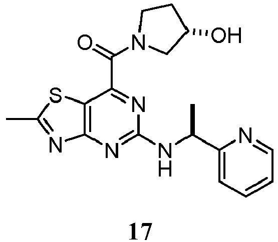

**Synthesis ((*S*)-3-hydroxypyrrolidin-1-yl)(2-methyl-5-(((*S*)-1-(pyridin-2-yl)ethyl)amino)thiazolo[4,5-*d*]pyrimidin-7-yl)methanone (17):** The title compound (**17**) was synthesized in similar fashion to example 4. ¹H NMR (300 MHz, methanol-*d*₄) δ 1.61 (3H, d, *J* = 6.8 Hz), 1.80-2.00 (2H, m), 2.81 (3H, s), 3.62-3.75 (4H, m), 4.39 (1H, m), 5.18 (1H, q, *J* = 6.8 Hz), 7.27 (1H, m), 7.51 (1H, m), 7.76 (1H, m), 8.50 (1H, m) ppm. ESI MS: m/z [M+H⁺] for C₁₈H₂₀N₆O₂S, calcd 385.1, found 385.1.

### Example 18. ((S)-3-Hydroxypyrrolidin-1-yl)(2-methyl-5-(((S)-1-(pyridin-3-yl)ethyl)amino)thiazolo[4,5-d]pyrimidin-7-yl)methanone

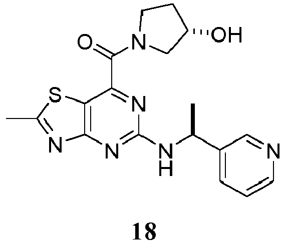

**Synthesis of ((*S*)-3-hydroxypyrrolidin-1-yl)(2-methyl-5-(((*S*)-1-(pyridin-3-yl)ethyl)amino)thiazolo[4,5-*d*]pyrimidin-7-yl)methanone (18):** The title compound **(18)** was synthesized in similar fashion to example 4. ¹H NMR (300 MHz, methanol-*d*₄) δ 1.62 (3H, d, *J* = 6.8 Hz), 1.86-2.05 (2H, m), 2.81 (3H, s), 3.71-3.76 (3H, m), 4.02 (1H, m), 4.41 (1H, m), 5.24 (1H, q, *J* = 6.8 Hz), 7.40 (1H, m), 7.92 (1H, m), 8.39 (1H, m), 8.63 (1H, m) ppm. ESI MS: m/z [M+H⁺] for C₁₈H₂₀N₆O₂S, calcd 385.1, found 385.2.

### Example 19. ((R)-3-Hydroxypyrrolidin-1-yl)(2-methyl-5-(((S)-1-(pyridin-2-yl)ethyl)amino)thiazolo[4,5-d]pyrimidin-7-yl)methanone

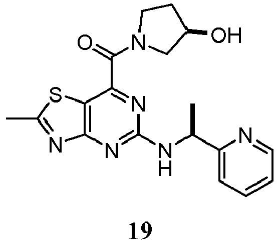

**Synthesis of ((*R*)-3-hydroxypyrrolidin-1-yl)(2-methyl-5-(((*S*)-1-(pyridin-2-yl)ethyl)amino)thiazolo[4,5-*d*]pyrimidin-7-yl)methanone (19):** The title compound (**19**) was synthesized in similar fashion to example 4. ¹H NMR (300 MHz, methanol-*d*₄) δ 1.61 (3H, d, *J* = 6.8 Hz), 1.86-2.09 (2H, m), 2.81 (3H, s), 3.64-3.74 (3H, m), 4.15 (1H, m), 4.52 (1H, m), 5.18 (1H, q, *J* = 6.8 Hz), 7.26 (1H, dd, *J* = 7.8, 5.4 Hz), 7.50 (1H, d, *J* = 7.8 Hz), 7.75 (1H, td, *J* = 7.8, 1.8 Hz), 8.49 (1H, m) ppm. ESI MS: m/z [M+H⁺] for C₁₈H₂₀N₆O₂S, calcd 385.1, found 385.1.

### Example 20. (S)-(2-Amino-5-((1-(pyridin-3-yl)ethyl)amino)thiazolo[4,5-d]pyrimidin-7-yl)(pyrrolidin-1-yl)methanone

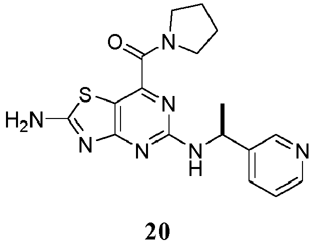

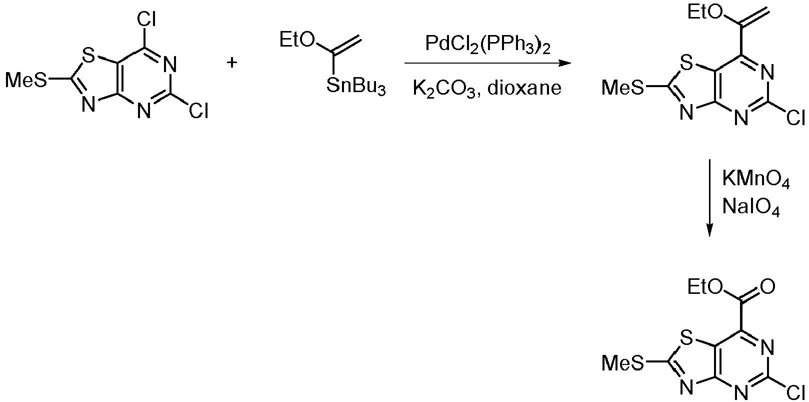

**Synthesis of ethyl 5-chloro-2-(methylthio)thiazolo[4,5-*d*]pyrimidine-7-carboxylate:** The title compound was prepared from 5,7-dichloro-2-(methylthio)thiazolo[4,5-*d*]pyrimidine (commercially obtained from Combi-Block, San Diego, CA) using chemistry similar to that described in Example 1. ESI MS: m/z [M+H⁺] for C₉H₈ClN₃O₂S₂, calcd 290.0, found 290.1.

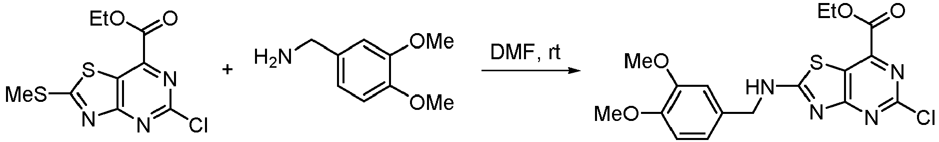

**Synthesis of ethyl 5-chloro-2-((3,4-dimethoxybenzyl)amino)thiazolo[4,5-*d*]pyrimidine-7-carboxylate:** To a solution of ethyl 5-chloro-2-(methylthio)thiazolo[4,5-d]pyrimidine-7-carboxylate (1.1 g, 3.80 mmol) in DMF (15 mL) was added (3,4-dimethoxyphenyl)methanamine (760 mg, 4.55 mmol) (commercially obtained from Sigma-Aldrich, St. Louis, MO) and the mixture thus obtained was stirred for 10 min at room temperature. The reaction mixture was poured into 30 mL of water and the precipitates were collected by filtration. The solid was dried under vacuum to provide ethyl 5-chloro-2-((3,4-dimethoxybenzyl)amino)thiazolo[4,5-*d*]pyrimidine-7-carboxylate as an yellow solid (1.2 g, 77% yield). ESI MS: m/z [M+H⁺] for C₁₇H₁₇ClN₄O₄S, calcd 409.1, found 409.2.

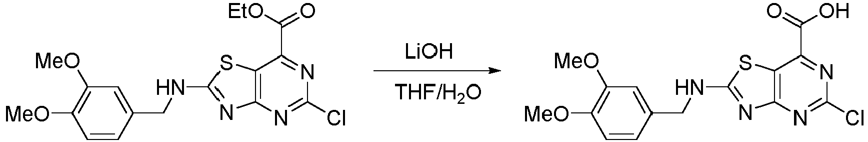

**Synthesis of 5-chloro-2-((3,4-dimethoxybenzyl)amino)thiazolo[4,5-*d*]pyrimidine-7-carboxylic acid:** To a solution of ethyl 5-chloro-2-((3,4-dimethoxybenzyl)amino)thiazolo[4,5-*d*]pyrimidine-7-carboxylate (1.2 g, 2.93 mmol) in THF (12 mL) and water (6 mL) was added LiOH (423 mg, 17.66 mmol) and the resulting solution was stirred for 15 min at room temperature. The pH value of reaction mixture was adjusted to 3 with hydrogen chloride (1 mmol/L) and the precipitates were collected by filtration. The solid was dried under vacuum to provide 5-chloro-2-((3,4-dimethoxybenzyl)amino)thiazolo[4,5-*d*]pyrimidine-7-carboxylic acid as a brown solid (1.16 g, 100% yield). ESI MS: m/z [M+H⁺] for C₁₅H₁₃ClN₄O₄S, calcd 381.0, found 381.2.

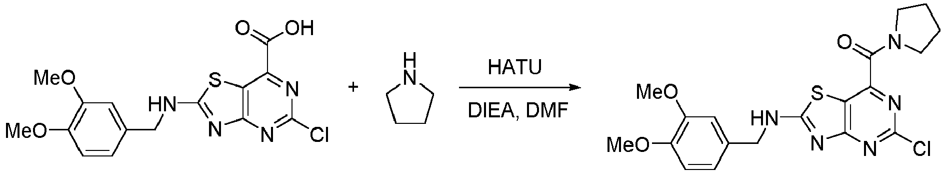

**Synthesis of (5-chloro-2-((3,4-dimethoxybenzyl)amino)thiazolo[4,5-*d*]pyrimidin-7-yl)(pyrrolidin-1-yl)methanone:** To a solution of 5-chloro-2-((3,4-dimethoxybenzyl)amino)thiazolo[4,5-*d*]pyrimidine-7-carboxylic acid (1.1 g, 2.89 mmol) in DMF (10 mL) were added pyrrolidine (164 mg, 2.31 mmol), HATU (1.3 g, 3.42 mmol), and DIEA (1.11 g, 8.59 mmol). The reaction mixture thus obtained was stirred for 2 h at room temperature and then was diluted with 200 mL of ethyl acetate and washed with water (150 mL x 3) and brine (150 mL). The organic layer was dried over anhydrous sodium sulfate and concentrated under vacuum to provide (5-chloro-2-((3,4-dimethoxybenzyl)amino)thiazolo[4,5-*d*]pyrimidin-7-yl)(pyrrolidin-1-yl)methanone as a gray solid (510 mg. 41% yield). ESI MS: m/z [M+H⁺] for C₁₉H₂₀ClN₅O₃S, calcd 434.1, found 434.3.

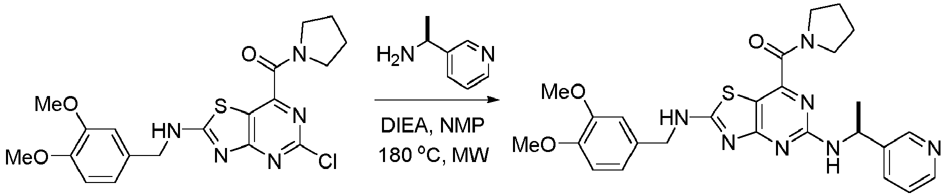

**Synthesis of (S)-(2-((3,4-dimethoxybenzyl)amino)-5-((1-(pyridin-3-yl)ethyl)amino)thiazolo[4,5-*d*]pyrimidin-7-yl)(pyrrolidin-1-yl)methanone:** A solution of (1*S*)-1-(pyridin-3-yl)ethan-1-amine (300 mg, 2.46 mmol), (5-chloro-2-((3,4-dimethoxybenzyl)amino)thiazolo[4,5-*d*]pyrimidin-7-yl)(pyrrolidin-1-yl)methanone (168 mg, 0.39 mmol), and DIEA (267 mg, 2.07 mmol) in NMP (4 mL) was irradiated under microwave radiation for 1.5 h at 180 °C. The reaction mixture was diluted with ethyl acetate and washed with brine (x4), then concentrated under reduced pressure. The residue was purified by flash chromatography on silica gel eluting with 2% methanol in DCM to provide (*S*)-(2-((3,4-dimethoxybenzyl)amino)-5-((1-(pyridin-3-yl)ethyl)amino)thiazolo[4,5-*d*]pyrimidin-7-yl)(pyrrolidin-1-yl)methanone as an yellow solid (120 mg, 59% yield). ESI MS: m/z [M+H⁺] for C₂₆H₂₉N₇O₃S, calcd 520.2, found 520.2.

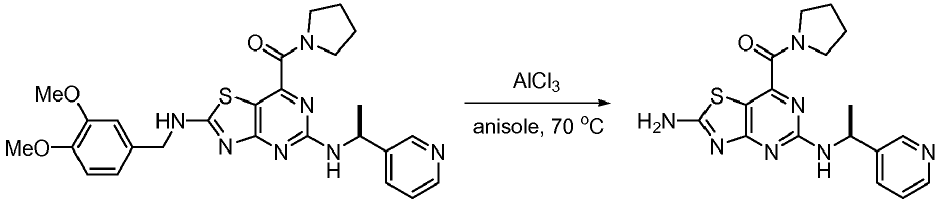

**Synthesis of (*S*)-(2-amino-5-((1-(pyridin-3-yl)ethyl)amino)thiazolo[4,5-*d*]pyrimidin-7-yl)(pyrrolidin-1-yl)methanone (20):** A mixture of (S)-(2-((3,4-dimethoxybenzyl)amino)-5-((1-(pyridin-3-yl)ethyl)amino)thiazolo[4,5-*d*]pyrimidin-7-yl)(pyrrolidin-1-yl)methanone (100 mg, 0.19 mmol), AlCl₃ (100 mg), and anisole (3 mL) was stirred for 16 h at 70 °C. The reaction mixture was extracted with ethyl acetate (30 mL x 3) and the combined organic layer was washed with brine (30 mL x 2), dried over anhydrous sodium sulfate and concentrated under vacuum. The residue was purified by prep-HPLC (C-18 OBD column and a gradient elution system of water-acetonitrile) to provide (*S*)-(2-amino-5-((1-(pyridin-3-yl)ethyl)amino)thiazolo[4,5-*d*]pyrimidin-7-yl)(pyrrolidin-1-yl)methanone as a white solid (8 mg, 10% yield). ¹H NMR (300 MHz, methanol-*d*₄) δ 1.57 (3H, d, *J* = 7.2 Hz), 1.78-1.94 (4H, m), 3.52-3.58 (3H, m), 3.85 (1H, m), 5.14 (1H, q, *J* = 7.2 Hz), 7.37 (1H, dd, *J* = 8.0, 4.8 Hz), 7.87 (1H, td, *J* = 8.0, 2.0 Hz), 8.40 (1H, m), 8.60 (1H, m) ppm. ESI MS: m/z [M+H⁺] for C₁₇H₁₉N₇OS, calcd 370.1, found 370.0.

### Example 21. (S)-5-((1-(5-Fluoropyridin-3-yl)ethyl)amino)-N-(4-hydroxyphenethyl)-2-methylthiazolo[4,5-d]pyrimidine-7-carboxamide

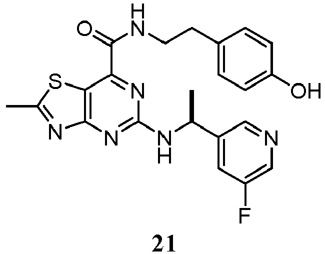

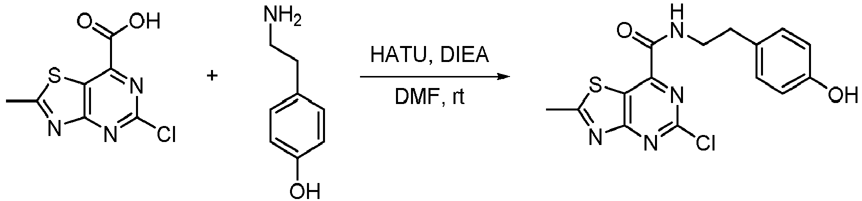

**Synthesis of 5-chloro-*N*-(4-hydroxyphenethyl)-2-methylthiazolo[4,5-*d*]pyrimidine-7-carboxamide:** A mixture of 5-chloro-2-methyl-thiazolo[4,5-*d*]pyrimidine-7-carboxylic acid hydrochloride (70 mg, 0.26 mmol) and HATU (130 mg, 0.34 mmol) in DMF (0.7 mL) was stirred at room temperature for 10 min, then DIEA (0.32 mL, 1.84 mmol) and 4-(2-aminoethyl)phenol (47 mg, 0.34 mmol) (commercially obtained from AK Scientific, Union City, CA) were added, and the resulting mixture was stirred at room temperature overnight, concentrated, and the residue was purified by flash chromatography on silica gel eluting with 10% to 80% of ethyl acetate in hexane to give 5-chloro-*N*-(4-hydroxyphenethyl)-2-methylthiazolo[4,5-*d*]pyrimidine-7-carboxamide (30 mg, 33% yield) as yellow paste. ESI MS: m/z [M+H⁺] for C₁₅H₁₃ClN₄O₂S, calcd 349.0, found 349.1.

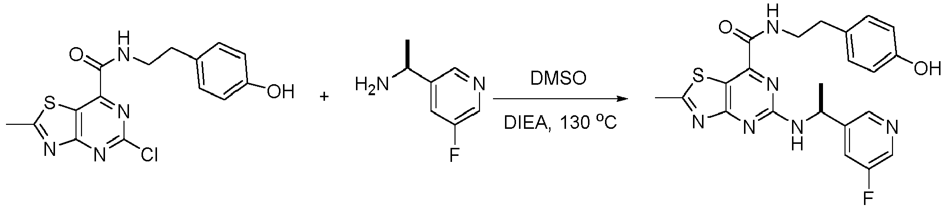

**Synthesis of (*S*)-5-((1-(5-fluoropyridin-3-yl)ethyl)amino)-*N*-(4-hydroxyphenethyl)-2-methylthiazolo[4,5-*d*]pyrimidine-7-carboxamide (21):** To a mixture of ethyl 5-chloro-*N*-(4-hydroxyphenethyl)-2-methylthiazolo[4,5-*d*]pyrimidine-7-carboxamide (50 mg) and DIEA (0.110 mL, 0.65 mmol) in DMSO (1 mL) was added (1*S*)-1-(5-fluoro-3-pyridyl)ethanamine (27 mg, 0.19 mmol). The resulting mixture was stirred at 130 °C for 8 h, cooled to room temperature, diluted with ethyl acetate and washed with 25% aqueous NaCl solution, then with brine three times, and extracts were dried over magnesium sulfate. The solvent was evaporated and the residue was purified by flash chromatography on silica gel eluting with 2% to 100% solvent A (DCM/MeOH/NH₄OH, 100/10/1) in DCM to provide (*S*)-5-((1-(5-fluoropyridin-3-yl)ethyl)amino)-*N*-(4-hydroxyphenethyl)-2-methylthiazolo[4,5-*d*]pyrimidine-7-carboxamide (7 mg, 59% yield) as a light yellow solid. ¹H NMR (400 MHz, CDCl₃) δ 1.61 (3H, s), 2.82-2.84 (2H, m), 2.86 (3H, s), 3.60-3.75 (2H, m), 5.24 (1H, m), 5.52 (1H, m), 6.0-6.50 (1H, br s), 6.79 (2H, d, *J* = 8.8 Hz), 7.05 (2H, d, *J* = 8.8 Hz), 7.42 (1H, d, *J* = 7.2 Hz), 7.63 (1H, br s), 8.33 (2H, d, *J* = 2.8 Hz) ppm. ESI MS: m/z [M+H⁺] for C₂₂H₂₁FN₆O₂S, calcd 453.1, found 453.2.

### Example 22. tert-Butyl (S)-4-(2-(5-((1-(5-fluoropyridin-3-yl)ethyl)amino)-2-methylthiazolo[4,5-d]pyrimidine-7-carboxamido)ethyl)piperidine-1-carboxylate

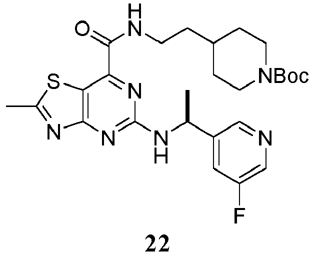

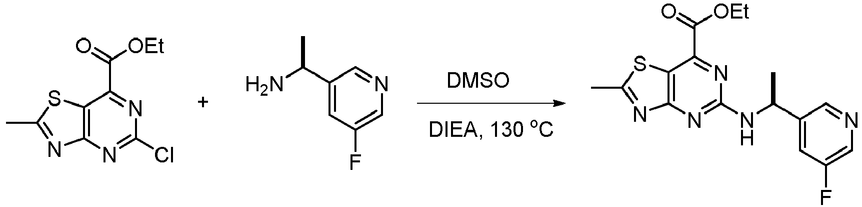

**Synthesis of ethyl (*S*)-5-((1-(5-fluoropyridin-3-yl)ethyl)amino)-2-methylthiazolo[4,5-*d*]pyrimidine-7-carboxylate:** To a mixture of ethyl 5-chloro-2-methyl-thiazolo[4,5-*d*]pyrimidine-7-carboxylate (200 mg, 0.78 mmol) and DIEA (0.41 mL, 2.33 mmol) in DMSO (3 mL) was added (1*S*)-1-(5-fluoro-3-pyridyl)ethanamine (163 mg, 1.16 mmol). The reaction mixture was stirred at 130 °C for 6 h, cooled to room temperature, diluted with ethyl acetate and washed with 25% aqueous NaCl solution, then with brine three times, and combined extracts were dried over magnesium sulfate. The solvent was evaporated, and the residue was purified by flash chromatography on silica gel eluting with 2% to 100% solvent A (DCM/MeOH/NH₄OH, 100/10/1) in DCM to provide ethyl (*S*)-5-((1-(5-fluoropyridin-3-yl)ethyl)amino)-2-methylthiazolo[4,5-*d*]pyrimidine-7-carboxylate (131 mg, 47% yield) as a light yellow solid. ESI MS: m/z [M+H⁺] for C₁₆H₁₆FN₅O₂S, calcd 362.1, found 362.1.

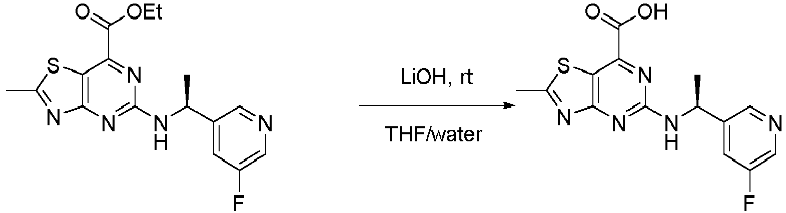

**Synthesis of (*S*)-5-((1-(5-fluoropyridin-3-yl)ethyl)amino)-2-methylthiazolo[4,5-*d*]pyrimidine-7-carboxylic acid:** Hydroxylithium hydrate (9.4 mg, 0.24 mmol) was added to a mixture of ethyl (*S*)-5-((1-(5-fluoropyridin-3-yl)ethyl)amino)-2-methylthiazolo[4,5-*d*]pyrimidine-7-carboxylate (56 mg, 0.16 mmol) in a THF (1 mL) -Water (0.2 mL) mixture. The resulting mixture was stirred at room temperature for 1 hour, concentrated, dissolved in water (1 mL), and freeze-dried overnight. The dark red powder was dissolved in DCM - MeOH mixture (10:1) and purified by flash chromatography eluting with 2% to 100% solvent A (DCM/MeOH/NH₄OH, 100/10/1) in DCM to provide (*S*)-5-((1-(5-fluoropyridin-3-yl)ethyl)amino)-2-methylthiazolo[4,5-*d*]pyrimidine-7-carboxylic acid (30 mg, 58% yield) as white solids. ESI MS: m/z [M+H⁺] for C₁₄H₁₂FN₅O₂S, calcd 334.1, found 334.1.

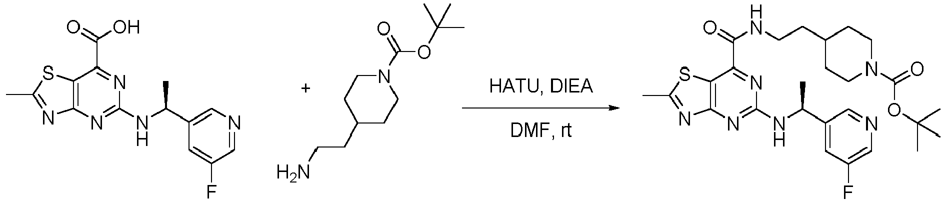

**Synthesis of tert-butyl (*S*)-4-(2-(5-((1-(5-fluoropyridin-3-yl)ethyl)amino)-2-methylthiazolo[4,5-*d*]pyrimidine-7-carboxamido)ethyl)piperidine-1-carboxylate (22):** A mixture of (*S*)-5-((1-(5-fluoropyridin-3-yl)ethyl)amino)-2-methylthiazolo[4,5-*d*]pyrimidine-7-carboxylic acid (15 mg, 0.04 mmol), tert-butyl 4-(2-aminoethyl)piperidine-1-carboxylate (commercially obtained from Combi-Blocks, San Diego, CA) (0.01mL, 0.0600mmol), HATU (22 mg, 0.06 mmol), and DIEA (0.06 mL, 0.31 mmol) in DMF (0.7 mL) was stirred at room temperature for 4 hours, concentrated, and the residue was purified by flash chromatography eluting with 2% to 100% solvent A (DCM/MeOH/NH₄OH, 100/10/1) in DCM to provide tert-butyl (*S*)-4-(2-(5-((1-(5-fluoropyridin-3-yl)ethyl)amino)-2-methylthiazolo[4,5-*d*]pyrimidine-7-carboxamido)ethyl)piperidine-1-carboxylate (10 mg, 41% yield) as white solids. ¹H NMR (400 MHz, CDCl₃) δ 1.12-1.18 (2H, m), 1.43-1.50 (2H, m), 1.45 (9H, s), 1.52-1.62 (2H, m), 1.66 (3H, d, *J* = 6.8 Hz), 2.65-2.75 (2H, m), 2.86 (3H, s), 3.45-3.52 (2H, m), 3.98-4.04 (2H, m), 5.21-5.35 (1H, br s), 5.54 (1H, m), 7.43 (1H, d, *J* = 8.8 Hz), 7.52 (1H, br s), 8.35 (1H, d, *J* = 2.8 Hz), 8.53 (1H, s) ppm. ESI MS: m/z [M+H⁺] for C₂₆H₃₄FN₇O₃S, calcd 544.2, found 544.3.

### Example 23. (S)-5-((1-(5-Fluoropyridin-3-yl)ethyl)amino)-2-methyl-N-(pyridin-3-ylmethyl)thiazolo[4,5-d]pyrimidine-7-carboxamide

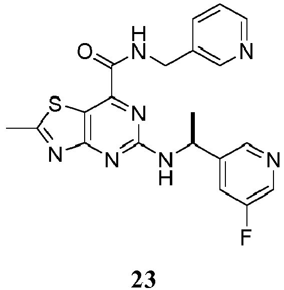

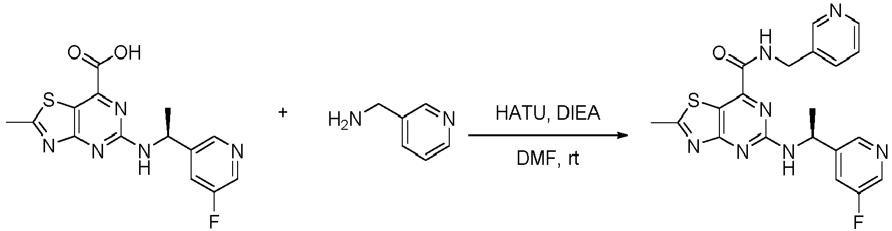

**Synthesis of (*S*)-5-((1-(5-fluoropyridin-3-yl)ethyl)amino)-2-methyl-*N*-(pyridin-3-ylmethyl)thiazolo[4,5-*d*]pyrimidine-7-carboxamide (23):** The title compound **(23)** was prepared from (*S*)-5-((1-(5-fluoropyridin-3-yl)ethyl)amino)-2-methylthiazolo[4,5-*d*]pyrimidine-7-carboxylic acid (25 mg, 0.075 mmol) using chemistry similar to that described in Example 22 using 3-(aminomethyl)pyridine (commercially available from Ark Pharm, Arlington Heights, IL) in place of tert-butyl 4-(2-aminoethyl)piperidine-1-carboxylate (70% yield). ¹H NMR (400 MHz, CDCl₃) δ 1.64 (3H, d, *J* = 7.2 Hz), 2.88 (3H, s), 4.66 (2H, d, *J* = 6.0 Hz), 5.30 (1H, m), 5.59 (1H, m), 7.30-7.34 (1H, m), 7.42 (1H, d, *J* = 7.2 Hz), 7.72 (1H, d, *J* = 7.6 Hz), 8.05 (1H, br s), 8.33 (1H, d, *J* = 2.8 Hz), 8.5 (1H, s), 8.57 (1H, dd, *J* = 4.7, 2.0 Hz), 8.63 (1H, d, *J* = 2.0 Hz) ppm. ESI MS: m/z [M+H⁺] for C₂₀H₁₈FN₇OS, calcd 424.1, found 424.1.

### Example 24. (S)-N-(4-Acetamidophenethyl)-5-((1-(5-fluoropyridin-3-yl)ethyl)amino)-2-methylthiazolo[4,5-d]pyrimidine-7-carboxamide

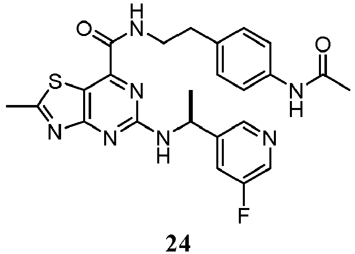

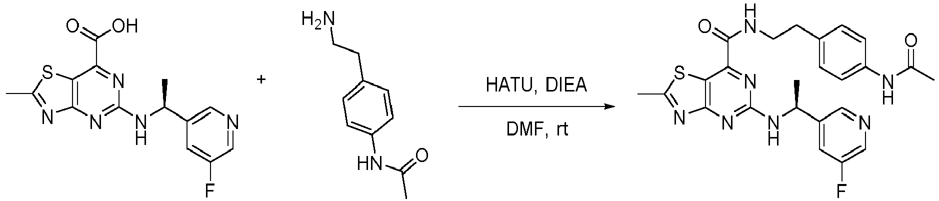

**Synthesis of (*S*)-*N*-(4-acetamidophenethyl)-5-((1-(5-fluoropyridin-3-yl)ethyl)amino)-2-methylthiazolo[4,5-*d*]pyrimidine-7-carboxamide (24):** The title compound **(24)** was prepared from (*S*)-5-((1-(5-fluoropyridin-3-yl)ethyl)amino)-2-methylthiazolo[4,5-*d*]pyrimidine-7-carboxylic acid (19 mg, 0.057 mmol) using chemistry similar to that described in Example 22 using *N*-[4-(2-aminoethyl)phenyl]acetamide (commercially available from Enamine, Monmouth Jct., NJ) in place of tert-butyl 4-(2-aminoethyl)piperidine-1-carboxylate (37% yield). ¹H NMR (400 MHz, CDCl₃) δ 1.63 (3H, d, *J* = 7.2 Hz), 2.19 (3H, s), 2.86 (3H, s), 2.89 (2H, t, *J* = 6.8 Hz), 3.64-3.74 (2H, m), 5.25 (1H, br s), 5.60 (1H, m), 7.18 (2H, d, *J* = 8.4 Hz), 7.48 (2H, m), 7.53 (2H, d, *J* = 8.4 Hz), 7.70 (1H, br s), 8.33 (1H, d, *J* = 2.8 Hz), 8.40 (1H, br s) ppm. ESI MS: m/z [M+H⁺] for C₂₄H₂₄FN₇O₂S, calcd 494.2, found 494.1.

### Example 25. (S)-5-((1-(5-Fluoropyridin-3-yl)ethyl)amino)-N-(2-methoxyethyl)-2-methylthiazolo[4,5-d]pyrimidine-7-carboxamide

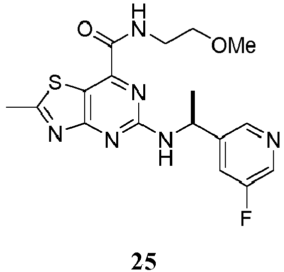

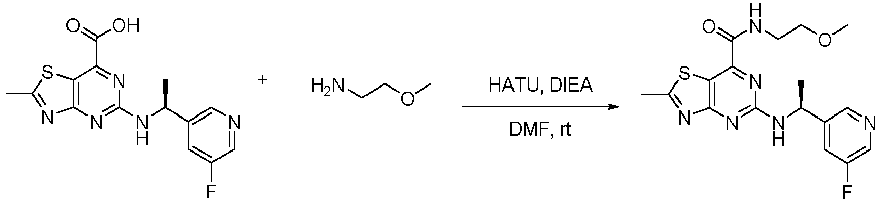

**Synthesis of (*S*)-5-((1-(5-fluoropyridin-3-yl)ethyl)amino)-*N*-(2-methoxyethyl)-2-methylthiazolo[4,5-*d*]pyrimidine-7-carboxamide (25):** The title compound **(25)** was prepared from (*S*)-5-((1-(5-fluoropyridin-3-yl)ethyl)amino)-2-methylthiazolo[4,5-*d*]pyrimidine-7-carboxylic acid (23 mg, 0.07 mmol) using chemistry similar to that described in Example 22 using 2-methoxyethylamine (commercially available from Oakwood Chemicals, Estill, SC) in place of tert-butyl 4-(2-aminoethyl)piperidine-1-carboxylate (52% yield). ¹H NMR (400 MHz, CDCl₃) δ 1.64 (3H, d, *J* = 7.2 Hz), 2.86 (3H, s), 3.44 (3H, s), 3.57 (2H, m), 3.64 (2H, m), 5.30 (1H, br s), 5.52 (1H, m), 7.48 (1H, dt, *J* = 9.2, 2.4 Hz), 8.05 (1H, br s), 8.35 (1H, d, *J* = 2.8 Hz), 8.55 (1H, t, *J* = 1.6 Hz) ppm. ESI MS: m/z [M+H⁺] for C₁₇H₁₉FN₆O₂S, calcd 391.1, found 391.1.

### Example 26. (S)-1-(5-((1-(5-Fluoropyridin-3-yl)ethyl)amino)-2-methylthiazolo[4,5-d]pyrimidine-7-carbonyl)-N,N-dimethylazetidine-3-carboxamide

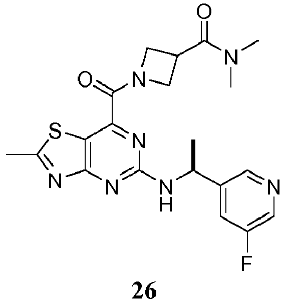

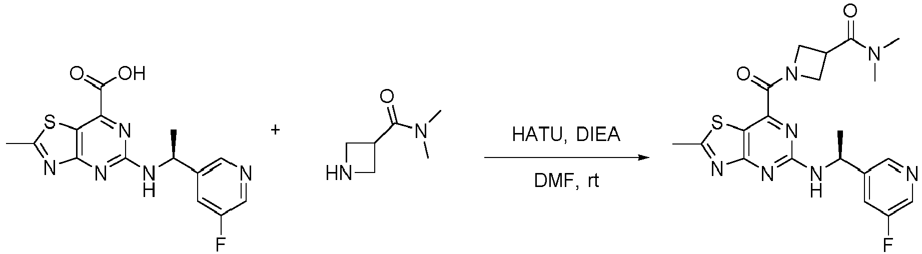

**Synthesis of (*S*)-1-(5-((1-(5-fluoropyridin-3-yl)ethyl)amino)-2-methylthiazolo[4,5-*d*]pyrimidine-7-carbonyl)-*N*,*N*-dimethylazetidine-3-carboxamide (26):** The title compound **(26)** was prepared from (*S*)-5-((1-(5-fluoropyridin-3-yl)ethyl)amino)-2-methylthiazolo[4,5-*d*]pyrimidine-7-carboxylic acid (26 mg, 0.078 mmol) using chemistry similar to that described in Example 22 using *N*,*N*-dimethylazetidine-3-carboxamide hydrochloride (commercially available from PharmaBlock, Cupertino, CA) in place of tert-butyl 4-(2-aminoethyl)piperidine-1-carboxylate (16% yield). ¹H NMR (400 MHz, CDCl₃) δ 1.65 (3H, dd, *J* = 6.8, 2.4 Hz), 2.84 (3H, d, *J* = 1.2 Hz), 2.95 (3H, d, *J* = 3.2 Hz), 3.01 (3H, d, *J* = 5.2 Hz), 3.55-3.68 (1H, m), 4.34-4.40 (2H, m), 4. 85 (1H, m), 5.01 (1H, m), 5.32 (1H, m), 5.46 (1H, m), 7.41-7.47 (1H, m), 8.33 (1H, d, *J* = 2.8 Hz), 8.51 (1H, d, *J* = 10.0 Hz) ppm. ESI MS: m/z [M+H⁺] for C₂₀H₂₂FN₇O₂S, calcd 444.2, found 444.2.

### Example 27. 2-Methyl-N-((S)-1-(pyridin-3-yl)ethyl)-5-(((S)-1-(pyridin-3-yl)ethyl)amino)thiazolo[4,5-d]pyrimidine-7-carboxamide

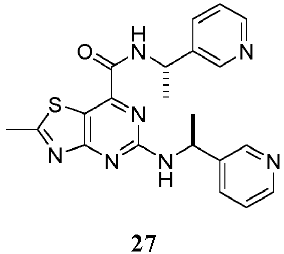

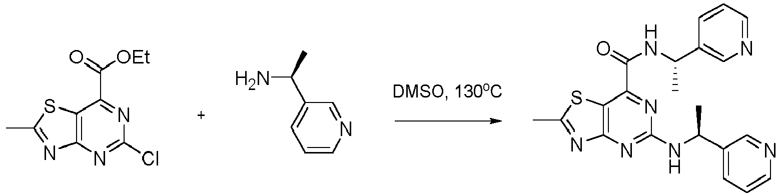

**Synthesis of 2-methyl-*N*-((*S*)-1-(pyridin-3-yl)ethyl)-5-(((*S*)-1-(pyridin-3-yl)ethyl)amino)thiazolo[4,5-*d*]pyrimidine-7-carboxamide (27):** The title compound **(27)** was prepared from ethyl 5-chloro-2-methyl-thiazolo[4,5-*d*]pyrimidine-7-carboxylate (398 mg, 1.51 mmol) using chemistry similar to that described in Example 22 using (1*S*)-1-(3-pyridyl)ethanamine (commercially available from J&W PharmLab, Levittown, PA) in place of tert-butyl 4-(2-aminoethyl)piperidine-1-carboxylate (11% yield). ¹H NMR (400 MHz, MeOD) δ 1.66 (6H, t, *J* = 6.4 Hz), 2.85 (3H, s), 5.23-5.28 (1H, m), 5.59-5.62 (1H, m), 7.20-7.29 (1H, m), 7.52-7.64 (2H, m), 7.71-7.73 (1H, m), 7.80-8.00 (1H, br s), 8.48-8.55 (1H, m), 8.58-8.61 (1H, m), 8.72 (1H, d, *J* = 2.0 Hz) ppm. ESI MS: m/z [M+H⁺] for C₂₁H₂₁N₇OS, calcd 419.1, found 420.2.

### Example 28. (S)-N-(1-(5-((1-(5-Fluoropyridin-3-yl)ethyl)amino)-2-methylthiazolo[4,5-d]pyrimidine-7-carbonyl)azetidin-3-yl)acetamide

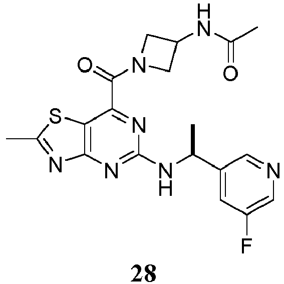

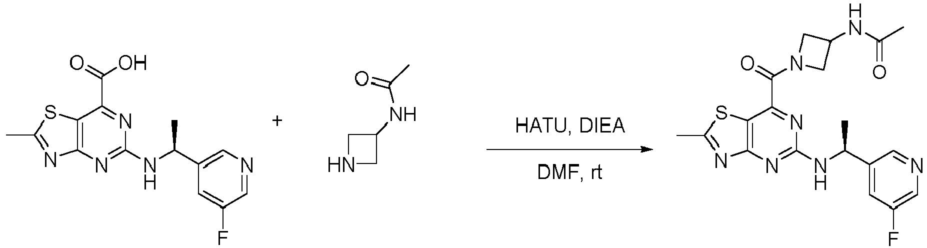

**Synthesis of (*S*)-*N*-(1-(5-((1-(5-fluoropyridin-3-yl)ethyl)amino)-2-methylthiazolo[4,5-*d*]pyrimidine-7-carbonyl)azetidin-3-yl)acetamide (28):** The title compound **(28)** was prepared from (*S*)-5-((1-(5-fluoropyridin-3-yl)ethyl)amino)-2-methylthiazolo[4,5-*d*]pyrimidine-7-carboxylic acid (16 mg, 0.048 mmol) using chemistry similar to that described in Example 22 using N-(azetidin-3-yl)acetamide (commercially available from Enamine, Monmouth Jct., NJ) in place of tert-butyl 4-(2-aminoethyl)piperidine-1-carboxylate (21% yield). ¹H NMR (400 MHz, CDCl₃) δ 1.64 (3H, d, *J* = 6.8 Hz), 2.06 (3H, s), 2.84 (3H, s), 4.02-4.08 (2H, m), 4.45-4.65 (2H, m), 4.78 (1H, m), 5.58 (1H, m), 7.36 (1H, m), 7.48 (1H, d, *J* = 9.2 Hz) 8.34 (1H, m), 8.51 (1H, s), 8.64 (1H, d, *J* = 3.6 Hz) ppm. ESI MS: m/z [M+H⁺] for C₁₉H₂₀FN₇O₂S, calcd 430.1, found 430.1.

### Example 29. N-((S)-1-(5-(((S)-1-(5-Fluoropyridin-3-yl)ethyl)amino)-2-methylthiazolo[4,5-d]pyrimidine-7-carbonyl)pyrrolidin-3-yl)acetamide

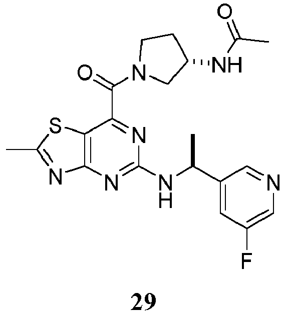

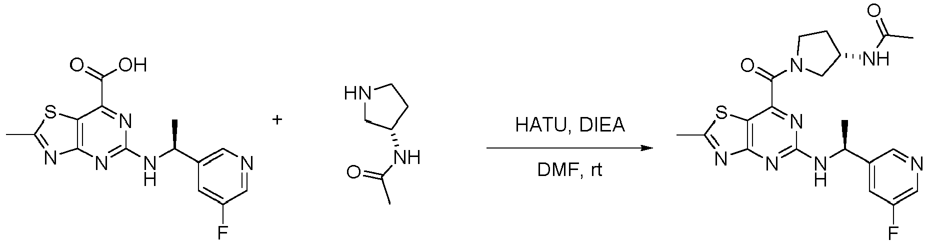

**Synthesis of *N*-((*S*)-1-(5-(((*S*)-1-(5-fluoropyridin-3-yl)ethyl)amino)-2-methylthiazolo[4,5-*d*]pyrimidine-7-carbonyl)pyrrolidin-3-yl)acetamide (29):** The title compound **(29)** was prepared from (*S*)-5-((1-(5-fluoropyridin-3-yl)ethyl)amino)-2-methylthiazolo[4,5-*d*]pyrimidine-7-carboxylic acid (33 mg, 0.099 mmol) using chemistry similar to that described in Example 22 using *N*-[(3*S*)-pyrrolidin-3-yl]acetamide (commercially available from PharmaBlock, Cupertino, CA) in place of tert-butyl 4-(2-aminoethyl)piperidine-1-carboxylate (24% yield). ¹H NMR (400 MHz, CDCl₃) δ 1.64 (3H, d, *J* = 7.2 Hz), 2.01-2.07 (3H, m), 2.84 (3H, d, *J* = 1.2 Hz), 3.61-3.91 (3H, m), 4.52 (2H, m), 5.23 (1H, m), 5.59-5.66 (2H, m), 5.86 (1H, br s), 7.45-7.52 (1H, m), 8.34 (1H, m), 8.48-8.53 (1H, m) ppm. ESI MS: m/z [M+H⁺] for C₂₀H₂₂FN₇O₂S, calcd 444.2, found 444.2.

### Example 30. N-((R)-1-(5-(((S)-1-(5-Fluoropyridin-3-yl)ethyl)amino)-2-methylthiazolo[4,5-d]pyrimidine-7-carbonyl)pyrrolidin-3-yl)acetamide

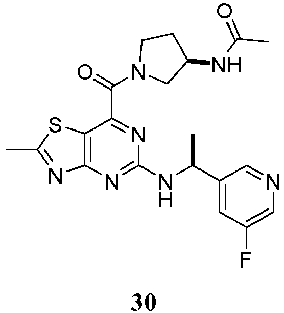

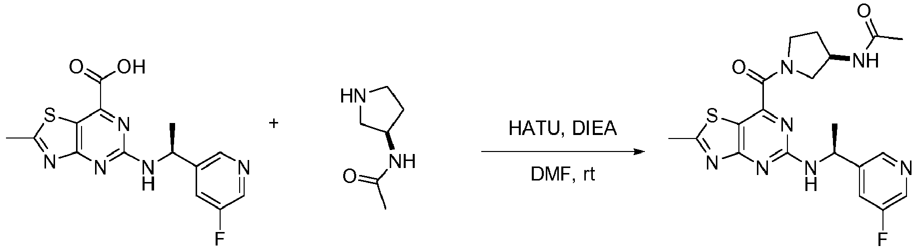

**Synthesis of *N*-((*R*)-1-(5-(((*S*)-1-(5-fluoropyridin-3-yl)ethyl)amino)-2-methylthiazolo[4,5-*d*]pyrimidine-7-carbonyl)pyrrolidin-3-yl)acetamide (30):** The title compound **(30)** was prepared from (*S*)-5-((1-(5-fluoropyridin-3-yl)ethyl)amino)-2-methylthiazolo[4,5-*d*]pyrimidine-7-carboxylic acid (33 mg, 0.099 mmol) using chemistry similar to that described in Example 22 using *N*-[(3*R*)-pyrrolidin-3-yl]acetamide (commercially available from PharmaBlock, Cupertino, CA) in place of tert-butyl 4-(2-aminoethyl)piperidine-1-carboxylate (31% yield). ¹H NMR (400 MHz, CDCl₃) δ 1.64 (3H, dt, *J* = 7.2, 1.6 Hz), 2.01-2.07 (3H, m), 2.84 (3H, m), 3.61-3.91 (4H, m), 4.52 (2H, m), 5.23 (1H, m), 5.59-5.66 (1H, m), 5.86 (1H, br s), 7.45-7.52 (1H, m), 8.34 (1H, m), 8.48-8.53 (1H, m) ppm. ESI MS: m/z [M+H⁺] for C₂₀H₂₂FN₇O₂S, calcd 444.2, found 444.2.

### Example 31. (S)-5-((1-(5-Fluoropyridin-3-yl)ethyl)amino)-2-methyl-N-(4-sulfamoylphenethyl)thiazolo[4,5-d]pyrimidine-7-carboxamide

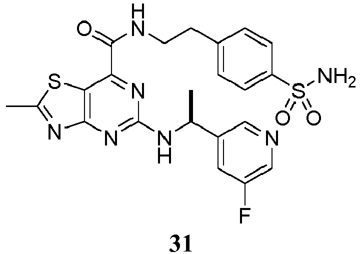

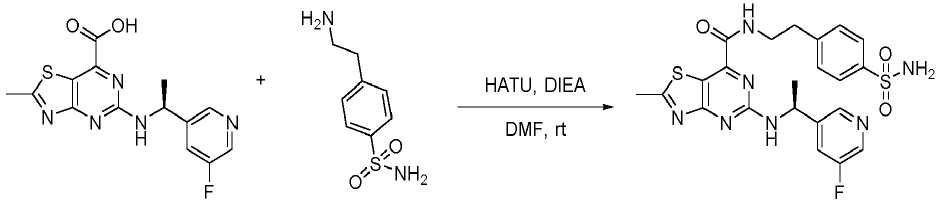

**Synthesis of (*S*)-5-((1-(5-fluoropyridin-3-yl)ethyl)amino)-2-methyl-*N*-(4-sulfamoylphenethyl)thiazolo[4,5-*d*]pyrimidine-7-carboxamide (31):** The title compound **(31)** was prepared from (*S*)-5-((1-(5-fluoropyridin-3-yl)ethyl)amino)-2-methylthiazolo[4,5-*d*]pyrimidine-7-carboxylic acid (25 mg, 0.075 mmol) using chemistry similar to that described in Example 22 using 4-(2-aminoethyl)benzenesulfonamide (commercially available from AK Scientific, Union City, CA) in place of tert-butyl 4-(2-aminoethyl)piperidine-1-carboxylate (12% yield). ¹H NMR (400 MHz, CDCl₃) δ 1.62 (3H, d, *J* = 6.8 Hz), 2.86 (3H, s), 2.98-3.05 (2H, m), 3.71-3.75 (2H, m), 5.12-5.48 (2H, m), 5.60 (1H, br s), 7.34 (1H, d, *J* = 8.4 Hz), 7.54 (1H, d, *J* = 9.2 Hz), 7.60-7.70 (1H, br s), 7.72 (2H, d, *J* = 8.4 Hz), 8.28-8.36 (2H, m) ppm. ESI MS: m/z [M+H⁺] for C₂₂H₂₂FN₇O₃S₂, calcd 516.1, found 516.0.

### Example 32. (S)-5-((1-(5-Fluoropyridin-3-yl)ethyl)amino)-2-methyl-N-(2-(piperidin-4-yl)ethyl)thiazolo[4,5-d]pyrimidine-7-carboxamide

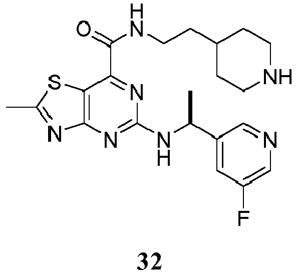

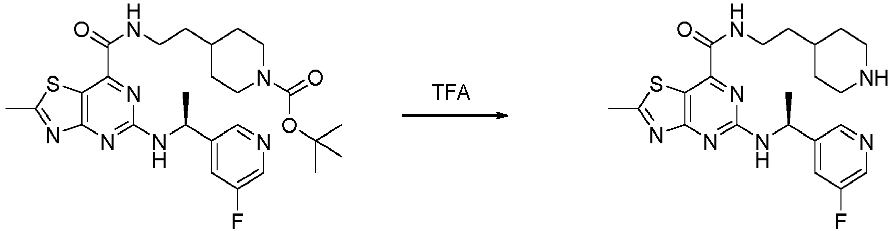

**Synthesis of (*S*)-5-((1-(5-fluoropyridin-3-yl)ethyl)amino)-2-methyl-*N*-(2-(piperidin-4-yl)ethyl)thiazolo[4,5-*d*]pyrimidine-7-carboxamide (32):** To a solution of tert-butyl (*S*)-4-(2-(5-((1-(5-fluoropyridin-3-yl)ethyl)amino)-2-methylthiazolo[4,5-*d*]pyrimidine-7-carboxamido)-ethyl)piperidine-1-carboxylate (28 mg, 0.051 mmol) in DCM (0.5 mL) was added TFA (0.2 mL, 0.65 mmol), and the resulting mixture was stirred for 1 hour and concentrated. The residue was purified by reverse phase HPLC eluting with 5% to 95% acetonitrile in 0.1% TFA in water solution. The fractions containing the desired product were combined, concentrated, dissolved in water, and freeze-dried to provide (*S*)-5-((1-(5-fluoropyridin-3-yl)ethyl)amino)-2-methyl-*N*-(2-(piperidin-4-yl)ethyl)thiazolo[4,5-*d*]pyrimidine-7-carboxamide as TFA salt (36% yield). ¹H NMR (400 MHz, CDCl₃) δ 1.60-1.66 (2H, m), 1.69 (3H, d, *J* = 7.2 Hz), 1.98 (2H, m), 2.86 (3H, s), 3.22-3.56 (6H, m), 5.34 (1H, m), 6.2 (1H, br s), 7.74 (2H, m), 8.41 (1H, d, *J* = 2.0 Hz), 8.69 (1H, s), 9.00 (1H, br s), 9.34 (1H, br s) ppm. ESI MS: m/z [M+H⁺] for C₂₁H₂₆FN₇OS, calcd 444.2, found 444.2.

### Example 33. (S)-1-(5-((1-(5-Fluoropyridin-3-yl)ethyl)amino)-2-methylthiazolo[4,5-d]pyrimidine-7-carbonyl)azetidine-3-carboxamide

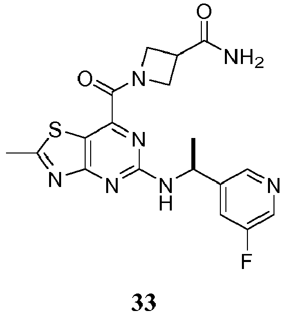

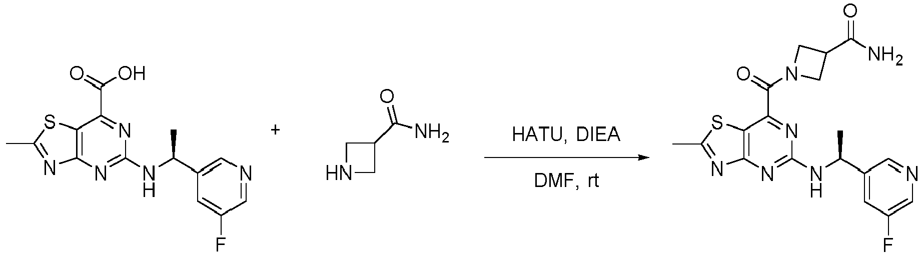

**Synthesis of (*S*)-1-(5-((1-(5-fluoropyridin-3-yl)ethyl)amino)-2-methylthiazolo[4,5-*d*]pyrimidine-7-carbonyl)azetidine-3-carboxamide (33):** The title compound **(33)** was prepared from (*S*)-5-((1-(5-fluoropyridin-3-yl)ethyl)amino)-2-methylthiazolo[4,5-*d*]pyrimidine-7-carboxylic acid (35 mg, 0.105 mmol) using chemistry similar to that described in Example 22 using azetidine-3-carboxamide hydrochloride (commercially available from PharmaBlock, Cupertino, CA) in place of tert-butyl 4-(2-aminoethyl)piperidine-1-carboxylate (15% yield). ¹H NMR (400 MHz, CDCl₃) δ 1.64 (3H, d, *J* = 6.8 Hz), 2.86 (3H, s), 3.30-3.46 (1H, m), 4.28-4.44 (3H, m), 4.84 (1H, br s), 5.25 (1H, br s), 5.45-5.65 (3H, m), 7.46 (1H, d, *J* = 9.2 Hz), 8.34 (1H, t, *J* = 2.8 Hz), 8.49 (1H, d, *J* = 7.2 Hz) ppm. ESI MS: m/z [M+H⁺] for C₁₈H₁₈FN₇O₂S, calcd 416.1, found 416.1.

### Example 34. (S)-1-(3-(5-((1-(5-Fluoropyridin-3-yl)ethyl)amino)-2-methylthiazolo[4,5-d]pyrimidine-7-carbonyl)imidazolidin-1-yl)-3,3-dimethylbutan-1-one

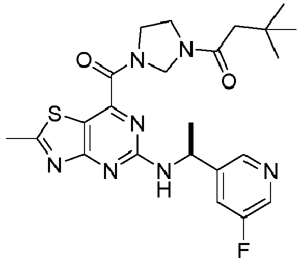

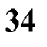

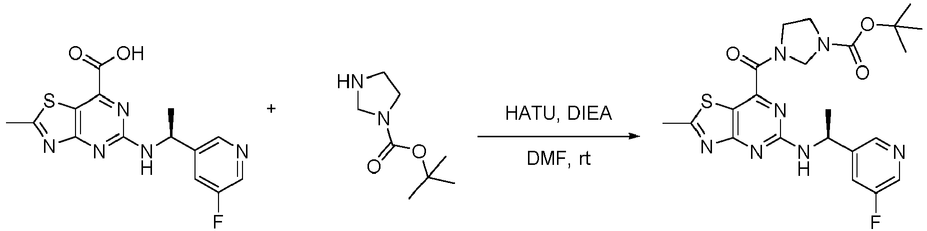

**Synthesis of tert-butyl (*S*)-3-(5-((1-(5-fluoropyridin-3-yl)ethyl)amino)-2-methylthiazolo[4,5-*d*]pyrimidine-7-carbonyl)imidazolidine-1-carboxylate:** The title compound was prepared from (*S*)-5-((1-(5-fluoropyridin-3-yl)ethyl)amino)-2-methylthiazolo[4,5-*d*]pyrimidine-7-carboxylic acid (42 mg, 0.11 mmol) using chemistry similar to that described in Example 22 using tert-butyl imidazolidine-1-carboxylate (commercially available from Oxchem, Wood Dale, IL) in place of tert-butyl 4-(2-aminoethyl)piperidine-1-carboxylate (56% yield). ESI MS: m/z [M+H⁺] for C₂₂H₂₆FN₇O₃S, calcd 488.2, found 488.1.

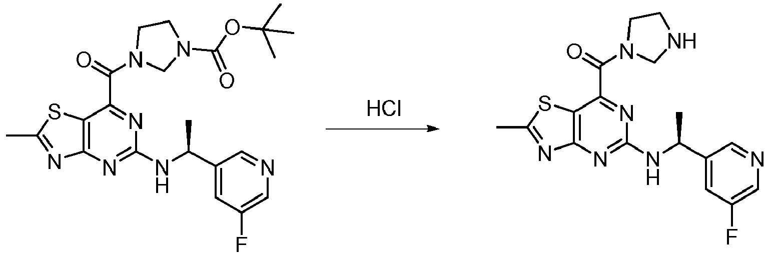

**Synthesis of (*S*)-(5-((1-(5-fluoropyridin-3-yl)ethyl)amino)-2-methylthiazolo[4,5-*d*]pyrimidin-7-yl)(imidazolidin-1-yl)methanone:** To a solution of tert-butyl (*S*)-3-(5-((1-(5-fluoropyridin-3-yl)ethyl)amino)-2-methylthiazolo[4,5-*d*]pyrimidine-7-carbonyl)imidazolidine-1-carboxylate (48 mg, 0.097 mmol) in DCM (0.5 mL) was added a solution of 4M hydrogen chloride in 1,4-dioxane (0.2 mL), the resulting mixture was stirred for 30 min and concentrated to provide crude (*S*)-(5-((1-(5-fluoropyridin-3-yl)ethyl)amino)-2-methylthiazolo[4,5-*d*]pyrimidin-7-yl)(imidazolidin-1-yl)methanone as HCl salt (99% yield). ESI MS: m/z [M+H⁺] for C₁₇H₁₈FN₇OS, calcd 388.1, found 388.1.

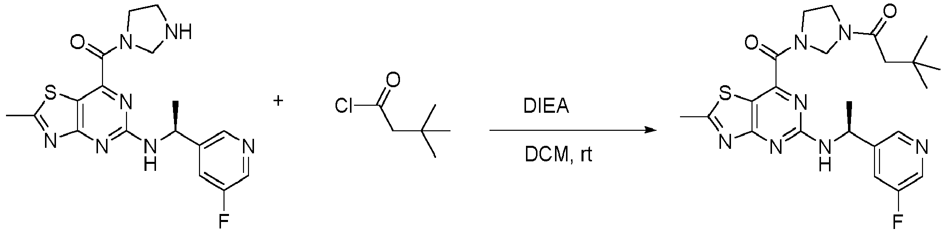

**Synthesis of (*S*)-1-(3-(5-((1-(5-fluoropyridin-3-yl)ethyl)amino)-2-methylthiazolo[4,5-*d*]pyrimidine-7-carbonyl)imidazolidin-1-yl)-3,3-dimethylbutan-1-one (34):** tert-Butylacetyl chloride (commercially available from Sigma-Aldrich, St. Louis, MO) (0.0075 mL, 0.053 mmol) was added to the mixture of (*S*)-(5-((1-(5-fluoropyridin-3-yl)ethyl)amino)-2-methylthiazolo[4,5-*d*]pyrimidin-7-yl)(imidazolidin-1-yl)methanone trihydrochloride (20 mg, 0.04 mmol) and DIEA (0.05 mL, 0.028 mmol) in DCM (1 mL), the resulting mixture was stirred at room temperature for 30 min, concentrated, and the residue was purified by flash chromatography eluting with 2% to 100% solvent A (DCM/MeOH/NH₄OH, 100/10/1) in DCM to provide (*S*)-1-(3-(5-((1-(5-fluoropyridin-3-yl)ethyl)amino)-2-methylthiazolo[4,5-*d*]pyrimidine-7-carbonyl)imidazolidin-1-yl)-3,3-dimethylbutan-1-one (6 mg, 31% yield) as white solids. ¹H NMR (400 MHz, CDCl₃) δ 1.08-1.12 (9H, m), 1.65 (3H, d, *J* = 6.4 Hz), 2.85-2.87 (3H, m), 3.74-3.80 (2H, m), 4.04-4.10 (2H, m), 5.16-5.82 (4H, m), 5.28 (1H, q, *J* = 7.2 Hz), 7.43-7.54 (1H, m), 8.32-8.36 (1H, m), 8.50-8.58 (1H, m) ppm. ESI MS: m/z [M+H⁺] for C₂₃H₂₈FN₇O₂S, calcd 486.2, found 486.2.

### Example 35 and 36. (S)-N-(tert-Butyl)-3-(5-((1-(5-fluoropyridin-3-yl)ethyl)amino)-2-methylthiazolo[4,5-d]pyrimidine-7-carbonyl)imidazolidine-1-carboxamide and (S)-N-(2-(3-(tert-butyl)ureido)ethyl)-5-((1-(5-fluoropyridin-3-yl)ethyl)amino)-2-methylthiazolo[4,5-d]pyrimidine-7-carboxamide

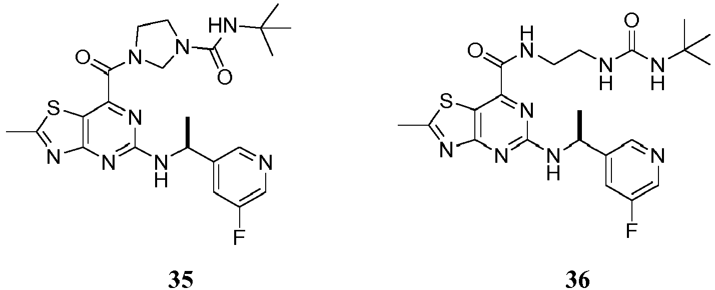

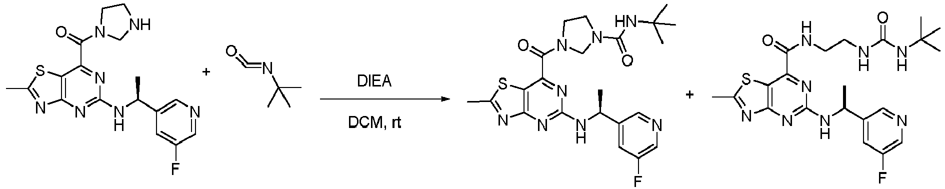

**Synthesis of (*S*)-*N*-(tert-butyl)-3-(5-((1-(5-fluoropyridin-3-yl)ethyl)amino)-2-methyl-thiazolo[4,5-*d*]pyrimidine-7-carbonyl)imidazolidine-1-carboxamide (35) and (*S*)-*N*-(2-(3-(tert-butyl)ureido)ethyl)-5-((1-(5-fluoropyridin-3-yl)ethyl)amino)-2-methylthiazolo[4,5-d]pyrimidine-7-carboxamide (36):** The title compounds **(35 and 36)** were prepared from (*S*)-(5-((1-(5-fluoropyridin-3-yl)ethyl)amino)-2-methylthiazolo[4,5-*d*]pyrimidin-7-yl)(imidazolidin-1-yl)methanone trihydrochloride (27 mg, 0.054 mmol) using chemistry similar to that described in Example 34 using tert-butyl isocyanate (commercially available from Sigma-Aldrich, St. Louis, MO) in place of tert-butylacetyl chloride.

**35:** ¹H NMR (400 MHz, CDCl₃) δ 1. 35-1.40 (9H, m), 1.62-1.66 (3H, m), 2.84-2.86 (3H, m), 3.46-3.50 (2H, m), 4.14-4.98 (2H, m), 5.35-5.85 (3H, m), 7.43-7.54(1H, m), 8.33-8.36 (1H, m), 8.50-8.55 (1H, m) ppm. ESI MS: m/z [M+H⁺] for C₂₂H₂₇FN₈O₂S, calcd 487.2, found 487.3.

**36:** ¹H NMR (400 MHz, CDCl₃) δ 1.34 (9H, s), 1.66 (3H, d, *J* = 6.8 Hz), 2.85 (3H, s), 3.12 (1H, m), 3.46 (1H, m), 3.55-3.80 (2H, m), 4.80 (1H, m), 5.28 (1H, m), 5.36 (1H, m), 7.56 (1H, d, *J* = 8.8 Hz), 8.18 (1H, br s), 8.34 (1H, d, *J* = 2.4 Hz), 8.60 (1H, s) ppm. ESI MS: m/z [M+H⁺] for C₂₁H₂₇FN₈O₂S, calcd 475.2, found 475.3.

### Example 37. (S)-N-(1-(5-((1-(5-Fluoropyridin-3-yl)ethyl)amino)-2-methylthiazolo[4,5-d]pyrimidine-7-carbonyl)azetidin-3-yl)pivalamide

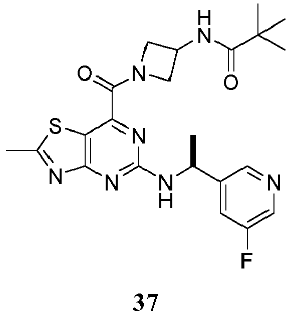

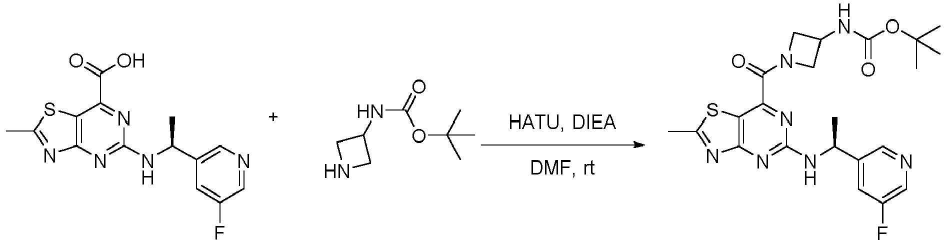

**Synthesis of tert-butyl (*S*)-(1-(5-((1-(5-fluoropyridin-3-yl)ethyl)amino)-2-methyl-thiazolo [4,5-*d*]pyrimidine-7-carbonyl)azetidin-3-yl)carbamate:** The title compound was prepared from (*S*)-5-((1-(5-fluoropyridin-3-yl)ethyl)amino)-2-methylthiazolo[4,5-*d*]pyrimidine-7-carboxylic acid (50 mg, 0.15 mmol) using chemistry similar to that described in Example 22 using tert-butyl *N*-(azetidin-3-yl)carbamate (commercially available from Ark Pharm, Arlington Heights, IL) in place of tert-butyl 4-(2-aminoethyl)piperidine-1-carboxylate (76% yield). ESI MS: m/z [M+H⁺] for C₂₂H₂₆FN₇O₃S, calcd 488.2, found 488.1.

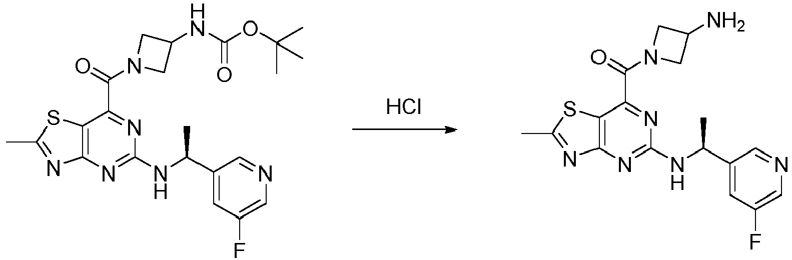

**Synthesis of (*S*)-(3-aminoazetidin-1-yl)(5-((1-(5-fluoropyridin-3-yl)ethyl)amino)-2-methylthiazolo[4,5-*d*]pyrimidin-7-yl)methanone:** The title compounds was prepared from tert-butyl (*S*)-(1-(5-((1-(5-fluoropyridin-3-yl)ethyl)amino)-2-methylthiazolo[4,5-*d*]pyrimidine-7-carbonyl)azetidin-3-yl)carbamate (55 mg, 0.11 mmol) using chemistry similar to that described in Example 34 (100%). ESI MS: m/z [M+H⁺] for C₁₇H₁₈FN₇OS, calcd 388.1, found 388.1.

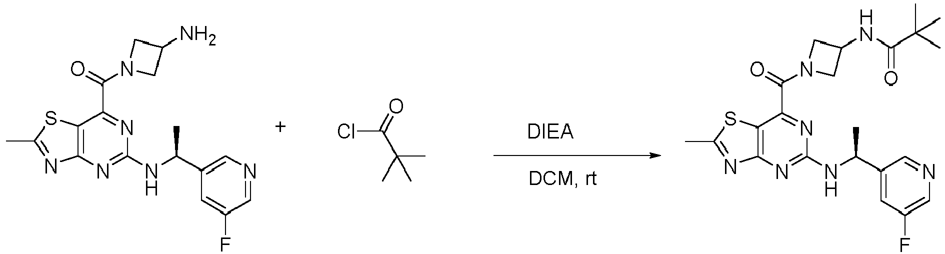

**Synthesis of (*S*)-*N*-(tert-butyl)-3-(5-((1-(5-fluoropyridin-3-yl)ethyl)amino)-2-methylthiazolo[4,5-*d*]pyrimidine-7-carbonyl)imidazolidine-1-carboxamide (37):** The title compound **(37)** was prepared from (*S*)-(3-aminoazetidin-1-yl)(5-((1-(5-fluoropyridin-3-yl)ethyl)amino)-2-methylthiazolo[4,5-*d*]pyrimidin-7-yl)methanone trihydrochloride (16 mg, 0.03 mmol) using chemistry similar to that described in Example 34 using pivaloyl chloride (commercially available from Sigma-Aldrich, St. Louis, MO) in place of tert-butylacetyl chloride (40%). ¹H NMR (400 MHz, CDCl₃) δ 1.24 (9H, s), 1.65 (3H, d, *J* = 6.8 Hz), 2.84 (3H, d, *J* = 1.2 Hz), 3.96-4.10 (1H, m), 4.20-4.30 (1H, m), 4.35-4.60 (1H, m), 4.63-4.70 (1H, m), 4.90-5.15 (1H, m), 5.31 (1H, m), 5.52 (1H, m), 5.98-6.05 (1H, m), 7.43 (1H, t, *J* = 9.2 Hz), 8.35 (1H, t, *J* = 2.4 Hz), 8.51 (1H, d, *J* = 12.4 Hz) ppm. ESI MS: m/z [M+H⁺] for C₂₂H₂₆FN₇O₂S, calcd 472.2, found 472.3.

### Example 38. N-Ethyl-1-(5-(((S)-1-(5-fluoropyridin-3-yl)ethyl)amino)-2-methylthiazolo[4,5-d]pyrimidine-7-carbonyl)pyrrolidine-3-carboxamide

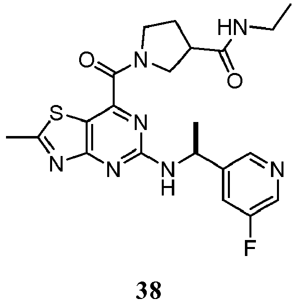

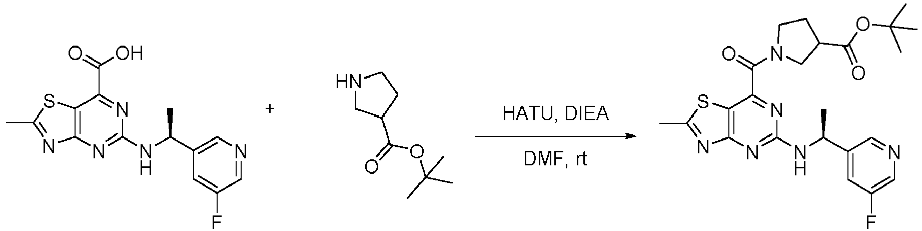

**Synthesis of tert-butyl 1-(5-(((*S*)-1-(5-fluoropyridin-3-yl)ethyl)amino)-2-methylthiazolo[4,5-*d*]pyrimidine-7-carbonyl)pyrrolidine-3-carboxylate:** The title compound was prepared from (*S*)-5-((1-(5-fluoropyridin-3-yl)ethyl)amino)-2-methylthiazolo[4,5-*d*]pyrimidine-7-carboxylic acid (30 mg, 0.09 mmol) using chemistry similar to that described in Example 22 using tert-butyl pyrrolidine-3-carboxylate (commercially available from Acella Pharmaceuticals, Alpharetta, GA) in place of tert-butyl 4-(2-aminoethyl)piperidine-1-carboxylate (55% yield). ESI MS: m/z [M+H⁺] for C₂₃H₂₇FN₆O₃S, calcd 487.2, found 487.1.

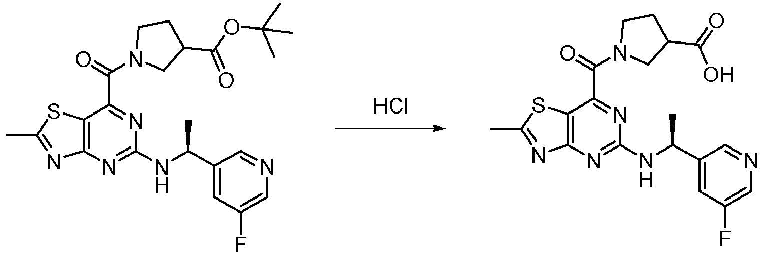

**Synthesis of 1-(5-(((*S*)-1-(5-fluoropyridin-3-yl)ethyl)amino)-2-methylthiazolo[4,5-*d*]pyrimidine-7-carbonyl)pyrrolidine-3-carboxylic acid:** To a solution of tert-butyl (*S*)-3-(5-((1-(5-fluoropyridin-3-yl)ethyl)amino)-2-methylthiazolo[4,5-*d*]pyrimidine-7-carbonyl)imidazolidine-1-carboxylate (48 mg, 0.097 mmol) in DCM (0.5 mL) was added solution of 4M hydrogen chloride in 1,4-dioxane (0.2 mL), the resulting mixture was stirred at room temperature for 6 hours and concentrated to provide crude (*S*)-(5-((1-(5-fluoropyridin-3-yl)ethyl)amino)-2-methylthiazolo[4,5-*d*]pyrimidin-7-yl)(imidazolidin-1-yl)methanone as HCl salt (99% yield). ESI MS: m/z [M+H⁺] for C₁₉H₁₉FN₆O₃S, calcd 431.1, found 431.1.

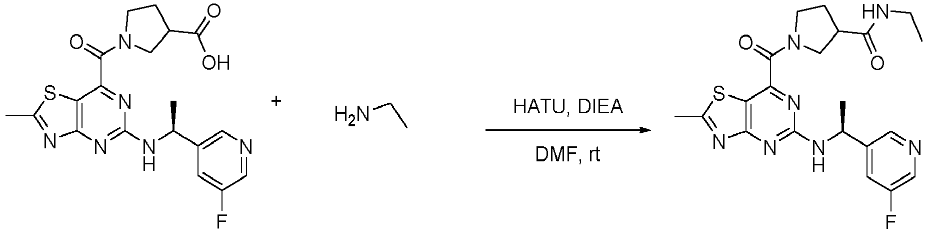

**Synthesis of *N*-ethyl-1-(5-(((*S*)-1-(5-fluoropyridin-3-yl)ethyl)amino)-2-methylthiazolo[4,5-*d*]pyrimidine-7-carbonyl)pyrrolidine-3-carboxamide (38):** The title compound **(38)** was prepared from 1-[5-[[(1*S*)-1-(5-fluoro-3-pyridyl)ethyl]amino]-2-methyl-thiazolo[4,5-*d*]pyrimidine-7-carbonyl]pyrrolidine-3-carboxylic acid dihydrochloride (22 mg, 0.043 mmol) using chemistry similar to that described in Example 22 using ethanamine hydrochloride (commercially available from TCI, Portland, OR) in place of tert-butyl 4-(2-aminoethyl)-piperidine-1-carboxylate (73% yield). ¹H NMR (400 MHz, CDCl₃) δ 1.14-1.20 (3H, m), 1.64 (3H, d, *J* = 6.4 Hz), 2.83 (3H, m), 2.74-2.88 (1H, m), 3.30-3.45 (2H, m), 3.62-4.20 (4H, m), 5.20-5.35 (1H, m), 5.50-5.66 (2H, m), 7.44-7.65 (1H, m), 8.32-8.35 (1H, m), 8.44-8.50 (1H, m), 8.52 (1H, m) ppm. ESI MS: m/z [M+H⁺] for C₂₁H₂₄FN₇O₂S, calcd 458.2, found 458.1.

### Example 39. (S)-N-((1-(5-((1-(5-Fluoropyridin-3-yl)ethyl)amino)-2-methylthiazolo[4,5-d]pyrimidine-7-carbonyl)azetidin-3-yl)methyl)acetamide

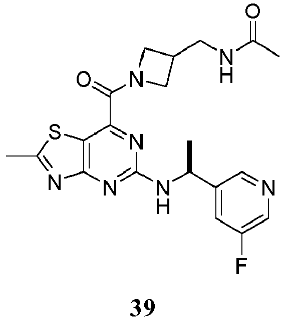

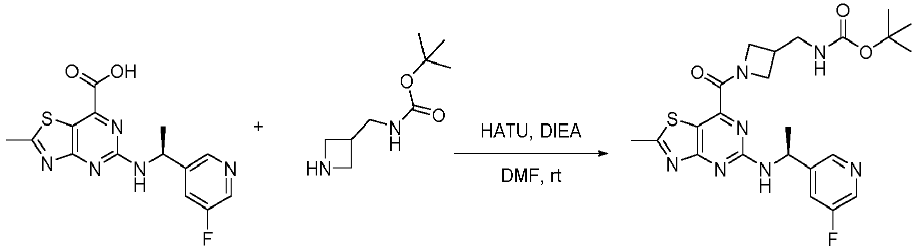

**Synthesis of tert-butyl (*S*)-((1-(5-((1-(5-fluoropyridin-3-yl)ethyl)amino)-2-methyl-thiazolo[4,5-*d*]pyrimidine-7-carbonyl)azetidin-3-yl)methyl)carbamate:** The title compound was prepared from (*S*)-5-((1-(5-fluoropyridin-3-yl)ethyl)amino)-2-methylthiazolo[4,5-*d*]pyrimidine-7-carboxylic acid (90 mg, 0.27 mmol) using chemistry similar to that described in Example 22 using tert-butyl *N*-(azetidin-3-ylmethyl)carbamate (commercially available from PharmaBlock, Cupertino, CA) in place of tert-butyl 4-(2-aminoethyl)piperidine-1-carboxylate (65% yield). ESI MS: m/z [M+H⁺] for C₂₃H₂₈FN₇O₃S, calcd 502.2, found 502.3.

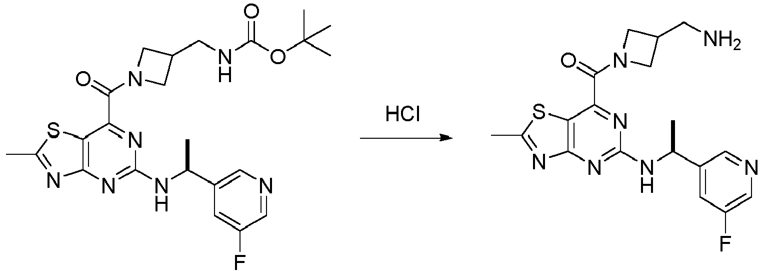

**Synthesis of (*S*)-(3-(aminomethyl)azetidin-1-yl)(5-((1-(5-fluoropyridin-3-yl)ethyl)amino)-2-methylthiazolo[4,5-d]pyrimidin-7-yl)methanone:** The title compounds was prepared from tert-butyl -(*S*)-((1-(5-((1-(5-fluoropyridin-3-yl)ethyl)amino)-2-methyl thiazolo[4,5-*d*]pyrimidine-7-carbonyl)azetidin-3-yl)methyl)carbamate (15 mg, 0.03 mmol) using chemistry similar to that described in Example 34 (100%). ESI MS: m/z [M+H⁺] for C₁₈H₂₀FN₇OS, calcd 402.1, found 402.2.

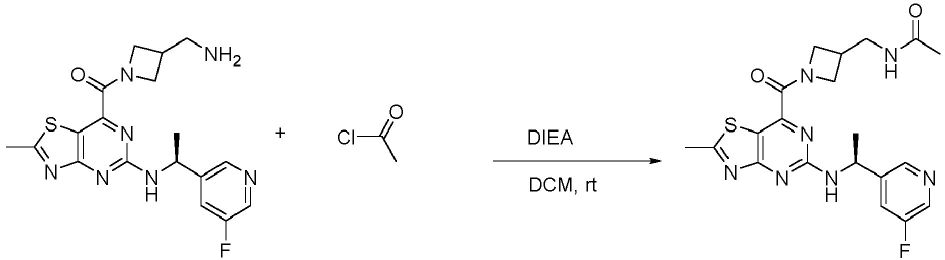

**Synthesis of (*S*)-*N*-((1-(5-((1-(5-fluoropyridin-3-yl)ethyl)amino)-2-methylthiazolo[4,5-*d*]pyrimidine-7-carbonyl)azetidin-3-yl)methyl)acetamide (39):** The title compound **(39)** was prepared from (*S*)-(3-(aminomethyl)azetidin-1-yl)(5-((1-(5-fluoropyridin-3-yl)ethyl)amino)-2-methylthiazolo[4,5-*d*]pyrimidin-7-yl)methanone trihydrochloride (15 mg, 0.029 mmol) using chemistry similar to that described in Example 34 using acetyl chloride (commercially available from Sigma-Aldrich, St. Louis, MO) in place of tert-butylacetyl chloride (40%). ¹H NMR (400 MHz, CDCl₃) δ 1. 44 (1H, d, *J* = 6.8 Hz), 1.52-1.55 (2H, m), 1.65 (3H, dd, *J* = 8.0, 2.0 Hz), 2.09 (3H, d, *J* = 5.2 Hz), 2.90-2.98 (1H, m), 3.62-3.72 (1H, m), 3.75-3.90 (1H, m), 4.25 (2H, t, *J* = 10.0 Hz), 4.74 (1H, m), 5.22 (1H, m), 5.55-5.65 (1H, m), 6.00-6.50 (1H, m), 7.43 (1H, dt, *J* = 9.6, 2.0 Hz), 8.34 (1H, dd, *J* = 6.8, 2.8 Hz), 8.45-8.50 (1H, m) ppm. ESI MS: m/z [M+H⁺] for C₂₀H₂₂FN₇O₂S, calcd 444.2, found 444.2.

### Example 40. (5-(((S)-1-(5-Fluoropyridin-3-yl)ethyl)amino)-2-methylthiazolo[4,5-d]pyrimidin-7-yl)((R)-3-methoxypyrrolidin-1-yl)methanone

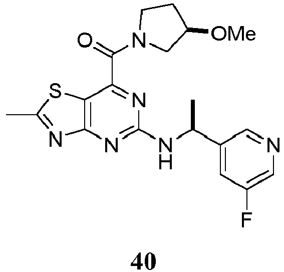

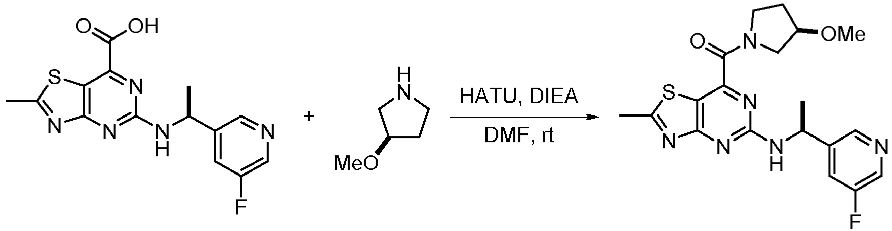

**Synthesis of (5-(((*S*)-1-(5-fluoropyridin-3-yl)ethyl)amino)-2-methylthiazolo[4,5-*d*]pyrimidin-7-yl)((*R*)-3-methoxypyrrolidin-1-yl)methanone (40):** To a solution of (S)-5-((1-(5-fluoropyridin-3-yl)ethyl)amino)-2-methylthiazolo[4,5-*d*]pyrimidine-7-carboxylic acid (17 mg, 0.05 mmol) in DMF (2 mL) were added (3*R*)-3-methoxypyrrolidine hydrochloride (10.3 mg, 0.075 mmol) (commercially obtained from Ark Pharm, Arlington Heights, IL), HATU (28 mg, 0.075 mmol), and *N*,*N*-diisopropylethylamine (35 uL, 0.2 mmol). The mixture thus obtained was stirred at room temperature for 2 h and the reaction mixture was diluted with ethyl acetate and washed with 5% NaHCO₃, water, and brine, then dried over MgSO₄. The solvent was evaporated in vacuo and the residue was purified flash chromatography on silica gel (4 g HP silica, Teledyne Isco) eluting with 10% to 50% solvent A (DCM/MeOH/NH₄OH, 100/10/1) in DCM to provide (5-(((*S*)-1-(5-fluoropyridin-3-yl)ethyl)amino)-2-methylthiazolo[4,5-*d*]pyrimidin-7-yl)((*R*)-3-methoxypyrrolidin-1-yl)methanone as a white solid (16 mg, 77% yield). ¹H NMR (400 MHz, CDCl₃) δ 1.65 (3H, d, *J* = 6.8 Hz), 1.89 (1H, m), 2.08 (1H, m), 2.84 (3H, s), 3.36 (3H, s), 3.64-3.99 (5H, m), 5.27 (1H, m), 5.53 (1H, m), 7.44 (1H, m), 8.35 (1H, m), 8.51 (1H, m) ppm. ESI MS: m/z [M+H⁺] for C₁₉H₂₁FN₆O₂S, calcd 417.1, found 417.2.

### Example 41. (5-(((S)-1-(5-Fluoropyridin-3-yl)ethyl)amino)-2-methylthiazolo[4,5-d]pyrimidin-7-yl)((S)-3-methoxypyrrolidin-1-yl)methanone

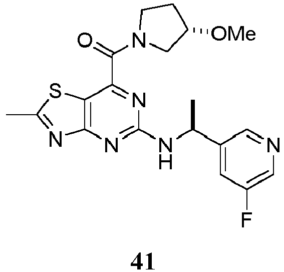

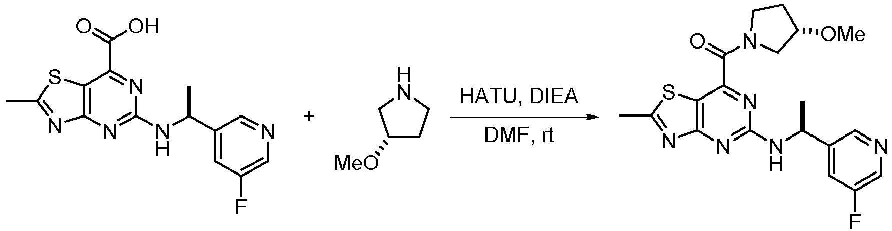

**Synthesis of (5-(((*S*)-1-(5-fluoropyridin-3-yl)ethyl)amino)-2-methylthiazolo[4,5-*d*]pyrimidin-7-yl)((*S*)-3-methoxypyrrolidin-1-yl)methanone (41):** The title compound **(41)** was prepared from (*S*)-5-((1-(5-fluoropyridin-3-yl)ethyl)amino)-2-methylthiazolo[4,5-*d*]pyrimidine-7-carboxylic acid (17 mg, 0.05 mmol) using chemistry similar to that described in Example 40 using (3*S*)-3-methoxypyrrolidine hydrochloride (commercially obtained from Ark Pharm, Arlington Heights, IL) in place of (3*R*)-3-methoxypyrrolidine hydrochloride (14 mg, 67% yield). ¹H NMR (400 MHz, CDCl3) δ 1.63 (3H, d, *J* = 7.2 Hz), 1.88 (1H, m), 2.07 (1H, m), 2.83 (3H, s), 3.34 (3H, s), 3.64-3.97 (5H, m), 5.27 (1H, m), 5.51 (1H, m), 7.43 (1H, m), 8.33 (1H, m), 8.50 (1H, m) ppm. ESI MS: m/z [M+H⁺] for C₁₉H₂₁FN₆O₂S, calcd 417.1, found 417.2.

### Example 42. ((R)-3-Aminopyrrolidin-1-yl)(5-(((S)-1-(5-fluoropyridin-3-yl)ethyl)amino)-2-methylthiazolo[4,5-d]pyrimidin-7-yl)methanone

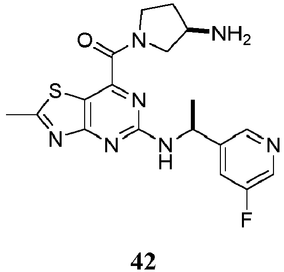

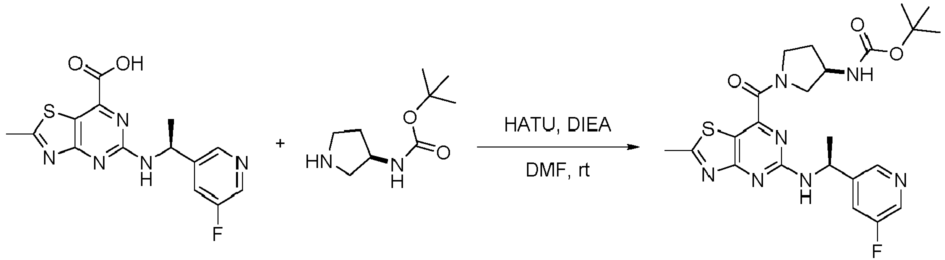

**Synthesis of tert-butyl ((*R*)-1-(5-(((*S*)-1-(5-fluoropyridin-3-yl)ethyl)amino)-2-methylthiazolo[4,5-*d*]pyrimidine-7-carbonyl)pyrrolidin-3-yl)carbamate:** The title compound was prepared from (*S*)-5-((1-(5-fluoropyridin-3-yl)ethyl)amino)-2-methylthiazolo[4,5-*d*]pyrimidine-7-carboxylic acid (45 mg, 0.13 mmol) using chemistry similar to that described in Example 22 using tert-butyl *N*-[(3*R*)-pyrrolidin-3-yl]carbamate (commercially available from CombiBlocks, San Diego, CA) in place of tert-butyl 4-(2-aminoethyl)piperidine-1-carboxylate (41% yield). ESI MS: m/z [M+H⁺] for C₂₃H₂₈FN₇O₃S, calcd 502.2, found 502.3.

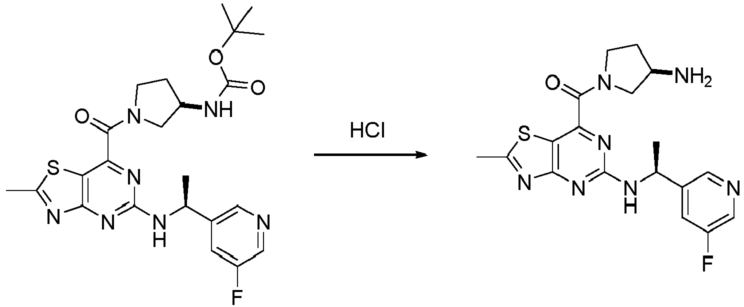

**Synthesis of ((*R*)-3-aminopyrrolidin-1-yl)(5-(((*S*)-1-(5-fluoropyridin-3-yl)ethyl)amino)-2-methylthiazolo[4,5-*d*]pyrimidin-7-yl)methanone (42):** The title compound **(42)** was prepared from tert-butyl ((*R*)-1-(5-(((*S*)-1-(5-fluoropyridin-3-yl)ethyl)amino)-2-methylthiazolo[4,5-*d*]pyrimidine-7-carbonyl)pyrrolidin-3-yl)carbamate using chemistry similar to that described in Example 32 to provide ((*R*)-3-aminopyrrolidin-1-yl)(5-(((*S*)-1-(5-fluoropyridin-3-yl)ethyl)amino)-2-methylthiazolo[4,5-*d*]pyrimidin-7-yl)methanone as trifluoroacetate (15% yield). ¹H NMR (400 MHz, methanol-*d*₄) δ 1.64 (3H, m), 2.08-2.13 (1H, m), 2.36-2.40 (1H, s), 2.82 (3H, s), 3.70-3.90 (1H, m), 3.93-4.01 (2H, m), 4.02-4.50 (1H, m), 5.23-5.29 (1H, m), 7.75 (1H, m), 8.33 (1H, m), 8.52 (1H, m) ppm. ESI MS: m/z [M+H⁺] for C₁₈H₂₀FN₇OS, calcd 402.1, found 402.2.

### Example 43. ((S)-3-Aminopyrrolidin-1-yl)(5-(((S)-1-(5-fluoropyridin-3-yl)ethyl)amino)-2-methylthiazolo[4,5-d]pyrimidin-7-yl)methanone

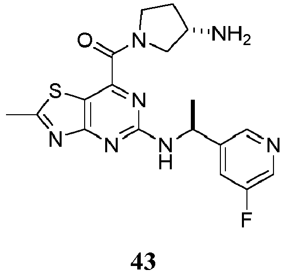

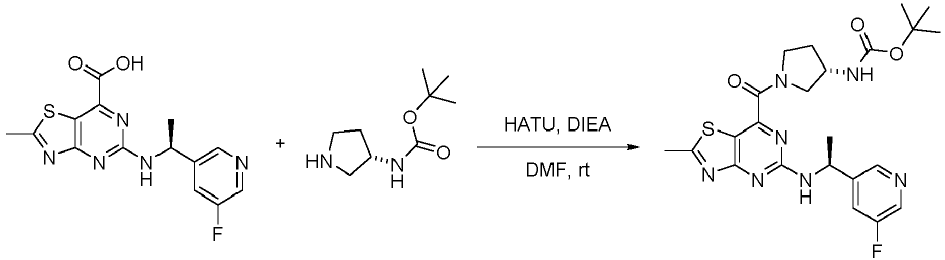

**Synthesis of tert-butyl ((*S*)-1-(5-(((*S*)-1-(5-fluoropyridin-3-yl)ethyl)amino)-2-methylthiazolo[4,5-*d*]pyrimidine-7-carbonyl)pyrrolidin-3-yl)carbamate:** The title compound was prepared from (*S*)-5-((1-(5-fluoropyridin-3-yl)ethyl)amino)-2-methylthiazolo[4,5-*d*]pyrimidine-7-carboxylic acid (45 mg, 0.13 mmol) using chemistry similar to that described in Example 22 using tert-butyl *N*-[(3*S*)-pyrrolidin-3-yl]carbamate (commercially available from CombiBlocks, San Diego, CA) in place of tert-butyl 4-(2-aminoethyl)piperidine-1-carboxylate (45% yield). ESI MS: m/z [M+H⁺] for C₂₃H₂₈FN₇O₃S, calcd 502.2, found 502.3.

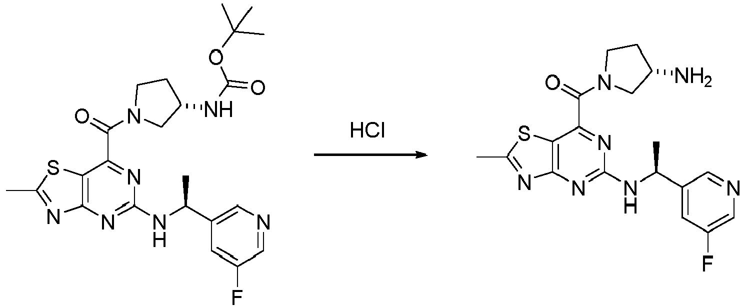

**Synthesis of ((*S*)-3-aminopyrrolidin-1-yl)(5-(((*S*)-1-(5-fluoropyridin-3-yl)ethyl)amino)-2-methylthiazolo[4,5-*d*]pyrimidin-7-yl)methanone (43):** The title compound **(43)** was prepared from tert-butyl ((*S*)-1-(5-(((*S*)-1-(5-fluoropyridin-3-yl)ethyl)amino)-2-methylthiazolo[4,5-*d*]pyrimidine-7-carbonyl)pyrrolidin-3-yl)carbamate using chemistry similar to that described in Example 32 to provide ((*S*)-3-aminopyrrolidin-1-yl)(5-(((*S*)-1-(5-fluoropyridin-3-yl)ethyl)amino)-2-methylthiazolo[4,5-*d*]pyrimidin-7-yl)methanone as trifluoroacetate salt (15% yield). ¹H NMR (400 MHz, methanol-*d*₄) δ 1.65 (3H, m), 2.09-2.13 (1H, m), 2.36-2.40 (1H, s), 2.82 (3H, s), 3.70-3.90 (1H, m), 3.90-4.10 (2H, m), 4.12-4.50 (1H, m), 5.26-5.32 (1H, m), 7.71-7.76 (1H, m), 8.33 (1H, t, *J* = 3.2 Hz), 8.53 (1H, t, *J* = 4.8 Hz) ppm. ESI MS: m/z [M+H⁺] for C₁₈H₂₀FN₇OS, calcd 402.1, found 402.2.

### Example 44. (S)-N-(1-(5-((1-(5-Fluoropyridin-3-yl)ethyl)amino)-2-methylthiazolo[4,5-d]pyrimidine-7-carbonyl)azetidin-3-yl)-N-methylacetamide

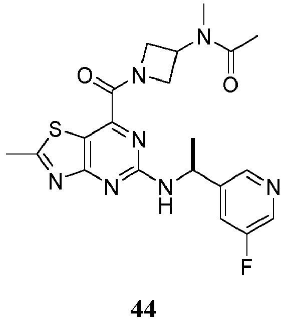

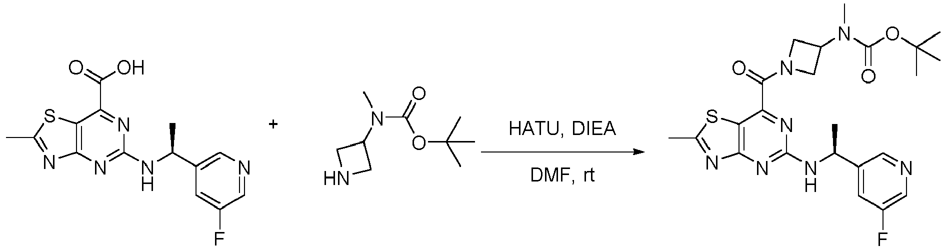

**Synthesis of tert-butyl (*S*)-(1-(5-((1-(5-fluoropyridin-3-yl)ethyl)amino)-2-methylthiazolo[4,5-*d*]pyrimidine-7-carbonyl)azetidin-3-yl)(methyl)carbamate:** The title compound was prepared from (*S*)-5-((1-(5-fluoropyridin-3-yl)ethyl)amino)-2-methylthiazolo[4,5-*d*]pyrimidine-7-carboxylic acid (45 mg, 0.13 mmol) using chemistry similar to that described in Example 22 using tert-butyl *N*-(azetidin-3-yl)-*N*-methylcarbamate hydrochloride (commercially available from Sigma-Aldrich, St. Louis, MO) in place of tert-butyl 4-(2-aminoethyl)piperidine-1-carboxylate (76% yield). ESI MS: m/z [M+H⁺] for C₂₃H₂₈FN₇O₃S, calcd 502.2, found 502.3.

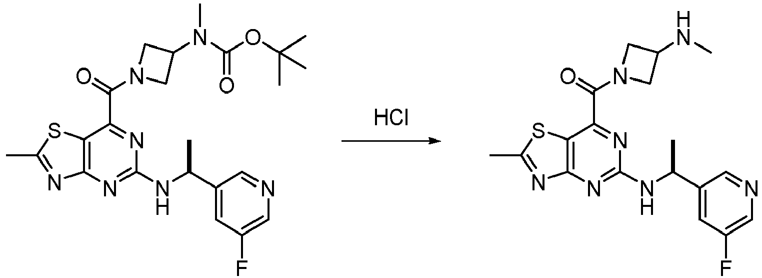

**Synthesis of (*S*)-(5-((1-(5-fluoropyridin-3-yl)ethyl)amino)-2-methylthiazolo[4,5-*d*]pyrimidin-7-yl)(3-(methylamino)azetidin-1-yl)methanone:** The title compounds was prepared from tert-butyl (*S*)-(1-(5-((1-(5-fluoropyridin-3-yl)ethyl)amino)-2-methylthiazolo[4,5-*d*]pyrimidine-7-carbonyl)azetidin-3-yl)(methyl)carbamate (55 mg, 0.11 mmol) using chemistry similar to that described in Example 34 (100%). ESI MS: m/z [M+H⁺] for C₁₈H₂₀FN₇OS, calcd 402.1, found 402.2.

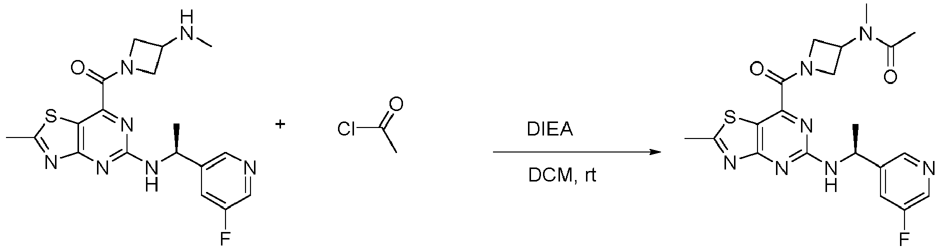

**Synthesis of (*S*)-*N*-(1-(5-((1-(5-fluoropyridin-3-yl)ethyl)amino)-2-methylthiazolo[4,5-*d*]pyrimidine-7-carbonyl)azetidin-3-yl)-*N*-methylacetamide (44):** The title compounds **(44)** was prepared from (*S*)-(5-((1-(5-fluoropyridin-3-yl)ethyl)amino)-2-methylthiazolo[4,5-*d*]pyrimidin-7-yl)(3-(methylamino)azetidin-1-yl)methanone trihydrochloride (16 mg, 0.03 mmol) using chemistry similar to that described in Example 34 using acetyl chloride (commercially available from Sigma-Aldrich, St. Louis, MO) in place of tert-butylacetyl chloride (35%). ¹H NMR (400 MHz, methanol-*d*₄) δ 1.62 (3H, d, *J* = 6.8 Hz), 2.15 (3H, s), 2.84 (3H, s), 3.30 (3H, s), 4.20-4.52 (2H, m), 5.00-5.20 (2H, m), 5.25-5.31 (2H, m), 7.68-7.73 (1H, m), 8.31 (1H, t, *J* = 3.2 Hz), 8.51 (1H, m) ppm. ESI MS: m/z [M+H⁺] for C₂₀H₂₂FN₇O₂S, calcd 444.2, found 444.2.

### Example 45. N-(1-(5-(((S)-1-(5-Fluoropyridin-3-yl)ethyl)amino)-2-methylthiazolo[4,5-d]pyrimidine-7-carbonyl)azetidin-3-yl)tetrahydrofuran-3-carboxamide

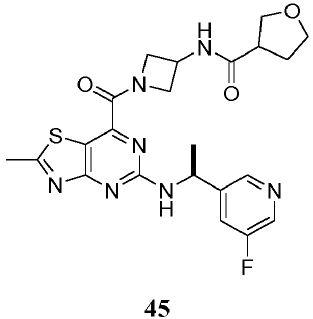

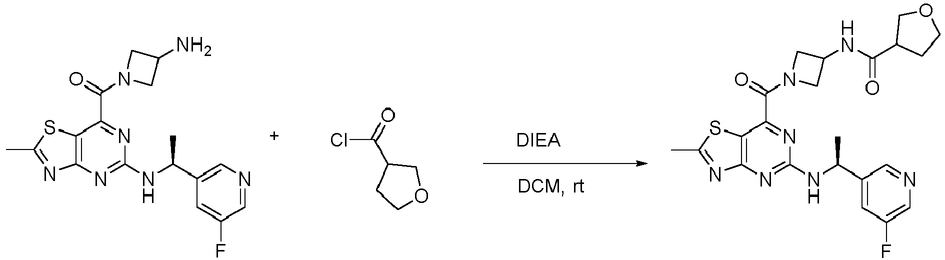

**Synthesis of *N*-(1-(5-(((*S*)-1-(5-fluoropyridin-3-yl)ethyl)amino)-2-methylthiazolo[4,5-*d*]pyrimidine-7-carbonyl)azetidin-3-yl)tetrahydrofuran-3-carboxamide (45):** The title compounds **(45)** was prepared from (*S*)-(3-aminoazetidin-1-yl)(5-((1-(5-fluoropyridin-3-yl)ethyl)amino)-2-methylthiazolo[4,5-*d*]pyrimidin-7-yl)methanone trihydrochloride (16 mg, 0.03 mmol) using chemistry similar to that described in Example 34 using tetrahydrofuran-3-carbonyl chloride (commercially available from Enamine, Monmouth Jct., NJ) in place of tert-butylacetyl chloride (10%). ESI MS: m/z [M+H⁺] for C₂₂H₂₄FN₇O₃S, calcd 486.2, found 486.2.

### Example 46. (S)-5-((1-(5-Fluoropyridin-3-yl)ethyl)amino)-2-methyl-N-(2-morpholinoethyl)thiazolo[4,5-d]pyrimidine-7-carboxamide

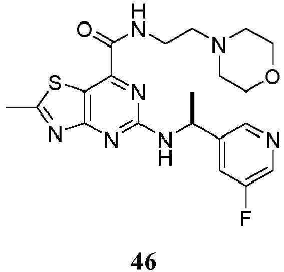

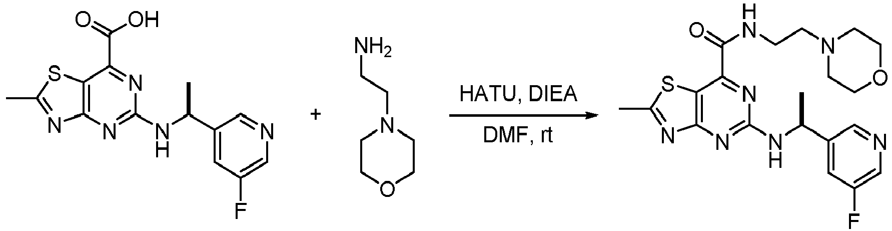

**Synthesis of (*S*)-5-((1-(5-fluoropyridin-3-yl)ethyl)amino)-2-methyl-*N*-(2-morpholinoethyl)thiazolo[4,5-*d*]pyrimidine-7-carboxamide (46):** The title compound **(46)** was prepared from (*S*)-5-((1-(5-fluoropyridin-3-yl)ethyl)amino)-2-methylthiazolo[4,5-*d*]pyrimidine-7-carboxylic acid (17 mg, 0.05 mmol) using chemistry similar to that described in Example 40 using 2-morpholinoethan-1-amine (commercially obtained from Sigma-Aldrich, St. Louis, MO) in place of (3*R*)-3-methoxypyrrolidine hydrochloride (11 mg, 49% yield). ¹H NMR (400 Hz, CDCl3) δ 1.68 (3H, d, *J* = 6.8 Hz), 2.51-2.58 (4H, m), 2.59-2.67 (2H, m), 2.86 (3H, s), 3.57 (2H, q, *J* = 5.6 Hz), 3.77 (4H, t, *J* = 4.8 Hz), 5.30 (1H, m), 5.61 (1H, m), 4.48 (1H, dt, *J* = 9.2, 2.0 Hz), 8.06 (1H, m), 8.36 (1H, d, *J* = 2.8 Hz), 8.57 (1H, m) ppm. ESI MS: m/z [M+H⁺] for C₂₀H₂₄FN₇O₂S, calcd 446.2, found 446.1.

### Example 47. (5-(((S)-1-(5-Fluoropyridin-3-yl)ethyl)amino)-2-methylthiazolo[4,5-d]pyrimidin-7-yl)((R)-3-(methoxymethyl)pyrrolidin-1-yl)methanone

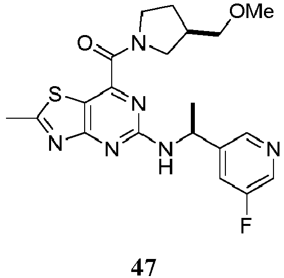

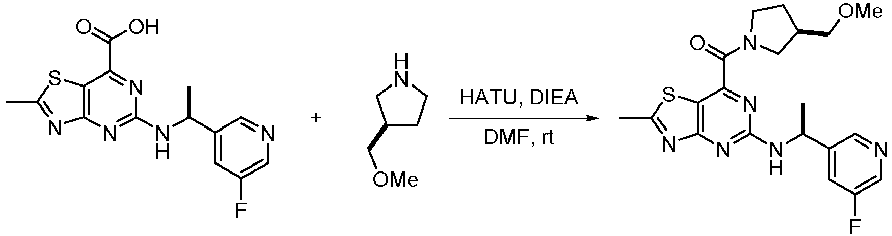

**Synthesis of (5-(((*S*)-1-(5-fluoropyridin-3-yl)ethyl)amino)-2-methylthiazolo[4,5-*d*]pyrimidin-7-yl)((*R*)-3-(methoxymethyl)pyrrolidin-1-yl)methanone (47):** The title compound **(47)** was prepared from (*S*)-5-((1-(5-fluoropyridin-3-yl)ethyl)amino)-2-methylthiazolo[4,5-*d*]pyrimidine-7-carboxylic acid (17 mg, 0.05 mmol) using chemistry similar to that described in Example 40 using (*R*)-3-(methoxymethyl)pyrrolidine (commercially obtained from Ark Pharm, Arlington Heights, IL) in place of (3*R*)-3-methoxypyrrolidine hydrochloride (10 mg, 46% yield). ¹H NMR (400 MHz, CDCl₃) δ 1.63 (3H, d, *J* = 7.2 Hz), 1.72 (1H, m), 2.00 (1H, m), 2.48 (1H, m), 2.82 (3H, s), 3.25-3.46 (6H, m), 3.65 (1H, m), 3.73-3.85 (2H, m), 5.27 (1H, m), 5.54 (1H, m), 7.43 (1H, m), 8.33 (1H, m), 8.49 (1H, m) ppm. ESI MS: m/z [M+H⁺] for C₂₀H₂₃FN₆O₂S, calcd 431.1, found 431.1.

### Example 48. (5-(((S)-1-(5-Fluoropyridin-3-yl)ethyl)amino)-2-methylthiazolo[4,5-d]pyrimidin-7-yl)((S)-3-(methoxymethyl)pyrrolidin-1-yl)methanone

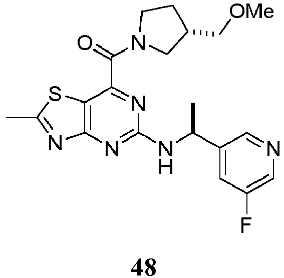

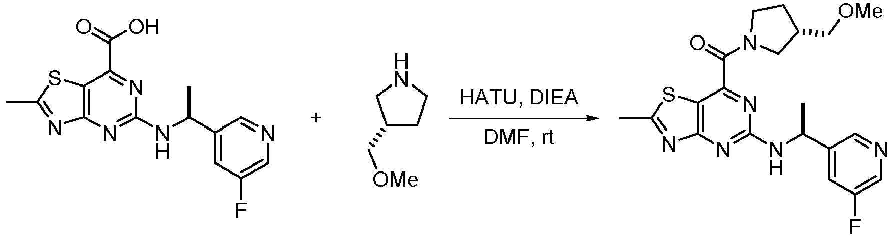

**Synthesis of (5-(((*S*)-1-(5-fluoropyridin-3-yl)ethyl)amino)-2-methylthiazolo[4,5-*d*]pyrimidin-7-yl)((*S*)-3-(methoxymethyl)pyrrolidin-1-yl)methanone (48):** The title compound **(48)** was prepared from (*S*)-5-((1-(5-fluoropyridin-3-yl)ethyl)amino)-2-methylthiazolo[4,5-*d*]pyrimidine-7-carboxylic acid (17 mg, 0.05 mmol) using chemistry similar to that described in Example 40 using (*S*)-3-(methoxymethyl)pyrrolidine (commercially obtained from Ark Pharm, Arlington Heights, IL) in place of (3*R*)-3-methoxypyrrolidine hydrochloride (10 mg, 46% yield). ¹H NMR (400 MHz, CDCl₃) δ 1.65 (3H, d, *J* = 6.8 Hz), 1.74 (1H, m), 2.01 (1H, m), 2.49 (1H, m), 2.84 (3H, s), 3.28-3.48 (6H, m), 3.67 (1H, m), 3.74-3.88 (2H, m), 5.29 (1H, m), 5.55 (1H, m), 7.44 (1H, m), 8.34 (1H, m), 8.51 (1H, m) ppm. ESI MS: m/z [M+H⁺] for C₂₀H₂₃FN₆O₂S, calcd 431.1, found 431.1.

### Example 49. (5-(((S)-1-(5-Fluoropyridin-3-yl)ethyl)amino)-2-methylthiazolo[4,5-d]pyrimidin-7-yl)((R)-3-(hydroxymethyl)pyrrolidin-1-yl)methanone

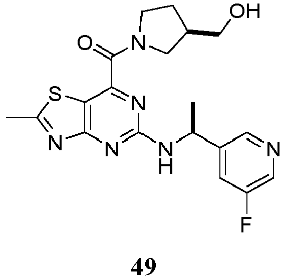

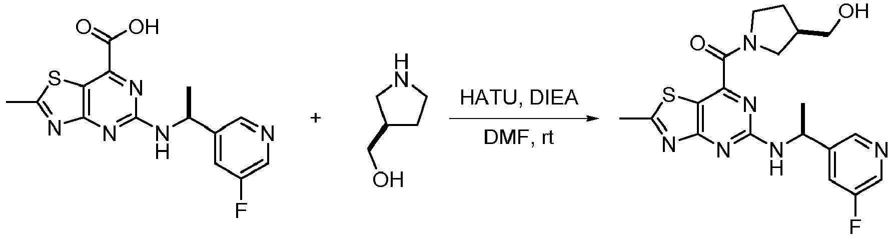

**Synthesis of (5-(((*S*)-1-(5-fluoropyridin-3-yl)ethyl)amino)-2-methylthiazolo[4,5-*d*]pyrimidin-7-yl)((*R*)-3-(hydroxymethyl)pyrrolidin-1-yl)methanone (49):** The title compound **(49)** was prepared from (*S*)-5-((1-(5-fluoropyridin-3-yl)ethyl)amino)-2-methylthiazolo[4,5-*d*]pyrimidine-7-carboxylic acid (17 mg, 0.05 mmol) using chemistry similar to that described in Example 40 using (*R*)-pyrrolidin-3-ylmethanol (commercially obtained from Ark Pharm, Arlington Heights, IL) in place of (3*R*)-3-methoxypyrrolidine hydrochloride (5 mg, 24% yield). ESI MS: m/z [M+H⁺] for C₁₉H₂₁FN₆O₂S, calcd 417.1, found 417.2.

### Example 50. (5-(((S)-1-(5-Fluoropyridin-3-yl)ethyl)amino)-2-methylthiazolo[4,5-d]pyrimidin-7-yl)((S)-3-(hydroxymethyl)pyrrolidin-1-yl)methanone

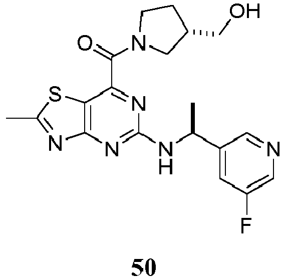

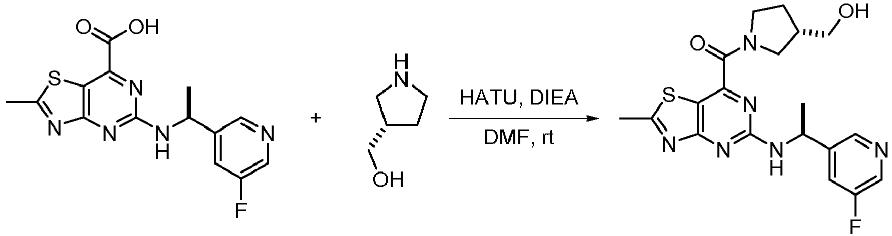

**Synthesis of (5-(((*S*)-1-(5-fluoropyridin-3-yl)ethyl)amino)-2-methylthiazolo[4,5-d]pyrimidin-7-yl)((*S*)-3-(hydroxymethyl)pyrrolidin-1-yl)methanone (50):** The title compound **(50)** was prepared from (*S*)-5-((1-(5-fluoropyridin-3-yl)ethyl)amino)-2-methylthiazolo[4,5-*d*]pyrimidine-7-carboxylic acid (17 mg, 0.05 mmol) using chemistry similar to that described in Example 40 using (*S*)-pyrrolidin-3-ylmethanol (commercially obtained from Ark Pharm, Arlington Heights, IL) in place of (3*R*)-3-methoxypyrrolidine hydrochloride (11 mg, 54% yield). ¹H NMR (400 MHz, CDCl₃) δ 1.64 (3H, d, *J* = 7.2 Hz), 1.70-2.10 (2H, m), 2.46 (1H, m), 2.84 (3H, s), 3.45-3.88 (6H, m), 5.26 (1H, m), 5.65 (1H, m), 7.51 (1H, m), 8.34 (1H, m), 8.53 (1H, m) ppm. ESI MS: m/z [M+H⁺] for C₁₉H₂₁FN₆O₂S, calcd 417.1, found 417.2.

### Example 51. (S)-(5-((1-(5-Fluoropyridin-3-yl)ethyl)amino)-2-methylthiazolo[4,5-d]pyrimidin-7-yl)(3-(methoxymethyl)azetidin-1-yl)methanone

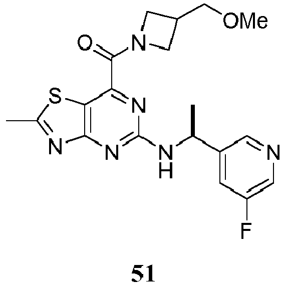

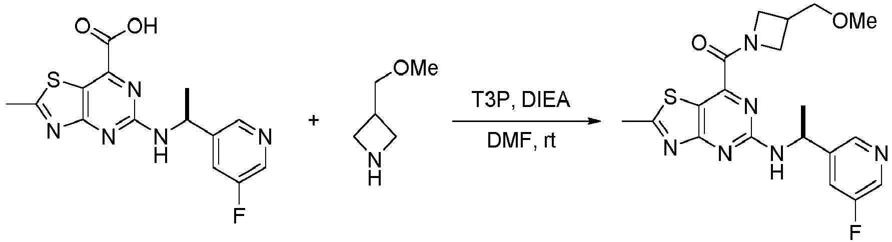

**Synthesis of (*S*)-(5-((1-(5-fluoropyridin-3-yl)ethyl)amino)-2-methylthiazolo[4,5-*d*]pyrimidin-7-yl)(3-(methoxymethyl)azetidin-1-yl)methanone (51):** To a solution of (*S*)-5-((1-(5-fluoropyridin-3-yl)ethyl)amino)-2-methylthiazolo[4,5-*d*]pyrimidine-7-carboxylic acid (17 mg, 0.05 mmol) in DMF (2 mL) were added 3-(methoxymethyl)azetidine hydrochloride (10.3 mg, 0.075 mmol) (commercially obtained from Ark Pharm, Arlington Heights, IL), 50% T3P in DMF (60 uL, 0.10 mmol), and DIEA (35 uL, 0.2 mmol). The mixture thus obtained was stirred at room temperature overnight and the reaction mixture was concentrated and the residue was dissolved in ethyl acetate and washed with 5% NaHCO₃, water, and brine, then dried over MgSO4. The solvent was evaporated and the residue purified by flash chromatography on silica gel (4 g HP silica, Teledyne Isco) eluting with 10% to 50% solvent A (DCM/MeOH/NH₄OH, 100/10/1) in DCM to provide (*S*)-(5-((1-(5-fluoropyridin-3-yl)ethyl)amino)-2-methylthiazolo[4,5-*d*]pyrimidin-7-yl)(3-(methoxymethyl)azetidin-1-yl)methanone as a white solid (8 mg, 38% yield). ¹H NMR (400 MHz, CDCl₃) δ 1.64 (3H, d, *J* = 6.8 Hz), 2.84 (3H, s), 2.91 (1H, m), 3.40 (3H, s), 3.50 (1H, m), 3.56 (1H, d, *J* = 6.0 Hz), 3.96 (1H, td, *J* = 10.4, 5.6 Hz), 4.27 (1H, t, *J* = 10.4 Hz), 4.47 (1H, m), 4.74 (1H, m), 5.30 (1H, m), 5.47 (1H, m), 7.43 (1H, m), 8.35 (1H, d, *J* = 2.4 Hz), 8.50 (1H, m) ppm. ESI MS: m/z [M+H⁺] for C₁₉H₂₁FN₆O₂S, calcd 417.1, found 417.2.

### Example 52. N-(((S)-1-(5-(((S)-1-(5-Fluoropyridin-3-yl)ethyl)amino)-2-methylthiazolo[4,5-d]pyrimidine-7-carbonyl)pyrrolidin-3-yl)methyl)acetamide

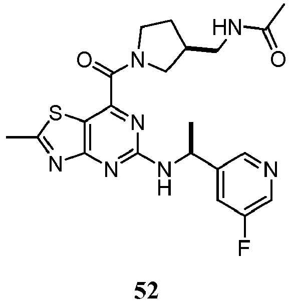

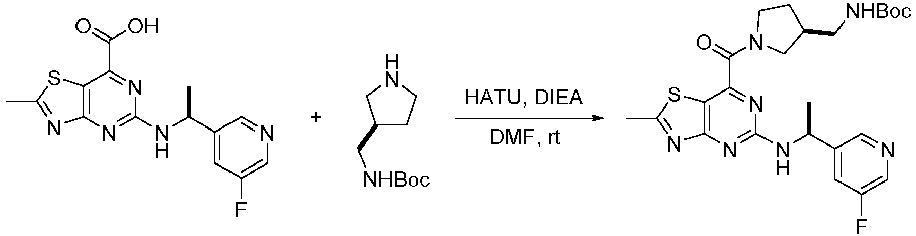

**Synthesis of tert-butyl (((*S*)-1-(5-(((*S*)-1-(5-fluoropyridin-3-yl)ethyl)amino)-2-methylthiazolo[4,5-*d*]pyrimidine-7-carbonyl)pyrrolidin-3-yl)methyl)carbamate:** The title compound was prepared from (*S*)-5-((1-(5-fluoropyridin-3-yl)ethyl)amino)-2-methylthiazolo[4,5-*d*]pyrimidine-7-carboxylic acid (34 mg, 0.1 mmol) using chemistry similar to that described in Example 40 using tert-butyl (*R*)-(pyrrolidin-3-ylmethyl)carbamate (commercially obtained from Ark Pharm, Arlington Heights, IL) in place of (3R)-3-methoxypyrrolidine hydrochloride (32 mg, 63% yield). ESI MS: m/z [M+H⁺] for C₂₄H₃₀FN₇O₃S, calcd 516.2, found 516.2.

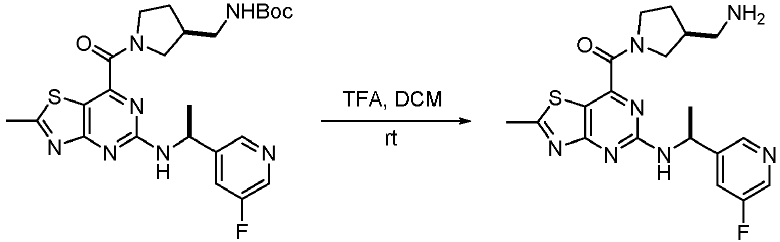

**Synthesis of ((*S*)-3-(aminomethyl)pyrrolidin-1-yl)(5-(((*S*)-1-(5-fluoropyridin-3-yl)ethyl)amino)-2-methylthiazolo[4,5-d]pyrimidin-7-yl)methanone:** A solution of tert-butyl (((*S*)-1-(5-(((*S*)-1-(5-fluoropyridin-3-yl)ethyl)amino)-2-methylthiazolo[4,5-*d*]pyrimidine-7-carbonyl)pyrrolidin-3-yl)methyl)carbamate (29 mg, 0.056 mmol) in TFA/DCM (1/1, 2 mL) was stirred at room temperature for 30 min and the reaction mixture was concentrated and the residue was purified by flash chromatography on silica gel (4 g HP silica, Teledyne Isco) eluting with 50% to 75% solvent A (DCM/MeOH/NH₄OH, 100/10/1) in DCM to provide ((*S*)-3-(aminomethyl)pyrrolidin-1-yl)(5-(((*S*)-1-(5-fluoropyridin-3-yl)ethyl)amino)-2-methylthiazolo[4,5-*d*]pyrimidin-7-yl)methanone as a light yellow solid (22 mg, 94% yield). ESI MS: m/z [M+H⁺] for C₁₉H₂₂FN₇OS, calcd 416.2, found 416.1.

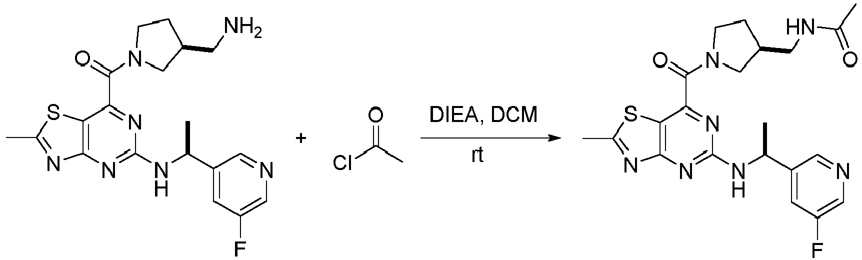

**Synthesis of *N*-(((*S*)-1-(5-(((*S*)-1-(5-fluoropyridin-3-yl)ethyl)amino)-2-methylthiazolo[4,5-*d*]pyrimidine-7-carbonyl)pyrrolidin-3-yl)methyl)acetamide (52):** To a solution of ((*S*)-3-(aminomethyl)pyrrolidin-1-yl)(5-(((*S*)-1-(5-fluoropyridin-3-yl)ethyl)amino)-2-methylthiazolo[4,5-*d*]pyrimidin-7-yl)methanone (22 mg, 0.053 mmol) in DCM (2 mL) were added DIEA (18 uL, 0.106 mmol) and acetyl chloride (5 uL, 0.064 mmol) and the mixture thus obtained was stirred at room temperature for 1 h. The reaction mixture was diluted with ethyl acetate and washed with 5% NaHCO₃, water, and brine, then dried over MgSO₄. The solvent was removed in vacuo and the residue was purified by flash chromatography on silica gel (4 g HP silica, Teledyne Isco) eluting with 10% to 50% solvent A (DCM/MeOH/NH₄OH, 100/10/1) in DCM to provide *N*-(((*S*)-1-(5-(((*S*)-1-(5-fluoropyridin-3-yl)ethyl)amino)-2-methylthiazolo[4,5-*d*]pyrimidine-7-carbonyl)pyrrolidin-3-yl)methyl)acetamide as a white solid (13 mg, 54% yield). ¹H NMR (400 MHz, CDCl₃) δ 1.67 (3H, d, *J* = 7.2 Hz), 1.79 (1H, m), 2.06 (3H, s), 2.10 (1H, m), 2.57 (1H, m), 2.86 (3H, s), 3.24 (1H, m), 3.34-3.75 (5H, m), 5.30 (1H, m), 5.64 (1H, m), 7.47 (1H, m), 7.79 (1H, m), 8.36 (1H, d, *J* = 2.8 Hz), 8.55 (1H, m) ppm. ESI MS: m/z [M+H⁺] for C₂₁H₂₄FN₇O₂S, calcd 458.2, found 458.1.

### Example 53. N-(((R)-1-(5-(((S)-1-(5-Fluoropyridin-3-yl)ethyl)amino)-2-methylthiazolo[4,5-d]pyrimidine-7-carbonyl)pyrrolidin-3-yl)methyl)acetamide

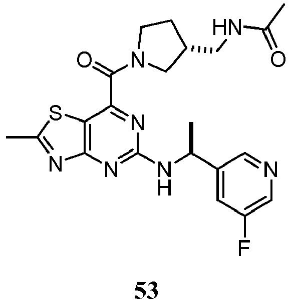

**Synthesis of *N*-(((*R*)-1-(5-(((*S*)-1-(5-fluoropyridin-3-yl)ethyl)amino)-2-methylthiazolo[4,5-*d*]pyrimidine-7-carbonyl)pyrrolidin-3-yl)methyl)acetamide (53):** The title compound (**53**) was synthesized in similar fashion to example 52. ¹H NMR (400 MHz, CDCl₃) δ 1.67 (3H, d, *J* = 6.8 Hz), 1.79 (1H, m), 2.06 (3H, s), 2.10 (1H, m), 2.56 (1H, m), 2.86 (3H, s), 3.24 (1H, m), 3.33-3.75 (5H, m), 5.30 (1H, m), 5.66 (1H, m), 7.48 (1H, m), 7.81 (1H, m), 8.36 (1H, d, *J* = 2.8 Hz), 8.55 (1H, m) ppm. ESI MS: m/z [M+H⁺] for C₂₁H₂₄FN₇O₂S, calcd 458.2, found 458.1.

### Example 54. (S)-N-(1-(5-((1-(5-Fluoropyridin-3-yl)ethyl)amino)-2-methylthiazolo[4,5-d]pyrimidine-7-carbonyl)azetidin-3-yl)-1-methylcyclopropane-1-carboxamide

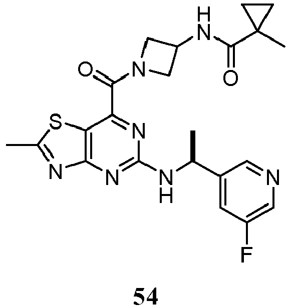

**Synthesis of (*S*)-*N*-(1-(5-((1-(5-fluoropyridin-3-yl)ethyl)amino)-2-methylthiazolo[4,5-*d*]pyrimidine-7-carbonyl)azetidin-3-yl)-1-methylcyclopropane-1-carboxamide (54):** To a solution of (*S*)-(3-aminoazetidin-1-yl)(5-((1-(5-fluoropyridin-3-yl)ethyl)amino)-2-methylthiazolo[4,5-*d*]pyrimidin-7-yl)methanone (9.7 mg, 0.025 mmol) in DCM (1 mL) were added DIEA (9 uL, 0.05 mmol) and 1-methylcyclopropanecarbonyl chloride (4 uL, 0.0375 mmol) (commercially available from PharmaBlock, Nanjing, China) and the mixture thus obtained was stirred at room temperature for 1 h. The reaction mixture was diluted with ethyl acetate and washed with 5% NaHCO₃, water, and brine, then dried. The solvent was evaporated and the residue was purified by flash chromatography on silica gel (4 g HP silica, Teledyne Isco) eluting with 10% to 45% solvent A (DCM/MeOH/NH₄OH, 100/10/1) in DCM to provide **54** as a white solid (9.2 mg, 78% yield). ¹H NMR (400 MHz, DMSO-d₆ at 80 °C) δ 0.52-0.56 (2H, m), 0.98-1.04 (2H, m), 1.30 (3H, s), 1.57 (3H, d, *J* = 6.8 Hz), 2.78 (3H, s), 4.06 (1H, m), 4.32 (1H, m), 4.50 (1H, m), 4.61 (1H, m), 4.92 (1H, m), 5.25 (1H, quintet, *J* = 6.8 Hz), 7.70 (1H, m), 7.82-7.89 (2H, m), 8.38 (1H, d, *J* = 2.4 Hz), 8.52 (1H, d, *J* = 6.0 Hz) ppm. ESI MS: m/z [M+H⁺] for C₂₂H₂₄FN₇O₂S, calcd 470.1, found 470.1.

### Example 55. (S)-N-(1-(5-((1-(5-Fluoropyridin-3-yl)ethyl)amino)-2-methylthiazolo[4,5-d]pyrimidine-7-carbonyl)azetidin-3-yl)cyclopentanecarboxamide

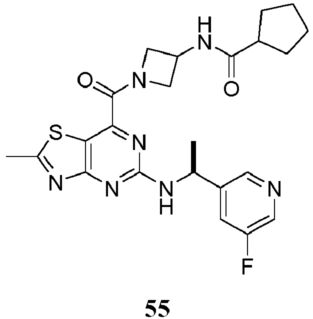

**Synthesis of (*S*)-*N*-(1-(5-((1-(5-fluoropyridin-3-yl)ethyl)amino)-2-methylthiazolo[4,5-*d*]pyrimidine-7-carbonyl)azetidin-3-yl)cyclopentanecarboxamide (55):** The title compound **55** was synthesized in similar fashion to example 54. ¹H NMR (400 MHz, DMSO-d₆ at 80 °C) δ 1.47-1.58 (2H, m), 1.57 (3H, d, *J* = 7.2 Hz), 1.60-1.73 (4H, m), 1.73-1.83 (2H, m), 2.58 (1H, m), 2.78 (3H, s), 3.96 (1H, m), 4.35 (1H, m), 4.45 (1H, m), 4.55 (1H, m), 4.94 (1H, m), 5.25 (1H, quintet, *J* = 7.2 Hz), 7.70 (1H, m), 7.84 (1H, d, *J* = 8.0 Hz), 8.19 (1H, m), 8.39 (1H, s), 8.53 (1H,m) ppm. ESI MS: m/z [M+H⁺] for C₂₃H₂₆FN₇O₂S, calcd 484.2, found 484.1.

### Example 56. (S)-N-(1-(5-((1-(5-Fluoropyridin-3-yl)ethyl)amino)-2-methylthiazolo[4,5-d]pyrimidine-7-carbonyl)azetidin-3-yl)cyclobutanecarboxamide

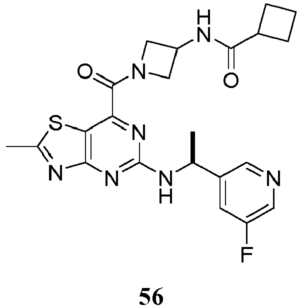

**Synthesis of (*S*)-*N*-(1-(5-((1-(5-fluoropyridin-3-yl)ethyl)amino)-2-methylthiazolo[4,5-*d*]pyrimidine-7-carbonyl)azetidin-3-yl)cyclobutanecarboxamide (56):** The title compound **56** was synthesized in similar fashion to example 54. ¹H NMR (400 MHz, DMSO-d₆ at 80 °C) δ1.57 (3H, d, *J* = 7.2 Hz), 1.82 (1H, m), 1.90 (1H, m), 2.06-2.12 (2H, m), 2.12-2.22 (2H, m), 2.78 (3H, s), 3.02 (1H, m), 3.96 (1H, m), 4.34 (1H, m), 4.45 (1H, m), 4.55 (1H, m), 4.93 (1H, m), 5.25 (1H, quintet, *J* = 7.2 Hz), 7.70 (1H, m), 7.84 (1H, d, *J* = 8.0 Hz), 8.09 (1H, m), 8.38 (1H, s), 8.53 (1H, m) ppm. ESI MS: m/z [M+H⁺] for C₂₂H₂₄FN₇O₂S, calcd 470.2, found 470.1.

### Example 57. (S)-1-(tert-Butyl)-3-(1-(5-((1-(5-fluoropyridin-3-yl)ethyl)amino)-2-methylthiazolo[4,5-d]pyrimidine-7-carbonyl)azetidin-3-yl)urea

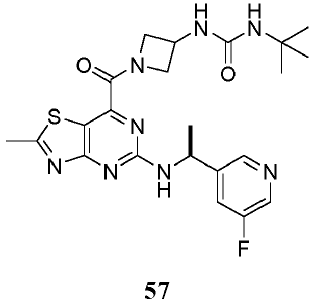

**Synthesis of (*S*)-1-(tert-butyl)-3-(1-(5-((1-(5-fluoropyridin-3-yl)ethyl)amino)-2-methylthiazolo[4,5-*d*]pyrimidine-7-carbonyl)azetidin-3-yl)urea (57):** To a solution of (*S*)-(3-aminoazetidin-1-yl)(5-((1-(5-fluoropyridin-3-yl)ethyl)amino)-2-methylthiazolo[4,5-*d*]pyrimidin-7-yl)methanone (9.7 mg, 0.025 mmol) in DCM (1 mL) were added DIEA (13 uL, 0.075 mmol) and tert-butyl isocyanate (6 uL, 0.05 mmol) (commercially obtained from Sigma-Aldrich, St. Louis, MO) and the mixture thus obtained was stirred at room temperature for 2 h and 70% conversion was observed. Additional tert-butyl isocyanate (6 uL) was added and the reaction mixture was stirred additional 2 h and completion of the reaction was observed. The reaction mixture was concentrated and the residue was purified by flash chromatography on silica gel (4 g HP silica, Teledyne Isco) eluting with 15% to 50% solvent A (DCM/MeOH/NH₄OH, 100/10/1) in DCM to provide **57** as a white solid (12 mg, 99% yield). ¹H NMR (400 MHz, DMSO-d₆ at 80 °C) δ 1.26 (9H, s), 1.57 (3H, d, *J* = 7.2 Hz), 2.78 (3H, s), 3.85 (1H, dd, *J* = 10.0, 4.4 Hz), 4.33 (1H, m), 4.40 (1H, m), 4.44 (1H, m), 4.92 (1H, m), 5.26 (1H, quintet, *J* = 7.2 Hz), 5.56 (1H, s), 6.26 (1H, m), 7.71 (1H, m), 7.83 (1H, m), 8.39 (1H, d, *J* = 2.4 Hz), 8.53 (1H, s) ppm. ESI MS: m/z [M+H⁺] for C₂₂H₂₇FN₈O₂S, calcd 487.2, found 487.3.

### Example 58. (S)-N-(1-(5-((1-(5-Fluoropyridin-3-yl)ethyl)amino)-2-methylthiazolo[4,5-d]pyrimidine-7-carbonyl)azetidin-3-yl)benzamide

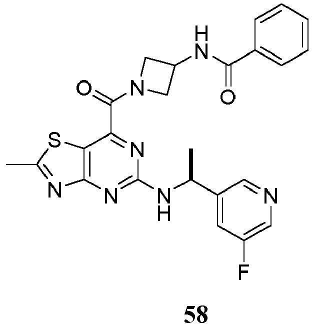

**Synthesis of (*S*)-*N*-(1-(5-((1-(5-fluoropyridin-3-yl)ethyl)amino)-2-methylthiazolo[4,5-*d*]pyrimidine-7-carbonyl)azetidin-3-yl)benzamide (58):** The title compound **58** was synthesized in similar fashion to example 54. ¹H NMR (400 MHz, DMSO-d₆ at 80 °C) δ 1.57 (3H, d, *J* = 7.2 Hz), 2.79 (3H, s), 4.17 (1H, dd, *J* = 10.4, 4.4 Hz), 4.44 (1H, m), 4.64 (1H, m), 4.81 (1H, m), 5.03 (1H, m), 5.27 (1H, quintet, *J* = 7.2 Hz), 7.46-7.50 (2H, m), 7.55 (1H, m), 7.71 (1H, m), 7.85 (1H, d, *J* = 8.0 Hz), 7.88-7.91(2H, m), 8.37 (1H, dd, *J* = 8.0, 2.4 Hz), 8.54 (1H, m), 8.85 (1H, m) ppm. ESI MS: m/z [M+H⁺] for C₂₄H₂₂FN₇O₂S, calcd 492.2, found 492.2.

### Example 59. (S)-2-Fluoro-N-(1-(5-((1-(5-fluoropyridin-3-yl)ethyl)amino)-2-methylthiazolo[4,5-d]pyrimidine-7-carbonyl)azetidin-3-yl)benzamide

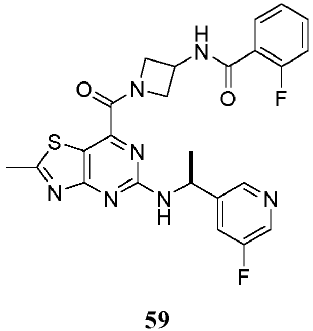

**Synthesis of (*S*)-2-fluoro-*N*-(1-(5-((1-(5-fluoropyridin-3-yl)ethyl)amino)-2-methylthiazolo[4,5-*d*]pyrimidine-7-carbonyl)azetidin-3-yl)benzamide (59):** The title compound **59** was synthesized in similar fashion to example 54. ¹H NMR (400 MHz, DMSO-d₆ at 80 °C) δ 1.57 (3H, d, *J* = 7.2 Hz), 2.79 (3H, s), 4.12 (1H, dd, *J* = 10.0, 4.4 Hz), 4.43 (1H, m), 4.62 (1H, m), 4.79 (1H, m), 5.03 (1H, m), 5.27 (1H, quintet, *J* = 7.2 Hz), 7.25-7.31 (2H, m), 7.55 (1H, m), 7.65-7.72 (2H, m), 7.86 (1H, d, *J* = 8.0 Hz), 8.38 (1H, dd, *J* = 8.0, 2.4 Hz), 8.54 (1H, m), 8.78 (1H, m) ppm. ESI MS: m/z [M+H⁺] for C₂₄H₂₁F₂N₇O₂S, calcd 510.1, found 510.2.

### Example 60. (S)-1-Cyclopentyl-3-(1-(5-((1-(5-fluoropyridin-3-yl)ethyl)amino)-2-methylthiazolo[4,5-d]pyrimidine-7-carbonyl)azetidin-3-yl)urea

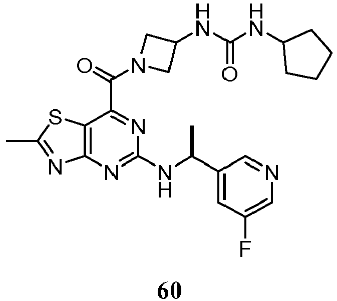

**Synthesis of (*S*)-1-cyclopentyl-3-(1-(5-((1-(5-fluoropyridin-3-yl)ethyl)amino)-2-methylthiazolo[4,5-*d*]pyrimidine-7-carbonyl)azetidin-3-yl)urea (60):** The title compound **60** was synthesized in similar fashion to example 57. ¹H NMR (400 MHz, DMSO-d₆ at 80 °C) δ 1.30-1.38 (2H, m), 1.48-1.53 (2H, m), 1.57 (3H, d, *J* = 7.2 Hz), 1.60-1.65 (2H, m), 1.77-1.83 (2H, m), 2.78 (3H, s), 3.86-3.91 (2H, m), 4.32 (1H, m), 4.43 (1H, m), 4.47 (1H, m), 4.91 (1H, m), 5.26 (1H, quintet, *J* = 7.2 Hz), 5.78 (1H, d, *J* = 7.2 Hz), 6.28 (1H, m), 7.71 (1H, m), 7.83 (1H, d, *J* = 8.0 Hz), 8.39 (1H, d, *J* = 2.0 Hz), 8.53 (1H, m) ppm. ESI MS: m/z [M+H⁺] for C₂₃H₂₇FN₈O₂S, calcd 499.2, found 499.3.

### Example 61. (S)-N-(1-(5-((1-(5-Fluoropyridin-3-yl)ethyl)amino)-2-methylthiazolo[4,5-d]pyrimidine-7-carbonyl)azetidin-3-yl)cyclohexanecarboxamide

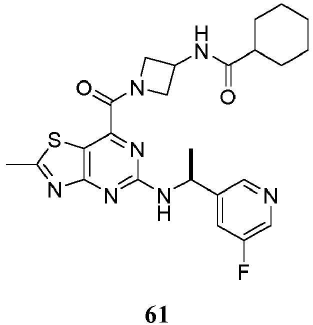

**Synthesis of (*S*)-*N*-(1-(5-((1-(5-fluoropyridin-3-yl)ethyl)amino)-2-methylthiazolo[4,5-*d*]pyrimidine-7-carbonyl)azetidin-3-yl)cyclohexanecarboxamide (61):** The title compound **61** was synthesized in similar fashion to example 54. ¹H NMR (400 MHz, DMSO-d₆ at 80 °C) δ 1.16-1.30 (3H, m), 1.33-1.42 (2H, m), 1.57 (3H, d, *J* = 7.2 Hz), 1.63 (1H, m), 1.71-1.78 (4H, m), 2.12 (1H, m), 2.78 (3H, s), 3.95 (1H, m), 4.34 (1H, m), 4.44 (1H, m), 4.53 (1H, m), 4.93 (1H, m), 5.25 (1H, quintet, *J* = 7.2 Hz), 7.70 (1H, m), 7.84 (1H, d, *J* = 8.0 Hz), 8.12 (1H, m), 8.38 (1H, m), 8.52 (1H, m) ppm. ESI MS: m/z [M+H⁺] for C₂₄H₂₈FN₇O₂S, calcd 498.2, found 498.2.

### Example 62. 2,2-Dimethyl-N-[1-[2-methyl-5-[[(S)-1-(2-pyridyl)ethyl]amino]thiazolo[4,5-d]pyrimidine-7-carbonyl]azetidin-3-yl]propanamide

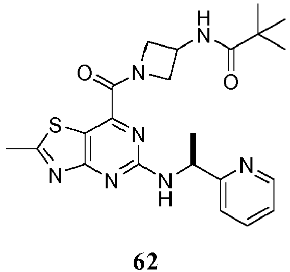

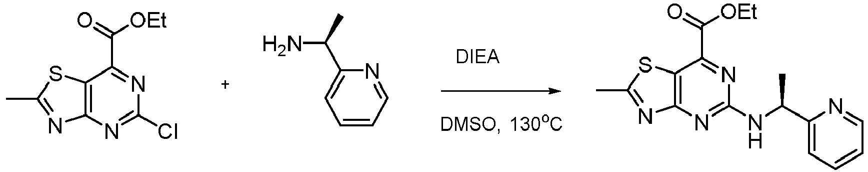

**Synthesis of ethyl 2-methyl-5-[[(*S*)-1-(2-pyridyl)ethyl]amino]thiazolo[4,5-*d*]pyrimidine-7-carboxylate:** To a mixture of ethyl 5-chloro-2-methyl-thiazolo[4,5-d]pyrimidine-7-carboxylate (743 mg, 2.88 mmol) and DIEA (1.51 mL, 8.65 mmol) in DMSO (8 mL) was added (1*S*)-1-(2-pyridyl)ethanamine (commercially obtained from Ark Pharm, Arlington Heights, IL) (704 mg, 5.77 mmol). The reaction mixture was stirred at 130 °C for 6 h, cooled to room temperature, diluted with ethyl acetate and washed with 25% aqueous NaCl solution, then with brine three times, and combined extracts were dried over magnesium sulfate. The solvent was evaporated, and the residue was purified by flash chromatography on silica gel eluting with 2% to 50% solvent A (DCM/MeOH/NH₄OH, 100/10/1) in DCM to provide ethyl2-methyl-5-[[(*S*)-1-(2-pyridyl)ethyl]amino]thiazolo[4,5-*d*]pyrimidine-7-carboxylate (148 mg, 15% yield) as a brown solid. LCMS m/z = 344.1[M+H⁺].

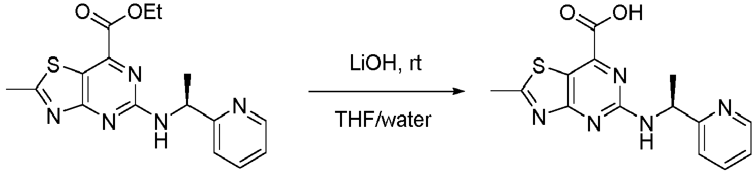

**Synthesis of 2-methyl-5-[[(*S*)-1-(2-pyridyl)ethyl]amino]thiazolo[4,5-*d*]pyrimidine-7-carboxylic acid:** Hydroxylithium hydrate (21.7 mg, 0.43 mmol) was added to a mixture of ethyl 2-methyl-5-[[(*S*)-1-(2-pyridyl)ethyl]amino]thiazolo[4,5-*d*]pyrimidine-7-carboxylate (148 mg, 0.43 mmol) in a THF (1 mL) -Water (0.2 mL) mixture. The resulting mixture was stirred at room temperature for 5 hours, concentrated, dissolved in water (1 mL), and freeze-dried overnight. The dark red powder was dissolved in DCM - MeOH mixture (10:1) and purified by flash chromatography eluting with 2% to 100% solvent A (DCM/MeOH/NH₄OH, 100/10/1) in DCM to provide 2-methyl-5-[[(*S*)-1-(2-pyridyl)ethyl]amino]thiazolo[4,5-*d*]pyrimidine-7-carboxylic acid (117 mg, 86% yield) as white solids. LCMS m/z = 316.1[M+H⁺].

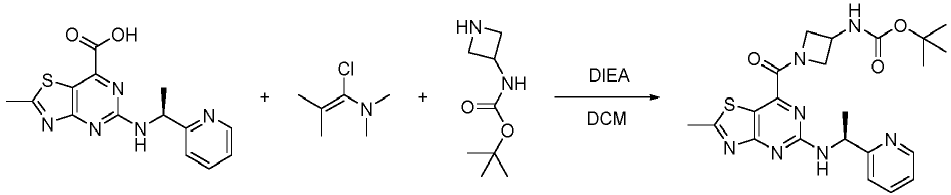

**Synthesis of tert-butyl *N*-[1-[2-methyl-5-[[(*S*)-1-(2-pyridyl)ethyl]amino]thiazolo[4,5-*d*]pyrimidine-7-carbonyl]azetidin-3-yl]carbamate:** To a solution of 2-methyl-5-[[(*S*)-1-(2-pyridyl)ethyl]amino]thiazolo[4,5-*d*]pyrimidine-7-carboxylic acid (42.2 mg, 0.13 mmol) in DCM (4 mL) was added 1-chloro-*N*,*N*-2-trimethylpropenylamine (commercially obtained from Sigma-Aldrich, St. Louis, MO) (0.07 mL, 0.53 mmol), the resulting mixture was stirred at room temperature for 2 hours and concentrated. The residue was dissolved in DCM (4 mL), and a mixture of tert-butyl *N-*(azetidin-3-yl)carbamate (commercially obtained from Ark Pharm, Arlington Heights, IL) (27.6 mg, 0.16 mmol) and DIEA (0.09 mL, 0.53 mmol) in DCM (4 mL) was added at room temperature. The resulting mixture was stirred at room temperature for 4 hours, concentrated, and the residue was purified by flash chromatography eluting with 2% to 50% solvent A (DCM/MeOH/NH₄OH, 100/10/1) in DCM to provide tert-butyl *N*-[1-[2-methyl-5-[[(*S*)-1-(2-pyridyl)ethyl]amino]thiazolo[4,5-*d*]pyrimidine-7-carbonyl]azetidin-3-yl]carbamate (19.4 mg, 31% yield) as yellow solids. LCMS m/z = 470.1[M+H⁺].

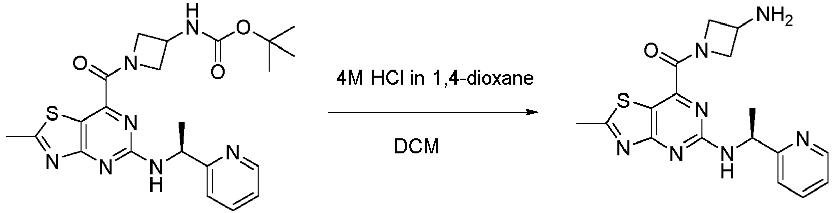

**Synthesis of (3-aminoazetidin-1-yl)-[2-methyl-5-[[(*S*)-1-(2-pyridyl)ethyl]amino]thiazolo[4,5-*d*]pyrimidin-7-yl]methanone trihydrochloride:** A solution of tert-butyl *N*-[1-[2-methyl-5-[[(*S*)-1-(2-pyridyl)ethyl]amino]thiazolo[4,5-*d*]pyrimidine-7-carbonyl]azetidin-3-yl]carbamate (19.4 mg, 0.041 mmol) in DCM (1 mL) was treated with 4M solution of hydrogen chloride in 1,4 -dioxane (0.1 mL), the resulting mixture was kept at room temperature for 1 hour and concentrated to give (3-aminoazetidin-1-yl)-[2-methyl-5-[[(*S*)-1-(2-pyridyl)ethyl]amino]thi-azolo[4,5-*d*]pyrimidin-7-yl]methanone trihydrochloride (19.8 mg, 100%) as a yellow solid. LCMS m/z = 370.3[M+H⁺].

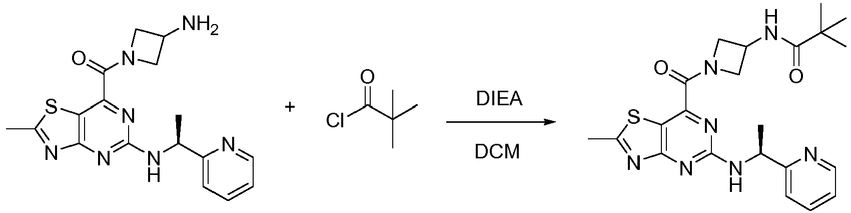

**Synthesis of 2,2-dimethyl-*N*-[1-[2-methyl-5-[[(*S*)-1-(2-pyridyl)ethyl]amino]thiazolo[4,5-*d*]pyrimidine-7-carbonyl]azetidin-3-yl]propanamide (62):** To the mixture of (3-aminoazetidin-1-yl)-[2-methyl-5-[[(*S*)-1-(2-pyridyl)ethyl]amino]thiazolo[4,5-*d*]pyrimidin-7-yl]methanone trihydrochloride (19.8 mg, 0.04 mmol) and DIEA (0.03 mL, 0.19 mmol) in DCM (2 mL) was added pivaloyl chloride (0.006 mL, 0.047 mmol), the resulting mixture was stirred at room temperature for 1 hour, concentrated, and the residue was purified by flash chromatography on silica gel (4 g HP silica, Teledyne Isco) eluting with 5% to 50% solvent A (DCM/MeOH/NH₄OH, 100/10/1) in DCM to provide **62** as a white solid. ¹H NMR (400 MHz, DMSO-d₆ at 80 °C) δ 1.44 (9H, d, *J* = 3.6 Hz), 1.55 (3H, d, *J* = 7.2 Hz), 2.78 (3H, s), 3.96-4.05 (1H, m), 4.27-4.33 (2H, m), 4.51-4.60 (2H, m), 5.19 (1H, quintet, *J* = 7.6 Hz), 7.20 (1H, m), 7.42 (1H, d, *J* = 7.6 Hz), 7.67-7.72 (2H, m), 7.75-7.88 (1H, m), 8.50 (1H, q, *J* = 4.8 Hz) ppm. ESI MS: m/z [M+H⁺] for C₂₂H₂₇N₇O₂S, calcd 454.2, found 454.3.

### Example 63. (S)-1-(tert-Butyl)-3-(1-(2-methyl-5-((1-(pyridin-2-yl)ethyl)amino)thiazolo[4,5-d]pyrimidine-7-carbonyl)azetidin-3-yl)urea

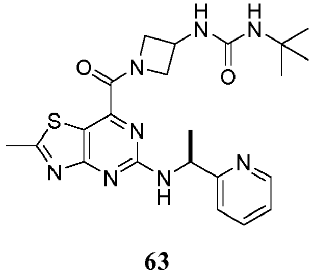

**Synthesis of (*S*)-1-(tert-Butyl)-3-(1-(2-methyl-5-((1-(pyridin-2-yl)ethyl)amino)thiazolo[4,5-*d*]pyrimidine-7-carbonyl)azetidin-3-yl)urea (63):** The title compound **63** was synthesized in similar fashion to example 57. ¹H NMR (400 MHz, DMSO-d₆ at 80 °C) δ 1.26 (9H, s), 1.55 (3H, d, *J* = 6.8 Hz), 2.78 (3H, s), 3.81-3.84 (1H, m), 4.20-4.35 (2H, m), 4.35-4.45 (2H, m), 5.18 (1H, quintet, *J* = 7.2 Hz), 5.59 (1H, s), 6.23 (1H, dd, *J* = 6.8, 28.4 Hz), 7.18-7.22 (1H, m), 7.43 (1H, d, *J* = 8.0 Hz), 7.65-7.74 (2H, m), 8.51 (1H, d, *J* = 4.8 Hz) ppm. ESI MS: m/z [M+H⁺] for C₂₂H₂₈N₈O₂S, calcd 469.2, found 469.3.

### Example 64. (S)-N-(1-(2-Methyl-5-((1-(pyridin-3-yl)ethyl)amino)thiazolo[4,5-d]pyrimidine-7-carbonyl)azetidin-3-yl)pivalamide

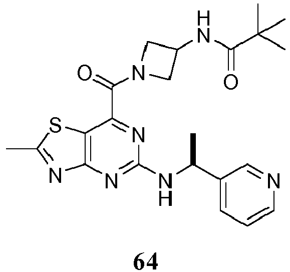

**Synthesis of (*S*)-*N*-(1-(2-methyl-5-((1-(pyridin-3-yl)ethyl)amino)thiazolo[4,5-*d*]pyrimidine-7-carbonyl)azetidin-3-yl)pivalamide (64):** The title compound **64** was synthesized in similar fashion to example 62. ¹H NMR (400 MHz, DMSO-d₆ at 80 °C) δ 1.14 (9H, d, s), 1.55 (3H, dd, *J* = 6.4, 6.8 Hz), 2.78 (3H, d, *J* = 4.0 Hz), 3.96-4.06 (1H, m), 4.30-4.35 (2H, m), 4.56-4.61 (2H, m), 5.20 (1H, quintet, *J* = 7.2 Hz), 7.27-7.32 (1H, m), 7.79-7.88 (3H, m), 8.49 (1H, m), 8.64 (1H, d, *J* = 5.2 Hz) ppm. ESI MS: m/z [M+H⁺] for C₂₂H₂₇N₇O₂S, calcd 454.2, found 454.3.

### Example 65. (S)-1-(1-(5-((1-(5-Fluoropyridin-3-yl)ethyl)amino)-2-methylthiazolo[4,5-d]pyrimidine-7-carbonyl)azetidin-3-yl)-3-phenylurea

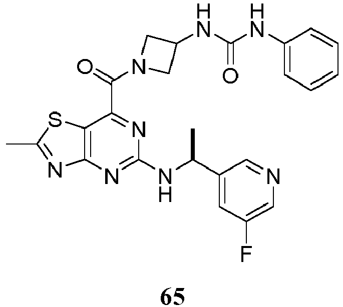

**Synthesis of (*S*)-1-(1-(5-((1-(5-fluoropyridin-3-yl)ethyl)amino)-2-methylthiazolo[4,5-*d*]pyrimidine-7-carbonyl)azetidin-3-yl)-3-phenylurea (65):** The title compound **65** was synthesized in similar fashion to example 57. ¹H NMR (400 MHz, DMSO-d₆ at 80 °C) δ 1.57 (3H, d, *J* = 6.8 Hz), 2.79 (3H, s), 3.98 (1H, dd, *J* = 10.4, 4.8 Hz), 4.39 (1H, m), 4.52 (1H, m), 4.55 (1H, m), 4.97 (1H, m), 5.27 (1H, quintet, *J* = 6.8 Hz), 6.75 (1H, m), 6.93 (1H, tt, *J* = 7.2, 1.2 Hz), 7.21-7.25 (2H, m), 7.39-7.42 (2H, m), 7.72 (1H, dt, *J* = 8.0, 2.0 Hz), 7.84 (1H, d, *J* = 8.0 Hz), 8.36-8.40 (2H, m), 8.54 (1H, m) ppm. ESI MS: m/z [M+H⁺] for C₂₄H₂₃FN₈O₂S, calcd 507.2, found 507.2.

### Example 66. (S)-N-(1-(5-((1-(5-Fluoropyridin-3-yl)ethyl)amino)-2-methylthiazolo[4,5-d]pyrimidine-7-carbonyl)azetidin-3-yl)bicyclo[1.1.1]pentane-1-carboxamide

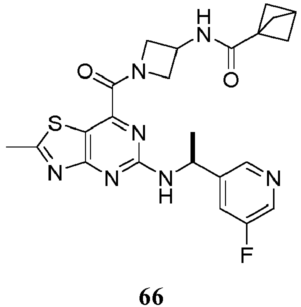

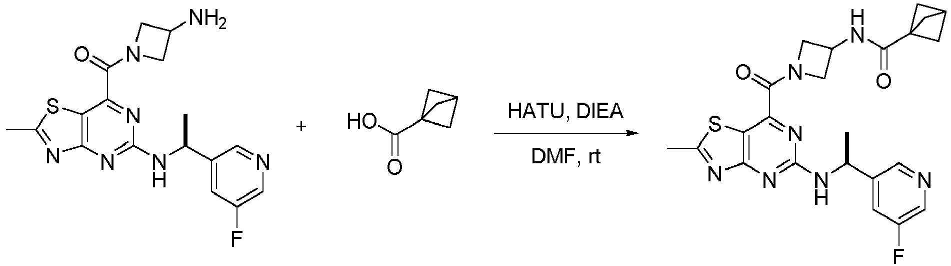

**Synthesis of (*S*)-*N*-(1-(5-((1-(5-fluoropyridin-3-yl)ethyl)amino)-2-methylthiazolo[4,5-*d*]pyrimidine-7-carbonyl)azetidin-3-yl)bicyclo[1.1.1]pentane-1-carboxamide (66):** To a solution of (*S*)-(3-aminoazetidin-1-yl)(5-((1-(5-fluoropyridin-3-yl)ethyl)amino)-2-methylthiazolo[4,5-*d*]pyrimidin-7-yl)methanone (9.7 mg, 0.025 mmol) in DMF (1 mL) were added bicyclo[1.1.1]pentane-3-carboxylic acid (4.2 mg, 0.0375 mmol) (commercially available from PharmaBlock, Nanjing, China), HATU (14 mg, 0.0375 mmol), and DIEA (13 uL, 0.0725 mmol). The mixture thus obtained was stirred at room temperature for 2 h. The reaction mixture was diluted with ethyl acetate and washed with 5% NaHCO₃, water, and brine, then dried over MgSO₄. The solvent was evaporated in vacuo and the residue was purified flash chromatography on silica gel (4 g HP silica, Teledyne Isco) eluting with 10% to 50% solvent A (DCM/MeOH/NH₄OH, 100/10/1) in DCM to provide **66** as a white solid (11 mg, 91% yield). ¹H NMR (400 MHz, DMSO-d₆ at 80 °C) δ 1.57 (3H, d, *J* = 6.8 Hz), 1.99 (6H, d, *J* = 1.6 Hz), 2.43 (1H, br s), 2.78 (3H, s), 4.02 (1H, m), 4.33 (1H, m), 4.47 (1H, m), 4.58 (1H, m), 4.93 (1H, m), 5.25 (1H, quintet, *J* = 6.8 Hz), 7.70 (1H, m), 7.84 (1H, d, *J* = 8.0 Hz), 8.12 (1H, m), 8.39 (1H, d, *J* = 2.8 Hz), 8.52 (1H, d, *J* = 8.0 Hz) ppm. ESI MS: m/z [M+H⁺] for C₂₃H₂₄FN₇O₂S, calcd 482.1, found 482.1.

### Example 67. (S)-N-(1-(5-((1-(5-Fluoropyridin-3-yl)ethyl)amino)-2-methylthiazolo[4,5-d]pyrimidine-7-carbonyl)azetidin-3-yl)bicyclo[2.2.2]octane-1-carboxamide

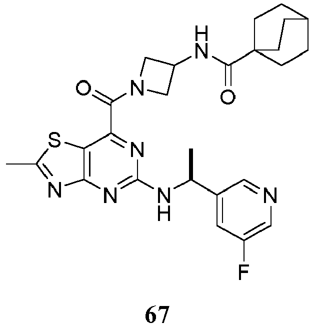

**Synthesis of (*S*)-*N*-(1-(5-((5-(5-fluoropyridin-3-yl)ethyl)amino)-2-methylthiazolo[4,5-*d*]pyrimidine-7-carbonyl)azetidin-3-yl)bicyclo[2.2.2]octane-1-carboxamide (67):** The title compound **67** was synthesized in similar fashion to example 66. ¹H NMR (400 MHz, DMSO-d₆ at 80 °C) δ 1.52-1.61(10H, m), 1.63-1.68 (6H, m), 2.78 (3H, s), 4.00 (1H, m), 4.30 (1H, m), 4.43 (1H, m), 4.53 (1H, m), 4.91 (1H, m), 5.25 (1H, quintet, *J* = 7.2 Hz), 7.66-7.75 (2H, m), 7.20 (1H, m), 7.84 (1H, d, *J* = 8.0 Hz), 8.38 (1H, m), 8.52 (1H, d, *J* = 8.0 Hz) ppm. ESI MS: m/z [M+H⁺] for C₂₆H₃₀FN₇O₂S, calcd 524.2, found 524.2.

### Example 68. (S)-N-(1-(5-((1-(5-Fluoropyridin-3-yl)ethyl)amino)-2-methylthiazolo[4,5-d]pyrimidine-7-carbonyl)azetidin-3-yl)tetrahydro-2H-pyran-4-carboxamide

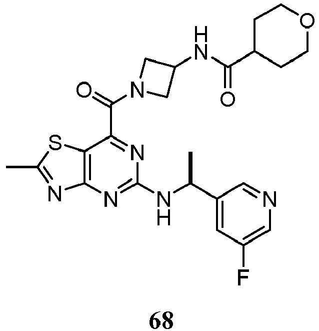

**Synthesis of (*S*)-*N*-(1-(5-((1-(5-Fluoropyridin-3-yl)ethyl)amino)-2-methylthiazolo[4,5-*d*]pyrimidine-7-carbonyl)azetidin-3-yl)tetrahydro-2*H*-pyran-4-carboxamide (68):** The title compound **68** was synthesized in similar fashion to example 66. ¹H NMR (400 MHz, DMSO-d₆ at 80 °C) δ 1.57 (3H, d, *J* = 7.2 Hz), 1.60-1.66 (4H, m), 2.38 (1H, m), 2.76 (3H, s), 3.34 (2H, td, *J* = 11.2, 4.0 Hz), 3.87 (2H, dt, *J* = 11.2, 2.4 Hz), 3.96 (1H, m), 4.35 (1H, m), 4.45 (1H, m), 4.55 (1H, m), 4.94 (1H, m), 5.22 (1H, quintet, *J* = 7.2 Hz), 6.70 (1H, m), 7.84 (1H, d, *J* = 8.0 Hz), 8.23 (1H, m), 8.38 (1H, d, *J* = 2.0 Hz), 8.52 (1H, d, *J* = 6.4 Hz) ppm. ESI MS: m/z [M+H⁺] for C₂₃H₂₆FN₇O₃S, calcd 500.2, found 500.1.

### Example 69. (S)-3,3-Difluoro-N-(1-(5-((1-(5-fluoropyridin-3-yl)ethyl)amino)-2-methylthiazolo[4,5-d]pyrimidine-7-carbonyl)azetidin-3-yl)cyclobutane-1-carboxamide

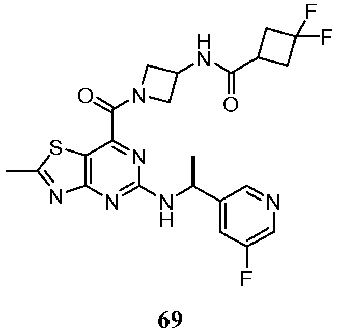

**Synthesis of (*S*)-3,3-difluoro-*N*-(1-(5-((1-(5-fluoropyridin-3-yl)ethyl)amino)-2-methylthiazolo[4,5-*d*]pyrimidine-7-carbonyl)azetidin-3-yl)cyclobutane-1-carboxamide (69):** The title compound **69** was synthesized in similar fashion to example 66. ¹H NMR (400 MHz, DMSO-d₆ at 80 °C) δ 1.57 (3H, d, *J* = 7.2 Hz), 2.67-2.77 (4H, m), 2.78 (3H, s), 2.89 (1H, m), 3.96 (1H, m), 4.36 (1H, m), 4.46 (1H, m), 4.56 (1H, m), 4.95 (1H, m), 5.25 (1H, quintet, *J* = 7.2 Hz), 7.70 (1H, m), 7.84 (1H, d, *J* = 8.0 Hz), 8.38 (1H, d, *J* = 2.0 Hz), 8.47-8.55 (2H, m) ppm. ESI MS: m/z [M+H⁺] for C₂₂H₂₂F₃N₇O₂S, calcd 506.1, found 506.1.

### Example 70. (S)-N-(1-(5-((1-(5-Fluoropyridin-3-yl)ethyl)amino)-2-methylthiazolo[4,5-d]pyrimidine-7-carbonyl)azetidin-3-yl)-1-(trifluoromethyl)cyclopropane-1-carboxamide

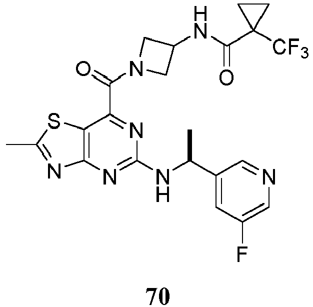

**Synthesis of (*S*)-*N*-(1-(5-((1-(5-fluoropyridin-3-yl)ethyl)amino)-2-methylthiazolo[4,5-*d*]pyrimidine-7-carbonyl)azetidin-3-yl)-1-(trifluoromethyl)cyclopropane-1-carboxamide (70):** The title compound 70 was synthesized in similar fashion to example 66. ¹H NMR (400 MHz, DMSO-d₆ at 80 °C) δ 1.22-1.25 (2H, m), 1.33-1.40 (2H, m), 1.57 (3H, d, *J* = 6.8 Hz), 2.78 (3H, s), 4.05 (1H, m), 4.33 (1H, m), 4.48 (1H, m), 4.61 (1H, m), 4.93 (1H, m), 5.24 (1H, quintet, *J* = 6.8 Hz), 7.70 (1H,m), 7.84 (1H, d, *J* = 8.0 Hz), 8.19 (1H, m), 8.32 (1H, d, *J* = 2.0 Hz), 8.52 (1H, d, *J* = 8.0 Hz) ppm. ESI MS: m/z [M+H⁺] for C₂₂H₂₁F₄N₇O₂S, calcd 524.1, found 524.2.

### Example 71. (S)-N-(1-(5-((1-(5-Fluoropyridin-3-yl)ethyl)amino)-2-methylthiazolo[4,5-d]pyrimidine-7-carbonyl)azetidin-3-yl)-3-methoxybenzamide

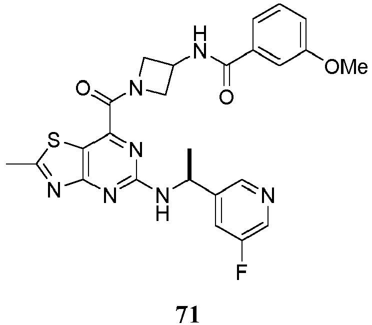

### Synthesis of (S)-N-(1-(5-((1-(5-fluoropyridin-3-yl)ethyl)amino)-2-methylthiazolo[4,5-d]pyrimidine-7-carbonyl)azetidin-3-yl)-3-methoxybenzamide (71):

The title compound **71** was synthesized in similar fashion to example 54. ¹H NMR (400 MHz, DMSO-d₆ at 80 °C) δ 1.57 (3H, d, *J* = 7.2 Hz), 2.79 (3H, s), 3.83 (3H, s), 4.16 (1H, dd, *J* = 10.0, 4.8 Hz), 4.44 (1H, dd, *J* = 10.4, 8.0 Hz), 4.63 (1H, m), 4.80 (1H, m), 5.03 (1H, m), 5.27 (1H, quintet, *J* = 7.2 Hz), 7.11 (1H, ddd, *J* = 8.0, 6.8, 1.2 Hz), 7.39 (1H, t, *J* = 8.0 Hz), 7.44-7.49 (2H, m), 7.71 (1H, m), 7.85 (1H, d, *J* = 8.0 Hz), 8.37 (1H, m), 8.54 (1H, m), 8.85 (1H, m) ppm. ESI MS: m/z [M+H⁺] for C₂₅H₂₄FN₇O₃S, calcd 522.2, found 522.2.

### Example 72. (S)-N-(1-(5-((1-(5-Fluoropyridin-3-yl)ethyl)amino)-2-methylthiazolo[4,5-d]pyrimidine-7-carbonyl)azetidin-3-yl)furan-3-carboxamide

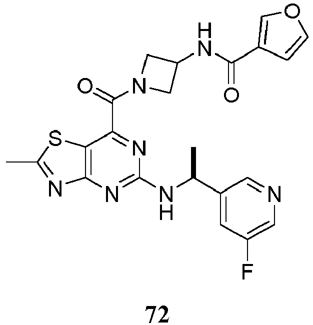

### Synthesis of (S)-N-(1-(5-((1-(5-fluoropyridin-3-yl)ethyl)amino)-2-methylthiazolo[4,5-d]pyrimidine-7-carbonyl)azetidin-3-yl)furan-3-carboxamide (72):

The title compound **72** was synthesized in similar fashion to example 54. ¹H NMR (400 MHz, DMSO-d₆ at 80 °C) δ 1.57 (3H, d, *J* = 6.8 Hz), 2.79 (3H, s), 4.10 (1H, dd, *J* = 10.4, 4.8 Hz), 4.42 (1H, dd, *J* = 10.4, 8.4 Hz), 4.60 (1H, m), 4.76 (1H, m), 5.01 (1H, m), 5.26 (1H, quintet, *J* = 6.8 Hz), 6.85 (1H, m), 7.69 (1H, t, *J* = 2.0 Hz), 7.72 (1H, m), 7.85 (1H, m), 8.17 (1H, d, *J* = 4.8 Hz), 8.38 (1H, dd, *J* = 8.0, 2.4 Hz), 8.53 (1H,m), 8.59 (1H, m) ppm. ESI MS: m/z [M+H⁺] for C₂₂H₂₀FN₇O₃S, calcd 482.1, found 482.1.

### Example 73. N-[1-[2-Methyl-5-[[(S)-1-(3-pyridyl)ethyl]amino]thiazolo[4,5-d]pyrimidine-7-carbonyl]azetidin-3-yl]benzamide

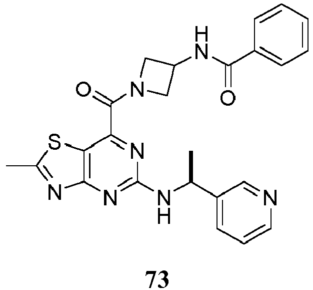

**Synthesis of *N*-[1-[2-methyl-5-[[(*S*)-1-(3-pyridyl)ethyl]amino]thiazolo[4,5-*d*]pyrimidine-7-carbonyl]azetidin-3-yl]benzamide (73):** The title compound 73 was synthesized in similar fashion to example 54. ¹H NMR (400 MHz, DMSO-d₆ at 80 °C) δ 1.55 (3H, d, *J* = 7.2 Hz), 2.78 (3H, s), 3.96-4.06 (1H, m), 4.41-4.60 (2H, m), 4.61-4.85 (2H, m), 5.20 (1H, quintet, *J* = 7.2 Hz), 7.25-7.32 (1H, m), 7.48 (2H, t, *J* = 7.2 Hz), 7.55 (1H, t, *J* = 7.2 Hz), 7.80-7.84 (2H, m), 7.85-7.95 (2H, m), 8.30-8.41 (1H, m), 8.65 (1H, s), 8.81-8.90 (1H, m) ppm. ESI MS: m/z [M+H⁺] for C₂₄H₂₃N₇O₂S, calcd 474.2, found 474.2.

### Example 74. 3-Fluoro-N-[1-[2-methyl-5-[[(S)-1-(3-pyridyl)ethyl]amino]thiazolo[4,5-d]pyrimidine-7-carbonyl]azetidin-3-yl]benzamide

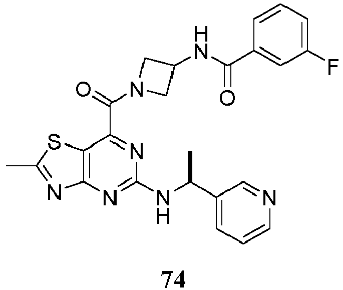

### Synthesis of 3-fluoro-N-[1-[2-methyl-5-[[(S)-1-(3-pyridyl)ethyl]amino]thiazolo[4,5-d]pyrimidine-7-carbonyl]azetidin-3-yl]benzamide (74):

The title compound **74** was synthesized in similar fashion to example 54. ¹H NMR (400 MHz, DMSO-d₆ at 80 °C) δ 1.56 (3H, d, *J* = 7.2 Hz), 2.78 (3H, s), 4.12-4.18 (1H, m), 4.41-4.46 (2H, m), 4.67-4.83 (2H, m), 5.21 (1H, quintet, *J* = 7.2 Hz), 7.23-7.32 (1H, m), 7.37 (1H, dt, *J* = 2.4, 5.6 Hz), 7.54 (1H, dd, *J* = 8.0, 14.9 Hz), 7.65-7.70 (1H, m), 7.72-7.78 (1H, m), 7.80-7.85 (2H, m), 8.35-8.45 (1H, m), 8.65 (1H, m), 8.92-9.00 (1H, m) ppm. ESI MS: m/z [M+H⁺] for C₂₄H₂₂FN₇O₂S, calcd 492.2, found 492.2.

### Example 75. N-[1-[2-Methyl-5-[[(S)-1-(3-pyridyl)ethyl]amino]thiazolo[4,5-d]pyrimidine-7-carbonyl]azetidin-3-yl]cyclobutanecarboxamide

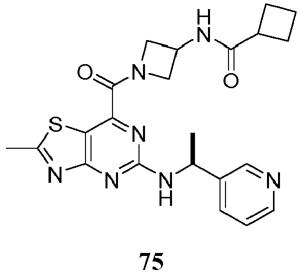

**Synthesis of *N*-[1-[2-methyl-5-[[(*S*)-1-(3-pyridyl)ethyl]amino]thiazolo[4,5-*d*]pyrimidine-7-carbonyl]azetidin-3-yl]cyclobutanecarboxamide (75):** The title compound **75** was synthesized in similar fashion to example 54. ¹H NMR (400 MHz, DMSO-d₆ at 80 °C) δ 1.55 (3H, d, *J* = 7.2 Hz), 1.77-1.84 (1H, m), 1.87-1.97 (1H, m), 2.02-2.22 (4H, m), 2.77 (3H, s), 3.02-3.05 (1H, m), 3.91-3.98 (1H, m), 4.31-4.40 (2H, m), 4.60-4.60 (2H, m), 5.21 (1H, quintet, *J* = 7.2 Hz), 7.27-7.32 (1H, m), 7.80 (2H, d, *J* = 7.6 Hz), 8.05-8.15 (1H, m), 8.41 (1H, d, *J* = 4.8 Hz), 8.64 (1H, m) ppm. ESI MS: m/z [M+H⁺] for C₂₂H₂₅N₇O₂S, calcd 452.2, found 452.1.

### Example 76. 1-Methyl-N-[1-[2-methyl-5-[[(S)-1-(3-pyridyl)ethyl]amino]thiazolo[4,5-d]pyrimidine-7-carbonyl]azetidin-3-yl]cyclopropanecarboxamide

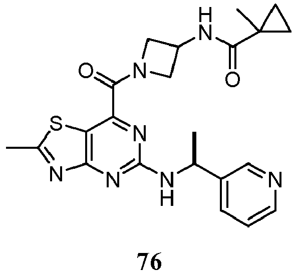

**Synthesis of 1-methyl-*N*-[1-[2-methyl-5-[[(*S*)-1-(3-pyridyl)ethyl]amino]thiazolo[4,5-*d*]pyrimidine-7-carbonyl]azetidin-3-yl]cyclopropanecarboxamide (76):** The title compound **76** was synthesized in similar fashion to example 54. ¹H NMR (400 MHz, DMSO-d₆ at 80 °C) δ 0.54 (2H, d, *J* = 2.8 Hz), 1.01 (2H, d, *J* = 5.6 H), 1.30 (3H, d, *J* = 2.0 Hz), 1.55 (3H, d, *J* = 6.8 Hz), 2.77 (3H, s), 4.04-4.08 (1H, m), 4.31 (2H, t, *J* = 7.6 Hz), 4.55-4.65 (2H, m), 5.21 (1H, quintet, *J* = 6.8 Hz), 7.27-7.32 (1H, m), 7.80 (2H, d, *J* = 7.6 Hz), 7.82-7.90 (1H, m), 8.41 (1H, dd, *J* = 1.6, 4.8 Hz), 8.64 (1H, d, *J* = 4.8 Hz) ppm. ESI MS: m/z [M+H⁺] for C₂₂H₂₅N₇O₂S, calcd 452.2, found 452.1.

### Example 77. N-[1-[2-Methyl-5-[[(S)-1-(3-pyridyl)ethyl]amino]thiazolo[4,5-d]pyrimidine-7-carbonyl]azetidin-3-yl]adamantane-1-carboxamide

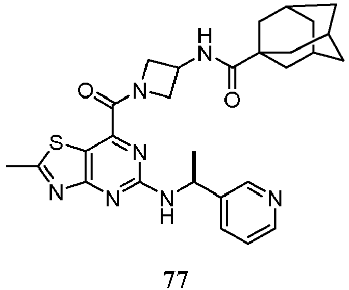

**Synthesis of *N*-[1-[2-Methyl-5-[[(*S*)-1-(3-pyridyl)ethyl]amino]thiazolo[4,5-*d*]pyrimidine-7-carbonyl]azetidin-3-yl]adamantane-1-carboxamide (77):** The title compound 77 was synthesized in similar fashion to example 54. ¹H NMR (400 MHz, DMSO-d₆ at 80 °C) δ 1.56 (3H, dd, *J* = 2.0, 6.8 Hz), 1.65-1.74 (6H, m), 1.82 (6H, s), 1.99 (3H, br s), 2.77 (3H, s), 3.99-4.05 (1H, m), 4.27-4.40 (2H, t, *J* = 7.6 Hz), 4.50-4.62 (2H, m), 5.21 (1H, quintet, *J* = 6.8 Hz), 7.27-7.32 (1H, m), 7.75-7.82 (3H, m), 8.41 (1H, d, *J* = 4.8 Hz), 8.64 (1H, d, *J* = 4.0 Hz) ppm. ESI MS: m/z [M+H⁺] for C₂₈H₃₃N₇O₂S, calcd 532.2, found 532.3.

### Example 78. 1-[1-[2-Methyl-5-[[(S)-1-(2-pyridyl)ethyl]amino]thiazolo[4,5-d]pyrimidine-7-carbonyl]azetidin-3-yl]-3-phenyl-urea

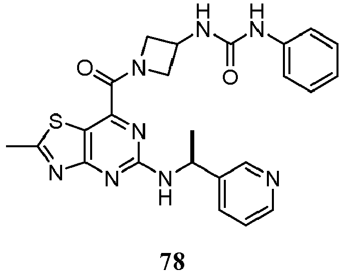

**Synthesis of 1-[1-[2-Methyl-5-[[(*S*)-1-(2-pyridyl)ethyl]amino]thiazolo[4,5-*d*]pyrimidine-7-carbonyl]azetidin-3-yl]-3-phenyl-urea (78):** The title compound **78** was synthesized in similar fashion to example 57. ¹H NMR (400 MHz, DMSO-d₆ at 80 °C) δ 1.56 (3H, d, *J* = 7.2 Hz), 2.78 (3H, s), 3.99-4.05 (1H, m), 4.35-4.46 (2H, t, *J* = 7.6 Hz), 4.50-4.62 (2H, m), 5.21 (1H, quintet, *J* = 7.2 Hz), 6.71-6.77 (1H, m), 6.92 (1H, t, *J* = 7.6 Hz), 7.23 (2H, t, *J* = 8.0 Hz), 7.26-7.32 (1H, m), 7.41 (2H, d, *J* = 8.0 Hz), 7.80 (2H, dt, *J* = 2.4, 8.0 Hz), 8.37-8.42 (2H, m), 8.65 (1H, d, *J* = 1.6 Hz) ppm. ESI MS: m/z [M+H⁺] for C₂₄H₂₄N₈O₂S, calcd 489.2, found 489.2.

### Example 79. 1-Cyclopentyl-3-[1-[2-methyl-5-[[(S)-1-(3-pyridyl)ethyl]amino]thiazolo[4,5-d]pyrimidine-7-carbonyl]azetidin-3-yl]urea

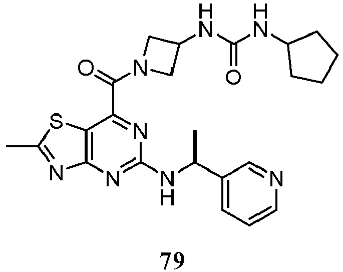

**Synthesis of 1-cyclopentyl-3-[1-[2-methyl-5-[[(*S*)-1-(3-pyridyl)ethyl]amino]thiazolo[4,5-*d*]pyrimidine-7-carbonyl]azetidin-3-yl]urea (79):** The title compound **79** was synthesized in similar fashion to example 57. ¹H NMR (400 MHz, DMSO-d₆ at 80 °C) δ 1.30-1.38 (2H, m), 1.48-1.54 (2H, m), 1.56 (3H, d, *J* = 6.8 Hz), 1.60-1.66 (2H, m), 1.77-1.83 (2H, s), 2.77 (3H, s), 3.86-3.91 (2H, q, *J* = 6.8 Hz), 4.29-4.37 (2H, m), 4.40-4.62 (2H, m), 5.21 (1H, quintet, *J* = 6.8 Hz), 6.78 (1H, d, *J* = 7.2 Hz), 6.29 (1H, q, *J* = 2.8 Hz), 7.30 (1H, m), 7.81 (2H, dt, *J* = 2.0, 7.6 Hz), 8.41 (1H, dd, *J* = 1.2, 4.8 Hz), 8.64 (1H, d, *J* = 1.6 Hz) ppm. ESI MS: m/z [M+H⁺] for C₂₃H₂₈N₈O₂S, calcd 481.2, found 481.3.

### Example 80. N-[1-[2-Methyl-5-[[(S)-1-(3-pyridyl)ethyl]amino]thiazolo[4,5-d]pyrimidine-7-carbonyl]azetidin-3-yl]furan-3-carboxamide

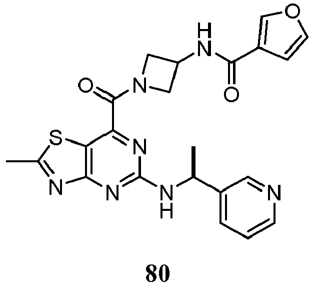

**Synthesis of *N*-[1-[2-methyl-5-[[(*S*)-1-(3-pyridyl)ethyl]amino]thiazolo[4,5-*d*]pyrimidine-7-carbonyl]azetidin-3-yl]furan-3-carboxamide (80):** The title compound **80** was synthesized in similar fashion to example 54. ¹H NMR (400 MHz, DMSO-d₆ at 80 °C) δ 1.55 (3H, d, *J* = 7.2 Hz), 2.77 (3H, s), 4.05-4.12 (1H, m), 4.38-4.49 (2H, t, *J* = 7.6 Hz), 4.62-4.80 (2H, m), 5.20 (1H, quintet, *J* = 6.8 Hz), 6.84 (1H, s), 7.25-7.31 (1H, m), 7.68 (1H, t, *J* = 1.6 Hz), 7.78-7.82 (2H, m), 8.17 (1H, s), 8.37-8.40 (1H, m), 8.57 (1H, t, *J* = 8.0 Hz), 8.64 (1H, s) ppm. ESI MS: m/z [M+H⁺] for C₂₂H₂₁N₇O₃S, calcd 464.1, found 464.1.

### Example 81. N-[1-[2-Methyl-5-[[(S)-1-(3-pyridyl)ethyl]amino]thiazolo[4,5-d]pyrimidine-7-carbonyl]azetidin-3-yl]cyclohexanecarboxamide

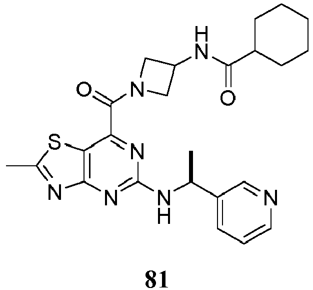

**Synthesis of *N*-[1-[2-methyl-5-[[(*S*)-1-(3-pyridyl)ethyl]amino]thiazolo[4,5-*d*]pyrimidine-7-carbonyl]azetidin-3-yl]cyclohexanecarboxamide (81):** The title compound **81** was synthesized in similar fashion to example 54. ¹H NMR (400 MHz, DMSO-d₆ at 80 °C) δ 1.19-1.29 (3H, m), 1.33-1.42 (2H, m), 1.55 (3H, d, *J* = 6.8 Hz), 1.60-1.64 (1H, m), 1.70-1.75 (4H, m), 2.05-2.17 (1H, m), 2.77 (3H, s), 3.93-3.98 (1H, m), 4.31-4.35 (2H, m), 4.49-4.60 (2H, m), 5.20 (1H, quintet, *J* = 6.8 Hz), 7.27-7.32 (1H, m), 7.80 (2H, t, *J* = 7.6 Hz), 8.11-8.15 (1H, m), 8.41 (1H, d, *J* = 4.4 Hz), 8.64 (1H, d, *J* = 1.2 Hz) ppm. ESI MS: m/z [M+H⁺] for C₂₄H₂₉N₇O₂S, calcd 480.2, found 480.2.

### Example 82. N-[1-[2-Methyl-5-[[(S)-1-(3-pyridyl)ethyl]amino]thiazolo[4,5-d]pyrimidine-7-carbonyl]azetidin-3-yl]tetrahydropyran-4-carboxamide

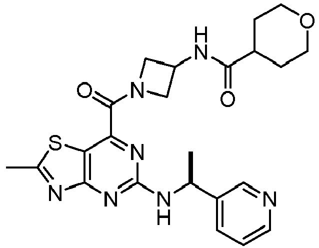

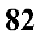

**Synthesis of *N*-[1-[2-methyl-5-[[(*S*)-1-(3-pyridyl)ethyl]amino]thiazolo[4,5-*d*]pyrimidine-7-carbonyl]azetidin-3-yl]tetrahydropyran-4-carboxamide (82):** The title compound **82** was synthesized in similar fashion to example 54. ¹H NMR (400 MHz, DMSO-d₆ at 80 °C) δ 1.55 (3H, d, *J* = 7.2 Hz), 1.60-1.66 (4H, m), 2.38-2.44 (1H, m), 2.77 (3H, s), 3.33 (2H, dt, *J* = 2.8, 11.2 Hz), 3.86 (1H, t, *J* = 3.2 Hz), 3.88 (1H, t, *J* = 3.2 Hz), 3.95-4.00 (1H, m), 4.30-4.38 (2H, m), 4.48-4.60 (2H, m), 5.20 (1H, quintet, *J* = 7.2 Hz), 7.28-7.32 (1H, m), 7.79-7.83 (2H, m), 8.18-8.28 (1H, m), 8.41 (1H, d, *J* = 4.8 Hz), 8.64 (1H, d, *J* = 2.8 Hz) ppm. ESI MS: m/z [M+H⁺] for C₂₃H₂₇N₇O₃S, calcd 482.2, found 482.1.

### Example 83. 3,5-Dimethyl-N-[1-[2-methyl-5-[[(S)-1-(3-pyridyl)ethyl]amino]thiazolo[4,5-d]pyrimidine-7-carbonyl]azetidin-3-yl]benzamide

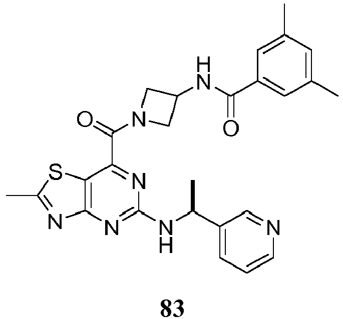

### Synthesis of 3,5-dimethyl-N-[1-[2-methyl-5-[[(S)-1-(3-pyridyl)ethyl]amino]thiazolo[4,5-d]pyrimidine-7-carbonyl]azetidin-3-yl]benzamide (83):

The title compound **83** was synthesized in similar fashion to example 54. ¹H NMR (400 MHz, DMSO-d₆ at 80 °C) δ 1.56 (3H, d, *J* = 7.2 Hz), 2.32 (6H, m), 2.78 (3H, d, *J* = 2.0 Hz), 4.13-4.18 (1H, m), 4.40-4.46 (2H, m), 4.69-4.86 (2H, m), 5.21 (1H, quintet, *J* = 7.2 Hz), 7.17 (1H, m), 7.26-7.32 (1H, m), 7.50 (2H, d, *J* = 4.0 Hz), 7.80-7.85 (2H, m), 8.37-8.42 (1H, m), 8.65 (1H, m), 8.77 (1H, m) ppm. ESI MS: m/z [M+H⁺] for C₂₆H₂₇N₇O₂S, calcd 502.2, found 502.3.

### ASSAY RESULTS OF COMPOUNDS

Compounds (e.g., adenosine receptor modulators) were tested for its biological activity. The assay results in Table 1 indicate (+ means >1 µM; ++ means 100 nM to 1 µM; +++ means < 100 nM).

**TABLE 1. Compounds (e.g., adenosine receptor modulators) and activity assay results. (+ means >1 µM; ++ means 100 nM to 1 µM; +++ means < 100 nM)**

| **Compound** | **A_{2A}R cAMP IC₅₀** | **A_{2B}R cAMP IC₅₀** | **A₁RcAMP IC₅₀** | **A₃R cAMP IC₅₀** |
|---|---|---|---|---|
| 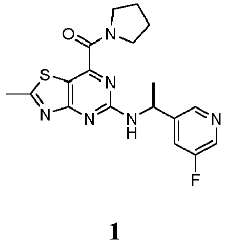 | ++ | +++ | + | ++ |
| 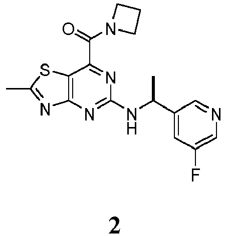 | ++ | +++ | + | ++ |
| 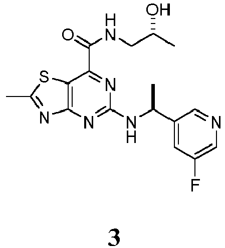 | ++ | +++ | ++ | ++ |
| 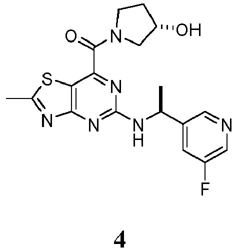 | + | +++ | + | + |
| 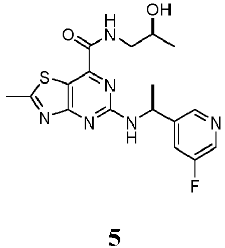 | + | +++ | ++ | ++ |
| 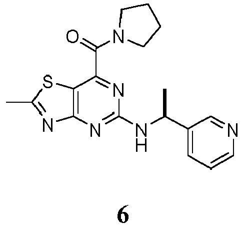 | ++ | +++ | + | ++ |
| 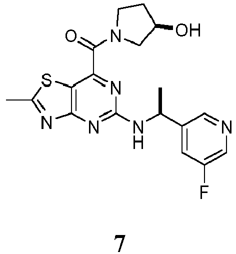 | ++ | +++ | + | + |
| 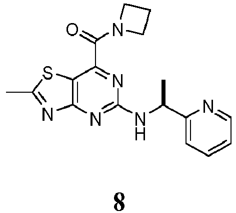 | ++ | +++ | + | ++ |
| 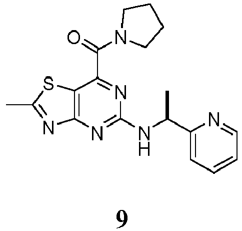 | ++ | +++ | + | ++ |
| 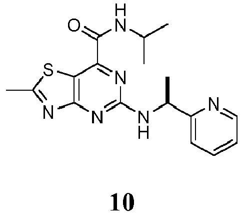 | ++ | +++ | **n/a** | **n/a** |
| 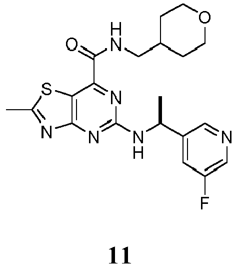 | ++ | +++ | ++ | + |
| 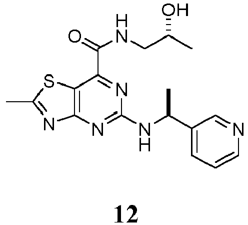 | + | +++ | + | + |
| 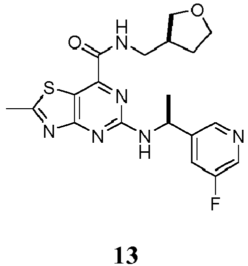 | ++ | +++ | ++ | ++ |
| 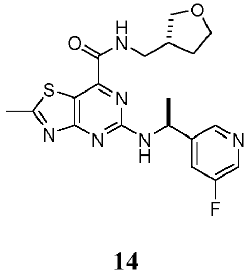 | + | +++ | ++ | + |
| 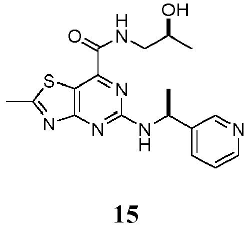 | + | +++ | + | + |
| 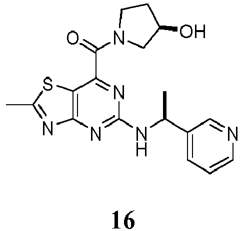 | + | +++ | + | + |
| 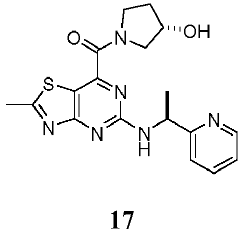 | + | +++ | + | + |
| 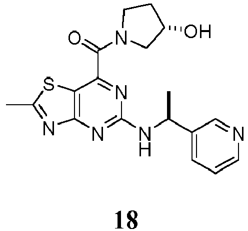 | + | +++ | + | + |
| 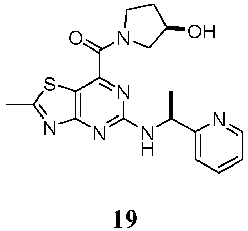 | + | +++ | **n/a** | **n/a** |
| 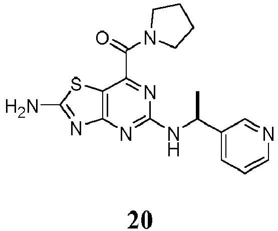 | + | ++ | **n/a** | **n/a** |
| 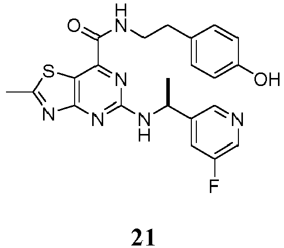 | + | +++ | + | + |
| 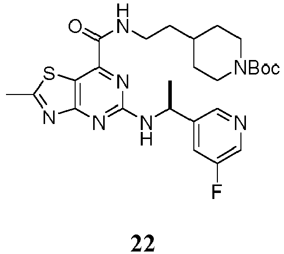 | + | ++ | **n/a** | **n/a** |
| 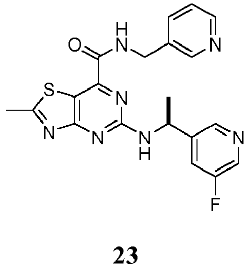 | ++ | +++ | **n/a** | **n/a** |
| 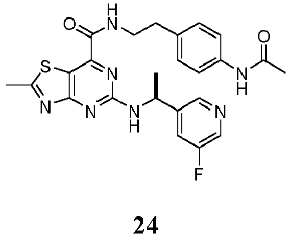 | + | +++ | + | + |
| 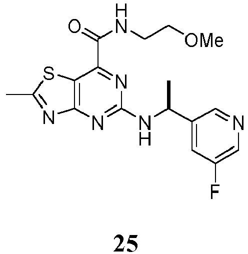 | ++ | +++ | + | + |
| 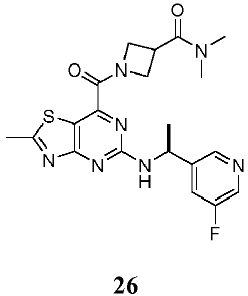 | + | +++ | **n/a** | **n/a** |
| 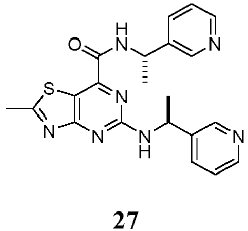 | ++ | ++ | **n/a** | **n/a** |
| 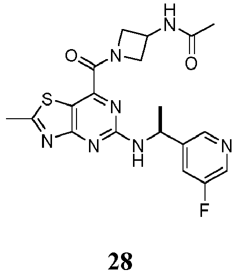 | ++ | +++ | + | + |
| 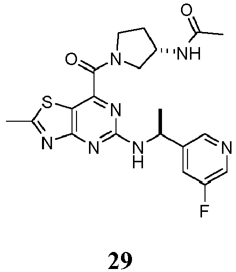 | ++ | +++ | + | ++ |
| 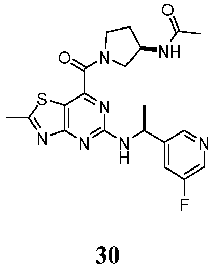 | + | ++ | + | + |
| 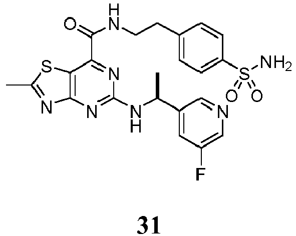 | + | +++ | + | + |
| 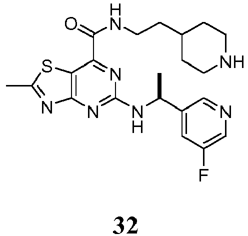 | + | +++ | + | + |
| 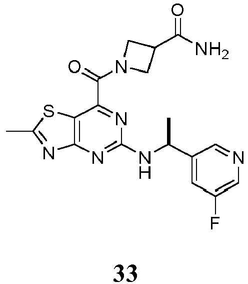 | + | +++ | + | + |
| 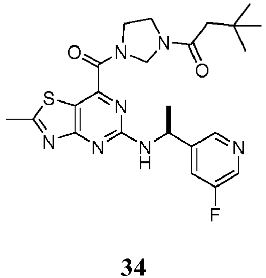 | + | ++ | **n/a** | **n/a** |
| 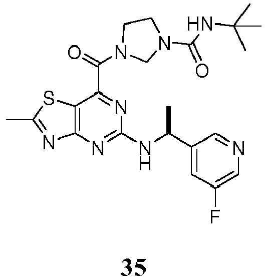 | ++ | ++ | **n/a** | **n/a** |
| 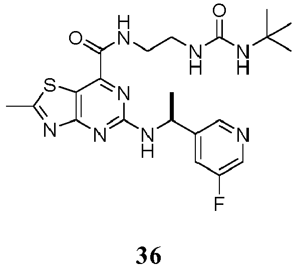 | ++ | + | **n/a** | **n/a** |
| 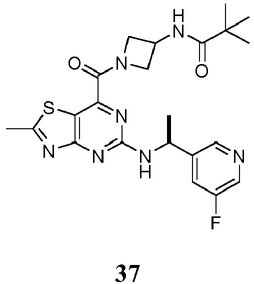 | +++ | +++ | + | +++ |
| 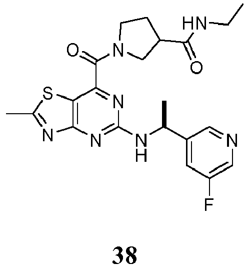 | + | +++ | + | + |
| 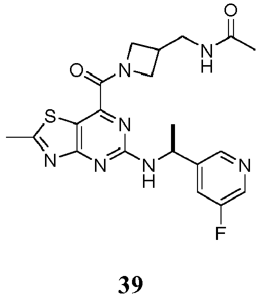 | + | +++ | + | + |
| 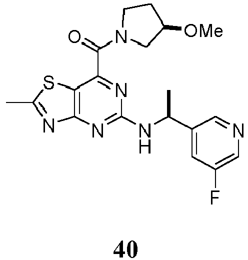 | ++ | +++ | + | + |
| 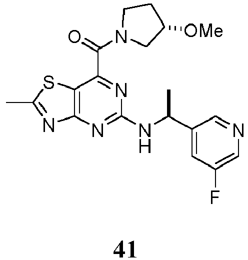 | + | +++ | + | + |
| 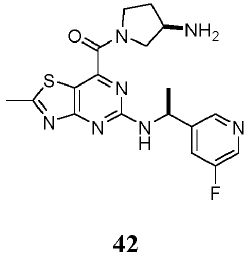 | + | ++ | **n/a** | **n/a** |
| 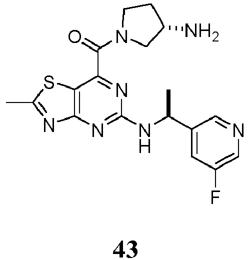 | + | ++ | **n/a** | **n/a** |
| 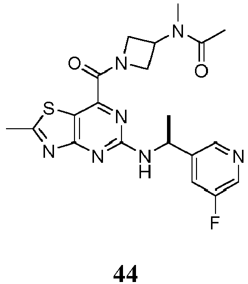 | + | ++ | **n/a** | **n/a** |
| 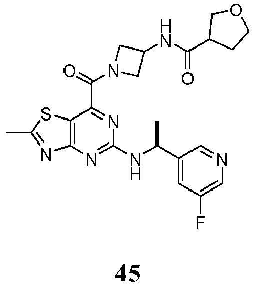 | ++ | ++ | **n/a** | **n/a** |
| 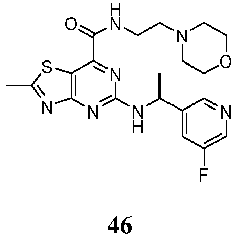 | + | ++ | **n/a** | **n/a** |
| 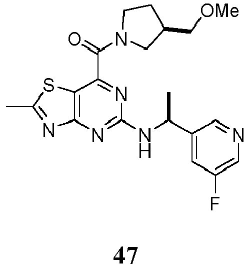 | + | +++ | + | + |
| 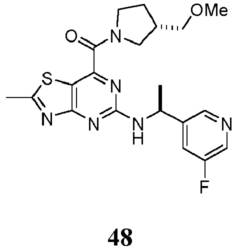 | ++ | +++ | + | + |
| 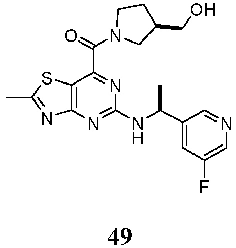 | + | +++ | + | + |
| 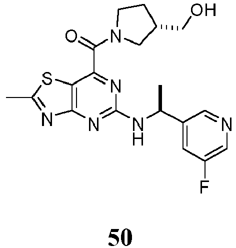 | ++ | +++ | + | + |
| 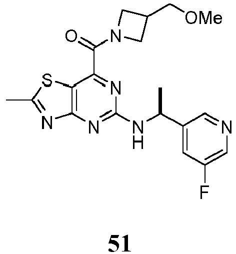 | ++ | +++ | + | + |
| 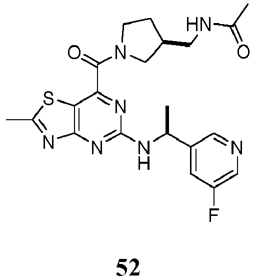 | + | +++ | + | + |
| 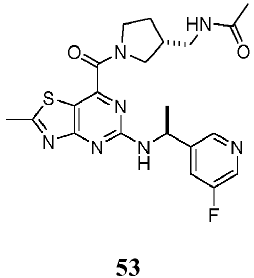 | + | ++ | + | + |
| 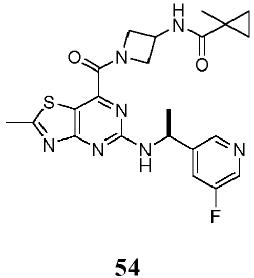 | +++ | +++ | + | +++ |
| 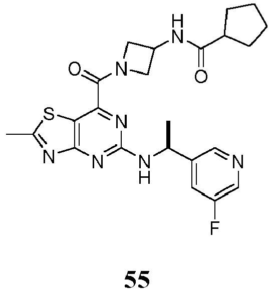 | +++ | +++ | + | +++ |
| 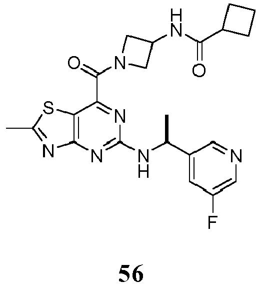 | +++ | +++ | + | +++ |
| 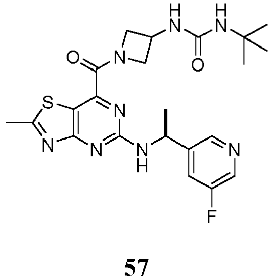 | +++ | +++ | + | ++ |
| 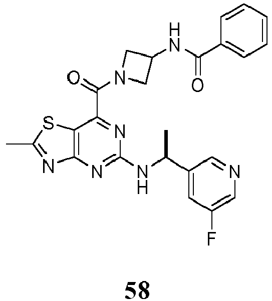 | +++ | +++ | + | +++ |
| 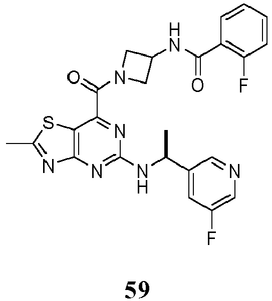 | +++ | +++ | + | ++ |
| 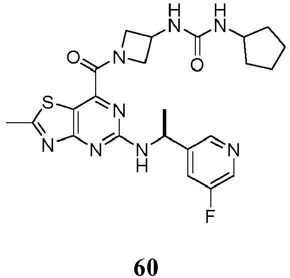 | +++ | +++ | + | ++ |
| 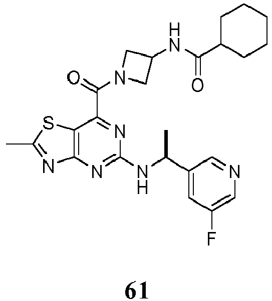 | +++ | +++ | + | +++ |
| 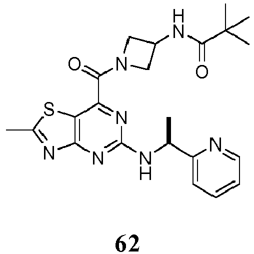 | +++ | +++ | + | ++ |
| 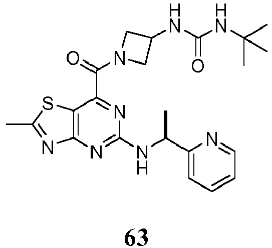 | +++ | ++ | + | ++ |
| 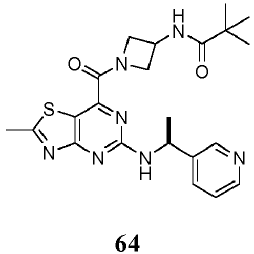 | +++ | ++ | + | ++ |
| 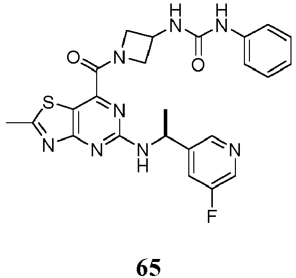 | +++ | ++ | + | ++ |
| 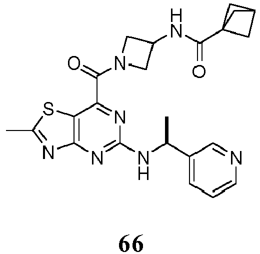 | +++ | +++ | + | ++ |
| 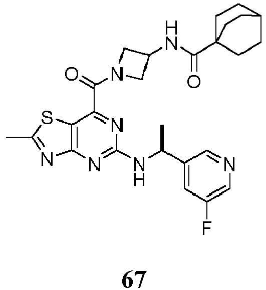 | +++ | +++ | + | +++ |
| 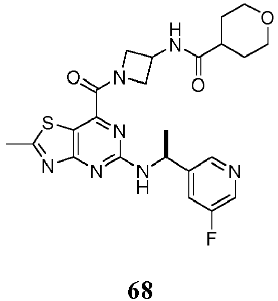 | +++ | ++ | + | ++ |
| 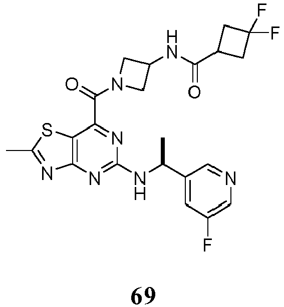 | +++ | ++ | + | ++ |
| 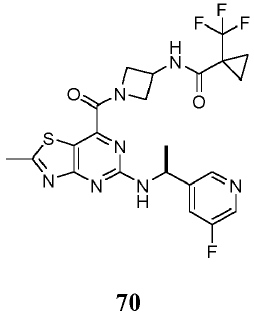 | +++ | +++ | + | ++ |
| 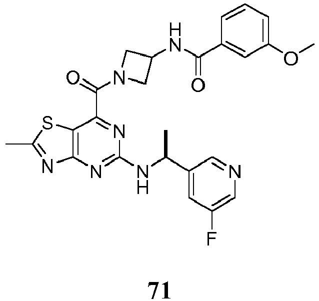 | +++ | +++ | + | ++ |
| 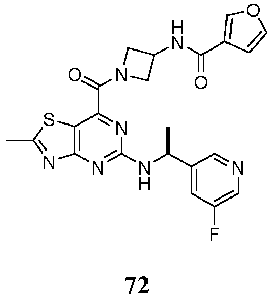 | +++ | ++ | + | ++ |
| 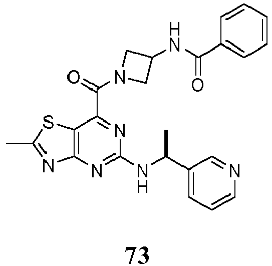 | +++ | ++ | + | ++ |
| 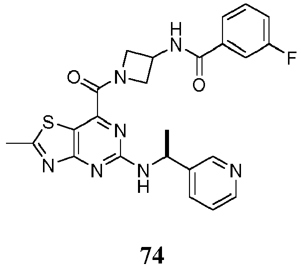 | +++ | ++ | + | + |
| 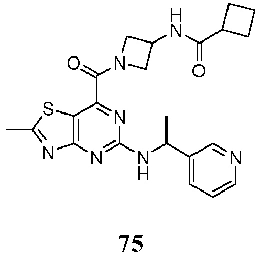 | +++ | ++ | + | + |
| 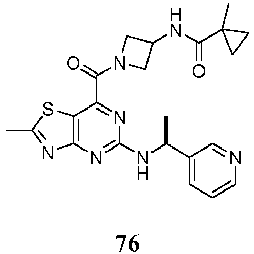 | +++ | ++ | + | ++ |
| 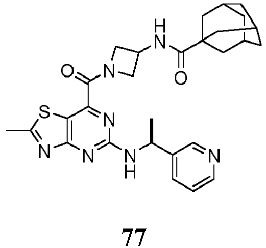 | +++ | +++ | + | ++ |
| 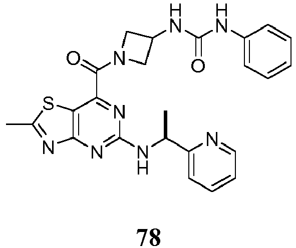 | +++ | ++ | + | + |
| 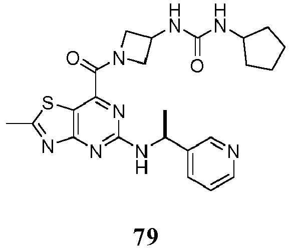 | +++ | +++ | + | ++ |
| 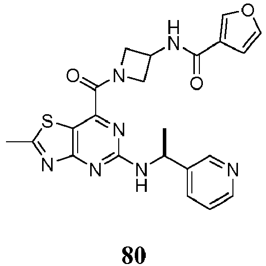 | +++ | ++ | + | + |
| 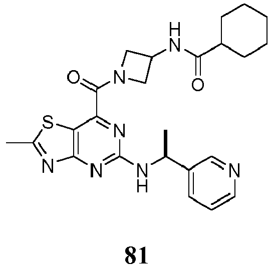 | +++ | +++ | + | ++ |
| 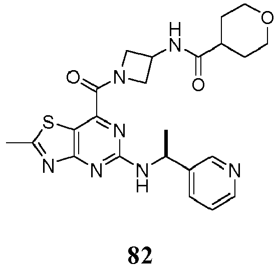 | +++ | ++ | + | + |
| 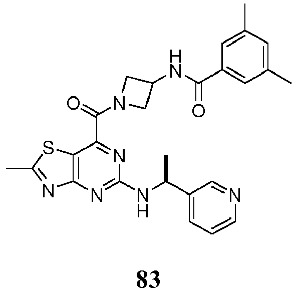 | +++ | +++ | + | + |

## Claims

1. A compound having the formula: 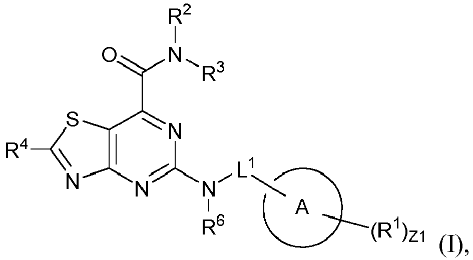 or a pharmaceutically acceptable salt thereof,
wherein
Ring A is a heteroaryl, cycloalkyl, heterocycloalkyl, or aryl;
L¹ is an unsubstituted or substituted alkylene, a bond, -SOₙ₅L^{1A}- -SO_{V5}NR⁵L^{1A}-, -NHC(O)NR⁵L^{1A}-, -NR⁵L^{1A}-, -C(O)L^{1A}-, -C(O)OL^{1A}-, -C(O)NR⁵L^{1A}-, -OL^{1A}-, -NR⁵SO₂L^{1A}-, -NR⁵C(O)L^{1A}-, -NR⁵C(O)OL^{1A}-, -NR⁵OL^{1A}-, -SL^{1A}-, substituted or unsubstituted heteroalkylene, substituted or unsubstituted cycloalkylene, substituted or unsubstituted heterocycloalkylene, substituted or unsubstituted arylene, or substituted or unsubstituted heteroarylene;
R¹ is halogen, -CX¹₃, -CHX¹₂, -CH₂X¹, -OCX¹₃, -OCH₂X¹, -OCHX¹₂, -N₃, -CN, -SOₙ₁R^{1D}, -SOᵥ₁NR^{1A}R^{1B}, -NHC(O)NR^{1A}R^{1B}, -N(O)ₘ₁, -NR^{1A}R^{1B}, -C(O)R^{1C}, -C(O)-OR^{1C}, -C(O)NR^{1A}R^{1B}, -OR^{1D}, -NR^{1A}SO₂R^{1D}, -NR^{1A}C(O)R^{1C}, -NR^{1A}C(O)OR^{1C}, -NR^{1A}OR^{1C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
R² is hydrogen, -CX²₃, -CHX²₂, -CH₂X², -OCX²₃, -OCH₂X², -OCHX²₂, -C(O)R^{2C}, -C(O)OR^{2C}, -C(O)NR^{2A}R^{2B}, -OR^{2D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
R³ is a substituted or unsubstituted alkyl, hydrogen, -CX³₃, -CHX³₂, -CH₂X³, -OCX³₃, -OCH₂X³, -OCHX³₂, -C(O)R^{3C}, -C(O)OR^{3C}, -C(O)NR^{3A}R^{3B}, -OR^{3D}, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
R² and R³ together with the nitrogen attached thereto may be optionally joined to form a substituted or unsubstituted heterocycloalkyl, or substituted or unsubstituted heteroaryl;
R⁴ is an unsubstituted or substituted alkyl, hydrogen, halogen, -CX⁴₃, -CHX⁴₂, -CH₂X⁴, -OCX⁴₃, -OCH₂X⁴, -OCHX⁴₂, -N₃, -CN, -SOₙ₄R^{4D}, -SOᵥ₄NR^{4A}R^{4B}, -NHC(O)NR^{4A}R^{4B}, -N(O)ₘ₄, -NR^{4A}R^{4B}, -C(O)R^{4C}, -C(O)-OR^{4C}, -C(O)NR^{4A}R^{4B}, -OR^{4D}, -NR^{4A}SO₂R^{4D}, -NR^{4A}C(O)R^{4C}, -NR^{4A}C(O)OR^{4C}, -NR^{4A}OR^{4C}, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
R⁵ is hydrogen, -CX⁵₃, -CHX⁵₂, -CH₂X⁵, -OCX⁵₃, -OCH₂X⁵, -OCHX⁵₂, -C(O)R^{5C}, -C(O)OR^{5C}, -C(O)NR^{5A}R^{5B}, -OR^{5D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
R⁶ is hydrogen, -CX⁶₃, -CHX⁶₂, -CH₂X⁶, -OCX⁶₃, -OCH₂X⁶, -OCHX⁶₂, -C(O)R^{6C}, -C(O)OR^{6C}, -C(O)NR^{6A}R^{6B}, -OR^{6D}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
wherein R^{1A}, R^{1B}, R^{1C} , R^{1D}, R^{2A}, R^{2B}, R^{2C}, R^{2D}, R^{3A}, R^{3B}, R^{3C}, R^{3D}, R^{4A}, R^{4B}, R^{4C}, R^{4D} , R^{5A}, R^{5B}, R^{5C}, R^{5D}, R^{6A}, R^{6B}, R^{6C}, and R^{6D} are independently hydrogen, -CX₃, -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
L^{1A} is a bond, substituted or unsubstituted alkylene, substituted or unsubstituted heteroalkylene, substituted or unsubstituted cycloalkylene, substituted or unsubstituted heterocycloalkylene, substituted or unsubstituted arylene, or substituted or unsubstituted heteroarylene;
z1 is an integer from 0 to 12;
n1 and n4 are independently an integer from 0 to 4;
m1, m4, v1, and v4 are independently an integer from 1 to 2; and
X, X¹, X², X³, X⁴, X⁵ and X⁶ are independently -F, -Cl, -Br, or -I.

2. The compound of claim 1, wherein the Ring A is 5 to 10 membered heteroaryl or C₆-C₁₀ aryl;
optionally wherein Ring A is pyridyl,
for example wherein Ring A is

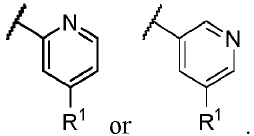

3. The compound of claim 1, wherein the compound has the formula: 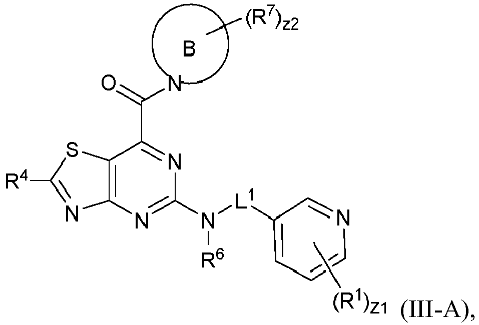 or 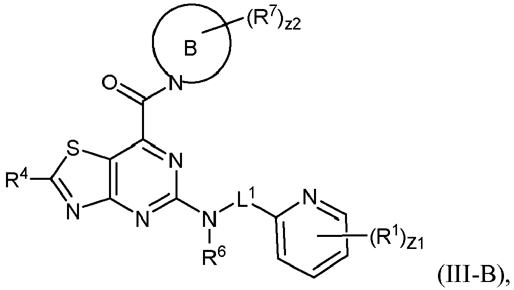 or a pharmaceutically acceptable salt thereof;
wherein
Ring B is a 4 to 7 membered heterocycloalkyl;
R⁷ is independently halogen, -CX⁷₃, -CHX⁷₂, -CH₂X⁷, -OCX⁷₃, -OCH₂X⁷, -OCHX⁷₂, -N₃, -CN, -SOₙ₇R^{7D}, -SOᵥ₇NR^{7A}R^{7B}, -NR^{7A}C(O)NR^{7A}R^{7B}, -NR^{7A}S(O)NR^{7A}R^{7B}, -N(O)ₘ₇, -NR^{7A}R^{7B}, -C(O)R^{7C}, -C(O)-OR^{7C}, -C(O)NR^{7A}R^{7B}, -C(O)NR^{7A}OR^{7B}, -OR^{7D}, -NR^{7A}SO₂R^{7D}, -NR^{7A}C(O)R^{7C}, -NR^{7A}C(O)OR^{7C}, -NR^{7A}OR^{7C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
R^{7A}, R^{7B}, R^{7C}, and R^{7D} are independently hydrogen, -CN, -CX₃, -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
z1 is an integer from 0 to 4;
z2 is an integer from 0 to 12;
n7 is independently an integer from 0 to 4;
m7 and v7 are independently an integer from 1 to 2; and
X⁷ is independently -F, -Cl, -Br, or -I;
optionally wherein
**Option A:** the compound has a formula 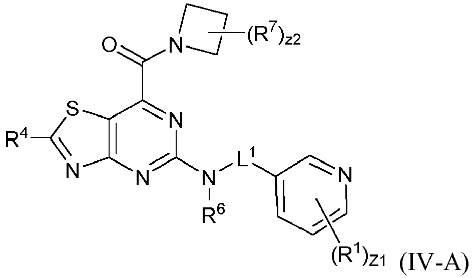 or 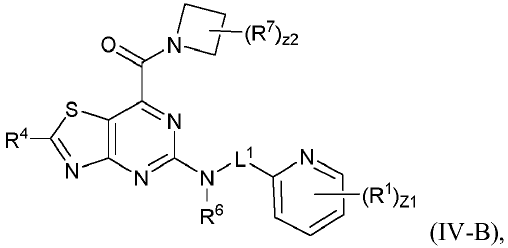 or a pharmaceutically acceptable salt thereof, wherein z2 is an integer from 0 to 6; or
**Option B:** the compound has a formula: 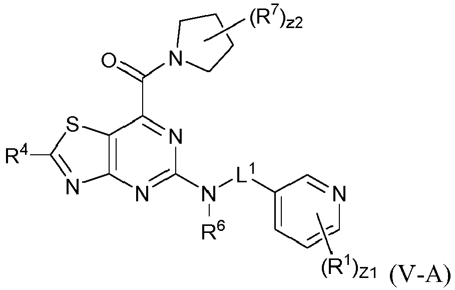 or 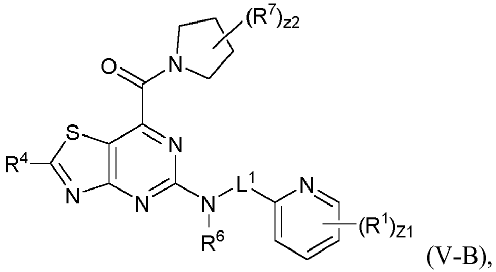 or a pharmaceutically acceptable salt thereof, wherein z2 is an integer 0 to 8; or
Option C: the compound has the formula: 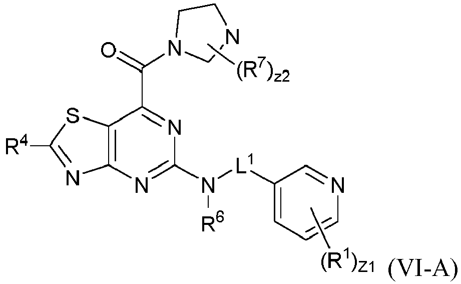 or 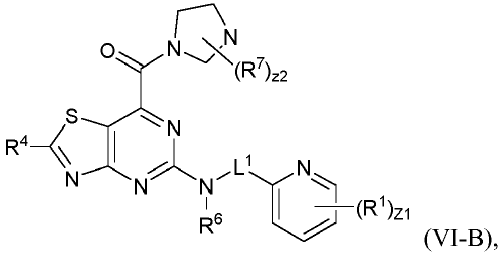 or a pharmaceutically acceptable salt thereof, wherein z2 is an integer from 0 to 7.

4. The compound of claim 3, wherein:
**A:** z2 is 0 or 1;
R⁷ is independently -OR^{7D}, -NR^{7A}R^{7B}, -C(O)NR^{7A}R^{7B}, -C(O)NR^{7A}OR^{7B}, -NR^{7A}C(O)R^{7C}, -NR^{7A}C(O)NR^{7A}R^{7B}, -C(O)R^{7C}, or substituted or unsubstituted 2 to 6 membered heteroalkyl;
R^{7A} and R^{7B} are independently hydrogen, or unsubstituted C₁-C₄ alkyl;
R^{7C} is independently substituted or unsubstituted C₁-C₁₂ alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
R^{7D} is independently hydrogen, or unsubstituted C₁-C₄ alkyl;
for example wherein R⁷ is independently -NR^{7A}C(O)R^{7C}, or -C(O)NR^{7A}R^{7B}; or
**B:** z2 is 0 or 1;
R⁷ is independently -NR^{7A}R^{7B}, -C(O)NR^{7A}R^{7B}, -C(O)NR^{7A}OR^{7B}, -NR^{7A}C(O)R^{7C}, -OR^{7D}, -C(O)R^{7C}, or substituted or unsubstituted 2 to 6 membered heteroalkyl;
R^{7A} and R^{7B} are independently hydrogen, or unsubstituted C₁-C₄ alkyl;
R^{7C} is independently unsubstituted C₁-C₆ alkyl; and
R^{7D} is independently hydrogen, or unsubstituted C₁-C₄ alkyl; or
**C:** z2 is 0 or 1;
R⁷ is independently -C(O)R^{7C}, or -C(O)NR^{7A}R^{7B};
R^{7A} and R^{7B} are independently hydrogen, or unsubstituted C₁-C₄ alkyl; and
R^{7C} is independently unsubstituted C₁-C₆ alkyl.

5. The compound of claim 3, wherein R^{7A}, R^{7B}, and R^{7C} are independently hydrogen, substituted or unsubstituted C₁-C₄ alkyl, substituted or unsubstituted 3 to 8-membered cycloalkyl, substituted or unsubstituted 5 to 6 membered heterocycloalkyl, substituted or unsubstituted phenyl, or substituted or unsubstituted 5 to 6 membered heteroaryl.

6. The compound of claim 3 wherein:
**(i)** R⁷ is independently: 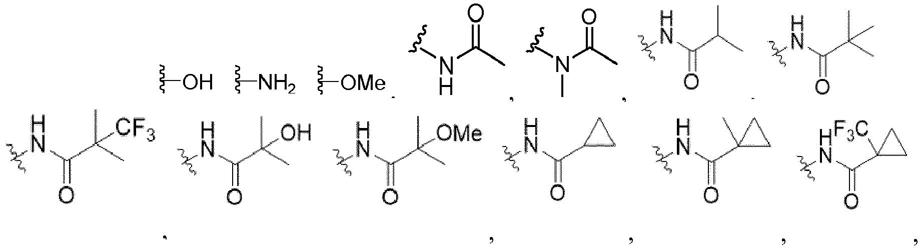 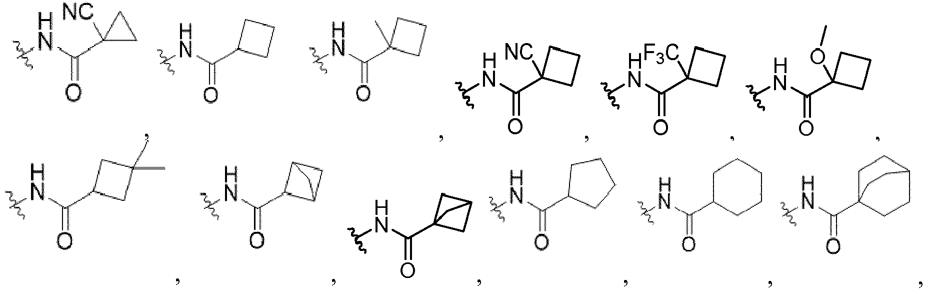 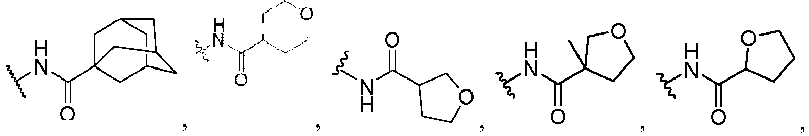 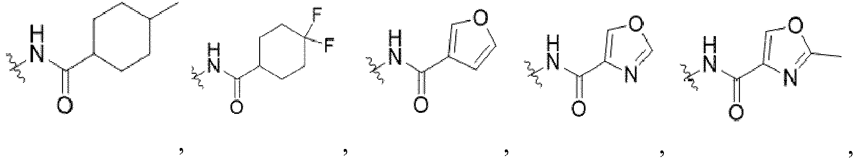 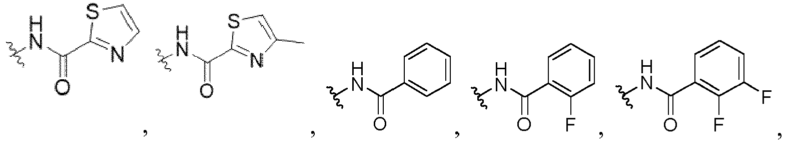 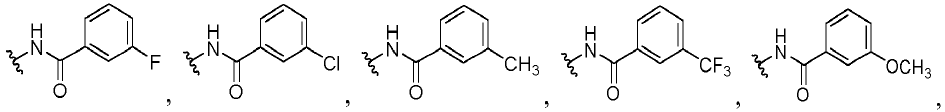 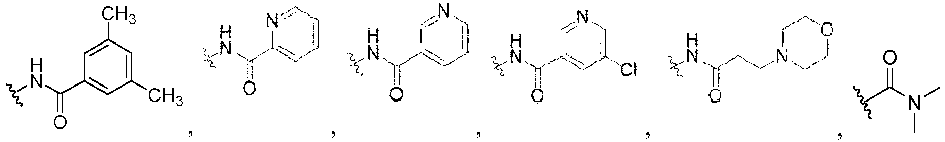 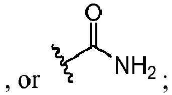 or
**(ii)** R⁷ is independently -OH; or
**(iii)** R⁷ is independently -NHC(O)NHR^{7B}; optionally wherein R^{7B} is independently substituted or unsubstituted C₁-C₄ alkyl, substituted or unsubstituted 3 to 8-membered cycloalkyl, substituted or unsubstituted 5 to 6 membered heterocycloalkyl, substituted or unsubstituted phenyl, or substituted or unsubstituted 5 to 6 membered heteroaryl;
for example wherein R⁷ is: 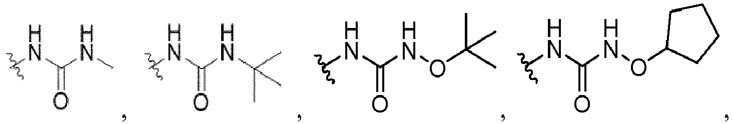 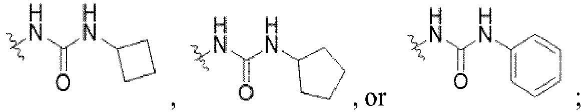 or
**(iv)** R⁷ is independently substituted or unsubstituted C₁-C₄ alkyl or substituted or unsubstituted 2 to 6 membered heteroalkyl;
optionally wherein R⁷ is independently 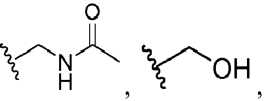 or 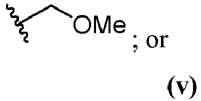 z2 is 1 and R⁷ is independently -OH.

7. The compound of any of claims 3 to 6 wherein z2 is 0 or 1.

8. The compound of claim 1, wherein:
**A:** the compound has the formula: 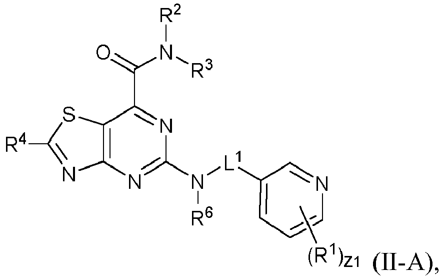 wherein z1 is an integer from 0 to 4,
for example wherein the compound has the formula: 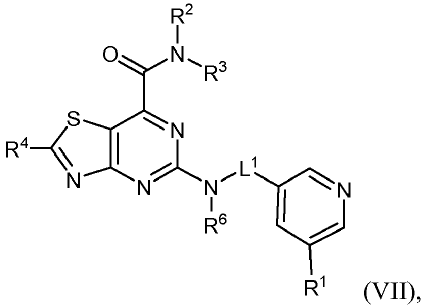 or a pharmaceutically acceptable salt thereof; or
**B:** the compound has the formula: 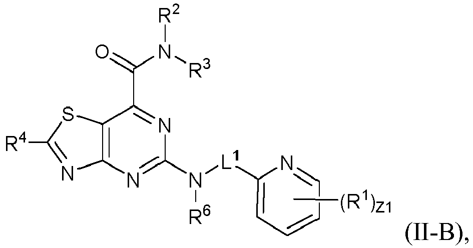 or a pharmaceutically acceptable salt thereof,
wherein z1 is an integer from 0 to 4;
for example wherein the compound has a formula: 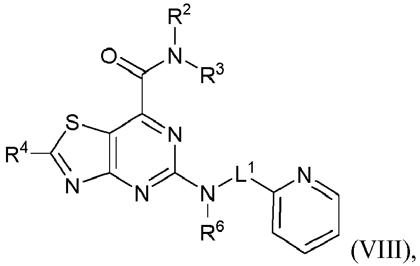 or a pharmaceutically acceptable salt thereof.

9. The compound of claim 8, wherein R² and R³ together with the nitrogen attached thereto are joined to form 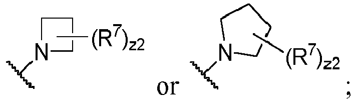 wherein
R⁷ is independently halogen, -CX⁷₃, -CHX⁷₂, -CH₂X⁷, -OCX⁷₃, -OCH₂X⁷, -OCHX⁷₂, -N₃, -CN, -SOₙ₇R^{7D}, -SOᵥ₇NR^{7A}R^{7B}, -NR^{7A}C(O)NR^{7A}R^{7B}, -NR^{7A}S(O)NR^{7A}R^{7B}, -N(O)ₘ₇, -NR^{7A}R^{7B}, -C(O)R^{7C}, -C(O)-OR^{7C}, -C(O)NR^{7A}R^{7B}, -C(O)NR^{7A}OR^{7B}, -OR^{7D}, -NR^{7A}SO₂R^{7D}, -NR^{7A}C(O)R^{7C}, -NR^{7A}C(O)OR^{7C}, -NR^{7A}OR^{7C}, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
R^{7A}, R^{7B}, R^{7C}, and R^{7D} are independently hydrogen, -CN, -CX₃, -CHX₂, -CH₂X, -COOH, -CONH₂, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl;
z2 is an integer from 0 to 1;
n7 is independently an integer from 0 to 4;
m7 and v7 are independently an integer from 1 to 2; and
X⁷ is independently -F, -Cl, -Br, or -I;
optionally wherein:
**(i)** z2 is 0; or
**(ii)** z2 is 1; or
**(iii)** z2 is 1 and R⁷ is

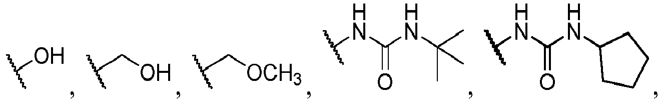

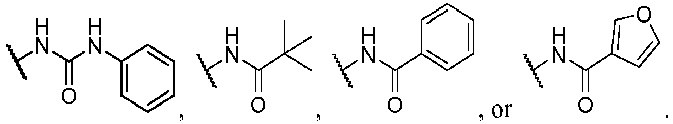

10. The compound of any of claims 1 to 9 wherein:
**A:** R¹ is halogen, -CX¹₃,
-CHX¹₂, -CH₂X¹, -OCX¹₃, -OCH₂X¹, -OCHX¹₂, -CN, -SR^{1D}, -NHC(O)NR^{1A}R^{1B}, -NR^{1A}R^{1B}, -C(O)R^{1C}, -C(O)-OR^{1C}, -C(O)NR^{1A}R^{1B}, -OR^{1D}, -NR^{1A}C(O)R^{1C}, -NR^{1A}C(O)OR^{1C}, substituted or unsubstituted alkyl, or substituted or unsubstituted heteroalkyl; or
**B:** R¹ is independently a halogen, for example, wherein z1 is 0 or 1, and R¹ is independently a halogen.

11. The compound of any one of claims 1 to 10, wherein L¹ is an unsubstituted or substituted alkylene, a bond, or substituted or unsubstituted heteroalkylene;
optionally wherein:
**(i)** L¹ is substituted or unsubstituted C₁-C₄ alkylene; or
**(ii)** L¹ is

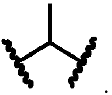

12. The compound of any one of claims 1 to 11, wherein:
**A:** R⁴ is unsubstituted or substituted alkyl, hydrogen, halogen, -CX⁴₃, -CHX⁴₂, -CH₂X⁴, -OCX⁴₃, -OCH₂X⁴, -OCHX⁴₂, -CN, -SR^{4D}, -NHC(O)NR^{4A}R^{4B}, -NR^{4A}R^{4B}, -C(O)R^{4C}, -C(O)-OR^{4C}, -C(O)NR^{4A}R^{4B}, -OR^{4D}, -NR^{4A}C(O)R^{4C}, -NR^{4A}C(O)OR^{4C}, -NR^{4A}OR^{4 C}, substituted or unsubstituted heteroalkyl, or substituted or unsubstituted cycloalkyl; or
**B:** R⁴ is hydrogen, substituted or unsubstituted C₁-C₈ alkyl, or substituted or unsubstituted 2 to 6 membered heteroalkyl; or
**C:** R⁴ is substituted or unsubstituted C₁-C₄ alkyl, or substituted or unsubstituted 2 to 4 membered heteroalkyl; or
**D:** R⁴ is unsubstituted C₁-C₄ alkyl; or
**E:** R⁴ is unsubstituted methyl; or
**F:** R⁴ is -NR^{4A}R^{4B}, and each R^{4A} and R^{4B} is independently hydrogen, or substituted or unsubstituted C₁-C₄ alkyl; or
**G:** R⁴ is -NH₂.

13. The compound of any one of claims 1 to 12, wherein:
**A:** R⁶ is hydrogen, -CX⁶₃, -CHX⁶₆, -CH₆X⁶, -OCX⁶₃,-OCH₆X⁶, -OCHX⁶₆, -C(O)R^{6C}, -C(O)OR^{6C}, -C(O)NR^{6A}R^{6B}, -OR^{6D}, or substituted or unsubstituted alkyl; or
**B:** R⁶ is hydrogen.

14. The compound of claim 1, wherein the compound is: 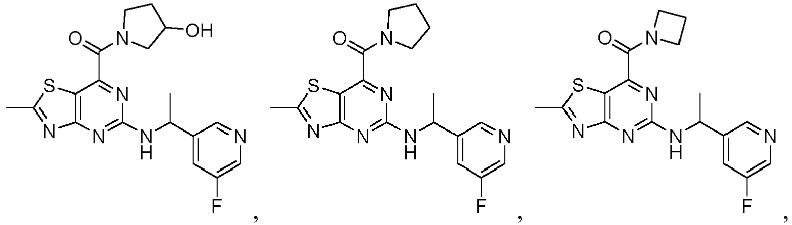 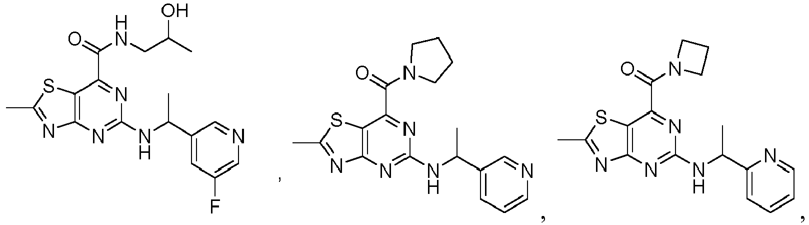 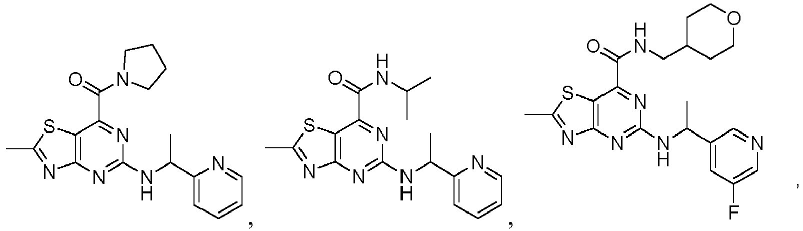 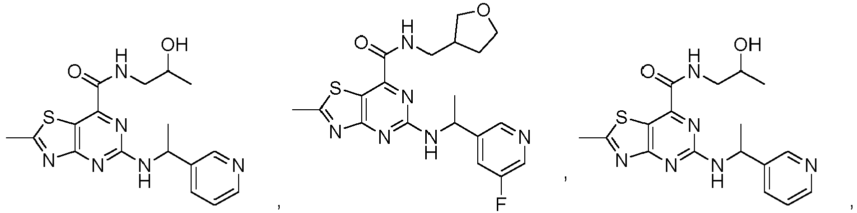 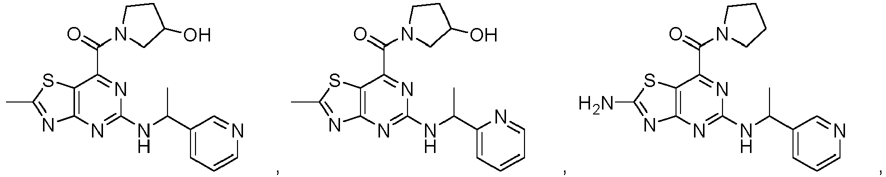 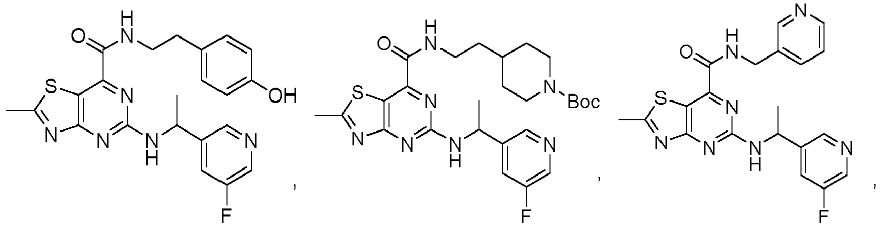 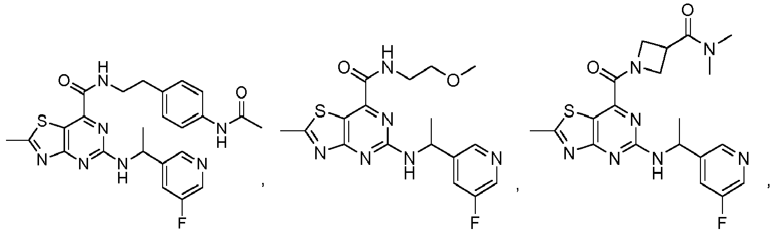 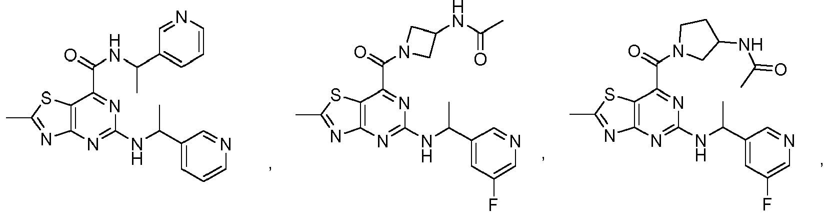 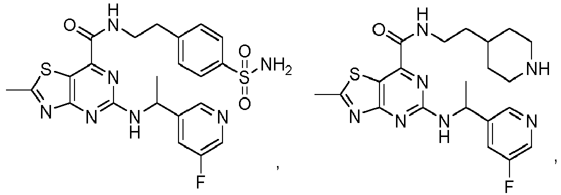 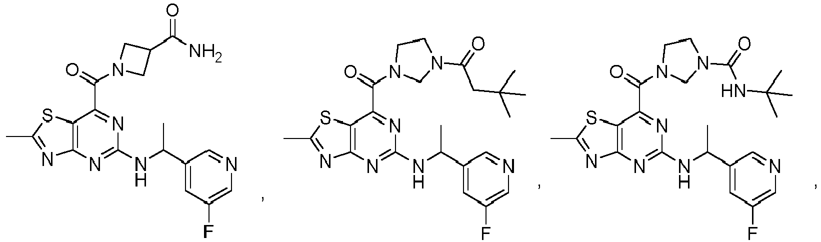 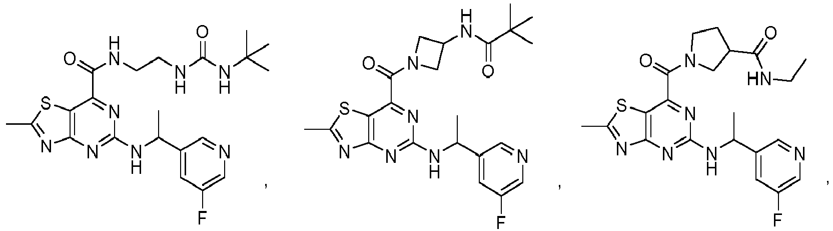 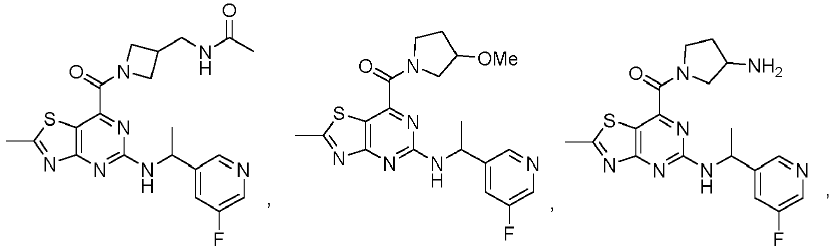 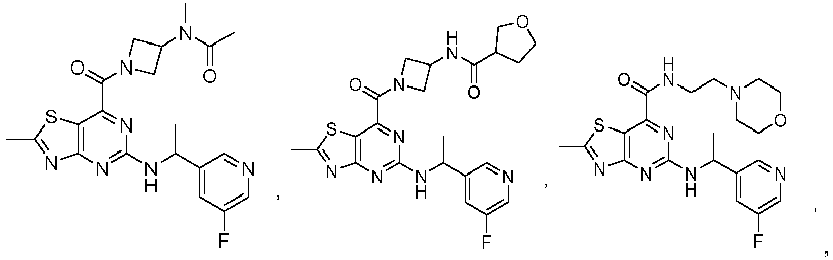 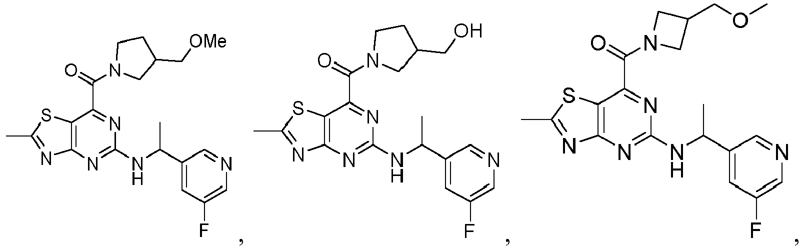 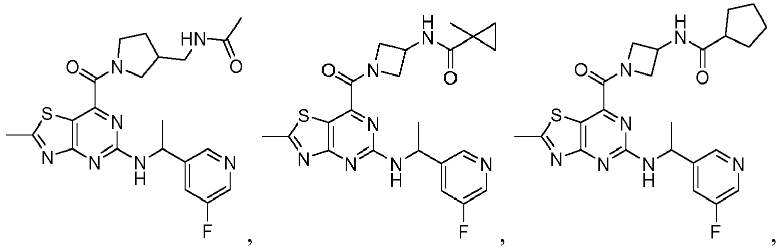 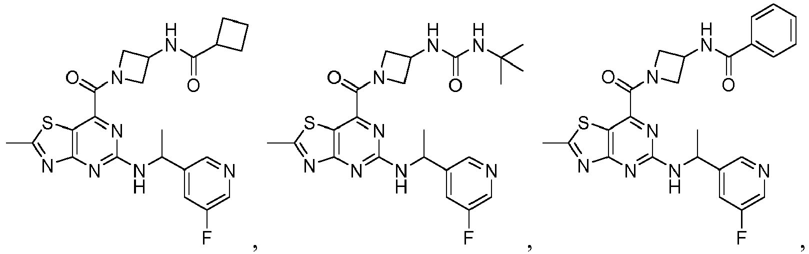 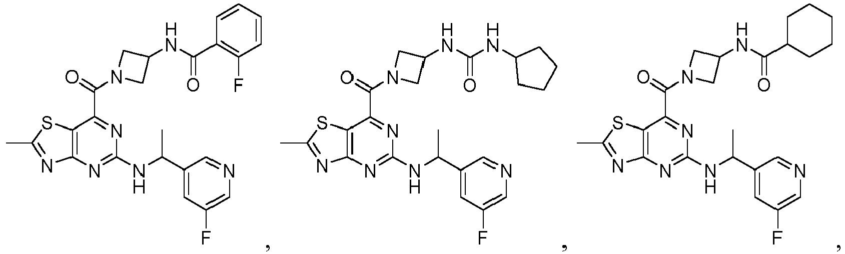 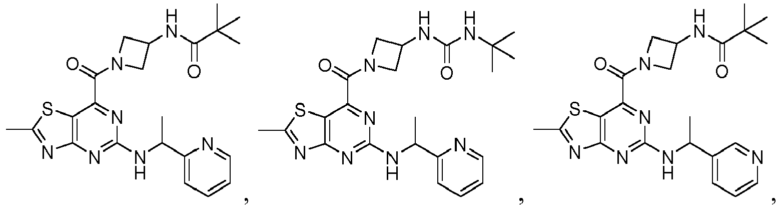 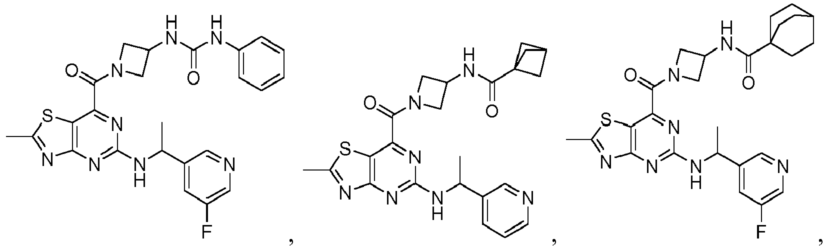 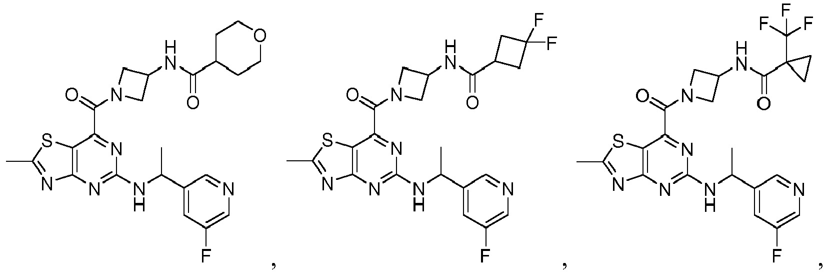 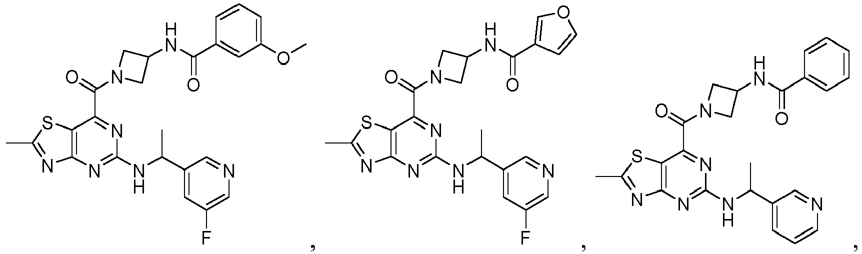 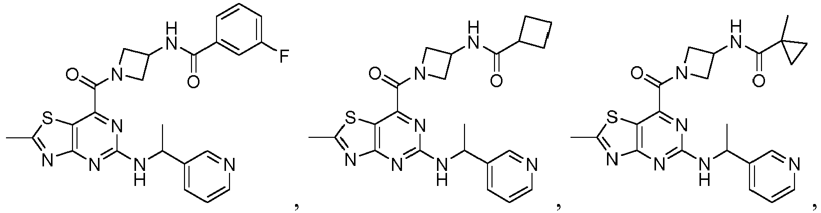 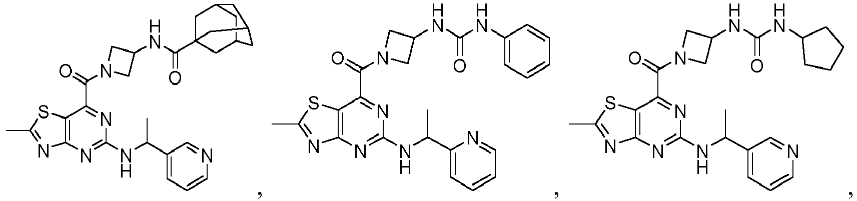 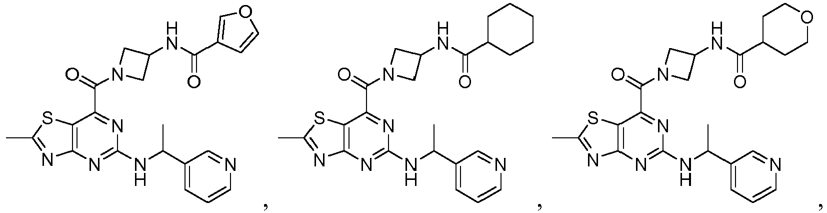 or 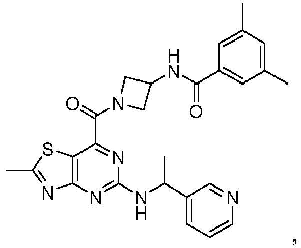 or a pharmaceutically acceptable salt thereof;
optionally wherein:
**(i)** the compound is: 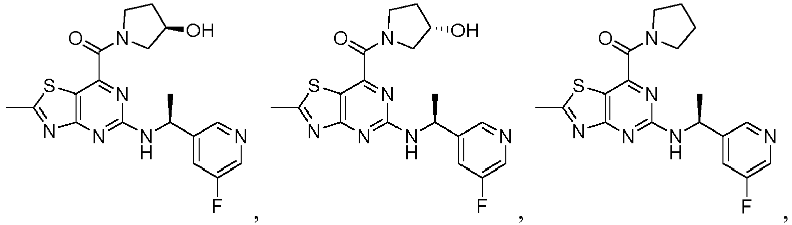 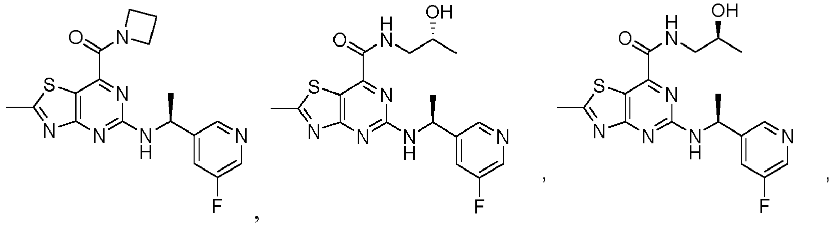 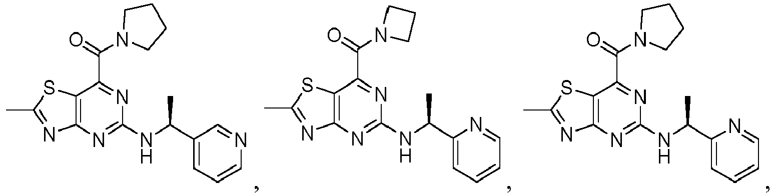 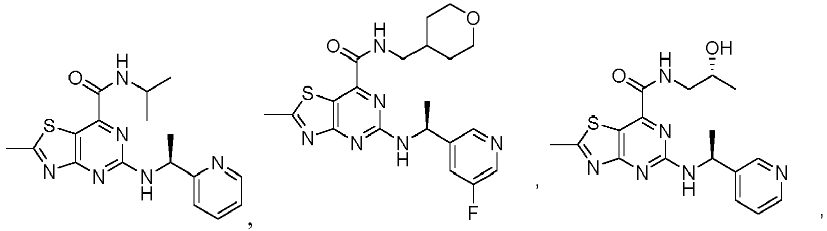 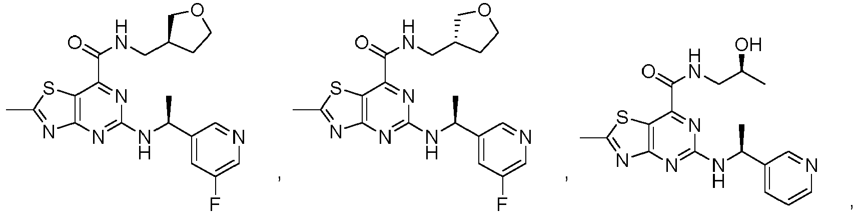 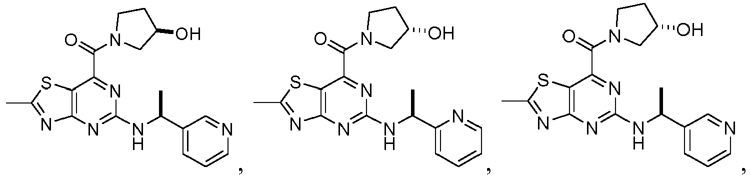 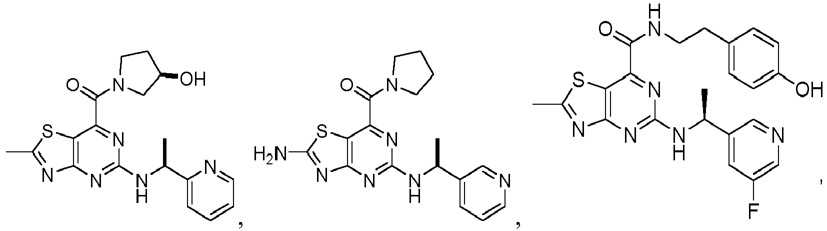 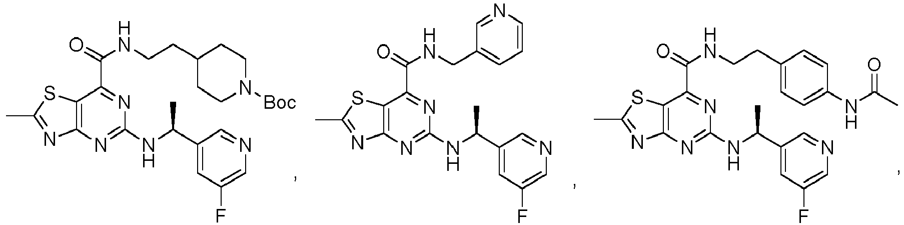 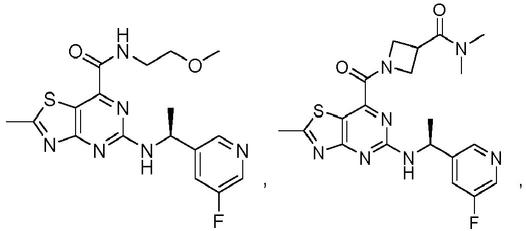 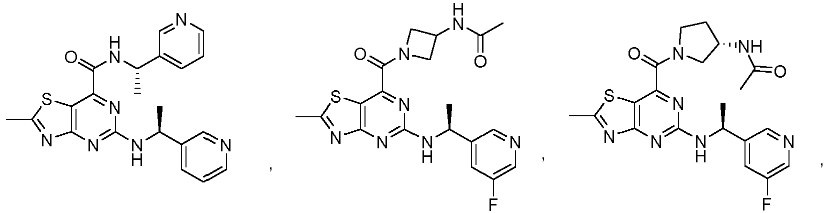 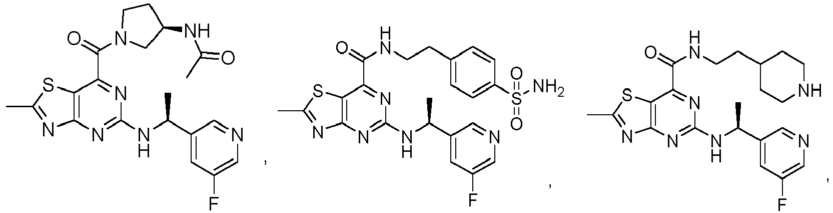 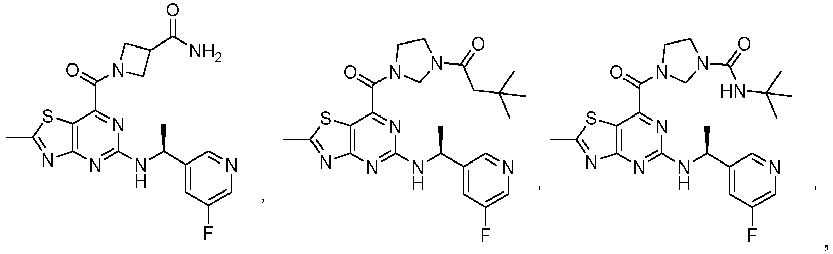 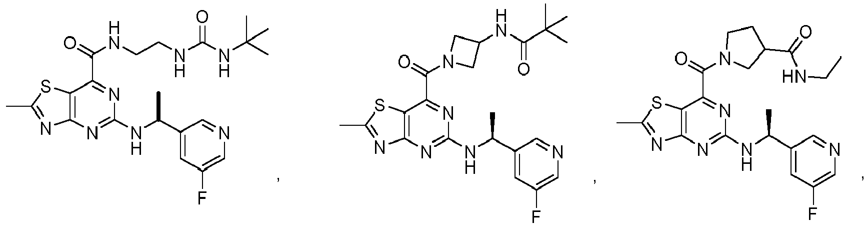 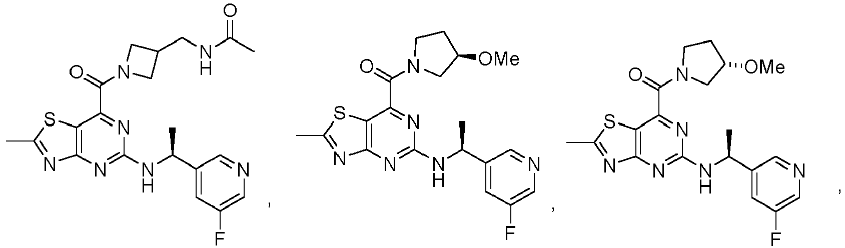 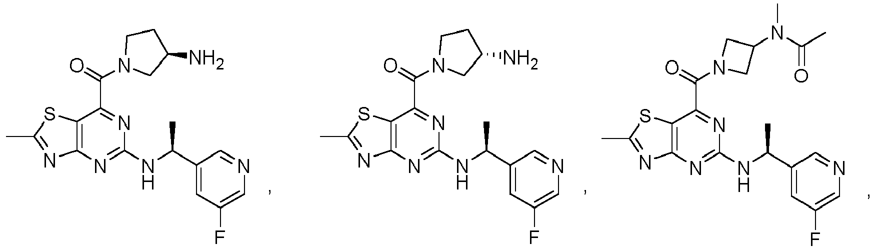 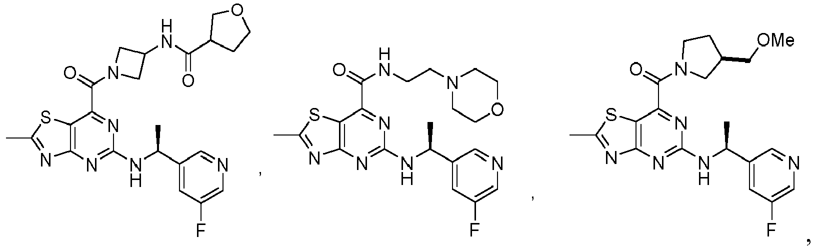 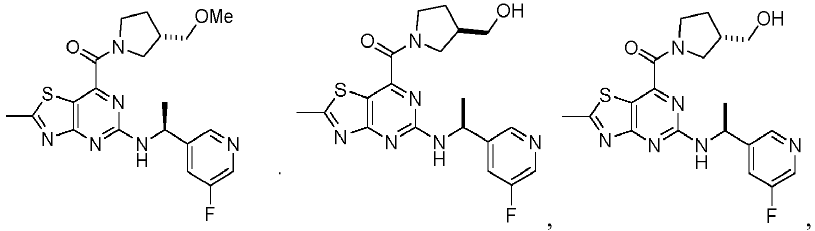 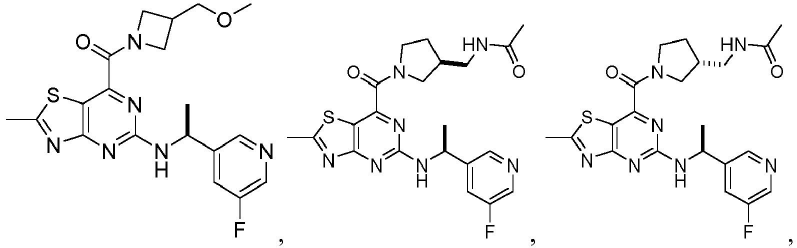 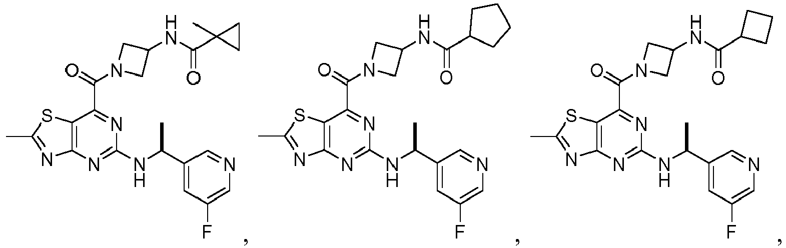 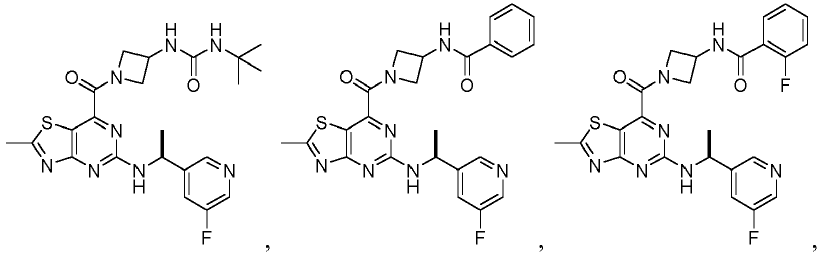 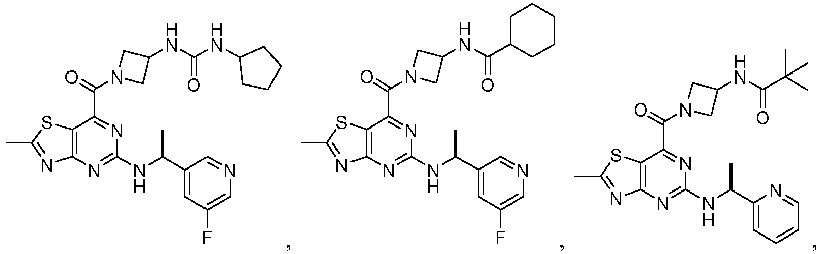 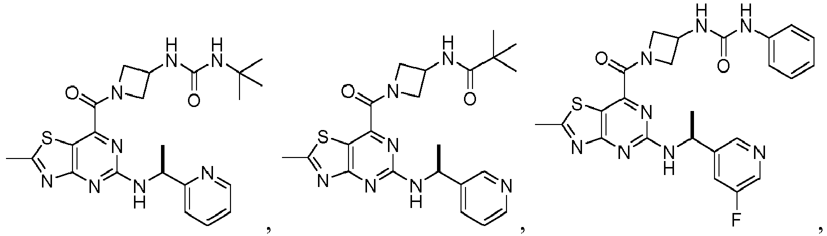 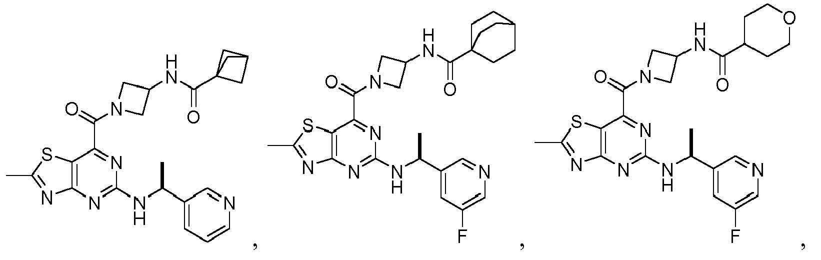 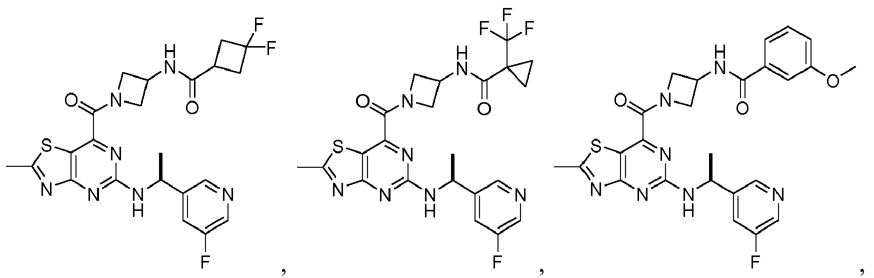 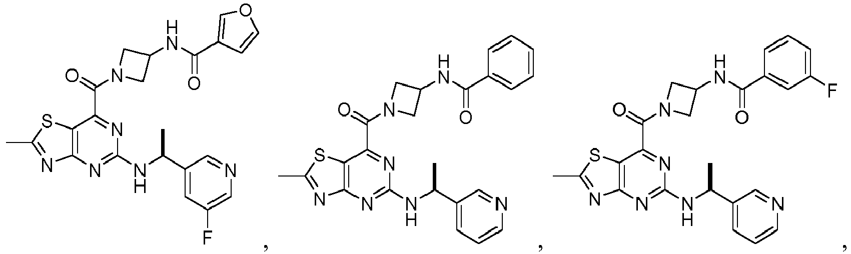 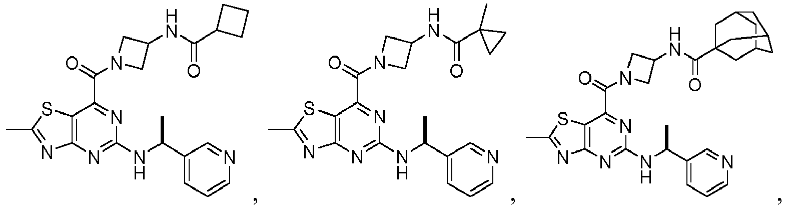 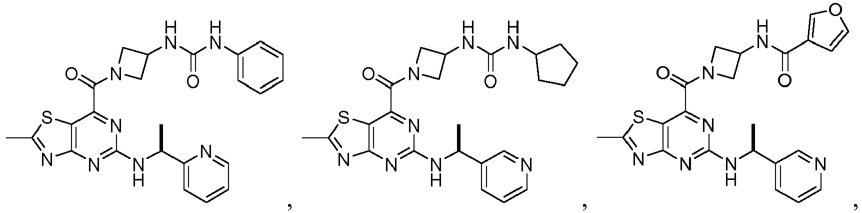 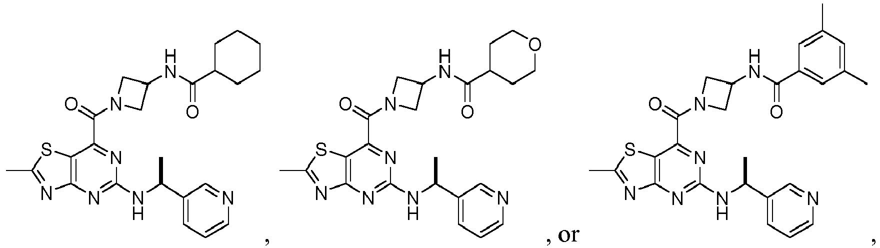 or a pharmaceutically acceptable salt thereof; or
**(ii)** the compound is: 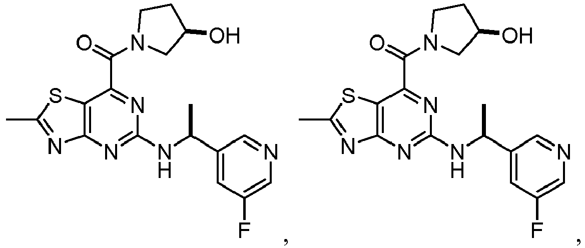 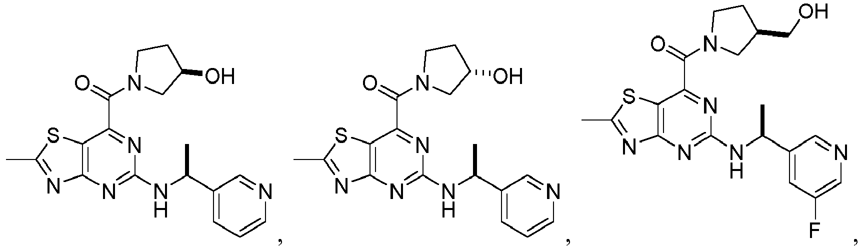 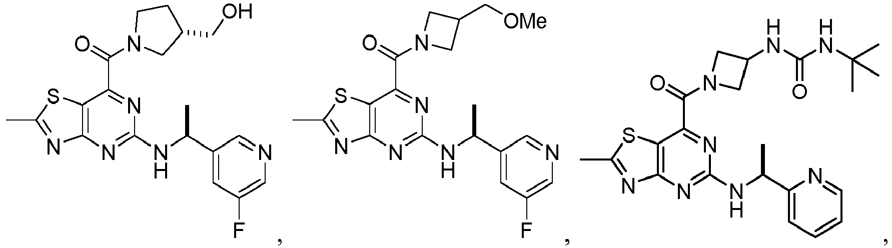 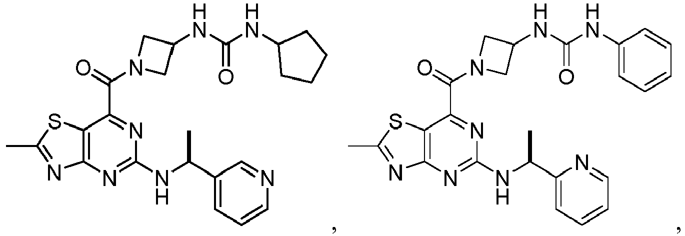 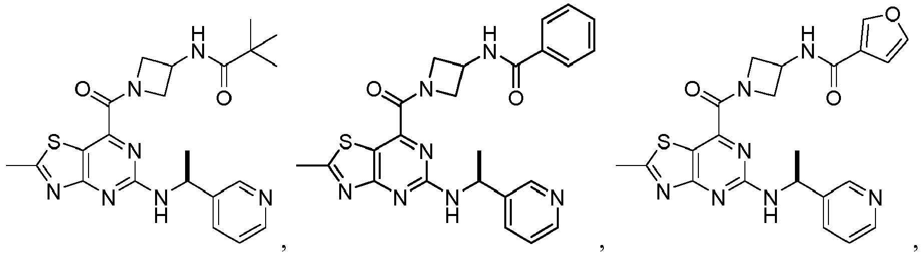 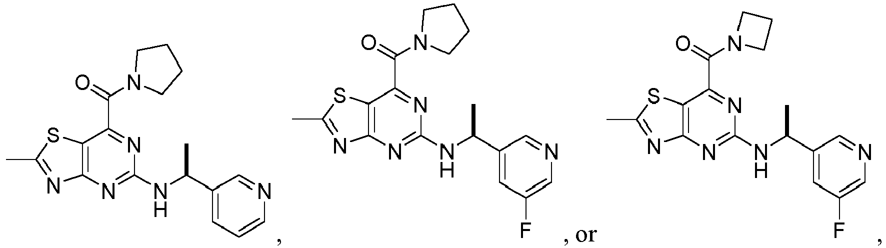 or a pharmaceutically acceptable salt thereof; or
**(iii)** the compound is: 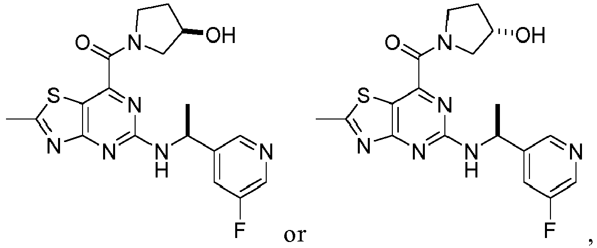 or a pharmaceutically acceptable salt thereof.

15. A pharmaceutical composition comprising the compound of any one of claims 1 to 14, or a pharmaceutically acceptable salt thereof, and a pharmaceutically acceptable excipient.

16. A compound of any one of claims 1 to 14, or a pharmaceutically acceptable salt thereof, for use as a medicament.

17. A compound of any one of claims 1 to 14, or a pharmaceutically acceptable salt thereof, for use in the treatment of a disease or disorder associated with Adenosine A2B Receptor activity and/or Adenosine A2A Receptor activity;
optionally wherein:
**(i)** the disease or disorder is a cancer, for example breast cancer, lung cancer, colon cancer, bladder cancer, kidney cancer, liver cancer, pancreatic cancer, melanoma, leukemia, lymphoma, or prostate cancer; or
**(ii)** the disease or disorder is a neurodegenerative disorder; or
**(iii)** the disease or disorder is a fibrotic disorder.

18. A compound of any one of claims 1 to 14, or a pharmaceutically acceptable salt thereof, for use in the treatment of a cancer, neurodegenerative disorder, or fibrotic disorder;
optionally wherein the cancer is breast cancer, lung cancer, colon cancer, bladder cancer, kidney cancer, liver cancer, pancreatic cancer, melanoma, leukemia, lymphoma, or prostate cancer.
